# EUROPEAN PATENT SPECIFICATION

(11) **EP 1 660 131 B1**
(45) Date of publication and mention of the grant of the patent: **24.06.2009**
(21) Application number: 04741321.6
(22) Date of filing: 29.07.2004
(51) Int. Cl.: A61K 45/06, A61K 31/00, A61P 3/04, A61P 3/10

(54) **ACTIVE SUBSTANCE COMBINATION COMPRISING A COMPOUND WITH NPY RECEPTOR AFFINITY AND A COMPOUND WITH 5-HT6 RECEPTOR AFFINITY**
WIRKSUBSTANZKOMBINATION MIT EINER VERBINDUNG MIT NPY REZEPTOR-AFFINITÄT UND EINER VERBINDUNG MIT 5-HT6 REZEPTOR-AFFINITÄT
COMBINAISON DE SUBSTANCES ACTIVES COMPORTANT UN COMPOSE A AFFINITE DE RECEPTEUR NPY ET UN COMPOSE A AFFINITE DE RECEPTEUR 5-HT6

(30) Priority: 30.07.2003 ES 200301815
(43) Date of publication of application: 31.05.2006
(73) Proprietor: LABORATORIOS DEL DR. ESTEVE, S.A., 08041 Barcelona (ES)
(72) Inventor: TORRENS, Jover, Antoni, E-08221 Terrasa (Barcelona) (ES); MAS PRIO, Josep, E-08191 Rubi (Barcelona) (ES); DORDAL ZUERAS, Alberto, E-08016 Barcelona (ES); CODONY SOLER, Xavier, E-08301 Mataro (Barcelona) (ES); MERCE VIDAL, Ramon, E-08021 Barcelona (ES); AURELIO CASTRILLO PEREZ, Jose, E-08032 Barcelona (ES); FRIGOLA CONSTANSA, Jordi, E-08960 Sant Just Desvern (Barcelona) (ES); BUSCHMANN, Helmut-Heinrich, E-08950 Esplugues de Llobregat (Barcelon (ES)
(74) Representative: Ponti Sales, Adelaida
(86) International application number: PCT/EP2004/008514
(87) International publication number: WO 2005/014045

(56) References cited:
- WO-A-97/35881
- WO-A-03/039547
- WO-A-03/042175
- WO-A-03/084952

## Description

The present invention relates to an active substance combination comprising at least one compound with neuropeptide Y-receptor affinity, preferably neuropeptide Y5-receptor affinity, and at least one compound with 5-HT₆ receptor affinity, a medicament comprising said active substance combination, and the use of said active substance combination for the manufacture of a medicament.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma, et al., Mol. Pharmacol., 1993, 43, 320; M. Ruat, et al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacol. Exp. Ther., 1995, 274, 173; A:J. Sleight, et al., Behav. Brain Res. , 1996, 73, 245; T.A. Branchek, et al., Annu. Rev. Pharmacol. Toxicol. , 2000, 40, 319; C. Routledge, et al., Br. J. Pharmacol. , 2000, 130, 1606]. It has been shown that typical and atypical antipsychotic drugs for treating schizophrenia have a high affinity for 5-HT₆ receptors [B.L. Roth, et al., J. Pharmacol. Exp. Ther. , 1994, 268, 1403; C.E. Glatt, et al., Mol. Med. , 1995, 1, 398; F.J. Mosma, et al., Mol. Pharmacol. , 1993, 43, 320; T. Shinkai, et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol., 2000, 130, 1597; C. Gérard, et al., Brain Research , 1997, 746, 207; M.R. Pranzatelli, Drugs of Today , 1997, 33, 379].

Moreover, it has been shown that the 5-HT₆ receptor also plays a role in food ingestion [Neuropharmacology, 41, 2001, 210-219].
Food ingestion disorders, particularly obesity, are a serious, fast growing threat to the health of humans of all age groups, since they increase the risk of developing other serious, even life-threatening diseases such as diabetes or coronary diseases.

Neuropeptide Y (NPY), first isolated in porcine brain extracts (Tatemoto et. al. Nature 1982, 296, 659), is a 36-aminoacid peptide belonging to the family of pancreatic polypeptides, and is one of the most abundant peptides in the brain and in the central nervous system. In addition, NPY is also distributed in several parts of the peripheral nervous system.

Several studies suggest a significant role of NPY in food ingestion regulation and particularly in food dysfunctions like obesity, anorexia and bulimia. Specifically, NPY is a powerful stimulant of food ingestion. Thus, appetite is significantly increased when NPY is injected directly into the CNS of satiated mice (Clark J. T. et. al. Endocrinology 1984, 115, 427; Levine A. S. et. al. Peptides 1984, 5, 1025; Stanley B. G. et. al. Life Sci. 1984, 35, 2635; Stanley B. G. et. al. Proc. Nat. Acad. Sci. USA 1985, 82, 3940). On the other hand, NPY may play a role in cognitive function regulation, e. g. memory (Flood J. F. et. al. Brain Res. 1987, 421, 280; Redrobe J. P. et. Al. Brain Res. 1999, 848, 153), and be active in anxiety (Heilig M. et. al. Reg. Peptides 1992, 41, 61) and depression (Heilig M. et. al. Eur. J. Pharmacol. 1988, 147, 465) processes.

NPY is also distributed in the peripheral system. Some studies suggest that it might be involved in hypertensive (Michel M. C: et. al. J. Hypertens. 1995, 13, 385), and analgesic (Gehlert D. R. Life Sci. 1994, 55, 551) processes, among others.

The endogenous proteins that constitute NPY-binding receptors have been widely studied. Several have been cloned and expressed. At present, six different receptor subtypes, named Y1 to Y6, are recognized (Hispkind P. A. et. al. Annu. Rep. Med. Chem. 1996, 31, 1; Grundemar L. et. al. TIPS Reviews., 15, 153, 1994). Each NPY receptor subtype is generally associated to a different biological activity. For example, Y2 receptor is involved in the induction of convulsions in rats (Dumont Y. et. al. Brit. J. Pharmacol. 2000, 129, 1075).

The most recently identified receptor is Y5 (Hu et. al. J. Biol. Chem. 1996, 271, 26315). There is evidence that Y5 receptor has a unique pharmacological profile related to food ingestion as compared to the other receptor subtypes. The fact that [D-Trp³²]NPY peptide, a selective Y5-receptor agonist with no affinity for Y1 receptor, stimulates food ingestion in rats (Gerald C. et. al. Nature, 1996, 382, 168), supports the hypothesis that Y5 receptor is related to exaggerated food consumption. Consequently, compounds having an affinity to the Y5 receptor should be effective to inhibit food ingestion and very useful to control diseases like obesity or other disorders of food ingestion (food intake), such as anorexia, bulimia, cachexia or type II diabetes. Moreover, it has been suggested that such compounds are useful to control diseases such as arthritis or epilepsy.

Whereas known compounds with NPY-receptor affinity and known compounds with 5-HT₆ receptor affinity are generally effective for treating disorders related to NPY-receptors and to 5-HT₆ receptors respectively, in some instances they show undesirable side effects.

It was therefore an object of the present invention to provide a medicament suitable for the prophylaxis and/or treatment of disorders related to NPY-receptors, preferably NPY5-receptors, and to 5-HT₆ receptors, which preferably does not show the undesired side effects of the conventional compounds with NPY-receptor affinity or 5-HT₆ receptor affinity, or at least less frequent and/or less pronounced.

Said object has been achieved by providing an active substance combination comprising
(A) at least one compound with neuropeptide Y (NPY)-receptor affinity and
(B) at least one compound with 5-HT₆ receptor affinity.

It has surprisingly been found that the compounds with NPY-receptor affinity and the compounds with 5-HT₆ receptor affinity show a synergic effect in their pharmacological activities. Consequently, the dose of the corresponding compounds may be reduced in comparison to the dose necessary for an individual administration of said compounds.

The active substance combination of the present invention comprises as a component (A) at least one compound with neuropeptide Y5.

(NPY5)-receptor affinity, which is a 1,4-disubstituted piperidine compound of general formula (Ia), 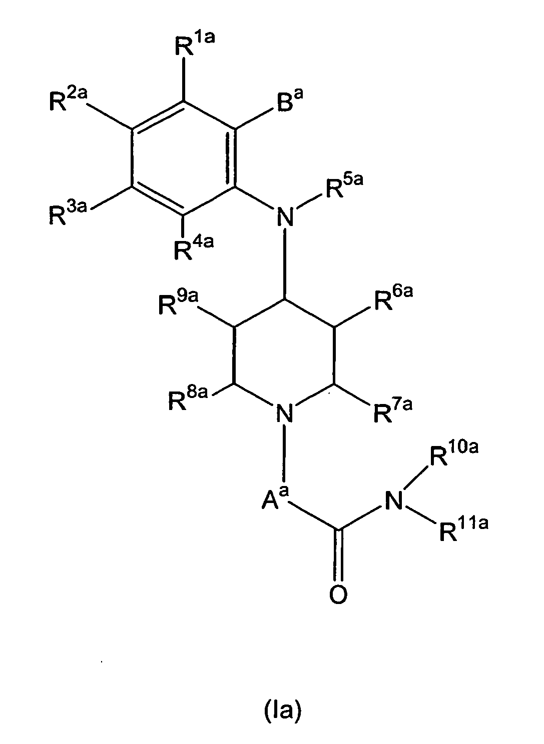 wherein
R^{1a}, R^{2a}, R^{3a}, R^{4a} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, -OR^{12a}, -O-(C=O)R^{13a}, (C=O)-OR^{13a},-SR^{14a}, -SOR^{14a}, -SO₂R^{14a}, -NH-SO₂R^{14a}, -SO₂NH₂ and -NR^{15a}R^{16a} moiety, R^{5a} represents hydrogen, an unbranched or branched, saturated or unsaturated, optionally at least mono-substituted aliphatic radical, or a saturated or unsaturated, optionally at least mono-substituted cycloaliphatic radical,
R^{6a}, R^{7a}, R^{8a}, R^{9a} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano-moiety and a -COOR^{17a} moiety,
A^{a} represents a bridge member -CHR^{18a}- or -CHR^{18a}-CH₂-,
B^{a} represents an unbranched or branched, saturated or unsaturated, optionally at least mono-substituted aliphatic radical, a saturated or unsaturated, optionally at least mono-substituted cycloaliphatic radical, a COOR^{19a}-moiety, a - (C=O)R^{20a}-moiety, or a -CH₂OR^{23a}-moiety,
R^{10a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11a} represents an unbranched or branched, saturated or unsaturated, optionally at least mono-substituted aliphatic radical, a saturated or unsaturated, optionally at least mono-substituted, optionally at least-one heteroatom as ring member containing cycloaliphatic radical, which may be bonded via an optionally at least mono-substituted alkylene group and/or may be condensed with an optionally at least mono-substituted mono- or polycyclic ringsystem, or an optionally at least mono substituted aryl- or heteroaryl radical, which may be bonded via an optionally at least mono-substituted alkylene group and/or may be condensed with an optionally at least mono-substituted mono- or polycyclic ringsystem, or
R^{10a} and R^{11a} together with the bridging nitrogen atom form an optionally at least mono-substituted, saturated, unsaturated or aromatic heterocyclic ring that may contain at least one further heteroatom as a ring member and/or may be condensed with an optionally at least mono-substituted mono- or polycyclic ringsystem,
R^{12a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{14a} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{15a} and R^{16a} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{15a} and R^{16a} together with the bridging nitrogen atom form a saturated, unsaturated or aromatic heterocyclic ring, which may be at least mono-substituted and/or contain at least one further heteroatom as a ring member,
R^{17a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{18a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{19a} represents hydrogen, an unbranched or branched, saturated or unsaturated, optionally at least mono-substituted aliphatic radical, a saturated or unsaturated, optionally at least mono-substituted cycloaliphatic radical, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{20a} represents hydrogen, an unbranched or branched, saturated or unsaturated, optionally at least mono-substituted aliphatic radical, a saturated or unsaturated, optionally at least mono-substituted cycloaliphatic radical, an optionally at least mono-substituted aryl- or heteroaryl radical, which may be bonded via an optionally at least mono-substituted alkylene group and/or may be condensed with an optionally at least mono-substituted mono- or polycyclic ring-system, or a NR^{21a}R^{22a}-moiety,
R^{21a} represents hydrogen, an unbranched or branched, saturated or unsaturated, optionally at least mono-substituted aliphatic radical, a saturated or unsaturated, optionally at least mono-substituted cycloaliphatic radical, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{22a} represents hydrogen, an unbranched or branched, saturated or unsaturated, optionally at least mono-substituted aliphatic radical, a saturated or unsaturated, optionally at least mono-substituted cycloaliphatic radical, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{23a} represents hydrogen, an unbranched or branched, saturated or unsaturated, optionally at least mono-substituted aliphatic radical, which may comprise at least one heteroatom as a chain member, or a -(C=O)R^{13a}-moiety,
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a physiologically acceptable salt thereof, or a corresponding solvate.

A mono- or polycyclic ring-system according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

Those skilled in the art understand that the term "condensed" indicates that the condensed rings share more than one atom. The terms "annulated" or "fused" may also be used for this type of bonding.

If one or more of the residues R^{1a}-R^{23a} and B^{a} represents an aliphatic radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, - SO₂-C₁₋₄-alkyl, -NH-SO₂-C₁₋₄-alkyl, wherein the C₁₋₄-alkyl may in each case be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and an unsubstituted phenyl radical. If any one of these substituents itself is at least mono-substituted, said substituents may preferably be selected from the group consisting of F, Cl, methyl and methoxy.

If one or more of the residues R^{1a}-R^{22a} and B^{a} represents or comprises a cycloaliphatic radical, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, phenoxy, benzoyl, cyclohexyl, branched or unbranched C₁₋₄-perfluoroalkyl, -NR^{Aa}R^{Ba} wherein R^{Aa}, R^{Ba} are each independently selected from the group consisting of H, a branched or unbranched C₁₋₄-alkyl-radical, -CH₂-CH₂-OH and phenyl, carboxy, amido, cyano, nitro, -SO₂NH₂, -CO-C₁₋₄-alkyl, - CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may in each case be branched or unbranched, unsubstituted or at least mono-substituted phenyl or naphthyl and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, methoxy, ethoxy, benzoyl, phenoxy, cyclohexyl, -CF₃, -CO-CH₃, -CO-OCH₃, -NR^{Aa}R^{Ba} wherein R^{Aa}, R^{Ba} are each independently selected from the group consisting of H, a branched or unbranched C₁₋₄-alkyl-radical, -CH₂-CH₂-OH and phenyl, and an unsubstituted phenyl radical. If any one of these substituents itself is at least mono-substituted, said substituents may preferably be selected from the group consisting of F, Cl, methyl and methoxy.

If one or more of the residues R^{1a}-R^{4a} and R^{10a}-R^{18a} comprises an alkylene group, which is substituted by one or more substituents, unless defined otherwise, each of these substituents may preferably be selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, - SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and unsubstituted phenyl. If any one of these substituents itself is at least mono-substituted, said substituents may preferably be selected from the group consisting of F, Cl, methyl and methoxy.

If one or more of the residues R^{1a}-R^{4a} and R^{10a}-R^{22a} comprises a mono- or polycyclic ringsystem, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, branched or unbranched C₁₋₄-perfluoroalkyl, amino, carboxy, amido, cyano, keto (=O), nitro, -SO₂NH₂, -CO-C₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl, more preferably from the group consisting of hydroxy, F, Cl, Br, methyl, ethyl, methoxy, ethoxy, CF₃, keto, cyano and an unsubstituted phenyl radical. If any one of these substituents itself is at least mono-substituted, said substituents may preferably be selected from the group consisting of F, Cl, methyl and methoxy.

If one or more of the residues R^{1a}-R^{4a} and R^{10a}-R^{22a} represents or comprises an aryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{Aa}R^{Ba} wherein R^{Aa}, R^{Ba} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂ -CO-C₁₋₄-alkyl, -CO-OC₁₋₄-alkyl, -SO-Cₗ-₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, -CH(OH)(phenyl), methoxy, ethoxy, unsubstituted or at least mono-substituted benzoyl, unsubstituted or at least mono-substituted phenoxy, cyclohexyl, CF₃, -CO-CH₃, -CO-OCH₃, -NR^{Aa}R^{Ba} wherein R^{Aa}, R^{Ba} are each independently selected from the group consisting of H, a branched or unbranched C₁₋₄-alkyl-radical, -CH₂-CH₂-OH and phenyl, and an unsubstituted phenyl radical. If any of these substituents itself is at least mono-substituted, said substituents may preferably be selected from the group consisting of F, Cl, methyl and methoxy.

If one or more of the residues R^{1a}-R^{4a} and R^{10a}-R^{22a} represents or comprises a heteroaryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{Aa}R^{Ba} wherein R^{Aa}, R^{Ba} are each independently selected from the group consisting of H, a branched or unbranched C₁₋₄-alkyl-radical, -CH₂-CH₂-OH and phenyl, carboxy, amido, cyano, nitro, -CH(OH)(phenyl), -SO₂NH₂, -CO-C₁₋₄-alkyl, -CO-OC₁₋₄-alkyl, SO-C₁₋₄-alkyl, SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, methoxy, ethoxy, unsubstituted or at least mono-substituted benzoyl, unsubstituted or at least mono-substituted phenoxy, cyclohexyl, CF₃, -CH(OH)(phenyl), -CO-CH₃, -CO-OCH₃, -NR^{Aa}R^{Ba} wherein R^{Aa}, R^{Ba} are each independently selected from the group consisting of H, a branched or unbranched C₁₋₄-alkyl-radical, -CH₂-CH₂-OH and phenyl, and an unsubstituted phenyl radical. If any one of these substituents itself is at least mono-substituted, said substituents may preferably be selected from the group consisting of F, Cl, methyl and methoxy.

If R^{10a} and R^{11a} and/or R^{15a} and R^{16a} form a heterocyclic ring, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, - SO₂NH₂, -CO-C₁₋₄-alkyl, -SO-C₁₋₄-alkyl, -SO₂-C₁₋₄-alkyl, -NH-SO₂-C₁₋₄-alkyl,
wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, methyl, CF₃ and an unsubstituted phenyl radical. If any of these substituents itself is at least mono-substituted, said substituents may preferably be selected from the group consisting of F, Cl, methyl and methoxy.

If R^{10a} and R^{11a} and/or R^{15a} and R^{16a} form a heterocyclic ring, which contains one or more further heteroatoms as ring members, unless defined otherwise, each of these heteroatoms may preferably be selected from the group consisting of N, O and S, more preferably from the group consisting of N and O.

If one or more of the residues R^{1a}-R^{22a} and B^{a} represents or comprises a cycloaliphatic radical, which contains one or more heteroatoms as ring members, unless defined otherwise, each of these heteroatoms may preferably be selected from the group consisting of N, O, S and P, more preferably from the group consisting of N, O and S.

If one or more of the residues R^{1a}-R^{4a} and R^{10a}-R^{22a} represents or comprises an heteroaryl radical, which contains one or more heteroatoms as ring members, unless defined otherwise, each of these heteroatoms may preferably be selected from the group consisting of N, O, S and P, more preferably from the group consisting of N, O and S.

If R^{23a} represents an aliphatic radical, which comprises at least one heteroatom as a chain member, each of these heteroatoms may preferably be O or S, more preferably O.

Preferred compounds of general formula (Ia) are those, wherein R^{1a}, R^{2a}, R^{3a}, R^{4a} are each independently selected from the group consisting of H, F, Cl, Br, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a nitro group, a cyano group, - OR^{12a}, -O(C=O)R^{13a}, -(C=O)-OR^{13a}, -SR^{14a}, -SOR^{14a}, -SO₂R^{14a}, -NH-SO₂R^{14a}, - SO₂NH₂ and -NR^{15a}R^{16a} moiety,
R^{5a} represents hydrogen, an unbranched or branched, saturated or unsaturated, optionally at least mono-substituted C₁₋₆-aliphatic radical, or a saturated or unsaturated, optionally at least mono-substituted C₃₋₈-cycloaliphatic radical,
R^{6a}, R^{7a}, R^{8a} , R^{9a} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, a cyano-moiety and a COOR^{17a} moiety,
A^{a} represents a bridge member -CHR^{18a}- or -CHR^{18a}-CH₂-,
B^{a} represents an unbranched or branched, saturated or unsaturated, optionally at least mono-substituted C₁₋₆-aliphatic radical, a saturated or unsaturated, optionally at least mono-substituted C₃₋₈-cycloaliphatic radical, a COOR^{19a}-moiety, a COR^{20a}-moiety, or a -CH₂-OR^{23a}-moiety,
R^{10a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
R^{11a} represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ringsystem, or an optionally at least mono substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, or
R^{10a} and R^{11a} together with the bridging nitrogen atom form an optionally at least mono-substituted, saturated, unsaturated or aromatic, 5- or 6-membered heterocyclic ring, which may contain at least one further heteroatom as a ring member and/or may be condensed with an optionally at least mono-substituted mono- or polycyclic ringsystem,
R^{12a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as a ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
R^{13a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
R^{14a} represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
R^{15a} and R^{16a} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
or R^{15a} and R^{16a} together with the bridging nitrogen atom form a saturated, unsaturated or aromatic, 5- or 6-membered heterocyclic ring, which may be at least mono-substituted and/or contain at least one further heteroatom as a ring member,
R^{17a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system,
R^{18a} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
R^{19a} represents hydrogen, an unbranched or branched, saturated or unsaturated, optionally at least mono-substituted C₁₋₆-aliphatic radical, a saturated or unsaturated, optionally at least mono-substituted C₃₋₈ cycloaliphatic radical, or an optionally at least mono-substituted, 5- or 6-membered aryl- or heteroaryl radical, which may be condensed with an optionally at least mono-substituted mono- or polycyclic ring-system,
R^{20a} represents hydrogen, an unbranched or branched, saturated or unsaturated, optionally at least mono-substituted C₁₋₆-aliphatic radical, a saturated or unsaturated, optionally at least mono-substituted C₃₋₈ cycloaliphatic radical, an optionally at least mono-substituted, 5- or 6-membered aryl- or heteroaryl radical, which may be condensed with an optionally at least mono-substituted mono- or polycyclic ring-system, or a NR^{21a}R^{22a}-moiety,
R^{21a} represents hydrogen, an unbranched or branched, saturated or unsaturated, optionally at least mono-substituted C₁₋₆-aliphatic radical, a saturated or unsaturated, optionally at least mono-substituted C₃₋₈ cycloaliphatic radical, or an optionally at least mono-substituted, 5- or 6-membered aryl- or heteroaryl radical, which may be condensed with an optionally at least mono-substituted mono- or polycyclic ring-system,
R^{22a} represents hydrogen, an unbranched or branched, saturated or unsaturated, optionally at least mono-substituted C₁₋₆-aliphatic radical, a saturated or unsaturated, optionally at least mono-substituted C₃₋₈ cycloaliphatic radical, or an optionally at least mono-substituted, 5- or 6-membered aryl- or heteroaryl radical, which may be condensed with an optionally at least mono-substituted mono- or polycyclic ring-system,
R^{23a} represents hydrogen, an unbranched or branched, saturated or unsaturated, optionally at least mono-substituted C₁₋₆-aliphatic radical, which may comprise at least one heteroatom as a chain member, or a -(C=O)R^{13a}-moiety,
optionally in form of one of their stereoisomers, preferably enantiomers or diastereomers, their racemates or in form of a mixture of at least two of its stereoisomers, preferably enantiomers or diastereomers, in any mixing ratio, or physiologically acceptable salts thereof, or corresponding solvates, respectively.

Particularly preferred are compounds of general formula (Ia), wherein R^{1a}, R^{2a}, R^{3a}, R^{4a} are each independently selected from the group consisting of H, F, Cl, Br, a saturated or unsaturated, branched or unbranched, optionally at least mono-substituted C₁₋₃-aliphatic radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group, a nitro group, a cyano group, -OR^{12a},-OC(=O)R^{13a}, -SR^{14a} and -NR^{15a}R^{16a} moiety, preferably are each independently selected from the group consisting of H, F, Cl, CH₃, CH₂CH₃, CF₃, CF₂CF₃, cyclopentyl, cyclohexyl, a nitro group, a cyano group and -OR^{12a} and the remaining residues R^{5a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or correspoding solvates, respectively.

Also particularly preferred are compounds of general formula (Ia), wherein R^{5a} represents H or a branched or unbranched C₁₋₃-alkyl radical, preferably H, CH₃ or CH₂CH₃, and the remaining residues R^{1a}-R^{4a}, R^{6a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{6a}, R^{7a}, R^{8a}, R^{9a} are each independently selected from the group consisting of H, a branched or unbranched C₁₋₃-alkyl radical, a cyano and a COOR^{17a} moiety, preferably selected from the group consisting of H, CH₃, CH₂CH₃ and a cyano moiety, more preferably all represent H, and the remaining residues R^{1a}-R^{5a}, R^{10a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein B^{a} represents a branched or unbranched, optionally at least mono-substituted C₁₋₃-alkyl radical, a COOR^{19a}-moiety, or a CH₂OR^{23a}-moiety, preferably a COOR^{19a}-moiety, a CH₂OR^{23a-}moiety or a d₁₋₂-alkyl radical, more preferably a COOR^{19a}-moiety or a CH₂OR^{23a}-moiety, and the remaining residues R^{1a}-R^{23a} and A^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{10a} represents hydrogen or a branched or unbranched C_{1- 4}-alkyl radical, more preferably hydrogen, and the remaining residues R^{1a}-R^{9a}, R^{11a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or solvates, respectively.

Also particularly preferred are compounds of general formula (Ia), wherein R^{11a} is selected from the group consisting of an unsubstituted phenyl radical, a phenyl radical optionally at least mono-substituted with a branched or unbranched C₁₋₄-alkyl-radical, a branched or unbranched C₁₋₄-alkoxy-radical, a branched or unbranched C₁₋₄-perfluoroalkyl-radical, a branched or unbranched C₁₋₄-perfluoroalkoxy-radical, F, Cl, Br, cyclohexyl, phenyl, phenoxy, phenylthio, benzoyl, cyano, -C(=O)C₁₋₂-alkyl, -C(=O)OC₁₋₂-alkyl, -carboxy,-CH(OH)(phenyl), -NR^{Aa}R^{Ba} wherein R^{Aa}, R^{Ba} are each independently selected from the group consisting of H, a branched or unbranched
C₁₋₄-alkyl-radical, -CH₂-CH₂-OH and an unsubstituted phenyl radical,
an unsubstituted thiazole radical,
a group of general formula (Aa) 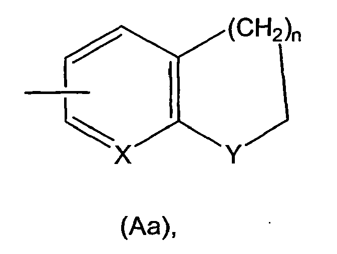 wherein
n is 1 or 2,
X represents CH or N,
Y represents CH₂, O, N-R^{C}, CH-OH or C(=O),
R^{C} is H or a branched or unbranched C₁₋₄-alkyl radical,
a group of formula (Ba), 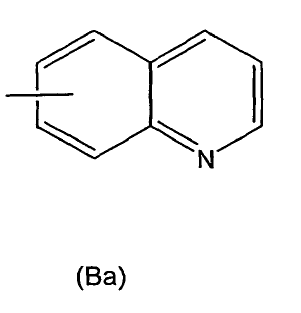 a group of formula (Ca), 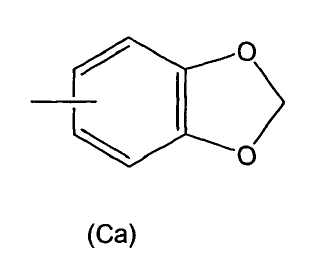 a group of general formula (Da), 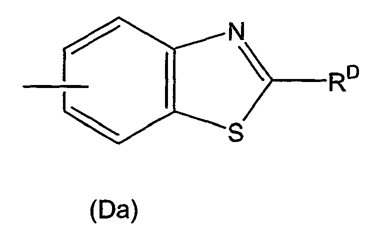 wherein R_{D} is H or a branched or unbranched C₁₋₄-alkyl radical and a group of general formula (Ea), 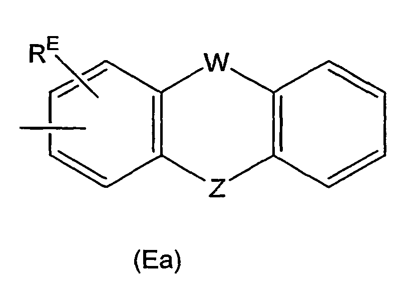 wherein
R^{E} represents H, a branched or unbranched C₁₋₄-alkyl radical or a branched or unbranched C₁₋₄-alkoxy radical,
W represents a bond between the two aromatic rings, CH₂, CH-OH or C(=O),
Z represents CH₂, O, S, CH-OH, C(=O) or N-R^{F} where R^{F} represents H or a branched or unbranched C₁₋₄-alkyl-radical, and the remaining residues R^{1a}-R^{10a}, R^{12a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{10a} and R^{11a} together with the bridging nitrogen atom form a saturated, 6-membered heterocyclic ring, which is optionally at least mono-substituted with a methyl radical and/or condensed with an unsubstituted or at least mono-substituted phenyl- or cyclohexyl-radical, said phenyl- or cyclohexyl-radical preferably being at least mono-substituted with F and/or OCH₃, and the remaining residues R^{1a}-R^{9a}, R^{12a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{12a} represents H, an unbranched or branched C₁₋₄-alkyl radical, a cyclohexyl radical or a phenyl radical, preferably H, CH₃, C₂H₅ or a phenyl radical, and the remaining residues R^{1a}-R^{11a}, R^{13a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{13a} represents H, a an unbranched or branched C₁₋₄-alkyl radical, a cyclohexyl radical or a phenyl radical, preferably H, CH₃, C₂H₅ or phenyl, and the remaining residues R^{1a}-R^{12a}, R^{14a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{14a} represents H, a an unbranched or branched C₁₋₄-alkyl radical, a cyclohexyl radical or a phenyl radical, preferably H, CH₃, C₂H₅ or phenyl, and the remaining residues R^{1a}-R^{13a}, R^{15a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{15a} and R^{16a} are each independently selected from the group consisting of H, an unbranched or branched C₁₋₄-alkyl radical, a cyclohexyl radical and a phenyl radical, preferably from the group consisting of H, CH₃, C₂H₅ and phenyl, and the remaining residues R^{1a}-R^{14a}, R^{17a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{17a} represents H, an unbranched or branched C₁₋₄-alkyl radical, a cyclohexyl radical or a phenyl radical, preferably H, CH₃, C₂H₅ or phenyl, and the remaining residues R^{1a}-R^{16a}, R^{18a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{18a} represents H, an unbranched or branched C₁₋₄-alkyl radical or a phenyl radical, preferably H, CH₃ or phenyl, and the remaining residues R^{1a}-R^{17a}, R^{19a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{19a} represents H or an unbranched or branched C₁₋₄ alkyl radical, preferably H or a C₁₋₂ alkyl radical and the remaining residues R^{1a}-R^{18a}, R^{20a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{20a} represents H, an unbranched or branched C₁₋₄ alkyl radical or a NR^{21a}R^{22a}-moiety, preferably H, a C₁₋₂ alkyl radical or a NR^{21a}R^{22a}-moiety and the remaining residues R^{1a}-R^{19a}, R^{21a}-R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{21a} represents H or an unbranched or branched C₁₋₄ alkyl radical, preferably H or a C₁₋₂ alkyl radical and the remaining residues R^{1a}-R^{20a}, R^{22a}, R^{23a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{22a} represents H or an unbranched or branched C₁₋₄ alkyl radical, preferably H or a C₁₋₂ alkyl radical and the remaining residues R^{1a}-R^{21a}, R^{23a} , A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (Ia), wherein R^{23a} represents H or an unbranched or branched C₁₋₄ alkyl radical, preferably H or a C₁₋₂ alkyl radical and the remaining residues R^{1a}-R^{22a}, A^{a} and B^{a} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are compounds of general formula (I), wherein A represents a -CH₂-group and the remaining residues R¹-R²³ and B have the meaning given above, optionally in form of one of their stereoisomers, preferably enantiomers or diastereomers, their racemates or in form of a mixture of at least two of its stereoisomers, preferably enantiomers or diastereomers, in any mixing ratio, or salts, preferably physiologically acceptable salts thereof, or corresponding solvates, respectively.

Also particularly preferred are 1,4-disubstituted piperidine compounds of general formula (Ia) given above, wherein

R^{1a}, R^{2a}, R^{3a} , R^{4a} are each independently selected from the group consisting of H, F, Cl, Br, OH, CH₃ and OCH₃,
R^{5a} represents hydrogen,
R^{6a}, R^{7a}, R^{8a}, R^{9a} all represent H,
A^{a} represents -CH₂-,
B^{a} represents a -CH₂-OH or -(C=O)-O-CH₃ group,
R^{10a} represents hydrogen,
R^{11a} is selected from the group consisting of unsubstituted phenyl, phenyl that is optionally at least mono-substituted with one or more substituents independently selected from the group consisting cyclohexyl, phenyl, phenoxy, benzoyl, -C(=O)-C₁₋₂-alkyl, -C(H)(OH)(phenyl) and -C(H)(OH)(CH₃),
a group of general formula (Aa) 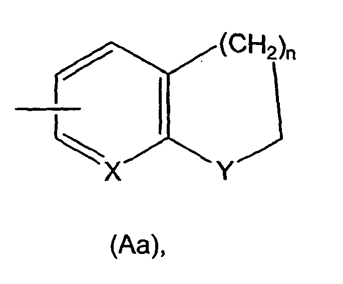 wherein
n is 1 or 2,
X represents CH,
Y represents CH-OH or C(=O),
a group of formula (Ba), 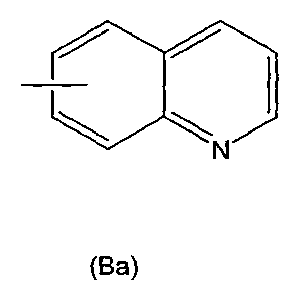 a group of formula (Ca), 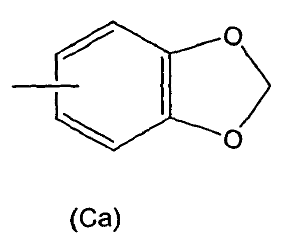 and a group of general formula (Ea), 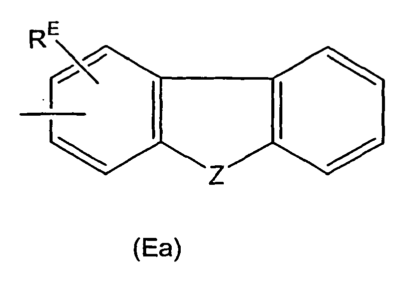 wherein
R^{E} represents H, a branched or unbranched C₁₋₄-alkyl radical or a branched or unbranched C₁₋₄-alkoxy radical,
Z represents CH₂, O, S, CH-OH, C(=O) or N-R^{F} where R^{F} represents H or an alkyl-radical selected from the group consisting of methyl, ethyl, n-propyl, iso-propyl, n-butyl, iso-butyl, sec-butyl and tert.-butyl,
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

Most preferred are the following 1,4-disubstituted piperidine compounds of general formula (Ia):
[1] N-(9-Ethyl-9H-carbazol-3-yl)-2-[4-(2-hydroxymethyl-6-methylphenylamino)-piperidine-yl]acetamide;
[2] 2-[4-(2-Hydroxymethyl-4-methyl-phenylamino)-piperidine-1-yl]-N-(9-oxo-9H-fluoren-3-yl)-acetamide;
[3] 2-[4-(2-Hydroxymethyl-6-methyl-phenylamino)-pipeddine-1-y-]-N-(9-oxo-9H-fluoren-3-yl)-acetamide;
[4] N-(9-Hydroxy-9H-fluoren-3-yl)-2-[4-(2-hydroxymethyl-4-methylphenylamino)-piperidine-1-yl]-acetamide;
[5] N-(9-Hydroxy-9H-fluoren-3-yl)-2-[4-(2-hydroxymethyl-6-methylphenylamino)-piperidine-1yl]-acetamide;
[6] 2-{1-[(9-Oxo-9H-fluoren-3ylcarbamoyl)-methyl]-piperidin-4-ylamino}benzoic acid methyl ester and
[7] 2-[4-(2-Hydroxymethyl-4-methyl-phenylamino)-piperidin-1-yl]-N-phenylacetamide,
[8] 2-[4-(2-Hydroxymethyl-phenylamino)-piperidin-1-yl]-N-(1-oxo-indan-5-yl)-acetamide,
optionally in form of a salt, preferably a physiologically acceptable salt, particularly preferably in form of a physiologically acceptable acid addition salt, most preferably a hydrochloride salt, or a corresponding solvate.

Also most preferred are the following 1,4-disubstituted piperidine compounds of general formula (Ia):

| N^{o} | |
|---|---|
| 1 | 2-[4-(2-Hydroxymethyl-phenylamino)-piperidin-1-yl]-N-quinolin-3-yl-acetamide |
| 2 | 2-[4-(2-Hydroxymethyl-phenylamino)-pipeidin-1-yl]-N-quinolin-5-yl-acetamide |
| 3 | 2-[4-(2-Hydroxymethyl-phenylamino)-piperidin-1-yl]-N-quinolin-6-yl-acetamide |
| 4 | 2-[4-(2-Hydroxymethyl-phenylamino)-piperidin-1-yl)-N-quinolin-8-yl-acetamide |
| 5 | 2-[4-(2-Hydroxymethyl-4-methyl-phenylamino)-piperidin-1-yl]-N-quinolin-3-yl-acetamide |
| 6 | 2-[4-(2-Hydroxymethyl-4-methyl-phenylamino)-piperidin-1-yl]-N-quinolin-5-yl-acetamide |
| 7 | 2-[4-(2-Hydroxymethyl-4-methyl-phenylamino)-piperidin-1-yl]-N-quinolin-6-yl-acetamide |
| 8 | 2-[4-(2-Hydroxymethyl-4-methyl-phenylamino)-piperidin-1-yl]-N-quinolin-8-yl-acetamide |
| 9 | 2-[4-(2-Hydroxymethyl-6-methyl-phenylamino)-piperidin-1-yl]-N-quinolin-3-yl-acetamide |
| 10 | 2-[4-(2-Hydroxymethyl-6-methyl-phenylamino)-piperidin-1-yl]-N-quinolin-5-yl-acetamide |
| 11 | 2-[4-(2-Hydroxymethyl-6-methyl-phenylamino)-piperidin-1-yl]-N-quinolin-6-yl-acetamide |
| 12 | 2-[4-(2-Hydroxymethyl-6-methyl-phenylamino)-piperidin-1-yl]-N-quinolin-8-yl-acetamide |
| 13 | N-(4-Benzoyl-phenyl)-2-[4-(2-hydroxymethyl-phenylamino)-piperidin-1-yl]-acetamide |
| 14 | N-(4-Benzoyl-phenyl)-2-[4-(2-hydroxymethyl-4-methyl-phenylamino)-piperidin-1-yl]-acetamide |
| 15 | N-(4-Benzoyl-phenyl)-2-[4-(2-hydroxymethyl-6-methyl-phenylamino)-piperidin-1-yl]-acetamide |
| 16 | N-Benzo[1,3]dioxol-5-yl-2-[4-(2-hydroxymethyl-phenylamino)-piperidin-1-yl]-acetamide |
| 17 | N-Benzo[1,3]dioxol-5-yl-2-[4-(2-hydroxymethyl-4-methyl-phenylamino)-piperidin-1-yl]-acetamide |
| 18 | N-Benzo[1,3]dioxol-5-yl-2-[4-(2-hydroxymethyl-6-methyl-phenylamino)-piperidin-1-yl]-acetamide hydrochloride |
| 19 | 2-[4-(4-Chloro-2-hydroxymethyl-phenylamino)-piperidin-1-yl]-N-quinolin-3-yl-acetamide |
| 20 | 2-[4-(4-Fluoro-2-hydroxymethyl-phenylamino)-piperidin-1-yl]-N-quinolin-3-yl-acetamide |
| 21 | 2-[4-(3-Chloro-2-hydroxymethyl-phenylamino)-piperidin-1-yl]-N-quinolin-3-yl-acetamide |
| 22 | 2-[4-(4-Chloro-2-hydroxymethyl-phenylamino)-piperidin-1-yl]-N-quinolin-5-yl-acetamide |
| 23 | 2-[4-(4-Fluoro-2-hydroxymethyl-phenylamino)-piperidin-1-yl]-N-quinolin-5-yl-acetamide |
| 24 | 2-[4-(3-Chloro-2-hydroxymethyl-phenylamino)-piperidin-1-yl]-N-quinolin-5-yl-acetamide |
| 25 | 2-[4-(4-Chloro-2-hydroxymethyl-phenylamino)-piperidin-1-yl]-N-quinolin-6-yl-acetamide |
| 26 | 2-[4-(4-Fluoro-2-hydroxymethyl-phenylamino)-piperidin-1-yl]-N-quinolin-6-yl-acetamide |
| 27 | 2-[4-(3-Chloro-2-hydroxymethyl-phenylamino)-piperidin-1-yl]-N-quinolin-6-yl-acetamide |
| 28 | 2-[4-(2-Hydroxymethyl-6-methoxy-phenylamino)-piperidin-1-yl]-N-quinolin-6-yl-acetamide |
| 29 | 2-[4-(4,5-Difluoro-2-hydroxymethyl-phenylamino)-piperidin-1-yl]-N-quinolin-6-yl-acetamide |
| 30 | 2-[4-(4-Chloro-2-hydroxymethyl-phenylamino)-piperidin-1-yl]-N-quinolin-8-yl-acetamide |
| 31 | 2-[4-(4-Fluoro-2-hydroxymethyl-phenylaminol)-piperidin-1-yl]-N-quinolin-8-yl-acetamide |
| 32 | 2-[4-(3-Chloro-2-hydroxymethyl-phenylamino)-piperidin-1-yl]-N-quinolin-8-yl-acetamide |
| 33 | N-(4-Benzoyl-phenyl)-2-[4-(4-chloro-2-hydroxymethyl-phenylamino)-piperidin-1-yl]-acetamide |
| 34 | N-(4-Benzoyl-phenyl)-2-[4-(4-fluoro-2-hydroxymethyl-phenylamino)-piperidin-1-yl]-acetamide |
| 35 | N-(4-Benzoyl-phenyl)-2-14-(2-hydroxymethyl-6-methoxy-phenylamino)-piperidin-1-yl]-acetamide |
| 36 | N-(4-Benzoyl-phenyl)-2-[4-(3-chloro-2-hydroxymethyl-phenylamino)-piperidin-1-yl]-acetamide |
| 37 | 2-[4-(2-Hydroxymethyl-phenylaminohpiperiidin-1-yl]-N-[4-(hydroxy-phenyl-methyl)-phenyl]-acetamide |
| 38 | 2-[4-(2-Hydroxymethyl-6-methyl-phenylamino)-piperidin-1-yl]-N-[4-(hydroxy-phenyl-methyl)-phenyl]-acetamide |
| 39 | 2-[4-(4-Chloro-2-hydroxymethyl-phenylamino)-piperidin-1-yl]-N-[4-hydroxy-phenyl-methyl)-phenyl]-acetamide |
| 40 | 2-[4-(2-Hydroxymethyl-4-methyl-phenylaminol)-pipeidin-1-yl]-N-[4-hydroxy-phenyl-methyl)-phenyl]-acetamide |
| 41 | 2-[4-(4-Fluoro-2-hydroxymethyl-phenylamino)-piperidin-1-yl]-N-[4-(hydroxy-phenyl-methyl)-phenyl]-acetamide |
| 42 | 2-[4-(2-Hydroxymethyl-6-methoxy-phenylamino)-piperidin-1-yl]-N-[4-(hydroxy-phenyl-methyl)-phenyl]-acetamide |
| 43 | 2-[4-(3-Chloro-2-hydroxymethyl-phenylamino)-piperidin-1-yl]-N-[4-(hydroxy-phenyl-methyl)-phenyl]-acetamide |
| 44 | 2-[4-(2-Hydroxymethyl-phenylamino)-piperidin-1-yl]-N-(9-oxo-9H-fluoren-3-yl)-acetamide |
| 45 | 2-[4-(4-Chloro-2-hydroxymethyl-phenylamino)-piperidin-1-yl]-N-(9-oxo-9H-fluoren-3-yl)-acetamide |
| 46 | 2-[4-(4-Fluoro-2-hydroxymethyl-phenylamino)-piperidin-1-yl]-N-(9-oxo-9H-fluoren-3-yl)-acetamide |
| 47 | 2-[4-(2-Hydroxymethyl-6-methoxy-phenylamino)-piperidin-1-yl]-N-(9-oxo-9H-fluoren-3-yl)-acetamide |
| 48 | 2-[4-(4,5-Difluoro-2-hydroxymethyl-phenylamino)-piperidin-1-yl]-N-(9-oxo-9H-fluoren-3-yl)-acetamide |
| 49 | 2-[4-(3-Chloro-2-hydroxymethyl-phenylamino)-piperidin-1-yl]-N-(9-oxo-9H-fluoren-3-yl)-acetamide |
| 50 | N-(9-Hydroxy-9H-fluoren-3-yl)-2-[4-(2-hydroxymethyl-phenylamino)-piperidin-1-yl]-acetamide |
| 51 | 2-[4-(4-Chloro-2-hydroxymethyl-phenylamino)-piperidin-1-yl]-N-(9-hydroxy-9H-fluoren-3-yl)-acetamide |
| 52 | 2-[4-(4-Fluoro-2-hydroxymethyl-phenylamino)-piperidin-1-yl]-N-(9-hydroxy-9H-fluoren-3-yl)-acetamide |
| 53 | N-(9-Hydroxy-9H-fluoren-3-yl)-2-[4-(2-hydroxymethyl-6-methoxy-phenylamino)-piperidin-1-yl]-acetamide |
| 54 | 2-[4-(4,5-Difluoro-2-hydroxymethyl-phenylamino)-piperidin-1-yl]-N-(9-hydroxy-9H-fluoren-3-yl)-acetamide |
| 55 | 2-[4-(3-Chloro-2-hydroxymethyl-phenylamino)-piperidin-1-yl]-N-(9-hydroxy-9H-fluoren-3-yl)-acetamide |
| 56 | N-(3-Acetyl-phenyl)-2-[4-(2-hydroxymethyl-phenylamino)-piperidin-1-yl]-acetamide |
| 57 | N-(3-Acetyl-phenyl)-2-[4-(2-hydroxymethyl-6-methyl-phenylamino)-piperidin-1-yl]-acetamide |
| 58 | N-(3-Acetyl-phenyl)-2-[4-(4-chloro-2-hydroxymethyl-phenylamino)-piperidin-1-yl]-acetamide |
| 59 | N-(3-Acetyl-phenyl)-2-[4-(2-hydroxymethyl-4-methyl-phenylamino)-piperidin-1-yl]-acetamide |
| 60 | N-[3-(1-Hydroxy-ethyl)-phenyl]-2-[4-(2-hydroxymethyl-phenylamino)-piperidin-1-yl]-acetamide |
| 61 | N-[3-(1-Hydroxy-ethyl)-phenyl]-2-[4-(2-hydroxymethyl-6-methyl-phenylamino)-piperidin-1-yl]-acetamide |
| 62 | 2-[4-(4-Chloro-2-hydroxymethyl-phenylamino)-piperidin-1-yl]-N-[3-(1-hydroxy-ethyl)-phenyl]-acetamide |
| 63 | N-[3-(1-Hydroxy-ethyl)-phenyl]-2-[4-(2-hydroxymethyl-4-methyl-phenylamino)-piperidin-1-yl]-acetamide |
| 64 | N-Benzo[1,3]dioxol-5-yl-2-[4-(4-chloro-2-hydroxymethyl-phenylamino)-piperidin-1-yl]-acetamide |
| 65 | N-Benzo[1,3]dioxol-5-yl-2-[4-(4-fluoro-2-hydroxymethyl-phenylamino)-piperidin-1-yl]-acetamide |
| 66 | N-Benzo[1,3]dioxol-5-yl-2-[4-(2-hydroxymethyl-6-methoxy-phenylamino)-piperidin-1-yl]-acetamide |
| 67 | N-Benzo[1,3]dioxol-5-yl-2-[4-(4,5-difluoro-2-hydroxymethyl-phenylamino)-piperidin-1-yl]-acetamide |
| 68 | N-Benzo[1,3]dioxol-5-yl-2-[4-(3-chloro-2-hydroxymethyl-phenylamino)-piperidin-1-yl]-acetamide |
| 69 | N-(4-Acetyl-phenyl)-2-[4-(2-hydroxymethyl-phenylamino)-piperidin-1-yl]-acetamide |
| 70 | N-(4-Acetyl-phenyl)-2-[4-(2-hydroxymethyl-6-methyl-phenylamino)-piperidin-1-yl]-acetamide |
| 71 | N-(4-Acetyl-phenyl)-2-[4-(4-chloro-2-hydroxymethyl-phenylamino)-piperidin-1-yl]-acetamide |
| 72 | N-(4-Acetyl-phenyl)-2-[4-(2-hydroxymethyl-4-methyl-phenylamino)-piperidin-1-yl]-acetamide |
| 73 | N-(4-Acetyl-phenyl)-2-[4-(4-fluoro-2-hydroxymethyl-phenylamino)-piperidin-1-yl]-acetamide |
| 74 | N-(4-Acetyl-phenyl)-2-[4-(2-hydroxymethyl-6-methoxy-phenylamino)-piperidin-1-yl]-acetamide |
| 75 | N-(4-Acetyl-phenyl)-2-[4-(3-chloro-2-hydroxymethyl-phenylamino)-piperidin-1-yl]-acetamide |
| 76 | N-[4-(1-Hydroxy-ethyl)-phenyl]-2-[4-(2-hydroxymethyl-phenylamino)-piperidin-1-yl]-acetamide |
| 77 | N-[4-(1-Hydroxy-ethyl)-phenyl]-2-[4-(2-hydroxymethyl-6-methyl-phenylamino)-piperidin-1-yl]-acetamide |
| 78 | 2-[4-(4-Chloro-2-hydroxymethyl-phenylamino)-piperidin-1-yl]-N-[4-(1-hydroxy-ethyl)-phenyl]-acetamide |
| 79 | N-[4-(1-Hydroxy-ethyl)-phenyl]-2-[4-(2-hydroxymethyl-4-methyl-phenylamino)-piperidin-1-yl]-acetamide |
| 80 | 2-[4-(4-Fluoro-2-hydroxymethyl-phenylamino)-piperidin-1-yl]-N-[4-(1-hydroxy-ethyl)-phenyl]-acetamide |
| 81 | N-[4-(1-Hydroxy-ethyl)-phenyl]-2-[4-(2-hydroxymethyl-6-methoxy-phenylamino)-piperidin-1-yl)-acetamide |
| 82 | 2-[4-(3-Chloro-2-hydroxymethyl-phenylamino)-piperidin-1-yl]-N-[4-(1-hydroxy-ethyl)-phenyl]-acetamide |
| 83 | N-(9-Ethyl-9H-carbazol-3-yl)-2-[4-(2-hydroxymethyl-phenylamino)-piperidin-1-yl]-acetamide |
| 84 | 2-[4-(4-Chloro-2-hydroxymethyl-phenylamino)-piperidin-1-yl]-N-(9-ethyl-9H-carbazol-3-yl)-acetamide |
| 85 | N-(9-Ethyl-9H-carbazol-3-yl)-2-[4-(2-hydroxymethyl-4-methyl-phenylamino)-piperidin-1-yl]-acetamide |
| 86 | N-(9-Ethyl-9H-carbazol-3-yl)-2-[4-(4-fluoro-2-hydroxymethyl-phenylamino)-piperidin-1-yl]-acetamide |
| 87 | N-(9-Ethyl-9H-carbazol-3-yl)-2-[4-(2-hydroxymethyl-6-methoxy-phenylaminorpiperidin-1-yl)-acetamide |
| 88 | 2-[4-(4,5-Difluoro-2-hydroxymethyl-phenylamino)-piperidin-1-yl]-N-(9-ethyl-9H-carbazol-3-yl)-acetamide |
| 89 | 2-[4-(3-Chloro-2-hydroxymethyl-phenylamino)-piperidin-1-yl]-N-(9-ethyl-9H-carbazol-3-yl)-acetamide |
| 90 | 2-[4-(2-Hydroxymethyl-phenylamino)-piperidin-1-yl]-N-(9-methyl-9H-carbazol-3-yl)-acetamide |
| 91 | 2-[4-(2-Hydroxymethyl-6-methyl-phenylamino)-piperidin-1-yl]-N-(9-methyl-9H-carbazol-3-yl)-acetamide |
| 92 | 2-[4-(4-Chloro-2-hydroxymethyl-phenylamino)-piperidin-1-yl]-N-(9-methyl-9H-carbazol-3-yl)-acetamide |
| 93 | 2-[4-(2-Hydroxymethyl-4-methyl-phenylamino)-piperidin-1-yl]-N-(9-methyl-9H-carbazol-3-yl)-acetamide |
| 94 | 2-[4-(2-Hydroxymethyl-phenylamino)-piperidin-1-yl]-N-(5-oxo-5,6,7,8-tetrahydro-naphthalen-2-yl)-acetamide |
| 95 | 2-[4-(2-Hydroxymethyl-6-methyl-phenylamino)-piperidin-1-yl]-N-(5-oxo-5,6,7,8-tetrahydro-naphthalen-2-yl)-acetamide |
| 96 | 2-[4-(4-Chloro-2-hydroxymethyl-phenylamino)-piperidin-1-yl]-N-(5-oxo-5,6,7,8-tetrahydro-naphthalen-2-yl)-acetamide |
| 97 | 2-[4-(2-Hydroxymethyl-4-methyl-phenylamino)-piperidin-1-yl]-N-(5-oxo-5,6,7,8-tetrahydro-naphthalen-2-yl)-acetamide |
| 98 | 2-[4-(4-Fluoro-2-hydroxymethyl-phenylamino)-piperidin-1-yl]-N-(5-oxo-5,6,7,8-tetrahydro-naphthalen-2-yl)-acetamide |
| 99 | 2-[4-(2-Hydroxymethyl-phenylamino)-piperidin-1-yl]-N-(5-hydroxy-5,6,7,8-tetrahydro-naphthalen-2-yl)-acetamide |
| 100 | 2-[4-(2-Hydroxymethyl-6-methyl-phenylamino)-piperidin-1-yl]-N-(5-hydroxy-5,6,7,8-tetrahydro-naphthaten-2-yl)-acetamide |
| 101 | 2-[4-(4-Chloro-2-hydroxymethyl-phenylamino)-piperidin-1-yl]-N-(5-hydroxy-5,6,7,8-tetrahydro-naphthalen-2-yl)-acetamide |
| 102 | 2-[4-(2-Hydroxymethyl-4-methyl-phenylamino)-piperidin-1-yl]-N-(5-hydroxy-5,6,7,8-tetrahydro-naphthalen-2-yl)-acetamide |
| 103 | 2-[4-(4-Fluoro-2-hydroxymethyl-phenylamino)-piperidin-1-yl]-N-(5-hydroxy-5,6,7,8-tetrahydro-naphthalen-2-yl)-acetamide |
| 104 | 2-[4-(2-Hydroxymethyl-6-methyl-phenylamino)-piperidin-1-yl]-N-(1-oxo-indan-5-yl)-acetamide |
| 105 | 2-[4-(4-Chloro-2-hydroxymethyl-phenylamino)-piperidin-1-yl]-N-(1-oxo-indan-5-yl)-acetamide |
| 106 | 2-[4-(2-Hydroxymethyl-4-methyl-phenylamino)-piperidin-1-yl]-N-(1-oxo-indan-5-yl)-acetamide |
| 107 | N-(1-Hydroxy-indan-5-yl)-2-[4-(2-hydroxymethyl-phenylamino)-piperidin-1-yl]-acetamide |
| 108 | N-(1-Hydroxy-indan-5-yl)-2-[4-(2-hydroxymethyl-6-methyl-phenylamino)-piperidin-1-yl)-acetamide |
| 109 | 2-[4-(4-Chloro-2-hydroxymethyl-phenylamino)-piperidin-1-yl]-N-(1-hydroxy-indan-5-yl)-acetamide |
| 110 | N-(1-Hydroxy-indan-5-yl)-2-[4-(2-hydroxymethyl-4-methyl-phenylamino)-piperidin-1-yl]-acetamide |
| 111 | 2-[4-(4-Bromo-2-hydroxymethyl-phenylamino)-piperidin-1-yl]-N-(9-ethyl-9H-carbazol-3-yl)-acetamide |
| 112 | 2-[4-(2-Hydroxymethyl-6-methyl-phenylamino)-piperidin-1-yl]-N-(9-oxo-9H-fluoren-2-yl)-acetamide |
| 113 | 2-[4-(2-Hydroxymethyl-4-methyl-phenylamino)-piperidin-1-yl]-N-(9-oxo-9H-fluoren-2-yl)-acetamide |
| 114 | N-Dibenzofuran-2-yl-2-[4-(2-hydroxymethyl-4-methyl-phenylamino)-piperidin-1-yl]-acetamide |
| 115 | N-Dibenzofuran-2-yl-2-[4-(2-hydroxymethyl-6-methyl-phenylamino)-piperidin-1-yl]-acetamide |
| 116 | 2-[4-(2-Hydroxymethyl-6-methoxy-phenylamino)-piperidin-1-yl]-N-quinolin-3-yl-acetamide |
| 117 | 2-[4-(4,5-Difluoro-2-hydroxymethyl-phenylamino)-piperidin-1-yl]-N-quinolin-3-yl-acetamide |
| 118 | N-(9-Hydroxy-9H-fluoren-2-yl)-2-[4-(2-hydroxymethyl-6-methyl-phenylamino)-piperidin-1-yl]-acetamide |
| 119 | N-(9-Hydroxy-9H-fluoren-2-yl)-2-[4-(2-hydroxymethyl-4-methyl-phenylamino)-piperidin-1-yl]-acetamide |
| 120 | 2-[4-(2-Hydroxymethyl-phenylamino)-piperidin-1-yl]-N-(4-phenoxy-phenyl)-acetamide |
| 121 | 2-[4-(2-Hydroxymethyl-6-methyl-phenylamino)-piperidin-1-yl]-N-(4-phenoxy-phenyl)-acetamide |
| 122 | 2-[4-(4-Chloro-2-hydroxymethyl-phenylamino)-piperidin-1-yl]-N-(4-phenoxy-phenyl)-acetamide |
| 123 | 2-[4-(2-Hydroxymethyl-4-methyl-phenylamino)-piperidin-1-yl]-N-(4-phenoxy-phenyl)-acetamide |
| 124 | N-(4-Cyclohexyl-phenyl)-2-[4-(2-hydroxymethyl-phenylamino)-piperidin-1-yl]-acetamide |
| 125 | 2-[4-(2-Hydroxymethyl-3-methoxy-phenylamino)-piperidin-1-yl]-N-quinolin-3-yl-acetamide |
| 126 | 2-[4-(3-Hydroxy-2-hydroxymethyl-phenylamino)-piperidin-1-yl]-N-quinolin-3-yl-acetamide |
| 127 | 2-[4-(4-Hydroxy-2-hydroxymethyl-phenylamino)-piperidin-1-yl]-N-quinolin-3-yl-acetamide |
| 128 | 2-[4-(2-Hydroxy-6-hydroxymethyl-phenylamino)-piperidin-1-yl]-N-quinolin-3-yl-acetamide |
| 129 | 2-[4-(2-Hydroxymethyl-3-methoxy-phenylamino)-piperidin-1-yl]-N-quinolin-6-yl-acetamide |
| 130 | 2-[4-(3-Hydroxy-2-hydroxymethyl-phenylamino)-piperidin-1-yl]-N-quinolin-6-yl-acetamide |
| 131 | 2-[4-(4-Hydroxy-2-hydroxymethyl-phenylamino)-piperidin-1-yl]-N-quinolin-6-yl-acetamide |
| 132 | 2-[4-(2-Hydroxy-6-hydroxymethyl-phenylamino)-pipeidin-1-yl]-N-quinolin-6-yl-acetamide |
| 133 | N-(4-Benzoyl-phenyl)2-[4-(2-hydroxymethyl-3-methoxy-phenylamino)-piperidin-1-yl]-acetamide |
| 134 | N-(4-Benzoyl-phenyl)-2-[4-(3-hydroxy-2-hydroxymethyl-phenylamino)-piperidin-1-yl]-acetamide |
| 135 | N-(4-Benzoyl-phenyl)-2-[4-(4-hydroxy-2-hydroxymethyl-phenylamino)-piperidin-1-yl]-acetamide |
| 136 | N-(4-Benzoyl-phenyl)-2-[4-(2-hydroxy-6-hydroxymethyl-phenylamino)-piperidin-1-yl]-acetamide |
| 137 | 2-[4-(2-Hydroxymethyl-3-methoxy-phenylamino)-piperidin-1-yl]-N-[4-(hydroxy-phenyl-methyl)-phenyl]-acetamide |
| 138 | 2-[4-(3-Hydroxy-2-hydroxymethyl-phenylamino)-piperidin-1-yl]-N-[4-(hydroxy-phenyl-methyl)-phenyl]-acetamide |
| 139 | 2-[4-(4-Hydroxy-2-hydroxymethyl-phenylamino)-piperidin-1-yl]-N-[4-(hydroxy-phenyl-methyl)-phenyl]-acetamide |
| 140 | 2-[4-(2-Hydroxy-6-hydroxymethyl-phenylamino)-piperidin-1-yl]-N-[4-(hydroxy-phenyl-methyl)-phenyl]-acetamide |
| 141 | 2-[4-(2-Hydroxymethyl-3-methoxy-phenylamino)-piperidin-1-yl]-N-(9-oxo-9H-fluoren-3-yl)-acetamide |
| 142 | 2-[4-(3-Hydroxy-2-hydroxymethyl-phenylamino)-piperidin-1-yl]-N-(9-oxo-9H-fluoren-3-yl)-acetamide |
| 143 | 2-[4-(4-Hydroxy-2-hydroxymethyl-phenylamino)-piperidin-1-yl]-N-(9-oxo-9H-fluoren-3-yl)-acetamide |
| 144 | 2-[4-(2-Hydroxy-6-hydroxymethyl-phenylamino)-piperidin-1-yl]-N-(9-oxo-9H-fluoren-3-yl)-acetamide |
| 145 | N-(9-Hydroxy-9H-fluoren-3-yl)-2-[4-(2-hydroxymethyl-3-methoxy-phenylamino)-piperidin-1-yl]-acetamide |
| 146 | N-(9-Hydroxy-9H-fluoren-3-yl)-2-[4-(3-hydroxy-2-hydroxymethyl-phenylamino)-piperidin-1-yl]-acetamide |
| 147 | N-(9-Hydroxy-9H-fluoren-3-yl)-2-[4-(4-hydroxy-2-hydroxymethyl-phenylamino)-piperidin-1-yl]-acetamide |
| 148 | N-(9-Hydroxy-9H-fluoren-3-yl)-2-[4-(2-hydroxy-6-hydroxymethyl-phenylamino)-piperidin-1-yl]-acetamide |
| 149 | N-(3-Acetyl-phenyl)2-[4-(2-hydroxymethyl-3-methoxy-phenylamino)-piperidin-1-yl]-acetamide |
| 150 | N-(3-Acetyl-phenyl)-2-[4-(3-hydroxy-2-hydroxymethyl-phenylamino)-piperidin-1-yl]-acetamide |
| 151 | N-(3-Acetyl-phenyl)-2-[4-(4-hydroxy-2-hydroxymethyl-phenylamino)-piperidin-1-yl)-acetamide |
| 152 | N-(3-Acetyl-phenyl)-2-[4-(2-hydroxy-6-hydroxymethyl-phenylamino)-piperidin-1-yl]-acetamide |
| 153 | N-[3-(1-Hydroxy-ethyl)-phenyl]-2-[4-(2-hydroxymethyl-3-methoxy-phenylamino)-piperidin-1-yl]-acetamide |
| 154 | N-[3-(1-Hydroxy-ethyl)-phenyl]-2-[4-(3-hydroxy-2-hydroxymethyl-phenylamino)-piperidin-1-yl]-acetamide |
| 155 | N-[3-(1-Hydroxy-ethyl)-phenyl]-2-[4-(4-hydroxy-2-hydroxymethyl-phenylamino)-piperidin-1-yl]-acetamide |
| 156 | N-[3-(1-Hydroxy-ethyl)-phenyl]-2-[4-(2-hydroxy-6-hydroxymethyl-phenylamino)-pipeidin-1-yl]-acetamide |
| 157 | N-(4-Acetyl-phenyl)-2-[4-(2-hydroxymethyl-3-methoxy-phenylamino)-piperidin-1-yl]-acetamide |
| 158 | N-(4-Acetyl-phenyl)-2-[4-(3-hydroxy-2-hydroxymethyl-phenylamino)-piperidin-1-yl]-acetamide |
| 159 | N-(4-Acetyl-phenyl)-2-[4-(4-hydroxy-2-hydroxymethyl-phenylamino)-piperidin-1-yl]-acetamide |
| 160 | N-(4-Acetyl-phenyl)-2-[4-(2-hydroxy-6-hydroxymethyl-phenylamino)-piperidin-1-yl]-acetamide |
| 161 | N-[4-(1-Hydroxy-ethyl)-phenyl]-2-[4-(2-hydroxymethyl-3-methoxy-phenylamino)-piperidin-1-yl]-acetamide |
| 162 | N-[4-(1-Hydroxy-ethyl)-phenyl]-2-[4-(3-hydroxy-2-hydroxymethyl-phenylamino)-piperidin-1-yl]-acetamide |
| 163 | N-[4-(1-Hydroxy-ethyl)-phenyl]-2-[4-(4-hydroxy-2-hydroxymethyl-phenylamino)-piperidin-1-yl]-acetamide |
| 164 | N-[4-(1-Hydroxy-ethyl)-phenyl]-2-[4-(2-hydroxy-6-hydroxymethyl-phenylamino)-piperidin-1-yl]-acetamide |
| 165 | N-(9-Ethyl-9H-carbazol-3-yl)-2-[4-(2-hydroxymethyl-3-methoxy-phenylamino)-piperidin-1-yl]-acetamide |
| 166 | N-(9-Ethyl-9H-carbazol-3-yl)-2-[4-(3-hydroxy-2-hydroxymethyl-phenylamino)-piperidin-1-yl]-acetamide |
| 167 | N-(9-Ethyl-9H-carbazol-3-yl)-2-[4-(4-hydroxy-2-hydroxymethyl-phenylamino)-piperidin-1-yl]-acetamide |
| 168 | N-(9-Ethyl-9H-carbazol-3-yl)-2-[4-(2-hydroxy-6-hydroxymethyl-phenylamino)-piperidin-1-yl]-acetamide |
| 169 | 2-[4-(2-Hydroxymethyl-3-methoxy-phenylamino)-piperidin-1-yl]-N-(5-oxo-5,6,7,8-tetrahydro-naphthalen-2-yl)-acetamide |
| 170 | 2-[A-(3-Hydroxy-2-hydroxymethyl-phenylamino)-piperidin-1-yl]-N-(5-oxo-5,6,7,8-tetrahydro-naphthalen-2-yl)-acetamide |
| 171 | 2-[4-(4-Hydroxy-2-hydroxymethyl-phenylamino)-piperidin-1-yl]-N-(5-oxo-5,6,7,8-tetrahydro-naphthalen-2-yl)-acetamide |
| 172 | 2-[4-(2-Hydroxy-6-hydroxymethyl-phenylamino)-piperidin-1-yl]-N-(5-oxo-5,6,7,8-tetrahydro-naphthalen-2-yl)-acetamide |
| 173 | 2-[4-(2-Hydroxymethyl-3-methoxy-phenylamino)-piperidin-1-yl]-N-(5-hydroxy-5,6,7,8-tetrahydro-naphthalen-2-yl)-acetamide |
| 174 | 2-[4-(3-Hydroxy-2-hydroxymethyl-phenylamino)-piperidin-1-yl]-N-(5-hydroxy-5,6,7,8-tetrahydro-naphthalen-2-yl)-acetamide |
| 175 | 2-[4-(4-Hydroxy-2-hydroxymethyl-phenylamino)-piperidin-1-yl]-N-(5-hydroxy-5,6,7,8-tetrahydro-naphthalen-2-yl)-acetamide |
| 176 | 2-[4-(2-Hydroxy-6-hydroxymethyl-phenylamino)-piperidin-1-yl]-N-(5-hydroxy-5,6,7,8-tetrahydro-naphthalen-2-yl)-acetamide |
| 177 | 2-[4-(2-Hydroxymethyl-3-methoxy-phenylamino)-piperidin-1-yl]-N-(4-phenoxy-phenyl)-acetamide |
| 178 | 2-[4-(3-Hydroxy-2-hydroxymethyl-phenylamino)-piperidin-1-yl]-N-(4-phenoxy-phenyl)-acetamide |
| 179 | 2-[4-(4-Hydroxy-2-hydroxymethyl-phenylamino)-piperidin-1-yl]-N-(4-phenoxy-phenyl)-acetamide |
| 180 | 2-[4-(2-Hydroxy-6-hydroxymethyl-phenylamino)-piperidin-1-yl]-N-(4-phenoxy-phenyl)-acetamide |

optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

The 1,4-disubstituted piperidine compounds of general formula (Ia), wherein R^{1a}-R^{23a}, A^{a} and B^{a} have the meaning given above, may be prepared preferably in such a way that at least one compound of general formula (IIa), 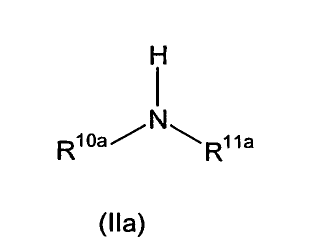 wherein R^{10a} and R^{11a} have the meaning given above, is reacted with at least one compound of general formula (IIIa), 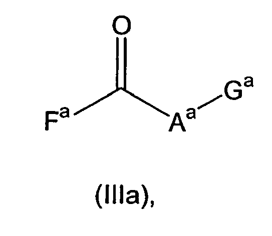 wherein A^{a} has the meaning given above, F^{a} represents halogen, hydroxy or an O-acyl group and G^{a} represents halogen, preferably chlorine, in a suitable reaction medium and preferably in the presence of at least one base and/or optionally at least one auxiliary agent, and reacting the so obtained compound of general (IVa) 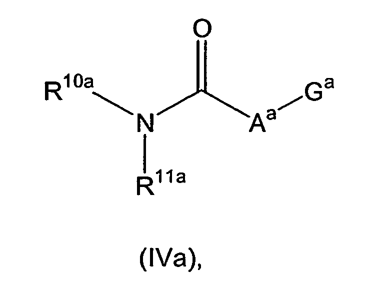 wherein A^{a}, G^{a}, R^{10a} and R^{11a} have the above defined meaning, with at least one piperidine compound of general formula (Va) and/or a salt, preferably hydrochloride salt, thereof, 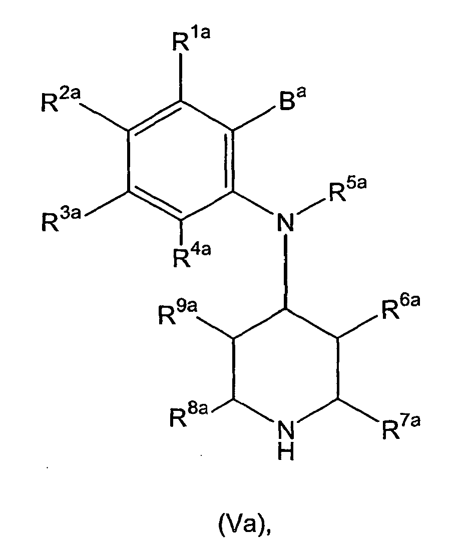 wherein R^{1a} to R^{9a} and B^{a} have the meaning as defined above, in a suitable reaction medium, optionally in the presence of at least one base and/or at least one auxiliary agent to yield a compound of general formula (Ia).

The process may be illustrated as an example by the following reaction scheme A: 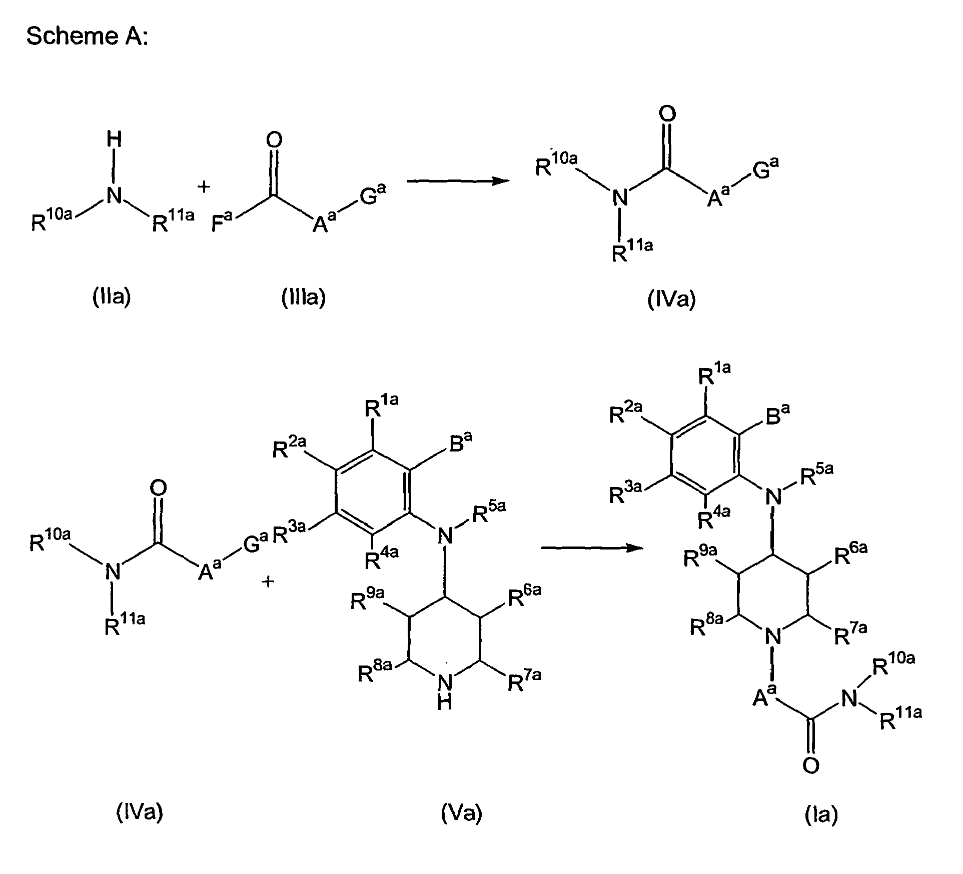 wherein R^{1a} to R^{11a}, A^{a} and B^{a} have the meaning as given above.

The 1,4 disubstituted piperidine compounds of general formula (Ia), wherein R^{1a} -R^{23a} and A^{a} have the meaning given above and B^{a} represents a substituted aliphatic radical or a -CH₂OR^{23a}-moiety, may be prepared preferably in a way that at least one compound of general formula (IIa), 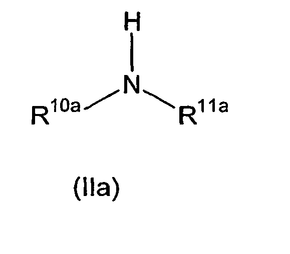 wherein R^{10a} and R^{11a} have the meaning given above, is reacted with at least one compound of general formula (IIIa), 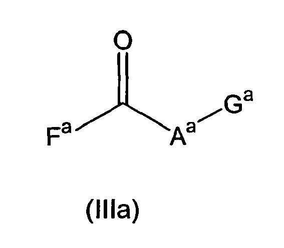 wherein A^{a} has the meaning given above, F^{a} represents halogen, hydroxy or an O-acyl group and G^{a} represents halogen, preferably chlorine, in a suitable reaction medium and preferably in the presence of at least one base and/or at least one auxiliary agent, and reacting the so obtained compound of general (IVa) 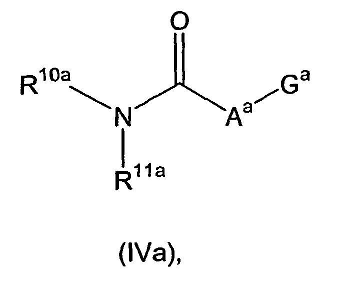 wherein A^{a}, G^{a}, R^{10a} and R^{11a} have the above defined meaning, with at least one piperidin compound of general formula (Va) and/or a salt, preferably hydrochloride, thereof, 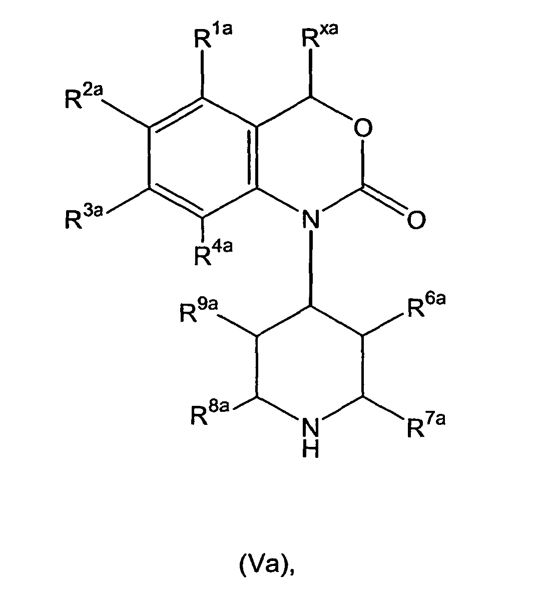 wherein R^{1a} to R^{9a} have the meaning as definded above and R^{xa} represents any substituent including hydrogen, preferably hydrogen, in a suitable reaction medium, optionally in the presence of at least one base and/or at least one auxiliary agent, to yield a compound of general formula (VIa), 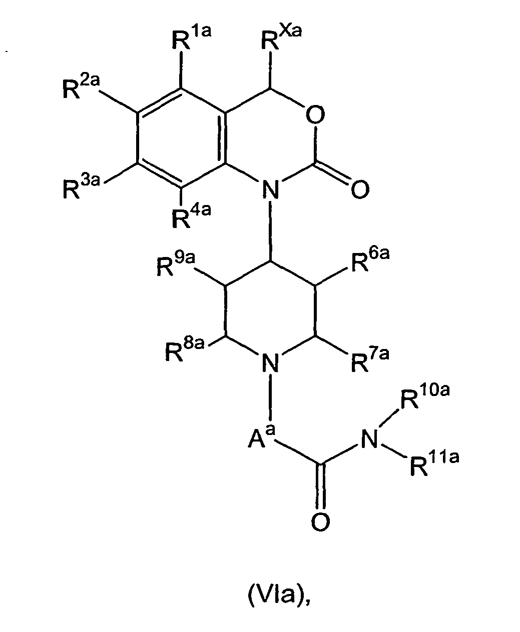 which is reacted with a base, preferably in a suitable reaction medium, more preferably in a mixture of water and ethanol, to yield a compound of general formula (Ia), wherein R^{1a}-R^{4a} and R^{6a}-R^{23a} and A^{a} have the meaning as defined above, R^{5a} represents H and B^{a} represents a substituted aliphatic radical or a - CH₂OR^{23a}-moiety.

The process may be illustrated as an example by the following reaction scheme B:

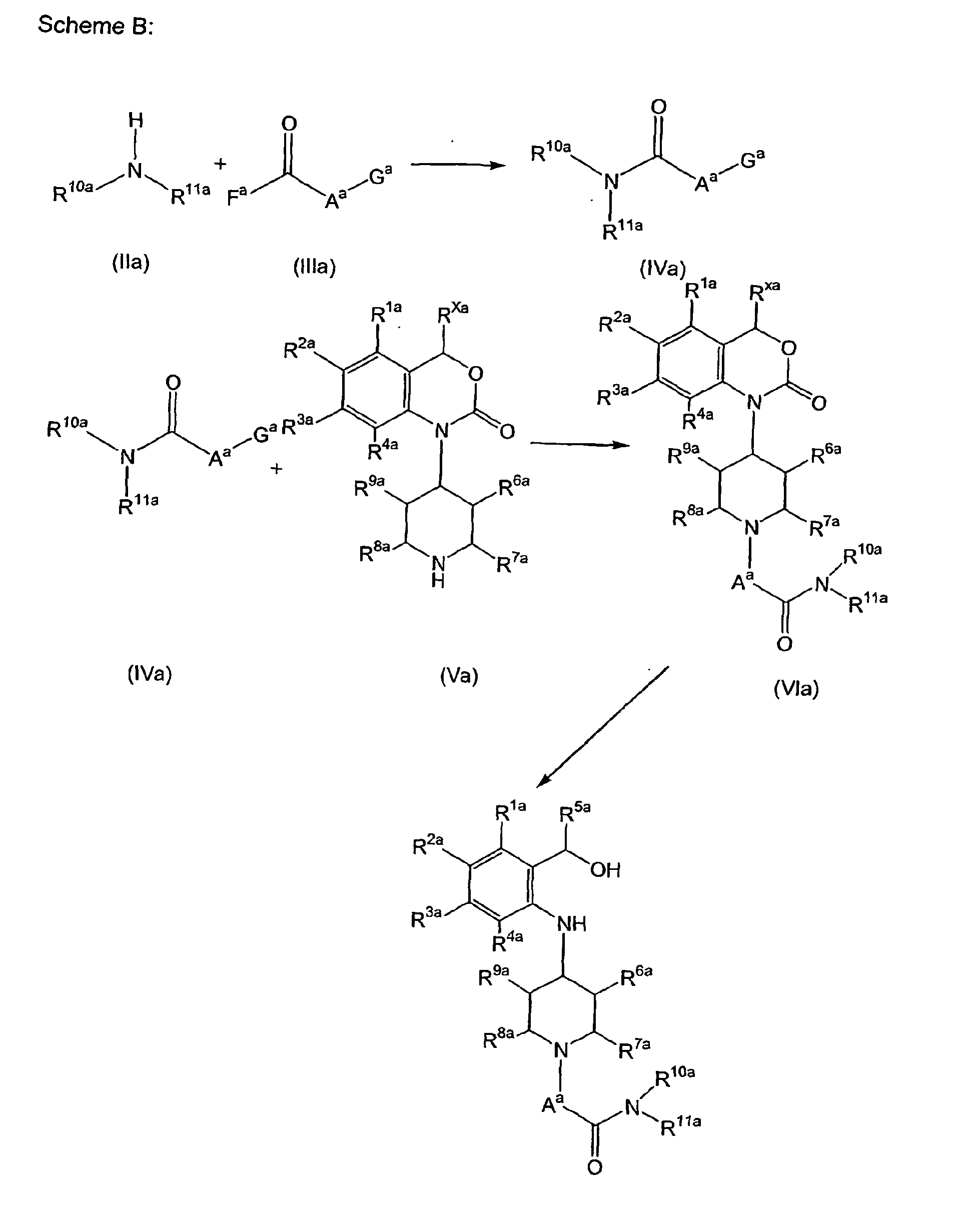

Suitable reaction media are e.g. organic solvents, such as ethers, preferably diethyl ether, dioxane, tetrahydrofurane, dimethyl glycol ether, or alcohols, e.g. methanol, ethanol, propanol, isopropanol, butanol, isobutanol, tert-butanol, or hydrocarbons, preferably benzene, toluene, xylene, hexane, cyclohexane, petroleum ether, or halogenated hydrocarbons, e.g. dichloromethane, trichloromethane, tetrachloromethane, dichloroethylene, trichloroethylene, chlorobenzene or/and other solvents, preferably ethyl acetate, triethylamine, pyridine, dimethylsulfoxide, diemthylformamide, hexamethylphosphoramide, acetonitril, acetone or nitromethane, are included. Mixtures based one or more of the afore mentioned solvents may also be used.

Bases that may be used in the processes according to the present invention are generally organic or inorganic bases, preferably alkali metal hydroxides, e.g. sodium hydroxide or potassium hydroxide, or obtained from other metals such as barium hydroxide or different carbonates, preferably potassium carbonate, sodium carbonate, calcium carbonate, or alkoxides, e.g. sodium methoxide, potassium methoxide, sodium ethoxide, potassium methoxide, potassium ethoxide or potassium tert-butoxide, or organic amines, preferably triethylamine, diisopropyethylamine or heterocycles, e.g. 1,4-diazabicyclo[2.2.2] octane, 1,8-diazabicyclo[5.4.0]undec-7-ene pyridine, diamino pyridine, dimethylaminopyridine, methylpiperidine or morpholine. Alkali metals such as sodium or its hydrides, e.g. sodium hydride, may also be used. Mixtures based one or more of the afore mentioned bases may also be used.

The above mentioned bases may be used for the process as auxiliary agents, when appropriate. Other suitable auxiliary agents for the above mentioned reactions are, for example, dehydrating agents like carbodiimides, e.g. diisopropylcarbodiimide, cyclohexylcarbodiimide or N-(3-dimethylaminopropyl)-N'-ethylcarbodiimide hydrochloride, or carbonylic compounds, e.g. carbonyldiimidazol or compounds like isobutylchloroformiate or methansulfonyl chloride, among others. These reagents are generally used in amounts from 0.5 to 5 mol versus 1 mol of the corresponding reactands. The bases are generally used in amounts from 0.05 to 10 mol versus 1 mol of the corresponding reactands.

During some of the synthetic reactions described or while preparing the compounds of general formulas (Ia), (IIa), (IIIa), (IVa), (Va) and (VIa), the protection of sensitive groups or of reagents may be necessary and/or desirable. This can be performed by using conventional protective groups like those described in the literature. The protective groups may also be eliminated as convenient by means well-known to those skilled in the art.

The compounds of general formulas (IIa), (IIIa), (IVa) and (Va) are either commercially available or can be produced according to methods known to those skilled in the art. The reaction of compounds of general formulas (IVa) and (Va) to yield 1,4-disubstituted piperidine compounds of general formula (Ia) may also be facilitated by conventional methods known to those skilled in the art.

The compounds of general formula (IVa) are commercially available or may be produced according to scheme I by conventional methods known to those skilled in the art. In particular, the respective compound of general formula (IIa) is reacted with chloroacetyl chloride or the respective compound of general formula (IIIa) in the presence of an organic reaction medium, preferably dichloromethane and a base, preferably triethylamine and/or diisopropylethylamine.

The preparation of compounds of general formula (Va) and their use for forming compounds with the general formula (Ia) are illustrated by way of examples in the schemes 1 and 2, wherein R¹-R¹¹ and A represent R^{1a}-R^{11a} and A^{a}.

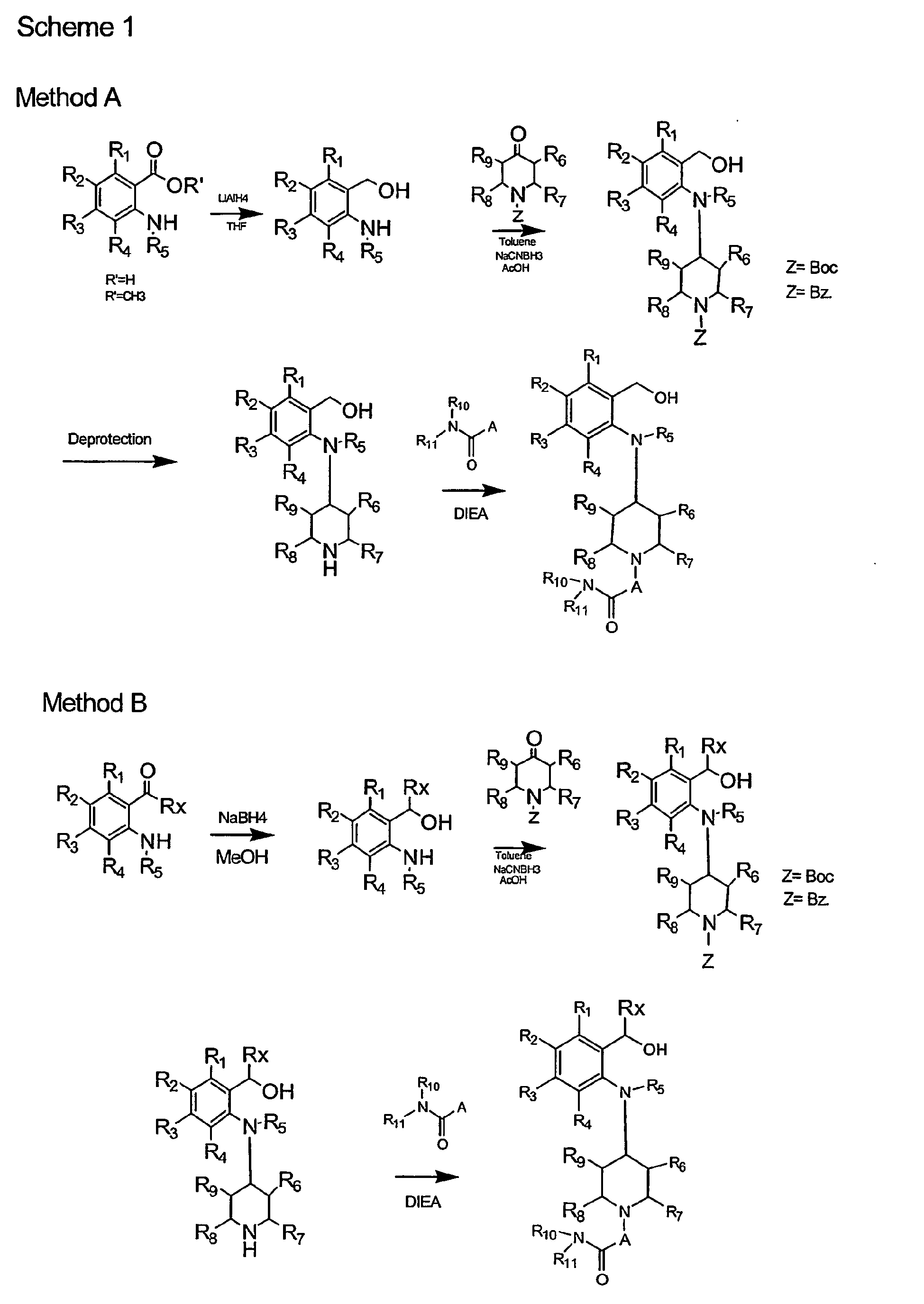

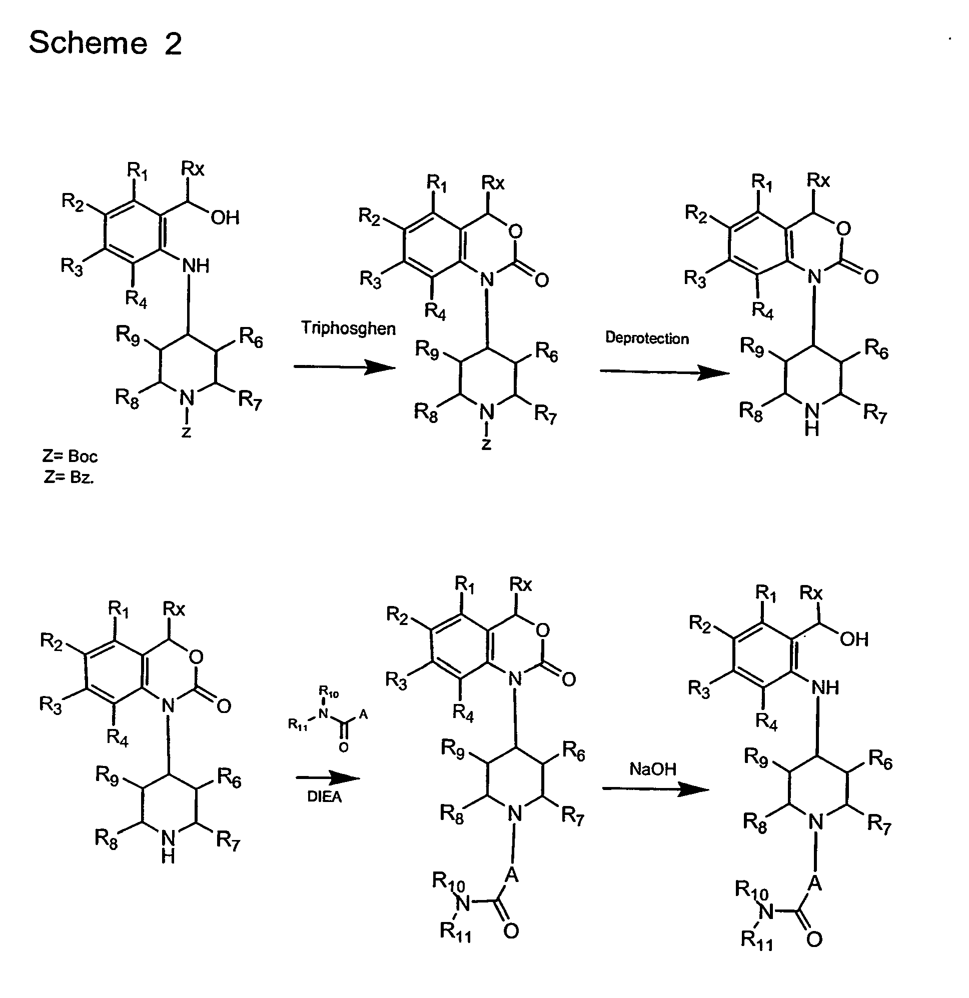

The salts of 1,4-disubstituted piperidine compounds of general formula (Ia), may be preferably prepared in such a way that at least one compound of general formula (Ia) having at least one basic group is reacted with an inorganic and/or organic acid, preferably in the presence of a suitable reaction medium. Suitable reaction media are the ones given above. Suitable inorganic acids are for example hydrochloric acid, hydrobromic acid, phosphoric acid, sulfuric acid, nitric acid, suitable organic acids are e.g. citric acid, maleic acid, fumaric acid, tartaric acid, or derivatives thereof, such as p-toluenesulfonic acid, methanesulfonic acid or camphersulfonic acid.

The salts of 1,4-disubstituted piperidine compounds of general formula (Ia), may be preferably prepared in a way that at least one compound of general formula (Ia) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙP₄₋ₙ]⁺, wherein n is 0, 1, 2, 3 or 4 and R represents a branched or unbranched C₁₋₄-alkyl-radical.

Solvates, preferably hydrates, of the 1,4-disubstituted piperidine compounds of general formula (Ia), or corresponding stereoisomers, or corresponding salts may also be obtained by standard procedures known to those skilled in the art.

If the 1,4-disubstituted piperidine compounds of general formula (Ia) are obtained in form of a mixture of stereoisomers, particularly enantiomers or diastereomers, said mixtures may be separated by standard procedures known to those skilled in the art, e.g. chromatographic methods or crystallization with chiral reagents.

The purification and isolation of the 1,4-disubstituted piperidine compounds of general formula (Ia) or a corresponding stereoisomer, or a corresponding salt, or corresponding solvate respectively, if required, may be carried out by conventional methods known to those skilled in the art, e.g. chromatographic methods or recrystallization.

The active substance combination of the present invention comprises as a component (B) at least one compound with 5-HT₆ receptor affinity, which is selected form the group consisting of the benzoxazinone-derived sulfonamide compounds of general formula (Ib), 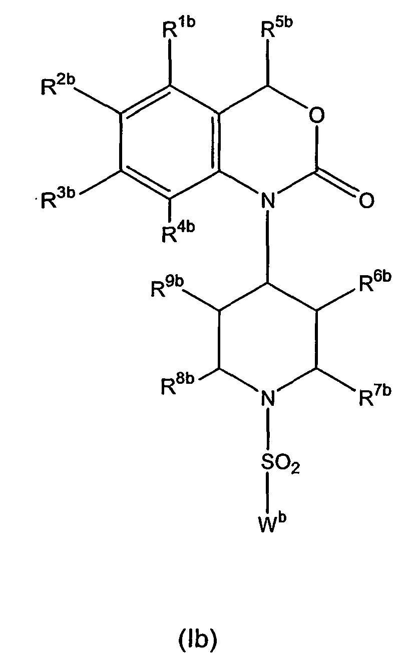 wherein
R^{1b}, R^{2b}, R^{3b}, R^{ab} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro group, a cyano group, -OR^{10b}, -O(C=O)R^{11b}, -(C=O)-O-R^{11b}, -SR^{12b}, -SOR^{12b}, -SO₂R^{12b}, -NH-SO₂R^{12b}, -SO₂NH₂ and a -NR^{13b}R^{14b} moiety,
R^{5b} represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R^{6b}, R^{7b}, R^{8b}, R^{9b} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a COOR^{15b} moiety,
W^{b} represents an unbranched or branched, saturated or unsaturated, optionally at least mono-substituted aliphatic radical,
a saturated or unsaturated, optionally at least mono-substituted, optionally at least one heteroatom as ring member containing cycloaliphatic radical, which may be bonded via an optionally mono-substituted alkylene group and/or may be condensed with an optionally at least mono-substituted mono- or polycyclic ring-system,
an optionally at least mono-substituted aryl or heteroaryl radical, which may be bonded via an optionally mono-substituted alkylene group and/or may be condensed with an optionally at least mono-substituted mono- or polycyclic ring-system,
a NR^{16b}R^{17b}-moiety,
or a COR^{18b}-moiety,
R^{10b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{12b} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13b} and R^{14b} each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{13b} and R^{14b} together with the bridging nitrogen atom form a saturated, unsaturated or aromatic heterocyclic ring, which may be at least mono-substituted and/or contain at least one further heteroatom as a ring member,
R^{15b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{16b} represents an unbranched or branched, saturated or unsaturated, optionally at least mono-substituted aliphatic radical,
R^{17b} represents an unbranched or branched, saturated or unsaturated, optionally at least mono-substituted aliphatic radical, and
R^{18b} represents an optionally at least mono-substituted aryl radical, optionally in form of one of its stereoisomers, preferably enantiomers or diastereomers, its racemate or in form of a mixture of at least two of its stereoisomers, preferably enantiomers or diastereomers, in any mixing ratio, or a corresponding salt thereof, preferably physiologically acceptable salts thereof, or a solvate, respectively,
and sulphonamide-derived compounds of general formula (Ic), 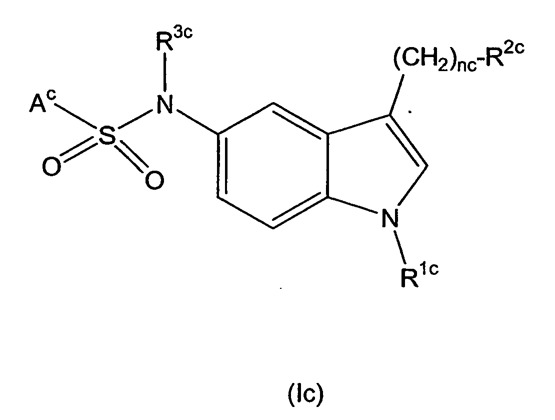 wherein
R^{1c} represents hydrogen, an optionally at least mono-substituted, linear or branched alkyl radical, an optionally at least mono-substituted phenyl radical or an optionally at least mono-substituted benzyl radical,
R^{2c} represents a -NR^{4c}R^{5c} moiety or a saturated or unsaturated, optionally at least mono-substituted, optionally at least one heteroatom as ring member containing cycloaliphatic radical, which may be condensed with a saturated or unsaturated, optionally at least mono-substituted, optionally at least one heteroatom as ring member containing mono- or bicyclic cycloaliphatic ringsystem,
R^{3c} represents hydrogen or an optionally at least mono-substituted, linear or branched alkyl radical,
R^{4c} and R^{5c}, identical or different, represent hydrogen or an optionally at least mono-substituted, linear or branched alkyl radical, or
R^{4c} and R^{5c} together with the bridging nitrogen atom form an optionally at least mono-substituted, saturated or unsaturated heterocyclic ring, which may contain at least one further heteroatom as a ring member and/or may be condensed with a saturated or unsaturated, optionally at least mono-substituted, optionally at least one heteroatom as ring member containing mono- or bicyclic cycloaliphatic ringsystem,
A^{c} represents an optionally at least mono-substituted mono- or polycyclic aromatic ringsystem, which may be bonded via an optionally at least mono-substituted alkylene group, an optionally at least mono-substituted alkenylene group or an optionally at least mono-substituted alkynylene group and/or may contain at least one heteroatom as a ring member in one or more of its rings,
nc represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate,
and compounds of the general formula (Id) 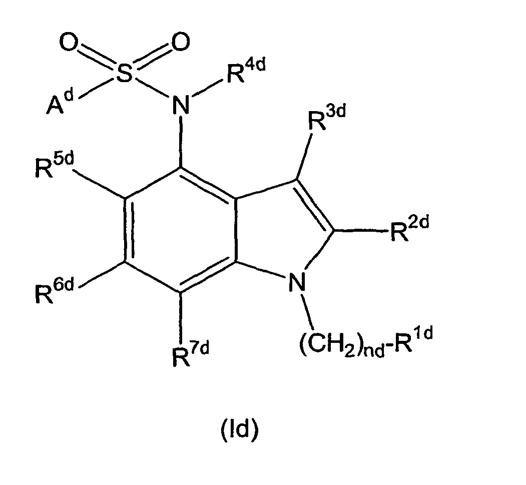 wherein
R^{1d} represents a -NR^{8d}R^{9d} radical or a saturated or unsaturated, optionally at least mono-substituted cycloaliphatic radical, which may contain at least one heteroatom as a ring member and/or which may be condensed with a saturated or unsaturated, optionally at least mono-substituted, optionally at least one heteroatom as a ring member containing mono- or bicyclic cycloaliphatic ring system,
R^{2d}, R^{3d}, R^{5d}, R^{6d} and R^{7d}, identical or different, each represent hydrogen, halogen, nitro, alkoxy, cyano, a saturated or unsaturated, linear or branched, optionally at least mono-substituted aliphatic radical, or an optionally at least mono-substituted phenyl or an optionally at least mono-substituted heteroaryl radical,
R^{4d} is hydrogen or a saturated or unsaturated, linear or branched, optionally at least mono-substituted aliphatic radical,
R^{8d} and R^{9d}, identical or different, each represent hydrogen or a saturated or unsaturated, linear or branched, optionally at least mono-substituted aliphatic radical,
R^{8d} and R^{9d} together with bridging nitrogen atom form a saturated or unsaturated, optionally at least mono-substituted heterocyclic ring, which may contain at least one additional heteroatom as a ring member and/or may be condensed with a saturated or unsaturated, optionally at least mono-substituted mono- or bicyclic cycloaliphatic ring system, which may optionally contain at least one heteroatom as a ring member,
A^{d} represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
and
nd is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof
and sulphonamide-derived compounds of general formula (Ie), 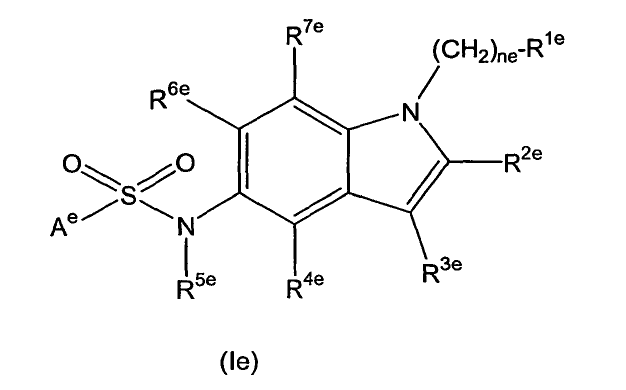 wherein
R^{1e} represents a -NR^{8e}R^{9e} radical or a saturated or unsaturated, optionally at least mono-substituted cycloaliphatic radical, which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member,
R^{2e}, R^{3e}, R^{4e}, R^{6e} and R^{7e}, identical or different, each represent hydrogen, halogen, nitro, alkoxy, cyano, a saturated or unsaturated, linear or branched, optionally at least mono-substituted aliphatic radical or an optionally at least mono-substituted phenyl or an optionally at least mono-substituted heteroaryl radical,
R^{5e} represents hydrogen or a saturated or unsaturated, linear or branched, optionally at least mono-substituted aliphatic radical,
R^{8e} and R^{9e}, identical or different, each represent hydrogen or a saturated or unsaturated, linear or branched, optionally at least mono-substituted aliphatic radical,
or
R^{8e} and R^{9e} together with the bridging nitrogen atom form a saturated or unsaturated, optionally at least mono-substituted heterocyclic ring, which may contain at least one additional heteroatom as a ring member and/or which may be condensed with a saturated or unsaturated, optionally at least mono-substituted, mono- or bicyclic cycloaliphatic ring system which may optionally contain at least one heteroatom as a ring member,
A^{e} represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings
and
ne is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof,
and sulphonamide-derived compounds of general formula (If), 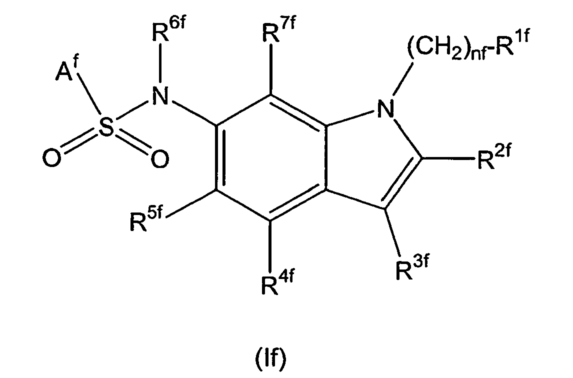 wherein
R^{1f} represents a -NR^{8f}R^{9f} radical or a saturated or unsaturated, optionally at least mono-substituted, optionally at least one heteroatom as a ring member containing cycloaliphatic radical, which may be condensed with a saturated or unsaturated, optionally at least mono-substituted, optionally at least one heteroatom as a ring member containing mono- or bicyclic cycloaliphatic ring system,
R^{2f}, R^{3f}, R^{4f}, R^{5f} and R^{7f}, identical or different, each represent hydrogen, halogen, nitro, alkoxy, cyano, a saturated or unsaturated, linear or branched, optionally at least mono-substituted aliphatic radical, or an optionally at least mono-substituted phenyl or optionally at least mono-substituted heteroaryl radical,
R^{6f} represents hydrogen or a saturated or unsaturated, linear or branched, optionally at least mono-substituted aliphatic radical,
R^{8f} and R^{9f}, identical or different, each represent hydrogen or a saturated or unsaturated, linear or branched, optionally at least mono-substituted aliphatic radical,
or
R^{8f} and R^{9f}, together with the bridging nitrogen atom, form a saturated or unsaturated, optionally at least mono-substituted heterocyclic ring, which may contain at least one further heteroatom as a ring member and/or which may be condensed with a saturated or unsaturated, optionally at least mono-substituted, optionally at least one heteroatom as a ring member containing mono- or bicyclic cycloaliphatic ring system,
A^{f} represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
and
nf is 0, 1, 2, 3 or 4;
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof,
and sulphonamide-derived compounds of general formula (Ig), 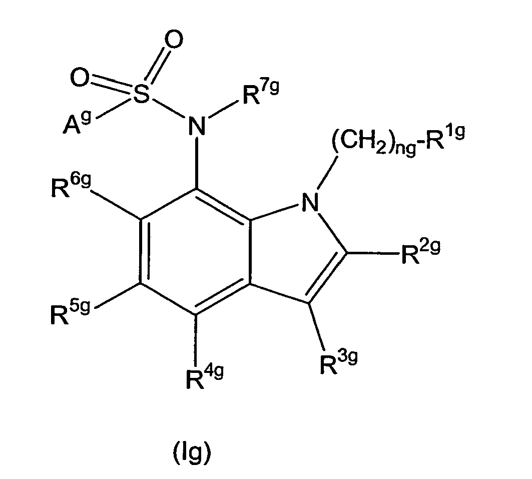 wherein
R^{1g} is a -NR^{8g}R^{9g} radical or a saturated or unsaturated, optionally at least mono-substituted cycloaliphatic radical, which may optionally contain at least one heteroatom as a ring member and which may be condensed with a saturated or unsaturated, optionally at least mono-substituted mono- or bicyclic cycloaliphatic ring system which may optionally contain at least one heteroatom as a ring member,
R^{2g}, R^{3g}, R^{4g}, R^{5g} and R^{6g}, identical or different, each represent hydrogen, halogen, nitro, alkoxy, cyano, a saturated or unsaturated, linear or branched, optionally at least mono-substituted aliphatic radical, or an optionally at least mono-substituted phenyl radical or an optionally at least mono-substituted heteroaryl radical,
R^{7g} represents hydrogen or a saturated or unsaturated, linear or branched, optionally at least mono-substituted aliphatic radical,
R^{8g} and R^{9g}, identical or different, represent hydrogen or a saturated or unsaturated, linear or branched, optionally at least mono-substituted aliphatic radical,
or
R^{8g} and R^{9g} together with the bridging nitrogen atom form a saturated or unsaturated, optionally at least mono-substituted heterocyclic ring, which may contain at least one additional heteroatom as a ring member and/or which may be condensed with a saturated or unsaturated, optionally at least mono-substituted mono- or bicyclic cycloaliphatic ring system, which may optionally contain at least one heteroatom as a ring member,
A^{g} represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
ng is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof,
and sulphonamide-derived compounds of general formula (Ih) 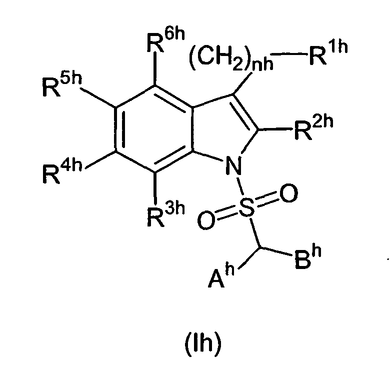 wherein
R^{1h} represents a -NR^{7h}R^{8h} radical or a saturated or unsaturated, optionally at least mono-substituted, optionally at least one heteroatom as a ring member containing cycloaliphatic radical, which may be condensed with a saturated or unsaturated, optionally at least mono-substituted, optionally at least one heteroatom as a ring member containing mono- or bicyclic cycloaliphatic ring system,
R^{2h}, R^{3h}, R^{4h}, R^{5h} and R^{6h}, identical or different, each represent hydrogen, halogen, cyano, nitro, a saturated or unsaturated, linear or branched, optionally at least mono-substituted aliphatic radical, a linear or branched alkoxy radical, a linear or branched alkylthio radical, hydroxy, trifluoromethyl, a saturated or unsaturated cycloaliphatic radical, an alkylcarbonyl radical, a phenylcarbonyl or a -NR⁹R¹⁰ group,
R^{7h} and R⁸ⁿ, identical or different, each represent hydrogen or a saturated or unsaturated, optionally at least mono-substituted linear or branched aliphatic radical,
or
R^{7h} and R^{8h}, together with the bridging nitrogen atom form a saturated or unsaturated, optionally at least mono-substituted, optionally at least one further heteroatom as a ring member containing heterocyclic ring which may be condensed with a saturated or unsaturated, optionally at least mono-substituted, optionally at least one heteroatom as a ring member containing mono- or bicyclic cycloaliphatic ring system,
R^{9h} and R^{10h}, identical or different, each represent hydrogen or a saturated or unsaturated, linear or branched, optionally at least mono-substituted aliphatic radical,
or
R^{9h} and R^{10h}, together with the bridging nitrogen atom form a saturated or unsaturated, optionally at least mono-substituted, optionally at least one further heteroatom as a ring member containing heterocyclic ring which may be condensed with a saturated or unsaturated, optionally at least mono-substituted, optionally at least one heteroatom as a ring member containing mono- or bicyclic cycloaliphatic ring system,
A^{h} and B^{h}, identical or different, each represent a saturated or unsaturated, linear or branched, optionally at least mono-substituted aliphatic radical
or
A^{h} and B^{h}, together with the carbon atom to which they are attached, form a saturated or unsaturated, but not aromatic, optionally at least mono-substituted cycloalkyl ring,
and
nh is 0, 1, 2, 3, or 4,
optionally in form of one of their stereoisomers, preferably enantiomers or diastereomers, their racemate or in form of a mixture of at least two of their stereoisomers, preferably enantiomers or diastereomers, in any mixing ratio, or a salt thereof, preferably a corresponding physiologically acceptable salt thereof or a corresponding solvate thereof.

The persons skilled in the state of the art understand that the active substance combination according to the present invention may comprise one or more compounds of one class of active substances with 5-HT₆ receptor affinity or one or more compounds of one or more different classes of active substances with 5-HT₆ receptor affinity.

If one or more of the residues R^{1b}-R^{17b} and W^{b} represents an aliphatic radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, - SO₂-C₁₋₄-alkyl, -NH-SO₂-C₁₋₄-alkyl, wherein the C₁₋₄-alkyl may in each case be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, CF₃ and an unsubstituted phenyl radical. If any one of the above mentioned substitutents itself is at least mono-substituted, said substituents may preferably be selected from the group consisting of F, Cl, methyl and methoxy.

If one or more of the residues R^{1b}-R^{15b} represents or comprises a cycloaliphatic radical, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, phenoxy, benzoyl, cyclohexyl, branched or unbranched C₁₋₄-perfluoroalkyl, -NR^{Ab}R^{Bb} wherein R^{Ab}, R^{Bb} are each independently selected from the group consisting of H, a branched or unbranched C₁₋₄-alkyl-radical, -CH₂-CH₂-OH and phenyl, carboxy, keto, amido, cyano, nitro, SO₂NH₂, -CO-C₁₋₄-alkyl, -CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may in each case be branched or unbranched, unsubstituted or at least mono-substituted phenyl or naphthyl and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, methoxy, ethoxy, keto, benzoyl, phenoxy, cyclohexyl, -CF₃, -CO-CH₃, -CO-OCH₃, -NR^{Ab}R^{Bb} wherein R^{Ab}, R^{Bb} are each independently selected from the group consisting of H, a branched or unbranched C₁₋₄-alkyl-radical, -CH₂-CH₂-OH and phenyl, and an unsubstituted phenyl radical. If any one of the above mentioned substitutents itself is at least mono-substituted, said substituents may preferably be selected from the group consisting of F, Cl, methyl and methoxy.

If one or more of the residues R^{1b}-R^{4b}, R^{10b}-R^{15b} and W^{b} comprises an alkylene group, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, -SO₂-C₁₋₄-alkyl,-NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, methoxy, ethoxy, CF₃ and unsubstituted phenyl. If any one of the above mentioned substitutents itself is at least mono-substituted, said substituents may preferably be selected from the group consisting of F, Cl, methyl and methoxy.

If one or more of the residues R^{1b}-R^{4b} and R^{10b}-R^{15b} comprises a mono- or polycyclic ringsystem, which is substituted by one or more substituents, unless defined otherwise, each of these substituents may preferably be selected from the group consisting of hydroxy, halogen, branched or unbranched C₁₋₄-alkyl, branched or unbranched C₁₋₄-alkoxy, branched or unbranched C₁₋₄-perfluoroalkoxy, branched or unbranched C₁₋₄-perfluorocarbonyl, branched or unbranched C₁₋₄-perfluoroalkyl, amino, carboxy, amido, cyano, keto, nitro,-SO₂NH₂, -CO-C₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl, more preferably from the group consisting of hydroxy, F, Cl, Br, methyl, ethyl, methoxy, ethoxy, CF₃, -(C=O)-CF₃, keto, cyano and an unsubstituted phenyl radical. If any one of the above mentioned substitutents itself is at least mono-substituted, said substituents may preferably be selected from the group consisting of F, Cl, methyl and methoxy.

If one or more of the residues R^{1b}-R^{4b}, R^{10b}-R^{15b} and R^{18b} represents or comprises an aryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{Ab}R^{Bb} wherein R^{Ab}, R^{Bb} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂, -CO-C₁₋₄-alkyl, -CO-OC₁₋₄-alkyl, -SO-C₁₋₄-alkyl, -SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, nitro, -CH(OH)(phenyl), methoxy, ethoxy, unsubstituted or at least mono-substituted benzoyl, unsubstituted or at least mono-substituted phenoxy, cyclohexyl, CF₃, OCF₃, -CO-CH₃, -CO-OCH₃, SO₂-CH₃, -NR^{Ab}R^{Bb} wherein R^{Ab}, R^{Bb} are each independently selected from the group consisting of H, a branched or unbranched C₁₋₄-alkyl-radical, -CH₂-CH₂-OH and phenyl, and an unsubstituted phenyl radical. If any of the above mentioned substitutents itself is at least mono-substituted, said substituents may preferably be selected from the group consisting of F, Cl, Br, CF₃, OCF₃, methyl and methoxy.

If one or more of the residues R^{1b}-R^{4b} and R^{10b}-R^{15b} represents or comprises a heteroaryl radical, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, unsubstituted or at least mono-substituted phenoxy, unsubstituted or at least mono-substituted benzoyl, cyclohexyl, branched or unbranched C₁₋₄-perfluoroalkyl, NR^{Ab}R^{Bb} wherein R^{Ab}, R^{Bb} are each independently selected from the group consisting of H, a branched or unbranched C₁₋₄-alkyl-radical, -CH₂-CH₂-OH and phenyl, carboxy, amido, cyano, -CH(OH)(phenyl), nitro, -SO₂NH₂, -CO-C₁₋₄-alkyl, -CO-OC₁₋₄-alkyl, SO-C₁₋₄-alkyl, SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methyl, ethyl, cyano, nitro, CH(OH)(phenyl), methoxy, ethoxy, unsubstituted or at least mono-substituted benzoyl, unsubstituted or at least mono-substituted phenoxy, cyclohexyl, CF₃, OCF₃, -CO-CH₃, -CO-OCH₃, -SO₂CH₃, -NR^{Ab}R^{Bb} wherein R^{Ab}, R^{Bb} are each independently selected from the group consisting of H, a branched or unbranched C₁₋₄-alkyl-radical, -CH₂-CH₂-OH and phenyl, and an unsubstituted phenyl radical. If any one of the above mentioned substitutents itself is at least mono-substituted, said substituents may preferably be selected from the group consisting of F, Cl, Br, CF₃, OCF₃, methyl and methoxy.

If R^{13b} and R^{14b} form a heterocyclic ring, which is substituted by one or more substituents, unless defined otherwise, each of these substituents may preferably be selected from the group consisting of hydroxy, halogen, branched or unbranched C₁₋₄-alkoxy, branched or unbranched C₁₋₄-alkyl, branched or unbranched C₁₋₄-perfluoroalkoxy, branched or unbranched C₁₋₄-perfluoroalkyl, amino, carboxy, amido, cyano, nitro, -SO₂NH₂, -CO-C₁₋₄-alkyl, -SO-C₁₋₄-alkyl, - SO₂-C₁₋₄-alkyl, -NH-SO₂-C₁₋₄-alkyl, wherein C₁₋₄-alkyl may be branched or unbranched, an unsubstituted or at least mono-substituted phenyl or naphthyl radical and an unsubstituted or at least mono-substituted furanyl-, thienyl-, pyrrolyl-, imidazolyl-, pyrazolyl-, pyridinyl-, pyrimidinyl-, quinolinyl- and isoquinolinyl radical, more preferably be selected from the group consisting of hydroxy, F, Cl, Br, methoxy, ethoxy, methyl, CF₃ and an unsubstituted phenyl radical. If any of the above mentioned substitutents itself is at least mono-substituted, said substituents may preferably be selected from the group consisting of F, Cl, methyl and methoxy.

If R^{13b} and R^{14b} form a heterocyclic ring, which contains one or more further heteroatoms as ring members, unless defined otherwise, each of these heteroatoms may preferably be selected from the group consisting of N, O and S, more preferably from the group consisting of N and O.

If one or more of the residues R^{1b}-R^{15b} and W^{b} represents a cycloaliphatic radical, which contains one or more heteroatoms as ring members, unless defined otherwise, each of these heteroatoms may preferably be selected from the group consisting of N, O, S and P, more preferably from the group consisting of N, O and S.

If one or more of the residues R^{1b}-R^{4b}, R^{10b}-R^{15b} and W^{b} represents or comprises an heteroaryl radical, which contains one or more heteroatoms as ring members, unless defined otherwise, each of these heteroatoms may preferably be selected from the group consisting of N, O, S and P, more preferably from the group consisting of N, O and S.

If W^{b} represents or comprises a cycloaliphatic radical, a heteroaryl radical, an aryl radical and/or a mono- or polycyclic ring system, which is substituted by one or more substituents, unless defined otherwise, each of these substituents may preferably be selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₆-alkenyl, SO₂-C₁₋₄-alkyl, -(C=O)-C₁₋₅-alkyl, -(C=O)-O-C₁₋₅-alkyl,-(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{Ab}R^{Bb}, wherein R^{Ab} and R^{Bb} are independently selected from the group consisting of H, C₁₋₄-alkyl and phenyl, NH-(C=O)-C₁₋₅-alkyl, -C₁₋₅-alkylen-(C=O)-C1-5-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-,-SO₂-pyrrolidinyl morpholinyl, SO2-morpholinyl-, thiadiazolyl, oxadiazolyl, oxazolyl, thiazolyl, isoxazolyl, O-CH₂-thiazolyl,-, NH-phenyl, and -C₁₋₄-Alkylen-NH-(C=O)-phenyl, more preferably from the group consisting of hydroxy, nitro, carboxy, cyano, keto, F, Cl, Br, I, C₁₋₁₂-alkyl, CH₂F, CHF₂, CF₃, CH₂Cl, CH₂Cl₂, CCl₃, CH₂Br, CHBr₂, CBr₃, OCF₃, OCHF₂, OCH₂F, O-CH₂-CF₃, vinyl, SO2-CH₃, -(C=O)-CH₃, -(C=O)-C₂H₅, -(C=O)-O-CH₃, -(C=O)-O-C₂CH₅, -(C=O)-Cl, -S-CH₃-, -(C=O)-H, -NH-(C=O)-NH-CH₃, -(C=O)-CF₃, dimethylamino, diethylamino, din-propylamino, di-iso-propylamino, di-n-butylamino, di-tert-butyamino, NH-(C=O)-CH₃, -CH₂-(C=O)-CH₃, -CH₂-(C=O)-C₂H₅, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl,-, NH-phenyl, and -CH₂-NH-(C=O)-phenyl. If any of the afore mentioned substituents itself is substituted by one or more substituents, said substituents may preferably be selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, -C(=O)-O-C₁₋₅-alkyl, -(C=O)-CH₂-F, -(C=O)-CH₂-Cl,-(C=O)-CH₂-Br, preferably from the group consisting of F, Cl, Br, CH₂F, CHF₂, CF₃, CH₂Cl, CHCl₂, CCl₃, CH₂Br, CHBr₂, CBr₃, nitro, cyano, hydroxy, -(C=O)-CH₃, CH₃, C₂H₅, -S-CH₃, -C(=O)-O-CH₃, -C(=O)-O-C₂H₅, -(C=O)-CH₂-F, - (C=O)-CH₂-Cl and -(C=O)-CH₂-Br.

Also preferably, the substituents for W^{b} may be selected from the group consisting of hydroxy, nitro, carboxy, cyano, keto, halogen, C₁₋₂₀-alkyl, partially fluorinated C₁₋₄ alkyl, partially chlorinated C₁₋₄ alkyl, partially brominated C₁₋₄ alkyl, C₁₋₅-alkoxy, partially fluorinated C₁₋₄ alkoxy, partially chlorinated C₁₋₄ alkoxy, partially brominated C₁₋₄ alkoxy, C₂₋₆-alkenyl, SO₂-C₁₋₄-alkyl, -(C=O)-C₁₋₅-alkyl, -(C=O)-O-C₁₋₅-alkyl, -(C=O)-Cl, -S-C₁₋₄-alkyl-, -(C=O)-H, -NH-(C=O)-NH-C₁₋₅-alkyl, -(C=O)-C₁₋₄-perfluoroalkyl, -NR^{A}R^{B}, wherein R^{A} and R^{B} are independently selected from the group consisting of H, C₁₋₄-alkyl and phenyl, NH-(C=O)-C₁₋₅-alkyl, -C₁₋₅-alkylen-(C=O)-C₁₋₅-alkyl, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-, -SO₂-pyrrolidinyl, morpholinyl, SO₂-morpholinyl-, thiadiazolyl, oxadiazolyl, oxazolyl, thiazolyl, isoxazolyl, O-CH₂-thiazolyl,-, NH-phenyl, and -C₁₋₄-Alkylen-NH-(C=O)-phenyl, more preferably from the group consisting of hydroxy, nitro, carboxy, cyano, keto, F, Cl, Br, l, C₁₋₁₂-alkyl, CH₂F, CHF₂, CF₃, CH₂Cl, CH₂Cl₂, CCl₃, CH₂Br, CHBr₂, CBr₃, OCF₃, OCHF₂, OCH₂F, O-CH₂-CF₃, vinyl, SO₂-CH₃, -(C=O)-CH₃, -(C=O)-C₂H₅,-(C=O)-O-CH₃, -(C=O)-O-C₂CH₅, -(C=O)-Cl, -S-CH₃-, -(C=O)-H, -NH-(C=O)-NH-CH₃, -(C=O)-CF₃, dimethylamino, diethylamino, di-n-propylamino, di-iso-propylamino, di-n-butylamino, di-tert-butyamino, NH-(C=O)-CH₃, -CH₂-(C=O)-CH₃, -CH₂-(C=O)-C₂H₅, (1,3-Dihydro-1-oxo-2H-isoindol-2-yl), N-Phthalimidinyl-, (1,3-Dioxo-2-azaspiro[4,4]-non-2-yl, substituted or unsubstituted phenyl, -SO₂-phenyl, phenoxy, pyridinyl, pyridinyloxy, pyrazolyl, pyrimidinyl, pyrrolidinyl-,-SO₂-pyrrolidinyl, morpholinyl, SO₂-morpholinyl-, thiadiazolyl, oxadiazolyl, oxazolyl, thiazolyl, isoxazolyl, O-CH₂-thiazolyl,-, NH-phenyl, and -CH₂-NH-(C=O)-phenyl. If any of the afore mentioned substituents itself is substituted by one or more substituents, said substituents may preferably be selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, -C(=O)-O-C₁₋₅-alkyl, -(C=O)-CH₂-F, -(C=O)-CH₂-Cl, -(C=O)-CH₂-Br, preferably from the group consisting of F, Cl, Br, CH₂F, CHF₂, CF₃, CH₂Cl, CHCl₂, CCl₃, CH₂Br, CHBr₂, CBr₃, nitro, cyano, hydroxy, -(C=O)-CH₃, CH₃, C₂H₅, -S-CH₃, -C(=O)-O-CH₃, -C(=O)-O-C₂H₅, -(C=O)-CH₂-F, -(C=OFCH₂-Cl and -(C=O)-CH₂-Br.

If any of the afore mentioned substituents itself is substituted by one or more substituents, said substituents may preferably be selected from the group consisting of halogen, nitro, cyano, hydroxy, -(C=O)-C₁₋₄-alkyl, C₁₋₄-alkyl, at least partially fluorinated C₁₋₄-alkyl, at least partially chlorinated C₁₋₄-alkyl, at least partially brominated C₁₋₄-alkyl, -S-C₁₋₄-alkyl, -C(=O)-O-C₁₋₅-alkyl, -(C=O)-CH₂-F, -(C=O)-CH₂-Cl, -(C=O)-CH₂-Br, preferably from the group consisting of F, Cl, Br, CH₂F, CHF₂, CF₃, CH₂Cl, CHCl₂, CCl₃, CH₂Br, CHBr₂, CBr₃, nitro, cyano, hydroxy, -(C=O)-CH₃, CH₃, C₂H₅, -S-CH₃, -C(=O)-O-CH₃, -C(=O)-O-C₂H₅, -(C=O)-CH₂-F, -(C=O)-CH₂-Cl and -(C=O)-CH₂-Br.

The use of compounds of general formula (Ib) is preferrred, wherein R^{1b}, R^{2b}, R^{3b}, R^{4b} are each independently selected from the group consisting of H, F, Cl, Br, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁-₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a nitro, cyano, -OR^{10b}, -OC(=O)R^{11b}, -SR^{12b}, -SOR^{12b}, -SO₂R^{12b}, -NH-SO₂R^{12b}, -SO₂NH₂ and a -NR^{13b}R^{14b} moiety,
preferably selected from the group consisting of H, F, CI, Br, a saturated, branched or unbranched, optionally at least mono-substituted C₁₋₃-aliphatic radical, a saturated, optionally at least mono-substituted, optionally at least one heteroatom as ring member containing C₅- or C₆- cycloaliphatic radical, which may be bonded via an optionally at least mono-substituted C₁- or C₂-alkylene group, a nitro, cyano, -OR^{10b}, -OC(=O)R^{11b}, -SR^{12b} and -NR^{13b}R^{14b} moiety,
more preferably selected from the group consisting of H, F, Cl, CH₃, CH₂CH₃, CF₃, CF₂CF₃, cyclopentyl, cyclohexyl, nitro, cyano and -OR^{10b},
and R^{5b}-R^{18b} and W^{b} have the meaning as defined above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (lb), wherein R^{5b} represents hydrogen, an unbranched or branched, saturated or unsaturated, optionally at least mono-substituted C₁₋₆-aliphatic radical or a saturated or unsaturated, optionally at least mono-substituted, optionally at least one heteroatom as ring member containing C₃₋₈-cycloaliphatic radical,
preferably represents H or a branched or unbranched C₁₋₃-alkyl radical,
more preferably H, CH₃ or CH₂CH₃,
and R^{1b}-R^{4b}, R^{6b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Preferred is also the use of compounds of general formula (lb), wherein R^{6b}, R^{7b}, R^{8b}, R^{9b} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₈-cycloaliphatic radical, a cyano and COOR^{15b} moiety,
preferably selected from the group consisting of H, a branched or unbranched C₁₋₃-alkyl radical, a cyano and a COOR^{15b} group,
more preferably from the group consisting of H, CH₃, CH₂CH₃ and a cyano moiety,
and R^{1b}-R^{5b}, R^{10b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (lb), wherein W^{b} represents an an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ringsystem, a NR^{16b}R^{17b}-moiety or a COR^{18b}-moiety,
preferably selected from the group consisting of 1-Naphthyl-, 5-Dimethylaminonapth-1-yl, 2-Naphthyl-, 2-Acetamido-4-methyl-5-thiazolyl-, 2-Thienyl-, 8-Quinolinyl-, Phenyl-, Pentafluorophenyl-, 2,4,5-Trichloro-phenyl-, 2,5-Dichlorophenyl-, 2-Nitrophenyl-, 2,4-Dinitro-phenyl-, 3,5-Dichloro-2-hydroxy-phenyl-, 2,4,6-Trisisopropyl-phenyl-, 2-Mesityl-, 3-Nitro-phenyl-, 4-Bromo-phenyl-, 4-Fluoro-phenyl-, 4-Chlorophenyl-, 4-Chloro-3-nitro-phenyl-, 4-lodo-phenyl-, N-Acetyl-sulfanilyl-, 4-Nitro-phenyl-, 4-Methoxy-phenyl-, Benzoic-acid-4-yl-, 4-tert-Butyl-phenyl-, p-Tolyl-, Trifluoromethyl-, Trichloromethyl-, Isopropyl-, Methyl-, Benzyl-, trans-styryl-, 2,2,2-Trifluoroethyl-, Ethyl-, Hexadecyl-, 2-Chloroethyl-, n-Propyl-, 3-Chloro-propyl-, n-Butyl-, Methyl-benzoate-2-yl-, 2-Nitro-4-(trifluoromethyl)-phenyl-, Pentamethyl-phenyl-, 2,3,5,6-Tetramethyl-phenyl-, 3-(Trifluoromethyl)-phenyl-, 3,5-Bis-(Trifluoromethyl)-phenyl-, Dichloromethyl-, Chloromethyl-, Dodecyl-, 1-Octyl-, 2,3,4-Trichloro-phenyl-, 2,5-Dimethoxy-phenyl-, o-Tolyl-, p-xylyl-2-yl-, Benzoic-acid-3-yl-, 4-Chloro-3-(trifluoromethyl)-phenyl-, 4-Chloro-5-nitro-benzoic acid-3-yl-, 6-(p-toluidino)-naphth-2-yl-, 4-Methoxy-2,3,6-trimethylphenyl-, 3,4-Dichlorophenyl-, 4,5-Dibromo-thiophene-2-yl-, 3-Chloro-4-fluoro-phenyl-, 4-Ethyl-phenyl-, 4-n-Propyl-phenyl-, 4-(1,1-Dimethylpropyl)-phenyl-, 4-Isopropyl-phenyl-, 4-Bromo-2,5-difluoro-phenyl-, 2-Fluoro-phenyl-, 3-Fluoro-phenyl-, 4-(Trifluoromethoxy)-phenyl-, 4-(Trifluoromethyl)-phenyl-, 2,4-Difluoro-phenyl-, 2,4-Dichloro-5-methyl-phenyl-, 4-Chloro-2,5-dimethyl-phenyl-, 5-Diethylamino-napth-2-yl-, Benzoyl chloride-3-yl-, 2-Chloro-phenyl-, 1-Octadecyl-, 4-Bromo-2,5-dichloro-thiophene-3-yl-, 2,5-Dichloro-thiophene-3-yl-, 5-Chloro-thiophene-2-yl-, 2-Methyl-5-nitro-phenyl-, 2-(Trifluoromethyl)-phenyl-, 3-Chloro-phenyl-, 3,5-Dichloro-phenyl-, 1-Decyl-, 3-Methyl-phenyl-, 2-Chloro-6-methyl-, 5-Bromo-2-methoxy-phenyl-, 3,4-Dimethoxy-phenyl-, 2-3-Dichloro-phenyl-, 2-Bromo-phenyl-, 3,5-Dichloro-4-(2-chloro-4-nitrophenoxy)-phenyl-, 2,3-Dichloro-thiophene-5-yl-, 3-Bromo-2-chlorothiophene-5-yl-, 3-Bromo-5-chloro-thiophene-2-yl-, 2-(Benzoylaminomethyl)-thiophene-5-yl-, 4-(Phenyl-sulphonyl)-thiophene-2-yl-, 2-Phenyl-sulphonylthiophene-5-yl-, 3-Chloro-2-methyl-phenyl-, 2-[1-Methyl-5-(trifluoromethyl)pyrazol-3-yl]-thiophene-5-yl-, 5-Pyrid-2-yl-thiophene-2-yl-, 2-Chloro-5-(trifluoromethyl)-phenyl-, 2,6-Dichloro-phenyl-, 3-Bromo-phenyl-, 2-(Trifluoromethoxy)-phenyl-, 4-Cyano-phenyl-, 2-Cyano-phenyl-, 4-n-Butoxyphenyl-, 4-Acetamido-3-chloro-phenyl, 2,5-Dibromo-3,6-difluoro-phenyl-, 5-Chloro-1,3-dimethylpyrazole-4-yl-, 3,5-Dimethylisoxazole-4-yl-, 2-(2,4-Dichlorophenoxy)-phenyl-, 4-(2-Chloro-6-nitro-phenoxy)-phenyl-, 4-(3-Chloro-2-cyano-phenoxy)-phenyl-, 2,4-Dichloro-phenyl-, 2,4-Dimethyl-1,3-thiazole-5-yl-, Methyl-methane-sulfonyl-, 2,5-Bis-(2,2,2-Trifluoroethoxy)-phenyl-, 2-Chloro-4-(trifluoromethyl)-phenyl-, 2-Chloro-4-fluoro-phenyl-, 5-Fluoro-2-methyl-phenyl-, 5-Chloro-2-methoxy-phenyl-, 2,4,6-Trichloro-phenyl-, 2-Hydroxy-benzoic acid-5-yl-, 5-(Di-n-propylamino)-naphth-1-yl-, 6-Methoxy-m-tolyl-, 2,5-Difluoro-phenyl-, 2,4-Dimethoxy-phenyl-, 2,5-Dibromo-phenyl-, 3,4-Dibromo-phenyl-, 2,2,5,7,8-Pentamethyl-chroman-6-yl-, 2-Methoxy-benzoic-acid-5-yl-, 5-Chloro-4-nitrothiophene-2-yl-, 2,1,3-Benzothiadiazole-4-yl-, 1-Methyl-imidazole-4-yl-, Benzofurazan-4-yl-, 2-(Methoxycarbonyl)-thiophene-3-yl-, 5-(Isoxazol-3-yl)-thiophene-2-yl-, 2,4,5-Trifluoro-phenyl-, Biphenyl-4-yl-, Vinyl-phenyl-4-yl-, 2-Nitro-benzyl-, 5-Dichloro-methyl-furan-2-yl-, 5-Bromo-thiophene-2-yl-, 5-(4-Chlorobenzamidomethyl)-thiophene-2-yl-, 2,6-Difluoro-phenyl-, 2,5-Dimethoxy-4-nitro-phenyl-, Dibenzo[B,D]-furan-2-yl-, 2,3,4-Trifluoro-phenyl-, 3-Nitro-p-tolyl-, 4-Methoxy-2-nitro-phenyl-, 3,4-Difluoro-phenyl-, 4-(Bromoethyl)-phenyl-, 3,5-Dichloro-4-hydroxy-phenyl-, 4-n-Amyl-phenyl-, 5-Chloro-3-methylbenzo[B]-thiophene-2-yl-, 3-Methoxy-4-(methoxycarbonyl)-thiophene-2-yl-, 4-n-Butyl-phenyl-, 2-Chloro-4-cyano-phenyl-, 5-[2-(Methylthio)-pyrimidin-4-yl-]-thiophene-2-yl-, 3,5-Dinitro-4-methoxy-phenyl-, 4-Bromo-2-(trifluoromethoxy)-phenyl-, 4-Chloro-2,1,3-Benzoxadiazole-7-yl-, 2-(1-Naphthyl)-ethyl-, 3-Cyano-phenyl-, 5-Chloro-2,1,3-Benzoxadiazole-4-yl-, 3-Chloro-4-methyl-phenyl-, 4-Bromo-2-ethylphenyl-, 2,4-Dichloro-6-methyl-phenyl-, 6-Chloro-imidazo(2,1-B)-thiazole-5-yl-, 3-Methyl-benzo[B]-thiophene-2-yl-, 4-Methyl-sulphonyl-phenyl-, 2-Methyl-sulphonyl-phenyl-, 4-Bromo-2-methyl-phenyl-, 2,6-Dichloro-4-(trifluoromethyl)-phenyl-, 4-[[3-Chloro-5-(trifluoromethyl)-2-pyridinyl]oxy]-phenyl-, 5-Chloronaphth-1-yl-, 5-Chloro-naphth-2-yl-, 9,10-Dibromoanthracene-2-yl-, Isoquinoline-5-yl-, 4-Methoxy-2,3,6-trimethyl-phenyl-, 4'-Nitro-biphenyl-4-yl-, [(4-Phenoxy)-phenyl-, (1,3-Dihydro-1-oxo-2H-isoindol-2-yl-)-4-phenyl-, 4-Acetyl-phenyl-, 5-(2-Methyl-1,3-thiazole-4-yl)-thiophene-2-yl-, 5-(1-Methyl-3-(trifluoromethyl)pyrazol-5-yl-]-thiophene-2-yl-, 5-[5-Trifluoromethyl)-isoxazol-3-yl]-thiophene-2-yl-, 2-lodo-phenyl-, p-Dodecyl-phenyl-, 4-[(3-Cyano-4-methoxy-2-pyridinyl)oxy]-phenyl-, 4-(N-phthalimidinyl)-phenyl-, 1,2,3,4-Tetrahydro-2-(trifluoroacetyl)-isoquinoline-7-yl-, 4-Bromo-2-fluoro-phenyl-, 2-Fluoro-5-(trifluoromethyl)-phenyl-, 4-Fluoro-2-(trifluoromethyl)-phenyl-, 4-Fluoro-3-(trifluoromethyl)-phenyl-, 2,4,6-Trifluoro-phenyl-, 3-(Trifluoromethoxy)-phenyl-, 1,2-Dimethylimidazole-4-yl-, Ethyl-4-Carboxylate-3-yl-, 2,2,4,6,7-Pentamethyldihydrobenzofuran-5-yl-, 3-Bromo-2-chloropyridine-5-yl-, 3-Methoxy-phenyl-, 2-Methoxy-4-methyl-phenyl-, 2-Chloro-4-fluorobenzoic-acid-5-yl-, 4-Chloro-naphth-1-yl-, 2,5-Dichloro-4-nitro-thiophene-3-yl-, 4-(4-Methoxyphenoxy)-phenyl-, 4-(4-Chloro-phenoxy)-phenyl-, 4-(3,5-Dichloro-phenoxy)-phenyl-, 4-(3,4-Dichloro-phenoxy)-phenyl-, 4-(4-Fluoro-phenoxy)-phenyl-, 4-(4-Methyl-phenoxy)-phenyl-, 4-[4-(Trifluormethyl)-phenoxy-phenyl-, 4-[3,5-Bis-(trifluoromethyl)-phenoxy]-phenyl-, 3-(2-Methoxy-phenoxy)-phenyl-, [3-(2-Chloro-phenoxy)-phenyl-, 3-(2-Methyl-phenoxy)-phenyl-, 4-[2-(Trifluoromethyl)-phenoxy]-phenyl-, 3-Phenyl-phenyl-, 3-(4-Methoxy-phenyl)-phenyl-, 3-(4-Chlorophenyl)-phenyl-, 3-(3,5-Dichloro-phenyl)-phenyl-, 3-(3,4-Dichloro-phenyl)-phenyl-, 3-(4-Fluorophenyl)-phenyl-, 3-(4-Methylphenyl)-phenyl-, 3-[4-(Trifluoromethyl)-phenyl]-phenyl-, 3-[3,5-Bis-(Trifluoromethyl)-phenyl]-phenyl-, 4-(4-Pyridyloxy)-phenyl)-, 4-(2-Methoxy-phenoxy)-phenyl-, 4-(2-Chlorophenoxy)-phenyl-, 4-(2-Methyl-phenoxy)-phenyl-, 4-(4-Methoxy-phenoxy)-phenyl-, 4-(4-Chlorophenyl)-phenyl-, 4-(3,5-Dichlorophenyl)-phenyl-, 4-(3,4-Dichlorophenyl)-phenyl-, 4-(4-Fluorophenyl)-phenyl-, 4-(4-Methylphenyl)-phenyl-, 4-[4-(Trifluormethyl)-phenyl]-phenyl-, 4-[3,5-Bis-(Trifluoromethyl)-phenyl]-phenyl-, [3-(Trifluoromethyl)-phenyl]-methyl-, (4-Chlorophenyl)-methyl-, (3,5-Dichlorophenyl)-methyl-, (3,5-Dichlorophenyl)-methyl-, (4-Fluorophenyl)-methyl-, 4-Methylphenylmethyl-, [4-(Trifluoromethyl)-phenyl]-methyl-, Cyclopropyl-, 2-(2-Chlorophenyl)-2-Phenylethyl-, 2-(2-Trifluoromethylphenyl)-2-phenylethyl-, 5-[4-Cyano-1-methyl-5-(methylthio)-1H-pyrazol-3-yl-thiophene-2-yl-, 3-Cyano-2,4-bis-(2,2,2-Trifluorothoxy)-phenyl-, 4-[(2-Chloro-1,3-Thiazol-5-yl)-methoxy]-phenyl-, 3-Nitro-phenylmethyl-, 4-Formylphenyl-, 2-(1,3-Dioxo-1,3-dihydro-isoindol-2-yl)-ethyl-, [3,5-Bis-(Trifluoromethyl)-phenyl]-methyl-, (4-(2-Pyridyloxy)-phenyl)-, (4-(3-Pyridyloxy)-phenyl)-, 5-lodo-naphth-1-yl-, Ethyl-2,5-dimethyl-1-phenylpyrrole-4-carboxylate-3-yl-, Ethyl-2-methyl-1,5-diphenyl-1H-pyrrole-3-carboxylate-4-yl-, Ethyl-5-(4-chlorophenyl)-2-methyl-3-furoate-4-yl, Ethyl-5-(4-chlorophenyl)-2-methyl-1-phenyl-3-carboxylate-4-yl-, Ethyl-2,5-dimethyl-3-furoate-4-yl-, 3-Chloro-4-(1,3-dioxo-2-Azaspiro[4,4]non-2-yl)-phenyl-, 5-Bromo-2,4-difluoro-phenyl-, 5-Chloro-2,4-difluorophenyl-, Coumarin-6-yl, 2-Methoxy-phenyl, (3-Phenoxy)-phenyl-, 3-(4-Methoxy-phenoxy)-phenyl-, 3-(4-Chlorophenoxy)-phenyl-, 3-(3,5-Dichlorophenoxy)-phenyl-, 3-(3,4-Dichlorophenoxy)-phenyl-, 3-(4-Fluorophenoxy)-phenyl-, 3-(4-Methylphenoxy)-phenyl-, 3-[4-(Trifluoromethyl)-phenoxy]-phenyl-, 3-[3,5-(Trifluoromethyl)-phenoxy]-phenyl-, 3-[2-(Trifluoromethyl)-phenoxy]-phenyl-, 2,2-Diphenylethyl-, 4-Phenyl-5-(trifluoromethyl)-thiophene-3-yl-, Methyl-4-Phenyl-5-(Trifluoromethyl)-thiophene-2-carboxylate-3-yl-, Methyl-1,2,5-trimethylpyrrole-3-Carboxylate-4-yl-, 4-Fluoro-naphth-1-yl-, 3,5-Difluorophenyl-, 3-Fluoro-4-methoxy-phenyl-, 4-Chloro-2,5-difluorophenyl-, 2-Chloro-4,5-difluoro-phenyl-, 5-Fluoro-3-methylbenzo[B]-thiophene-2-yl-, Methyl-3-phenylpropionate-4-yl, Dihydrocinnamic Acid-4-yl-, Methyl-2,5-dimethyl-3-furoate-4-yl-, Methyl-2-furoate-5-yl-, Methyl-2-methyl-3-furoate-5-yl-, Methyl-1-methyl-1H-pyrrole-2-Carboxylate-5-yl-, 2-(5-Chloro-1,2,4-Thiadiazol-3-yl)-thiophene-5-yl-, 1,3,5-Trimethyl-1 H-pyrazole-4-yl-, 3-Chloro-5-fluoro-2-methylphenyl-, Pentafluoroethoxytetrafluoroethyl-, 5-(5-Isoxazyl)-thiophene-2-yl-, 5-(5-Isoxazolyl)-2-furyl-, 5-Methyl-2,1,3-benzothiadiazole-4-yl-, Biphenyl-2-yl-, 2,3-Dihydro-1,4-benzodioxine-6-yl-, 4-Methyl-Naphth-1-yl-, 5-Methyl-2-(Trifluormethyl)-3-Furyl-, 2,3-Dihydrobenzo[B]furan-5-yl-, 1-Benzothiophene-3-yl-, 4-Methyl-3,4-dihydro-2H-1,4-Benzoxazine-7-yl-, 5-Methyl-1-pheny)-1H-pyrazole-4-yl-, 6-Morpholino-3-Pyridinyl-, 4-(1H-Pyrazol-1-yl)-phenyl-, 6-Phenoxy-3-Pyridyl-, 3,4-Dihydro-2H-1,5-benzodioxepine-7-yl-, 5-(1,3-Oxazol-5-yl)-2-thienyl-, 4-(1,3-Oxazol-5-yl)-phenyl-, 5-Methyl-4-isoxazolyl, 2,1,3-Benzothiadiazole-5-yl-, 3-Thienyl-, 2-Methyl-benzyl-, 3-Chloro-benzyl-, 5-Acetamido-naphth-1-yl-, 3-Methyl-8-Quinolinyl-, 4-Chloro-2-nitrophenyl-, 6-Quinolinyl-, 1,3-Benzothiazole-6-yl-, 2-Morpholino-3-Pyridyl-, 2,5-Dimethyl-3-thienyl-, 5-[5-(Chloromethyl)-1,2,4-oxadiazol-3-yl]-2-thienyl-, Ethyl-3-[5-yl-2-thienyl-]1,2,4-oxadiazole-5-carboxylate-, 3-(5-Methyl-1,3,4-oxadiazol-2-yl)-phenyl-, 4-lsopropoxyphenyl-, 2,4-Dibromophenyl-, 3-Cyano-4-fluorophenyl-, 2,5-Bis-(Trifluoromethyl)-phenyl, 2-Bromo-4-fluorophenyl-, 4-Bromo-3-fluorophenyl-, 4-(Difluoromethoxy)-phenyl-, 3-(Difluoromethoxy)-phenyl-, 5-Chloro-2-fluoro-phenyl-, 3-Chloro-2-fluorophenyl-, 2-Fluoro-4-methylphenyl-, 4 Nitro-3-(trifluoromethyl)-phenyl-, 3-Fluoro-4-methylphenyl-, 4-Fluoro-2-methylphenyl-, 4-Bromo-3-(tifluoromethyl)-phenyl-, 4-Bromo-2-(trifluoromethyl)-phenyl-, 3-Bromo-5-(trifluoromethyl)-phenyl-, 2-Bromo-4-(trifluoromethyl)-phenyl-, 2-Bromo-5-(trifluoromethyl)-phenyl-, 2,4-Dichloro-5-fluorophenyl-, 4,5-Dichloro-2-fluorophenyl-, 3,4,5-Trifluorophenyl-, 4-Chloro-2-fluorophenyl-, 2-Bromo-4,6-Difluorophenyl-, 2-Ethylphenyl-, 4-Bromo-2-chlorophenyl-, 4-Bromo-2,6-dichlorophenyl-, 2-Bromo-4,6-dichloro-phenyl-, 4-Bromo-2,6-dimethylphenyl-, 3,5-Dimethylphenyl-, 4-Bromo-3-methylphenyl-, 2-Methoxy-4-nitrophenyl-, 2,2-Dimethyl-6-Chromanyl-, Ethyl-3,5-dimethyl-1H-pyrrole-2-carboxylate-4-yl-, Imidazo[1,2-A]pyridine-3-yl-, 3-(1,3-Oxazol-5-yl)-phenyl-, Ethyl-5-[4-yl)-phenyl]-2-methyl-3-furoate, Methyl-3-(yl)-4-methoxybenzoate, 1-Pyrrolidinylphenylsulfonyl-, Methyl-5-yl-4-methyl-2-thiophene-carboxylate, Methyl-3-yl-4-(isopropylsulfonyl)-2-thiophene, 2-Pyridyl-, 3-Fluoro-4-nitrophenyl-7-Chlorochromone-3-yl-, 4'-Bromobiphenyl-4-yl-, 4'-Acetyl-biphenyl-4-yl-, 4'-Bromo-2'-fluoro-biphenyl-4-yl-, 2-Chloro-4-(3-propyl-Ureido)-phenyl-, 3-(-Bromoacetyl)-phenyl-, 2-Bromo-3-(trifluoromethyl)-phenyl-, 1-Methyl-5-isatinyl-, 4-Isopropyl-benzoic-acid-3-yl-, 2-Chloro-3-thiophenecarboxylic-acid-5-yl-, 3-Pyridyl-, Cyclohexylmethyl-, 2-Methoxy-5-(N-phthalimidinyl)-phenyl-, 1-Benzothiophene-2-yl-, Morpholinophenylsulfonyl-, 3-(2-Methyl-4-pyrimidinyl)-phenyl-, and 2-Cyano-5-methylphenyl-,
and R^{1b}-R^{15b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Furthermore, the use of compounds of general formula (lb) is preferred, wherein R¹⁰ represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably H, a C₁₋₄-alkyl radical, cyclohexyl or a phenyl radical,
more preferably H, CH₃, C₂H₅ or phenyl,
and R^{1b}-R^{9b}, R^{12b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Moreover, the use of compounds of general formula (lb) is preferred, wherein R^{11b} represents hydrogen, an unbranched or branched, saturated or unsaturated, optionally at least mono-substituted C₁₋₆-allphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably H, a C₁₋₄-alkyl radical, cyclohexyl or a phenyl radical, more preferably H, CH₃, C₂H₅ or phenyl,
and R^{1b}-R^{10b}, R^{12b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Preference is also given to the use of compounds of general formula (lb),
wherein R^{12b} represents an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably represents H, a C₁₋₄-alkyl radical, cyclohexyl or a phenyl radical,
more preferably H, CH₃, C₂H₅ or phenyl,
and R^{1b}-R^{11b}, R^{13b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (lb), wherein R^{13b} and R^{14b} are each independently selected from the group consisting of hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical, which may be bonded via an optionally at least mono-substituted C₁₋₆-alkylene group and/or may be condensed with an optionally at least mono-substituted mono- or polycyclic ring-system, or an optionally at least mono-substituted, 5- or 6-membered aryl- or heteroaryl radical, which may be bonded via an optionally at least mono-substituted C₁₋₆-alkylene group and/or may be condensed with an optionally at least mono-substituted mono- or polycyclic ring-system,
preferably are each independently selected from the group consisting of H, a C₁₋₄-alkyl radical, cyclohexyl and a phenyl radical,
more preferably are each independently selected from the group consisting of H, CH₃, C₂H₅ and phenyl,
and R^{1b}-R^{12b}, R^{15b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Furthermore, the use of compounds of general formula (lb) is preferred, wherein R^{13b} and R^{14b} together with the bridging nitrogen atom form a saturated, unsaturated or aromatic, 5- or 6-membered heterocyclic ring, which may be at least mono-substituted and/or contain at least one further heteroatom as a ring member,
preferably form an unsubstituted piperidin or morpholine group,
and R^{1b}-R^{12b} , R^{15b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (lb), wherein R^{15b} represents hydrogen, an unbranched or branched, saturated or unsaturated, optionally at least mono-substituted C₁₋₆-aliphatic radical, a saturated or unsaturated, optionally at least mono-substituted, optionally at least one heteroatom as ring member containing C₃₋₈-cycloaliphatic radical or an optionally at least mono-substituted, 5- or 6- membered aryl- or heteroaryl radical, which may be bonded via an optionally at least mono-substituted C₁₋₆-alkylene group and/or may be condensed with an optionally at least mono-substituted mono- or polycyclic ring-system,
preferably represents H, a C₁₋₄-alkyl radical, cyclohexyl or a phenyl radical,
more preferably represents H, CH₃, C₂H₅ or phenyl,
and R^{1b}-R^{14b} , R^{16b}-R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred is the use of compounds of general formula (lb), wherein R^{16b} represents an unbranched or branched, saturated or unsaturated, optionally at least mono-substituted C₁₋₆ aliphatic radical,
preferably an unbranched or branched, saturated, unsubstituted C₁₋₃ alkyl radical,
more preferably a methyl radical,
and R^{1b}-R^{15b}, R^{17b} , R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (lb), wherein R^{17b} represents an unbranched or branched, saturated or unsaturated, optionally at least mono-substituted C₁₋₆ aliphatic radical,
preferably an unbranched or branched, saturated, unsubstituted C₁₋₃ alkyl radical,
more preferably a methyl radical,
and R^{1b}-R^{16b}, R^{18b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (I), wherein R^{18b} represents a phenyl radical, which is optionally at least mono-substituted by a C₁₋₆ aliphatic radical, more preferably a phenyl radical, which is optionally at least mono-substituted by a methyl group,
and R^{1b}-R^{17b} and W^{b} have the meaning given above, optionally in form of one of their stereoisomers, preferably enantiomers or diastereomers, their racemates or in form of a mixture of at least two of its stereoisomers, preferably enantiomers or diastereomers, in any mixing ratio, or corresponding salts thereof, or corresponding solvates.

Also preferred are compounds of general formula (lb), wherein
R^{1b}, R^{2b}, R^{3b}, R^{4b} are each independently selected from the group consisting of a hydrogen atom; a fluorine atom; a chlorine atom; a bromine atom; a methyl group and a methoxy group;
R^{5b} represents a hydrogen atom;
R^{6b}, R^{7b}, R^{8b}, R^{9b} each represent a hydrogen atom;
W^{b} represents
an alkyl radical selected from the group consisting of methyl; ethyl; n-propyl; iso-propyl; n-butyl; sec.butyl; iso-butyl and tert-butyl; vinyl (CH₂=CH-); -N(CH₃)₂; 1-naphthyl; benzyl; 2-naphtyl; phenyl; 2-methyl-phenyl; 3-methyl-phenyl; 4-methyl-phenyl; 2-ethyl-phenyl; 3-ethyl-phenyl; 4-ethyl-phenyl; 2-n-propyl-phenyl; 3-n-propyl-phenyl; 4-n-propyl-phenyl; 2-isopropyl-phenyl; 3-isopropyl-phenyl; 4-isopropyl-phenyl; 2-n-butyl-phenyl; 3-n-butyl-phenyl; 4-n-butyl-phenyl; 2-iso-butyl-phenyl; 3-iso-butyl-phenyl; 4-iso-butyl-phenyl; 2-tert-butyl-phenyl; 3-tert-butyl-phenyl; 4-tert-butyl-phenyl; 1,1-dimethylpropyl-phenyl; 2-cyclopentyl-phenyl; 3-cyclopentyl-phenyl; 4-cyclopentyl-phenyl 2-cyclohexyl-phenyl; 3-cyclohexyl-phenyl; 4-dyclohexyl-phenyl; 2-methoxy-phenyl; 3-methoxy-phenyl; 4-methoxy-phenyl; 2-ethoxy-phenyl; 3-ethoxy-phenyl; 4-ethoxy-phenyl; 2-n-propoxy-phenyl; 3-n-propoxy-phenyl; 4-n-propoxy-phenyl; 2-iso-propoxy-phenyl; 3-iso-propoxy-phenyl; 4-isopropoxy-phenyl;2-fluoro-phenyl; 3-fluoro-phenyl; 4-fluoro-phenyl; 2-chloro-phenyl; 3-chloro-phenyl; 4-chloro-phenyl; 2-bromo-phenyl; 3-bromo-phenyl; 4-bromo-phenyl; 2-trifluoromethyl-phenyl; 3-trifluoromethyl-phenyl; 4-trifluoromethyl-phenyl; 2-trifluoromethoxy-phenyl; 3-trifluoromethoxy-phenyl; 4-trifluoromethoxy-phenyl; 2-carboxy-phenyl; 3-carboxy-phenyl; 4-carboxy-phenyl; 2-acetyl-phenyl; 3-acetyl-phenyl; 4-acetyl-phenyl; 2-(C=O)-O-CH₃-phenyl; 3-(C=O)-O-CH₃-phenyl; 4-(C=O)-O-CH₃-phenyl; 2-(CH₂)-(CH₂)-(C=O)-O-CH₃; 3-(CH₂)-(CH₂)-(C=O)-O-CH₃; 4-(CH₂)-(CH₂)-(C=OF_{O}-CH₃; 2-cyano-phenyl; 3-cyano-phenyl; 4-cyano-phenyl; 2-nitro-phenyl; 3-nitro-phenyl; 4-nitro-phenyl; 4-(4-bromophenoxy)-phenyl; 2-methylsulfonyl-phenyl; 3-methylsulfonyl-phenyl; 4-methylsulfonyl-phenyl; 2-phenyl-phenyl (biphenyl-2-yl); 3-phenyl-phenyl (biphenyl-3-yl); 4-phenyl-phenyl (biphenyl-4-yl); 2-phenoxy-phenyl; 3-phenoxy-phenyl; 4-phenoxy-phenyl; 2,4-dimethyl-phenyl; 3,4-dimethyl-phenyl; 2,4,6-trimethyl-phenyl; 2,3,5,6-tetramethyl-phenyl; pentamethyl-phenyl; 2,5-dimethoxy-phenyl; 3,4-dimethoxy-phenyl; 2,3-dichlorophenyl; 2,4-dichloro-phenyl; 2,5-dichloro-phenyl; 3,4-dichloro-phenyl; 3,5-dichloro-phenyl; 2,6-dichloro-phenyl; 2,4-difluoro-phenyl; 3,4-difluoro-phenyl; 2,5-difluoro-phenyl; 2,6-difluoro-phenyl; 3-chloro-2-fluoro-phenyl; 3-chloro-4-fluoro-phenyl; 5-chloro-2-fluoro-phenyl; 2,3,4-trichloro-phenyl; 2,4,5-trichloro-phenyl; 2,4,6-trichloro-phenyl; 2,4,5-trifluoro-phenyl; 2,3,4-trifluoro-phenyl-; 2-chloro-4,5-difluoro-phenyl; 2-bromo-4-fluoro-phenyl; 2-bromo-4,6-difluorophenyl; 4-chloro-2,5-difluoro-phenyl; 5-chloro-2,4-difluoro-phenyl; 4-bromo-2,5-difluoro-phenyl; 5-bromo-2,4-difluoro-phenyl; pentafluoro-phenyl; 2,4-dinitro-phenyl; 4-chloro-3-nitro-phenyl; 2-methyl-5-nitro-phenyl; 5-bromo-2-methoxy-phenyl; 3-chloro-2-methyl-phenyl; 4-bromo-3-methyl-phenyl; 4-chloro-2,5-dimethyl-phenyl; 4-fluoro-3-methyl-phenyl; 5-fluoro-2-methyl-phenyl; 2-nitro-4-trifluoromethyl-phenyl; 2-methoxy-4-methyl-phenyl; 3,5-dichloro-2-hydroxyphenyl; 3,5-dichloro-4-hydroxy-phenyl; 5-chloro-2,4-difluoro-phenyl; 3-chloro-4-(NH)-(C=O)-CH3-phenyl; 2-chloro-6-methyl-phenyl; 2-chloro-5-trifluoromethyl-phenyl; 2-chloro-5-trifluoromethoxy-phenyl; 4-bromo-2-trifluoromethoxy-phenyl; 4-bromo-2-trifluoromethyl-phenyl; 4-bromo-3-trifluoromethyl-phenyl; 3-carboxy-4-fluoro-phenyl; 3-carboxy-4-chloro-6-fluoro-phenyl; 4-methoxy-2,3,6-trimethyl-phenyl-; or one of the following groups: 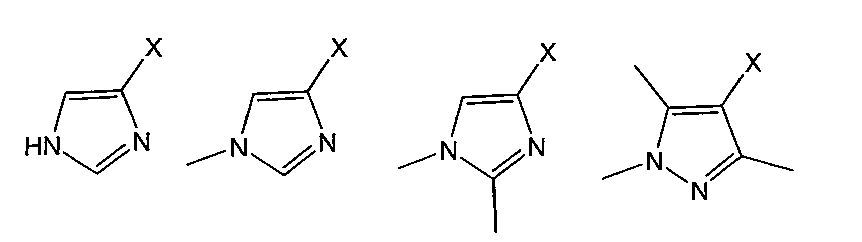 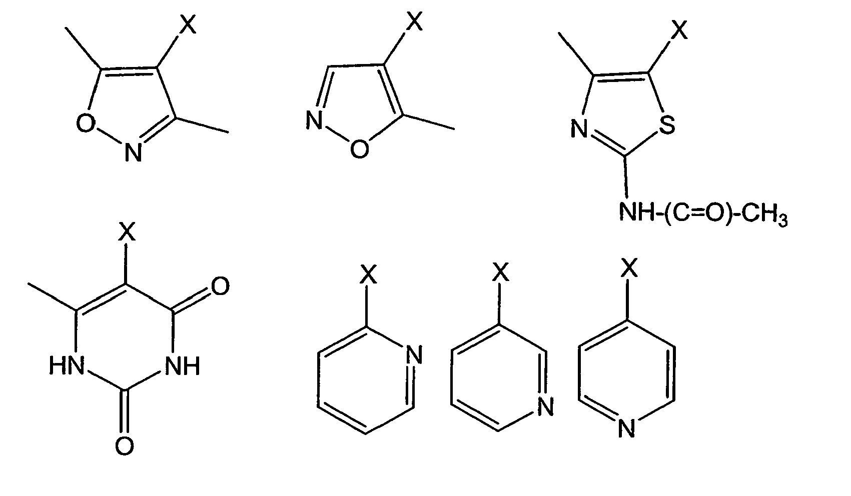 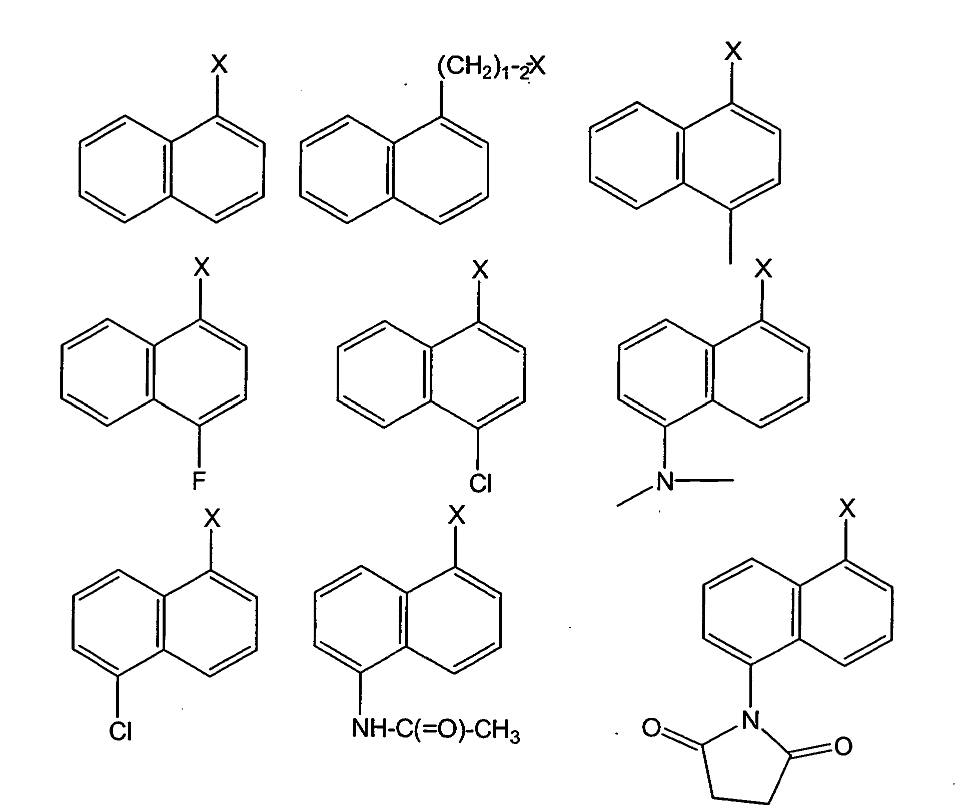 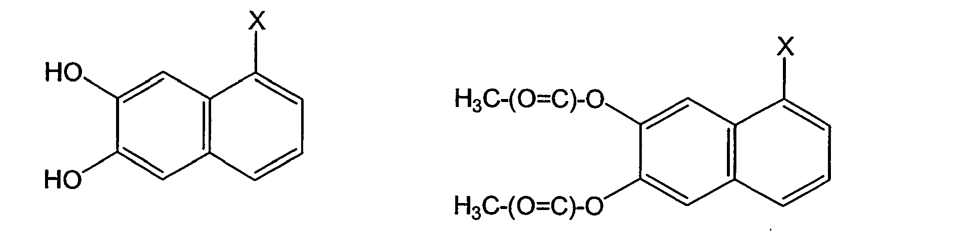 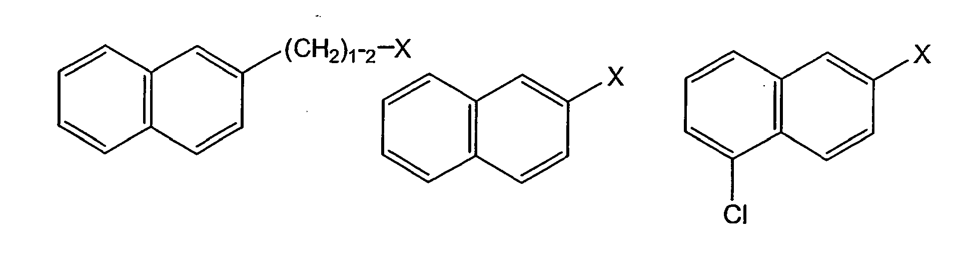 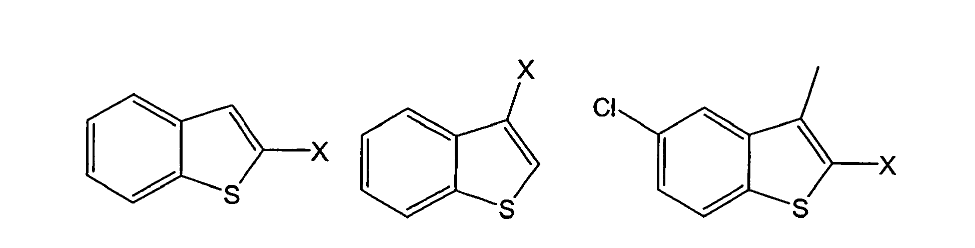 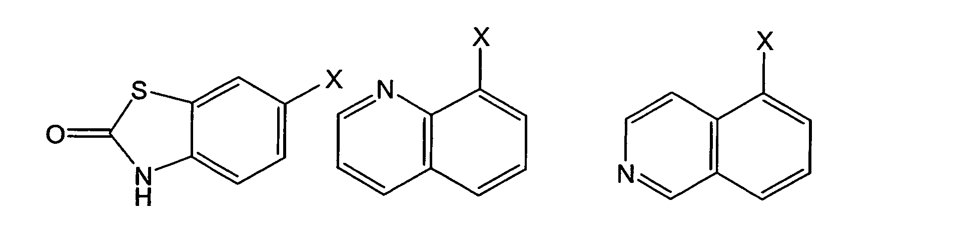 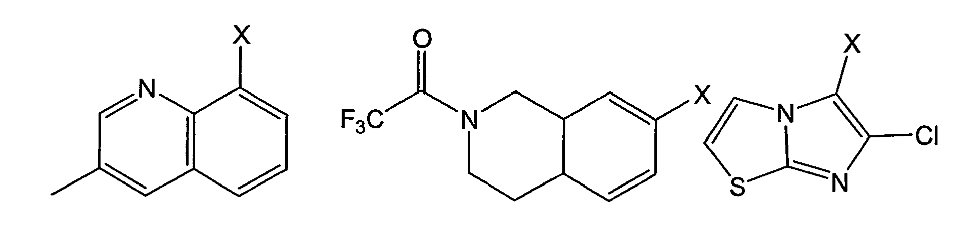 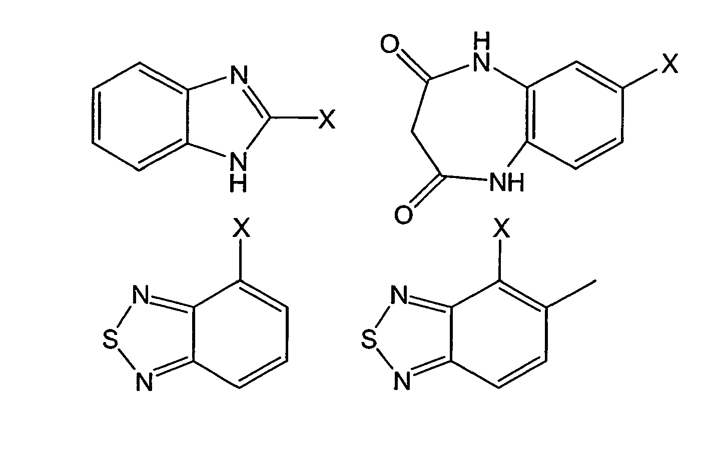 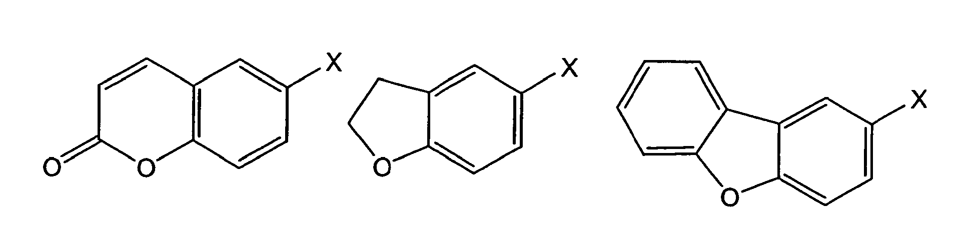 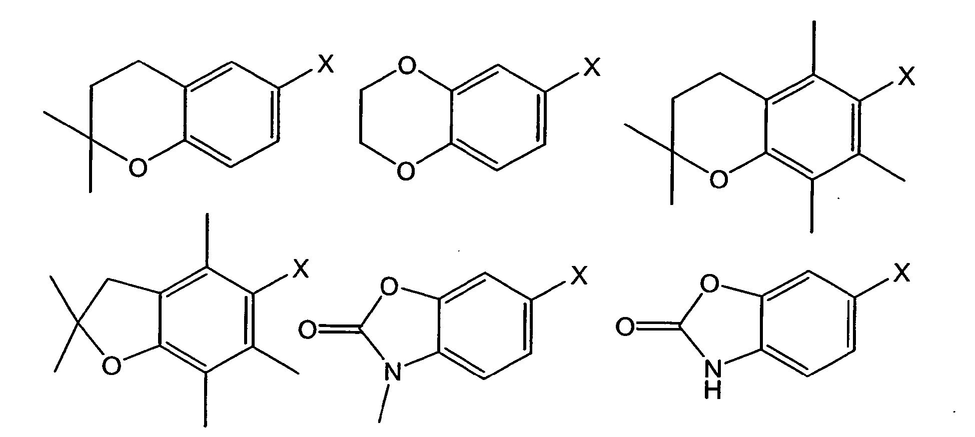 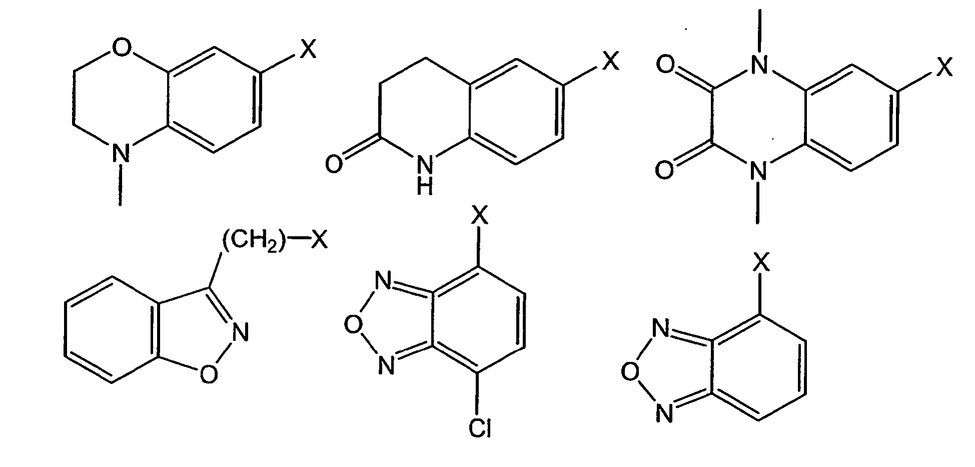 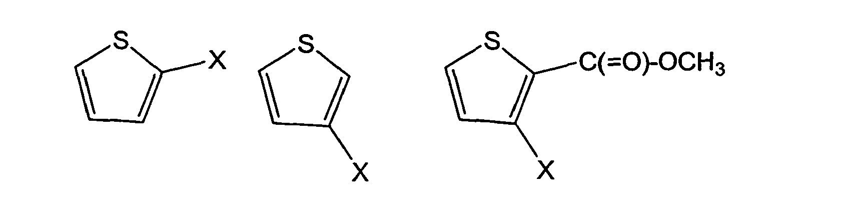 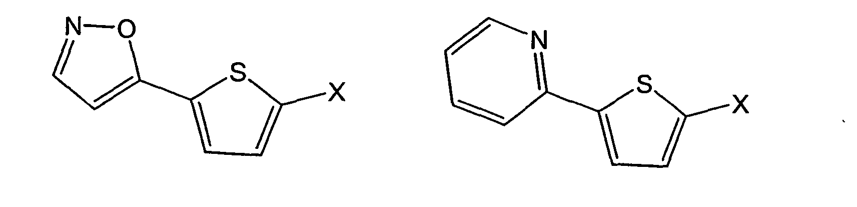 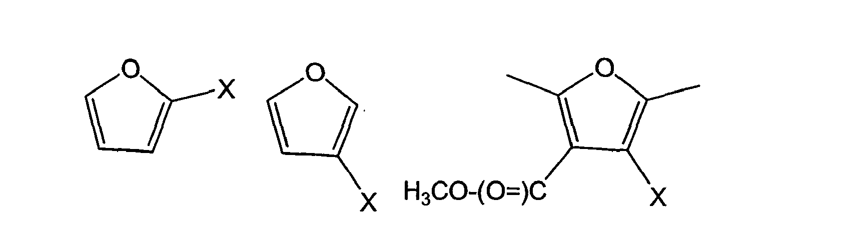 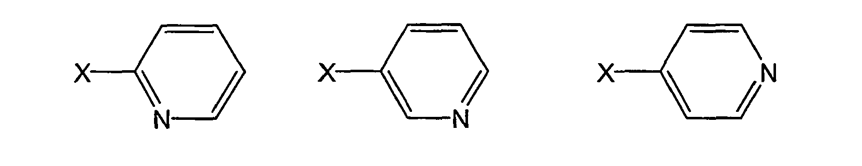 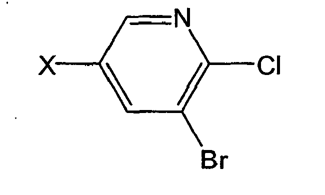 whereby in each case X denotes the position by which the respective substituent W^{b} is bonded to the -SO₂ group of formula (Ib).
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

Particularly preferred is the use of one or more benzoxazinone-derived sulfonamide compounds of general formula (lb) selected from the group consisting of:

| | |
|---|---|
| 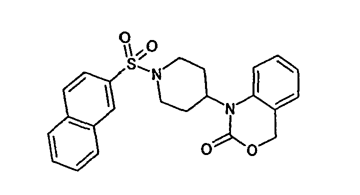 | 1-[1-(Naphthalene-2-sulfonyl)-piperidin-4-yl]-1,4-dihydrobenzo[d][1,3]oxazin-2-one |
| 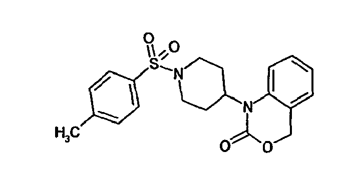 | 1-[1-(Toluene-4-sulfonyl)-piperidin-4-yl]-1,4-dihydrobenzo[d][1,3]oxazin-2-one |
| 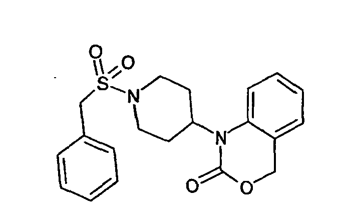 | 1-(1-Phenylmethanesulfonyl-piperidin-4-yl)-1,4-dihydrobenzo[d][1,3]oxazin-2-one |
| 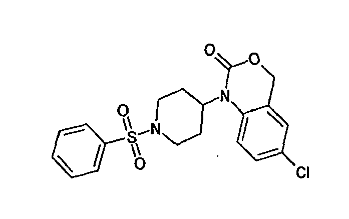 | 1-(1-Benzenesulfonyl-piperidin-4-yl)-6-chloro-1,4-dihydrobenzo[d][1,3]oxazin-2-one |
| 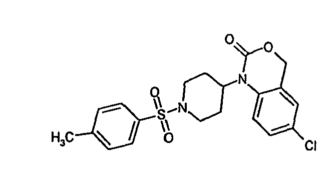 | 6-Chloro-1-[1-(toluene-4-sulfonyl)-piperidin-4-yl]-1,4-dihydrobenzo[d][1,3]oxazin-2-one |
| 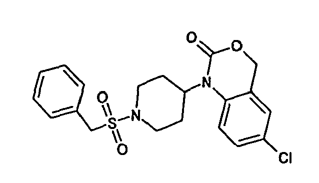 | 6-Chloro-1-(1-phenylmethanesulfonyl-piperidin-4-yl)-1,4-dihydrobenzo[d][1,3]oxazin-2-one |
| 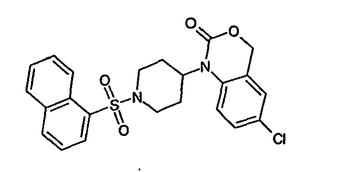 | 6-Chloro-1-[1-(naphthatene-1-sulfonyl)-piperidin-4-yl]-1,4-dihydrobenzo[d][1,3]oxazin-2-one |
| 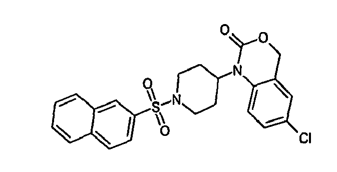 | 6-Chloro-1-[1-(naphthalene-2-sulfonyl)-piperidin-4-yl]-1,4-dihydrobenzo[d][1,3]oxazin-2-one |
| 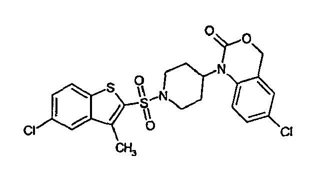 | 6-Chloro-1-[1-(5-chloro-3-methylbenzo[b]thiophene-2-sulfonyl)-piperidin-4-yl]-1,4-dihydrobenzo[d][1,3]oxazin-2-one |
| 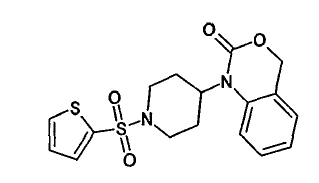 | 6-Chloro-1-[1-(5-chloro-3-methylbenzo[b]thiophene-2-sulfonyl)-piperidin-4-yl]-1,4-dihydrobenzo[d][1,3]oxazin-2-one |
| 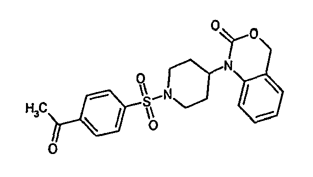 | 1-[1-(4-Acetyl-benzenesulfonyl)-piperidin-4-yl]-1,4-dihydrobenzo[d][1,3]oxazin-2-one |
| 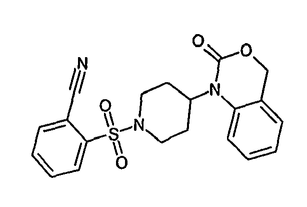 | 2-[4-(2-Oxo-4H-benzo[d][1,3]oxazin-1-yl)-piperidine-1-sulfonyl]-benzonitrile |
| 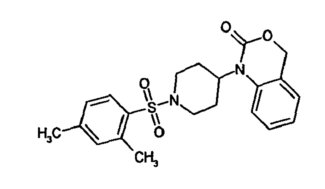 | 1-[1-(2,4-Dimethyl-benzenesulfonyl)-piperidin-4-yl]-1,4-dihydrobenzo[d][1,3]oxazin-2-one |
| 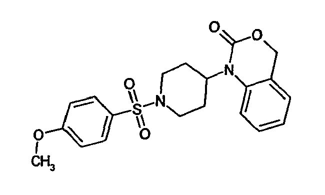 | 1-[1-(4-Methoxy-benzenesulfonyl)-piperidin-4-yl]-1,4-dihydrobenzo[d][1,3]oxazin-2-one |
| 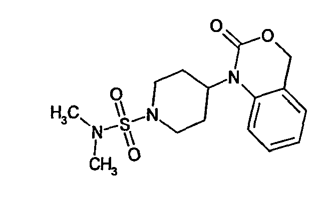 | 4-(2-Oxo-4H-benzo[d][1,3]oxazin-1-yl)-piperidine-1-sulfonic acid dimethylamide |
| 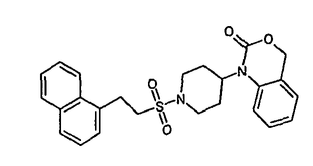 | 1-[1-(2-Naphthalen-1-yl-ethanesulfonyl)-piperidin-4-yl]-1,4-dihydro-benzo[d][1,3]oxazin-2-one |
| 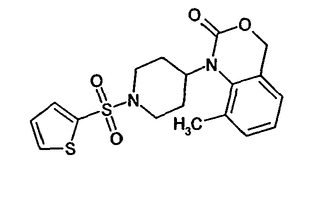 | 8-Methyl-1-[1-(thiophene-2-sulfonyl)-piperidin-4-yl]-1,4-dihydrobenzo[d][1,3]oxazin-2-one |
| 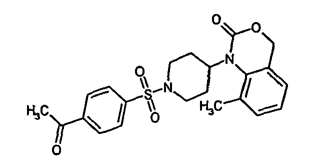 | 1-[1-(4-Acetyl-benzenesulfonyl)-piperidin-4-yl]-8-methyl-1,4-dihydrobenzo[d][1,3]oxazin-2-one |
| 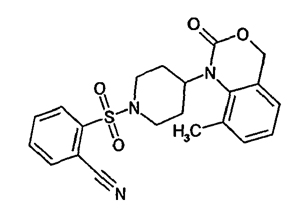 | 2-[4-(8-Methyl-2-oxo-4H-benzo[d][1,3]oxazin-1-yl)-piperidine-1-sulfonyl]-benzonitrile |
| 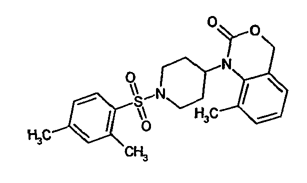 | 1-[1-(2,4-Dimethyl-benzenesulfonyl)-piperidin-4-yl]-8-methyl-1,4-dihydrobenzo[d][1,3]oxazin-2-one |
| 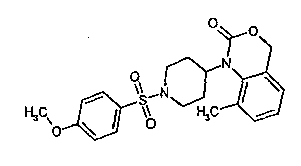 | 1-[1-(4-Methoxy-benzenesulfonyl)-piperidin-4-yl]-8-methyl-1,4-dihydrobenzo[d][1,3]oxazin-2-one |
| 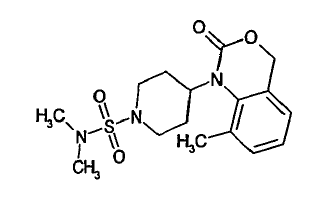 | 4-(8-Methyl-2-oxo-4H-benzo[d][1,3]oxazin-1-yl)-piperidine-1-sulfonic acid dimethylamide |
| 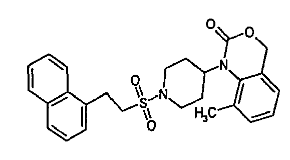 | 8-Methyl-1-[1-(2-naphthalen-1-yl-ethanesulfonyl)-piperidin-4-yl]-1,4-dihydro-benzo[d][1,3]oxazin-2-one |
| 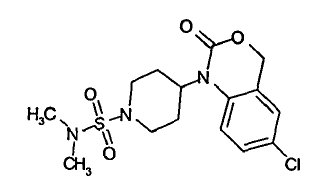 | 4-(6-Chloro-2-oxo-4H-benzo[d][1,3]oxazin-1-yl)-piperidine-1-sulfonic acid dimethylamide |
| 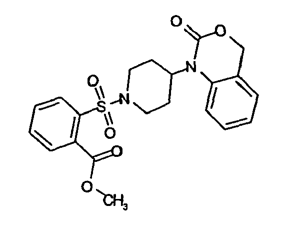 | 2-[4-(2-Oxo-4H-benzo[d][1,3]oxazin-1-yl)-piperidine-1-sulfonyl]-benzoic acid methyl ester |
| 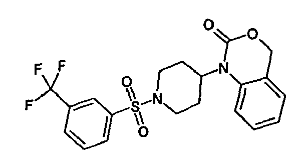 | 1-[1-(3-Trifluoromethylbenzenesulfonyl)-piperidin-4-yl]-1,4-dihydro-benzo[d][1,3]oxazin-2-one |
| 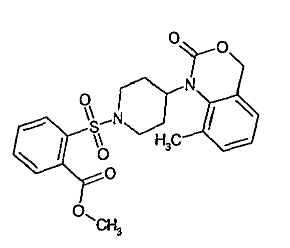 | 2-[4-(8-Methyl-2-oxo-4H-benzo[d][1,3]oxazin-1-yl)-piperidine-1-sulfonyl]-benzoic acid methyl ester |
| 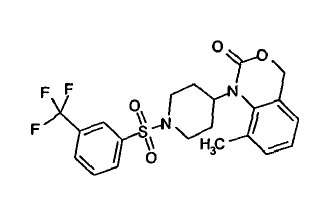 | 8-Methyl-1-[1-(3-trifluoromethylbenzenesulfonyl)-piperidin-4-yl]-1,4-dihydro-benzo[d][1,3]oxazin-2-one |
| 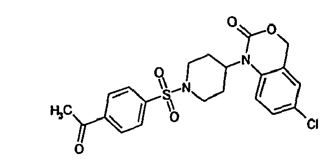 | 1-[1-(4-Acetyl-benzenesulfonyl)-piperidin-4-yl]-6-chloro-1,4-dihydrobenzo[d][1,3]oxazin-2-one |
| 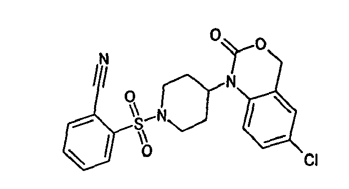 | 2-[4-(6-Chloro-2-oxo-4H-benzo[d][1,3]oxazin-1-yl)-pipendine-1-sulfonyl]-benzonitrile |
| 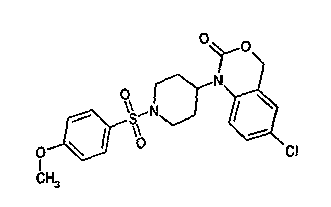 | 6-Chloro-1-[1-(4-methoxybenzenesulfonyl)-piperidin-4-yl]-1,4-dihydro-benzo[d][1,3]oxazin-2-one |
| 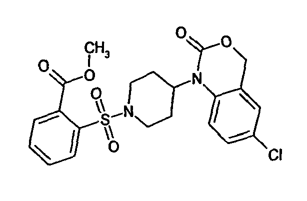 | 2-[4-(6-Chloro-2-oxo-4H-benzo[d][1,3]oxazin-1-yl)-piperidine-1-sulfonyl]-benzoic acid methyl ester |
| 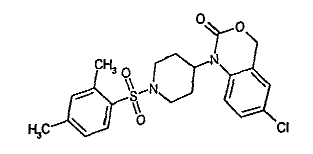 | 6-Chloro-1-[1-(2,4-dimethylbenzenesulfonyl)-piperidin-4-yl]-1,4-dihydro-benzo[d][1,3]oxazin-2-one |
| 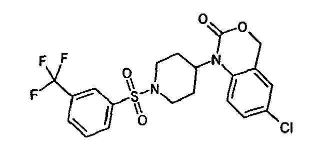 | 6-Chloro-1-[1-(3-trifluoromethylbenzenesulfonyl)-piperidin-4-yl]-1,4-dihydro-benzo[d][1,3]oxazin-2-one |
| 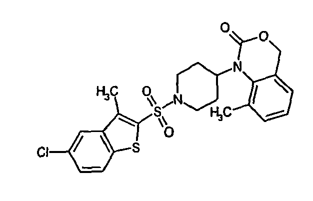 | 1-[1-(5-Chloro-3-methylbenzo[b]thiophene-2-sulfonyl)-piperidin-4-yl]-8-methyl-1,4-dihydrobenzo[d][1,3]oxazin-2-one |
| 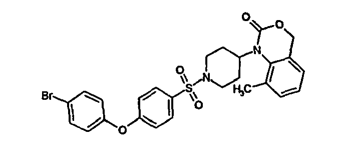 | 1-{1-[4-(4-Bromo-phenoxy)-benzenesulfonyl]-piperidin-4-yl}-8-methyl-1,4-dihydrobenzo[d][1,3]oxazin-2-one |
| 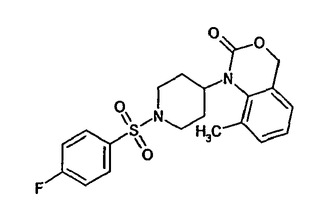 | 1-[1-(4-Fluoro-benzenesulfonyl)-piperidin-4-yl]-8-methyl-1,4-dihydrobenzo[d][1,3]oxazin-2-one |
| 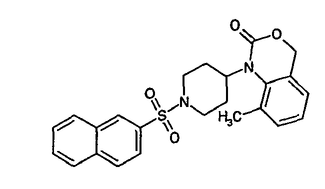 | 8-Methyl-1-[1-(naphthalene-2-sulfonyl)-piperidin-4-yl]-1,4-dihydrobenzo[d][1,3]oxazin-2-one |
| 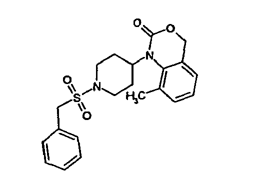 | 8-Methyl-1-(1-phenylmethanesulfonylpiperidin-4-yl)-1,4-dihydrobenzo[d][1,3]oxazin-2-one |
| 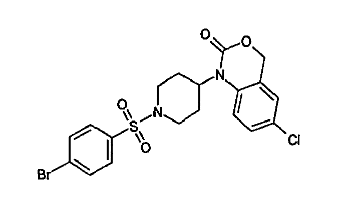 | 1-[1-(4-Bromo-benzenesulfonyl)-piperidin-4-yl]-6-chloro-1,4-dihydrobenzo[d][1,3]oxazin-2-one |
| 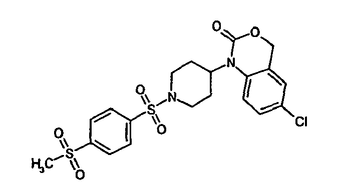 | 6-Chloro-1-[1-(4-methanesulfonylbenzenesulfonyl)-piperidin-4-yl]-1,4-dihydro-benzo[d][1,3]oxazin-2-one |
| 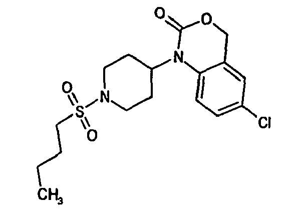 | 1-[1-(Butane-1-sulfonyl)-piperidin-4-yl]-6-chloro-1,4-dihydrobenzo[d][1,3]oxazin-2-one |
| 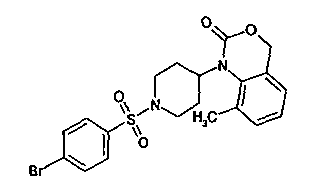 | 1-[1-(4-Bromo-benzenesulfonyl)-piperidin-4-yl]-8-methyl-1,4-dihydrobenzo[d][1,3]oxazin-2-one |
| 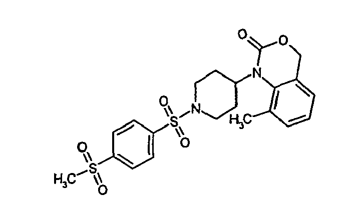 | 1-[1-(4-Methanesulfonylbenzenesulfonyl)-piperidin-4-yl]-8-methyl-1,4-dihydrobenzo[d][1,3]oxazin-2-one |
| 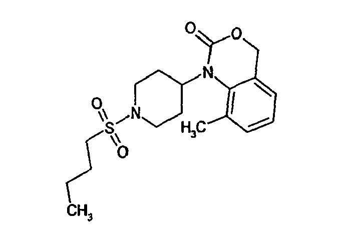 | 1-[1-(Butane-1-sulfonyl)-piperidin-4-yl]-8-methyl-1,4-dihydrobenzo[d][1,3]oxazin-2-one |
| 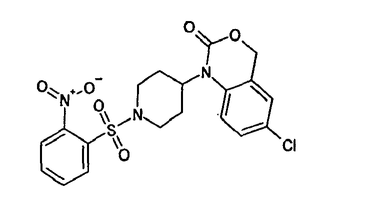 | 6-Chloro-1-[1-(2-nitrobenzenesulfonyl)-piperidin-4-yl]-1,4-dihydro-benzo[d][1,3]oxazin-2-one |
| 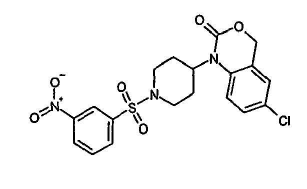 | 6-Chloro-1-[1-(3-nitrobenzenesulfonyl)-piperidin-4-yl]-1,4-dihydro-benzo[d][1,3]oxazin-2-one |
| 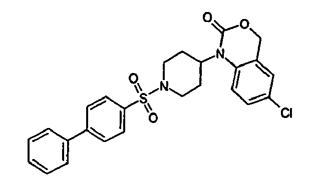 | 1-[1-(Biphenyl-4-sulfonyl)-piperidin-4-yl]-6-chloro-1,4-dihydrobenzo[d][1,3]oxazin-2-one |
| 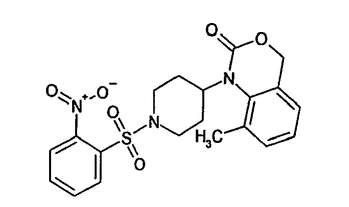 | 8-Methyl-1-[1-(2-nitrobenzenesulfonyl)-piperid in-4-yl]-1 ,4-dihydro-benzo[d][1,3]oxazin-2-one |
| 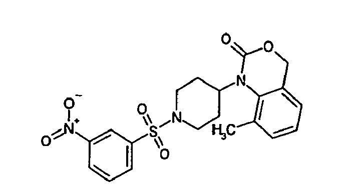 | 8-Methyl-1-[1-(3-nitrobenzenesulfonylrpiperidin-4-yl]-1,4-dihydro-benzo[d][1,3]oxazin-2-one |
| 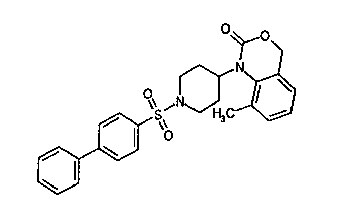 | 1-[1-(Biphenyl-4-sulfonyl)-piperidin-4-yl]-8-methyl-1,4-dihydrobenzo[d][1,3]oxazin-2-one |
| 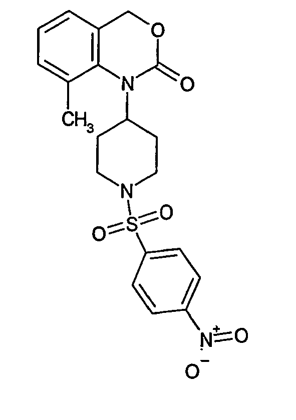 | 8-Methyl-1-[1-(4-nitrobenzenesulfonyl)-piperidin-4-yl]-1,4-dihydro-benzo[d][1,3]oxazin-2-one |
| 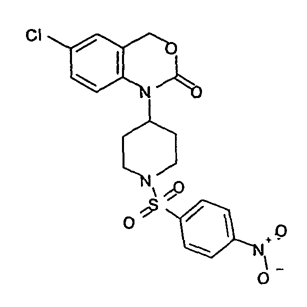 | 6-Chloro-1-[1-(4-nitrobenzenesulfonyl)-piperidin-4-yl]-1,4-dihydro-benzo[d][1,3]oxazin-2-one |
| 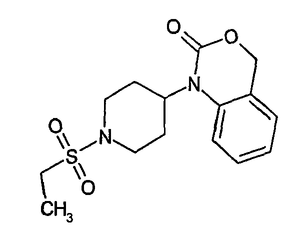 | 1-(1-Ethanesulfonyl-piperidin-4-yl)-1,4-dihydro-benzo[d][1,3]oxazin-2-one |
| 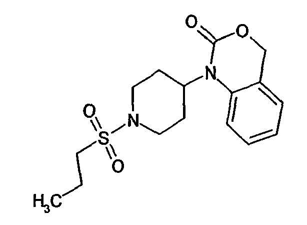 | 1-[1-(Propane-1-sulfonyl)-piperidin-4-yl]-1,4-dihydro-benzo[d][1,3]oxazin-2-one |
| 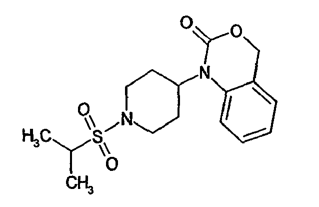 | 1-[1-(Propane-2-sulfonyl)-piperidin-4-yl]-1,4-dihydro-benzo[d][1,3]oxazin-2-one |
| 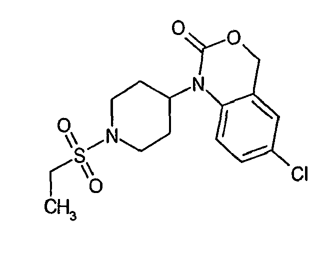 | 6-Chloro-1-(1-ethanesulfonyl-piperidin-4-yl)-1,4-dihydro-benzo[d][1,3]oxazin-2-one |
| 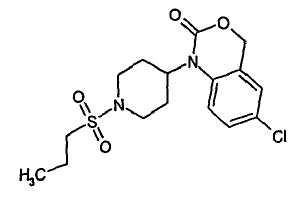 | 6-Chloro-1-[1-(propane-1-sulfonyl)-piperidin-4-yl]-1,4-dihydrobenzo[d][1,3]oxazin-2-one |
| 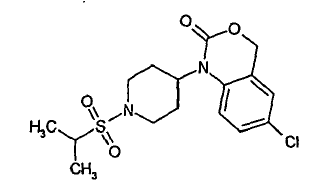 | 6-Chloro-1-[1-(propane-2-sulfonyl)-piperidin-4-yl]-1,4-dihydrobenzo[d][1,3]oxazin-2-one |
| 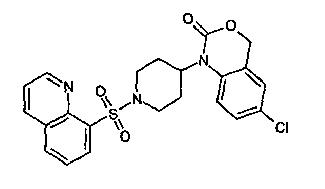 | 6-Chloro-1-[1-(quinoline-8-sulfonyl)-piperidin-4-yl]-1,4-dihydrobenzo[d][1,3]oxazin-2-one |
| 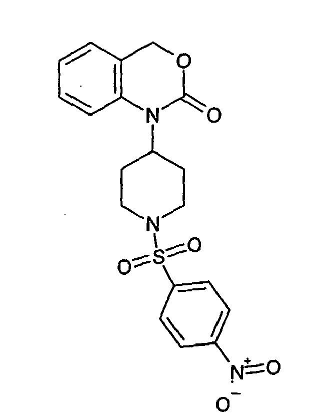 | 1-[1-(4-Nitro-benzenesulfonyl)-piperidin-4-yl]-1,4-dihydrobenzo[d][1,3]oxazin-2-one |
| 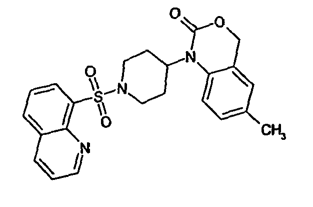 | 6-Methyl-1-[1-(quinoline-8-sulfonyl)-piperidin-4-yl]-1,4-dihydrobenzo[d][1,3]oxazin-2-one |
| 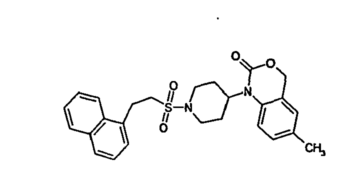 | 6-Methyl-1-[1-(2-naphthalen-1-yl-ethanesulfonyl)-piperidin-4-yl]-1,4-dihydro-benzo[d][1,3]oxazin-2-one |
| 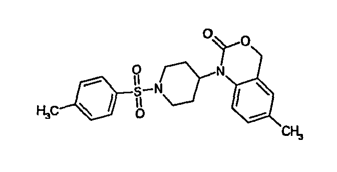 | 6-Methyl-1-[1-(toluene-4-sulfonyl)-piperidin-4-yl]-1,4-dihydrobenzo[d][1,3]oxazin-2-one |
| 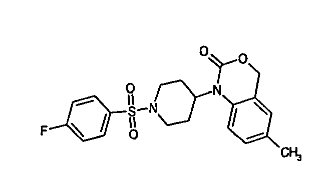 | 1-[1-(4-Fluoro-benzenesulfonyl)-piperidin-4-yl]-6-methyl-1,4-dihydrobenzo[d][1,3]oxazin-2-one |
| 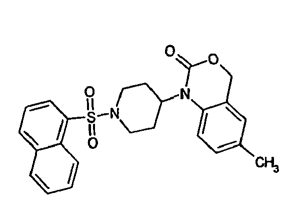 | 6-Methyl-1-[1-(naphthalene-1-sulfonyl)-piperidin-4-yl]-1,4-dihydrobenzo[d][1,3]oxazin-2-one |
| 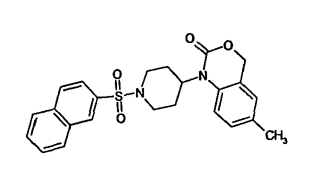 | 6-Methyl-1-[1-(naphthalene-2-sulfonyl)-piperidin-4-yl]-1,4-dihydrobenzo[d][1,3]oxazin-2-one |
| 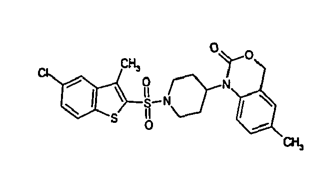 | 1-[1-(5-Chloro-3-methylbenzo[b]thiophene-2-sulfonyl)-piperidin-4-yl]-6-methyl-1,4-dihydrobenzo[d][1,3]oxazin-2-one |
| 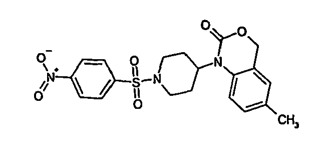 | 6-Methyl-1-[1-(4-nitrobenzenesulfonyl)-piperidin-4-yl]-1,4-dihydro-benzo[d][1,3]oxazin-2-one |
| 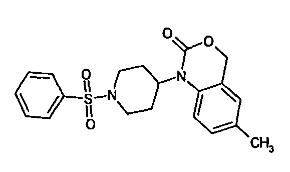 | 1-(1-Benzenesulfonyl-piperidin-4-yl)-6-methyl-1,4-dihydrobenzo[d][1,3]oxazin-2-one |
| 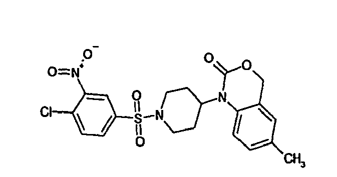 | 1-[1-(4-Chloro-3-nitrobenzenesulfonyl)-piperidin-4-yl]-6-methyl-1,4-dihydrobenzo[d][1,3]oxazin-2-one |
| 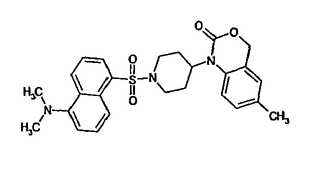 | 1-[1-(5-Dimethylamino-naphthalene-1-sulfonyl)-piperidin-4-yl]-6-methyl-1,4-dihydro-benzo[d][1,3]oxazin-2-one |
| 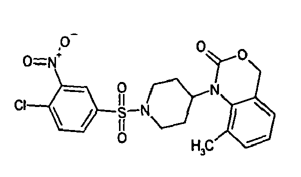 | 1-[1-(4-Chloro-3-nitrobenzenesulfonyl)-piperidin-4-yl]-8-methyl-1,4-dihydrobenzo[d][1,3]oxazin-2-one |
| 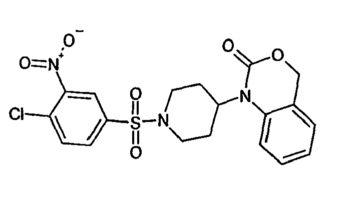 | 1-[1-(4-Chloro-3-nitrobenzenesulfonyl)-piperidin-4-yl]-1,4-dihydro-benzo[d][1,3]oxazin-2-one |
| 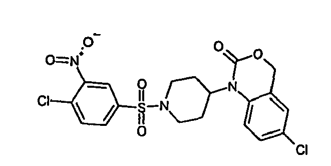 | 6-Chloro-1-[1-(4-chloro-3-nitrobenzenesulfonyl)-piperidin-4-yl]-1,4-dihydro-benzo[d][1,3]oxazin-2-one |
| 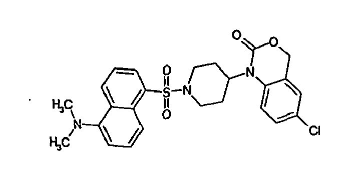 | 6-Chloro-1-[1-(5-dimethylaminonaphthalene-1-sulfonyl)-piperidin-4-yl]-1,4-dihydro-benzo[d][1,3]oxazin-2-one |
| 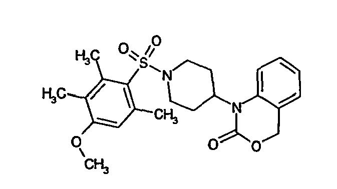 | 1-[1-(4-Methoxy-2,3,6-trimethylbenzenesulfonyl)-piperidin-4-yl]-1,4-dihydro-benzo[d][1,3]oxazin-2-one |
| 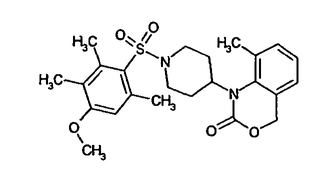 | 1-[1-(4-Methoxy-2,3,6-trimethylbenzenesulfonyl)-piperidin-4-yl]-8-methyl-1,4-dihydrobenzo[d][1,3]oxazin-2-one |
| 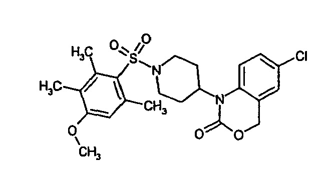 | 6-Chloro-1-[1-(4-methoxy-2,3,6-trimethyl-benzenesulfonyl)-piperidin-4-yl]-1,4-dihydro-benzo[d][1,3]oxazin-2-one |
| 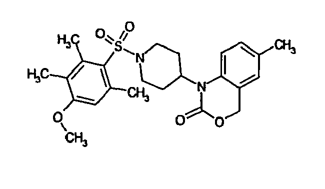 | 1-[1-(4-Methoxy-2,3,6-trimethylbenzenesulfonyl)-piperidin-4-yl]-6-methyl-1,4-dihydrobenzo[d][1,3]oxazin-2-one |
| 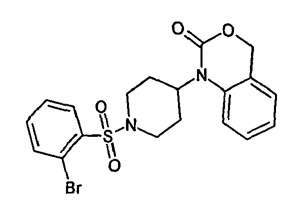 | 1-[1-(2-Bromo-benzenesulfonyl)-piperidin-4-yl]-1,4-dihydrobenzo[d][1,3]oxazin-2-one |
| 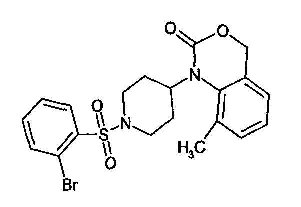 | 1-[1-(2-Bromo-benzenesulfonyl)-piperidin-4-yl]-8-methyl-1,4-dihydrobenzo[d][1,3]oxazin-2-one |
| 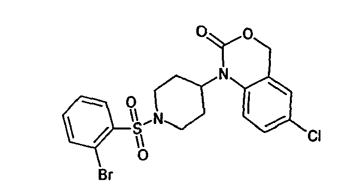 | 1-[1-(2-Bromo-benzenesulfonyl)-piperidin-4-yl]-6-chloro-1,4-dihydrobenzo[d][1,3]oxazin-2-one |
| 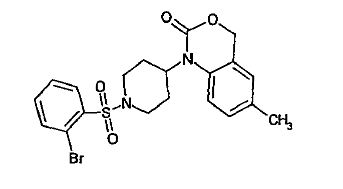 | 1-[1-(2-Bromo-benzenesulfonyl)-piperidin-4-yl]-6-methyl-1,4-dihydrobenzo[d][1,3]oxazin-2-one |
| 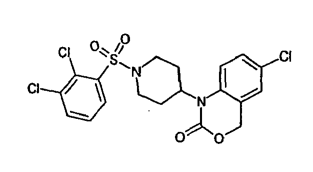 | 6-Chloro-1-[1-(2,3-dichlorobenzenesulfonyl)-piperidin-4-yl]-1,4-dihydro-benzo[d][1,3]oxazin-2-one |
| 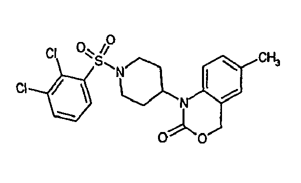 | 1-[1-(2,3-Dichloro-benzenesulfonyl)-piperidin-4-yl]-6-methyl-1,4-dihydrobenzo[d][1,3]oxazin-2-one |
| 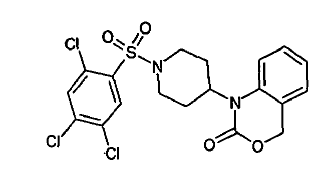 | 1-[1-(2,4,5-Trichloro-benzenesulfonyl)-piperidin-4-yl]-1,4-dihydrobenzo[d][1,3]oxazin-2-one |
| 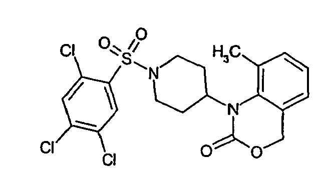 | 8-Methyl-1-[1-(2,4,5-trichlorobenzenesulfonylrpiperidin-4-yl]-1,4-dihydro-benzo[d][1,3]oxazin-2-one |
| 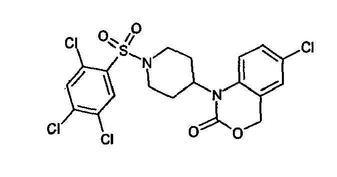 | 6-Chloro-1-[1-(2,4,5-trichlorobenzenesulfonyl)-piperidin-4-yl]-1,4-dihydro-benzo[d][1,3]oxazin-2-one |
| 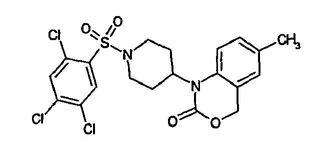 | 6-Methyl-1-[1-(2,4,5-trichlorobenzenesulfonyl)-piperidin-4-yl]-1,4-dihydro-benzo[d][1,3]oxazin-2-one |
| 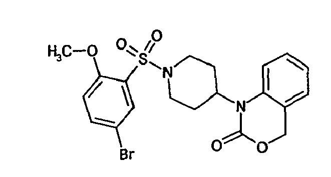 | 1-[1-(5-Bromo-2-methoxybenzenesulfonyl)-piperidin-4-yl]-1,4-dihydro-benzo[d][1,3]oxazin-2-one |
| 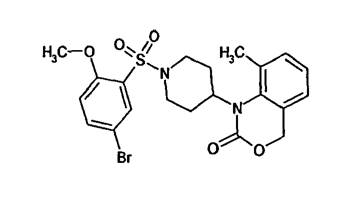 | 1-[1-(5-Bromo-2-methoxybenzenesulfonyl)-piperidin-4-yl]-8-methyl-1,4-dihydrobenzo[d][1,3]oxazin-2-one |
| 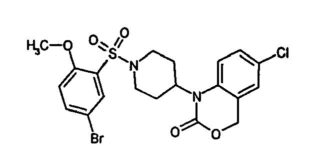 | 1-[1-(5-Bromo-2-methoxybenzenesulfonyl)-piperidin-4-yl]-6-chloro-1,4-dihydro-benzo[d][1,3]oxazin-2-one |
| 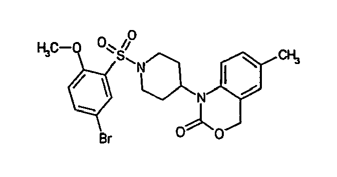 | 1-[1-(5-Bromo-2-methoxybenzenesulfonyl)-piperidin-4-yl]-6-methyl-1,4-dihydrobenzo[d][1,3]oxazin-2-one |
| 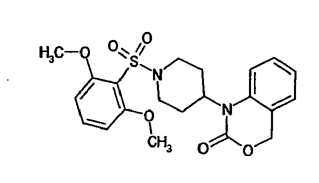 | 1-[1-(2,6-Dimethoxy-benzenesulfonyl)-piperidin-4-yl]-1,4-dihydrobenzo[d][1,3]oxazin-2-one |
| 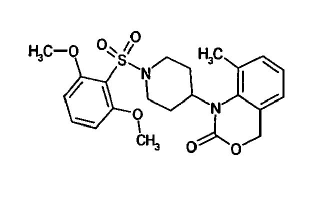 | 1-[1-(2,6-Dimethoxy-benzenesulfonyl)-piperidin-4-yl]-8-methyl-1,4-dihydrobenzo[d][1,3]oxazin-2-one |
| 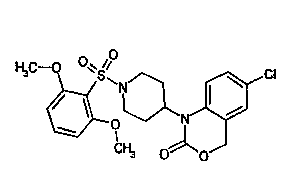 | 6-Chloro-1-[1-(2,6-dimethoxybenzenesulfonyl)-piperidin-4-yl]-1,4-dihydro-benzo[d][1,3]oxazin-2-one |
| 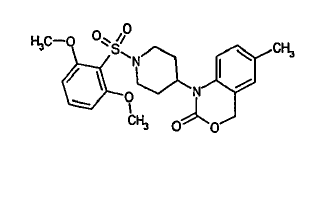 | 1-[1-(2,6-Dimethoxy-benzenesulfonyl)-piperidin-4-yl]-6-methyl-1,4-dihydrobenzo[d][1,3]oxazin-2-one |
| 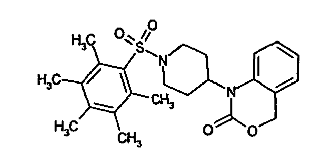 | 1-(1-Pentamethylbenzenesulfonylpiperidin-4-yl)-1,4-dihydrobenzo[d][1,3]oxazin-2-one |
| 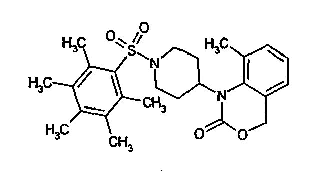 | 8-Methyl-1-(1-pentamethylbenzenesulfonyl-piperidin-4-yl)-1,4-dihydro-benzo[d][1,3]oxazin-2-one |
| 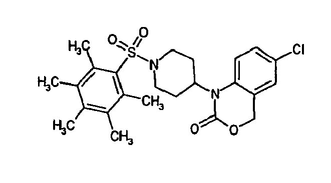 | 6-Chloro-1-(1-pentamethylbenzenesulfonyl-piperidin-4-yl)-1,4-dihydro-benzo[d][1,3]oxazin-2-one |
| 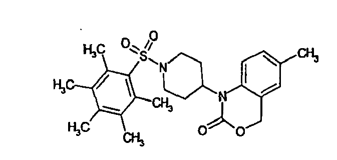 | 6-Methyl-1-(1-pentamethylbenzenesulfonyl-piperidin-4-yl)-1,4-dihydro-benzo[d][1,3]oxazin-2-one |
| 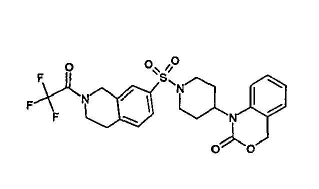 | 1-{1-[2-(2,2,2-Trifluoro-acetyl)-1,2,3,4-tetrahydro-isoquinoline-7-sulfonyl]-piperidin-4-yl}-1,4-dihydrobenzo[d][1,3]oxazin-2-one |
| 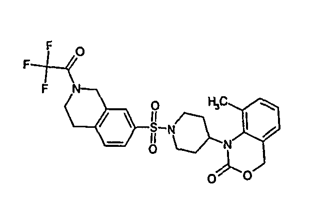 | 8-Methyl-1-{-[2-(2,2,2-trifluoro-acetyl)-1,2,3,4-tetrahydro-isoquinoline-7-sulfonyl]-piperidin-4-yl}-1,4-dihydrobenzo[d][1,3]oxazin-2-one |
| 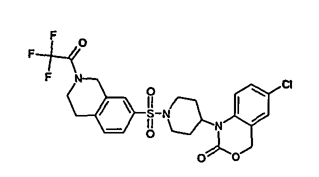 | 6-Chloro-1-{1-[2-(2,2,2-trifluoro-acetyl)-1,2,3,4-tetrahydro-isoquinoline-7-sulfonyl]-piperidin-4-yl}-1,4-dihydrobenzo[d][1,3]oxazin-2-one |
| 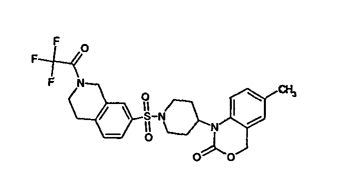 | 6-Methyl-1-{1-[2-(2,2,2-trifluoro-acetyl)-1,2,3,4-tetrahydro-isoquinoline-7-sulfonyl]-piperidin-4-yl}-1,4-dihydrobenzo[d][1,3]oxazin-2-one |
| 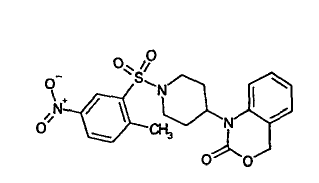 | 1-[1-(2-Methyl-5-nitrobenzenesulfonyl)-piperidin-4-yl]-1,4-dihydro-benzo[d][1,3]oxazin-2-one |
| 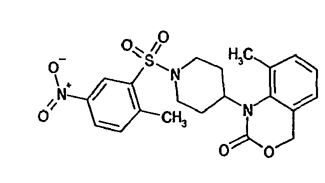 | 8-Methyl-1-[1-(2-methyl-5-nitrobenzenesulfonyl)-piperidin-4-yl]-1,4-dihydro-benzo[d][1,3]oxazin-2-one |
| 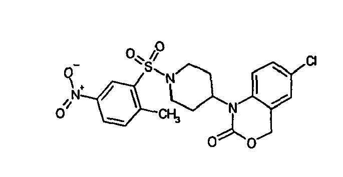 | 6-Chloro-1-[1-(2-methyl-5-nitrobenzenesulfonyl)-piperidin-4-yl]-1,4-dihydro-benzo[d][1,3]oxazin-2-one |
| 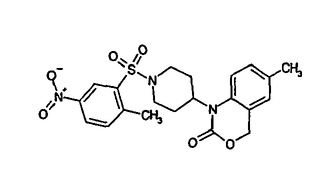 | 6-Methyl-1-[1-(2-methyl-5-nitrobenzenesulfonyl)-piperidin-4-yl]-1,4-dihydro-benzo[d][1,3]oxazin-2-one |
| 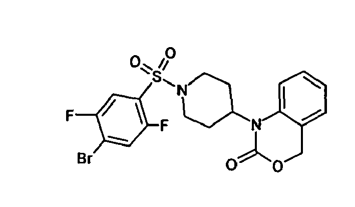 | 1-[1-(4-Bromo-2,5-difluorobenzenesulfonyl)-piperidin-4-yl]-1,4-dihydro-benzo[d][1,3]oxazin-2-one |
| 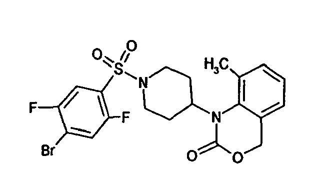 | 1-[1-(4-Bromo-2,5-difluorobenzenesulfonyl)-piperidin-4-yl]-8-methyl-1,4-dihydrobenzo[d][1,3]oxazin-2-one |
| 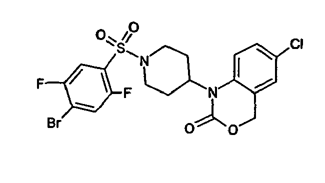 | 1-[1-(4-Bromo-2,5-difluorobenzenesulfonyl)-piperidin-4-yl]-6-chloro-1,4-dihydro-benzo[d][1,3]oxazin-2-one |
| 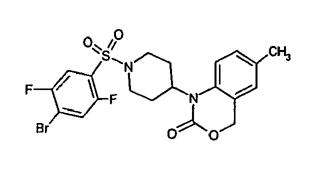 | 1-[1-(4-Bromo-2,5-difluorobenzenesulfonyl)-piperidin-4-yl]-6-methyl-1,4-dihydrobenzo[d][1,3]oxazin-2-one |
| 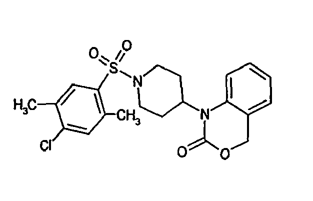 | 1-[1-(4-Chloro-2,5-dimethylbenzenesulfonyl)-piperidin-4-yl]-1,4-dihydro-benzo[d][1,3]oxazin-2-one |
| 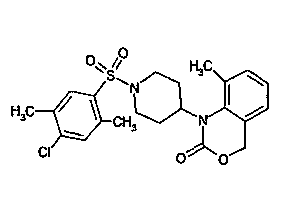 | 1-[1-(4-Chloro-2,5-dimethylbenzenesulfonyl)-piperidin-4-yl]-8-methyl-1,4-dihydrobenzo[d][1,3]oxazin-2-one |
| 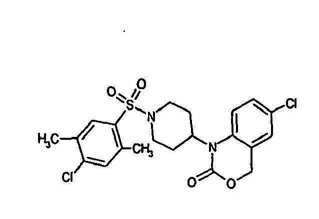 | 6-Chloro-1-[1-(4-chloro-2,5-dimethylbenzenesulfonyl)-piperidin-4-yl]-1,4-dihydro-benzo[d][1,3]oxazin-2-one |
| 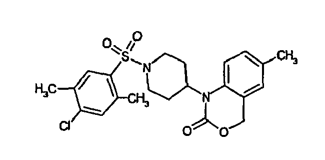 | 1-[1-(4-Chloro-2,5-dimethy)-benzenesulfonyl)-piperidin-4-yl]-6-methyl-1,4-dihydrobenzo[d][1,3]oxazin-2-one |
| 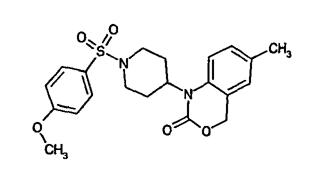 | 1-[1-(4-Methoxy-benzenesulfonyl)-piperidin-4-yl]-6-methyl-1,4-dihydrobenzo[d][1,3]oxazin-2-one |
| 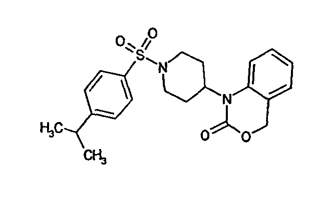 | 1-[1-(4-Isopropyl-benzenesutfonyl)-piperidin-4-yl]-1,4-dihydrobenzo[d][1,3]oxazin-2-one |
| 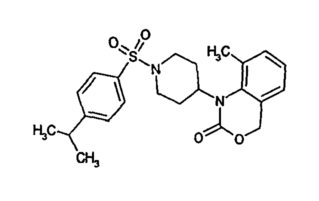 | 1-[1-(4-Isopropyl-benzenesulfonyl)-piperidin-4-yl]-8-methyl-1,4-dihydrobenzo[d][1,3]oxazin-2-one |
| 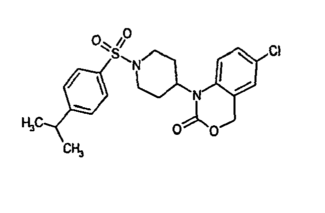 | 6-Chloro-1-[1-(4-isopropylbenzenesulfonyl)-piperidin-4-yl]-1,4-dihydro-benzo[d][1,3]oxazin-2-one |
| 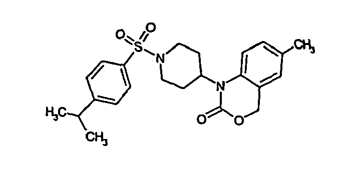 | 1-[1-(4-Isopropyl-benzenesulfonyl)-piperidin-4-yl]-6-methyl-1,4-dihydrobenzo[d][1,3]oxazin-2-one |
| 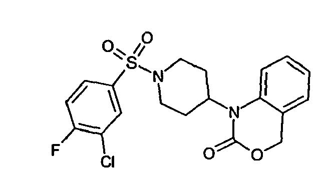 | 1-[1-(3-Chloro-4-fluorobenzenesulfonyl)-piperidin-4-yl]-1,4-dihydro-benzo[d][1,3]oxazin-2-one |
| 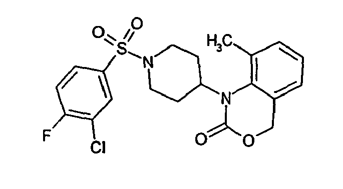 | 1-[1-(3-Chloro-4-fluorobenzenesulfonyl)-piperidin-4-yl]-8-methyl-1,4-dihydrobenzo[d][1,3]oxazin-2-one |
| 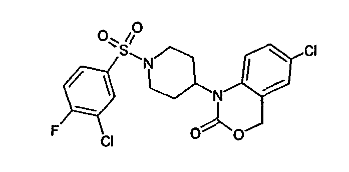 | 6-Chloro-1-[1-(3-chloro-4-fluorobenzenesulfonyl)-piperidin-4-yl]-1,4-dihydro-benzo[d][1,3]oxazin-2-one |
| 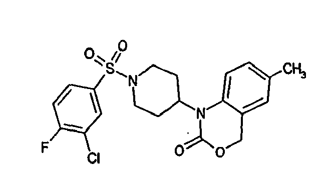 | 1-[1-(3-Chloro-4-fluorobenzenesulfonyl)-piperidin-4-yl]-6-methyl-1,4-dihydrobenzo[d][1,3]oxazin-2-one |
| 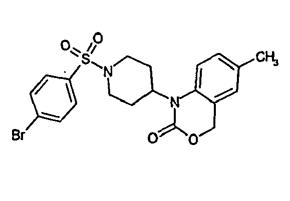 | 1-[1-(4-Bromo-benzenesulfonyl)-piperidin-4-yl]-6-methyl-1,4-dihydrobenzo[d][1,3]oxazin-2-one |
| 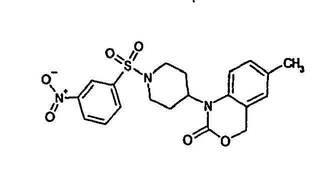 | 6-Methyl-1-[1-(3-nitrobenzenesulfonyl)-piperidin-4-yl]-1,4-dihydro-benzo[d][1,3]oxazin-2-one |
| 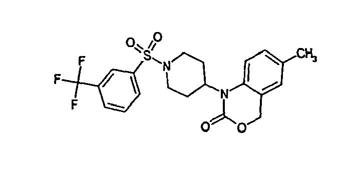 | 6-Methyl-1-[1-(3-trifluoromethylbenzenesulfonyl)-piperidin-4-yl]-1,4-dihydro-benzo[d][1,3]oxazin-2-one |
| 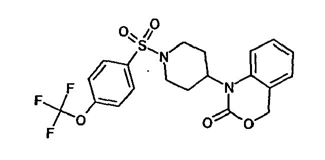 | 1-[1-(4-Trifluoromethoxybenzenesulfonyl)-piperidin-4-yl]-1,4-dihydro-benzo[d][1,3]oxazin-2-one |
| 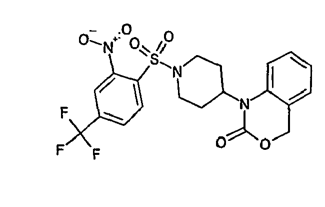 | 1-[1-(2-Nitro-4-trifluoromethylbenzenesulfonyl)-piperidin-4-yl]-1,4-dihydro-benzo[d][1,3]oxazin-2-one |
| 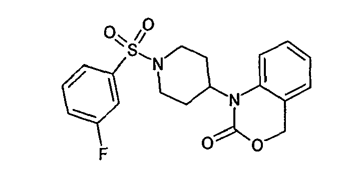 | 1-[1-(3-Fluoro-benzenesulfonyl)-piperidin-4-yl]-1,4-dihydro-benzo[d][1,3]oxazin-2-one |
| 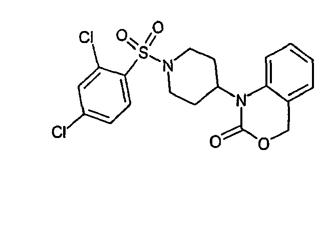 | 1-[1-(2,4-Dichloro-benzenesulfonyl)-piperidin-4-yl]-1,4-dihydrobenzo[d][1,3]oxazin-2-one |
| 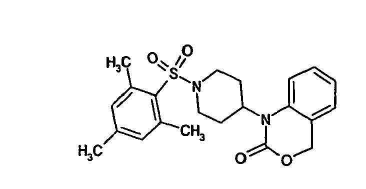 | 1-[1-(2,4,6-Trimethyl-benzenesulfonyl)-piperidin-4-yl]-1,4-dihydrobenzo[d][1,3]oxazin-2-one |
| 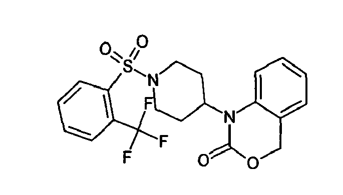 | 1-[1-(2-Trifluoromethylbenzenesulfonyl)-piperidin-4-yl]-1 ,4-dihydro-benzo[d][1,3]oxazin-2-one |
| 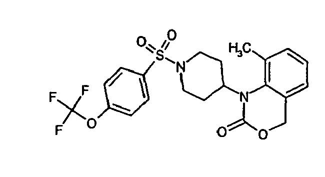 | 8-Methyl-1-[1-(4-trifluoromethoxybenzenesulfonyl)-piperidin-4-yl]-1,4-dihydro-benzo[d][1,3]oxazin-2-one |
| 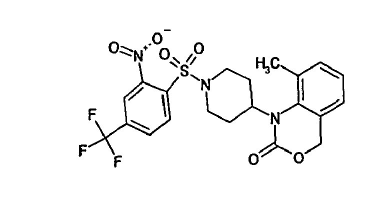 | 8-Methyl-1-[1-(2-nitro-4-trifluoromethylbenzenesulfonyl)-piperidin-4-yl]-1,4-dihydro-benzo[d][1,3]oxazin-2-one |
| 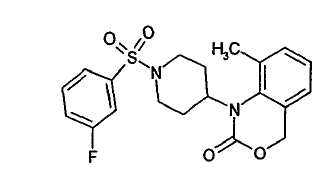 | 1-[1-(3-Fluoro-benzenesulfonyl)-piperidin-4-yl]-8-methyl-1,4-dihydrobenzo[d][1,3]oxazin-2-one |
| 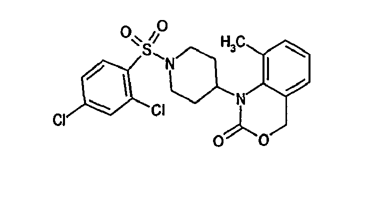 | 1-[1-(2,4-Dichloro-benzenesulfonyl)-piperidin-4-yl]-8-methyl-1,4-dihydrobenzo[d][1,3]oxazin-2-one |
| 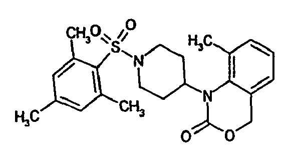 | 8-Methyl-1-[1-(2,4,6-trimethylbenzenesulfonyl)-piperidin-4-yl]-1,4-dihydro-benzo[d][1,3]oxazin-2-one |
| 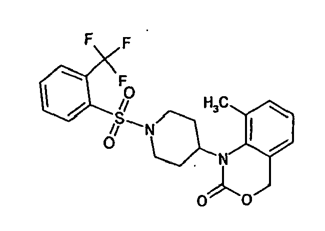 | 8-Methyl-1-[1-(2-trifluoromethylbenzenesulfonyl)-piperidin-4-yl]-1,4-dihydro-benzo[d][1,3]oxazin-2-one |
| 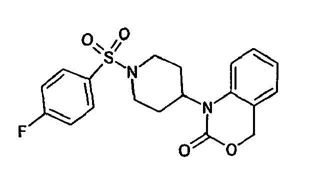 | 1-[1-(4-Fluoro-benzenesulfonyl)-piperidin-4-yl]-1,4-dihydrobenzo[d][1,3]oxazin-2-one |
| 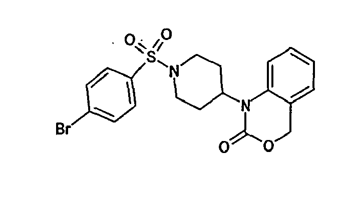 | 1-[1-(4-Bromo-benzenesulfonyl)-piperidin-4-yl]-1,4-dihydrobenzo[d][1,3]oxazin-2-one |
| 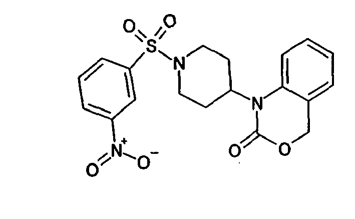 | 1-[1-(3-Nitro-benzenesulfonyl)-piperidin-4-yl]-1,4-dihydrobenzo[d][1,3]oxazin-2-one |
| 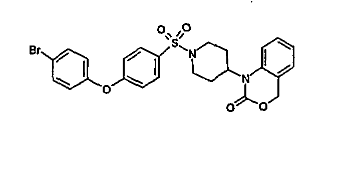 | 1-{1-[4-(4-Bromo-phenoxy)-benzenesulfonyl]-piperidin-4-yl}-1,4-dihydro-benzo[d][1,3]oxazin-2-one |
| 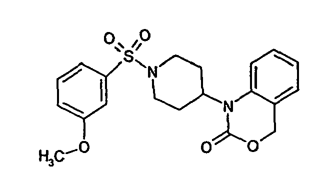 | 1-[1-(3-Methoxy-benzenesulfonyl)-piperidin-4-yl]-1,4-dihydrobenzo[d][1,3]oxazin-2-one |
| 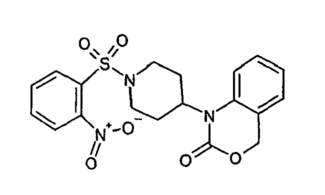 | 1-[1-(2-Nitro-benzenesulfonyl)-piperidin-4-yl]-1,4-dihydrobenzo[d][1,3]oxazin-2-one |
| 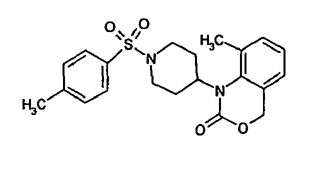 | 8-Methyl-1-[1-(toluene-4-sulfonyl)-piperidin-4-yl]-1,4-dihydrobenzo[d][1,3]oxazin-2-one |
| 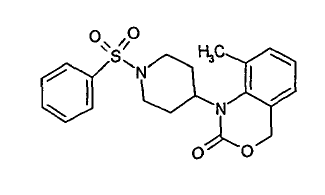 | 1-(1-Benzenesulfonyl-piperidin-4-yl)-8-methyl-1,4-dihydrobenzo[d][1,3]oxazin-2-one |
| 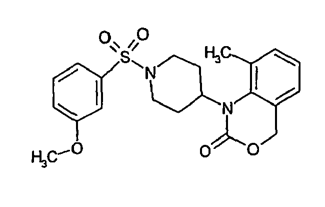 | 1-[1-(3-Methoxy-benzenesulfonyl)-piperidin-4-yl]-8-methyl-1,4-dihydrobenzo[d][1,3]oxazin-2-one |
| 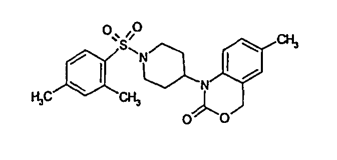 | 1-[1-(2,4-Dimethyl-benzenesulfonyl)-piperidin-4-yl]-6-methyl-1,4-dihydrobenzo[d][1,3]oxazin-2-one |
| 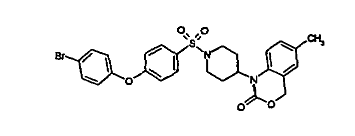 | 1-{1-[4-(4-Bromo-phenoxy)-benzenesulfonyl]-piperidin-4-yl}-6-methyl-1,4-dihydrobenzo[d][1,3]oxazin-2-one |
| 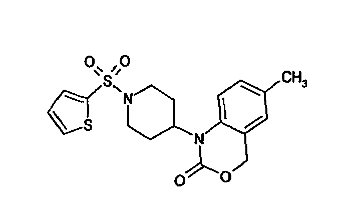 | 6-Methyl-1-[1-(thiophene-2-sulfonyl)-piperidin-4-yl]-1,4-dihydrobenzo[d][1,3]oxazin-2-one |
| 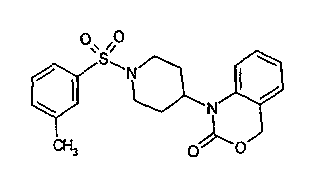 | 1-[1-(Toluene-3-sulfonyl)-piperidin-4-yl]-1,4-dihydro-benzo[d][1,3]oxazin-2-one |
| 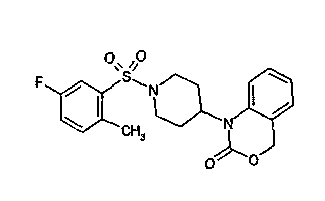 | 1-[1-(5-Fluoro-2-methyl-benzenesulfonyl)-piperidin-4-yl]-1,4-dihydro-benzo[d][1,3]oxazin-2-one |
| 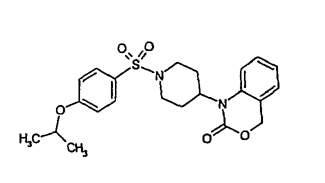 | 1-[1-(4-Isopropoxy-benzenesulfonyl)-piperidin-4-yl]-1,4-dihydrobenzo[d][1,3]oxazin-2-one |
| 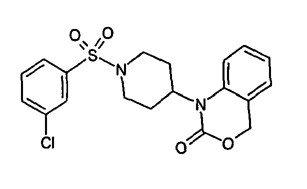 | 1-[1-(3-Chloro-benzenesulfonyl)-piperidin-4-yl]-1,4-dihydrobenzo[d][1,3]oxazin-2-one |
| 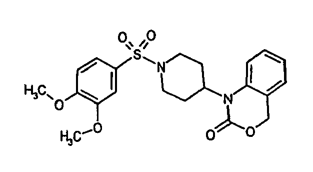 | 1-[1-(3,4-Dimethoxy-benzenesulfonyl)-piperidin-4-yl]-1,4-dihydrobenzo[d][1,3]oxazin-2-one |
| 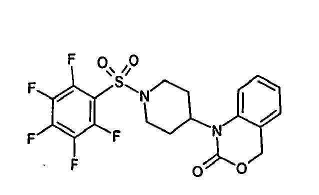 | 1-(1-Pentafluorobenzenesulfonylpiperidin-4-yl)-1,4-dihydrobenzo[d][1,3]oxazin-2-one |
| 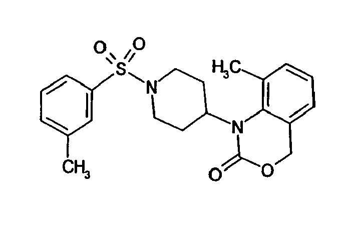 | 8-Methyl-1-[1-(toluene-3-sulfonyl)-piperidin-4-yl]-1,4-dihydrobenzo[d][1,3]oxazin-2-one |
| 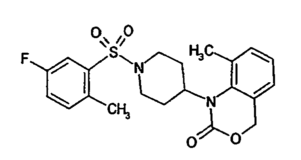 | 1-[1-(5-Fluoro-2-methylbenzenesulfonyl)-piperidin-4-yl]-8-methyl-1,4-dihydrobenzo[d][1,3]oxazin-2-one |
| 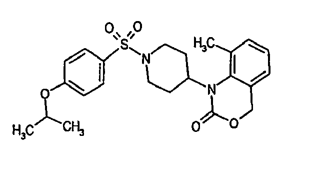 | 1-[1-(4-Isopropoxy-benzenesulfonyl)-piperidin-4-yl]-8-methyl-1,4-dihydrobenzo[d][1,3]oxazin-2-one |
| 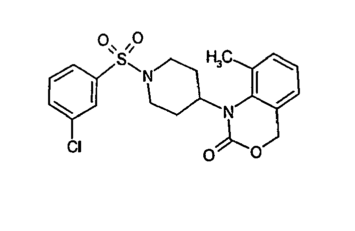 | 1-[1-(3-Chloro-benzenesulfonyl)-piperidin-4-yl]-8-methyl-1,4-dihydrobenzo[d][1,3]oxazin-2-one |
| 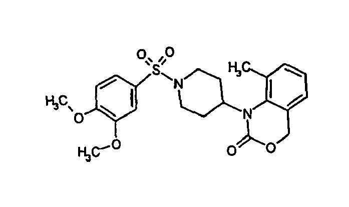 | 1-[1-(3,4-Dimethoxy-benzenesulfonyl)-piperidin-4-yl]-8-methyl-1,4-dihydrobenzo[d][1,3]oxazin-2-one |
| 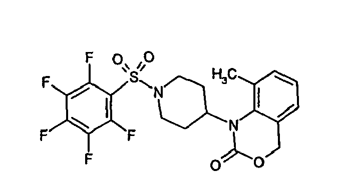 | 8-Methyl-1-(1-pentafluorobenzenesulfonyl-piperidin-4-yl)-1,4-dihydro-benzo[d][1,3]oxazin-2-one |
| 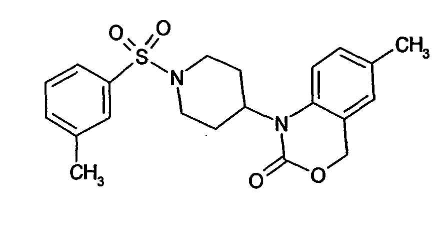 | 6-Methyl-1-[1-(toluene-3-sulfonyl)-piperidin-4-yl]-1,4-dihydrobenzo[d][1,3]oxazin-2-one |
| 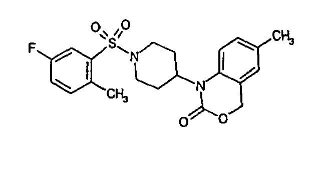 | 1-[1-(5-Fluoro-2-methylbenzenesulfonyl)-piperidin-4-yl]-6-methyl-1,4-dihydrobenzo[d][1,3]oxazin-2-one |
| 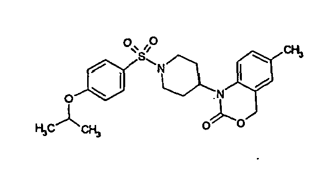 | 1-[1-(4-Isopropoxy-benzenesulfonyl)-piperidin-4-yl]-6-methyl-1,4-dihydrobenzo[d][1,3]oxazin-2-one |
| 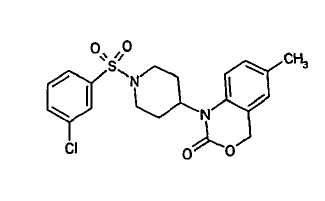 | 1-[1-(3-Chloro-benzenesulfonyl)-piperidin-4-yl]-6-methyl-1,4-dihydrobenzo[d][1,3]oxazin-2-one |
| 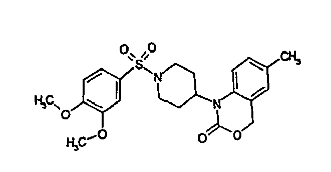 | 1-[1-(3,4-Dimethoxy-benzenesulfonyl)-piperidin-4-yl]-6-methyl-1,4-dihydrobenzo[d][1,3]oxazin-2-one |
| 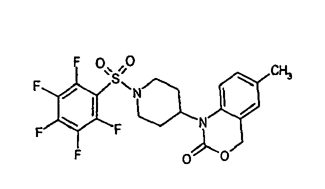 | 6-Methyl-1-(1-pentafluorobenzenesulfonyl-piperidin-4-yl)-1,4-dihydro-benzo[d][1,3]oxazin-2-one |
| 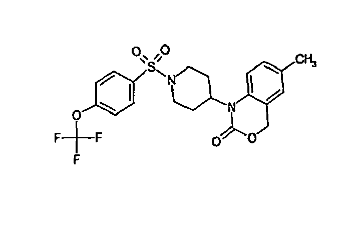 | 6-Methyl-1-[1-(4-trifluoromethoxybenzenesulfonyl)-piperidin-4-yl]-1,4-dihydro-benzo[d][1,3]oxazin-2-one |
| 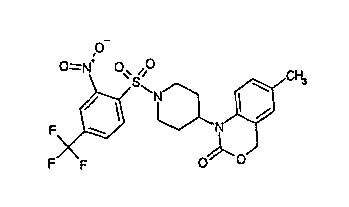 | 6-Methyl-1-[1-(2-nitro-4-trifluoromethylbenzenesulfonyl)-piperidin-4-yl]-1,4-dihydro-benzo[d][1,3]oxazin-2-one |
| 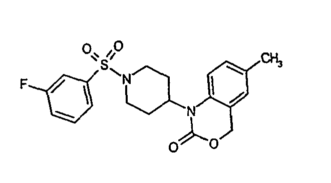 | 1-[1-(3-Fluoro-benzenesulfonyl)-piperidin-4-yl]-6-methyl-1,4-dihydrobenzo[d][1,3]oxazin-2-one |
| 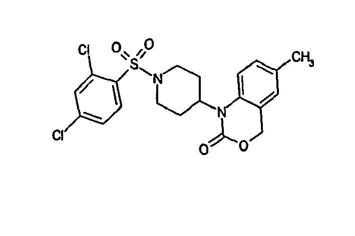 | 1-[1-(2,4-Dichloro-benzenesulfonyl)-piperidin-4-yl]-6-methyl-1,4-dihydrobenzo[d][1,3]oxazin-2-one |
| 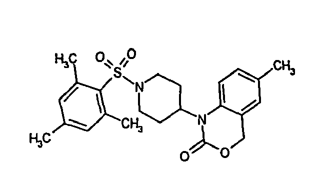 | 6-Methyl-1-[1-(2,4,6-trimethylbenzenesulfonyl)-piperidin-4-yl]-1 ,4-dihydro-benzo[d][1,3]oxazin-2-one |
| 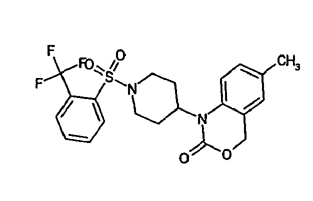 | 6-Methyl-1-[1-(2-trifluoromethylbenzenesulfonyl)-piperidin-4-yl]-1,4-dihydro-benzo[d][1,3]oxazin-2-one |
| 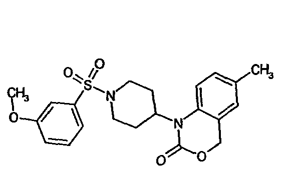 | 1-[1-(3-Methoxy-benzenesulfonyl)-piperidin-4-yl]-6-methyl-1,4-dihydrobenzo[d][1,3]oxazin-2-one |
| 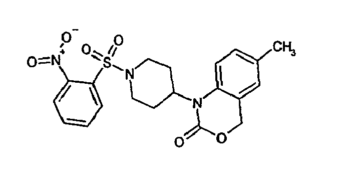 | 6-Methyl-1-[1-(2-nitrobenzenesulfonyl)-piperidin-4-yl]-1,4-dihydro-benzo[d][1,3]oxazin-2-one |
| 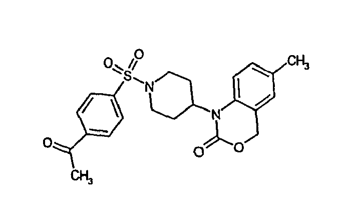 | 1-[1-(4-Acetyl-benzenesulfonyl)-piperidin-4-yl]-6-methyl-1,4-dihydrobenzo[d][1,3]oxazin-2-one |
| 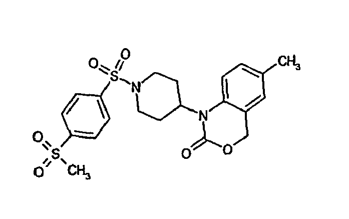 | 1-[1-(4-Methanesulfonylbenzenesulfonyl)-piperidin-4-yl]-6-methyl-1,4-dihydrobenzo[d][1,3]oxazin-2-one |
| 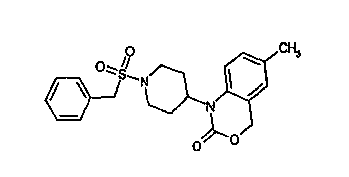 | 6-Methyl-1-(1-phenylmethanesulfonylpiperidin-4-yl)-1,4-dihydrobenzo[d][1,3]oxazin-2-one |
| 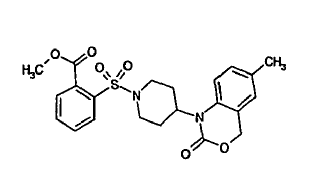 | 2-[4-(6-Methyl-2-oxo-4H-benzo[d][1,3]oxazin-1-yl)-piperidine-1-sulfonyl]-benzoic acid methyl ester |
| 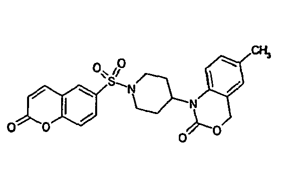 | 6-Methyl-1-[1-(2-oxo-2H-chromene-6-sulfonyl)-piperidin-4-yl]-1,4-dihydrobenzo[d][1,3]oxazin-2-one |
| 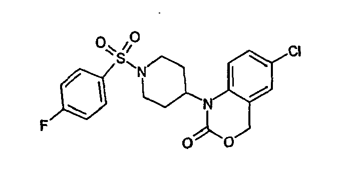 | 6-Chloro-1-[1-(4-fluorobenzenesulfonyl)-piperidin-4-yl]-1,4-dihydro-benzo[d][1,3]oxazin-2-one |
| 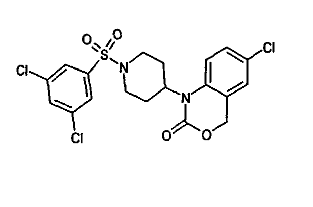 | 6-Chloro-1-[1-(3,5-dichlorobenzenesulfonyl)-piperidin-4-yl]-1,4-dihydro-benzo[d][1,3]oxazin-2-one |
| 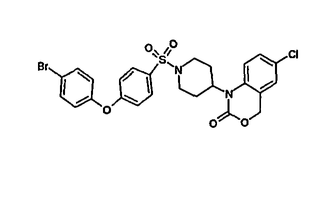 | 1-{1-[4-(4-Bromo-phenoxy)-benzenesulfonyl]-piperidin-4-yl}-6-chloro-1,4-dihydro-benzo[d][1,3]oxazin-2-one |
| 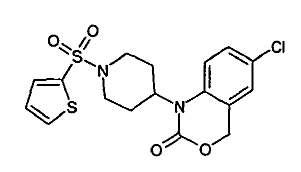 | 6-Chloro-1-[1-(thiophene-2-sulfonyl)-piperidin-4-yl]-1,4-dihydrobenzo[d][1,3]oxazin-2-one |
| 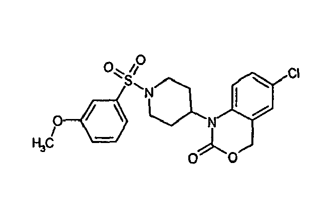 | 6-Chloro-1-[1-(3-methoxybenzenesulfonyl)-piperidin-4-yl]-1,4-dihydro-benzo[d][1,3]oxazin-2-one |
| 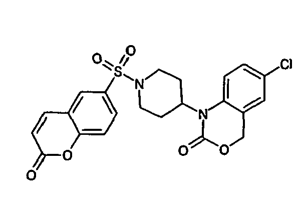 | 6-Chloro-1-[1-(2-oxo-2H-chromene-6-sulfonyl)-piperidin-4-yl]-1,4-dihydrobenzo[d][1,3]oxazin-2-one |
| 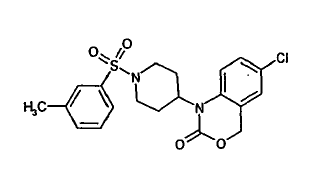 | 6-Chloro-1-[1-(toluene-3-sulfonyl)-piperidin-4-yl]-1,4-dihydrobenzo[d][1,3]oxazin-2-one |
| 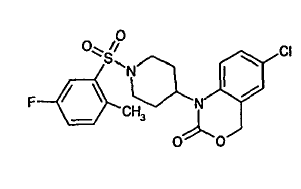 | 6-Chloro-1-[1-(5-fluoro-2-methylbenzenesulfonyl)-piperidin-4-yl]-1,4-dihydro-benzo[d][1,3]oxazin-2-one |
| 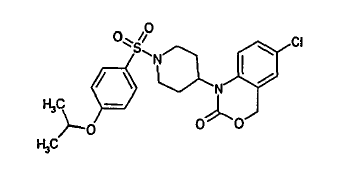 | 6-Chloro-1-[1-(4-isopropoxybenzenesulfonyl)-piperidin-4-yl]-1,4-dihydro-benzo[d][1,3]oxazin-2-one |
| 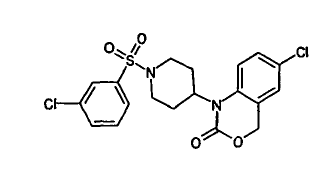 | 6-Chloro-1-[1-(3-chlorobenzenesulfonyl)-piperidin-4-yl]-1,4-dihydro-benzo[d][1,3]oxazin-2-one |
| 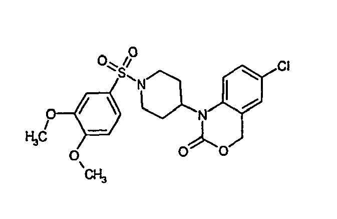 | 6-Chloro-1-[1-(3,4-dimethoxybenzenesulfonyl)-piperidin-4-yl]-1,4-dihydro-benzo[d][1,3]oxazin-2-one |
| 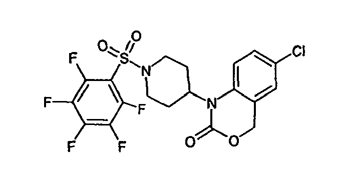 | 6-Chloro-1-(1-pentafluorobenzenesulfonyl-piperidin-4-yl)-1,4-dihydro-benzo[d][1,3]oxazin-2-one |
| 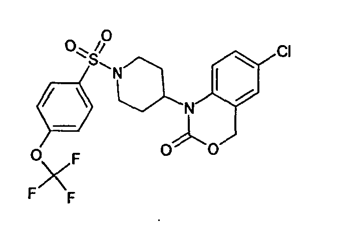 | 6-Chloro-1-[1-(4-trifluoromethoxybenzenesulfonylrpiperidin-4-yl]-1,4-dihydro-benzo[d][1,3]oxazin-2-one |
| 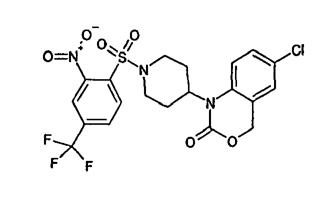 | 6-Chloro-1-[1-(2-nitro-4-trifluoromethylbenzenesulfonyl)-piperidin-4-yl]-1,4-dihydro-benzo[d][1,3]oxazin |
| 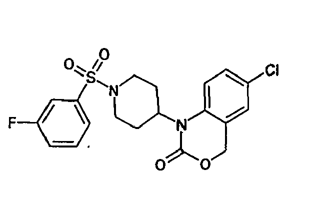 | 6-Chloro-1-[1-(3-fluorobenzenesulfonyl)-piperidin-4-yl]-1,4-dihydro-benzo[d][1,3]oxazin-2-one |
| 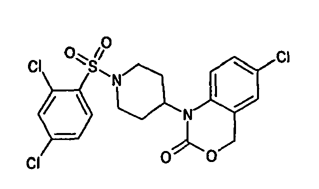 | 6-Chloro-1-[1-(2,4-dichlorobenzenesulfonyl)-piperidin-4-yl]-1,4-dihydro-benzo[d][1,3]oxazin-2-one |
| 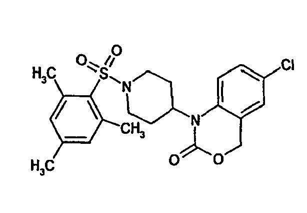 | 6-Chloro-1-[1-(2,4,6-trimethylbenzenesulfonyl)-piperidin-4-yl]-1,4-dihydro-benzo[d][1,3]oxazin-2-one |
| 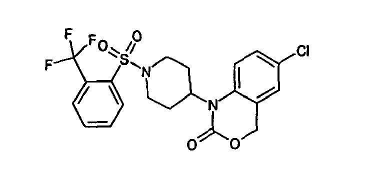 | 6-Chloro-1-[1-(2-trifluoromethylbenzenesulfonyl)-piperidin-4-yl]-1,4-dihydro-benzo[d][1,3]oxazin-2-one |
| 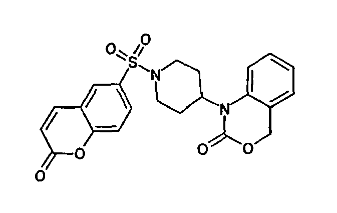 | 1-[1-(2-Oxo-2H-chromene-6-sulfonyl)-piperidin-4-yl]-1,4-dihydrobenzo[d][1,3]oxazin-2-one |
| 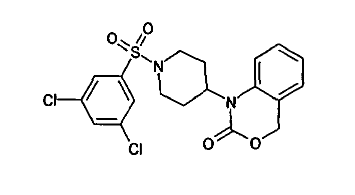 | 1-[1-(3,5-Dichloro-benzenesulfonyl)-piperidin-4-yl]-1,4-dihydrobenzo[d][1,3]oxazin-2-one |
| 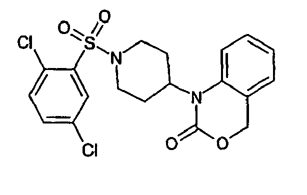 | 1-[1-(2,5-Dichloro-benzenesulfonyl)-piperidin-4-yl]-1,4-dihydrobenzo[d][1,3]oxazin-2-one |
| 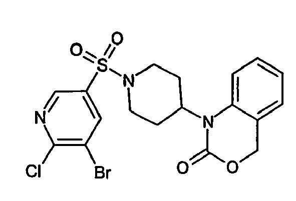 | 1-[1-(5-Bromo-6-chloro-pyridine-3-sulfonyl)-piperidin-4-yl]-1,4-dihydrobenzo[d][1,3]oxazin-2-one |
| 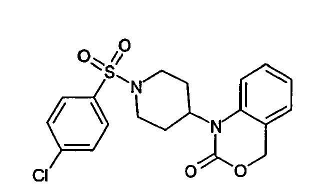 | 1-[1-(4-Chloro-benzenesulfonyl)-piperidin-4-yl]-1,4-dihydrobenzo[d][1,3]oxazin-2-one |
| 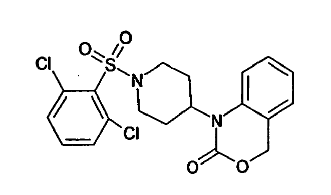 | 1-[1-(2,6-Dichloro-benzenesulfonyl)-piperidin-4-yl]-1,4-dihydrobenzo[d][1,3]oxazin-2-one |
| 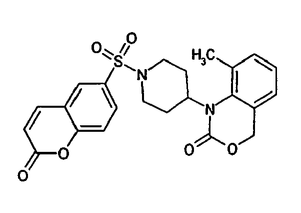 | 8-Methyl-1-[1-(2-oxo-2H-chromene-6-sulfonyl)-piperidin-4-yl]-1,4-dihydrobenzo[d][1,3]oxazin-2-one |
| 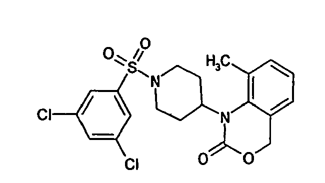 | 1-[1-(3,5-Dichloro-benzenesulfonyl)-piperidin-4-yl]-8-methyl-1,4-dihydrobenzo[d][1,3]oxazin-2-one |
| 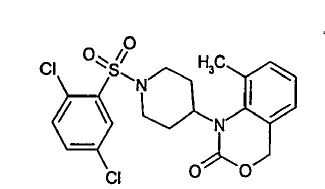 | 1-[1-(2,5-Dichloro-benzenesulfonyl)-piperidin-4-yl]-8-methyl-1,4-dihydrobenzo[d][1,3]oxazin-2-one |
| 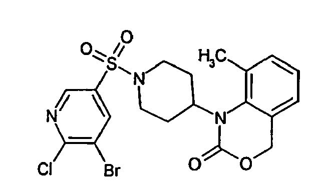 | 1-[1-(5-Bromo-6-chloro-pyridine-3-sulfonyl)-piperidin-4-yl]-8-methyl-1,4-dihydro-benzo[d][1,3]oxazin-2-one |
| 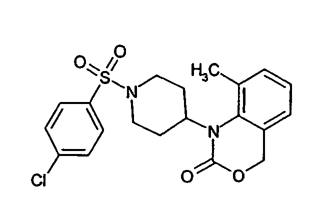 | 1-[1-(4-Chloro-benzenesulfonyl)-piperidin-4-yl]-8-methyl-1,4-dihydrobenzo[d][1,3]oxazin-2-one |
| 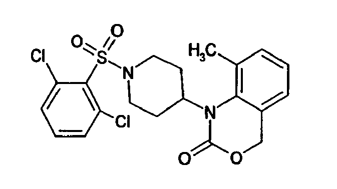 | 1-[1-(2,6-Dichloro-benzenesulfonyl)-piperidin-4-yl]-8-methyl-1,4-dihydrobenzo[d][1,3]oxazin-2-one |
| 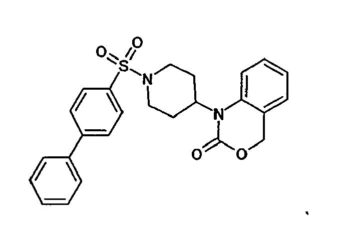 | 1-[1-(Biphenyl-4-sulfonyl)-piperidin-4-yl]-1,4-dihydro-benzo[d][1,3]oxazin-2-one |
| 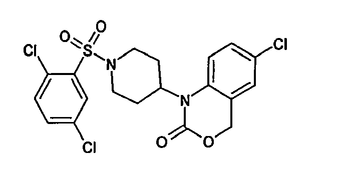 | 6-Chloro-1-[1-(2,5-dichlorobenzenesulfonyl)-piperidin-4-yl]-1,4-dihydro-benzo[d][1,3]oxazin-2-one |
| 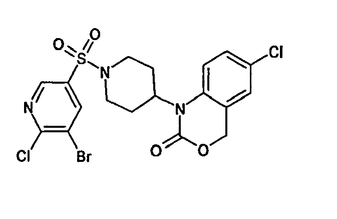 | 1-[1-(5-Bromo-6-chloro-pyridine-3-sulfonyl)-piperidin-4-yl]-6-chloro-1,4-dihydro-benzo[d][1,3]oxazin-2-one |
| 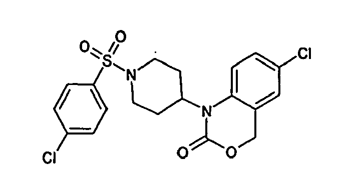 | 6-Chloro-1-[1-(4-chlorobenzenesulfonyl)-pipeidin-4-yl]-1,4-dihydro-benzo[d][1,3]oxazin-2-one |
| 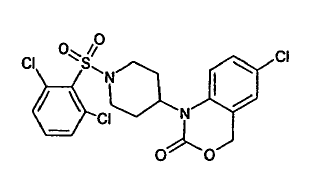 | 6-Chloro-1-[1-(2,6-dichlorobenzenesulfonyl)-piperidin-4-yl]-1,4-dihydro-benzo[d][1,3]oxazin-2-one |
| 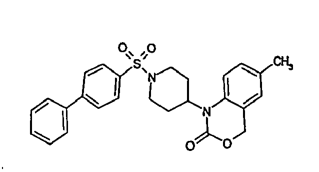 | 1-[1-(Biphenyl-4-sulfonyl)-piperidin-4-yl]-6-methyl-1,4-dihydrobenzo[d][1,3]oxazin-2-one |
| 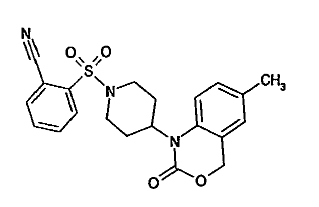 | 2-[4-(6-Methyl-2-oxo-4H-benzo[d][1,3]oxazin-1-yl)-piperidine-1-sulfonyl]-benzonitrile |
| 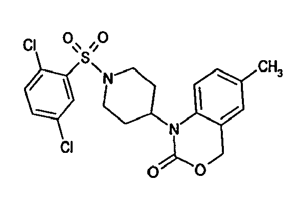 | 1-[1-(2,5-Dichloro-benzenesulfonyl)-piperidin-4-yl]-6-methyl-1,4-dihydrobenzo[d][1,3]oxazin-2-one |
| 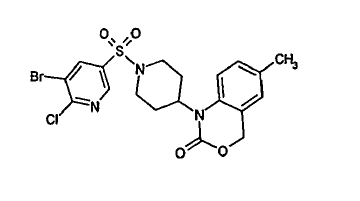 | 1-[1-(5-Bromo-6-chloro-pyridine-3-sulfonyl)-piperidin-4-yl]-6-methyl-1,4-dihydro-benzo[d][1,3]oxazin-2-one |
| 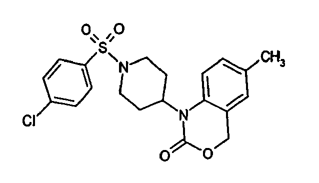 | 1-[1-(4-Chloro-benzenesulfonyl)-piperidin-4-yl]-6-methyl-1,4-dihydrobenzo[d][1,3]oxazin-2-one |
| 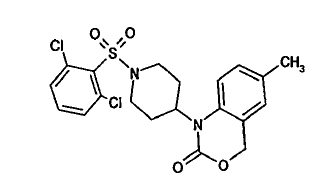 | 1-[1-(2,6-Dichloro-benzenesulfonyl)-piperidin-4-yl]-6-methyl-1,4-dihydrobenzo[d][1,3]oxazin-2-one |
| 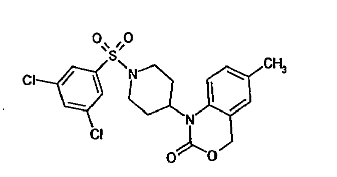 | 1-[1-(3,5-Dichloro-benzenesulfonyl)-piperidin-4-yl]-6-methyl-1,4-dihydrobenzo[d][1,3]oxazin-2-one |
| 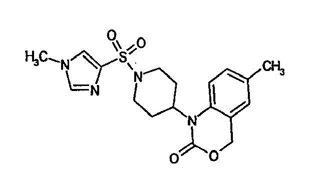 | 6-Methyl-1-[1-(1-methyl-1 H-imidazole-4-sulfonyl)-piperidin-4-yl]-1,4-dihydrobenzo[d][1,3]oxazin-2-one |
| 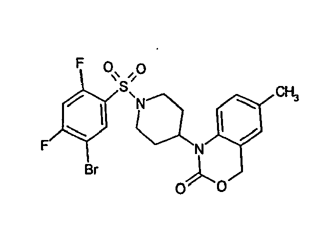 | 1-[1-(5-Bromo-2,4-difluorobenzenesulfonyl)-piperidin-4-yl]-6-methyl-1,4-dihydrobenzo[d][1,3]oxazin-2-one |
| 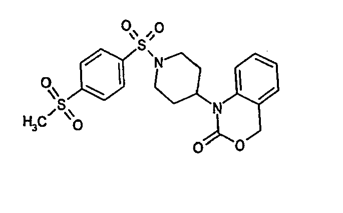 | 1-[1-(4-Methanesulfonylbenzenesulfonyl)-piperidin-4-yl]-1 ,4-dihydro-benzo[d][1,3]oxazin-2-one |
| 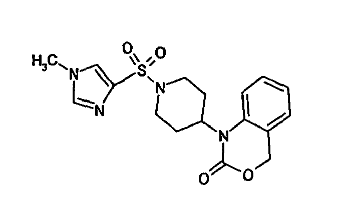 | 1-[1-(1-Methyl-1 H-imidazole-4-sulfonyl)-piperidin-4-yl]-1,4-dihydrobenzo[d][1,3]oxazin-2-one |
| 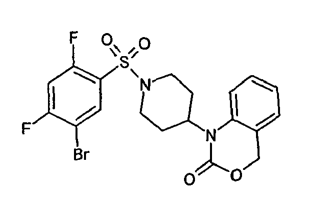 | 1-[1-(5-Bromo-2,4-difluorobenzenesulfonyl)-piperidin-4-yl]-1,4-dihydro-benzo[d][1,3]oxazin-2-one |
| 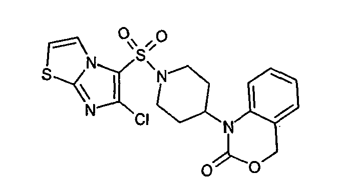 | 1-[1-(6-Chloro-imidazo[2,1-b]thiazole-5-sulfonyl)-piperidin-4-yl]-1,4-dihydrobenzo[d][1,3]oxazin-2-one |
| 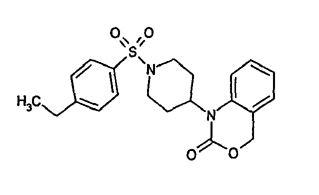 | 1-[1-(4-Ethyl-benzenesulfonyl)-piperidin-4-yl]-1,4-dihydrobenzo[d][1,3]oxazin-2-one |
| 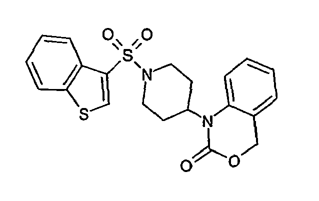 | 1-[1-(Benzo[b]thiophene-3-sulfonyl)-piperidin-4-yl]-1,4-dihydrobenzo[d][1,3]oxazin-2-one |
| 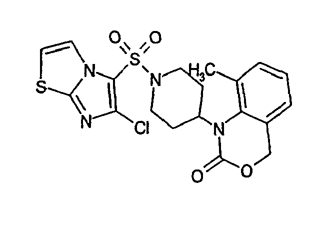 | 1-[1-(6-Chloro-imidazo[2,1-b]thiazole-5-sulfonyl)-piperidin-4-yl]-8-methyl-1,4-dihydro-benzo[d][1,3]oxazin-2-one |
| 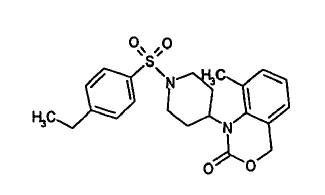 | 1-[1-(4-Ethyl-benzenesulfonyl)-piperidin-4-yl]-8-methyl-1,4-dihydrobenzo[d][1,3]oxazin-2-one |
| 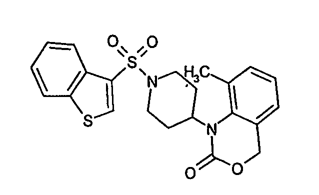 | 1-[1-(Benzo[b]thiophene-3-sulfonyl)-piperidin-4-yl]-8-methyl-1,4-dihydrobenzo[d][1,3]oxazin-2-one |
| 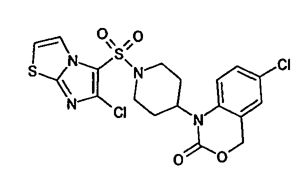 | 6-Chloro-1-[1-(6-chloro-imidazo[2,1-b]thiazole-5-sulfonyl)-piperidin-4-yl]-1,4-dihydro-benzo[d][1,3]oxazin-2-one |
| 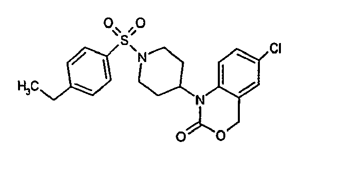 | 6-Chloro-1-[1-(4-ethylbenzenesulfonyl)-piperidin-4-yl]-1,4-dihydro-benzo[d][1,3]oxazin-2-one |
| 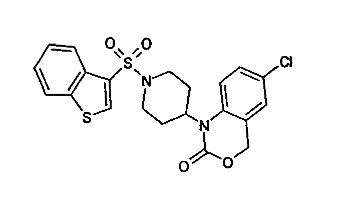 | 1-[1-(Benzo[b]thiophene-3-sulfonyl)-piperidin-4-yl]-6-chloro-1,4-dihydrobenzo[d][1,3]oxazin-2-one |
| 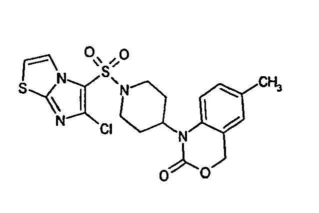 | 1-[1-(6-Chloro-imidazo[2,1-b]thiazole-5-sulfonyl)-piperidin-4-yl]-6-methyl-1,4-dihydro-benzo[d][1,3]oxazin-2-one |
| 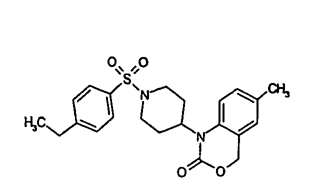 | 1-[1-(4-Ethyl-benzenesulfonyl)-piperidin-4-yl]-6-methyl-1,4-dihydrobenzo[d][1,3]oxazin-2-one |
| 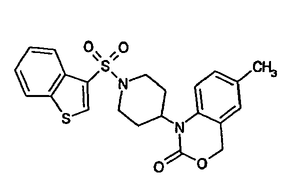 | 1-[1-(Benzo[b]thiophene-3-sulfonyl)-piperidin-4-yl]-6-methyl-1,4-dihydrobenzo[d][1,3]oxazin-2-one |
| 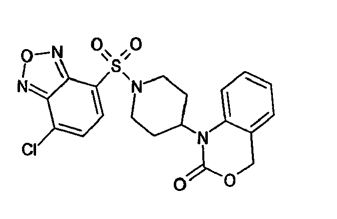 | 1-[1-(7-Chloro-benzo[1,2,5]oxadiazole-4-sulfonyl)-piperidin-4-yl]-1,4-dihydro-benzo[d][1,3]oxazin-2-one |
| 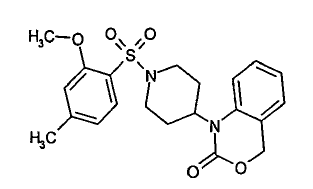 | 1-[1-(2-Methoxy-4-methylbenzenesulfonyl)-piperidin-4-yl]-1,4-dihydro-benzo[d][1,3]oxazin-2-one |
| 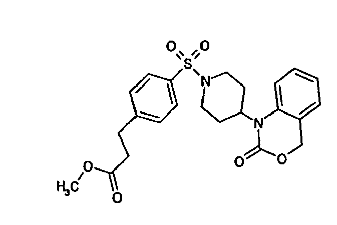 | 3-{4-[4-(2-Oxo-4H-benzo[d][1,3]oxazin-1-yl)-piperidine-1-sulfonyl]-phenyl}-propionic acid methyl ester |
| 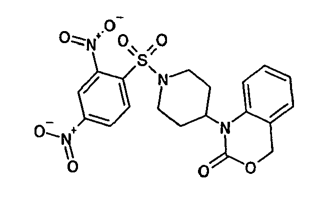 | 1-[1-(2,4-Dinitro-benzenesulfonyl)-piperidin-4-yl]-1,4-dihydrobenzo[d][1,3]oxazin-2-one |
| 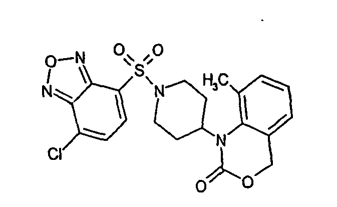 | 1-[1-(7-Chloro-benzo[1,2,5]oxadiazole-4-sulfonyl)-piperidin-4-yl]-8-methyl-1,4-dihydro-benzo[d][1,3]oxazin-2-one |
| 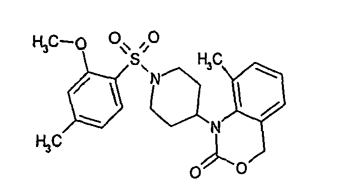 | 1-[1-(2-Methoxy-4-methylbenzenesulfonyl)-piperidin-4-yl]-8-methyl-1,4-dihydrobenzo[d][1,3]oxazin-2-one |
| 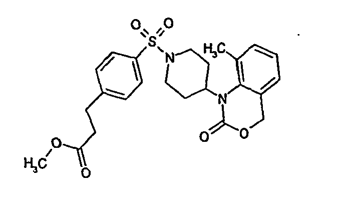 | 3-{4-[4-(8-Methyl-2-oxo-4H-benzo[d][1,3]oxazin-1-yl)-piperidine-1-sulfonyl]-phenyl)-propionic acid methyl ester |
| 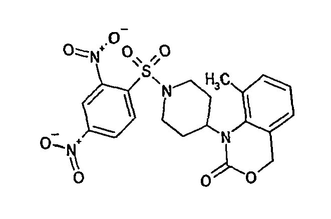 | 1-[1-(2,4-Dinitro-benzenesulfonyl)-piperidin-4-yl)-8-methyl-1,4-dihydrobenzo[d][1,3]oxazin-2-one |
| 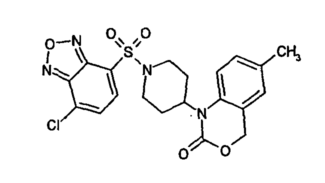 | 1-[1-(7-Chloro-benzo[1,2,5]oxadiazole-4-sulfonyl)-piperidin-4-yl]-6-methyl-1,4-dihydro-benzo[d][1,3]oxazin-2-one |
| 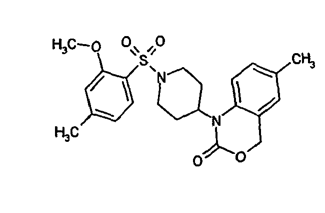 | 1-[1-(2-Methoxy-4-methylbenzenesulfonyl)-piperidin-4-yl]-6-methyl-1,4-dihydrobenzo[d][1,3]oxazin-2-one |
| 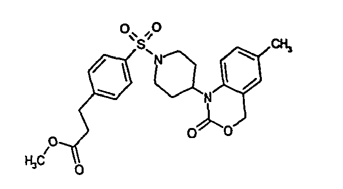 | 3-{4-[4-(6-Methyl-2-oxo-4H-benzo[d][1,3]oxazin-1-yl)-piperidine-1-sulfonyl]-phenyl}-propionic acid methyl ester |
| 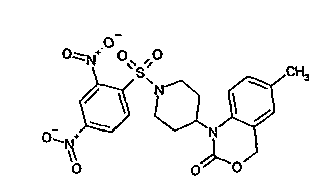 | 1-[1-(2,4-Dinitro-benzenesulfonyl)-piperidin-4-yl]-6-methyl-1,4-dihydrobenzo[d][1,3]oxazin-2-one |
| 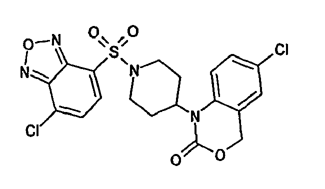 | 6-Chloro-1-[1-(7-chlorobenzo[1,2,5]oxadiazole-4-sulfonyl)-piperidin-4-yl]-1,4-dihydrobenzo[d][1,3]oxazin-2-one |
| 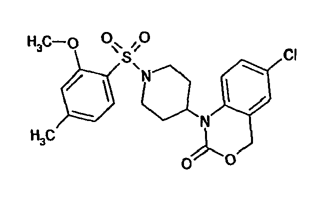 | 6-Chloro-1-[1-(2-methoxy-4-methylbenzenesulfonyl)-piperidin-4-yl]-1,4-dihydro-benzo[d][1,3]oxazin-2-one |
| 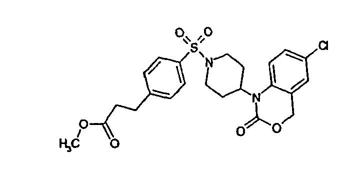 | 3-{4-[4-(6-Chloro-2-oxo-4H-benzo[d][1,3]oxazin-1-yl)-piperidine-1-sulfonyl]-phenyl}-propionic acid methyl ester |
| 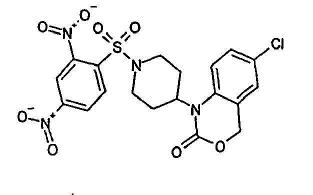 | 6-Chloro-1-[1-(2,4-dinitrobenzenesulfonyl)-piperidin-4-yl]-1,4-dihydro-benzo[d][1,3]oxazin-2-one |
| 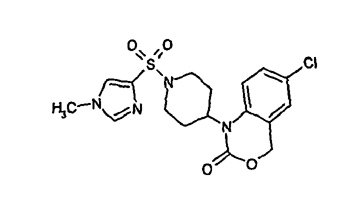 | 6-Chloro-1-[1-(1-methyl-1H-imidazole-4-sulfonyl)-piperidin-4-yl]-1,4-dihydrobenzo[d][1,3]oxazin-2-one |
| 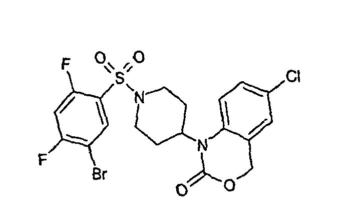 | 1-[1-(5-Bromo-2,4-difluorobenzenesulfonyl)-piperidin-4-yl]-6-chloro-1,4-dihydro-benzo[d][1,3]oxazin-2-one |
| 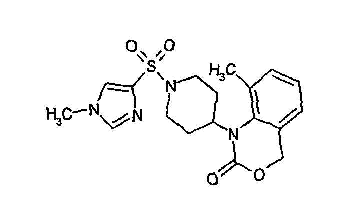 | 8-Methyl-1-[1-(1-methyl-1H-imidazole-4-sulfonyl)-piperidin-4-yl]-1,4-dihydrobenzo[d][1,3]oxazin-2-one |
| 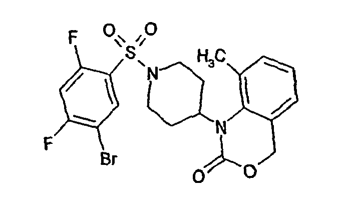 | 1-[1-(5-Bromo-2,4-difluorobenzenesulfonyl)-piperid in-4-yl]-8-methyl-1,4-dihydrobenzo[d][1,3]oxazin-2-one |
| 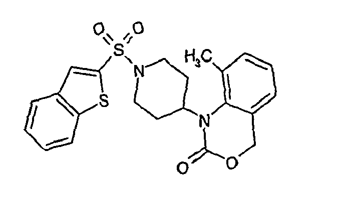 | 1-[1-(Benzo[b]thiophene-2-sulfonyl)-piperidin-4-yl]-8-methyl-1,4-dihydrbenzo[d][1,3]oxazin-2-one |
| 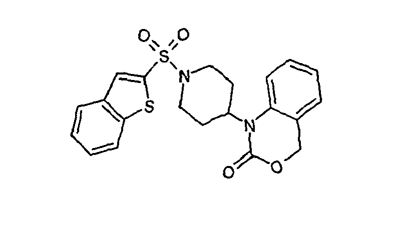 | 1-[1-(Benzo[b]thiophene-2-sulfonyl)-piperidin-4-yl]-1,4-dihydrobenzo[d][1,3]oxazin-2-one |
| 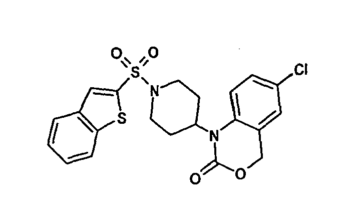 | 1-[1-(Benzo[b]thiophene-2-sulfonyl)-piperidin-4-yl]-6-chloro-1,4-dihydrobenzo[d][1,3]oxazin-2-one |
| 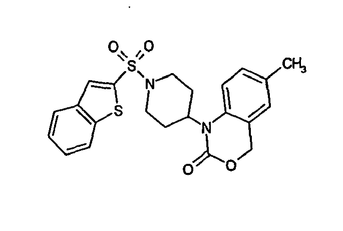 | 1-[1-(Benzo[b]thiophene-2-sulfonyl)-piperidin-4-yl]-6-methyl-1,4-dihydrobenzo[d][1,3]oxazin-2-one |
| 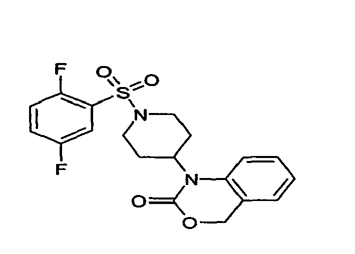 | 1-[1-(2,5-Difluorobenzenesulfonyl)-piperidin-4-yl]-1,4-dihydrobenzo[d][1,3]oxazin-2-one |
| 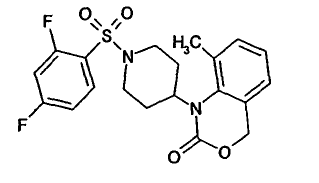 | 1-[1-(2,5-Difluorobenzenesuifonyl)-piperidin-4-yl]-8-methyl-1,4-dihydrobenzo[d][1,3]oxazin-2-one |
| 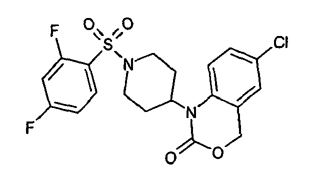 | 6-Chloro-1-[1-(2,5-difluorobenzenesulfonyl)-piperidin-4-yl]-1,4-dihydrobenzo[d][1,3]oxazin-2-one |
| 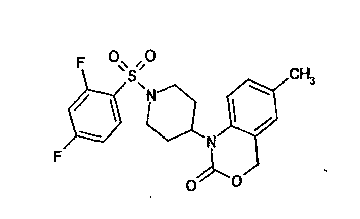 | 1-[1-(2,5-Difluorobenzenesulfonyl)-piperidin-4-yl]-6-methyl-1,4-dihydrobenzo[d][1,3]oxazin-2-one |
| 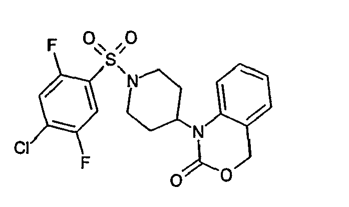 | 1-[1-(4-Chloro-2,5-difluorobenzenesulfonyl)-piperidin-4-yl]-1,4-dihydrobenzo[d][1,3]oxazin-2-one |
| 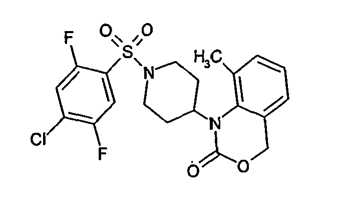 | 1-[1-(4-Chloro-2,5-difluorobenzenesulfonyl)-piperidin-4-yl]-8-methyl-1,4-dihydrobenzo[d][1,3]oxazin-2-one |
| 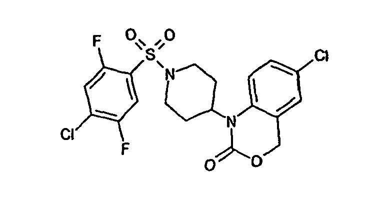 | 6-Chloro-1-[1-(4-chloro-2,5-difluoro-benzenesulfonyl)-piperidin-4-yl]-1,4-dihydrobenzo[d][1,3]oxazin-2-one |
| 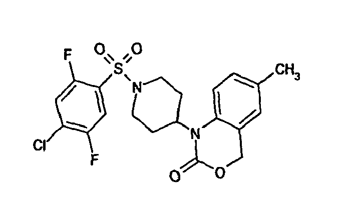 | 1-[1-(4-Chloro-2,5-difluorobenzenesulfonyl)-piperidin-4-yl]-6-methyl-1,4-dihydrobenzo[d][1,3]oxazin-2-one |
| 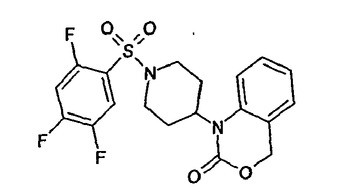 | 1-[1-(2,4,5-Trifluorobenzenesulfonyl)-piperidin-4-yl]-1,4-dihydrobenzo[d][1,3]oxazin-2-one |
| 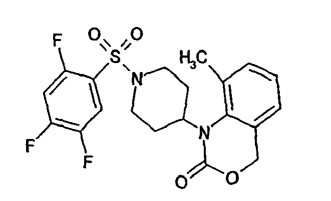 | 8-Methyl-1-[1-(2,4,5-trifluorobenzenesulfonyl)-piperidin-4-yl]-1,4-dihydrobenzo[d][1,3]oxazin-2-one |
| 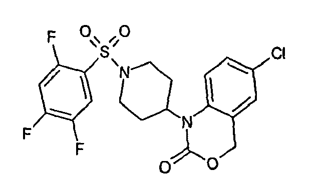 | 6-Chloro-1-[1-(2,4,5-trifluorobenzenesulfonyl)-piperidin-4-yl]-1,4-dihydrobenzo[d][1,3]oxazin-2-one |
| 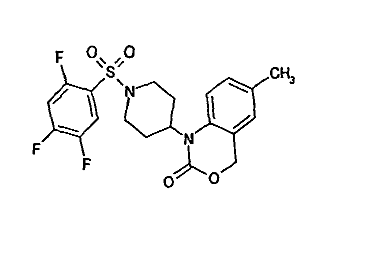 | 6-Methyl-1-[1-(2,4,5-trifluorobenzenesulfonyl)-piperidin-4-yl]-1,4-dihydrobenzo[d][1,3]oxazin-2-one |
| 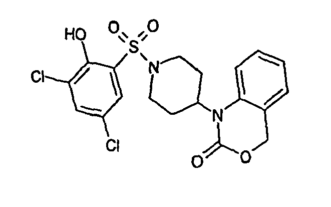 | 1-[1-(3,5-Dichloro-2-hydroxybenzenesulfonyl)-piperidin-4-yl]-1,4-dihydrobenzo[d][1,3]oxazin-2-one |
| 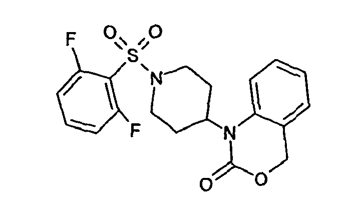 | 1-[1-(2,6-Difluorobenzenesulfonyl)-piperidin-4-yl]-1,4-dihydrobenzo[d][1,3]oxazin-2-one |
| 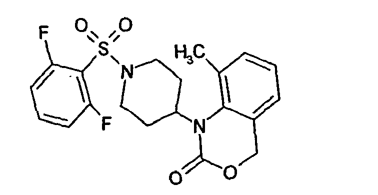 | 1-[1-(2,6-Difluorobenzenesulfonyl)-piperidin-4-yl]-8-methyl-1,4-dihydrobenzo[d][1,3]oxazin-2-one |
| 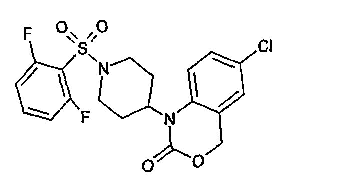 | 6-Chloro-1-[1-(2,6-difluorobenzenesulfonyl)-piperidin-4-yl]-1,4-dihydrobenzo[d][1,3]oxazin-2-one |
| 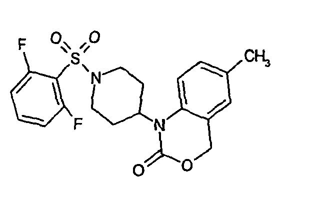 | 1-[1-(2,6-Difluorobenzenesulfonyl)-piperidin-4-yl]-6-methyl-1,4-dihydrobenzo[d][1,3]oxazin-2-one |
| 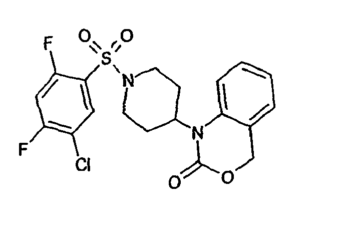 | 1-[1-(5-Chloro-2,4-difluorobenzenesulfonyl)-piperidin-4-yl]-1,4-dihydrobenzo[d][1,3]oxazin-2-one |
| 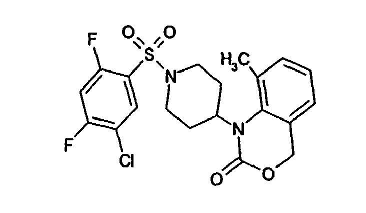 | 1-[1-(5-Chloro-2,4-difluorobenzenesulfonyl)-piperidin-4-yl]-8-methyl-1,4-dihydrobenzo[d][1,3]oxazin-2-one |
| 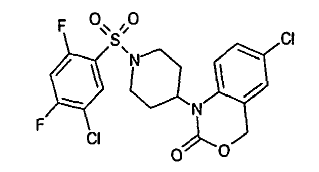 | 6-Chloro-1-[1-(5-chloro-2,4-difluoro-benzenesulfonyl)-piperidin-4-yl]-1,4-dihydrobenzo[d][1,3]oxazin-2-one |
| 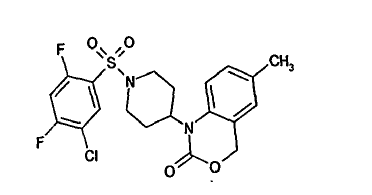 | 1-[1-(5-Chloro-2,4-difluorobenzenesulfonyl)-piperidin-4-yl]-6-methyl-1,4-dihydrobenzo[d][1,3]oxazin-2-one |
| 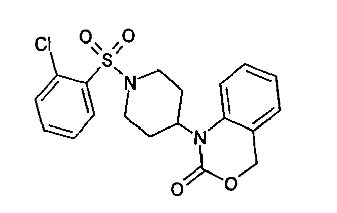 | 1-[1-(2-Chlorobenzenesulfonyl)-piperidin-4-yl]-1,4-dihydrobenzo[d][1,3]oxazin-2-one |
| 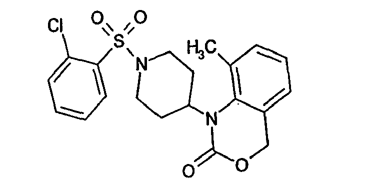 | 1-[1-(2-Chlorobenzenesulfonyl)-piperidin-4-yl]-8-methyl-1,4-dihydrobenzo[d][1,3]oxazin-2-one |
| 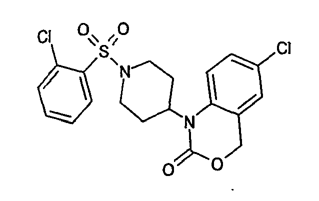 | 6-Chloro-1-[1-(2-chlorobenzenesulfonyl)-piperidin-4-yl]-1,4-dihydrobenzo[d][1,3]oxazin-2-one |
| 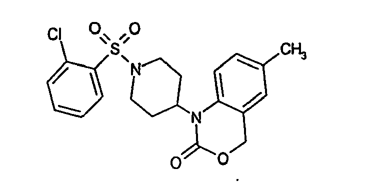 | 1-[1-(2-Chlorobenzenesulfonyl)-piperidin-4-yl]-6-methyl-1,4-dihydrobenzo[d][1,3]oxazin-2-one |
| 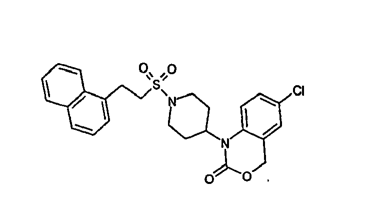 | 6-Chloro-1-[1-(2-naphthalen-1-yl-ethanesulfonyl)-piperidin-4-yl]-1,4-dihydrobenzo[d][1,3]oxazin-2-one |
| 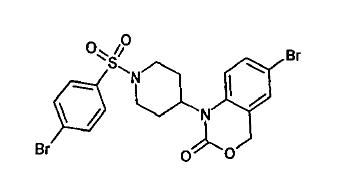 | 6-Bromo-1-[1-(4-bromobenzenesulfonyl)-piperidin-4-yl]-1,4-dihydrobenzo[d][1,3]oxazin-2-one |
| 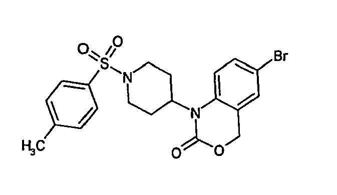 | 6-Bromo-1-[1-(toluene-4-sulfonyl)-piperidin-4-yl]-1,4-dihydro-benzo[d][1,3]oxazin-2-one |
| 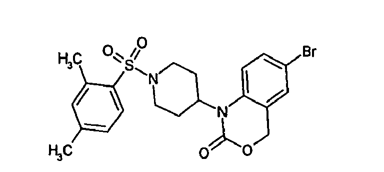 | 6-Bromo-1-[1-(2,4-dimethylbenzenesulfonyl)-piperidin-4-yl]-1,4-dihydrobenzo[d][1,3]oxazin-2-one |
| 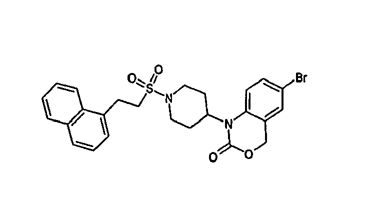 | 6-Bromo-1-[1-(2-naphthalen-1-yl-ethanesulfonyl)-piperidin-4-yl]-1,4-dihydrobenzo[d][1,3]oxazin-2-one |
| 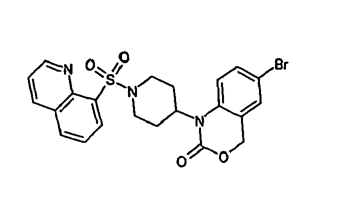 | 6-Bromo-1-[1-(quinoline-5-sulfonyl)-piperidin-4-yl]-1,4-dihydro-benzo[d][1,3]oxazin-2-one |
| 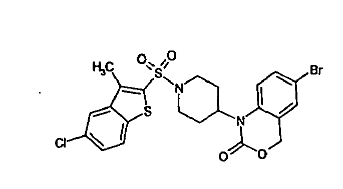 | 6-Bromo-1-[1-(5-chloro-3-methyl-benzo[b]thiophene-2-sulfonyl)-piperidin-4-yl]-1,4-dihydrobenzo[d][1,3]oxazin-2-one |
| 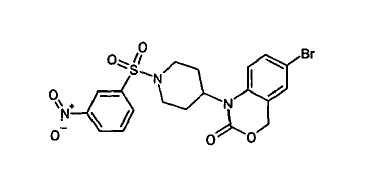 | 6-Bromo-1-[1-(3-nitrobenzenesulfonyl)-piperidin-4-yl]-1,4-dihydrobenzo[d][1,3]oxazin-2-one |
| 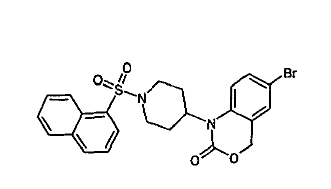 | 6-Bromo-1-[1-(naphthalene-1-sulfonyl)-piperidin-4-yl]-1,4-dihydro-benzo[d][1,3]oxazin-2-one |
| 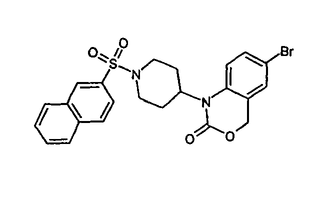 | 6-Bromo-1-[1-(naphthalene-2-sulfonyl)-piperidin-4-yl]-1,4-dihydro-benzo[d][1,3]oxazin-2-one |
| 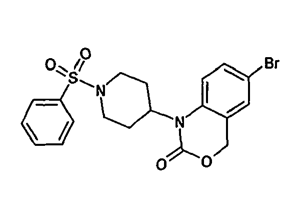 | 1-(1-Benzenesulfonylpiperidin-4-yl)-6-bromo-1,4-dihydro-benzo[d][1,3]oxazin-2-one |
| 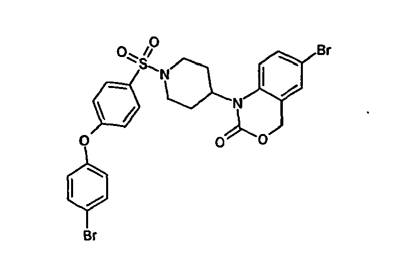 | 6-Bromo-1-{1-[4-(4-bromophenoxy)-benzenesulfonyl]-piperidin-4-yl}-1,4-dihydrobenzo[d][1,3]oxazin-2-one |
| 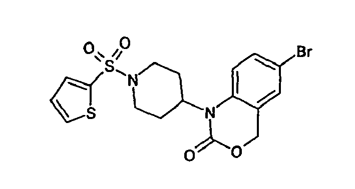 | 6-Bromo-1-[1-(thiophene-2-sulfonyl)-piperidin-4-yl]-1,4-dihydro-benzo[d][1,3]oxazin-2-one |
| 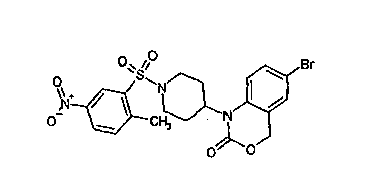 | 6-Bromo-1-[1-(2-methyl-5-nitro-benzenesulfonyl)-piperidin-4-yl]-1,4-dihydrobenzo[d][1,3]oxazin-2-one |
| 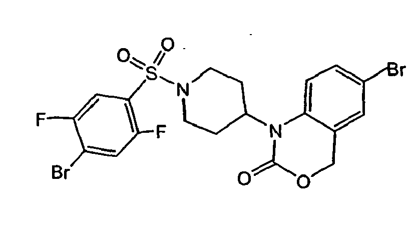 | 6-Bromo-1-[1-(4-bromo-2,5-difluoro-benzenesulfonyl)-piperidin-4-yl]-1,4-dihydrobenzo[d][1,3]oxazin-2-one |
| 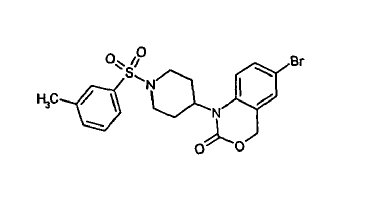 | 6-Bromo-1-[1-(toluene-3-sulfonyl)-piperidin-4-yl]-1,4-dihydro-benzo[d][1,3]oxazin-2-one |
| 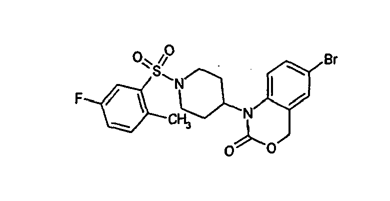 | 6-Bromo-1-[1-(5-fluoro-2-methyl-benzenesulfonyl)-piperidin-4-yl]-1,4-dihydrobenzo[d][1,3]oxazin-2-one |
| 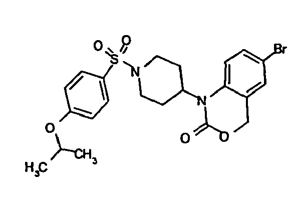 | 6-Bromo-1-[1-(4-isopropoxybenzenesulfonyl)-piperidin-4-yl]-1,4-dihydrobenzo[d][1,3]oxazin-2-one |
| 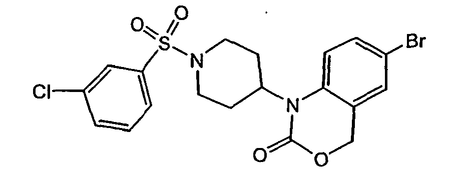 | 6-Bromo-1-[1-(3-chlorobenzenesulfonyl)-piperidin-4- yl]-1,4-dihydrobenzo[d][1,3]oxazin-2-one |
| 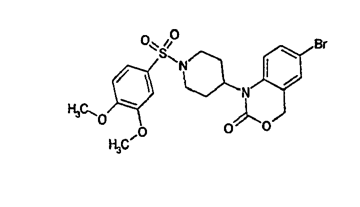 | 6-Bromo-1-[1-(3,4-dimethoxybenzenesulfonyl)-piperidin-4-yl]-1,4-dihydrobenzo[d][1,3]oxazin-2-one |
| 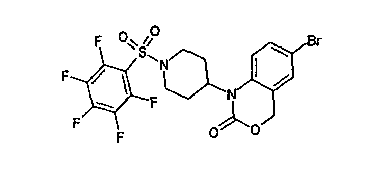 | 6-Bromo-1-(1-pentafluorobenzenesulfonylpiperidin-4-yl)-1,4-dihydrobenzo[d][1,3]oxazin-2-one |
| 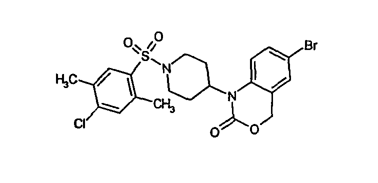 | 6-Bromo-1-[1-(4-chloro-2,5-dimethyl-benzenesulfonyl)-piperidin-4-yl]-1,4-dihydrobenzo[d][1,3]oxazin-2-one |
| 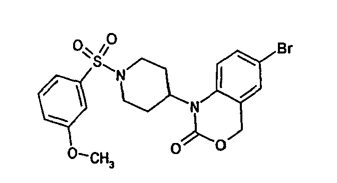 | 6-Bromo-1-[1-(3-methoxybenzenesulfonyl)-piperidin-4-yl]-1,4-dihydrobenzo[d][1,3]oxazin-2-one |
| 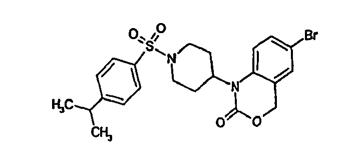 | 6-Bromo-1-[1-(4-isopropylbenzenesulfonyl)-piperidin-4-yl]-1,4-dihydrobenzo[d][1,3]oxazin-2-one |
| 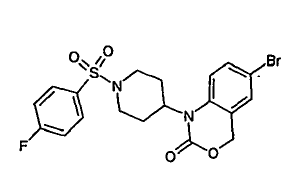 | 6-Bromo-1-[1-(4-fluorobenzenesulfonyl)-piperidin-4-yl]-1,4-dihydrobenzo[d][1,3]oxazin-2-one |
| 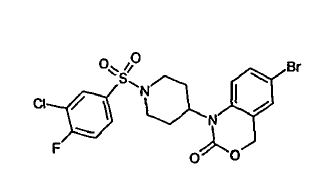 | 6-Bromo-1-[1-(3-chloro-4-fluoro-benzenesulfonyl)-piperidin-4-yl]-1,4-dihydrobenzo[d][1,3]oxazin-2-one |
| 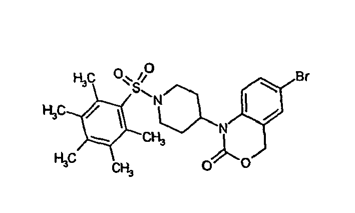 | 6-Bromo-1-(1-pentamethylbenzenesulfonylpiperidin-4-yl)-1,4-dihydrobenzo[d][1,3]oxazin-2-one |
| 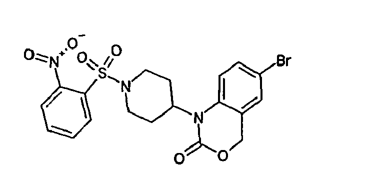 | 6-Bromo-1-[1-(2-nitrobenzenesulfonyl)-piperidin-4-yl]-1,4-dihydrobenzo[d][1,3]oxazin-2-one |
| 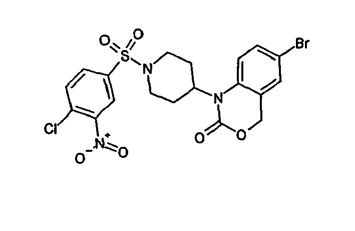 | 6-Bromo-1-[1-(4-chloro-3-nitro-benzenesulfonyl)-piperidin-4-yl]-1,4-dihydrobenzo[d][1,3]oxazin-2-one |
| 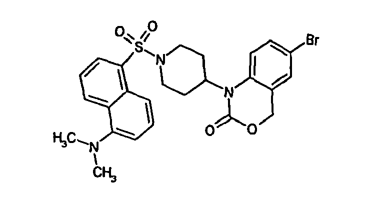 | 6-Bromo-1-[1-(5-dimethylamino-naphthalene-1-sulfonyl)-piperidin-4-yl]-1,4 dihydro-benzo[d][1,3]oxazin-2-one |
| 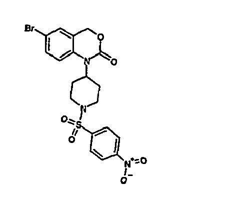 | 6-Bromo-1-[1-(4-nitrobenzenesulfonyl)-piperidin-4-yl]-1,4-dihydrobenzo[d][1,3]oxazin-2-one |
| 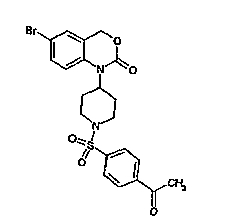 | 1-[1-(4-Acetylbenzenesulfonyl)-piperidin-4-yl]-6-bromo-1,4-dihydrobenzo[d][1,3]oxazin-2-one |
| 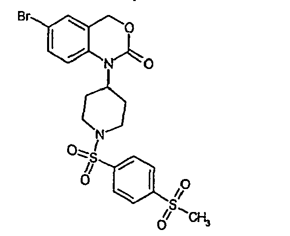 | 6-Bromo-1-[1-(4-methanesulfonylbenzenesulfonyl)-piperidin-4-yl]-1,4-dihydrobenzo[d][1,3]oxazin-2-one |
| 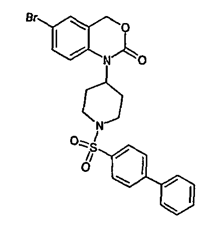 | 1-[1-(Biphenyl-4-sulfonyl)-piperidin-4-yl]-6-bromo-1,4-dihydro-benzo[d][1,3]oxazin-2-one |
| 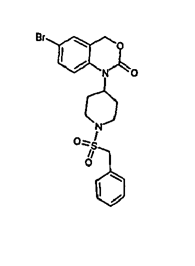 | 6-Bromo-1-(1-phenylmethanesulfonylpiperidin-4-yl)-1,4-dihydrobenzo[d][1,3]oxazin-2-one |
| 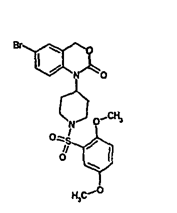 | 6-Bromo-1-[1-(2,5-dimethoxybenzenesulfonyl)-piperidin-4-yl]-1,4-dihydrobenzo[d][1,3]oxazin-2-one |
| 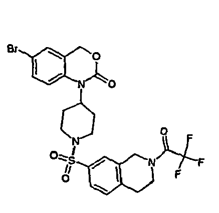 | 6-Bromo-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 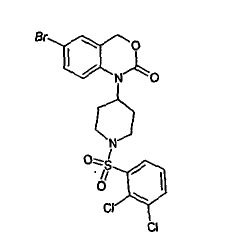 | 6-Bromo-1-[1-(2,3-dichlorobenzenesulfonyl)-piperidin-4-yl]-1,4-dihydrobenzo[d][1,3]oxazin-2-one |
| 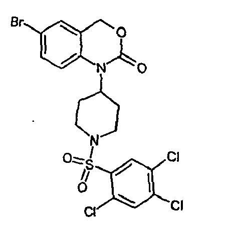 | 6-Bromo-1-[1-(2,4,5-trichlorobenzenesulfonyl)-piperidin-4-yl]-1,4-dihydrobenzo[d][1,3]oxazin-2-one |
| 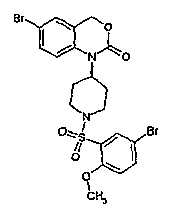 | 6-Bromo-1-[1-(5-promo-2-methoxy-benzenesulfonyl)-piperidin-4-yl]-1,4-dihydrobenzo[d][1,3]oxazin-2-one |
| 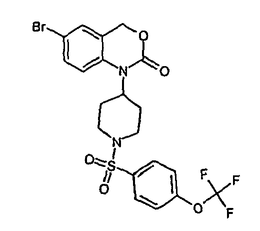 | 6-Bromo-1-[1-(4-trifluoromethoxybenzenesulfonyl)-piperidin-4-yl]-1,4-dihydrobenzo[d][1,3]oxazin-2-one |
| 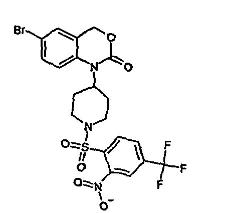 | 6-Bromo-1-[1-(2-nitro-4-trifluoromethylbenzenesulfonyl)-piperidin-4-yl]-1,4-dihydrobenzo[d][1,3]oxazin-2-one |
| 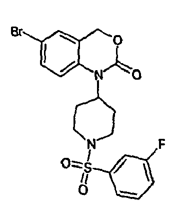 | 6-Bromo-1-[1-(3-fluorobenzenesulfonyl)-piperidin-4-yl]-1,4-dihydrobenzo[d][1,3]oxafin-2-one |
| 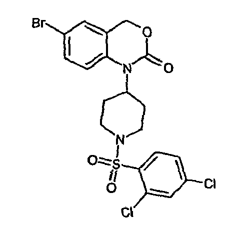 | 6-Bromo-1-[1-(2,4-dichlorobenzenesulfonyl)-piperidin-4-yl]-1,4-dihydrobenzo[d][1,3]oxazin-2-one |
| 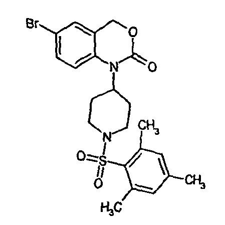 | 6-Bromo-1-[1-(2,4,6-trimethylbenzenesulfonyl)-piperidin-4-yl]-1,4-dihydrobenzo[d][1,3]oxazin-2-one |
| 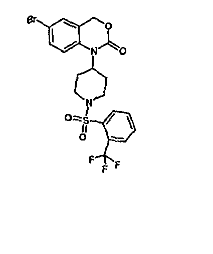 | 6-Bromo-1-[1-(2-trifluoromethylbenzenesulfonyl)-piperidin-4-yl]-1,4-dihydrobenzo[d][1,3]oxazin-2-one |
| 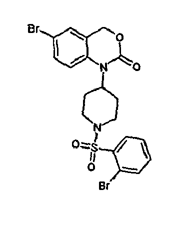 | 6-Bromo-1-[1-(2-bromobenzenesulfonyl)-piperidin-4-yl]-1,4-dihydro-benzo[d][1,3]oxazin-2-one |
| 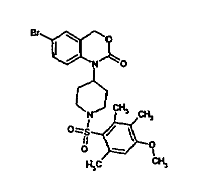 | 6-Bromo-1-[1-(4-methoxy-2,3,6-trimethylbenzenesulfonyl)-piperidin-4-yl]-1,4-dihydrobenzo[d][1,3]oxazin-2-one |
| 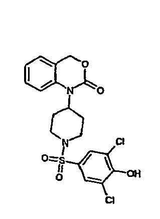 | 1-[1-(3,5-Dichloro-4-hydroxybenzenesulfonyl)-piperidin-4-yl]-1,4-dihydrobenzo[d][1,3]oxazin-2-one |
| 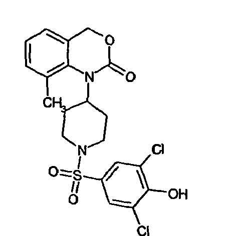 | 1-[1-(3,5-Dichloro-4-hydroxybenzenesulfonyl)-piperidin-4-yl]-8-methyl-1,4-dihydrobenzo[d][1,3]oxazin-2-one |
| 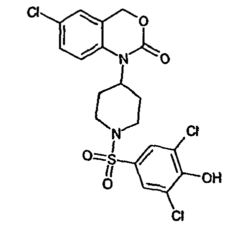 | 6-Chloro-1-[1-(3,5-dichloro-4-hydroxy-benzenesulfonyl)-piperidin-4-yl]-1,4-dihydrobenzo[d][1,3]oxazin-2-one |
| 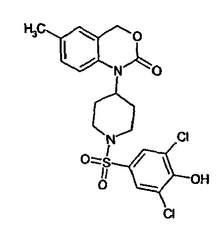 | 1-[1-(3,5-Dichloro-4-hydroxybenzenesulfonyl)-piperidin-4-yl]-6-methyl-1,4-dihydrobenzo[d][1,3]oxazin-2-one |
| 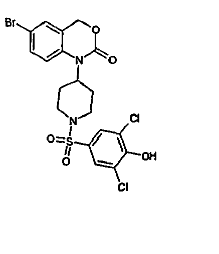 | 6-Bromo-1-[1-(3,5-dichloro-4-hydroxy-benzenesulfonyl)-piperidin-4-yl]-1,4-dihydrobenzo[d][1,3]oxazin-2-one |
| 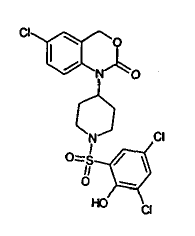 | 6-Chloro-1-[1-(3,5-dichloro-2-hydroxy-benzenesulfonyl)-piperidin-4-yl]-1,4-dihydrobenzo[d][1,3]oxazin-2-one |
| 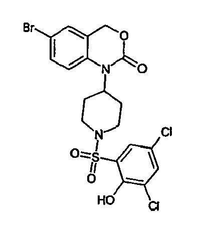 | 6-Bromo-1-[1-(3,5-dichloro-2-hydroxy-benzenesulfonyl)-piperidin-4-yl]-1,4-dihydrobenzo[d][1,3]oxazin-2-one |
| 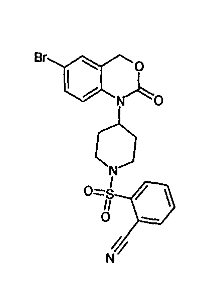 | 2-[4-(6-Bromo-2-oxo-4H-benzo[d][1,3]oxazin-1-yl)-piperidine-1-sulfonyl]-benzonitrile |
| 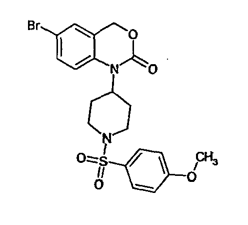 | 6-Bromo-1-[1-(4-methoxybenzenesulfonyl)-piperidin-4-yl]-1,4-dihydrobenzo[d][1,3]oxazin-2-one |
| 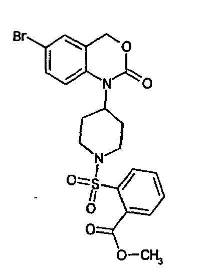 | 2-[4-(6-Bromo-2-oxo-4H-benzo[d][1,3]oxazin-1-yl)-piperidine-1-sulfonyl]-benzoic acid methyl ester |
| 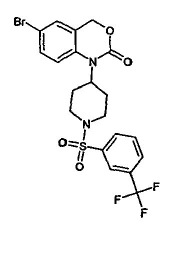 | 6-Bromo-1-[1-(2-oxo-2H-chromene-6-sulfonyl)-piperidin-4-yl]-1,4-dihydrobenzo[d][1,3]oxazin-2-one |
| 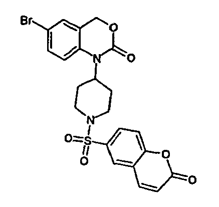 | 6-Bromo-1-[1-(3,5-dichlorobenzenesulfonyl)-piperidin-4-yl]-1,4-dihydrobenzo[d][1,3]oxazin-2-one |
| 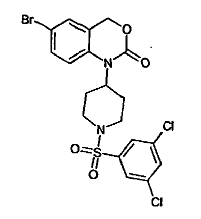 | 6-Bromo-1-[1-(2,5-dichlorobenzenesulfonyl)-piperidin-4-yl]-1,4-dihydrobenzo[d][1,3]oxazin-2-one |
| 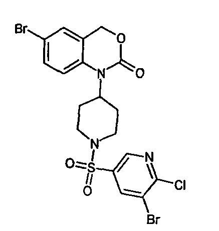 | 6-Bromo-1-[1-(5-bromo-6-chloro-pyridine-3-sulfonyl)-piperidin-4-yl]-1,4-dihydrobenzo[d][1,3]oxazin-2-one |
| 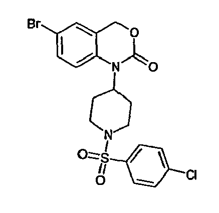 | 6-Bromo-1-[1-(4-chlorobenzenesulfonyl)-piperidin-4-yl]-1,4-dihyd robenzo[d][1,3]oxazin-2-one |
| 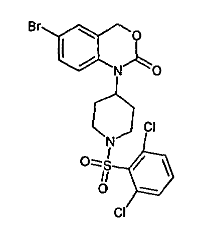 | 6-Bromo-1-[1-(2,6-dichlorobenzenesulfonyl)-piperidin-4-yl]-1,4-dihydrobenzo[d][1,3]oxazin-2-one |
| 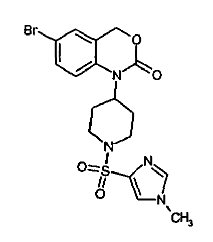 | 6-Bromo-1-[1-(1-methyl-1H-imidazole-4-sulfonyl)-piperidin-4-yl]-1,4-dihydro-benzo[d][1,3]oxazin-2-one |
| 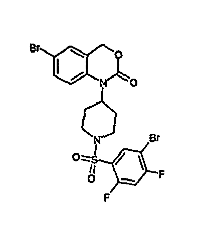 | 6-Bromo-1-[1-(5-bromo-2,4-difluorobenzenesulfonyl)-piperidin-4-yl]-1,4-dihydrobenzo[d][1,3]oxacin-2-one |

Also particularly preferred is the use of one or more benzoxazinone-derived sulfonamide compounds of general formula (lb) selected from the group consisting of:

| **No** | **Compound** |
|---|---|
| 1 | 1-[1-(Naphthalene-2-sulfonyl)-piperidin-4-yl]-1,4-dihydro-benzo[d][1,3]oxazin-2-one |
| 2 | 1-[1-(Toluene-4-sulfonyl)-piperidin-4-yl]-1,4-dihydro-benzo[d][1,3]oxazin-2-one |
| 3 | 1-(1-Phenylmethanesulfonyl-piperidin-4-yl)-1,4-dihydro-benzo[d][1,3]oxazin-2-one |
| 4 | 1-(1-Benzenesulfonyl-piperidin-4-yl)-6-chloro-1,4-dihydro-benzo[d][1,3]oxazin-2-one |
| 5 | 6-Chloro-1-[1-(toluene-4-sulfonyl)-piperidin-4-yl]-1,4-dihydro-benzo[d][1,3]oxazin-2-one |
| 6 | 6-Chloro-1-(1-phenylmethanesulfonyl-piperidin-4-yl)-1,4-dihydro-benzo[d][1,3]oxazin-2-one |
| 7 | 6-Chloro-1-[1-(naphthalene-1-sulfonyl)-piperidin-4-yl]-1,4-dihydro-benzo[d][1,3]oxazin-2-one |
| 8 | 6-Chloro-1-[1-(naphthalene-2-sulfonyl)-piperidin-4-yl]-1,4-dihydro-benzo[d][1,3]oxazin-2-one |
| 9 | 6-Chloro-1-[1-(5-chloro-3-methyl-benzo[b]thiophene-2-sulfonyl)-piperidin-4-yl]-1,4-dihydro-benzo[d][1,3]oxazin-2-one |
| 10 | 1-[1-(Thiophene-2-sulfonyl)-piperidin-4-yl]-1,4-dihydro-benzo[d][1,3]oxazin-2-one |
| 11 | 1-[1-(4-Acetyl-benzenesulfonyl)-piperidin-4-yl]-1,4-dihydro-benzo[d][1,3]oxazin-2-one |
| 12 | 2-[4-(2-Oxo-4H-benzo[d][1,3]oxazin-1-yl)-piperidine-1-sulfonyl]-benzonitrile |
| 13 | 1-[1-(2,4-Dimethyl-benzenesulfonyl)-piperidin-4-yl]-1,4-dihydro-benzo[d][1,3]oxazin-2-one |
| 14 | 1-[1-(4-Methoxy-benzenesulfonyl)-pipefidin-4-yl]-1,4-dihydro-benzo[d][1,3]oxazin-2-one |
| 15 | 1-[1-(2-Naphthaten-1-yl-ethanesulfonyl)-piperidin-4-yl]-1,4-dihydro-benzo[d][1,3]oxazin-2-one |
| 16 | 8-Methyl-1-[1-(thiophene-2-sulfonyl)-piperidin-4-yl]-1,4-dihydro-benzo[d][1,3]oxazin-2-one |
| 17 | 1-[1-(4-Acetyl-benzenesulfonyl)-piperidin-4-yl]-8-methyl-1,4-dihydro-benzo[d][1,3]oxazin-2-one |
| 18 | 2-[4-(8-Methyl-2-oxo-4H-benzo[d][1,3]oxazin-1-yl)-piperidine-1-sulfonyl]-benzonitrile |
| 19 | 1-[1-(2,4-Dimethyl-benzenesulfonyl)-piperidin-4-yl]-8-methyl-1,4-dihydro-benzo[d][1,3]oxazin-2-one |
| 20 | 1-[1-(4-Methoxy-benzenesulfonyl)-piperidin-4-yl]-8-methyl-1,4-dihydro-benzo[d][1,3]oxazin-2-one |
| 21 | 8-Methyl-1-[1-(2-naphthalen-1-yl-ethanesulfonyl)-piperidin-4-yl]-1,4-dihydro-benzo[d][1,3]oxazin-2-one |
| 22 | 4-(8-Methyl-2-oxo-4H-benzo[d][1,3]oxazin-1-yl)-piperidine-1-sulfonic acid dimethylamide |
| 23 | 2-[4-(2-Oxo-4H-benzo[d][1,3]oxazin-1-yl)-piperidine-1-sulfonyl]-benzoic acid methyl ester |
| 24 | 1-[1-(3-Trifluoromethyl-benzenesulfonyl)-piperidin-4-yl]-1,4-dihydro-benzo[d][1,3]oxazin-2-one |
| 25 | 2-[4-(8-Methyl-2-oxo-4H-benzo[d][1,3]oxazin-1-yl)-piperidine-1-sulfonyl]-benzoic acid methyl ester |
| 26 | 8-Methyl-1-[-(3-trifluoromethyl-benzenesulfonyl)-piperidin-4-yl]-1,4-dihydro-benzo[d][1,3]oxazin-2-one |
| 27 | 1-[1-(4-Acetyl-benzenesulfonyl)-piperidin-4-yl]-6-chloro-1,4-dihydro-benzo[d][1,3]oxazin-2-one |
| 28 | 2-[4-(6-Chloro-2-oxo-4H-benzo[d][1,3]oxazin-1-yl)-piperidine-1-sulfonyl]-benzonitrile |
| 29 | 6-Chloro-1-[1-(4-methoxy-benzenesulfonyl)-piperidin-4-yl]-1,4-dihydro-benzo[d][1,3]oxazin-2-one |
| 30 | 2-[4-(6-Chloro-2-oxo-4H-benzo[d][1,3]oxazin-1-yl)-piperidine-1-sulfonyl]-benzoic acid methyl ester |
| 31 | 6-Chtoro-1-[1-(2,4-dimethyl-benzenesulfonyl)-piperidin-4-yl]-1,4-dihydro-benzo[d][1,3]oxazin-2-one |
| 32 | 6-Chloro-1-[1-(3-trifluoromethyl-benzenesulfonyl)-piperidin-4-yl]-1,4-dihydro-benzo[d][1,3]oxazin-2-one |
| 33 | 1-[1-(5-Chloro-3-methyl-benzo[b]thiophene-2-sulfonyl)-piperidin-4-yl]-8-methyl-1,4-dihydro-benzo[d][1,3]oxazin-2-one |
| 34 | 1-{1-[4-(4-Bromo-phenoxy)-benzenesulfonyl]-piperidin-4-yl}-8-methyl-1,4-dihydro-benzo[d][1,3]oxazin-2-one |
| 35 | 1-[1-(4-Fluoro-benzenesulfonyl)-piperidin-4-yl]-8-methyl-1,4-dihydro-benzo[d][1,3]oxazin-2-one |
| 36 | 8-Methyl-1-[1-(naphthalene-2-sulfonyl)-piperidin-4-yl]-1,4-dihydro-benzo[d][1,3]oxazin-2-one |
| 37 | 8-Methyl-1-(1-phenylmethanesulfonyl-piperidin-4-yl)-1,4-dihydro-benzo[d][1,3]oxazin-2-one |
| 38 | 1-[1-(4-Bromo-benzenesulfonyl)-piperidin-4-yl]-6-chloro-1,4-dihydro-benzo[d][1,3]oxazin-2-one |
| 39 | 6-Chloro-1-[1-(4-methanesulfonyl-benzenesulfonyl)-piperidin-4-yl]-1,4-dihydro-benzo[d][1,3]oxazin-2-one |
| 40 | 1-[1-(Butane-1-sulfonyl)-piperidin-4-yl]-6-chloro-1,4-dihydro-benzo[d][1,3]oxazin-2-one |
| 41 | 1-[1-(4-Bromo-benzenesulfonyl)-piperidin-4-yl]-8-methyl-1,4-dihydro-benzo[d][1,3]oxazin-2-one |
| 42 | 1-[1-(4-Methanesulfonyl-benzenesulfonyl)-piperidin-4-yl]-8-methyl-1,4-dihydro-benzo[d][1,3]oxazin-2-one |
| 43 | 1-[1-(Butane-1-sulfonyl)-piperidin-4-yl]-8-methyl-1,4-dihydro-benzo[d][1,3]oxazin-2-one |
| 44 | 6-Chloro-1-[1-(2-nitro-benzenesulfonyl)-piperidin-4-yl]-1,4-dihydro-benzo[d][1,3]oxazin-2-one |
| 45 | 6-Chloro-1-[1-(3-nitro-benzenesulfonyl)-piperidin-4-yl]-1,4-dihydro-benzo[d][1,3]oxazin-2-one |
| 46 | 1-[1-(Biphenyl-4-sulfonyl)-piperidin-4-yl]-6-chloro-1,4-dihydro-benzo[d][1,3]oxazin-2-one |
| 47 | 8-Methy)-1-[1-(2-nitro-benzenesulfonyl)-piperidin-4-yl]-1,4-dihydro-benzo[d][1,3]oxazin-2-one |
| 48 | 8-Methyl-1-[1-(3-nitro-benzenesulfonyl)-piperidin-4-yl]-1,4-dihydro-benzo[d][1,3]oxazin-2-one |
| 49 | 1-[1-(Biphenyl-4-sulfonyl)-piperidin-4-yl]-8-methyl-1,4-dihydro-benzo[d][1,3]oxazin-2-one |
| 50 | 8-Methyl-1-[1-(4-nitro-benzenesulfonyl)-piperidin-4-yl]-1,4-dihydro-benzo[d][1,3]oxazin-2-one |
| 51 | 6-Chloro-1-[1-(4-nitro-benzenesulfonyl)-piperidin-4-yl]-1,4-dihydro-benzo[d][1,3]oxazin-2-one |
| 52 | 1-(1-Ethanesulfonyl-piperidin-4-yl)-1,4-dihydro-benzo[d][1,3]oxazin-2-one |
| 53 | 1-[1-(Propane-1-sulfonyl)-piperidin-4-yl]-1,4-dihydro-benzo[d][1,3]oxazin-2-one |
| 54 | 1-[1-(Propane-2-sulfonyl)-piperidin-4-yl]-1,4-dihydro-benzo[d][1,3]oxazin-2-one |
| 55 | 6-Chloro-1-(1-ethanesulfonyl-piperidin-4-yl)-1,4-dihydro-benzo[d][1,3]oxazin-2-one |
| 56 | 6-Chloro-1-[1-(propane-1-sulfonyl)-piperidin-4-yl]-1,4-dihydro-benzo[d][1,3]oxazin-2-one |
| 57 | 6-Chloro-1-[1-(propane-2-sulfonyl)-piperidin-4-yl]-1,4-dihydro-benzo[d][1,3]oxazin-2-one |
| 58 | 6-Chloro-1-[1-(quinoline-8-sulfonyl)-piperidin-4-yl]-1,4-dihydro-benzo[d][1,3]oxazin-2-one |
| 59 | 1-[1-(4-Nitro-benzenesulfonyl)-piperidin-4-yl]-1,4-dihydro-benzo[d][1,3]oxazin-2-one |
| 60 | 6-Methyl-1-[1-(quinoline-8-sulfonyl)-piperidin-4-yl]-1,4-dihydro-benzo[d][1,3]oxazin-2-one |
| 61 | 6-Methyl-1-[1-(2-naphthalen-1-yl-ethanesulfonyl)-piperidin-4-yl]-1,4-dihydro-benzo[d][1,3]oxazin-2-one |
| 62 | 6-Methyl-1-[1-(toluene-4-sulfonyl)-piperidin-4-yl]-1,4-dihydro-benzo[d][1,3]oxazin-2-one |
| 63 | 1-[1-(4-Fluoro-benzenesulfonyl)-piperidin-4-yl]-6-methyl-1,4-dihydro-benzo[d][1,3]oxazin-2-one |
| 64 | 6-Methyl-1-[1-(naphthalene-1-sulfonyl)-piperidin-4-yl]-1,4-dihydro-benzo[d][1,3]oxazin-2-one |
| 65 | 6-Methyl-1-[1-(naphthalene-2-sulfonyl)-piperidin-4-yl]-1,4-dihydro-benzo[d][1,3]oxazin-2-one |
| 66 | 1-[1₋(5-Chloro-3-methyl-benzo[b]thiophene-2-sulfonyl)-piperidin-4-yl]-6-methyl-1,4-dihydro-benzo[d][1,3]oxazin-2-one |
| 67 | 6-Methyl-1-[1-(4-nitro-benzenesulfonyl)-piperidin-4-yl]-1,4-dihydro-benzo[d][1,3]oxazin-2-one |
| 68 | 1-(1-Benzenesulfonyl-piperidin-4-yl)-6-methyl-1,4-dihydro-benzo[d][1,3]oxazin-2-one |
| 69 | 1-[1-(4-Chloro-3-nitro-benzenesulfonyl)-piperidin-4-yl]-6-methyl-1,4-dihydro-benzo[d][1,3]oxazin-2-one |
| 70 | 1-[1-(5-Dimethylamino-naphthalene-1-sulfonyl)-piperidin-4-yl]-6-methyl-1,4-dihydro-benzo[d][1,3]oxazin-2-one |
| 71 | 1-[1-(4-Chloro-3-nitro-benzenesulfonyl)-piperidin-4-yl]-8-methyl-1,4-dihydro-benzo[d][1,3]oxazin-2-one |
| 72 | 1-[-(4-Chloro-3-nitro-benzenesulfonyl)-piperidin-4-yl]-1,4-dihydro-benzo[d][1,3]oxazin-2-one |
| 73 | 6-Chloro-1-[1-(4-chloro-3-nitro-benzenesulfonyl)-piperidin-4-yl]-1,4-dihydro-benzo[d][1,3]oxazin-2-one |
| 74 | 6-Chloro-1-[1-(5-dimethylamino-naphthalene-1-sulfonyl)-piperidin-4-yl]-1,4-dihydro-benzo[d][1,3]oxazin-2-one |
| | |
| 75 | 1-[1-(4-Methoxy-2,3,6-trimethyl-benzenesulfonyl)-piperidin-4-yl]-1,4-dihydro-benzo[d][1,3]oxazin-2-one |
| 76 | 1-[1-(4-Methoxy-2,3,6-trimethyl-benzenesulfonyl)-piperidin-4-yl]-8-methyl-1,4-dihydro-benzo[d][1,3]oxazin-2-one |
| 77 | 6-Chloro-1-[1-(4-methoxy-2,3,6-trimethyl-benzenesulfonyl)-piperidin-4-yl]-1,4-dihydro-benzo[d][1,3]oxazin-2-one |
| 78 | 1-[1-(4-Methoxy-2,3,6-trimethyl-benzenesulfonyl)-piperidin-4-yl]-6-methyl-1,4-dihydro-benzo[d][1,3]oxazin-2-one |
| 79 | 1-[1-(2-Bromo-benzenesulfonyl)-piperidin-4-yl]-1,4-dihydro-benzo[d][1,3]oxazin-2-one |
| 80 | 1-[1-(2-Bromo-benzenesulfonyl)-piperidin-4-yl]-8-methyl-1,4-dihydro-benzo[d][1,3]oxazin-2-one |
| 81 | 1-[1-(2-Bromo-benzenesulfonyl)-piperidin-4-yl]-6-chloro-1,4-dihydro-benzo[d][1,3]oxazin-2-one |
| 82 | 1-[1-(2-Bromo-benzenesulfonyl)-piperidin-4-yl]-6-methyl-1,4-dihydro-benzo[d][1,3]oxazin-2-one |
| 83 | 6-Chloro-1-[1-(2,3-dichloro-benzenesulfonyl)-piperidin-4-yl]-1,4-dihydro-benzo[d][1,3]oxazin-2-one |
| 84 | 1-[1-(2,3-Dichloro-benzenesulfonyl)-piperidin-4-yl]-6-methyl-1,4-dihydro-benzo[d][1,3]oxazin-2-one |
| 85 | 1-[1-(2,4,5-Trichloro-benzenesulfonyl)-piperidin-4-yl]-1,4-dihydro-benzo[d][1,3]oxazin-2-one |
| 86 | 8-Methyl-1-[1-(2,4,5-trichloro-benzenesulfonyl)-piperidin-4-yl]-1,4-dihydro-benzo[d][1,3]oxazin-2-one |
| 87 | 6-Chloro-1-[1-(2,4,5-trichloro-benzenesulfonyl)-piperidin-4-yl]-1,4-dihydro-benzo[d][1,3]oxazin-2-one |
| 88 | 6-Methyl-1-[1-(2,4,5-trichloro-benzenesulfonyl)-piperidin-4-yl]-1,4-dihydro-benzo[d][1,3]oxazin-2-one |
| 89 | 1-[1-(5-Bromo-2-methoxy-benzenesulfonyl)-piperidin-4-yl]-1,4-dihydro-benzo[d][1,3]oxazin-2-one |
| 90 | 1-[1-(5-Bromo-2-methoxy-benzenesulfonyl)-pipeidin-4-yl]-8-methyl-1,4-dihydro-benzo[d][1,3]oxazin-2-one |
| 91 | 1-[1-(5-Bromo-2-methoxy-benzenesulfonyl)-piperidin-4-yl]-6-chloro-1,4-dihydro-benzo[d][1,3]oxazin-2-one |
| 92 | 1-[1-(5-Bromo-2-methoxy-benzenesulfonyl)-piperidin-4-yl]-6-methyl-1,4-dihydro-benzo[d][1,3]oxazin-2-one |
| 93 | 1-[1-(2,5-Dimethoxy-benzenesulfonyl)-piperidin-4-yl]-1,4-dihydro-benzo[d][1,3]oxazin-2-one |
| 94 | 1-[1-(2,5-Dimethoxy-benzenesulfonyl)-piperidin-4-yl]-8-methyl-1,4-dihydro-benzo[d][1,3]oxazin-2-one |
| 95 | 6-Chloro-1-[1-(2,5-dimethoxy-benzenesulfonyl)-piperidin-4-yl]-1,4-dihydro-benzo[d][1,3]oxazin-2-one |
| 96 | 1-[1-(2,5-Dimethoxy-benzenesulfonyl)-piperidin-4-yl]-6-methyl-1,4-dihydro-benzo[d][1,3]oxazin-2-one |
| 97 | 1-(1-Pentamethylbenzenesulfonyl-piperidin-4-yl)-1,4-dihydro-benzo[d][1,3]oxazin-2-one |
| 98 | 8-Methy)-1-(1-pentamethylbenzenesulfonyl-piperidin-4-yl)-1,4-dihydro-benzo[d][1,3]oxazin-2-one |
| 99 | 6-Chloro-1-(1-pentamethylbenzenesulfonyl-piperidin-4-yl)-1,4-dihydro-benzo[d][1,3]oxazin-2-one |
| 100 | 6-Methy)-1-(1-pentamethylbenzenesulfonyl-piperidin-4-yl)-1,4-dihydro-benzo[d][1,3]oxazin-2-one |
| 101 | 1-{1-[2-(2,2,2-Trifluoro-acetyl)-1,2,3,4-tetrahydro-isoquinoline-7-sulfonyl]-piperidin-4-yl}-1,4-dihydro-benzo[d][1,3]oxazin-2-one |
| 102 | 8-Methyl-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 103 | 6-Chloro-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 104 | 6-Methyl-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 105 | 1-[1-(2-Methyl-5-nitro-benzenesulfonyl)-piperidin-4-yl]-1,4-dihydro-benzo[d][1,3]oxazin-2-one |
| 106 | 8-Methyl-1-[1-(2-methyl-5-nitro-benzenesulfonyl)-piperidin-4-yl]-1,4-dihydro-benzo[d][1,3]oxazin-2-one |
| 107 | 6-Chloro-1-[1-(2-methyl-5-nitro-benzenesulfonyl)-piperidin-4-yl]-1,4-dihydro-benzo[d][1,3]oxazin-2-one |
| 108 | 6-Methyl-1-[1-(2-methyl-5-nitro-benzenesulfonyl)-piperidin-4-yl]-1,4-dihydro-benzo[d][1,3]oxazin-2-one |
| 109 | 1-[1-(4-Bromo-2,5-difluoro-benzenesulfonyl)-piperidin-4-yl]-1,4-dihydro-benzo[d][1,3]oxazin-2-one |
| 110 | 1-[1-(4-Bromo-2,5-difluoro-benzenesulfonyl)-piperidin-4-yl]-8-methyl-1,4-dihydro-benzo[d][1,3]oxazin-2-one |
| 111 | 1-[1-(4-Bromo-2,5-difluoro-benzenesulfonyl)-piperidin-4-yl]-6-chloro-1,4-dihydro-benzo[d][1,3]oxazin-2-one |
| 112 | 1-[1-(4-Bromo-2,5-difluoro-benzenesulfonyl)-piperidin-4-yl]-6-methyl-1,4-dihydro-benzo[d][1,3]oxazin-2-one |
| 113 | 1-[1-(4-Chloro-2,5-dimethyl-benzenesulfonyl)-piperidin-4-yl]-1,4-dihydro-benzo[d][1,3]oxazin-2-one |
| 114 | 1-[1-(4-Chloro-2,5-dimethyl-benzenesulfonyl)-piperidin-4-yl]-8-methyl-1,4-dihydro-benzo[d][1,3]oxazin-2-one |
| 115 | 6-Chloro-1-[1-(4-chloro-2,5-dimethyl-benzenesulfonyl)-piperidin-4-yl]-1,4-dihydro-benzo[d][1,3]oxazin-2-one |
| 116 | 1-[1-(4-Chloro-2,5-dimethyl-benzenesulfonyl)-pipendin-4-yl]-6-methyl-1,4-dihydro-benzo[d][1,3]oxazin-2-one |
| 117 | 1-[1-(4-Methoxy-benzenesulfonyl)-piperidin-4-yl]-6-methyl-1,4-dihydro-benzo[d][1,3]oxazin-2-one |
| 118 | 1-[1-(4-Isopropyl-benzenesulfonyl)-piperidin-4-yl]-1,4-dihydro-benzo[d][1,3]oxazin-2-one |
| 119 | 1-[1-(4-Isopropyl-benzenesulfonyl)-piperidin-4-yl]-8-methyl-1,4-dihydro-benzo[d][1,3]oxazin-2-one |
| 120 | 6-Chloro-1-[1-(4-isopropyl-benzenesulfonyl)-piperidin-4-yl]-1,4-dihydro-benzo[d][1,3]oxazin-2-one |
| 121 | 1-[1-(4-Isopropyl-benzenesulfonyl)-piperidin-4-yl]-6-methyl-1,4-dihydro-benzo[d][1,3]oxazin-2-one |
| 122 | 1-[1-(3-Chloro-4-fluoro-benzenesulfonyl)-piperidin-4-yl]-1,4-dihydro-benzo[d][1,3]oxazin-2-one |
| 123 | 1-[1-(3-Chloro-4-fluoro-benzenesulfonyl)piperidin-4-yl]-8-methyl-1,4-dihydro-benzo[d][1,3]oxazin-2-one |
| 124 | 6-Chloro-1-[1-(3-chloro-4-fluoro-benzenesulfonyl)-piperidin-4-yl]-1,4-dihydro-benzo[d][1,3]oxazin-2-one |
| 125 | 1-[1-(3-Chloro-4-fluoro-benzenesulfonyl)-piperidin-4-yl]-6-methyl-1,4-dihydro-benzo[d][1,3]oxazin-2-one |
| 126 | 1-[1-(4-Bromo-benzenesulfonyl)-piperidin-4-yl]-6-methyl-1,4-dihydro-benzo[d][1,3]oxazin-2-one |
| 127 | 6-Methyl-1-[1-(3-nitro-benzenesulfonyl)-piperidin-4-yl]-1,4-dihydro-benzo[d][1,3]oxazin-2-one |
| 128 | 6-Methyl-1-[1-(3-trifluoromethyl-benzenesulfonyl)-piperidin-4-yl]-1,4-dihydro-benzo[d][1,3]oxazin-2-one |
| 129 | 1-[1-(4-Trifluoromethoxy-benzenesulfonyl)-piperidin-4-yl]-1,4-dihydro-benzo[d][1,3]oxazin-2-one |
| 130 | 1-[1-(2-Nitro-4-trifluoromethyl-benzenesulfonyl)-piperidin-4-yl]-1,4-dihydro-benzo[d][1,3]oxazin-2-one |
| 131 | 1-[1-(3-Fluoro-benzenesulfonyl)-piperidin-4-yl]-1,4-dihydro-benzo[d][1,3]oxazin-2-one |
| 132 | 1-[1-(2,4-Dichloro-benzenesulfonyl)-piperidin-4-yl]-1,4-dihydro-benzo[d][1,3]oxazin-2-one |
| 133 | 1-[1-(2,4,6-Trimethyl-benzenesulfonyl)-piperidin-4-yl]-1,4-dihydro-benzo[d][1,3]oxazin-2-one |
| 134 | 1-[1-(2-Trifluoromethyl-benzenesulfonyl)-piperidin-4-yl]-1,4-dihydro-benzo[d][1,3]oxazin-2-one |
| 135 | 8-Methyl-1-[1-(4-trifluoromethoxy-benzenesulfonyl)-piperidin-4-yl]-1,4-dihydro-benzo[d][1,3]oxazin-2-one |
| 136 | 8-Methyl-1-[1-(2-nitro-4-trifluoromethyl-benzenesulfonyl)-piperidin-4-yl]-1,4-dihydro-benzo[d][1,3]oxazin-2-one |
| 137 | 1-[1-(3-Fluoro-benzenesulfonyl)-piperidin-4-yl]-8-methyl-1,4-dihydro-benzo[d][1,3]oxazin-2-one |
| 138 | 1-[1-(2,4-Dichloro-benzenesulfonyl)-piperidin-4-yl]-8-methyl-1,4-dihydro-benzo[d][1,3]oxazin-2-one |
| 139 | 8-Methyl-1-[1-(2,4,6-trimethyl-benzenesulfonyl)-piperidin-4-yl]-1,4-dihydro-benzo[d][1,3]oxazin-2-one |
| 140 | 8-Methyl-1-[1-(2-trifluoromethyl-benzenesulfonyl)-piperidin-4-yl]-1,4-dihydro-benzo[d][1,3]oxazin-2-one |
| 141 | 1-[1-(4-Fluoro-benzenesulfonyl)-piperidin-4-yl]-1,4-dihydro-benzo[d][1,3]oxazin-2-one |
| 142 | 1-[1-(4-Bromo-benzenesulfonyl)-piperidin-4-yl]-1,4-dihydro-benzo[d][1,3]oxazin-2-one |
| 143 | 1-[1-(3-Nitro-benzenesulfonyl)-piperidin-4-yl]-1,4-dihydro-benzo[d][1,3]oxazin-2-one |
| 144 | 1-{1-[4-(4-Bromo-phenoxy)-benzenesulfonyl]-piperidin-4-yl}-1,4-dihydro-benzo[d][1,3]oxazin-2-one |
| 145 | 1-[1-(3-Methoxy-benzenesulfonyl)-piperidin-4-yl]-1,4-dihydro-benzo[d][1,3]oxazin-2-one |
| 146 | 1-[1-(2-Nitro-benzenesulfonyl)-piperidin-4-yl]-1,4-dihydro-benzo[d][1,3]oxazin-2-one |
| 147 | 8-Methyl-1-[1-(toluene-4-sulfonyl)-piperidin-4-yl]-1,4-dihydro-benzo[d][1,3]oxazin-2-one |
| 148 | 1-(1-Benzenesulfonyl-piperidin-4-yl)-8-methyl-1,4-dihydro-benzo[d][1,3]oxazin-2-one |
| 149 | 1-[1-(3-Methoxy-benzenesulfonyl)-piperidin-4-yl]-8-methyl-1,4-dihydro-benzo[d][1,3]oxazin-2-one |
| 150 | 1-[1-(2,4-Dimethyl-benzenesulfonyl)-piperidin-4-yl]-6-methyl-1,4-dihydro-benzo[d][1,3]oxazin-2-one |
| 151 | 1-{1-[4-(4-Bromo-phenoxy)-benzenesulfonyl]-piperidin-4-yl)-6-methyl-1,4-dihydro-benzo[d][1,3]oxazin-2-one |
| 152 | 6-Methyl-1-[1-(thiophene-2-sulfonyl)-piperidin-4-yl]-1,4-dihydro-benzo[d][1,3]oxazin-2-one |
| 153 | 1-[1-(Toluene-3-sulfonyl)-piperidin-4-yl]-1,4-dihydro-benzo[d][1,3]oxazin-2-one |
| 154 | 1-[1-(5-Fluoro-2-methyl-benzenesulfonyl)-piperidin-4-yl]-1,4-dihydro-benzo[d][1,3]oxazin-2-one |
| 155 | 1-[1-(4-Isopropoxy-benzenesulfonyl)-piperidin-4-yl]-1,4-dihydro-benzo[d][1,3]oxazin-2-one |
| 156 | 1-[1-(3-Chloro-benzenesulfonyl)-piperidin-4-yl]-1,4-dihydro-benzo[d][1,3]oxazin-2-one |
| 157 | 1-[1-(3,4-Dimethoxy-benzenesulfonyl)-piperidin-4-yl]-1,4-dihydro-benzo[d][1,3]oxazin-2-one |
| 158 | 1-(1-Pentafluorobenzenesulfonyl-piperidin-4-yl)-1,4-dihydro-benzo[d][1,3]oxazin-2-one |
| 159 | 8-Methyl-1-[1-(toluene-3-sulfonyl)-piperidin-4-yl]-1,4-dihydro-benzo[d][1,3]oxazin-2-one |
| 160 | 1-[1-(5-Fluoro-2-methyl-benzenesulfonyl)-piperidin-4-yl]-8-methyl-1,4-dihydrobenzo[d][1,3]oxazin-2-one |
| 161 | 1-[1-(4-Isopropoxy-benzenesulfonyl)-piperidin-4-yl]-8-methyl-1,4-dihydro- benzo[d][1,3] oxazin-2-one |
| 162 | 1-[1-(3-Chloro-benzenesulfonyl)-piperidin-4-yl]-8-methyl-1,4-dihydro-benzo[d][1,3]oxazin-2-one |
| 163 | 1-[1-(3,4-Dimethoxy-benzenesulfonyl)-piperidin-4-yl]-8-methyl-1,4-dihydro-benzo[d][1,3] oxazin-2-one |
| 164 | 8-Methyl-1-(1-pentafluorobenzenesulfonyl-piperidin-4-yl)-1,4-dihydro-benzo[d][1,3]oxazin-2-one |
| 165 | 6-Methyl-1-[1-(toluene-3-sulfonyl)-piperidin-4-yl]-1 ,4-dihydro-benzo[d][1,3]oxazin-2-one |
| 166 | 1-[1-(5-Fluoro-2-methyl-benzenesulfonyl)-piperidin-4-yl]-6-methyl-1,4-dihydro-benzo[d][1,3]oxazin-2-one |
| 167 | 1-[1-(4-Isopropoxy-benzenesulfonyl)-piperidin-4-yl]-6-methyl-1,4-dihydro-benzo[d][1,3]oxazin-2-one |
| 168 | 1-[1-(3-Chloro-benzenesulfonyl)-piperidin-4-yl]-6-methyl-1,4-dihydro-benzo[d][1,3]oxazin-2-one |
| 169 | 1-[1-(3,4-Dimethoxy-benzenesulfonyl)-piperidin-4-yl]-6-methyl-1,4-dihydro-benzo[d][1,3]oxazin-2-one |
| 170 | 6-Methyl-1-(1-pentafluorobenzenesulfonyl-piperidin-4-yl)-1,4-dihydro-benzo[d][1,3]oxazin-2-one |
| 171 | 6-Methyl-1-[1-(4-trifluoromethoxy-benzenesulfonyl)-piperidin-4-yl]-1,4-dihydro-benzo[d][1,3]oxazin-2-one |
| 172 | 6-Methyl-1-[1-(2-nitro-4-trifluoromethyl-benzenesulfonyl)-piperidin-4-yl]-1,4-dihydro-benzo[d][1,3]oxazin-2-one |
| 173 | 1-[1-(3-Fluoro-benzenesulfonyl)-piperidin-4-yl]-6-methyl-1,4-dihydro-benzo[d][1,3]oxazin-2-one |
| 174 | 1-[1-(2,4-Dichloro-benzenesulfonyl)-piperidin-4-yl]-6-methyl-1,4-dihydro-benzo[d][1,3]oxazin-2-one |
| 175 | 6-Methyl-1-[1-(2,4,6-trimethyl-benzenesulfonyl)-piperidin-4-yl]-1,4-dihydro-benzo[d][1,3]oxazin-2-one |
| 176 | 6-Methyl-1-[1-(2-trifluoromethyl-benzenesulfonyl)-piperidin-4-yl]-1,4-dihydro-benzo[d][1,3]oxazin-2-one |
| 177 | 1-[1-(3-Methoxy-benzenesulfonyl)-piperidin-4-yl]-6-methyl-1,4-dihydro-benzo[d][1,3]oxazin-2-one |
| 178 | 6-Methyl-1-[1-(2-nitro-benzenesulfonyl)-piperidin-4-yl]-1,4-dihydro-benzo[d][1,3]oxazin-2-one |
| 179 | 1-[1-(4-Acetyl-benzenesulfonyl)-piperidin-4-yl]-6-methyl-1,4-dihydro-benzo[d][1,3]oxazin-2-one |
| 180 | 1-[1-(4-Methanesulfonyl-benzenesulfonyl)-piperid in-4-yl]-6-methyl-1,4-dihydro-benzo[d][1,3]oxazin-2-one |
| 181 | 6-Methyl-1-(1-phenylmethanesulfonyl-piperidin-4-yl)-1,4-dihydro-benzo[d][1,3]oxazin-2-one |
| 182 | 2-[4-(6-Methyl-2-oxo-4H-benzo[d][1,3]oxazin-1-yl)-piperidine-1-sulfonyl]benzoic acid methyl ester |
| 183 | 6-Methyl-1-[1-(2-oxo-2H-chromene-6-sulfonyl)-piperidin-4-yl]-1,4-dihydro-benzo[d][1,3]oxazin-2-one |
| 184 | 6-Chloro-1-[1-(4-fluoro-benzenesulfonyl)-piperidin-4-yl]-1,4-dihydro-benzo[d][1,3]oxazin-2-one |
| 185 | 6-Chloro-1-[1-(3,5-dichloro-benzenesulfonyl)-piperidin-4-yl]-1,4-dihydro-benzo[d][1,3]oxazin-2-one |
| 186 | 1-{1-[4-(4-Bromo-phenoxy)-benzenesulfonyl]-piperidin-4-yl}-6-chloro-1,4-dihydro-benzo[d][1,3]oxazin-2-one |
| 187 | 6-Chloro-1-[1-(thiophene-2-sulfonyl)-piperidin-4-yl]-1,4-dihydro-benzo[d][1,3]oxazin-2-one |
| 188 | 6-Chloro-1-[1-(3-methoxy-benzenesulfonyl)-piperidin-4-yl]-1,4-dihydro-benzo[d][1,3]oxazin-2-one |
| 189 | 6-Chloro-1-[1-(2-oxo-2H-chromene-6-sulfonyl)-piperidin-4-yl]-1,4-dihydro-benzo[d][1,3]oxazin-2-one |
| 190 | 6-Chloro-1-[1-(toluene-3-sulfonyl)-piperidin-4-yl]-1,4-dihydro-benzo[d][1,3]oxazin-2-one |
| 191 | 6-Chloro-1-[1-(5-fluoro-2-methyl-benzenesulfonyl)-piperidin-4-yl]-1,4-dihydro-benzo[d][1,3]oxazin-2-one |
| 192 | 6-Chloro-1-[1-(4-isopropoxy-benzenesulfonyl)-piperidin-4-yl]-1,4-dihydro-benzo[d][1,3] oxazin-2-one |
| 193 | 6-Chloro-1-[1-(3-chloro-benzenesulfonyl)-piperidin-4-yl]-1,4-dihydro-benzo[d][1,3]oxazin-2-one |
| 194 | 6-Chloro-1-[1-(3,4-dimethoxy-benzenesulfonyl)-piperidin-4-yl]-1,4-dihydro-benzo[d][1,3]oxazin-2-one |
| 195 | 6-Chloro-1-[1-pentafluorobenzenesulfonyl-piperidin-4-yl)-1,4-dihydro-benzo[d][1,3]oxazin-2-one |
| 196 | 6-Chloro-1-[1-(4-trifluoromethoxy-benzenesulfonyl)-piperidin-4-yl]-1,4-dihydro-benzo[d][1,3]oxazin-2-one |
| 197 | 6-Chloro-1-[1-(2-nitro-4-trifluoromethyl-benzenesulfonyl)-piperidin-4-yl]-1,4-dihydro-benzo[d][1,3]oxazin-2-one |
| 198 | 6-Chloro-1-[1-(3-fluoro-benzenesulfonyl)-piperidin-4-yl]-1,4-dihydro-benzo[d][1,3]oxazin-2-one |
| 199 | 6-Chloro-1-[1-(2,4-dichloro-benzenesulfonyl)-piperidin-4-yl]-1,4-dihydro-benzo[d][1,3]oxazin-2-one |
| 200 | 6-Chloro-1-[1-(2,4,6-trimethyl-benzenesulfonylrpiperidin-4-yl]-1,4-dihydro-benzo[d][1,3]oxazin-2-one |
| 201 | 6-Chloro-1-[1-(2-trifluoromethyl-benzenesulfonyl)-piperidin-4-yl]-1,4-dihydro-benzo[d][1,3]oxazin-2-one |
| 202 | 1-[1-(2-Oxo-2H-chromene-6-sulfonyl)-piperidin-4-yl]-1,4-dihydro-benzo[d][1,3]oxazin-2-one |
| 203 | 1-[1-(3,5-Dichloro-benzenesulfonyl)-piperidin-4-yl]-1,4-dihydro-benzo[d][1,3]oxazin-2-one |
| 204 | 1-[1-(2,5-Dichloro-benzenesulfonyl)-piperidin-4-yl]-1,4-dihydro-benzo[d][1,3]oxazin-2-one |
| 205 | 1-[1-(5-Bromo-6-chloro-pyridine-3-sulfonyl)-piperidin-4-yl]-1,4-dihydro-benzo[d][1,3]oxazin-2-one |
| 206 | 1-[1-(4-Chloro-benzenesulfonyl)-piperidin-4-yl]-1,4-dihydro-benzo[d][1,3]oxazin-2-one |
| 207 | 1-[1-(2,6-Dichloro-benzenesulfonyl)-piperidin-4-yl]-1,4-dihydro-benzo[d][1,3]oxazin-2-one |
| 208 | 8-Methyl-1-[1-(2-oxo-2H-chromene-6-sulfonyl)-piperidin-4-yl]-1,4-dihydro-benzo[d][1,3]oxazin-2-one |
| 209 | 1-[1-(3, 5-Dichloro-benzenesulfonyl)-piperidin-4-yl]-8-methyl-1,4-dihydro-benzo[d][1,3]oxazin-2-one |
| 210 | 1-[1-(2,5-Dichloro-benzenesulfonyl)-ppiperidin-4-yl]-8-methyl-1,4-dihydro-benzo[d][1,3]oxazin-2-one |
| 211 | 1-[1-(5-Bromo-6-chloro-pyridine-3-sulfonyl)-piperidin-4-yl]-8-methyl-1,4-dihydro-benzo[d][1,3]oxazin-2-one |
| 212 | 1-[1-(4-Chloro-benzenesulfonyl)-piperidin-4-yl]-8-methyl-1,4-dihydro-benzo[d][1,3]oxazin-2-one |
| 213 | 1-[1-(2,6-Dichloro-benzenesulfonyl)-piperidin-4-yl]-8-methyl-1,4-dihydro-benzo[d][1,3]oxazin-2-one |
| 214 | 1-[1-(Biphenyl-4-sulfonyl)-piperidin-4-yl]-1,4-dihydro-benzo[d][1,3]oxazin-2-one |
| 215 | 6-Chloro-1-[1-[(2,5-dichloro-benzenesulfonyl)-piperidin-4-yl]-1,4-dihydro-benzo[d][1,3]oxazin-2-one |
| 216 | 1-[1-(5-Bromo-6-chloro-pyridine-3-sulfonyl)-piperidin-4-yl]-6-chloro-1,4-dihydro-benzo[d][1,3]oxazin-2-one |
| 217 | 6-Chloro-1-[1-(4-chloro-benzenesulfonyl)-piperidin-4-yl]-1,4-dihydro-benzo[d][1,3]oxazin-2-one |
| 218 | 6-Chloro-1-[1-(2,6-dichloro-benzenesulfonyl)-piperidin-4-yl]-1,4-dihydro-benzo[d][1,3]oxazin-2-one |
| 219 | 1-[1-(Biphenyl-4-sulfonyl)-piperidin-4-yl]-6-methyl-1,4-dihydro-benzo[d][1,3]oxazin-2-one |
| 220 | 2-[4-(6-Methyl-2-oxo-4H-benzo[d][1,3]oxazin-1-yl)-piperidine-1-sulfonyl]-benzonitrile |
| 221 | 1-[1-(2,5-Dichloro-benzenesulfonyl)-piperidin-4-yl]-6-methyl-1,4-dihydro-benzo[d][1,3]oxazin-2-one |
| 222 | 1-[1-(5-Bromo-6-chloro-pyridine-3-sulfonyl)-piperidin-4-yl]-6-methyl-1,4-dihydro-benzo[d][1,3]oxazin-2-one |
| 223 | 1-[1-(4-Chloro-benzenesulfonyl)-piperidin-4-yl]-6-methyl-1,4-dihydro-benzo[d][1,3]oxazin-2-one |
| 224 | 1-[1-(2,6-Dichloro-benzenesulfonyl)-piperidin-4-yl]-6-methyl-1,4-dihydro-benzo[d][1,3]oxazin-2-one |
| 225 | 1-[1-(3,5-Dichloro-benzenesulfonyl)-piperidin-4-yl]-6-methyl-1,4-dihydro-benzo[d][1,3]oxazin-2-one |
| 226 | 6-Methyl-1-[1-(1-methyl-1H-imidazole-4-sulfonyl)-piperidin-4-yl]-1,4-dihydro-benzo[d][1,3]oxazin-2-one |
| 227 | 1-[1-(5-Bromo-2,4-difluoro-benzenesulfonyl)-piperidin-4-yl]-6-methyl-1,4-dihydro-benzo[d][1,3]oxazin-2-one |
| 228 | 1-[1-(4-Methanesulfonyl-benzenesulfonyl)-piperidin-4-yl]-1,4-dihydro-benzo[d][1,3]oxazin-2-one |
| 229 | 1-[1-(1-Methyl-1H-imidazole-4-sulfonyl)-piperidin-4-yl]-1,4-dihydro-benzo[d][1,3]oxazin-2-one |
| 230 | 1-[1-(5-Bromo-2,4-difluoro-benzenesulfonyl)-piperidin-4-yl]-1,4-dihydro-benzo[d][1,3]oxazin-2-one |
| 231 | 1-[1-(6-Chloro-imidazo[2,1-b]thiazole-5-sulfonyl)-piperidin-4-yl]-1,4-dihydro-benzo[d][1,3]oxazin-2-one |
| 232 | 1-[1-(4-Ethyl-benzenesulfonyl)-piperidin-4-yl-1,4-dihydro-benzo[d][1,3]oxazin-2-one |
| 233 | 1-[1-(Benzo[b]thiophene-3-sulfonyl)-piperidin-4-yl]-1,4-dihydro-benzo[d][1,3]oxazin-2-one |
| 234 | 1-[1-(6-Chloro-imidazo[2,1-b]thiazole-5-sulfonyl)-piperidin-4-yl]-8-methyl-1,4-dihydro-benzo[d][1,3]oxazin-2-one |
| 235 | 1-[1-(4-Ethyl-benzenesulfonyl)-piperidin-4-yl]-8-methyl-1,4-dihydro-benzo[d][1,3]oxazin-2-one |
| 236 | 1-[1-(Benzo[b]thiophene-3-sulfonyl)-piperidin-4-yl]-8-methyl-1,4-dihydro-benzo[d][1,3]oxazin-2-one |
| 237 | 6-Chloro-1-[1-(6-chloro-imidazo[2,1-b]thiazole-5-sulfonyl)-piperidin-4-yl]-1,4-dihydro-benzo[d][1,3]oxazin-2-one |
| 238 | 6-Chloro-1-[1-(4-ethyl-benzenesulfonyl)-piperidin-4-yl]-1,4-dihydro-benzo[d][1,3]oxazin-2-one |
| 239 | 1-[1-(Benzo[b]thiophene-3-sulfonyl)-piperidin-4-yl]-6-chloro-1,4-dihydro-benzo[d][1,3]oxazin-2-one |
| 240 | 1-[1-(6-Chloro-imidazo[2,1-b]thiazole-5-sulfonyl)-piperidin-4-yl]-6-methyl-1,4-dihydro-benzo[d][1,3]oxazin-2-one |
| 241 | 1-[1-(4-Ethyl-benzenesulfonyl)-piperidin-4-yl]-6-methyl-1,4-dihydro-benzo[d][1,3]oxazin-2-one |
| 242 | 1-[1-(Benzo[b]thiophene-3-sulfonyl)-piperidin-4-yl]-6-methyl-1,4-dihydro-benzo[d][1,3]oxazin-2-one |
| 243 | 1-[1-(7-Chloro-benzo[1,2,5]oxadiazole-4-sulfonyl)-piperidin-4-yl]-1,4-dihydro-benzo[d][1,3]oxazin-2-one |
| 244 | 1-[1-(2-Methoxy-4-methyl-benzenesulfonyl)-piperidin-4-yl]-1,4-dihydro-benzo[d][1,3]oxazin-2-one |
| 245 | 3-{4-[4-(2-Oxo-4H-benzo[d][1,3]oxazin-1-yl)-piperidine-1-sulfonyl]-phenyl}-propionic acid methyl ester |
| 246 | 1-[1-(2,4-Dinitro-benzenesulfonyl)-piperidin-4-yl]-1,4-dihydro-benzo[d][1,3]oxazin-2-one |
| 247 | 1-[1-(7-Chloro-benzo[1,2,5]oxadiazole-4-sulfonyl)-piperidin-4-yl]-8-methyl-1,4-dihydro-benzo[d][1,3]oxazin-2-one |
| 248 | 1-[1-(2-Methoxy-4-methyl-benzenesulfonyl)-piperidin-4-yl]-8-methyl-1,4-dihydro-benzo[d][1,3]oxazin-2-one |
| 249 | 3-{4-[4-(8-Methyl-2-oxo-4H-benzo[d][1,3]oxazin-1-yl)-piperidine-1-sulfonyl]-phenyl}-propionic acid methyl ester |
| 250 | 1-[1-(2,4-Dinitro-benzenesulfonyl)-piperidin-4-yl]-8-methyl-1,4-dihydro-benzo[d][1,3]oxazin-2-one |
| 251 | 1-[1-(7-Chloro-benzo[1,2,5]oxadiazole-4-sulfonyl)-piperidin-4-yl]-6-methyl-1,4-dihydro-benzo[d][1,3]oxazin-2-one |
| 252 | 1-[1-(2-Methoxy-4-methyl-benzenesulfonyl)-piperidin-4-yl]-6-methyl-1,4-dihydro-benzo[d][1,3]oxazin-2-one |
| 253 | 3-{4-[4-(6-Methyl-2-oxo-4H-benzo[d][1,3]oxazin-1-yl)-piperidine-1-sulfonyl]-phenyl}-propionic acid methyl ester |
| 254 | 1-[1-(2,4-Dinitro-benzenesulfonyl)-piperidin-4-yl]-6-methyl-1,4-dihydro-benzo[d][1,3]oxazin-2-one |
| 255 | 6-Chloro-1-[1-(7-chloro-benzo[1,2,5]oxadiazole-4-sulfonyl)-piperidin-4-yl]-1,4-dihydro-benzo[d][1,3]oxazin-2-one |
| 256 | 6-Chloro-1-[1-(2-methoxy-4-methyl-benzenesulfonyl)-piperidin-4-yl]-1,4-dihydro-benzo[d][1,3]oxazin-2-one |
| 257 | 3-{4-[4-(6-Chloro-2-oxo-4H-benzo[d][1,3]oxazin-1-yl)-piperidine-1-sulfonyl]-phenyl}-propionic acid methyl ester |
| 258 | 6-Chloro-1-[1-(2,4-dinitro-benzenesulfonyl)-piperidin-4-yl]-1,4-dihydro-benzo[d][1,3]oxazin-2-one |
| 259 | 6-Chloro-1-[1-(1-methyl-1H-imidazole-4-sulfonyl)-piperidin-4-yl]-1,4-dihydro-benzo[d][1,3]oxazin-2-one |
| 260 | 1-[1-(5-Bromo-2,4-difluoro-benzenesulfonyl)-piperidin-4-yl]-6-chloro-1,4-dihydro-benzo[d][1,3]oxazin-2-one |
| 261 | 8-Methyl-1-[1-(1-methyl-1H-imidazole-4-sulfonyl)-piperidin-4-yl]-1,4-dihydro-benzo[d][1,3]oxazin-2-one |
| 262 | 1-[1-(5-Bromo-2,4-difluoro-benzenesulfonyl)-piperidin-4-yl]-8-methyl-1,4-dihydro-benzo[d][1,3]oxazin-2-one |
| 263 | 1-[1-(Benzo[b]thiophene-2-sulfonyl)-piperidin-4-yl]-8-methyl-1,4-dihydro-benzo[d][1,3]oxazin-2-one |
| 264 | 1-[1-(Benzo[b]thiophene-2-sulfonyl)-piperidin-4-yl]-1,4-dihydro-benzo[d][1,3]oxazin-2-one |
| 265 | 1-[1-(Benzo[b]thiophene-2-sulfonyl)-piperidin-4-yl]-6-chloro-1,4-dihydro-benzo[d][1,3]oxazin-2-one |
| 266 | 1-[1-(Benzo[b]thiophene-2-sulfonyl)-piperidin-4-yl]-6-methyl-1,4-dihydro-benzo[d][1,3]oxazin-2-one |
| 267 | 1-[1-(2,5-Difluoro-benzenesulfonyl)-piperidin-4-yl]-1,4-dihydro-benzo[d][1,3]oxazin-2-one |
| 268 | 1-[1-(2,5-Difluoro-benzenesulfonyl)-piperidin-4-yl]-8-methyl-1,4-dihydro-benzo[d][1,3]oxazin-2-one |
| 269 | 6-Chloro-1-[1-(2,5-difluoro-benzenesulfonyl)-piperidin-4-yl]-1,4-dihydro-benzo[d][1,3]oxazin-2-one |
| 270 | 1-[1-(2,5-Difluoro-benzenesulfonyl)-piperidin-4-yl]-6-methyl-1,4-dihydro-benzo[d][1,3]oxazin-2-one |
| 271 | 1-[1-(4-Chloro-2,5-difluoro-benzenesulfonyl)-piperidin-4-yl]-1,4-dihydro-benzo[d][1,3]oxazin-2-one |
| 272 | 1-[1-(4-Chloro-2,5-difluoro-benzenesulfonyl)-pipeeidin-4-yl]-8-methyl-1,4-dihydro-benzo[d][1,3]oxazin-2-one |
| 273 | 6-Chloro-1-[1-(4-chloro-2,5-difluoro-benzenesulfonyl)-piperidin-4-yl]-1,4-dihydro-benzo[d][1,3]oxazin-2-one |
| 274 | 1-[1-(4-Chloro-2,5-difluoro-benzenesulfonyl)-piperidin-4-yl]-6-methyl-1,4-dihydro-benzo[d][1,3]oxazin-2-one |
| 275 | 1-[1-(2,4,5-Trifluoro-benzenesulfonyl)-piperidin-4-yl]-1,4-dihydro-benzo[d][1,3]oxazin-2-one |
| 276 | 8-Methyl-1-[1-(2,4,5-trifluoro-benzenesulfonyl)-piperidin-4-yl]-1,4-dihydro-benzo[d][1,3]oxazin-2-one |
| 277 | 6-Chloro-1-[1-(2,4,5-trifluoro-benzenesulfonyl)-piperidin-4-yl]-1,4-dihydro-benzo[d][1,3]oxazin-2-one |
| 278 | 6-Methyl-1-[1-(2,4,5-trifluoro-benzenesulfonyl)-piperidin-4-yl]-1,4-dihydro-benzo[d][1,3]oxazin-2-one |
| 279 | 1-[1-(3,5-Dichloro-2-hydroxy-benzenesulfonyl)-piperidin-4-yl]-1,4-dihydro-benzo[d][1,3]oxazin-2-one |
| 280 | 1-[1-(2,6-Difluoro-benzenesulfonyl)-piperidin-4-yl]-1,4-dihydro-benzo[d][1,3]oxazin-2-one |
| 281 | 1-[1-(2,6-Difluoro-benzenesulfonyl)-piperidin-4-yl]-8-methyl-1,4-dihydro- benzo[d][1,3]oxazin-2-one |
| 282 | 6-Chloro-1-[1-(2,6-difluoro-benzenesulfonyl)-piperidin-4-yl]-1,4-dihydro-benzo[d][1,3]oxazin-2-one |
| 283 | 1-[1-(2,6-Difluoro-benzenesulfonyl)-piperidin-4-yl]-6-methyl-1,4-dihydro-benzo[d][1,3]oxazin-2-one |
| 284 | 1-[1-(5-Chloro-2,4-difluoro-benzenesulfonyl)-piperidin-4-yl]-1,4-dihydro-benzo[d][1,3]oxazin-2-one |
| 285 | 1-[1-(5-Chloro-2,4-difluoro-benzenesulfonyl)-piperidin-4-yl]-8-methyl-1,4-dihydro-benzo[d][1,3]oxazin-2-one |
| 286 | 6-Chloro-1-[1-(5-chloro-2 ,4-difluoro-benzenesulfonyl)-piperidin-4-yl]-1,4-dihydro-benzo[d][1,3]oxazin-2-one |
| 287 | 1-[1-(5-Chloro-2,4-difluoro-benzenesulfonyl)-piperidin-4-yl]-6-methyl-1,4-dihydro-benzo[d][1,3]oxazin-2-one |
| 288 | 1-[1-(2-Chloro-benzenesulfonyl)-piperidin-4-yl]-1,4-dihydro-benzo[d][1,3]oxazin-2-one |
| 289 | 1-[1-(2-Chloro-benzenesulfonyl)-piperidin-4-yl]-8-methyl-1,4-dihydro-benzo[d][1,3]oxazin-2-one |
| 290 | 6-Chloro-1-[1-(2-chloro-benzenesulfonyl)-piperidin-4-yl]-1,4-dihydro-benzo[d][1,3]oxazin-2-one |
| 291 | 1-[1-(2-Chloro-benzenesulfonyl)-piperidin-4-yl]-6-methyl-1,4-dihydro-benzo[d][1,3]oxazin-2-one |
| 292 | 6-Chloro-1-[1-(2-naphthalen-1-yl-ethanesulfonyl)-piperidin-4-yl]-1,4-dihydro-benzo[d][1,3]oxazin-2-one |
| 293 | 6-Bromo-1-[1-(4-bromo-benzenesulfonyl)-piperidin-4-yl]-1,4-dihydro-benzo[d][1,3]oxazin-2-one |
| 294 | 6-Bromo-1-[1-(toluene-4-sulfonyl)-piperidin-4-yl]-1,4-dihydro-benzo[d][1,3]oxazin-2-one |
| 295 | 6-Bromo-1-[1-(2,4-dimethyl-benzenesulfonyl)-piperidin-4-yl]-1,4-dihydro-benzo[d][1,3]oxazin-2-one |
| 296 | 6-Bromo-1-[1-(2-naphthaten-1-yl-ethanesulfonyl)-piperidin-4-yl]-1,4-dihydro-benzo[d][1,3]oxazin-2-one |
| 297 | 6-Bromo-1-[1-(quinoline-8-sulfonyl)-piperidin-4-yl]-1,4-dihydro-benzo[d][1,3]oxazin-2-one |
| 298 | 6-Bromo-1-[1-(5-chloro-3-methyl-benzo[b]thiophene-2-sulfonyl)-piperidin-4-yl]-1,4-dihydro-benzo[d][1,3]oxazin-2-one |
| 299 | 6-Bromo-1-[1-(3-nitro-benzenesulfonyl)-pipendin-4-yl]-1,4-dihydro-benzo[d][1,3]oxazin-2-one |
| 300 | 6-Bromo-1-[1-(naphthalene-1-sulfonyl)-piperidin-4-yl]-1,4-dihydro-benzo[d][1,3]oxazin-2-one |
| 301 | 6-Bromo-1-[1-(naphthalene-2-sulfonyl)-piperidin-4-yl]-1,4-dihydro-benzo[d][1,3]oxazin-2-one |
| 302 | 1-(1-Benzenesulfonyl-piperidin-4-yl)-6-bromo-1,4-dihydro-benzo[d][1,3]oxazin-2-one |
| 303 | 6-Bromo-1-{1-[4-(4-bromo-phenoxy)-benzenesulfonyl]-piperidin-4-yl}-1,4-dihydro-benzo[d][1,3]oxazin-2-one |
| 304 | 6-Bromo-1-[1-(thiophene-2-sulfonyl)-piperidin-4-yl]-1,4-dihydro-benzo[d][1,3]oxazin-2-one |
| 305 | 6-Bromo-1-[1-(2-methyl-5-nitro-benzenesulfonyl)-piperidin-4-yl]-1,4-dihydro-benzo[d][1,3]oxazin-2-one |
| 306 | 6-Bromo-1-[1-(4-bromo-2,5-difluoro-benzenesulfonyl)-piperidin-4-yl]-1,4-dihydro-benzo[d][1,3]oxazin-2-one |
| 307 | 6-Bromo-1-[1-(toluene-3-sulfonyl)-piperidin-4-yl]-1,4-dihydro-benzo[d][1,3]oxazin-2-one |
| 308 | 6-Bromo-1-[1-(5-fluoro-2-methyl-benzenesulfonyl)-piperidin-4-yl]-1,4-dihydro-benzo[d][1,3]oxazin-2-one |
| 309 | 6-Bromo-1-[1-(4-isopropoxy-benzenesulfonyl)-piperidin-4-yl]-1,4-dihydro-benzo[d][1,3]oxazin-2-one |
| 310 | 6-Bromo-1-[1-(3-chloro-benzenesulfonyl)-piperidin-4-yl]-1,4-dihydro-benzo[d][1,3]oxazin-2-one |
| 311 | 6-Bromo-1-[1-(3,4-dimethoxy-benzenesulfonyl)-piperidin-4-yl]-1,4-dihydro-benzo[d][1,3]oxazin-2-one |
| 312 | 6-Bromo-1-(1-pentafluorobenzenesulfonyl-piperidin-4-yl)-1,4-dihydro-benzo[d][1,3]oxazin-2-one |
| 313 | 6-Bromo-1-[1-(4-chloro-2,5-dimethyl-benzenesulfonyl)-piperidin-4-yl]-1,4-dihydro-benzo[d][1,3]oxazin-2-one |
| 314 | 6-Bromo-1-[1-(3-methoxy-benzenesulfonyl)-piperidin-4-yl]-1,4-dihydro-benzo[d][1,3]oxazin-2-one |
| 315 | 6-Bromo-1-[1-(4-isopropyl-benzenesulfonyl)-piperidin-4-yl]-1,4-dihydro-benzo[d][1,3]oxazin-2-one |
| 316 | 6-Bromo-1-[1-(4-fluoro-benzenesulfonyl)-piperidin-4-yl]-1,4-dihydro-benzo[d][1,3]oxazin-2-one |
| 317 | 6-Bromo-1-[1-(3-chloro-4-fluoro-benzenesulfonyl)-piperidin-4-yl]-1,4-dihydro-benzo[d][1,3]oxazin-2-one |
| 318 | 6-Bromo-1-(1-pentamethylbenzenesulfonyl-piperidin-4-yl)-1,4-dihydro-benzo[d][1,3]oxazin-2-one |
| 319 | 6-Bromo-1-[1-(2-nitro-benzenesulfonyl)-piperidin-4-yl]-1,4-dihyd ro-benzo[d][1,3]oxazin-2-one |
| 320 | 6-Bromo-1-[1-(4-chloro-3-nitro-benzenesulfonyl)-piperidin-4-yl]-1,4-dihydro-benzo[d][1,3]oxazin-2-one |
| 321 | 6-Bromo-1-[1-(5-dimethylamino-naphthalene-1-sulfonyl)-piperidin-4-yl]-1,4-dihydro-benzo[d][1,3]oxazin-2-one |
| 322 | 6-Bromo-1-[1-(4-nitro-benzenesulfonyl)-piperidin-4-yl]-1,4-dihydro-benzo[d][1,3]oxazin-2-one |
| 323 | 1-[1-(4-Acetyl-benzenesulfonyl)-piperidin-4-yl]-6-bromo-1,4-dihydro-benzo[d][1,3]oxazin-2-one |
| 324 | 6-Bromo-1-[1-(4-methanesulfonyl-benzenesulfonyl)-piperidin-4-yl]-1,4-dihydro-benzo[d][1,3]oxazin-2-one |
| 325 | 1-[1-(Biphenyl-4-sulfonyl)-piperidin-4-yl]-6-bromo-1,4-dihydro-benzo[d][1,3]oxazin-2-one |
| 326 | 6-Bromo-1-(1-phenylmethanesulfonyl-piperidin-4-yl)-1,4-dihydro-benzo[d][1,3]oxazin-2-one |
| 327 | 6-Bromo-1-[1-(2,5-dimethoxy-benzenesulfonyl)-piperidin-4-yl]-1,4-dihydro-benzo[d][1,3]oxazin-2-one |
| 328 | 6-Bromo-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one |
| 329 | 6-Bromo-1-[1-(2,3-dichloro-benzenesulfonyl)-piperidin-4-yl]-1,4-dihydro-benzo[d][1,3]oxazin-2-one |
| 330 | 6-Bromo-1-[1-(2,4,5-trichloro-benzenesulfonyl)-piperidin-4-yl]-1,4-dihydro-benzo[d][1,3]oxazin-2-one |
| 331 | 6-Bromo-1-[1-(5-bromo-2-methoxy-benzenesulfonyl)-piperidin-4-yl]-1,4-dihydro-benzo[d][1,3]oxazin-2-one |
| 332 | 6-Bromo-1-[1-(4-trifluoromethoxy-benzenesulfonyl)-piperidin-4-yl]-1,4-dihydro-benzo[d][1,3]oxazin-2-one |
| 333 | 6-Bromo-1-[1-(2-nitro-4-trifluoromethyl-benzenesulfonyl)-piperidin-4-yl]-1,4-dihydro-benzo[d][1,3]oxazin-2-one |
| 334 | 6-Bromo-1-[1-(3-fluoro-benzenesulfonyl)-piperidin-4-yl]-1,4-dihydro-benzo[d][1,3]oxazin-2-one |
| 335 | 6-Bromo-1-[1-(2,4-dichloro-benzenesulfonyl)-piperidin-4-yl]-1,4-dihydro-benzo[d][1,3]oxazin-2-one |
| 336 | 6-Bromo-1-[1-(2,4,6-trimethyl-benzenesuifonyl)-piperidin-4-yl]-1,4-dihydro-benzo[d][1,3]oxazin-2-one |
| 337 | 6-Bromo-1-[1-(2-trifluoromethyl-benzenesulfonyl)-piperidin-4-yl]-1,4-dihydro-benzo[d][1,3]oxazin-2-one |
| 338 | 6-Bromo-1-[1-(2-bromo-benzenesulfonyl)-piperidin-4-yl]-1,4-dihydro-benzo[d][1,3]oxazin-2-one |
| 339 | 6-Bromo-1-[1-(4-methoxy-2,3,6-trimethyl-benzenesulfonyl)-piperidin-4-yl]-1,4-dihydro-benzo[d][1,3]oxazin-2-one |
| 340 | 1-[1-(3,5-Dichloro-4-hydroxy-benzenesulfonyl)-piperidin-4-yl]-1,4-dihydro-benzo[d][1,3]oxazin-2-one |
| 341 | 1-[1-(3,5-Dichloro-4-hydroxy-benzenesulfonyl)-piperidin-4-yl]-8-methyl-1,4-dihydro-benzo[d][1,3]oxazin-2-one |
| 342 | 6-Chloro-1-[1-(3,5-dichloro-4-hydroxy-benzenesulfonyl)-piperidin-4-yl]-1,4-dihydro-benzo[d][1,3]oxazin-2-one |
| 343 | 1-[1-(3,5-Dichloro-4-hydroxy-benzenesulfonyl)-piperidin-4-yl]-6-methyl-1,4-dihydro-benzo[d][1,3]oxazin-2-one |
| 344 | 6-Bromo-1-[1-(3,5-dichloro-4-hydroxy-benzenesulfonyl)-piperidin-4-yl]-1,4-dihydro-benzo[d][1,3]oxazin-2-one |
| 345 | 6-Chloro-1-[1-(3,5-dichloro-2-hydroxy-benzenesulfonyl)-piperidin-4-yl]-1,4-dihydro-benzo[d][1,3]oxazin-2-one |
| 346 | 6-Bromo-1-[1-(3,5-dichloro-2-hydroxy-benzenesulfonyl)-piperidin-4-yl]-1,4-dihydro-benzo[d][1,3]oxazin-2-one |
| 347 | 2-[4-(6-Bromo-2-oxo-4H-benzo[d][1,3]oxazin-1-yl)-piperidine-1-sulfonyl]-benzonitrile |
| 348 | 6-Bromo-1-[1-(4-methoxy-benzenesulfonyl)-piperidin-4-yl]-1,4-dihydro-benzo[d][1,3]oxazin-2-one |
| 349 | 2-[4-(6-Bromo-2-oxo-4H-benzo[d][1,3]oxazin-1-yl)-piperidine-1-sulfonyl]-benzoic acid methyl ester |
| 350 | 6-Bromo-1-[1-(3-trifluoromethyl-benzenesulfonyl)-piperidin-4-yl]-1,4-dihydro-benzo[d][1,3]oxazin-2-one |
| 351 | 6-Bromo-1-[1-(2-oxo-2H-chromene-6-sulfonyl)-piperid in-4-yl]-1,4-dihydro-benzo[d][1,3]oxazin-2-one |
| 352 | 6-Bromo-1-[1-(3,5-dichloro-benzenesulfonyl)-piperidin-4-yl]-1,4-dihydro-benzo[d][1,3]oxazin-2-one |
| 353 | 6-Bromo-1-[1-(2,5-dichloro-benzenesulfonyl)-piperidin-4-yl]-1,4-dihydro-benzo[d][1,3]oxazin-2-one |
| 354 | 6-Bromo-1-[1-(5-bromo-6-chloro-pyridine-3-sulfonyl)-piperidin-4-yl]-1,4-dihydro-benzo[d][1,3]oxazin-2-one |
| 355 | 6-Bromo-1-[1-(4-chloro-benzenesulfonyl)-piperidin-4-yl]-1,4-dihydro-benzo[d][1,3]oxazin-2-one |
| 356 | 6-Bromo-1-[1-(2,6-dichloro-benzenesulfonyl)-piperidin-4-yl]-1,4-dihydro-benzo[d][1,3]oxazin-2-one |
| 357 | 6-Bromo-1-[1-(1-methyl-1H-imidazole-4-sulfonyl)-piperidin-4-yl]-1,4-dihydro-benzo[d][1,3]oxazin-2-one |
| 358 | 6-Bromo-1-[1-(5-bromo-2,4-difluoro-benzenesulfonyl)-piperidin-4-yl]-1,4-dihydro-benzo[d][1,3]oxazin-2-one |
| 359 | 6-Bromo-1-[1-(4-ethyl-benzenesulfonyl)-piperidin-4-yl]-1,4-dihydro-benzo[d][1,3]oxazin-2-one |
| 360 | 6-Bromo-1-[1-(6-chloro-imidazo[2,1-b]thiazole-5-sulfonyl)-piperidin-4-yl]-1,4-dihydro-benzo[d][1,3]oxazin-2-one |
| 361 | 1-[1-(Benzo[b]thiophene-3-sulfonyl)-piperidin-4-yl]-6-bromo-1,4-dihydro-benzo[d][1,3]oxazin-2-one |
| 362 | 6-Bromo-1-[1-(7-chloro-benzo[1,2,5]oxadiazole-4-sulfonyl)-piperidin-4-yl]-1,4-dihydro-benzo[d][1,3]oxazin-2-one |
| 363 | 6-Bromo-1-[1-(2-methoxy-4-methyl-benzenesulfonyl)-piperidin-4-yl]-1,4-dihydro-benzo[d][1,3]oxazin-2-one |
| 364 | 3-{4-[4-(6-Bromo-2-oxo-4H-benzo[d][1,3]oxazin-1-yl)-piperidine-1-sulfonyl]-phenyl}-propionic acid methyl ester |
| 365 | 6-Bromo-1-[1-(2,4-dinitro-benzenesulfonyl)-piperidin-4-yl]-1,4-dihydro-benzo[d][1,3]oxazin-2-one |
| 366 | 1-[1-(Benzo[b]thiophene-2-sulfonyl)-piperidin-4-yl]-6-bromo-1,4-dihydro-benzo[d][1,3]oxazin-2-one |
| 367 | 6-Bromo-1-[1-(2,5-difluoro-benzenesulfonyl)-piperidin-4-yl]-1,4-dihydro-benzo[d][1,3]oxazin-2-one |
| 368 | 6-Bromo-1-[1-(4-chloro-2,5-difluoro-benzenesulfonyl)-piperidin-4-yl]-1,4-dihydro-benzo[d][1,3]oxazin-2-one |
| 369 | 6-Bromo-1-[1-(2,4,5-trifluoro-benzenesulfonyl)-piperidin-4-yl]-1,4-dihydro-benzo[d][1,3]oxazin-2-one |
| 370 | 6-Bromo-1-[1-(2,6-difluoro-benzenesulfonyl)-piperidin-4-yl]-1,4-dihydro-benzo[d][1,3]oxazin-2-one |
| 371 | 6-Bromo-1-[1-(5-chloro-2,4-difluoro-benzenesulfonyl)-piperidin-4-yl]-1,4-dihydro-benzo[d][1,3]oxazin-2-one |
| 372 | 6-Bromo-1-[1-(2-chforo-benzenesulfonyl)-piperidin-4-yl]-1,4-dihydro-benzo[d][1,3]oxazin-2-one |
| 373 | 6-Bromo-1-[1-(2,3,4-trifluoro-benzenesulfonyl)-piperidin-4-yl]-1,4-dihydro-benzo[d][1,3]oxazin-2-one |
| 374 | N-{4-[4-(6-Bromo-2-oxo-4H-benzo[d][1,3]oxazin-1-yl)-piperidine-1-sulfonyl]-2-chloro-phenyl}-acetamide |
| 375 | 1-[1-(2,3,4-Trifluoro-benzenesutfonyl)-piperidin-4-yl]-1,4-dihydro-benzo[d][1,3]oxazin-2-one |
| 376 | 8-Methyl-1-[1-(2,3,4-trifluoro-benzenesulfonyl)-piperidin-4-yl]-1,4-dihydro-benzo[d][1,3]oxazin-2-one |
| 377 | 6-Chloro-1-[1-(2,3,4-trifluoro-benzenesulfonyl)-piperidin-4-yl]-1,4-dihydro-benzo[d][1,3]oxazin-2-one |
| 378 | 6-Methyl-1-[1-(2,3,4-trifluoro-benzenesulfonyl)-piperidin-4-yl]-1,4-dihydro-benzo[d][1,3]oxazin-2-one |
| 379 | N-{2-Chloro-4-[4-(6-methyl-2-oxo-4H-benzo[d][1,3]oxazin-1-yl)-piperidine-1-sulfonyl]-phenyl}-acetamide |
| 380 | 1-[1-(3,4-Difluoro-benzenesulfonyl)-piperidin-4-yl]-1,4-dihydro-benzo[d][1,3]oxazin-2-one |
| 381 | 1-[1-(3,4-Difluoro-benzenesulfonyl)-piperidin-4-yl]-8-methyl-1,4-dihydro-benzo[d][1,3]oxazin-2-one |
| 382 | 6-Chloro-1-[1-(3,4-difluoro-benzenesulfonyl)-piperidin-4-yl]-1,4-dihydro-benzo[d][1,3]oxazin-2-one |
| 383 | 1-[1-(3,4-Difluoro-benzenesulfonyl)-piperidin-4-yl]-6-methyl-1,4-dihydro-benzo[d][1,3]oxazin-2-one |
| 384 | 6-Bromo-1-[1-(3,4-difluoro-benzenesulfonyl)-piperidin-4-yl]-1,4-dihydro-benzo[d][1,3]oxazin-2-one |
| 385 | N-{2-Chloro-4-[4-(8-methyl-2-oxo-4H-benzo[d][1,3]oxazin-1-yl)-piperidine-1-sulfonyl]-phenyl}-acetamide |
| 386 | 1-[1-(2-Chloro-4,5-difluoro-benzenesulfonyl)-piperidin-4-yl]-1,4-dihydro-benzo[d][1,3]oxazin-2-one |
| 387 | 1-[1-(2-Chloro-4,5-difluoro-benzenesulfonyl)-piperidin-4-yl]-8-methyl-1,4-dihydro-benzo[d][1,3]oxazin-2-one |
| 388 | 6-Chloro-1-[1-(2-chloro-4,5-difluoro-benzenesulfonyl)-piperidin-4-yl]-1,4-dihydro-benzo[d][1,3]oxazin-2-one |
| 389 | 1-[1-(2-Chloro-4,5-difluoro-benzenesulfonyl)-piperidin-4-yl]-6-methyl-1,4-dihydro-benzo[d][1,3]oxazin-2-one |
| 390 | 6-Bromo-1-[1-(2-chloro-4,5-difluoro-benzenesulfonyl)-piperidin-4-yl]-1,4-dihydro-benzo[d][1,3]oxazin-2-one |
| 391 | N-{2-Chloro-4-[4-(2-oxo-4H-benzo[d][1,3]oxazin-1-yl)-piperidine-1-sulfonyl]-phenyl}-acetamide |
| 392 | 1-[1-(Benzo[1,2,5]oxadiazole-4-sulfonyl)-piperidin-4-yl]-1,4-dihydro-benzo[d][1,3]oxazin-2-one |
| 393 | 1-[1-(Benzo[1,2,5]oxadiazole-4-sulfonyl)-piperidin-4-yl]-8-methyl-1,4-dihydro-benzo[d][1,3]oxazin-2-one |
| 394 | 1-[1-(Benzo[1,2,5]oxadiazole-4-sulfonyf)-piperidin-4-yl]-6-chloro-1,4-dihydro-benzo[d][1,3]oxazin-2-one |
| 395 | 1-[1-(Benzo[1,2,5]oxadiazole-4-sulfonyl)-piperidin-4-yl]-6-methyl-1,4-dihydro-benzo[d][1,3]oxazin-2-one |
| 396 | 1-[1-(Benzo[1,2,5]oxadiazole-4-sulfonyl)-piperidin-4-yl]-6-bromo-1,4-dihydro-benzo[d][1,3]oxazin-2-one |
| 397 | N-{2-Chloro-4-[4-(6-chloro-2-oxo-4H-benzo[d][1,3]oxazin-1-yl)-piperidine-1-sulfonyl]-phenyl}-acetamide |
| 398 | 1-[1-(Benzo[1,2,5]thiadiazole-4-sulfonyl)-piperidin-4-yl]-1,4-dihydro-benzo[d][1,3]oxazin-2-one |
| 399 | 1-[1-(Benzo[1,2,5]thiadiazole-4-sulfonyl)-piperidin-4-yl]-8-methyl-1,4-dihydro-benzo[d][1,3]oxazin-2-one |
| 400 | 1-[1-(Benzo[1,2,5]thiadiazole-4-sulfonyl)-piperidin-4-yl]-6-chloro-1,4-dihydro-benzo[d][1,3]oxazin-2-one |
| 401 | 1-[1-(Benzo[1,2,5]thiadiazole-4-sulfonyl)-piperidin-4-yl]-6-methyl-1,4-dihydro-benzo[d][1,3]oxazin-2-one |
| 402 | 1-[1-(Benzo[1,2,5]thiadiazole-4-sulfonyl)-piperidin-4-yl]-6-bromo-1,4-dihydro-benzo[d][1,3]oxazin-2-one |
| 403 | 1-(1-Ethanesulfonyl-piperidin-4-yl)-8-methyl-1,4-dihydro-benzo[d][1,3]oxazin-2-one |
| 404 | 1-[1-(2,4-Difluoro-benzenesulfonyl)-piperidin-4-yl]-1,4-dihydro-benzo[d][1,3]oxazin-2-one |
| 405 | 1-[1-(2,4-Difluoro-benzenesulfonyl)-piperidin-4-yl]-8-methyl-1,4-dihydro-benzo[d][1,3]oxazin-2-one |
| 406 | 6-Chloro-1-[1-(2,4-difluoro-benzenesulfonyl)-piperidin-4-yl]-1,4-dihydro-benzo[d][1,3]oxazin-2-one |
| 407 | 1-[1-(2,4-Difluoro-benzenesulfonyl)-piperidin-4-yl]-6-methyl-1,4-dihydro-benzo[d][1,3]oxazin-2-one |
| 408 | 6-Bromo-1-[1-(2,4-difluoro-benzenesulfonyl)-piperidin-4-yl]-1,4-dihydro-benzo[d][1,3]oxazin-2-one |
| 409 | 8-Methyl-1-[1-(propane-2-sulfonyl)-piperidin-4-yl]-1,4-dihydro-benzo[d][1,3]oxazin-2-one |
| 410 | 1-[1-(3,4-Dichloro-benzenesulfonyl)-piperidin-4-yl]-1,4-dihydro-benzo[d][1,3]oxazin-2-one |
| 411 | 1-[1-(3,4-Dichloro-benzenesulfonyl)-piperidin-4-yl]-8-methyl-1,4-dihydro-benzo[d][1,3]oxazin-2-one |
| 412 | 6-Chloro-1-[1-(3,4-dichloro-benzenesulfonyl)-piperidin-4-yl]-1,4-dihydro-benzo[d][1,3]oxazin-2-one |
| 413 | 1-[1-(3,4-Dichloro-benzenesulfonyl)-piperidin-4-yl]-6-methyl-1,4-dihydro-benzo[d][1,3]oxazin-2-one |
| 414 | 6-Bromo-1-[1-(3,4-dichloro-benzenesulfonyl)-piperidin-4-yl]-1,4-dihydro-benzo[d][1,3]oxazin-2-one |
| 415 | 8-Methyl-1-[1-(propane-1-sulfonyl)-piperidin-4-yl]-1,4-dihydro-benzo[d][1,3]oxazin-2-one |
| 416 | 1-[1-(2-Chloro-6-methyl-benzenesulfonyl)-piperidin-4-yl]-1,4-dihydro-benzo[d][1,3]oxazin-2-one |
| 417 | 1-[1-(2-Chloro-6-methyl-benzenesulfonyl)-piperidin-4-yl]-8-methyl-1,4-dihydro-benzo[d][1,3]oxazin-2-one |
| 418 | 6-Chloro-1-[1-(2-chloro-6-methyl-benzenesulfonyl)-piperidin-4-yl]-1,4-dihydro-benzo[d][1,3]oxazin-2-one |
| 419 | 1-[1-(2-Chloro-6-methyl-benzenesulfonyl)-piperidin-4-yl]-6-methyl-1 ,4-dihydro-benzo[d][1,3]oxazin-2-one |
| 420 | 1-[1-(2-Chloro-6-methyl-benzenesulfonyl)-piperidin-4-yl]-6-methyl-1,4-dihydro-benzo[d][1,3]oxazin-2-one |
| 421 | 8-Methyl-1-[1-(2,3,5,6-tetramethyl-benzenesulfonyl)-piperidin-4-yl]-1,4-dihydro-benzo[d][1,3]oxazin-2-one |
| 422 | 1-[1-(2,3,4-Trichloro-benzenesulfonyl)-piperidin-4-yl]-1,4-dihydro-benzo[d][1,3]oxazin-2-one |
| 423 | 8-Methyl-1-[1-(2,3,4-trichloro-benzenesulfonyl)-piperidin-4-yl]-1,4-dihydro-benzo[d][1,3]oxazin-2-one |
| 424 | 6-Chloro-1-[1-(2,3,4-trichloro-benzenesulfonyl)-piperidin-4-yl]-1,4-dihydro-benzo[d][1,3]oxazin-2-one |
| 425 | 6-Methyl-1-[1-(2,3,4-trichloro-benzenesulfonyl)-piperidin-4-yl]-1,4-dihydro-benzo[d][1,3]oxazin-2-one |
| 426 | 6-Bromo-1-[1-(2,3,4-trichloro-benzenesulfonyl)-piperidin-4-yl]-1,4-dihydro-benzo[d][1,3]oxazin-2-one |
| 427 | 1-[1-(2,3,5,6-Tetramethyl-benzenesulfonyl)-piperidin-4-yl]-1,4-dihydro-benzo[d][1,3]oxazin-2-one |
| 428 | 1-[1-(Thiophene-3-sulfonyl)-piperidin-4-yl]-1,4-dihydro-benzo[d][1,3]oxazin-2-one |
| 429 | 8-Methyl-1-[1-(thiophene-3-sulfonyl)-piperidin-4-yl]-1,4-dihydro-benzo[d][1,3]oxazin-2-one |
| 430 | 6-Chloro-1-[1-(thiophene-3-sulfonyl)-piperidin-4-yl]-1,4-dihydro-benzo[d][1,3]oxazin-2-one |
| 431 | 6-Methyl-1-[1-(thiophene-3-sulfonyl)-piperidin-4-yl]-1,4-dihydro-benzo[d][1,3]oxazin-2-one |
| 432 | 6-Bromo-1-[1-(thiophene-3-sulfonyl)-piperidin-4-yl]-1,4-dihydro-benzo[d][1,3]oxazin-2-one |
| 433 | 6-Chloro-1-[1-(2,3,5,6-tetramethyl-benzenesulfonyl)-piperidin-4-yl]-1,4-dihydro-benzo[d][1,3]oxazin-2-one |
| 434 | 1-[1-(2,4,6-Trichloro-benzenesulfonyl)-piperidin-4-yl]-1,4-dihydro-benzo[d][1,3]oxazin-2-one |
| 435 | 8-Methyl-1-[1-(2,4,6-trichloro-benzenesulfonyt)-piperidin-4-yl]-1,4-dihydro-benzo[d][1,3]oxazin-2-one |
| 436 | 6-Chloro-1-[1-(2,4,6-trichloro-benzenesulfonyl)-piperidin-4-yl]-1,4-dihydro-benzo[d][1,3]oxazin-2-one |
| 437 | 6-Methyl-1-[1-(2,4,6-trichloro-benzenesulfonyl)-piperidin-4-yl]-1,4-dihydro-benzo[d][1,3]oxazin-2-one |
| 438 | 6-Bromo-1-[1-(2,4,6-trichloro-benzenesulfonyl)-piperidin-4-yl]-1,4-dihydro-benzo[d][1,3]oxazin-2-one |
| 439 | 6-Methyl-1-[1-(2,3,5,6-tetramethyl-benzenesulfonyl)-piperidin-4-yl]-1,4-dihydro-benzo[d][1,3]oxazin-2-one |
| 440 | 1-[1-(2-Bromo-4,6-difluoro-benzenesulfonyl)-piperidin-4-yl]-8-methyl-1,4-dihydro-benzo[d][1,3]oxazin-2-one |
| 441 | 1-[1-(2-Bromo-4,6-difluoro-benzenesuifonyl)-piperidin-4-yl]-6-chloro-1,4-dihydro-benzo[d][1,3]oxazin-2-one |
| 442 | 1-[1-(2-Bromo-4,6-difluoro-benzenesulfonyl)-piperidin-4-yl]-6-methyl-1,4-dihydro-benzo[d][1,3]oxazin-2-one |
| 443 | 6-Bromo-1-[1-(2-bromo-4,6-difluoro-benzenesulfonyl)-piperidin-4-yl]-1,4-dihydro-benzo[d][1,3]oxazin-2-one |
| 444 | 6-Bromo-1-[1-(2,3,5,6-tetramethyl-benzenesulfonyl)-piperidin-4-yl]-1,4-dihydro-benzo[d][1,3]oxazin-2-one |
| 445 | 1-[1-(4-Bromo-2-trifluoromethoxy-benzenesulfonyl)-piperidin-4-yl]-1,4-dihydro-benzo[d][1,3]oxazin-2-one |
| 446 | 1-[1-(4-Bromo-2-trifluoromethoxy-benzenesulfonyl)-piperidin-4-yl]-8-methyl-1,4-dihydro-benzo[d][1,3]oxazin-2-one |
| 447 | 1-[1-(4-Bromo-2-trifluoromethoxy-benzenesulfonyl)-piperidin-4-yl]-6-chloro-1,4-dihydro-benzo[d][1,3]oxazin-2-one |
| 448 | 1-[1-(4-Bromo-2-trifluoromethoxy-benzenesulfonyl)-piperidin-4-yl]-6-methyl-1,4-dihydro-benzo[d][1,3]oxazin-2-one |
| 449 | 6-Bromo-1-[1-(4-bromo-2-trifluoromethoxy-benzenesulfonyl)-piperidin-4-yl]-1,4-dihydro-benzo[d][1,3]oxazin-2-one |
| 450 | 1-[1-(4-Phenoxy-benzenesulfonyl)-piperidin-4-yl]-1,4-dihydro-benzo[d][1,3]oxazin-2-one |
| 451 | 1-[1-(3-Bromo-benzenesulfonyl)-piperidin-4-yl]-1,4-dihydro-benzo[d][1,3]oxazin-2-one |
| 452 | 1-[1-(3-Bromo-benzenesulfonyl)-piperidin-4-yl]-8-methyl-1,4-dihydro-benzo[d][1,3]oxazin-2-one |
| 453 | 1-[1-(3-Bromo-benzenesulfonyl)-piperidin-4-yl]-6-chloro-1,4-dihydro-benzo[d][1,3]oxazin-2-one |
| 454 | 1-[1-(3-Bromo-benzenesulfonyl)-piperidin-4-yl]-6-methyl-1,4-dihydro-benzo[d][1,3]oxazin-2-one |
| 455 | 6-Bromo-1-[1-(3-bromo-benzenesulfonyl)-piperidin-4-yl]-1,4-dihydro-benzo[d][1,3]oxazin-2-one |
| 456 | 8-Methyl-1-[1-(4-phenoxy-benzenesulfonyl)-piperidin-4-yl]-1,4-dihydro-benzo[d][1,3]oxazin-2-one |
| 457 | 1-[1-(4-tert-Butyl-benzenesulfonyl)-piperidin-4-yl]-1,4-dihydro-benzo[d][1,3]oxazin-2-one |
| 458 | 1-[1-(4-tert-Butyl-benzenesulfonyl)-piperidin-4-yl]-8-methyl-1,4-dihydro-benzo[d][1,3]oxazin-2-one |
| 459 | 1-[1-(4-tert-Butyl-benzenesulfonyl)-piperidin-4-yl]-6-chloro-1,4-dihydro-benzo[d][1,3]oxazin-2-one |
| 460 | 1-[1-(4-tert-Butyl-benzenesulfonyl)-piperidin-4-yl]-6-methyl-1,4-dihydro-benzo[d][1,3]oxazin-2-one |
| 461 | 6-Bromo-1-[1-(4-tert-butyl-benzenesulfonyl)-piperidin-4-yl]-1,4-dihydro-benzo[d][1,3]oxazin-2-one |
| 462 | 6-Chloro-1-[1-(4-phenoxy-benzenesulfonyl)-piperidin-4-yl]-1,4-dihydro-benzo[d][1,3]oxazin-2-one |
| 463 | 1-[1-(2-Bromo-4,6-difluoro-benzenesulfonyl)-piperidin-4-yl]-1,4-dihydro-benzo[d][1,3]oxazin-2-one |
| 464 | 1-[1-(2-Methanesulfonyl-benzenesulfonyl)-piperidin-4-yl]-8-methyl-1,4-dihydro-benzo[d][1,3]oxazin-2-one |
| 465 | 6-Chloro-1-[1-(2-methanesulfonyl-benzenesulfonyl)-piperidin-4-yl]-1,4-dihydro-benzo[d][1,3]oxazin-2-one |
| 466 | 1-[1-(2-Methanesulfonyl-benzenesulfonyl)-piperidin-4-yl]-6-methyl-1,4-dihydro-benzo[d][1,3]oxazin-2-one |
| 467 | 6-Bromo-1-[1-(2-methanesulfonyl)-benzenesulfonyl)-piperidin-4-yl]-1,4-dihydro-benzo[d][1,3]oxazin-2-one |
| 468 | 8-Methyl-1-[1-(4-propyl-benzenesulfonyl)-piperidin-4-yl]-1,4-dihydro-benzo[d][1,3]oxazin-2-one |
| 469 | 6-Chloro-1-[1-(4-propyl-benzenesulfonyl)-piperidin-4-yl]-1,4-dihydro-benzo[d][1,3]oxazin-2-one |
| 470 | 6-Methyl-1-[1-(4-propyl-benzenesulfonyl)-piperidin-4-yl]-1,4-dihydro-benzo[d][1,3]oxazin-2-one |
| 471 | 6-Bromo-1-[1-(4-propyl-benzenesulfonyl)-piperidin-4-yl]-1,4-dihydro-benzo[d][1,3]oxazin-2-one |
| 472 | 1-[1-(3-Chloro-2-methyl-benzenesulfonyl)-piperidin-4-yl]-8-methyl-1,4-dihydro-benzo[d][1,3]oxazin-2-one |
| 473 | 6-Chloro-1-[1-(3-chloro-2-methyl-benzenesulfonyl)-piperidin-4-yl]-1,4-dihydro-benzo[d][1,3]oxazin-2-one |
| 474 | 1-[1-(3-Chloro-2-methyl-benzenesulfonyl)-piperidin-4-yl]-6-methyl-1,4-dihydro-benzo[d][1,3]oxazin-2-one |
| 475 | 6-Bromo-1-[1-(3-chloro-2-methyl-benzenesulfonyl)-piperidin-4-yl]-1,4-dihydro-benzo[d][1,3]oxazin-2-one |
| 476 | 1-[1-(4-Butyl-benzenesulfonyl)-piperidin-4-yl]-8-methyl-1,4-dihydro-benzo[d][1,3]oxazin-2-one |
| 477 | 1-[1-(4-Butyl-benzenesulfonyl)-piperidin-4-yl]-6-chloro-1,4-dihydro-benzo[d][1,3]oxazin-2-one |
| 478 | 1-[1-(4-Butyl-benzenesulfonyl)-piperidin-4-yl]-6-methyl-1,4-dihydro-benzo[d][1,3]oxazin-2-one |
| 479 | 6-Bromo-1-[1-(4-butyl-benzenesulfonyl)-piperidin-4-yl]-1,4-dihydro-benzo[d][1,3]oxazin-2-one |
| 480 | 1-[1-(4-Bromo-3-methyl-benzenesulfonyl)-piperidin-4-yl]-8-methyl-1,4-dihydro-benzo[d][1,3]oxazin-2-one |
| 481 | 1-[1-(4-Bromo-3-methyl-benzenesulfonyl)-piperidin-4-yl]-6-chloro-1,4-dihydro-benzo[d][1,3]oxazin-2-one |
| 482 | 1-[1-(4-Bromo-3-methyl-benzenesulfonyl)-piperidin-4-yl]-6-methyl-1,4-dihydro-benzo[d][1,3]oxazin-2-one |
| 483 | 6-Bromo-1-[1-(4-bromo-3-methyl-benzenesulfonyl)-piperidin-4-yl]-1,4-dihydro-benzo[d][1,3]oxazin-2-one |
| 484 | 1-{1-[4-(1,1-Dimethyl-propyl)-benzenesulfonyl]-piperidin-4-yl}-8-methyl-1,4-dihydro-benzo[d][1,3]oxazin-2-one |
| 485 | 6-Chloro-1-{1-[4-(1,1-dimethyl-propyl)-benzenesulfonyl]-piperidin-4-yl}-1,4-dihydro-benzo[d][1,3]oxazin-2-one |
| 486 | 1-{1-[4-(1,1-Dimethyl-propyl)-benzenesulfonyl]-piperidin-4-yl}-6-methyl-1,4-dihydro-benzo[d][1,3]oxazin-2-one |
| 487 | 6-Bromo-1-{1-[4-(1,1-dimethyl-propyl)-benzenesulfonyl]-piperidin-4-yl}-1,4-dihydro-benzo[d][1,3]oxazin-2-one |
| 488 | 1-(1-Ethenesulfonyl-piperidin-4-yl)-8-methyl-1,4-d ihydro-benzo[d][1,3]oxazin-2-one |
| 489 | 3-[4-(8-Methyl-2-oxo-4H-benzo[d][1,3]oxazin-1-yl)-piperidine-1-sulfonyl]-benzoic acid |
| 490 | 3-[4-(6-Methyl-2-oxo-4H-benzo[d][1,3]oxazin-1-yl)-piperidine-1-sulfonyl]-benzoic acid |
| 491 | 3-[4-(6-Bromo-2-oxo-4H-benzo[d][1,3]oxazin-1-yl)-piperidine-1-sulfonyl]-benzoic acid |
| 492 | 1-[1-(3-Chloro-2-fluoro-benzenesulfonyl)-piperidin-4-yl]-8-methyl-1,4-dihydro-benzo[d][1,3]oxazin-2-one |
| 493 | 6-Chloro-1-[1-(3-chloro-2-fluoro-benzenesulfonyl)-piperidin-4-yl]-1,4-dihydro-benzo[d][1,3]oxazin-2-one |
| 494 | 1-[1-(3-Chloro-2-fluoro-benzenesulfonyl)-piperidin-4-yl]-6-methyl-1,4-dihydro-benzo[d][1,3]oxazin-2-one |
| 495 | 6-Bromo-1-[1-(3-chloro-2-fluoro-benzenesulfonyl)-piperidin-4-yl]-1,4-dihydro-benzo[d][1,3]oxazin-2-one |
| 496 | N-{4-Methyl-5-[4-(8-methyl-2-oxo-4H-benzo[d][1,3]oxazin-1-yl)-piperidine-1-sulfonyl]-thiazol-2-yl}-acetamide |
| 497 | N-{5-[4-(6-Chloro-2-oxo-4H-benzo[d][1,3]oxazin-1-yl)-piperidine-1-sulfonyl]-4-methyl-thiazol-2-yl}-acetamide |
| 498 | N-{4-Methyl-5-[4-(6-methyl-2-oxo-4H-benzo[d][1,3]oxazin-1-yl)-piperidine-1-sulfonyl]-thiazol-2-yl}-acetamide |
| 499 | N-{5-[4-(6-Bromo-2-oxo-4H-benzo[d][1,3]oxazin-1-yl)-piperidine-1-sulfonyl]-4-methyl-thiazol-2-yl}-acetamide |
| 500 | 1-[1-(2-Bromo-4-fluoro-benzenesulfonyl)-piperidin-4-yl]-8-methyl-1,4-dihydro-benzo[d][1,3]oxazin-2-one |
| 501 | 1-[1-(2-Bromo-4-fluoro-benzenesulfonyl)-piperidin-4-yl]-6-chloro-1,4-dihydro-benzo[d][1,3]oxazin-2-one |
| 502 | 1-[1-(2-Bromo-4-fluoro-benzenesulfonyl)-piperidin-4-yl]-6-methyl-1,4-dihydro-benzo[d][1,3]oxazin-2-one |
| 503 | 6-Bromo-1-[1-(2-bromo-4-fluoro-benzenesulfonyl)-piperidin-4-yl]-1,4-dihydro-benzo[d][1,3]oxazin-2-one |
| 504 | 1-[1-(5-Chloro-2-fluoro-benzenesulfonyl)-piperidin-4-yl]-8-methyl-1,4-dihydro-benzo[d][1,3]oxazin-2-one |
| 505 | 6-Chloro-1-[1-(5-chloro-2-fluoro-benzenesulfonyl)-piperidin-4-yl]-1,4-dihydro-benzo[d][1,3]oxazin-2-one |
| 506 | 1-[1-(5-Chloro-2-fluoro-benzenesulfonyl)-piperidin-4-yl]-6-methyl-1,4-dihydro-benzo[d][1,3]oxazin-2-one |
| 507 | 6-Bromo-1-[1-(5-chloro-2-fluoro-benzenesulfonyl)-piperidin-4-yl]-1,4-dihydro-benzo[d][1,3]oxazin-2-one |
| 508 | 1-[1-(4-Bromo-3-trifluoromethyl-benzenesulfonyl)-piperidin-4-yl]-8-methyl-1,4-dihydro-benzo[d][1,3]oxazin-2-one |
| 509 | 1-[1-(4-Bromo-3-trifluoromethyl-benzenesulfonyl)-piperidin-4-yl]-6-chloro-1,4-dihydro-benzo[d][1,3]oxazin-2-one |
| 510 | 1-[1-(4-Bromo-3-trifluoromethyl-benzenesuffonyl)-piperidin-4-yl]-6-methyl-1,4-dihydro-benzo[d][1,3]oxazin-2-one |
| 511 | 6-Bromo-1-[1-(4-bromo-3-trifluoromethyl-benzenesulfonyl)-piperidin-4-yl]-1,4-dihydro-benzo[d][1,3]oxazin-2-one |
| 512 | 1-[1-(2-Methanesulfonyl-benzenesulfonyl)-piperidin-4-yl]-1,4-dihydro-benzo[d][1,3]oxazin-2-one |
| 513 | 1-[1-(4-Propyl-benzenesulfonyl)-piperidin-4-yl]-1,4-dihydro-benzo[d][1,3]oxazin-2-one |
| 514 | 1-[1-(3-Chloro-2-methyl-benzenesulfonyl)-piperidin-4-yl]-1,4-dihydro-benzo[d][1,3]oxazin-2-one |
| 515 | 1-[1-(4-Butyl-benzenesulfonyl)-piperidin-4-yl]-1,4-dihydro-benzo[d][1,3]oxazin-2-one |
| 516 | 1-[1-(4-Bromo-3-methyl-benzenesulfonyl)-piperidin-4-yl]-1,4-dihydro-benzo[d][1,3]oxazin-2-one |
| 517 | 1-{1-[4-(1,1-Dimethyl-propyl)-benzenesulfonyl]-piperidin-4-yl)-1,4-dihydro-benzo[d][1,3]oxazin-2-one |
| 518 | N-{4-Methyl-5-[4-(2-oxo-4H-benzo[d][1,3]oxazin-1-yl)-piperidine-1-sulfonyl]-thiazol-2-yl}-acetamide |
| 519 | 1-[1-(3-Chloro-2-fluoro-benzenesulfonyl)-piperidin-4-yl]-1,4-dihydro-benzo[d][1,3]oxazin-2-one |
| 520 | 1-[1-(2-Bromo-4-fluoro-benzenesulfonyl)-piperidin-4-yl]-1,4-dihydro-benzo[d][1,3]oxazin-2-one |
| 521 | 1-[1-(4-Bromo-3-trifluoromethyl-benzenesulfonyl)-piperidin-4-yl]-1,4-dihydro-benzo[d][1,3]oxazin-2-one |
| 522 | 1-[1-(5-Chloro-2-fluoro-benzenesulfonyl)-piperidin-4-yl]-1,4-dihydro-benzo[d][1,3]oxazin-2-one |
| 523 | 1-[1-(Isoquinoline-5-sulfonyl)-piperidin-4-yl]-1,4-dihydro-benzo[d][1,3]oxazin-2-one |
| 524 | 6-Fluoro-1-[1-(2-methanesulfonyl-benzenesulfonyl)-piperidin-4-yl]-1,4-dihydro-benzo[d][1,3]oxazin-2-one |
| 525 | 6-Fluoro-1-[1-(4-propyl-benzenesulfonyl)-piperidin-4-yl]-1,4-dihydro-benzo[d][1,3]oxazin-2-one |
| 526 | 1-[1-(3-Chloro-2-methyl-benzenesulfonyl)-piperidin-4-yl]-6-fluoro-1,4-dihydro-benzo[d][1,3]oxazin-2-one |
| 527 | 1-[1-(4-Butyl-benzenesulfonyl)-piperidin-4-yl]-6-fluoro-1,4-dihydro-benzo[d][1,3]oxazin-2-one |
| 528 | 1-[1-(4-Bromo-3-methyl-benzenesulfonyl)-piperidin-4-yl]-6-fluoro-1,4-dihydro-benzo[d][1,3]oxazin-2-one |
| 529 | 1-{1-[4-(1,1-Dimethyl-propyl)-benzenesulfonyl]-piperidin-4-yl}-6-fluoro-1,4-dihydro-benzo[d][1,3]oxazin-2-one |
| 530 | N-{5-[4-(6-Fluoro-2-oxo-4H-benzo[d][1,3]oxazin-1-yl)-piperidine-1-sulfonyl]-4-methyl-thiazol-2-yl}-acetamide |
| 531 | 1-[1-(3-Chloro-2-fluoro-benzenesulfonyl)-piperidin-4-yl]-6-fluoro-1,4-dihydro-benzo[d][1,3]oxazin-2-one |
| 532 | 1-[1-(2-Bromo-4-fluoro-benzenesulfonyl)-piperidin-4-yl]-6-fluoro-1,4-dihydro-benzo[d][1,3]oxazin-2-one |
| 533 | 1-[1-(4-Bromo-3-trifluoromethyl-benzenesulfonyl)-piperidin-4-yl]-6-fluoro-1,4-dihydro-benzo[d][1,3]oxazin-2-one |
| 534 | 1-[1-(5-Chloro-2-fluoro-benzenesulfonyl)-piperidin-4-yl]-6-fluoro-1,4-dihydro-benzo[d][1,3]oxazin-2-one |
| 535 | 6-Fluoro-1-[1-(isoquinoline-5-sulfonyl)-piperidin-4-yl]-1,4-dihydro-benzo[d][1,3]oxazin-2-one |
| 536 | 6-Fluoro-1-[1-(quinoline-8-sulfonyl)-piperidin-4-yl]-1,4-dihydro-benzo[d][1,3]oxazin-2-one |
| 537 | 1-[1-(5-Chloro-3-methyl-benzo[b]thiophene-2-sulfonyl)-piperidin-4-y1]-6-fluoro-1,4-dihydro-benzo[d][1,3]oxazin-2-one |
| 538 | 6-Fluoro-1-[1-(naphthalene-1-sulfonyl)-piperidin-4-yl]-1,4-dihydro-benzo[d][1,3]oxazin-2-one |
| 539 | 6-Fluoro-1-[1-(naphthalene-2-sulfonyl)-piperidin-4-yl]-1,4-dihydro-benzo[d][1,3]oxazin-2-one |
| 540 | 1-[1-(Benzo[b]thiophene-2-sulfonyl)-piperidin-4-yl]-6-fluoro-1,4-dihydro-benzo[d][1,3]oxazin-2-one |
| 541 | 1-[1-(Benzo[b]thiophene-3-sulfonyl)-piperidin-4-yl]-6-fluoro-1,4-dihydro-benzo[d][1,3]oxazin-2-one |
| 542 | 8-Methoxy-1-[1-(quinoline-8-sulfonyl)-piperidin-4-yl]-1,4-dihydro-benzo[d][1,3]oxazin-2-one |
| 543 | 1-[1-(5-Chloro-3-methyl-benzo[b]thiophene-2-sulfonyl)-piperidin-4-yl]-8-methoxy-1,4-dihydro-benzo[d][1,3]oxazin-2-one |
| 544 | 8-Methoxy-1-[1-(naphthalene-1-sulfonyl)-piperidin-4-yl]-1,4-dihydro-benzo[d][1,3]oxazin-2-one |
| 545 | 8-Methoxy-1-[1-(naphthalene-2-sulfonyl)-piperidin-4-yl]-1,4-dihydro-benzo[d][1,3]oxazin-2-one |
| 546 | 1-[1-(Benzo[b]thiophene-2-sulfonyl)-piperidin-4-yl]-8-methoxy-1,4-dihydro-benzo[d][1,3]oxazin-2-one |
| 547 | 1-[1-(Benzo[b]thiophene-3-sulfonyl)-piperidin-4-yl]-8-methoxy-1,4-dihydro-benzo[d][1,3]oxazin-2-one |
| 548 | 5-Chloro-1-[1-(2-methanesulfonyl-benzenesulfonyl)-piperidin-4-yl]-1,4-dihydro-benzo[d][1,3]oxazin-2-one |
| 549 | 5-Chloro-1-[1-(4-propyl-benzenesulfonyl)-piperidin-4-yl]-1,4-dihydro-benzo[d][1,3]oxazin-2-one |
| 550 | 5-Chloro-1-[1-(3-chloro-2-methyl-benzenesulfonyl)-piperidin-4-yl]-1,4-dihydro-benzo[d][1,3]oxazin-2-one |
| 551 | 1-[1-(4-Butyl-benzenesulfonyl)-piperidin-4-yl]-5-chloro-1,4-dihydro-benzo[d][1,3]oxazin-2-one |
| 552 | 1-[1-(4-Bromo-3-methyl-benzenesulfonyl)-piperidin-4-yl]-5-chloro-1,4-dihydro-benzo[d][1,3]oxazin-2-one |
| 553 | 5-Chloro-1-{1-[4-(1,1-dimethyl-propyl)-benzenesulfonyl]-piperidin-4-yl}-1,4-dihydro-benzo[d][1,3]oxazin-2-one |
| 554 | N-{5-[4-(5-Chloro-2-oxo-4H-benzo[d][1,3]oxazin-1-yl)-piperidine-1-sulfonyl]-4-methyl-thiazol-2-yl}-acetamide |
| 555 | 5-Chloro-1-[1-(3-chloro-2-fluoro-benzenesulfonyl)-piperidin-4-yl]-1,4-dihydro-benzo[d][1,3]oxazin-2-one |
| 556 | 1-[1-(2-Bromo-4-fluoro-benzenesulfonyl)-piperidin-4-yl]-5-chloro-1,4-dihydro-benzo[d][1,3]oxazin-2-one |
| 557 | 1-[1-(4-Bromo-3-trifluoromethyl-benzenesulfonyl)-piperidin-4-yl]-5-chloro-1,4-dihydro-benzo[d][1,3]oxazin-2-one |
| 558 | 5-Chloro-1-[1-(5-chloro-2-fluoro-benzenesulfonyl)-piperidin-4-yl]-1,4-dihydro-benzo[d][1,3]oxazin-2-one |
| 559 | 5-Chloro-1-[1-(isoquinoline-5-sulfonyl)-piperidin-4-yl]-1,4-dihydro-benzo[d][1,3]oxazin-2-one |
| 560 | 1-[1-(2-Methanesulfonyl-benzenesulfonyl)-piperidin-4-yl]-8-methoxy-1,4-dihydro-benzo[d][1,3]oxazin-2-one |
| 561 | 1-[1-(2-Methanesulfonyl-benzenesulfonyl)-piperidin-4-yl]-8-methoxy-1,4-dihydro-benzo[d][1,3]oxazin-2-one |
| 562 | 1-[1-(3-Chloro-2-methyl-benzenesulfonyl)-piperidin-4-yl]-8-methoxy-1,4-dihydro-benzo[d][1,3]oxazin-2-one |
| 563 | 1-[1-(4-Butyl-benzenesulfonyl)-piperidin-4-yl]-8-methoxy-1,4-dihydro-benzo[d][1,3]oxazin-2-one |
| 564 | 1-[1-(4-Bromo-3-methyl-benzenesulfonyl)-piperidin-4-yl]-8-methoxy-1,4-dihydro-benzo[d][1,3]oxazin-2-one |
| 565 | 1-{1-[4-(1,1-Dimethyl-propyl)-benzenesulfonyl]-piperidin-4-yl}-8-methoxy-1,4-dihydro-benzo[d][1,3]oxazin-2-one |
| 566 | N-{5-[4-(8-Methoxy-2-oxo-4H-benzo[d][1,3]oxazin-1-yl)-piperidine-1-sulfonyl]-4-methyl-thiazol-2-yl}-acetamide |
| 567 | 1-[1-(3-Chloro-2-fluoro-benzenesulfonyl)-piperidin-4-yl]-8-methoxy-1,4-dihydro-benzo[d][1,3]oxazin-2-one |
| 568 | 1-[1-(2-Bromo-4-fluoro-benzenesulfonyl)-piperidin-4-yl]-8-methoxy-1,4-dihydro-benzo[d][1,3]oxazin-2-one |
| 569 | 1-[1-(4-Bromo-3-trifluoromethyl-benzenesulfonyl)-piperidin-4-yl]-8-methoxy-1,4-dihydro-benzo[d][1,3]oxazin-2-one |
| 570 | 1-[1-(5-Chloro-2-fluoro-benzenesulfonyl)-piperidin-4-yl]-8-methoxy-1,4-dihydro-benzo[d][1,3]oxazin-2-one |
| 571 | 1-[1-(Isoquinoline-5-sulfonyl)piperidin-4-yl]-8-methoxy-1,4-dihydro-benzo[d][1,3]oxazin-2-one; hydrochloride |
| 572 | 1-[1-(4-Methyl-naphthalene-1-sulfonyl)-piperidin-4-yl]-1,4-dihydro-benzo[d][1,3]oxazin-2-one |
| 573 | 6-Chloro-1-[1-(4-methyl-naphthalene-1-sulfonyl)-piperidin-4-yl]-1,4-dihydro-benzo[d][1,3]oxazin-2-one |
| 574 | 6-Methyl-1-[1-(4-methyl-naphthalene-1-sulfonyl)-piperidin-4-yl]-1,4-dihydro-benzo[d][1,3]oxazin-2-one |
| 575 | 8-Methyl-1-[1-(4-methyl-naphthalene-1-sulfonyl)-piperidin-4-yl]-1,4-dihydro-benzo[d][1,3]oxazin-2-one |
| 576 | 6-Fluoro-1-[1-(4-methyl-naphthalene-1-sulfonyl)-piperidin-4-yl]-1,4-dihydro-benzo[d][1,3]oxazin-2-one |
| 577 | 8-Methoxy-1-[1-(4-methyl-naphthalene-1-sulfonyl)-piperidin-4-yl]-1,4-dihydro-benzo[d][1,3]oxazin-2-one |
| 578 | 5-Chloro-1-[1-(4-methyl-naphthalene-1-sulfonyl)-piperidin-4-yl]-1,4-dihydro-benzo[d][1,3]oxazin-2-one |
| 579 | 5-Chloro-1-[1-(naphthalene-1-sulfonyl)-piperidin-4-yl]-1,4-dihydro-benzo[d][1,3]oxazin-2-one |
| 580 | 5-Chloro-1-[1-(naphthalene-2-sulfonyl)-piperidin-4-yl]-1,4-dihydro-benzo[d][1,3]oxazin-2-one |
| 581 | 5-Chloro-1-[1-(quinoline-8-sulfonyl)-piperidin-4-yl]-1,4-dihydro-benzo[d][1,3]oxazin-2-one |
| 582 | 5-Chloro-1-[1-(5-chloro-3-methyl-benzo[b]thiophene-2-sulfonyl)-piperidin-4-yl]-1,4-dihydro-benzo[d][1,3]oxazin-2-one |
| 583 | 1-[1-(Benzo[b]thiophene-2-sulfonyl)-piperidin-4-yl]-5-chloro-1,4-dihydro-benzo[d][1,3]oxazin-2-one |
| 584 | 1-[1-(Benzo[b]thiophene-3-sulfonyl)-piperidin-4-yl]-5-chloro-1,4-dihydro-benzo[d][1,3]oxazin-2-one |
| 585 | 6-Bromo-1-[1-(4-methyl-naphthalene-1-sulfonyl)-piperidin-4-yl]-1,4-dihydro-benzo[d][1,3]oxazin-2-one |
| 586 | 2-Chloro-4-fluoro-5-[4-(8-methyl-2-oxo-4H-benzo[d][1,3]oxazin-1-yl)-piperidine-1-sulfonyl]-benzoic acid |
| 587 | 2-Chloro-5-[4-(6-chloro-2-oxo-4H-benzo[d][1,3]oxazin-1-yl)-piperidine-1-sulfonyl]-4-fluoro-benzoic acid |
| 588 | 2-Chloro-4-fluoro-5-[4-(6-methyl-2-oxo-4H-benzo[d][1,3]oxazin-1-yl)-piperidine-1-sulfonyl]-benzoic acid |
| 589 | 2-Chloro-4-fluoro-5-[4-(2-oxo-4H-benzo[d][1,3]oxazin-1-yl)-piperidine-1-sulfonyl]-benzoic acid |
| 590 | 2-Chloro-4-fluoro-5-[4-(8-methoxy-2-oxo-4H-benzo[d][1,3]oxazin-1-yl)-piperidine-1-sulfonyl]-benzoic acid |
| 591 | 2-Chloro-5-[4-(5-chloro-2-oxo-4H-benzo[d][1,3]oxazin-1-yl)-piperidine-1-sulfonyl]-4-fluoro-benzoic acid |
| 592 | 3-[4-(2-Oxo-4H-benzo[d][1,3]oxazin-1-yl)-piperidine-1-sulfonyl]-benzoic acid |
| 593 | 3-[4-(8-Methoxy-2-oxo-4H-benzo[d][1,3]oxazin-1-yl)-piperidine-1-sulfonyl]-benzoic acid |
| 594 | 3-[4-(5-Chloro-2-oxo-4H-benzo[d][1,3]oxazin-1-yl)-piperidine-1-sulfonyl]-benzoic acid |
| 595 | 1-[1-(Isoquinoline-5-sulfonyl)-piperidin-4-yl]-8-methyl-1,4-dihydro-benzo[d][1,3]oxazin-2-one; hydrochloride |
| 596 | 6-Chloro-1-[1-(isoquinoline-5-sulfonyl)-piperidin-4-yl]-1,4-dihydro-benzo[d][1,3]oxazin-2-one; hydrochloride |
| 597 | -[1-(Isoquinoline-5-sulfonyl)-piperidin-4-yl]-6-methyl-1,4-dihydro-benzo[d][1,3]oxazin-2-one;hydrochloride |
| 598 | 6,7-Difluoro-1-[1-(quinoline-8-sulfonyl)-piperidin-4-yl]-1,4-dihydro-benzo[d][1,3]oxazin-2-one |
| 599 | 1-[1-(5-Chloro-3-methyl-benzo[b]thiophene-2-sulfonyl)-piperidin-4-yl]-6,7-difluoro-1,4-dihydro-benzo[d][1,3]oxazin-2-one |
| 600 | 6,7-Difluoro-1-[1-(naphthalene-1-sulfonyl)-piperidin-4-yl]-1,4-dihydro-benzo[d][1,3]oxazin-2-one |
| 601 | 6,7-Difluoro-1-[1-(naphthalene-2-sulfonyl)-piperidin-4-yl]-1,4-dihydro-benzo[d][1,3]oxazin-2-one |
| 602 | 1-[1-(Benzo[b]thiophene-2-sulfonyl)-piperidin-4-yl]-6,7-difluoro-1,4-dihydro-benzo[d][1,3]oxazin-2-one |
| 603 | 1-[1-(Benzo[b]thiophene-3-sulfonyl)-piperidin-4-yl]-6,7-difluoro-1,4-dihydro-benzo[d][1,3]oxazin-2-one |
| 604 | 1-[1-(5-Dimethylamino-naphthalene-1-sulfonyl)-piperidin-4-yl]-6,7-difluoro-1,4-dihydro-benzo[d][1,3]oxazin-2-one |
| 605 | 1-[1-(Biphenyl-4-sulfonyl)-pipendin-4-yl]-6,7-difluoro-1,4-dihydro-benzo[d][1,3]oxazin-2-one |
| 606 | 1-[1-(Benzo[1,2,5]thiadiazole-4-sulfonyl)-piperidin-4-yl]-6,7-difluoro-1,4-dihydro-benzo[d][1,3]oxazin-2-one |
| 607 | 1-[1-(Benzo[1,2,5]oxadiazole-4-sulfonyl)-piperidin-4-yl]-6,7-difluoro-1,4-dihydro-benzo[d][1,3]oxazin-2-one |
| 608 | 1-[1-(7-Chloro-benzo[1,2,5]oxadiazole-4-sulfonyl)-piperidin-4-yl]-6,7-difluoro-1,4-dihydro-benzo[d][1,3]oxazin-2-one |
| 609 | 6,7-Difluoro-1-[1-( 4-methyl-naphthalene-1-sulfonyl)-piperidin-4-yl]-1,4-dihydro-benzo[d][1,3]oxazin-2-one |
| 610 | 1-[1-(4-Chloro-naphthalene-1-sulfonyl)-piperidin-4-yi]-1,4-dihydro-benzo[d][1,3]oxazin-2-one |
| 611 | 1-[1-(4-Fluoro-naphthalene-1-sulfonyl)-piperidin-4-yl]-1,4-dihydro-benzo[d][1,3]oxazin-2-one |
| 612 | 1-[1-(Dibenzofuran-2-sulfonyl)-piperidin-4-yl]-1,4-dihydro-benzo[d][1,3]oxazin-2-one |
| 613 | 1-[1-(2,3-Dihydro-benzofuran-5-sulfonyl)-piperidin-4-yl]-1,4-dihydro-benzo[d][1,3]oxazin-2-one |
| 614 | 1-[1-(Biphenyl-2-sulfonyl)-piperidin-4-yl]-1,4-dihydro-benzo[d][1,3]oxazin-2-one |
| 615 | 1-[1-(5-Isoxazol-5-yl-thiophene-2-sulfonyl)-piperidin-4-yl]-1,4-dihydro-benzo[d][1,3]oxazin-2-one |
| 616 | 1-[1-(4-Chloro-naphthalene-1-sulfonyl)-piperidin-4-yl]-8-methyl-1,4-dihydro-benzo[d][1,3]oxazin-2-one |
| 617 | 1-[1-(4-Fluoro-naphthalene-1-sulfonyl)-piperidin-4-yl]-8-methyl-1,4-dihydro-benzo[d][1,3]oxazin-2-one |
| 618 | 1-[1-(Dibenzofuran-2-sulfonyl)-piperidin-4-yl]-8-methyl-1,4-dihydro-benzo[d][1,3]oxazin-2-one |
| 619 | 1-[1-(2,3-Dihydro-benzofuran-5-sulfonyl)-piperidin-4-yl]-8-methyl-1,4-dihydro-benzo[d][1,3]oxazin-2-one |
| 620 | 1-[1-(Biphenyl-2-sulfonyl)-piperidin-4-yl]-8-methyl-1,4-dihydro-benzo[d][1,3]oxazin-2-one |
| 621 | 1-[1-(5-Isoxazol-5-yl-thiophene-2-sulfonyl)-piperidin-4-yl]-8-methyl-1,4-dihydro-benzo[d][1,3]oxazin-2-one |
| 622 | 5-Chloro-1-[1-(4-chloro-naphthalene-1-sulfonyl)-piperidin-4-yl]-1,4-dihydro-benzo[d][1,3]oxazin-2-one |
| 623 | 5-Chloro-1-[1-(4-fluoro-naphthalene-1-sulfonyl)-piperidin-4-yl]-1,4-dihydro-benzo[d][1,3]oxazin-2-one |
| 624 | 5-Chloro-1-[1-(dibenzofuran-2-sulfonyl)-piperidin-4-yl]-1,4-dihydro-benzo[d][1,3]oxazin-2-one |
| 625 | 5-Chloro-1-[1-(2,3-dihydro-benzofuran-5-sulfonyl)-piperidin-4-yl]-1,4-dihydro-benzo[d][1,3]oxazin-2-one |
| 626 | 1-[1-(Biphenyl-2-sulfonyl)-piperidin-4-yl]-5-chloro-1,4-dihydro-benzo[d][1,3]oxazin-2-one |
| 627 | 5-Chloro-1-[1-(5-isoxazol-5-yl-thiophene-2-sulfonyl)-piperidin-4-yl]-1,4-dihydro-benzo[d][1,3]oxazin-2-one |
| 628 | 1-[1-(4-Chloro-naphthalene-1-sulfonyl)-piperidin-4-yl]-8-methoxy-1,4-dihydro-benzo[d][1,3]oxazin-2-one |
| 629 | 1-[1-(4-Fluoro-naphthalene-1-sulfonyl)-piperidin-4-yl]-8-methoxy-1,4-dihydro-benzo[d][1,3]oxazin-2-one |
| 630 | 1-[1-(Dibenzofuran-2-sulfonyl)-piperidin-4-yl]-8-methoxy-1,4-dihydro-benzo[d][1,3]oxazin-2-one |
| 631 | 1-[1-(2,3-Dihydro-benzofuran-5-sulfonyl)-piperidin-4-yl]-8-methoxy-1,4-dihydro-benzo[d][1,3]oxazin-2-one |
| 632 | 1-[1-(Biphenyl-2-sulfonyl)-piperidin-4-yl]-8-methoxy-1,4-dihydro-benzo[d][1,3]oxazin-2-one |
| 633 | 1-[1-(5-Isoxazol-5-yl-thiophene-2-sulfonyl)-piperidin-4-yl]-8-methoxy-1,4-dihydro-benzo[d][1,3]oxazin-2-one |
| 634 | 6-Chloro-1-[1-(4-chloro-naphthalene-1-sulfonyl)-piperidin-4-yl]-1,4-dihydro-benzo[d][1,3]oxazin-2-one |
| 635 | 6-Chloro-1-[1-(4-fluoro-naphthalene-1-sulfonyl)-piperidin-4-yl]-1,4-dihydro-benzo[d][1,3]oxazin-2-one |
| 636 | 6-Chloro-1-[1-(dibenzofuran-2-sulfonyl)-piperidin-4-yl]-1,4-dihydro-benzo[d][1,3]oxazin-2-one |
| 637 | 6-Chloro-1-[1-(2,3-dihydro-benzofuran-5-sulfonyl)-piperidin-4-yl]-1,4-dihydro-benzo[d][1,3]oxazin-2-one |
| 638 | 1-[1-(Biphenyl-2-sulfonyl)-piperidin-4-yl]-6-chloro-1,4-dihydro-benzo[d][1,3]oxazin-2-one |
| 639 | 6-Chloro-1-[1-(5-isoxazol-5-yl-thiophene-2-sulfonyl)-piperidin-4-yl]-1,4-dihydro-benzo[d][1,3]oxazin-2-one |
| 640 | 1-[1-(4-Chloro-naphthalene-1-sulfonyl)-piperidin-4-yl]-6-methyl-1,4-dihydro-benzo[d][1,3]oxazin-2-one |
| 641 | 1-[1-(4-Fluoro-naphthalene-1-sulfonyl)-piperidin-4-yl]-6-methyl-1,4-dihydro-benzo[d][1,3]oxazin-2-one |
| 642 | 1-[1-(Dibenzofuran-2-sulfonyl)-piperidin-4-yl]-6-methyl-1,4-dihydro-benzo[d][1,3]oxazin-2-one |
| 643 | 1-[1-(2,3-Dihydro-benzofuran-5-sulfonyl)-piperidin-4-yl]-6-methyl-1,4-dihydro-benzo[d][1,3]oxazin-2-one |
| 644 | 1-[1-(Biphenyl-2-sulfonyl)-piperidin-4-yl]-6-methyl-1,4-dihydro-benzo[d][1,3]oxazin-2-one |
| 645 | 1-[1-(5-Isoxazol-5-yl-thiophene-2-sulfonyl)-piperidin-4-yl]-6-methyl-1,4-dihydro-benzo[d][1,3]oxazin-2-one |
| 646 | 1-[1-(4-Chloro-naphthalene-1-sulfonyl)-piperidin-4-yl]-6,7-difluoro-1,4-dihydro-benzo[d][1,3]oxazin-2-one |
| 647 | 6,7-Difluoro-1-[1-(4-fluoro-naphthalene-1-sulfonyl)-piperidin-4-yl]-1,4-dihydro-benzo[d][1,3]oxazin-2-one |
| 648 | 1-[1-(Dibenzofuran-2-sulfonyl)-piperidin-4-yl]-6,7-difluoro-1,4-dihydro-benzo[d][1,3]oxazin-2-one |
| 649 | 1-[1-(2,3-Dihydro-benzofuran-5-sulfonyl)-piperidin-4-yl]-6,7-difluoro-1,4-dihydro-benzo[d][1,3]oxazin-2-one |
| 650 | 1-[1-(Biphenyl-2-sulfonyl)-pipeddin-4-yl]-6,7-difluoro-1,4-dihydro-benzo[d][1,3]oxazin-2-one |
| 651 | 6,7-Difluoro-1-[1-(5-isoxazol-5-yl-thiophene-2-sulfonyl)-piperidin-4-yl]-1,4-dihydro-benzo[d][1,3]oxazin-2-one |
| 652 | 1-[1-(1,2-Dimethyl-1H-imidazole-4-sulfonyl)-piperidin-4-yl]-1,4-dihydro-benzo[d][1,3]oxazin-2-one |
| 653 | 1-[1-(5-Methyl-benzo[1,2,5]thiadiazole-4-sulfonyl)-piperidin-4-yl]-1,4-dihydro-benzo[d][1,3]oxazin-2-one |
| 654 | 1-[1-(3,5-Dimethyl-isoxazole-4-sulfonyl)-piperidin-4-yl]-1,4-dihydro-benzo[d][1,3]oxazin-2-one |
| 655 | 1-[1-(1,2-Dimethyl-1 H-imidazole-4-sulfonyl)-piperidin-4-yl]-8-methyl-1,4-dihydro-benzo[d][1,3]oxazin-2-one |
| 656 | 8-Methyl-1-[1-(5-methyl-benzo[1,2,5]thiadiazole-4-sulfonyl)-piperidin-4-yl]-1,4-dihydro-benzo[d][1,3]oxazin-2-one |
| 657 | 1-[1-(3,5-Dimethyl-isoxazole-4-sulfonyl)-piperidin-4-yl]-8-methyl-1,4-dihydro-benzo[d][1,3]oxazin-2-one |
| 658 | 6-Chloro-1-[1-(1,2-dimethyl-1H-imidazole-4-sulfonyl)-piperidin-4-yl]-1,4-dihydro-benzo[d][1,3]oxazin-2-one |
| 659 | 6-Chloro-1-[1-(5-methyl-benzo[1,2,5]thiadiazole-4-sulfonyl)-piperidin-4-yl]-1,4-dihydro-benzo[d][1,3]oxazin-2-one |
| 660 | 6-Chloro-1-[1-(3,5-dimethyl-isoxazole-4-sulfonyl)-piperidin-4-yl]-1,4-dihydro-benzo[d][1,3]oxazin-2-one |
| 661 | 1-[1-(1,2-Dimethyl-1H-imidazole-4-sulfonyl)-piperidin-4-yl]-8-methoxy-1,4-dihydro-benzo[d][1,3]oxazin-2-one |
| 662 | 8-Methoxy-1-[1-(5-methyl-benzo[1,2,5]thiadiazole-4-sulfonyl)-piperidin-4-yl]-1,4-dihydro-benzo[d][1,3]oxazin-2-one |
| 663 | 1-[1-(3,5-Dimethyl-isoxazole-4-sulfonyl)-piperidin-4-yl]-8-methoxy-1,4-dihydro-benzo[d][1,3]oxazin-2-one |
| 664 | 5-Chloro-1-[1-(1,2-dimethyl-1H-imidazole-4-sulfonyl)-piperidin-4-yl]-1,4-dihydro-benzo[d][1,3]oxazin-2-one |
| 665 | 5-Chloro-1-[1-(5-methyl-benzo[1,2,5]thiadiazole-4-sulfonyl)-piperidin-4-yl]-1,4-dihydro-benzo[d][1,3]oxazin-2-one |
| 666 | 5-Chloro-1-[1-(3, 5-dimethyl-isoxazole-4-sulfonyl)-piperidin-4-yl]-1,4-dihydro-benzo[d][1,3]oxazin-2-one |
| 667 | 1-[1-(1,2-Dimethyl-1H-imidazole-4-sulfonyl)-piperidin-4-yl]-6-methyl-1,4-dihydro-benzo[d][1,3]oxazin-2-one |
| 668 | 6-Methyl-1-[1-5-methyl-benzo[1,2,5]thiadiazole-4-sulfonyl)-piperidin-4-yl]-1,4-dihydro-benzo[d][1,3]oxazin-2-one |
| 669 | 1-[1-(3,5-Dimethyl-isoxazole-4-sulfonyl)-piperidin-4-yl]-6-methyl-1,4-dihydro-benzo[d][1,3]oxazin-2-one |
| 670 | 1-[1-(1,2-Dimethyl-1 H-imidazole-4-sulfonyl)-piperidin-4-yl]-6-fluoro-1,4-dihydro-benzo[d][1,3]oxazin-2-one |
| 671 | 6-Fluoro-1-[1-(5-methyl-benzo[1,2,5]thiadiazole-4-sulfonyl)-piperidin-4-yl]-1,4-dihydro-benzo[d][1,3]oxazin-2-one |
| 672 | 1-[1-(3,5-Dimethyl-isoxazole-4-sulfonyl)-pipeidin-4-yl]-6-fluoro-1,4-dihydro-benzo[d][1,3]oxazin-2-one |
| 673 | 1-[1-(1,2-Dimethyl-1H-imidazole-4-sulfonyl)-piperidin-4-yl]-6,7-difluoro-1,4-dihydro-benzo[d][1,3]oxazin-2-one |
| 674 | 6,7-Difluoro-1-[1-(5-methyl-benzo[1,2,5]thiadiazole-4-sulfonyl)-pipeidin-4-yl]-1,4-dihydro-benzo[d][1,3]oxazin-2-one |
| 675 | 1-[1-(3,5-Dimethyl-isoxazole-4-sulfonyl)-piperidin-4-yl]-6,7-difluoro-1,4-dihydro-benzo[d][1,3]oxazin-2-one |
| 676 | 1-[1-(5-Chloro-naphthalene-1-sulfonyl)-piperidin-4-yl]-1,4-dihydro-benzo[d][1,3]oxazin-2-one |
| 677 | 1-[1-(5-Chloro-naphthalene-2-sulfonyl)-piperidin-4-yl]-1,4-dihydro-benzo[d][1,3]oxazin-2-one |
| 678 | N-{5-[4-(2-Oxo-4H-benzo[d][1,3]oxazin-1-yl)-piperidine-1-sulfonyl]-naphthalen-1-yl}-acetamide |
| 679 | 1-[1-(5-Chloro-naphthalene-1-sulfonyl)-piperidin-4-yl]-8-methyl-1,4-dihydro-benzo[d][1,3]oxazin-2-one |
| 680 | 1-[1-(5-Chloro-naphthalene-2-sulfonyl)-piperidin-4-yl]-8-methyl-1,4-dihydro-benzo[d][1,3]oxazin-2-one |
| 681 | N-{5-[4-(8-Methyl-2-oxo-4H-benzo[d][1,3]oxazin-1-yl)-piperidine-1-sulfonyl]-naphthalen-1-yl}-acetamide |
| 682 | 5-Chloro-1-[1-(5-chloro-naphthalene-1-sulfonyl)-piperidin-4-yl]-1,4-dihydro-benzo[d][1,3]oxazin-2-one |
| 683 | 5-Chloro-1-[1-(5-chloro-naphthalene-2-sulfonyl)-piperidin-4-yl]-1,4-dihydro-benzo[d][1,3]oxazin-2-one |
| 684 | N-{5-[4-(5-Chloro-2-oxo-4H-benzo[d][1,3]oxazin-1-yl)-piperidine-1-sulfonyl]-naphthalen-1-yl}-acetamide |
| 685 | 1-[1-(5-Chloro-naphthalene-1-sulfonyl)-piperidin-4-yl]-8-methoxy-1,4-dihydro-benzo[d][1,3]oxazin-2-one |
| 686 | 1-[1-(5-Chloro-naphthalene-2-sulfonyl)-piperidin-4-yl]-8-methoxy-1,4-dihydro-benzo[d][1,3]oxazin-2-one |
| 687 | N-{5-[4-(8-Methoxy-2-oxo-4H-benzo[d][1,3]oxazin-1-yl)-piperidine-1-sulfonyl]-naphthalen-1-yl}-acetamide |
| 688 | 2,5-Dimethyl-4-[4-(8-methyl-2-oxo-4H-benzo[d][1,3]oxazin-1-yl)-piperidine-1-sulfonyl]-furan-3-carboxylic acid methyl ester |
| 689 | 8-Methyl-1-[1-(2-oxo-2,3-dihydro-benzothiazole-6-sulfonyl)-piperidin-4-yl]- 1,4-dihydro-benzo[d][1,3]oxazin-2-one |
| 690 | 1-[1-(4-Fluoro-3-methyl-benzenesulfonyl)-piperidin-4-yl]-8-methyl-1,4-dihydro-benzo[d][1,3]oxazin-2-one |
| 691 | 8-Methyl-1-[1-(2-oxo-2,3-dihydro-benzooxazole-6-sulfonyl)-piperidin-4-yl]-1,4-dihydro-benzo[d][1,3]oxazin-2-one |
| 692 | 1-[1-(4-Cyclohexyl-benzenesulfonyl)-piperidin-4-yl]-8-methyl-1,4-dihydro-benzo[d][1,3]oxazin-2-one |
| 693 | 2,5-Dimethyl-4-[4-(2-oxo-4H-benzo[d][1,3]oxazin-1-yl)-piperidine-1-sulfonyl]-furan-3-carboxylic acid methyl ester |
| 694 | 1-[1-(4-Fluoro-3-methyl-benzenesulfonyl)-piperidin-4-yl]-1,4-dihydro-benzo[d][1,3]oxazin-2-one |
| 695 | 1-[1-(2-Oxo-2,3-dihydro-benzooxazole-6-sulfonyl)-piperidin-4-yl]-1,4-dihydro-benzo[d][1,3]oxazin-2-one |
| 696 | 1-[1-(4-Cyclohexyl-benzenesulfonyl)-piperidin-4-yl]-1,4-dihydro-benzo[d][1,3]oxazin-2-one |
| 697 | 2-Fluoro-5-[4-(8-methyl-2-oxo-4H-benzo[d][1,3]oxazin-1-yl)-piperidine-1-sulfonyl]-benzoic acid |
| 698 | 2-Fluoro-5-[4-(2-oxo-4H-benzo[d][1,3]oxazin-1-yl)-piperidine-1-sulfonyl]-benzoic acid |
| 699 | 1-[1-(2-Oxo-2,3-dihydro-benzothiazole-6-sulfonyl)-piperidin-4-yl]-1,4-dihydro-benzo[d][1,3]oxazin-2-one |
| 700 | 1-[1-(5-Pyridin-2-yl-thiophene-2-sulfonyl)-piperidin-4-yl]-1,4-dihydro -benzo[d][1,3]oxazin-2-one |
| 701 | 3-[4-(2-Oxo-4H-benzo[d][1,3]oxazin-1-yl)-pipendine-1-sulfonyl]-benzonitrile |
| 702 | 3-[4-(2-Oxo-4H-benzo[d][1,3]oxazin-1-yl)-piperidine-1-sulfonyl]-thiophene-2-carboxylic acid methyl ester |
| 703 | 1-{5-[4-(2-Oxo-4H-benzo[d][1,3]oxazin-1-yl)-piperidine-1-sulfonyl]-naphthalen-1-yl}-pyrrolidine-2,5-dione |
| 704 | 1-[1-(2-Chloro-5-trifluoromethyl-benzenesulfonyl)-piperidin-4-yl]-1,4-dihydro-benzo[d][1,3]oxazin-2-one |
| 705 | 1-[1-(3,4-Dimethyl-benzenesulfonyl)-piperidin-4-yl]-1,4-dihydro-benzo[d][1,3]oxazin-2-one |
| 706 | 8-Methy)-1-[1-(5-pyridin-2-yl-thiophene-2-sulfonyl)-piperidin-4-yl]-1,4-dihydro-benzo[d][1,3]oxazin-2-one |
| 707 | 3-[4-(8-Methyl-2-oxo-4H-benzo[d][1,3]oxazin-1-yl)-piperidine-1-sulfonyl]-benzonitrile |
| 708 | 3-[4-(8-Methyl-2-oxo-4H-benzo[d][1,3]oxazin-1-yl)-piperidine-1-sulfonyl]-thiophene-2-carboxylic acid methyl ester |
| 709 | 1-{5-[4-(8-Methyl-2-oxo-4H-benzo[d][1,3]oxazin-1-yl)-piperidine-1-sulfonyl]-naphthalen-1-yl}-pyrrolidine-2,5-dione |
| 710 | 1-[1-(2-Chloro-5-trifluoromethyl-benzenesulfonyl)-piperidin-4-yl]-8-methyl-1,4-dihydro-benzo[d][1,3]oxazin-2-one |
| 711 | 1-[1-(3,4-Dimethyl-benzenesulfonyl)-piperidin-4-yl]-8-methyl-1,4-dihydro-benzo[d][1,3]oxazin-2-one |
| 712 | 5-Chloro-1-[1-(5-pyridin-2-yl-thiophene-2-sulfonyl)-piperidin-4-yl]-1,4-dihydro-benzo[d][1,3]oxazin-2-one |
| 713 | 3-[4-(5-Chloro-2-oxo-4H-benzo[d][1,3]oxazin-1-yl)-piperidine-1-sulfonyl]-benzonitrile |
| 714 | 3-[4-(5-Chloro-2-oxo-4H-benzo[d][1,3]oxazin-1-yl)-piperidine-1-sulfonyl]-thiophene-2-carboxylic acid methyl ester |
| 715 | 1-{5-[4-(5-Chloro-2-oxo-4H-benzo[d][1,3]oxazin-1-yl)-piperidine-1-sulfonyl]- naphthalen-1-yl}-pyrrolidine-2,5-dione |
| 716 | 5-Chloro-1-[1-(2-chloro-5-trifluoromethyl-benzenesulfonyl)-piperidin-4-yl]-1,4-dihydro-benzo[d][1,3]oxazin-2-one |
| 717 | 5-Chloro-1-[1-(3,4-dimethyl-benzenesulfonyl)-piperidin-4-yl]-1,4-dihydro-benzo[d][1,3]oxazin-2-one |
| 718 | 6-Methyl-1-[1-(5-pyridin-2-yl-thiophene-2-sulfonyl)-piperidin-4-yl]-1,4-dihydro-benzo[d][1,3]oxazin-2-one |
| 719 | 3-[4-(6-Methyl-2-oxo-4H-benzo[d][1,3]oxazin-1-yl)-piperidine-1-sulfonyl]-benzonitrile |
| 720 | 3-[4-(6-Methyl-2-oxo-4H-benzo[d][1,3]oxazin-1-yl)-piperid ine-1-sulfonyl]-thiophene-2-carboxylic acid methyl ester |
| 721 | 1-{5-[4-(6-Methyl-2-oxo-4H-benzo[d][1,3]oxazin-1-yl)-piperidine-1-sulfonyl]-naphthalen-1-yl}-pyrrolidine-2,5-dione |
| 722 | 1-[1-(2-Chloro-5-trifluoromethyl-benzenesulfonyl)-piperidin-4-yl]-6-methyl-1,4-dihydro-benzo[d][1,3]oxazin-2-one |
| 723 | 1-[1-(3,4-Dimethyl-benzenesulfonyl)-piperidin-4-yl]-6-methyl-1,4-dihydro-benzo[d][1,3]oxazin-2-one |
| 724 | 6-Chloro-1-[1-(5-pyridin-2-yl-thiophene-2-sulfonyl)-piperidin-4-yl]-1,4-dihydro-benzo[d][1,3]oxazin-2-one |
| 725 | 3-[4-(6-Chloro-2-oxo-4H-benzo[d][1,3]oxazin-1-yl)-piperidine-1-sulfonyl]-benzonitrile |
| 726 | 3-[4-(6-Ch loro-2-oxo-4H-benzo[d][1,3]oxazin-1-yl)-piperidine-1-sulfonyl]-thiophene-2-carboxylic acid methyl ester |
| 727 | 1-{5-[4-(6-Chloro-2-oxo-4H-benzo[d][1,3]oxazin-1-yl)-piperidine-1-sulfonyl]-naphthalen-1-yl}-pyrrolidine-2,5-dione |
| 728 | 6-Chloro-1-[1-(2-chloro-5-trifluoromethyl-benzenesulfonyl)-piperidin-4-yl]-1,4-dihydro-benzo[d][1,3]oxazin-2-one |
| 729 | 6-Chloro-1-[1-(3,4-dimethyl-benzenesulfonyl)-piperidin-4-yl]-1,4-dihydro-benzo[d][1,3]oxazin-2-one |
| 730 | 1-[1-(5-Methyl-isoxazole-4-sulfonyl)-piperidin-4-yl]-1,4-dihydro-benzo[d][1,3]oxazin-2-one |
| 731 | 1-[1-(2,2-Dimethyl-chroman-6-sulfonyl)-piperidin-4-yl]-1,4-dihydro-benzo[d][1,3]oxazin-2-one |
| 732 | 1-[1-(4-Methyl-3,4-dihydro-2H-benzo[1,4]oxazine-7-sulfonyl)-piperidin-4-yl]-1,4-dihydro-benzo[d][1,3]oxazin-2-one |
| 733 | 1-[1-(2,3-Dihydro-benzo[1,4]dioxine-6-sulfonyl)-piperidin-4-yl]-1,4-dihydro-benzo[d][1,3]oxazin-2-one |
| 734 | 1-[1-(1,3,5-Trimethyl-1H-pyrazole-4-sulfonyl)-piperidin-4-yl]-1,4-dihydro-benzo[d][1,3]oxazin-2-one |
| 735 | 1-[1-(3-Methyl-2-oxo-2,3-dihydro-benzooxazole-6-sulfonyl)-piperidin-4-yl]-1,4-dihydro-benzo[d][1,3]oxazin-2-one |
| 736 | 8-Methyl-1-[1-(5-methyl-isoxazole-4-sulfonyl)-piperidin-4-yl]-1,4-dihydro-benzo[d][1,3]oxazin-2-one |
| 737 | 1-[1-(2,2-Dimethyl-chroman-6-sulfonyl)-piperidin-4-yl]-8-methyl-1,4-dihydro-benzo[d][1,3]oxazin-2-one |
| 738 | 8-Methyl-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 739 | 1-[1-(2,3-Dihydro-benzo[1,4]dioxine-6-sulfonyl)-piperidin-4-yl]-8-methyl-1,4-dihydro-benzo[d][1,3]oxazin-2-one |
| 740 | 8-Methyl-1-[1-(1,3,5-trimethyl-1H-pyrazole-4-sulfonyl)-piperidin-4-yl]-1,4-dihydro-benzo[d][1,3]oxazin-2-one |
| 741 | 8-Methyl-1-[1-(3-methyl-2-oxo-2,3-dihydro-benzooxazole-6-sulfonyl)-piperidin-4-yl]-1,4-dihydro-benzo[d][1,3]oxazin-2-one |
| 742 | 8-Methoxy-1-[1-(1,3,5-trimethyl-1H-pyrazole-4-sulfonyl)-piperidin-4-yl]-1,4-dihydro-benzo[d][1,3]oxazin-2-one |
| 743 | 8-Methoxy-1-[1-(3-methyl-2-oxo-2,3-dihydro-benzooxazole-6-sulfonyl)-piperidin-4-yl]-1,4-dihydro-benzo[d][1,3]oxazin-2-one |
| 744 | 1-[1-(Benzo[d]isoxazol-3-ylmethanesulfonyl)-piperidin-4-yl]-1,4-dihydro-benzo[d][1,3]oxazin-2-one |
| 745 | 1-[1-(2,2,4,6,7-Pentamethyl-2,3-dihydro-benzofuran-5-sulfonyl)-piperidin-4-yl]-1,4-dihydro-benzo[d][1,3]oxazin-2-one |
| 746 | 6-Methyl-5-[4-(2-oxo-4H-benzo[d][1,3]oxazin-1-yl)-piperidine-1-sulfonyl]-1 H-pyrimidine-2,4-dione |
| 747 | 1-[1-(3-Methyl-quinoline-8-sulfonyl)-piperidin-4-yl]-1,4-dihydro-benzo[d][1,3]oxazin-2-one |
| 748 | 1-[1-(2,2,5,7,8-Pentamethyl-chroman-6-sulfonyl)-piperidin-4-yl]-1,4-dihydro-benzo[d][1,3]oxazin-2-one |
| 749 | 1,4-Dimethyl-6-[4-(2-oxo-4H-benzo[d][1,3]oxazin-1-yl)-piperidine-1-sulfonyl]-1,4-dihydro-quinoxaline-2,3-dione |
| 750 | 1-[1-(1H-Imidazole-4-sulfonyl)-piperidin-4-yl]-1,4-d ihydro-benzo[d][1,3]oxazin-2-one |
| 751 | 1-[1-(2-Oxo-1,2,3,4-tetrahydro-quinoline-6-sulfonyl)-piperidin-4-yl]-1,4-dihydro-benzo[d][1,3]oxazin-2-one |
| 752 | 7-[4-(2-Oxo-4H-benzo[d][1,3]oxazin-1-yl)-piperidine-1-sulfonyl]-1,5-dihydro-benzo[b][1,4]diazepine-2,4-dione |
| 753 | 8-Methyl-1-[1-(3-methyl-quinoline-8-sulfonyl)-piperidin-4-yl]-1,4-dihydro-benzo[d][1,3]oxazin-2-one |
| 754 | 6-Chloro-1-[1-(3-methyl-quinoline-8-sulfonyl)-piperidin-4-yl]-1,4-dihydro-benzo[d][1,3]oxazin-2-one |
| 755 | 5-Chloro-1-[1-(3-methyl-quinoline-8-sulfonyl)-piperidin-4-yl]-1,4-dihydro-benzo[d][1,3]oxazin-2-one |
| 756 | 8-Methoxy-1-[1-(3-methyl-quinoline-8-sulfonyl)-piperidin-4-yl]-1,4-dihydro-benzo[d][1,3]oxazin-2-one |
| 757 | 1-[1-(Pyridine-2-sulfonyl)-piperidin-4-yl]-1,4-dihydro-benzo[d][1,3]oxazin-2-one |
| 758 | 1-[1-(6,7-Dihydroxy-naphthalene-1-sulfonyl)-piperidin-4-yl]-1,4-dihydro-benzo[d][1,3]oxazin-2-one |
| 759 | Acetic acid 3-acetoxy-5-[4-(2-oxo-4H-benzo[d][1,3]oxazin-1-yl)-piperidine-1-sulfonyl]-naphthalen-2-yl ester |
| 760 | 1-[1-(1H-Benzoimidazole-2-sulfonyl)-piperidin-4-yl]-1,4-dihydro-benzo[d][1,3]oxazin-2-one |
| 761 | 1-[1-(1H-Benzoimidazole-2-sulfonyl)-piperidin-4-yl]-8-methoxy-1,4-dihydro-benzo[d][1,3]oxazin-2-one |
| 762 | 1-[1-(1H-Benzoimidazole-2-sulfonyl)-piperidin-4-yl]-5-chloro-1,4-dihydro-benzo[d][1,3]oxazin-2-one |
| 763 | 1-[1-(2,5-Dimethoxy-benzenesulfonyl)-piperidin-4-yl]-6-fluoro-1,4-dihydro-benzo[d][1,3]oxazin-2-one |
| 764 | 1-[1-(2,5-Dimethoxy-benzenesulfonyl)-piperidin-4-yl]-8-methoxy-1,4-dihydro- benzo[d][1,3]oxazin-2-one |
| 765 | 1-[1-(2,5-Dimethoxy-benzenesulfonyl)-piperidin-4-yl]-6,7-difluoro-1,4-dihydro-benzo[d][1,3]oxazin-2-one |
| 766 | 5-Chloro-1-[1-(2,5-dimethoxy-benzenesulfonyl)-piperidin-4-yl]-1,4-dihydro-benzo[d][1,3]oxazin-2-one |
| 767 | 1-[1-(5-Dimethylamino-naphthalene-1-sulfonyl)-piperidin-4-yl]-8-methoxy-1,4-dihydro-benzo[d][1,3]oxazin-2-one |
| 768 | 5-Chloro-1-[1-(5-dimethylamino-naphthalene-1-sulfonyl)-piperidin-4-yl]-1,4-dihydro-benzo[d][1,3]oxazin-2-one |
| 769 | 6-Chloro-1-[1-(5-chloro-naphthalene-1-sulfonyl)-piperidin-4-yl]-1,4-dihydro-benzo[d][1,3]oxazin-2-one |
| 770 | 1-[1-(5-Chloro-naphthalene-1-sulfonyl)-piperidin-4-yl]-6-methyl-1,4-dihydro-benzo[d][1,3]oxazin-2-one |
| 771 | 1-[1-(5-Chloro-naphthalene-1-sulfonyl)-piperidin-4-yl]-6-fluoro-1,4-dihydro-benzo[d][1,3]oxazin-2-one |
| 772 | 6-Chloro-1-[1-(5-chloro-naphthalene-2-sulfonyl)-piperidin-4-yl]-1,4-d ihyd ro-benzo[d][1,3]oxazin-2-one |
| 773 | 1-[1-(5-Chloro-naphthalene-2-sulfonyl)-piperidin-4-yl]-6-methyl-1,4-dihydro-benzo[d][1,3]oxazin-2-one |
| 774 | 1-[1-(5-Chloro-naphthalene-2-sulfonyl)-piperidin-4-yl]-6-fluoro-1,4-dihydro-benzo[d][1,3]oxazin-2-one |
| 775 | 6-Methyl-1-[1-(3-methyl-quinoline-8-sulfonyl)-piperidin-4-yl]-1,4-dihydro-benzo[d][1,3]oxazin-2-one |
| 776 | 6-Fluoro-1-[1-(3-methyl-quinoline-8-sulfonyl)-piperidin-4-yl]-1,4-dihydro-benzo[d][1,3]oxazin-2-one |
| 777 | 6,7-Difluoro-1-[1-(3-methyl-quinoline-8-sulfonyl)-piperidin-4-yl]-1,4-dihydro-benzo[d][1,3]oxazin-2-one |
| 778 | 6-Chloro-1-[1-(3-methyl-2-oxo-2,3-dihydro-benzooxazole-6-sulfonyl)-piperidin-4-yl]-1,4-dihydro-benzo[d][1,3]oxazin-2-one |
| 779 | 6-Methyl-1-[1-(3-methyl-2-oxo-2,3-dihydro-benzooxazole-6-sulfonyl)-piperidin-4-yl]-1,4-dihydro-benzo[d][1,3]oxazin-2-one |
| 780 | 6-Fluoro-1-[1-(3-methyl-2-oxo-2,3-dihydro-benzooxazole-6-sulfonyl)-piperidin-4-yl]-1,4-dihydro-benzo[d][1,3]oxazin-2-one |
| 781 | 6,7-Difluoro-1-[1-(3-methyl-2-oxo-2,3-dihydro-benzooxazole-6-sulfonyl)-piperidin-4-yl]-1,4-dihydro-benzo[d][1,3]oxazin-2-one |
| 782 | 5-Chloro-1-[1-(3-methyl-2-oxo-2,3-dihydro-benzooxazole-6-sulfonyl)-piperidin-4-yl]-1,4-dihydro-benzo[d][1,3]oxazin-2-one |
| 783 | 6-Chloro-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 784 | 6-Methyl-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 785 | 6-Fluoro-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 786 | 8-Methoxy-1-[1-(4-methyl-3,4-dihydro-2H-benzo[1,4]oxazine-7-sulfonyl)-piperidin-4-yl]-1,4-dihydro-benzo[d][1,3]oxazin-2-one |
| 787 | 5-Chloro-1-[1-(4-methyl-3,4-dihydro-2H-benzo[1,4]oxazine-7-sulfonyl)-piperidin-4-yl]-1,4-dihydro-benzo[d][1,3]oxazin-2-one. |

optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

The benzoxazinone-derived sulphonamide compounds of general formula (Ib), wherein R^{1b}-R^{9b} and W^{b} have the meaning given above, may be prepared preferably by way of reaction of at least one piperidine compound of general formula (IIb) and/or a corresponding salt thereof, preferably a hydrochloride salt, 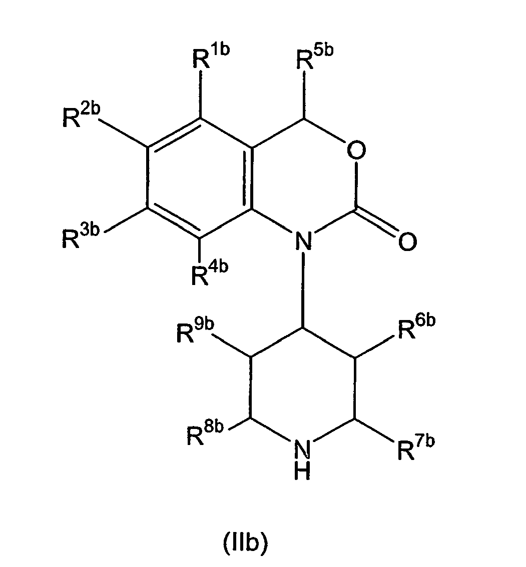 wherein R^{1b} to R^{9b} have the meaning given above, with at least one compound of general formula (IIIb), 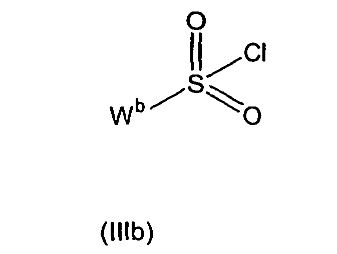 wherein W^{b} has the meaning given above, in a suitable reaction medium, optionally in the presence of at least one base and/or at least one auxiliary agent, to yield a compound of general formula (Ib).

Suitable reaction media include e.g. organic solvents, such as ethers, preferably diethyl ether, dioxane, tetrahydrofurane, dimethyl glycol ether, or alcohols, e.g. methanol, ethanol, propanol, isopropanol, butanol, isobutanol, tert-butanol, or hydrocarbons, preferably benzene, toluene, xylene, hexane, cyclohexane, petroleum ether, or halogenated hydrocarbons, e.g. dichloromethane, trichloromethane, tetrachloromethane, dichloroethylene, trichloroethylene, chlorobenzene or/and other solvents, preferably ethyl acetate, triethylamine, pyridine, dimethylsulfoxide, diemthylformamide, hexamethylphosphoramide, acetonitril, acetone or nitromethane, are included. Mixtures based one or more of the afore mentioned solvents may also be used.

Bases that may be used in the processes according to the present invention are generally organic or inorganic bases, preferably alkali metal hydroxides, e.g. sodium hydroxyde or potassium hydroxyde, or obtained from other metals such as barium hydroxyde or different carbonates, preferably potassium carbonate, sodium carbonate, calcium carbonate, or alkoxides, e.g. sodium methoxide, potassium methoxide, sodium ethoxide, potassium methoxide, potassium ethoxide or potassium tert-butoxide, or organic amines, preferably triethylamine, diisopropyethylamine or heterocycles, e.g. 1,4-diazabicyclo[2.2.2] octane, 1,8-diazabicyclo[5.4.0]undec-7-ene pyridine, diamino pyridine, dimethylaminopyridine, methylpiperidine or morpholine. Alkali metals such as sodium or ist hydrides, e.g. sodium hydride, may also be used. Mixtures based one or more of the afore mentioned bases may also be used.

During the synthetic reactions described above or while preparing the compounds of general formulas (IIb) or (IIIb) the protection of sensitive groups or of reagents may be necessary and/or desirable. This can be performed by using conventional protective groups like those described in the literature [Protective groups in Organic Chemistry, ed. J. F.W. McOmie, Plenum Press, 1973; T.W. Greene & P.G.M.Wuts, Protective Groups in Organic Chemistry, John Wiley & sons, 1991. The protective groups may also be eliminated as convenient by means well-known to those skilled in the art.

The compounds of general formulas (IIb) and (IIIb) are either commercially available or can be produced according to methods known to those skilled in the art. The reaction of compounds of general formulas (IIb) and (IIIb) to yield benzoxazinone-derived sulphonamide compounds of general formula (Ib) may also be facilitated by conventional methods known to those skilled in the art.

The substituted benzoxazinone compounds of general formula (IIb), wherein R^{5b} represents H, are preferably synthesized from substituted anthranilic acid or a corresponding ester via the corresponding substituted benzylalcohol (see scheme 1, method A). By reductive amination with 1-Boc-(tert.-Butylcarbonyloxy)-4-piperidone the Boc-piperidin-moiety is introduced into the substituted benzylalcohol. The benzoxazinone-ring is formed by cyclisation with triphosgene. The elimination of the Boc-protecting group is carried out by treatment in acidic media according to the method described in Williams et al., J. Med. Chem. 1995 38, 4634 and later by Bell et al., J. Med. Chem., 1998, 41, 2146 which are hereby incorporated by reference and form part of the disclosure. By reacting such a substituted benzoxazinone compound of general formula (IIb) with a substituted sulfuryl chloride of general formula (IIIb) compounds of general formula (Ib) are obtained.

By reduction of the corresponding ketones via conventional methods known to those skilled in the art, e.g. by reduction with sodium borohydride (see scheme 1, method B, R^{5b}=Z) benzoxazinone derived sulphonamide compounds of general formula (Ib), wherein R^{5b} represents an unbranched or branched, saturated or unsaturated, optionally at least mono-substituted aliphatic radical or a saturated or unsaturated, optionally at least mono-substituted, optionally at least one heteroatom as ring member containing cycloaliphatic radical (denoted by Z in method B) can be obtained.

The respective reagents used in said process for the preparation of benzoxazinone derived sulphonamide compounds of general formula (Ib) are either commercially available or can be obtained by methods well known to those skilled in the art. In the following scheme the respective substituents represent the afore mentioned substituents having an index b.

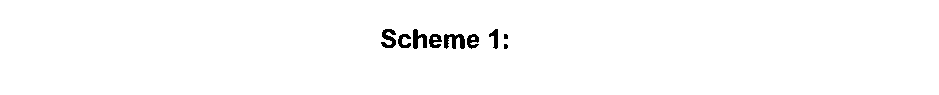

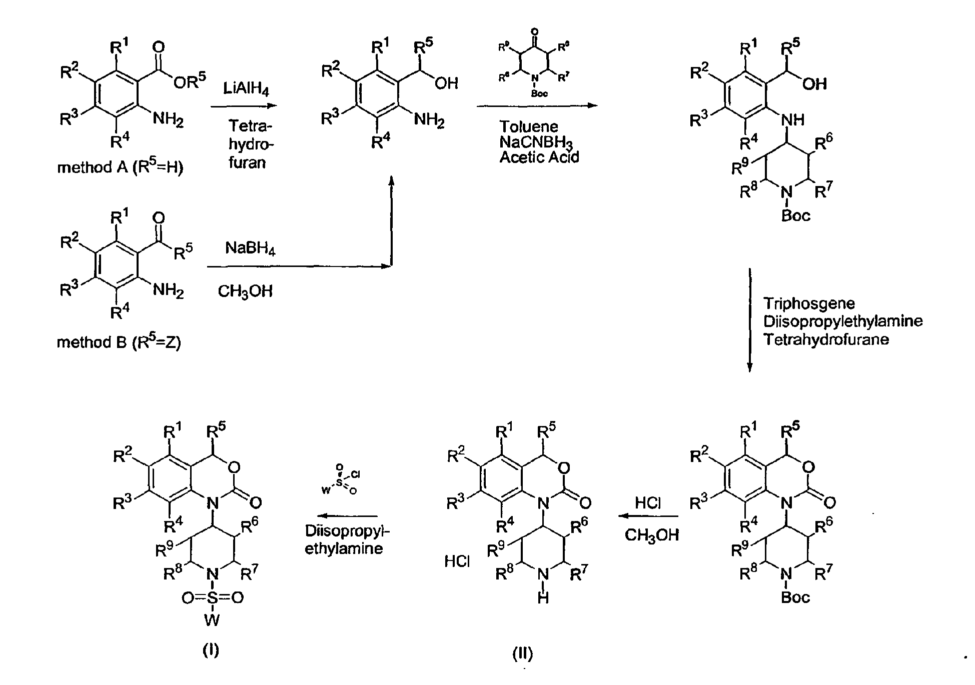

The salts of benzoxazinone-derived sulphonamide compounds of general formula (Ib), may be prepared in a way that at least one compound of general formula (Ib) having at least one basic group is reacted with at least one inorganic and/or organic acid, preferably in the presence of a suitable reaction medium. Suitable reaction media are, for example, the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

The salts of benzoxazinone-derived sulphonamide compounds of general formula (Ib), may be prepared in a way that at least one compound of general formula (Ib) having at least one acidic group is reacted with one or more suitable bases, preferably in the presence of a suitable reaction medium.

Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0,1, 2, 3 or 4 and R represents a branched or unbranched C₁₋₄-alkyl-radical. Suitable reaction media are, for example, the ones given above.

Solvates, preferably hydrates, of the Benzoxazinone-derived sulphonamide compounds of general formula (Ib) or of the salts thereof may also be obtained by standard procedures known to those skilled in the art.

If the Benzoxazinone-derived compounds of general formula (Ib) are obtained in form of a mixture of stereoisomers, particularly enantiomers or diastereomers, said mixtures may be separated by standard procedures known to those skilled in the art, e.g. chromatographic methods or crystallization with chiral reagents.

The purification and isolation of the Benzoxazinone-derived sulphonamide compounds of general formula (Ib) or a corresponding stereoisomer, or salt, or solvate respectively, if required, may be carried out by conventional methods known to those skilled in the art, e.g. chromatographic methods or recrystallization.

If one or more of the residues R^{1c}, R^{3c}, R^{4c} and R^{5c} represents an alkyl radical, which is substituted with one or more substituents, unless defined otherwise, each of the substituents may preferably be selected from the group consisting of hydroxy, fluorine, chlorine, bromine and trifluoromethyl.

If R^{1c} represents a phenyl radical or a benzyl radical, which is substituted with one or more substituents, unless defined otherwise, each of the substituents may preferably be selected from the group consisting of hydroxy, fluorine, chlorine, bromine, branched or unbranched C₁-C₄-alkyl, branched or unbranched C₁-C₄-alkoxy, branched or unbranched C₁-C₄-perfluoroalkyl and branched or unbranched C₁-C₄-perfluoroalkoxy.

If R^{2c} represents a saturated or unsaturated, optionally at least one heteroatom as ring member containing cycloaliphatic radical, which is substituted with one or more substituents and/or if it comprises a saturated or unsaturated, optionally at least one heteroatom as ring member containing mono- or bicyclic cycloaliphatic ringsystem, which is substituted with one or more substituents, unless defined otherwise, each of the substituents may preferably be selected from the group consisting of hydroxy, fluorine, chlorine, bromine, branched or unbranched C₁-C₄-alkyl, branched or unbranched C₁-C₄-alkoxy, branched or unbranched C₁-C₄-perfluoroalkyl, branched or unbranched C₁-C₄-perfluoroalkoxy and benzyl, preferably from the group consisting of branched or unbranched C₁-C₄-alkyl and benzyl. The heteroatoms of the cycloaliphatic radical and/or of the mono- or bicyclic cycloaliphatic ringsystem may, independent from one another, preferably be selected from the group consisting of nitrogen, sulphur and oxygen, more preferably the heteroatom is nitrogen.

If R^{4c} and R^{5c} together with the bridging nitrogen atom form a saturated or unsaturated, optionally at least one further heteroatom as ring member containing heterocyclic ring, which is substituted with one or more substituents and/or which is condensed with a saturated or unsaturated, optionally at least one heteroatom as ring member containing mono- or bicyclic cycloaliphatic ringsystem, which is substituted with one or more substituents, unless otherwise defined, each of the substituents, may preferably be selected from the group consisting of hydroxy, fluorine, chlorine, bromine, branched or unbranched C₁-C₄-alkyl, branched or unbranched C₁-C₄-alkoxy, branched or unbranched C₁-C₄-perfluoroalkyl, branched or unbranched C₁-C₄-perfluoroalkoxy and benzyl, preferably from the group consisting of branched or unbranched C₁-C₄-alkyl and benzyl. If the hetereocyclic ring contains one or more further heteroatoms and/or one or both of the mono- or bicyclic rings contain one or more heteroatoms, these heteroatoms may, independent from one another, preferably be selected from the group consisting of nitrogen, sulphur and oxygen, more preferably the heteroatom is nitrogen.

If A^{c} represents a mono- or polycyclic aromatic ringsystem, which is substituted with one or more substituents, and which may be bonded via an optionally at least mono-substituted alkylene-, alkenylene- or alkynylene group and/or may contain at least one heteroatom as a ring member, unless otherwise defined, each of the substituents, may preferably be selected from the group consisting of hydroxy, halogen, branched or unbranched C₁-C₄-alkyl, branched or unbranched C₁-C₄-alkoxy, branched or unbranched C₁-C₄-perfluoroalkyl, branched or unbranched C₁-C₄-perfluoroalkoxy, an optionally at least mono-substituted phenyl radical and 5-or 6 membered heteroaryl, preferably from the group consisting of halogen, branched or unbranched C₁-C₄-alkyl, an optionally at least mono-substituted phenyl radical and 5- or 6-membered heteroaryl, more preferably from the group consisting of fluorine, chlorine, branched or unbranched C₁-C₄-alkyl, an optionally at least mono-substituted phenyl radical, and 5- or 6-membered heteroaryl. If one or more of the rings of the mono- or polycyclic aromatic ringsystem contains one or more heteroatoms, these heteroatoms - like the heteroatoms of the afore mentioned 5-or 6 membered heteroaryl radical - may preferably be selected from the group consisting of oxygen, sulphur and nitrogen. If the afore mentioned phenyl radical is itself substituted with one or more substituents, each of the substituents may preferably be selected from the group consisting of fluorine, chlorine, bromine, linear or branched C₁-C₄-alkyl, linear or branched C₁-C₄-alkoxy, linear or branched C₁-C₄-alkylthio, a trifluoromethyl moiety, a cyano moiety and a NR^{8c}R^{9c}-moiety, wherein R^{ac} and R^{9c}, identical or different, represent hydrogen or linear or branched C₁-C₄-alkyl.

If the afore mentioned alkylene-, alkenylene- or alkynylene group is substituted with one or more substituents, each of the substituents may preferably be selected from the group consisting of hydroxy, halogen, branched or unbranched C₁-C₄-alkyl, branched or unbranched C₁-C₄-alkoxy, branched or unbranched C₁-C₄-perfluoroalkyl, branched or unbranched C₁-C₄-perfluoroalkoxy or an optionally at least mono-substituted phenyl radical. If said phenyl radical is itself substituted by one or more substituents, each of the substituents may preferably be selected from the group consisting of fluorine, chlorine, bromine, linear or branched C₁-C₄-alkyl, linear or branched C₁-C₄-alkoxy, linear or branched C₁-C₄-alkylthio, a trifluoromethyl moiety, a cyano moiety and a NR^{8c}R^{9c}-moiety, wherein R^{8c} and R^{9c}, identical or different, represent hydrogen or linear or branched C₁-C₄-alkyl.

Preferably used are sulphonamide derivatives of general formula (Ic), wherein R^{1c} represents hydrogen, an optionally at least mono-substituted, linear or branched C₁₋₄-alkyl radical, an optionally at least mono-substituted phenyl radical or an optionally at least mono-substituted benzyl radical, preferably hydrogen, a linear or branched C₁₋₄-alkyl radical or a benzyl radical, more preferably hydrogen, and R^{2c} to R^{5c}, A^{c} and nc are as defined above.

Preference is also given to the use of sulphonamide derivatives of general formula (Ic), wherein R^{2c} represents a -NR^{4c}R^{5c} moiety or a saturated or unsaturated, optionally at least mono-substituted, optionally at least one heteroatom as ring member containing 5- or 6-membered cycloaliphatic radical, which may be condensed with a saturated or unsaturated, optionally at least mono-substituted, optionally at least one heteroatom as ring member containing mono- or bicyclic cycloaliphatic ringsystem, wherein the ring(s) is/are 5- or 6-membered, preferably a -NR^{4c}R^{5c} moiety or a moiety selected from the group consisting of 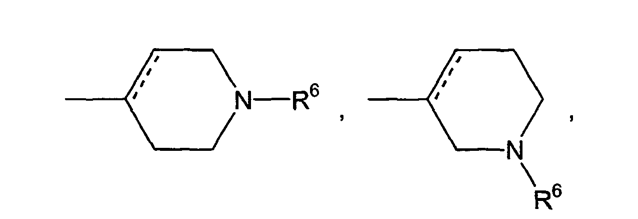 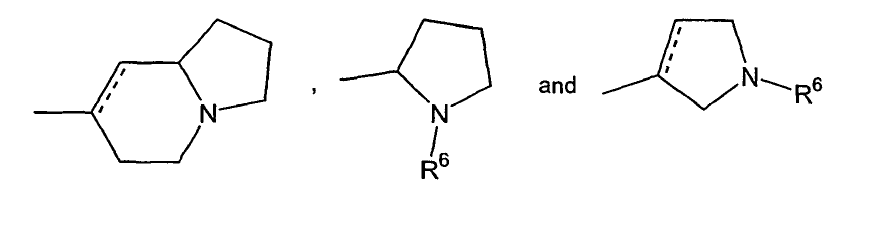 wherein, if present, the dotted line represents an optional chemical bond and R⁶ represents hydrogen, a linear or branched C₁-C₄-alkyl radical or a benzyl radical, preferably hydrogen or a C₁-C₂ alkyl radical, and R^{1c}, R^{3c}-R^{5c}, A^{c} and nc are as defined above.

Also preferred is the use of sulphonamide derivatives of general formula (Ic),
wherein R^{3c} represents hydrogen or an optionally at least mono-substituted, linear or branched C₁-C₄-alkyl radical, preferably hydrogen or a linear or branched C₁-C₄-alkyl radical, more preferably hydrogen or a C₁-C₂ alkyl radical, and R^{1c}, R^{2c} R^{4c}, R^{5c}, A^{c} and nc are as defined above.

Furthermore, preference is also given to the use of sulphonamide derivatives of general formula (Ic), wherein R^{4c} and R^{5c}, identical or different, represent hydrogen or an optionally at least mono-substituted, linear or branched C₁-C₄-alkyl radical, or

R^{4c} and R^{5c} together with the bridging nitrogen atom form an optionally at least mono-substituted, saturated or unsaturated, 5- or 6-membered heterocyclic ring, which may contain at least one further heteroatom as a ring member and/or may be condensed with a saturated or unsaturated, optionally at least mono-substituted, optionally at least one heteroatom as ring member containing mono- or bicyclic aliphatic ringsystem, wherein the ring(s) is/are 5-, 6- or 7-membered, and R^{1c}, R^{2c}; R^{3c}, A^{c} and nc are as defined above.

Particularly preferred is the use of sulphonamide derivatives of general formula (Ic), wherein R^{4c} and R^{5c}, identical or different, represent hydrogen or a linear or branched C₁-C₄-alkyl radical, preferably a linear or branched C₁-C₄-alkyl radical, or
R^{4c} and R^{5c} together with the bridging nitrogen atom form a moiety selected from the group consisting of 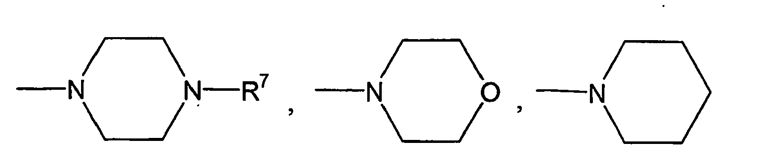 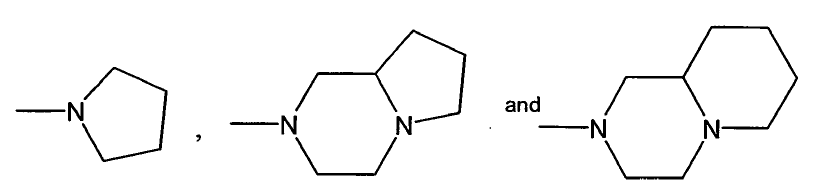 wherein R⁷ represents hydrogen, a linear or branched C₁-C₄-alkyl radical or a benzyl radical, preferably hydrogen or a C₁-C₂ alkyl radical, and R^{1c}-R^{3c}, A^{c} and nc are as defined above.

Moreover, the use of sulphonamide derivatives of general formula (Ic) is preferred, wherein A^{c} represents an optionally at least mono-substituted mono-or bicyclic aromatic ringsystem, wherein the ring(s) is/are 5- or 6-membered, which may be bonded via a an optionally at least mono-substituted C₁-C₄-alkylene group, an optionally at least mono-substituted C₂-C₄-alkenylene or an optionally at least mono-substituted C₂-C₄-alkinylene group and/or may contain at least one heteroatom as a ring member, preferably an optionally at least mono-substituted mono- or bicyclic aromatic ringsystem, wherein the ring(s) is/are 5- or 6-membered and wherein one or both of the rings contain(s) at least one heteroatom, or a moiety selected from the group consisting of 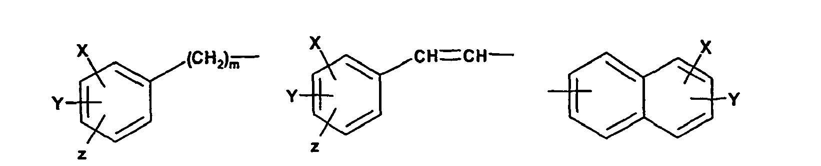 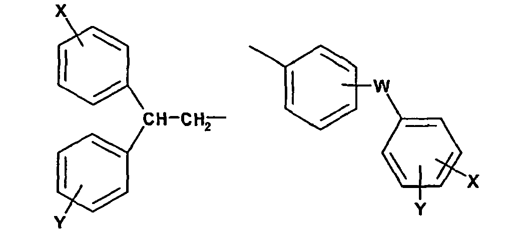 wherein X, Y, Z are each independently selected from the group consisting of hydrogen, fluorine, chlorine, bromine, linear or branched C₁-C₄-alkyl, linear or branched C₁-C₄-alkoxy, linear or branched C₁-C₄-alkylthio, a trifluoromethyl moiety, a cyano moiety and a NR⁸R⁹-moiety, wherein R⁸ and R⁹, identical or different, represent hydrogen or linear or branched C₁-C₄-alkyl,
W represents a single chemical bond between the two rings, a CH₂-group, O, S or a NR¹⁰-moiety, wherein R¹⁰ is hydrogen or linear or branched C₁-C₄-alkyl and
m is 0, 1, 2, 3 or 4.
and R^{1c}-R^{5c} and n are as defined above.

Most preferred is the use of one or more sulphonamide derivatives selected from the group consisting of:
[1] N-[3-(2-diethylaminoethyl)-1H-indol-5-yl]-5-chloro-3-methylbenzo[b]thiophene-2-sulphonamide,
[2] N-[3-(2-diethylaminoethyl)-1H-indol-5-yl]naphthalene-1-sulphonamide,
[3] Hydrochloride N-[3-(2-diethylaminoethyl)-1H-indol-5-yl]naphthalene-1-sulphonamide,
[4] N-[3-(2-diethylaminoethyl)-1*H*-indol-5-yl]-3,5-dichlorobenzenesulphonamide,
[5] N-[3-(2-diethylaminoethyl)-1*H*-indol-5-yl]-4-phenylbenzenesulphonamide,
[6] N-[3-(2-diethylaminoethyly)-1*H*-indol-5-yl]-5-chlorothiophene-2-sulphonamide,
[7] N-[3-(2-dimethylaminoethyl)-1*H*-indol-5-yl]-5-chloro-3-methylbenzo[b]thiophene-2-sulphonamide,
[8] N-[3-(2-dimethylaminoethyl)-1*H*-indol-5-yl]naphthalene-1-sulphonamide,
[9] N-[3-(2-dimethylamino-ethyl)-1*H*-indol-5-yl]-6-chloroimidazo[2,1-b]thiazol-5-sulphonamide,
[10] N-[3-(1-methylpiperidin-4-yl)-1*H*-indol-5-yl]-5-chloro-3-methylbenzo[b]thiophene-2-sulphonamide,
[11] N-[3-(1-methylpiperidin-4-yl)-1*H*-indol-5-yl]-5-chloro-3-methylbenzo[b]thiophene-2-sulphonamide hydrochloride,
[12] N-[3-(1-methylpiperidin-4-yl)-1*H*-indol-5-yl]naphthalene-1-sulphonamide, .
[13] N-[3-(1-methylpiperidin-4-yl)-1*H*-indol-5-yl]naphthalene-1-sulphonamide hydrochloride,
[14] N-[3-(1 -methylpiperidin-4-yl)-1*H*-indol-5-yl]-5-chlorothiophene-2-sulphonamide,
[15] N-[3-(1-methylpiperidin-4-yl)-1*H*-indol-5-yl]-4-phenylbenzenesulphonamide,
[16] N-[3-(1-methylpiperidin-4-yl)-1*H*-indol-5-yl]quinoline-8-sulphonamide,
[17] N-[3-(2-diethylaminoethyl)-1*H*-indol-5-yl]naphthalene-2-sulphonamide,
[18] N-[3-(1-methyl-1,2,3,6-tetrahydropyridin-4-yl)-1*H*-indol-5-yl]naphthalene-1-sulphonamide,
[19] N-[3-(4-methylpiperazin-1-yl)methyl-1*H*-indol-5-yl]-5-chloro-3-methylbenzo[b]thiophene-2-sulphonamide,
[20] N-[3-(2-dimethylaminoethyl)-1*H*-indol-5-yl]-5-(2-pyridil)thiophene-2-sulphonamide,
[21] N-[3-(2-dimethylaminoethyl)-1*H*-indol-5-yl]-2,1,3- benzothiadiazol-4-sulphonamide,
[22] N-[3-(2-dimethylaminoethyl)-1*H*-indol-5-yl]quinoline-8-sulphonamide,
[23] N-[3-(2-dimethylaminoethyl)-1*H*-indol-5-yl]-5-chloronaphthalene-2-sulphonamide,
[24] N-[3-(2-dimethylaminoethyl)-1*H*-indol-5-yl]-4-phenoxybenzenesulphonamide,
[25] N-[3-(2-dimethylaminoethyl)-1*H*-indol-5-yl]-4-phenylbenzenesulphonamide,
[26] N-[3-(2-diethylaminoethyl)-1*H*-indol-5-yl]-N-ethyl-naphthalene-2-sulphonamide,
[27] N-{3-[2-(morpholin-4-yl)ethyl)-1H-indol-5-yl)-5-chloro-3-methylbenzo[b]thiophene-2-sulphonamide,
[28] N-{3-[2-(morpholin-4-yl)ethyl]-1H-indol-5-yl}naphthalene-1-sulphonamide,
[29] N-[3-(2-diethylaminoethyl)-1H-indol-5-yl]naphthalene-2-sulphonamide,
[30] N-[3-dimethylaminomethyl-1 H-indol-5-yl]-5-chloro-3-methylbenzo[b]thiophene-2-sulphonamide,
[31] N-[3-(2-dipropylaminoethyl)-1H-indol-5-yl]naphthalene-1-sulphonamide,
[32] N-[3-(2-dipropylaminoethyl)-1H-indol-5-yl]-5-chloro-3-methylbenzo[b]thiophene-2-sulphonamide,
[33] N-[3-(2-dibutylaminoethyl)-1H-indol-5-yl]-5-chloro-3-methylbenzo[b]thiophene-2-sulphonamide,
[34] N-[3-(2-dibutylaminoethyl)-1H-indol-5-yl]naphthalene-1-sulphonamide,
[35] N-[3-(2-diethylaminoethyl)-1H-indol-5-yl]-5-chloronaphthalene-1-sulphonamide,
[36] N-[3-(2-diethylaminoethyl)-1H-indol-5-yl]-trans-β-styrenesulphonamide,
[37] N-[3-(4-methylpiperazin-1-yl)methyl-1H-indol-5-yl]-trans-p-styrenesulphonamide,
[38] N-[3-(octahydroindolizin-7-yl)-1H-indol-5-yl]-5-chloro-3-methylbenzo[b]thiophene-2-sulphonamide,
[39] N-[3-(2-diethylaminoethyl)-1*H*-indol-5-yl]-6-chloroimidazo[2,1-b]thiazol-5-sulphonamide,
[40] N-{3-[2-(morpholin-4-yl)ethyl]-1H-indol-5-yl}naphthalene-2-sulphonamide,
[41] N-[3-(4-methylpiperazin-1-yl)methyl-1*H*-indol-5-yl]-α-toluenesulphonamide,
[42] N-[3-(3-diethylaminopropyl)-1H-indol-5-yl]naphthalene-2-sulphonamide,
[43] N-[3-(3-diethylaminopropyl)-1H-indol-5-yl]-5-chloro-3-methylbenzo[b]thiophene-2-sulphonamide,
[44] N-{3-[2-(pyrrolidin-1-yl)ethyl]-1H-indol-5-yl}-5-chloro-3-methylbenzo[b]thiophene-2-sulphonamide,
[45] N-{3-[2-(pyrroridin-1-yl)ethyl]-1H-indol-5-yl)naphthalene-1-sulphonamide,
[46] N-{3-[2-(pyrrolidin-1-yl)ethyl]-1H-indol-5-yl}naphthalene-2-sulphonamide,
[47] N-[3-(2-dipropylaminoethyl)-1H-indol-5-yl]naphthalene-2-sulphonamide,
[48] N-[3-(2-dimethylaminoethyl)-1H-indol-5-yl]-5-chloronaphthalene-1-sulphonamide,
[49] N-[3-(2-dimethylaminoethyl)-1H-indol-5-yl]naphthalene-2-sulphonamide,
[50] N-{3-[2-(morpholin-4-yl)ethyl]-1H-indol-5-yl}quinoline-8-sulphonamide,
[51] N-{3-[2-(morpholin-4-yl)ethyl]-1H-indol-5-yl}-4-phenylbenzenesulphonamide,
[52] N-[3-(4-methylpiperazin-1-yl)ethyl-1H-indol-5-yl]naphthalene-2-sulphonamide and
[53] N-[3-(4-methylpiperazin-1-yl)ethyl-1H-indol-5-yl]-5-chloronaphthalene-1-sulphonamide.

The sulphonamide derivatives of general formula (Ic), wherein R^{1c}, R^{2c} , R^{3c}, nc and A^{c} have the above defined meaning, may preferably be prepared according to the following methods, wherein R¹, R², R³, n and A are R^{1c}, R^{2c}, R^{3c}, nc and A^{c}.

### METHOD A:

At least one compound of general formula (IIc), 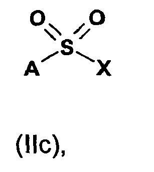 wherein A has the meaning as defined above in the general formula (Ic) and X is a suitable leaving group, preferably a halogen atom, more preferably chlorine; is reacted with at least one substituted 5-aminoindol of general formula (IIIc) 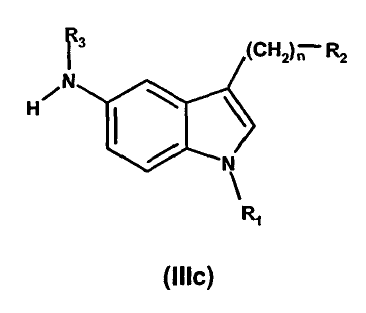 wherein R₁, R₂, R₃ and n have the meaning as defined above, or a suitably protected derivative thereof, and, if present, the protective groups are removed, in order to obtain the corresponding sulphonamide derivative of general formula (Ic), which may be purified and/or may be isolated by conventional methods known to those skilled in the art.

The reaction between the compounds of general formulas (IIc) and (IIIc) is usually carried out in the presence of an organic reaction medium, such as an dialkyl ether, particularly diethyl ether, or a cyclic ether, particularly tetrahydrofurane or dioxane, a halogenated organic hydrocarbon, particularly methylene chloride or chloroform, an alcohol, particularly methanol or ethanol, an aprotic dipolar solvent, particularly acetonitrile, pyridine or dimethylformamide, or any other suitable reaction medium. Mixtures of at least two of the above mentioned classes of compounds or of at least two compounds of one class may, of course, also be used.

The reaction is preferably carried out in the presence of a suitable base, e.g. an inorganic base such as hydroxides and/or carbonates of alkali metals, or an organic base, particularly triethylamine or pyridine.

The most suitable reaction temperatures range from 0° C to ambient temperature, i.e. approximately 25°C, and the reaction time is preferably from 5 minutes to 24 hours.

The resulting sulphonamide derivative of general formula (Ic) may be purified and/or isolated according to conventional methods known to those skilled in the art.

Preferably the sulphonamide derivatives of general formula (Ic) can be isolated by evaporating the reaction medium, adding water and eventually adjusting the pH so that it is obtained as a solid that can be isolated by filtration; or it can be extracted by a solvent immiscible with water, such as chloroform, and purified by chromatography or recrystallisation from a suitable solvent.

The compounds of general formula (IIc) are commercially available or can be prepared according to standard methods known to those skilled in the art, e.g. by methods analogous to those described in the literature [E.E. Gilbert, Synthesis, 1969, 1, 3]. The compounds of general formula (IIIc) may also be prepared according to standard methods known to those skilled in the art, e.g. by methods analogous to those described in the literature [J.E. Macor, R. Post and K. Ryan, Synt Comm., 1993, 23, 1, 65-72.; J. Guillaume, C. Dumont, J. Laurent and N. Nédélec, Eur. J. Med. Chem., 1987, 22, 33-43; M.L. Saccarello, R. Stradi, Synthesis, 1979, 727].

### METHOD B

The sulphonamide derivatives of general formula (Ic), wherein R¹, R², n and A are as defined above and R³ represents an optionally at least mono-substituted, linear or branched C₁-C₄ alkyl radical, may also be prepared by alkylation of a corresponding sulphonamide derivative of general formula (Ic), wherein R¹, R², n and A are as defined above and R³ represents a hydrogen atom, with an alkyl halogenide or a dialkyl sulphate.

The alkylation reaction is preferably carried out in the presence of a suitable base, such as hydroxides and/or carbonates of alkali metals, metal hydrides, alkoxides such as sodium methoxide or potassium tert-butoxide, organometallic compounds such as butyl lithium or tert.-butyl lithium, in the presence of an organic reaction medium, such as dialkyl ether, particularly diethyl ether, or a cyclic ether, particularly tetrahydrofurane or dioxane, a hydrocarbon, particularly toluene, an alcohol, particularly methanol or ethanol, an aprotic dipolar solvent, particularly acetonitrile, pyridine or dimethylformamide, or any other suitable reaction medium. Mixtures of at least two of the above mentioned classes of compounds and/or of at least two compounds of one class may, of course, also be used.

The most suitable reaction temperatures range from 0° C to the boiling point of the reaction medium, and reaction times preferably range from 1 to 24 hours.

The resulting sulphonamide derivative of general formula (Ic) can preferably be isolated by filtration, concentrating the filtrate at reduced pressure, adding water and eventually adjusting the pH so that it is obtained as a solid that can be isolated by filtration, or it can be extracted with a solvent immiscible in water such as chloroform and purified by chromatography or recrystallisation from a suitable solvent.

### METHOD C

By condensation of a compound of general formula (Ic), wherein R₁, R₃, and A are as defined above, n is 0 and R₂ represents a hydrogen atom, with a suitably substituted 4-piperidone the corresponding compound of general formula (Ic) is obtained, wherein R₁, R₃ and A are as defined above, n is 0 and R₂ represents a suitably substituted 1,2,3,6-tetrahydropyridine-4-yl radical.

The reaction can take place in both an acid and a basic reaction medium, preferably in a suitable solvent, preferably at temperatures ranging from 25 to 150°C.

Suitable basic conditions may be provided by the use of inorganic bases such as sodium or potassium hydroxide, or organic bases such as pyrrolidine or triethylamine in solvents such as methanol or ethanol. Preferably, solutions of sodium methoxide in methanol under reflux are used.
Reaction times range from 1 to 48 hours.

Suitable acidic conditions may be provided by the use of hydrochloric acid in ethanol or trifluoroacetic acid in acetic acid at temperatures ranging preferably from 50 to 100 °C and reaction times ranging from 1 to 48 hours.

The resulting sulphonamide derivative of general formula (Ic) can be isolated by dilution in water, eventually adjusting the pH, to obtain a solid that can be isolated by filtration; or it can be extracted with a solvent immiscible in water such as chloroform and purified by chromatography or by recrystallisation from a suitable solvent.

The compounds of general formula (Ic) wherein R₁, R₃ and A are as defined above, n is 0 and R₂ represents a hydrogen atom, can be prepared according to the method A from a corresponding 5-aminoindol.

### METHOD D

The compound of general formula (Ic) wherein R₁, R₃ and A are as defined above, n is 0 and R₂ represents a suitably substituted 4-piperidinyl radical, can be prepared by reducing a compound of general formula (Ic) wherein R₁, R₃ and A are as defined above, n is 0 and R₂ represents a suitably substituted 1,2,3,6-tetrahydropyridin-4-yl radical prepared according to the method C.

Hydrogenation preferably takes place with the aid of a metallic catalyst such as palladium, platinum or rhodium on a suitable support such as carbon, aluminum oxide or barium sulphate, preferably palladium on carbon, with an initial hydrogen pressure of between 1 and 10 atmospheres, preferably between 2 and 5 atmospheres, in a solvent such as methanol or ethanol. The reaction time ranges from 1 hour to 3 days.

The resulting sulphonamide can be isolated by filtering the catalyst and concentrating the filtrate at reduced pressure. The product recovered can be used as is or it can be purified by chromatography or by recrystallisation from a suitable solvent.

### METHOD E

The salts, preferably the pharmacologically acceptable salts of compounds with the general formula (Ic) can be prepared by conventional methods known to those skilled in the art, preferably by reaction with a mineral acid, such as hydrochloric, hydrobromic, phosphoric, sulphuric, nitric acids or with organic acids such as citric, maleic, fumaric, tartaric acids or their derivatives, p-toluensulphonic acid, methansulphonic acid, etc., in a suitable solvent such as methanol, ethanol, diethyl ether, ethyl acetate, acetonitrile or acetone and obtained with the usual techniques of precipitation or crystallisation of the corresponding salts.

Preferred physiologically acceptable salts of the sulphonamide derivatives of general formula (Ic) are the additions salts of mineral acids, such as hydrochloric acid, hydrobromic acid, phosphoric acid, sulphuric acid, nitric acid, and of organic acids, such as citric acid, maleic acid, tartaric acid or derivatives thereof, p-toluenesulphonic acid, methansulphonic acid, camphorsulphonic acid, etc.

The solvates, preferably the physiologically acceptable solvates, particularly hydrates, of the sulphonamide derivatives of general formula (Ic) or of the corresponding physiologically acceptable salts may be prepared by conventional methods known to those skilled in the art.

During one of the synthesis sequences described above, or in the preparation of suitable reactands used it may be necessary and/or desirable to protect sensitive or reactive groups in some of the molecules employed. This can be performed by means of conventional protective groups such as those described in the literature [Protective groups in Organic Chemistry, ed J. F.W. McOmie. Plenum Press, 1973; T.W. Greene & P.G.M. Wuts, Protective Groups in Organic Chemistry, John Wiley & sons, 1991]. The protective groups can be eliminated in a suitable latter stage by methods known to those skilled in the art.

If the sulphonamide derivatives of general formula (Ic) are obtained in form of a mixture of stereoisomers, particularly enantiomers or diastereomers, said mixtures may be separated by standard procedures known to those skilled in the art, e.g. chromatographic methods or crystallization with chiral reagents.

If one or more of the moieties R^{2d} -R^{9d} represent a saturated or unsaturated aliphatic radical, that is, an alkyl, alkenyl or alkynyl radical which is substituted by one or more substituents, each one of these substituents may preferably be chosen, unless otherwise defined, from the group consisting of hydroxy, fluorine, chlorine, bromine and trifluoromethyl.

If R^{1d} is a saturated or unsaturated, optionally at least one heteroatom as a ring member containing cycloaliphatic radical, which is substituted by one or more substituents and/or is condensed with a saturated or unsaturated, optionally at least one heteroatom as a ring member containing mono- or bicyclic cycloaliphatic ring system, which is substituted by one or more substituents, each one of these substituents may preferably be chosen, unless otherwise defined, from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl.

The heteroatoms of said cycloaliphatic radical and/or of said mono- or bicyclic cycloaliphatic ring may, independently from one another, preferably be chosen from the group consisting of nitrogen, sulphur and oxygen, more preferably nitrogen is chosen as a heteroatom.

Said cycloaliphatic radical may contain 0, 1, 2 or 3 heteroatoms chosen from the above mentioned group, preferably it contains 0, 1 or 2 heteroatoms chosen from the above mentioned group.

If R^{8d} and R^{9d} together with the bridging nitrogen atom form a saturated or unsaturated, optionally at least mono-substituted heterocyclic ring, which may contain at least one further heteroatom as a ring member and/or which is condensed with a saturated or unsaturated mono- or bicyclic cycloaliphatic ring system, which may contain at least one heteroatom as a ring member and/or which is substituted by one or more substituents, each one of these substituents may preferably be chosen, unless otherwise defined, from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl.

If the heterocyclic ring contains one or more additional heteroatoms, and/or if one or both rings of the mono- or bicyclic ring system contain one or more heteroatoms, these heteroatoms may, independently from one another, preferably be chosen from the group consisting of nitrogen, sulphur and oxygen, more preferably nitrogen is chosen as a heteroatom.

Said heterocyclic ring may contain 0, 1, 2 or 3 additional heteroatoms chosen from the above mentioned group, preferably it contains 0 or 1 heteroatoms chosen from the above mentioned group.

If A^{d} is a mono- or polycyclic aromatic ring system that is substituted with one or more substituents and which may be bonded via an optionally at least mono-substituted alkylene, alkenylene or alkynylene group and/or which may contain at least one heteroatom as a ring member, unless otherwise defined, each one of these substituents may preferably be chosen from the group consisting of hydroxy, halogen, linear or branched C₁-C₆ alkyl, linear or branched C₁-C₆ alkoxy, -O-phenyl, linear or branched C₁-C₆ perfluoroalkyl, linear or branched C₁-C₆ perfluoroalkoxy, an optionally at least mono-substituted phenyl radical and 5- or 6-membered heteroaryl, more preferably from the group consisting of halogen, linear or branched C₁-C₆ alkyl, -O-phenyl, an optionally at least mono-substituted phenyl radical and 5- or 6-membered heteroaryl, even more preferably from the group consisting of fluorine, chlorine, -O-phenyl, linear or branched C₁-C₆ alkyl, an optionally at least mono-substituted phenyl radical and 5- or 6-membered heteroaryl.
If one or more of the rings of the mono- or polycyclic aromatic ring system contain one or more heteroatoms, these heteroatoms - like the heteroatoms of a previously mentioned 5- or 6-membered heteroaryl radical - may preferably be chosen from the group consisting of nitrogen, sulphur and oxygen.

If the previously mentioned phenyl radical is itself substituted by one or more substituents, each one of these substituents may preferably be chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and a -NR^{12d}R^{13d} radical, wherein R^{12d} and R^{13d}, identical or different, represent hydrogen or a linear or branched C₁-C₆ alkyl.

If the previously mentioned alkylene, alkenylene or alkynylene group is substituted by one or more substituents, each of these substituents may preferably be chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy or an optionally at least mono-substituted phenyl radical. If said phenyl radical is itself substituted by one or more substituents, each one of these substituents may preferably be chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and a -NR^{12d}R^{13d} radical, wherein R^{12d} and R^{13d}, identical or different, represent hydrogen or a linear or branched C₁-C₆ alkyl.

If one or more of the substituents R^{2d}, R^{3d}, R^{5d}, R^{6d} and R^{7d} represents an alcoxy radical, said radical may have 1 to 6, preferably 1 to 3 carbon atoms.

Sulfonamide derivatives of general formula (Id) are preferred, wherein R^{1d} represents a -NR^{8d}R^{9d} radical or a saturated or unsaturated optionally at least mono-substituted 5- or 6-membered cycloaliphatic radical, which may optionally contain at least one heteroatom as a ring member and which may be condensed with a saturated or unsaturated, optionally at least mono-substituted mono- or bicyclic cycloaliphatic ring, which may optionally contain at least one heteroatom as a ring member, whereby the rings of the ring system are 5- or 6-membered,
more preferably R^{1d} represents a -NR^{8d}R^{9d} radical or a radical chosen from the group consisting of 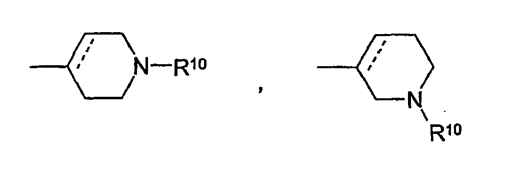 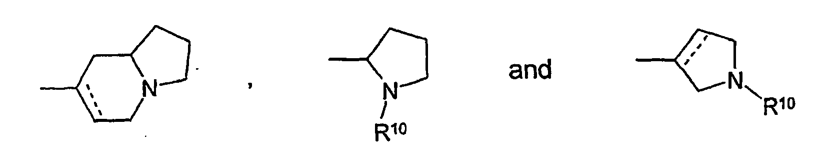 wherein, if present, the dotted line represents an optional chemical bond, and R¹⁰ represents hydrogen, a linear or branched C₁-C₆ alkyl radical or a benzyl radical, preferably hydrogen or a C₁-C₂ alkyl radical and R^{2d} -R^{9d} , A^{d} and nd are defined as above.

Sulfonamide derivatives of general formula (Id) are also preferred, wherein R^{2d}, R^{3d}, R^{5d}, R^{6d} and R^{7d}, identical or different, each represent hydrogen, a linear or branched, optionally at least mono-substituted C₁-C₆ alkyl radical, a linear or branched, optionally at least mono-substituted C₂-C₆ alkenyl radical, or a linear or branched, optionally at least mono-substituted C₂-C₆ alkynyl radical,
more preferably R^{2d} , R^{3a}, R^{5d}, R^{6d} and R^{7d}, identical or different, each represent hydrogen or a linear or branched , optionally at least mono-substituted C₁-C₆ alkyl radical,
even more preferably R^{2d}, R^{3a}, R^{5d}, R^{6d} and R^{7d} each represent hydrogen or a C₁-C₂ alkyl radical and R^{1d}, R^{4d}, R^{8d} , R^{9d} , A^{d} and nd are defined as above.

Sulfonamide derivatives of general formula (Id) are also preferred, wherein R^{4d} represents hydrogen, a linear or branched, optionally at least mono-substituted C₁-C₆ alkyl radical, a linear or branched, optionally at least mono-substituted C₂-C₆ alkenyl radical, a linear or branched, optionally at least mono-substituted C₂-C₆ alkynyl radical
more preferably R^{4d} represents hydrogen or a linear or branched , optionally at least mono-substituted C₁-C₆ alkyl radical,
even more preferably R^{4d} represents hydrogen or a C₁-C₂ alkyl radical and R^{1d}-R^{3d} , R^{5d}-R^{9d} A^{d} and nd are defined as above.

Furthermore, the use of sulfonamide derivatives of general formula (Id) is also preferred, wherein R^{8d} and R^{9d}, identical or different, each represent hydrogen, a linear or branched, optionally at least mono-substituted C₁-C₆ alkyl radical, or
R^{8d} and R^{9d} together with bridging nitrogen atom form a saturated or unsaturated, optionally at least mono-substituted 5- or 6-membered heterocyclic ring which may contain at least one additional heteroatom as a ring member and/or which may be condensed with a saturated or unsaturated, optionally at least mono-substituted mono- or bicyclic cycloaliphatic ring, which may optionally contain at least one heteroatom as a ring member, whereby the rings of the ring system are 5- 6- or 7-membered and R^{1d}-R^{7d}, A^{d} and nd are defined as above.

Particularly preferred is the use of sulfonamide derivatives of general formula (Id), wherein R^{8d} and R^{9d}, identical or different, each represent hydrogen or a linear or branched C₁-C₆ alkyl radical, preferably a linear or branched C₁-C₆ alkyl radical,
or
R^{8d} and R^{9d} together with bridging nitrogen atom form a radical chosen from the group consisting of 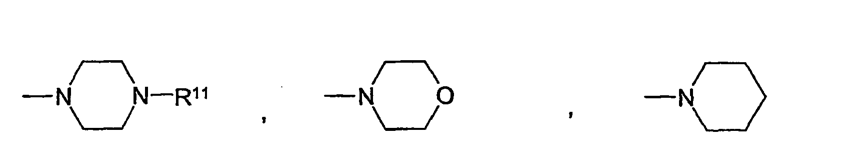 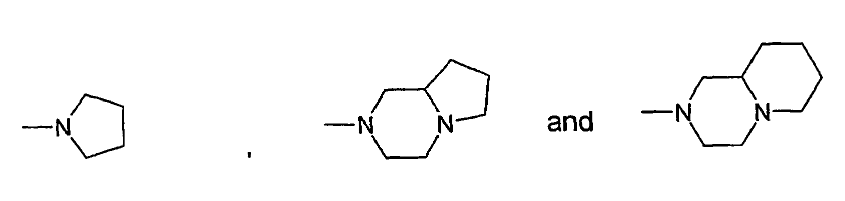 wherein R¹¹, if present, represents hydrogen, a linear or branched C₁-C₆ alkyl radical or a benzyl radical, preferably hydrogen, or a C₁-C₂ alkyl radical, and R^{1d}-R^{9d}, Ad and nd are defined as above.

Furthermore, sulfonamide derivatives of general formula (Id) are preferred, wherein A^{d} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂₋C₆ alkynylene group and/or wherein the ring(s) may contain at least one heteroatom as a ring member, preferably A^{d} represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 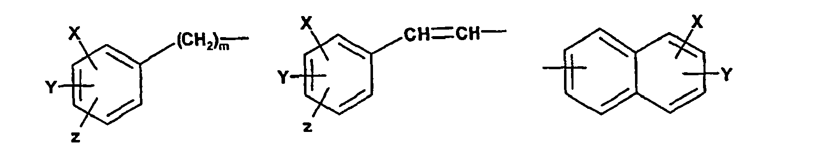 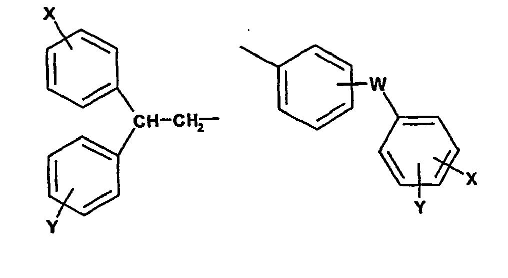 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical, wherein R¹² and R¹³, identical or different, each represent hydrogen or linear or branched C₁-C₆ alkyl,
W represents a single chemical bond between the two rings, a CH₂, O, S group or a NR¹⁴ radical, wherein R¹⁴ is hydrogen or a linear or branched C₁-C₆ alkyl,
m is 0, 1, 2, 3 or 4 and
and R^{1d}-R^{11d} are defined as described above.
Furthermore, sulfonamide derivatives of general formula (Id) are preferred, wherein A^{d} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member,
preferably A^{d} represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 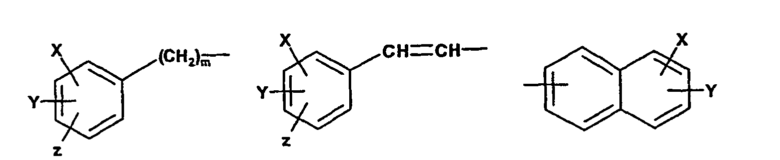 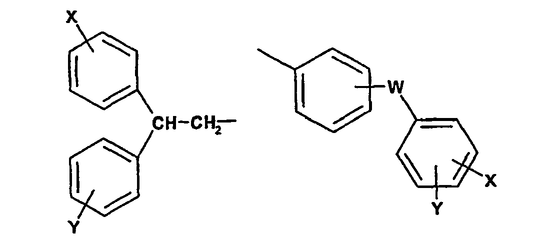 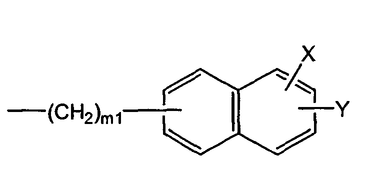 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R^{1d}-R^{9d} and nd are defined as above.

Further preferred is the use of compounds of general formula (Id),
wherein
R^{1d} represents a -NR^{8d} R^{9d} radical,
R^{2d}, R^{3d} , R^{5d}, R^{6d} and R^{7d} each represent hydrogen,
R^{4d} represents hydrogen,
R^{8d} and R^{9d}, identical or different, each represent methyl, ethyl, n-propyl, isopropyl, more preferably methyl,
A^{d} represents an aryl or heteroaryl radical selected from the group consisting of phenyl, naphthyl, benzo[b]thiophenyl and imidazo[2,1-b]thiazolyl which may be substituted by 1, 2 or 3 substituents selected from the group consisting of chlorine, methyl, phenyl and -O-phenyl and/or which may be bonded via a C₁₋₂ alkylene group,
and
nd is 2;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

The most preferred compounds of general formula (Id) are selected from the group consisting of
[1] N-[1-(2-dimethylaminoethyl)-1H-indole-4-yl]-5-chloro-3-methylbenzo[b]thiophene-2-sulfonamide,
[2] N-[1-(2-dimethylaminoethyl)-1H-indole-4-yl]-naphtalene-2-sulfonamide,
[3] N-[1-(2-dimethylaminoethyl)-1H-indole-4-yl]-naphtalene-1-sulfonamide,
[4] N-[1-(2-dimethylaminoethyl)-1H-indole-4-yl]-4-phenylbenzenesulfonamide,
[5] N-[1-(2-dimethylaminoethyl)-1H-indole-4-yl]-2-(naphtalene-1-yl)-ethanesulfonamide,
[6] N-[1-(2-dimethytaminoethyl)-1H-indole-4-yl]-4-phenoxybenzenesulfonamide,
[7] N-[1-(2-dimethylaminoethyl)-1H-indole-4-yl]-3,5-dichlorobenzenesulfonamide and
[8] 6-chloro-N-[1-(2-dimethylaminoethyl)-1H-indol-4-yl]-imidazo[2,1-b]thiazole-5-sulfonamide
and their corresponding salts and solvates.

Also, the most preferred compounds of general formula (Id) are selected from the group consisting of
[1] N-[1-(2-dimethylaminoethyl)-1H-indole-4-yl]-5-chloro-3-methylbenzo[b]thiophene-2-sulfonamide,
[2] N-[1-(2-dimethylaminoethyl)-1H-indole-4-yl]-naphtalene-2-sulfonamide,
[3] N-[1-(2-dimethylaminoethyl)-1H-indole-4-yl]-naphtalene-1-sulfonamide,
[4] N-[1-(2-dimethylaminoethyl)-1H-indole-4-yl]-4-phenylbenzenesulfonamide,
[5] N-[1-(2-dimethylaminoethyl)-1H-indole-4-yl]-2-(naphtalene-1-yl)-ethanesulfonamide,
[6] N-[1-(2-dimethylaminoethyl)-1H-indole-4-yl]-4-phenoxybenzenesulfonamide,
[7] N-[1-(2-dimethylaminoethyl)-1H-indole-4-yl]-3,5-dichlorobenzenesulfonamide and
and their corresponding salts and solvates.

The present invention likewise refers to the salts, preferably the physiologically acceptable salts of the compounds of general formula (Id), preferably the addition salts of mineral acids, more preferably of hydrochloric acid, hydrobromic acid acid, phosphoric acid, sulphuric acid, nitric acid, and the salts of organic acids, more preferably of citric acid, maleic acid acid, fumaric acid, tartaric acid or their derivatives, p-toluenesulphonic acid, methanesulphonic acid, camphorsulphonic acid, etc.

Below, the residues R¹-R⁷, A and n in the general formulas (IId) and (IIId) represent R^{1d}-R^{7d}, A^{d} and nd.

The derivatives of general formula (Id), wherein R^{1d}-R^{9d}, nd and A^{d} have the previously indicated meaning, may be preferably prepared in a way that:
At least one compound of general formula (IId), 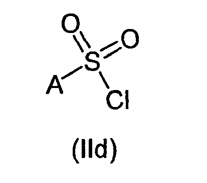
wherein A has the previously mentioned meaning, and X is an acceptable leaving group, preferably an halogen atom, more preferably chlorine; is reacted with at least one substituted 4-aminoindole of general formula (IIId) 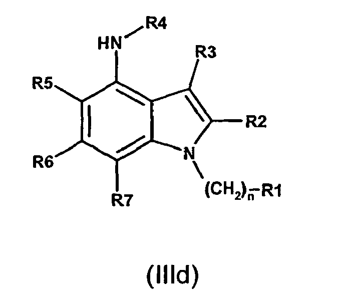 wherein R¹-R⁷ and n have the previously indicated meaning, or one of their suitable protected derivatives, and, if necessary, the protective groups are removed in order to obtain the corresponding sulfonamide derivative of formula (Id), which may be purified and/or isolated via conventional methods known in the prior art.

The reaction between the compounds of general formula (IId) and (IIId) is usually carried out in the presence of an organic reaction medium, preferably in the presence of dialkyl ether, more preferably diethyl ether or a cyclic ether, more preferably tetrahydrofuran or dioxane, an halogenated organic hydrocarbon, more preferably methylene chloride or chloroform, an alcohol, more preferably methanol or ethanol, a dipolar aprotic solvent, more preferably acetonitrile, pyridine or dimethylformamide, or any other suitable reaction medium. Naturally, mixtures of at least two of the classes of the mentioned compounds or at least two compounds of one class may also be used.

The reaction is preferably carried out in the presence of a suitable base, for example, an inorganic base, more preferably alkaline metal hydroxides and alkaline metal carbonates, or in the presence of an organic base, more preferably triethylamine, N-ethyldiisopropylamine or pyridine.

The most suitable reaction temperatures range from 0 °C to room temperature, that is, approximately 25 °C, and the reaction time is preferably from 5 minutes to 24 hours.

The resulting sulfonamide derivative of general formula (Id) may be purified and/or isolated according to conventional methods known in the prior art.

Preferably, the sulfonamide derivatives of general formula (Id) may be isolated by evaporating the reaction medium, adding water and, if necessary, adjusting the pH so that a solid which may be isolated by filtration is obtained; or the sulfonamide derivatives may be extracted with a water immiscible solvent, preferably chloroform, and be purified by chromatography or recrystallization of a suitable solvent.

The compounds of general formula (IId) are commercially available, or they may be prepared according to standard methods known in the prior art, for example by methods similar to those described in the literature [E.E.Gilbert, Synthesis, 1969, 1, 3]. The compounds of general formula (IIId) may also be prepared according to standard methods known in the prior art, for example by methods similar to those described in: [Abou-Gharbia, Magid; Patel, Usha; Tokolics, Joseph; Freed, Meier. European Journal of Medicinal Chemistry (1988), 23(4), 373-7].

Another aspect of the present invention consists in a process for preparing the sulfonamide derivatives of general formula (Id), wherein R^{1d}-R^{3d}, R^{5d}-R^{9d}, nd and A^{d} have the previously indicated meaning and R^{4d} is an alkyl radical, preferably a linear or branched , optionally at least mono-substituted C₁-C₆ alkyl radical, by alkylation of a sulfonamide derivative of general formula (I), wherein R^{1d}-R^{3d}, R^{5d}-R^{7d}, nd and A^{d} have the previously indicated meaning, and R^{4d} is an hydrogen atom, with an alkyl halogenide or a dialkyl sulfate.

The alkylation reaction is carried out preferably in the presence of a suitable base, more preferably in the presence of alkaline metal hydroxides and alkaline metal carbonates, metal hydrides, metal alkoxides, even more preferably sodium methoxide or potassium tert-butoxide, organometallic compounds, even more preferably butyllithium or tert-butyllithium, in the presence of an organic reaction medium, more preferably dialkyl ether, even more preferably diethyl ether, or a cyclic ether, even more preferably tetrahydrofuran or dioxane, an hydrocarbon, even more preferably toluene, an alcohol, even more preferably methanol or ethanol, a dipolar aprotic solvent, even more preferably acetonitrile, pyridine or dimethylformamide, or any other suitable reaction medium. Naturally, mixtures of at least two of the classes of the mentioned compounds or at least two compounds of one class may also be used.

The most suitable reaction temperatures range from 0 °C to the boiling temperature of the reaction medium, and the reaction times are preferably from 1 to 24 hours.

Preferably, the resulting sulfonamide derivative of general formula (Id) may be isolated by filtration, concentrating the filtrate under reduced pressure, adding water and, if necessary, adjusting the pH so that a solid which may be isolated by filtration is obtained; or the sulfonamide derivatives may be extracted with a water immiscible solvent, preferably chloroform, and be purified by chromatography or recrystallization of a suitable solvent.

The salts, preferably pharmaceutically acceptable salts of the compounds of general formula (Id), may.be prepared by means of conventional methods known in the prior art, preferably by reaction with a mineral acid, more preferably by reaction with hydrochloric acid, hydrobromic acid, phosphoric acid acid, sulphuric acid or nitric acid, or by reaction with organic acids, more preferably by reaction with citric acid, maleic acid, fumaric acid acid, tartaric acid, or their derivatives, p-toluenesulphonic acid, methanesulphonic acid, camphorsulphonic acid, etc., in a suitable solvent, preferably methanol, ethanol, diethyl ether, ethyl acetate, acetonitrile or acetone, and obtaining the resulting salts by using the usual techniques for the precipitation or crystallization of the corresponding salts.

The preferred physiologically acceptable salts of the sulfonamide derivatives of general formula (Id) are the addition salts of mineral acids, more preferably of hydrochloric acid, hydrobromic acid, phosphoric acid, sulphuric acid acid or nitric acid, and the addition salts of organic acids, more preferably citric acid, maleic acid, fumaric acid, tartaric acid, or their derivatives, p-toluenesulphonic acid, methanesulphonic acid, camphorsulphonic acid, etc.

The solvates, preferably the physiologically acceptable solvates, more preferably hydrates, of the sulfonamide derivatives of general formula (Id) or of the corresponding physiologically acceptable salts, may be prepared by methods known in the prior art.

During some of the synthetic sequences described or in the preparation of the suitable reagents used, it may be necessary and/or desirable to protect sensitive or reactive groups in some of the molecules used. This may be carried out via the use of conventional protective groups preferably those described in the literature [Protective groups in Organic Chemistry, ed. J.F.W. McOmie, Plenum Press, 1973; T.W. Greene & P.G.M. Wuts, Protective Groups in Organic Chemistry, John Wiley & Sons, 1991]. The protective groups may be removed in the suitable subsequent stage by methods known in the prior art.

If the sulfonamide derivatives of general formula (Id) are obtained in form of a mixture of stereoisomers, preferably enantiomers or diastereomers, said mixtures may be separated via standard processes known in the prior art, for example chromatographic methods or crystallization with chiral agents.

If one or more of the R^{2e}-R^{9e} moieties represent an alkyl radical which is substituted by one or more substituents, unless otherwise defined, each one of the substituents can preferably be chosen from the group consisting of hydroxy, fluorine, chlorine, bromine and trifluoromethyl.

If R^{1e} is a saturated or unsaturated cycloaliphatic radical, optionally containing at least one heteroatom as a ring member, which is substituted by one or more substituents and/or if it comprises a saturated or unsaturated, mono- or bicyclic cycloaliphatic ring system, optionally containing at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl. The heteroatoms of the cycloaliphatic radical and/or of the mono- or bi- cyclic cycloaliphatic ring can, independently from one another, be chosen preferably from the group consisting of nitrogen, sulfur and oxygen, more preferably nitrogen as an heteroatom.

If R^{8e} and R^{9e} together with the nitrogen atom bridge form a saturated or unsaturated heterocyclic ring, which can contain at least one additional heteroatom as a ring member, which is substituted by one or more substituents and/or condensed with a saturated or unsaturated mono- or bi- cyclic cycloaliphatic ring system, which can contain at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, more preferably from the group consisting of linear or branched C₁-C₆ alkyl and benzyl. If the heterocyclic ring contains one or more additional heteroatoms, and/or if one or both mono- or bicyclic rings contain one or more heteroatoms, these heteroatoms can, independently from one another, be preferably chosen from the group consisting of nitrogen, sulfur and oxygen, more preferably nitrogen as heteroatom.

If A^{e} is a mono or poly-cyclic aromatic ring system, which is substituted by one or more substituents, and which can be bonded by means of an optionally at least monosubstituted alkylene, alkenylene or alkynylene group, and/or can contain at least one heteroatom as a ring member, unless otherwise defined, each one of the substituents can be preferably chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, a phenyl radical, optionally at least monosubstituted, and heteroaryl of 5 or 6 members, more preferably from the group consisting of halogen, linear or branched C₁-C₆ alkyl, phenyl optionally at least monosubstituted and heteroaryl of 5 or 6 members, much more preferably from the group consisting of fluorine, chlorine, linear or branched C₁-C₆ alkyl, phenyl radical, optionally at least monosubstituted and heteroaryl of 5 or 6 members. If one or more of the rings of a mono or poly-cyclic aromatic ring system contains one or more heteroatoms, these heteroatoms - like the heteroatoms of a previously mentioned heteroaryl radical of 5 or 6 members-can be preferably chosen from the group consisting of nitrogen, sulfur and oxygen. If the previously mentioned phenyl radical is itself substituted by one or more substituents, each one of the substituents can be preferably chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and an NR^{12e}R^{13e} radical, wherein R^{12e} and R^{13e}, identical or different, are hydrogen or linear or branched C₁-C₆ alkyl.

Also preferably, the substituents for Ae may be selected from the group consisting of nitro, -O-phenyl, -O-C₁₋₆ alkyl, -C(=O)-C₁₋₆ alkyl, hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, an optionally at least mono-substituted phenyl radical and 5- or 6-membered heteroaryl, more preferably from the group consisting of nitro, -O-phenyl,-C(=O)-C₁₋₆ alkyl, linear or branched C₁-C₆ alkoxy, halogen, linear or branched C₁-C₆ alkyl, an optionally at least mono-substituted phenyl radical and 5- or 6-membered heteroaryl, even more preferably from the group consisting of nitro,-O-phenyl, -O-CH₃, -C(=O)-CH₃, fluorine, chlorine, bromine, linear or branched C₁-C₆ alkyl, an optionally at least mono-substituted phenyl radical and 5- or 6-membered heteroaryl.

If the previously mentioned alkylene, alkenylene or alkynylene group is substituted by one or more substituents, each of the substituents can be preferably chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy or a phenyl radical, optionally at least monosubstituted. If said phenyl radical is itself substituted by one or more substituents, each one of the substituents can be preferably chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and an NR^{12e}R^{13e} radical, wherein R^{12e} and R^{13e}, identical or different, are hydrogen or linear or branched C₁-C₆ alkyl.

Sulfonamide derivatives of general formula (le) are preferred, wherein R^{1e} is an -NR^{8e}R^{9e} radical or a saturated or unsaturated, optionally at least mono-substituted 5- or 6-membered cycloaliphatic radical which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- or 6-membered,
preferably R^{1e} represents an -NR^{8e}R^{ge} radical or a radical chosen from the group consisting of 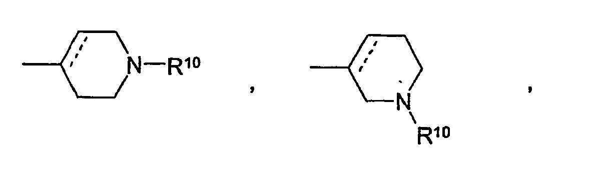 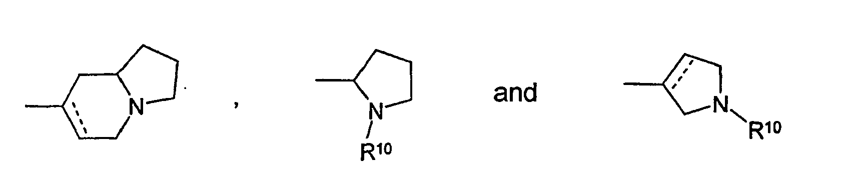 wherein, if present, the dotted line is an optional chemical bond, and R¹⁰ represents hydrogen, a linear or branched C₁-C₆ alkyl radical or a benzyl radical, preferably hydrogen or a C₁-C₂ alkyl radical.

Sulfonamide derivatives of general formula (le) are also preferred, wherein R^{2e}, R^{3e}, R^{4e}, R^{6e} and R^{7e}, are hydrogen, a linear or branched C₁-C₆ alkyl radical, a linear or branched C₂-C₆ alkenyl radical, or a linear or branched C₂-C₆ alkynyl radical, preferably hydrogen.

The use of sulfonamide derivatives of general formula (le) is also preferred, wherein R^{5e}, is hydrogen or a linear or branched C₁-C₆ alkyl radical, optionally at least monosubstituted, preferably hydrogen or a linear or branched C₁-C₆ alkyl radical, more preferably hydrogen or an C₁-C₂ alkyl radical and R^{1e}-R^{4e}, R^{6e}-R^{9e}, A^{e} and ne are defined as above.

Furthermore, the use of sulfonamide derivatives of general formula (Ie) is also preferred, wherein R^{8e} and R^{9e}, identical or different, are hydrogen or a linear or branched, optionally at least monosubstituted C₁-C₆ alkyl radical, or

R^{8e} and R^{9e}, together with the nitrogen atom bridge, form a saturated or unsaturated heterocyclic ring of 5 or 6 members, optionally at least monosubstituted, which can contain at least one additional heteroatom as a ring member, and/or can be condensed with a saturated or unsaturated, mono- or bi- cyclic cycloaliphatic ring system, optionally at least monosubstituted, which can optionally contain at least one heteroatom as a ring member, where the ring/rings is/are of 5, 6 or 7 members, and R^{1e}-R^{7e}, A^{e} and ne are defined as above.

Particularly preferred is the use of sulfonamide derivatives of general formula (le), wherein R^{8e} and R^{9e}, identical or different, are hydrogen or a linear or branched C₁-C₆ alkyl radical, preferably a linear or branched C₁-C₆ alkyl radical, or
R^{8e} and R^{9e} together with the nitrogen atom bridge form a radical chosen from the group consisting of 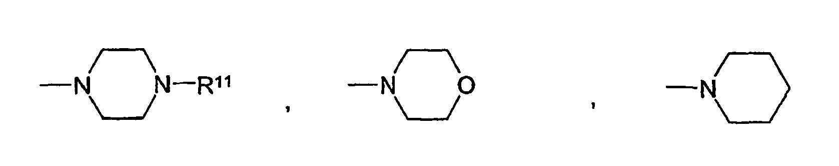 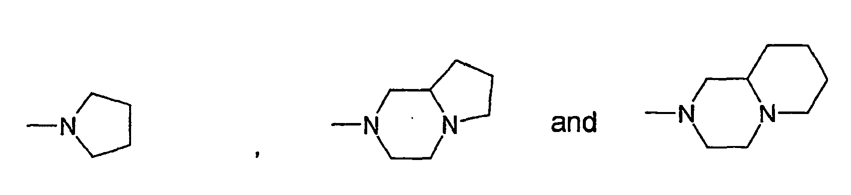 wherein R¹¹, if it is present, is hydrogen, a linear or branched C₁-C₆ alkyl radical or a benzyl radical, preferably hydrogen, or a C₁-C₂ alkyl radical, and R^{1e}-R^{9e}, A^{e} and ne are defined as above.

Furthermore, sulfonamide derivatives of general formula (Ie) are preferred, wherein A^{e} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member,
preferably A^{e} represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 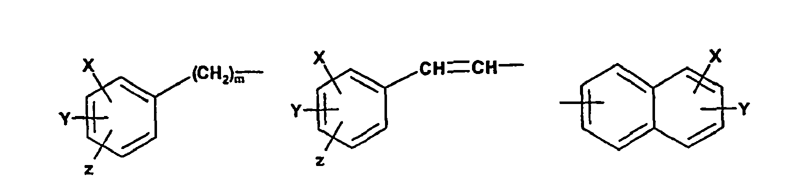 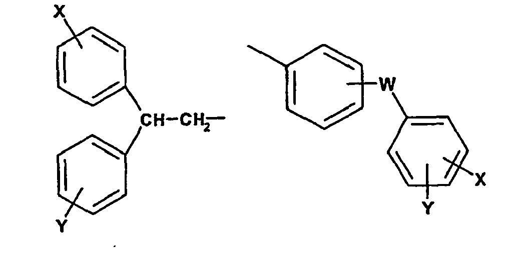 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl, W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
and R^{1e}-R^{11e} and ne are defined as above.

Furthermore, sulfonamide derivatives of general formula (le) are preferred, wherein A^{e} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or
wherein the ring(s) may contain at least one heteroatom as a ring member, preferably A represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 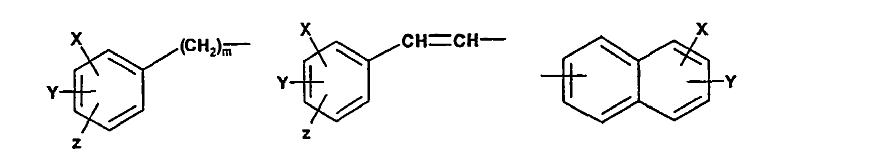 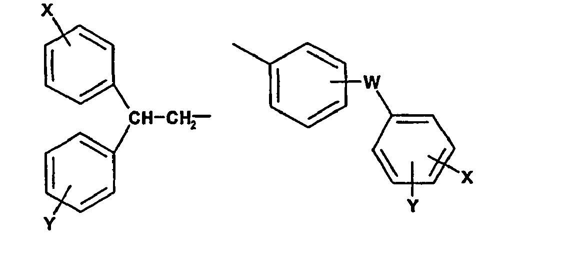 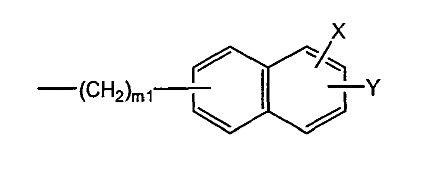 wherein X, Y, Z, independently from one another, each represent a radical selected from the group consisting of hydrogen, fluorine, chlorine, bromine, nitro, acetyl, linear or branched C₁-C₆ alkyl, linear or branched C₁-C₆ alkoxy, linear or branched C₁-C₆ alkylthio, a trifluoromethyl radical, a cyano radical and
a -NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
ml is 1 or 2, preferably 2, and R^{1e}-R^{9e} and ne are defined as above.

Also preferred are compounds of general formula (le) given above,
wherein
R^{1e} represents a -NR^{8e}R^{9e} radical,
R^{2e} represents hydrogen or an alkyl radical selected from the group consisting of methyl, ethyl, n-propyl and iso-propyl, more preferably hydrogen or methyl,
R^{3e}, R^{4e}, R^{6e} and R^{7e} each represent hydrogen,
R^{5e} represents hydrogen,
R^{8e} and R^{9e}, identical or different, each represent methyl, ethyl, n-propyl or isopropyl, more preferably methyl or ethyl,
or
R^{8e} and R^{9e} together with the bridging nitrogen form a 5- or 6-membered heterocyclic ring, more preferably form pyrrolidine or piperidine,
A^{e} represents an aryl or heteroaryl radical selected from the group consisting of phenyl, naphthyl, quinolinyl, benzo[b]thiophenyl, benzo[1,2,5]thiadiazolyl, thiophenyl and imidazo[2,1-b]thiazolyl which may be substituted by 1, 2 or 3 substituents selected from the group consisting of fluorine, bromine, chlorine, methyl, phenyl, nitro, -C(=O)-CH₃, -O-CH₃ and -O-phenyl and/or which may be bonded via a C₁-₂ alkylene group or a C₂ alkenylene group,
and
ne is 2 or 3,
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

The most preferred compounds of general formula (le) are:
[1] N-[1-(2-dimethylaminoethyl)-1H-indole-5-yl]-5-chloro-3-methylbenzo[b]thiophene-2-sulfonamide,
[2] N-[1-(2-dimethylaminoethyl)-1H-indole-5-yl]-naphthalene-2-sulfonamide,
[3] N-[1-(2-dimethylaminoethyl)-1H-indole-5-yl]-naphthalene-1-sulfonamide,
[4] N-[1-(2-dimethylaminoethyl)-1H-indole-5-yl]-5-chloronaphthalene-1-sulfonamide,
[5] N-[1-(2-dimethylaminoethyl)-1H-indole-5-yl]-benzenesulfonamide,
[6] N-[1-(2-dimethylaminoethyl)-1H-indole-5-yl]-quinoline-8- sulfonamide,
[7] N-[1-(2-dimethylaminoethyl)-1H-indole-5-yl]-4-phenoxybenzenesulfonamide,
[8] N-[1-(2-dimethylaminoethyl)-1H-indole-5-yl]-4-methylbenzenesulfonamide,
[9] N-[1-(2-dimethylaminoethyl)-1H-indole-5-yl]-5-chlorothiophene-2-sulfonamide,
[10] N-[1-(2-dimethylaminoethyl)-1H-indole-5-yl]-benzo[1,2,5]thiadiazole-4-sulfonamide,
[11] N-[1-(2-dimethylaminoethyl)-1H-indole-5-yi]-6-chloroimidazo[2,1-b]thiazole-5-sulfonamide,
[12] N-[1-(2-dimethylaminoethyl)-1H-indole-5-yl]-3,5-dichlorobenzenesulfonamide,
[13] N-[1-(2-dimethylaminoethyl)-1H-indole-5-yl]-3-bromobenzenesulfonamide,
[14] N-[1-(2-dimethylaminoethyl)-1H-indole-5-yl]-3-nitrobenzenesulfonamide,
[15] N-[1-(2-dimethylaminoethyl)-1H-indole-5-yl]-1-phenylmethanesulfonamide,
[16] N-[1-(2-pyrrolidine-1-yl-ethyl)-1H-indole-5-yl]-naphthalene-2-sulfonamide,
[17] N-[1-(2-pyrrolidine-1-yl-ethyl)-1H-indole-5-yl]-naphthalene-1-sulfonamide,
[18] N-[1-(2-pyrrolidine-1-yl-ethyl)-1H-indole-5-yl]- 5-chloro-3-methylbenzo[b]thiophene-2-sulfonamide,
[19] *trans*-N-[1-(2-dimethylaminoethyl)-1H-indole-5-yl]-2-phenylethenesulfonamide,
[20] N-[1-(2-dimethylaminoethyl)-1H-indole-5-yl]-4,5-dichlorothiophene-2-sulfonamide,
[21] N-[1-(2-dimethylaminoethyl)-1H-indole-5-yl]-4-acetylbenzenesulfonamide,
[22] N-[1-(2-dimethylaminoethyl)-1H-indole-5-yl]-4-bromobenzenesulfonamide,
[23] N-[1-(2-dimethylaminoethyl)-1H-indole-5-yl]-4-methoxybenzenesulfonamide,
[24] N-[3-(2-diethylaminoethyl)-1H-indole-5-yl]-5-chloro-3-methylbenzo[b]thiophene-2-sulfonamide,
[25] N-[1-(2-dimethylaminoethyl)-1H-indole-5-yl]-4-nitrobenzenesulfonamide,
[26] N-[1-(2-dimethy)aminoethyl)-1H-indole-5-yl]-4-fluorobenzenesulfonamide,
[27] N-[1-(2-diethylaminoethyl)-1H-indole-5-yl]-6-chloroimidazo[2,1-b]thiazole-5-sulfonamide,
[28] N-[1-(2-pyrrolidine-1-yl-ethyl)-1H-indole-5-yl]-]-6-chloroimidazo[2,1-b]thiazole-5-sulfonamide, and their corresponding salts and solvates.

Also, the most preferred compounds of general formula (le) may be selected from the group consisting of:
[1] N-[1-(2-dimethylaminoethyl)-1H-indole-5-yl]-5-chloro-3-methylbenzo[b]thiophene-2-sulfonamide,
[2] N-[1-(2-dimethylaminoethyl)-1H-indole-5-yl]-naphthalene-2-sulfonamide,
[3] N-[1-(2-dimethylaminoethyl)-1H-indole-5-yl]-naphthalene-1-sulfonamide,
[4] N-[1-(2-dimethylaminoethyl)-1H-indole-5-yl]-5-chloronaphthalene-1-sulfonamide,
[5] N-[1-(2-dimethylaminoethyl)-1H-indole-5-yl]-benzenesulfonamide,
[6] N-[1-(2-dimethylaminoethyl)-1H-indole-5-yl]-quinoline-8- sulfonamide,
[7] N-[1-(2-dimethylaminoethyl)-1H-indole-5-yl]-4-phenoxybenzenesulfonamide,
[8] N-[1-(2-dimethylaminoethyl)-1H-indole-5-yl]-4-methylbenzenesulfonamide,
[9] N-[1-(2-dimethylaminoethyl)-1H-indole-5-yl]-5-chlorothiophene-2-sulfonamide,
[10] N-[1-(2-dimethylaminoethyl)-1H-indole-5-yl]-benzo[1,2,5]thiadiazole-4-sulfonamide,
[11] N-[1-(2-dimethylaminoethyl)-1H-indole-5-yl]-6-chloroimidazo[2,1-b]thiazole-5-sulfonamide,
[12] N-[1-(2-dimethylaminoethyl)-1H-indole-5-yl]-3,5-dichlorobenzenesulfonamide,
[13] N-[1-(2-dimethylaminoethyl)-1H-indole-5-yl]-3-bromobenzenesulfonamide,
[14] N-[1-(2-dimethylaminoethyl)-1H-indole-5-yl]-3-nitrobenzenesulfonamide,
[15] N-[1-(2-dimethylaminoethyl)-1H-indole-5-yl]-1-phenylmethanesulfonamide,
[16] N-[1-(2-pyrrolidine-1-yl-ethyl)-1H-indole-5-yl]-naphthalene-2-sulfonamide,
[17] N-[1-(2-pyrrolidine-1-yl-ethyl)-1H-indole-5-yf]-naphthalene-1-sulfonamide,
[18] N-[1-(2-pyrrolidine-1-yl-ethyl)-1H-indole-5-yl]- 5-chloro-3-methylbenzo[b]thiophene-2-sulfonamide,
[19] *trans*-N-[1-(2-dimethylaminoethyl)-1H-indole-5-yl]-2-phenylethenesulfonamide,
[20] N-[1-(2-dimethylaminoethyl)-1H-indole-5-yl]-4,5-dichlorothiophene-2-sulfonamide,
[21] N-[1-(2-dimethylaminoethyl)-1H-indole-5-yl]-4-acetylbenzenesulfonamide,
[22] N-[1-(2-dimethylaminoethyl)-1H-indole-5-yl]-4-bromobenzenesulfonamide,
[23] N-[1-(2-dimethytaminoethyl)-1H-indole-5-yl]-4-methoxybenzenesulfonamide,
[24] N-[3-(2-diethylaminoethyl)-1H-indole-5-yl]-5-chloro-3-methylbenzo[b]thiophene-2-sulfonamide,
[25] N-[1-(2-dimethylaminoethyl)-1H-indole-5-yl]-4-nitrobenzenesulfonamide,
[26] N-[1-(2-dimethylaminoethyl)-1H-indole-5-yl]-4-fluorobenzenesulfonamide,
[27] N-[1-(2-diethylaminoethyl)-1H-indole-5-yl]-6-chloroimidazo[2,1-b]thiazole-5-sulfonamide,
[28] N-[1-(2-pyrrolidine-1-yl-ethyl)-1H-indole-5-yl]-]-6-chloroimidazo[2 ,1 - b]thiazole-5-sulfonamide,
[29] N-(1-(2-(diethylamino)ethyl)-1H-indol-5-yl)-naphthalene-2-sulfonamide,
[30] N-(1-(2-(diethylamino)ethyl)-1H-indol-5-yl)-naphthalene-1-sulfonamide,
[31] N-(1-(2-(diethylamino)ethyl)-1H-indol-5-yl)-4-phenylbenzenesulfonamide,
[32] 5-chloro-N-(1-(2-(dimethylamino)ethyl)-2-methyl-1H-indol-5-yl)-3-methylbenzo[b]thiophene-2-sulfonamide,
[33] N-(1-(2-(dimethylamino)ethyl)-2-methyl-1H-indo(-5-yl)-naphthalene-2-sulfonamide,
[34] N-(1-(2-(dimethylamino)ethyl)-2-methyl-1H-indol-5-yl)-naphthalene-1-sulfonamide,
[35] 6-chloro-N-(1-(2-(dimethylamino)ethyl)-2-methyl-1H-indol-5-yl)imidazo[2,1-b]thiazole-5-sulfonamide,
[36] N-(1-(2-(dimethylamino)ethyl)-2-methyl-1H-indol-5-yl)-4-phenylbenzenesulfonamide,
[37] N-(1-(2-dimethylamino)ethyl)-2-methyl-1H-indol-5-yl)-2-(naphth-1-yl)-ethanesulfonamide,
[38] N-(1-(2-(dimethylamino)ethyl)-2-methyl-1H-indol-5-yl)-4-phenoxybenzenesulfonamide,
[39] 3,5-dichloro-N-(1-(2-(dimethylamino)ethyl)-2-methyl-1H-indol-5-yl)-benzenesulfonamide,
[40] N-(1-(2-(dimethylamino)ethyl)-2-methyl-1H-indol-5-yl)benzo[b]thiophene-3-sulfonamide,
[41] N-(1-(2-(diethylamino)ethyl)-1H-indol-5-yl)benzo[b]thiophene-3-sulfonamide and
[42] N-(1-(2-(dimethylamino)ethyl)-1H-indol-5-yl)benzo[b]thiophene-3-sulfonamide,
[43] 5-chloro-3-methyl-N-(1-(3-(piperidin-1-yl)propyl)-1H-indol-5-yl)benzo[b]thiophene-2-sulfonamide,
[44] N-(1-(3-(piperidin-1-yl)propyl)-1H-indol-5-yl)naphthalene-2-sulfonamide,
[45] N-(1-(3-(piperidin-1-yl)propyl)-1H-indol-5-yl)naphthalene-1-sulfonamide,
[46] 6-chloro-N-(1-(3-piperidin-1-yl)propyl)-1H-indol-5-yl)imidazo[2,1-b]thiazole-5-sulfonamide,
[47] 4-phenyl-N-(1-(3-(piperidin-1-yl)propyl)-1H-indol-5-yl)benzenesulfonamide,
[48] 2-(naphth-1-yl)-N-(1-(3-(pipendin-1-yl)propyl)-1H-indol-5-yl)ethanesulfonamide,
[49] 4-phenoxy-N-(1-(3-(piperidin-1-yl)propyl)-1H-indol-5-yl)benzenesulfonamide,
[50] 3,5-dichloro-N-(1-(3-(pipeddin-1-yl)propyl)-1H-indol-5-yl)benzenesulfonylamide,
[51] 4,5-dichloro-N-(1-(3-(piperidin-1-yl)propyl)-1H-indol-5-yl)thiophene-2-sulfonamide and
[52] 5-chloro-N-(1-(3-(piperidin-1-yl)propyl)-1H-indol-5-yl)naphthalene-1-sulfonamide,
and their corresponding salts and solvates.

The present invention likewise refers to the physiologically acceptable salts of the compounds of general formula (Ie), particularly the addition salts of mineral acids, such as hydrochloric, hydrobromic, phosphoric, sulfuric, nitric acids, and with organic acids such as citric, maleic, fumaric, tartaric acids or their derivatives, *p*-toluenesulfonic, methanesulfonic, camphorsulfonic acids, etc.

Below, the residues R¹-R⁷, A and n in the general formulas (IIe) to (Ve) are R^{1e}-R^{7e}, A^{e} and ne.

The derivatives of general formula (Ie), wherein R^{1e}-R^{9e}, ne and A^{e} have the previously indicated meaning, may be preferably prepared in a way that:
At least one compound of general Formula (IIe), 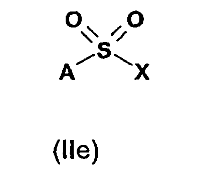
wherein A has the previously mentioned meaning in the general formula (le), and X is an acceptable leaving group, preferably an halogen atom, more preferably chlorine; reacts with at least one substituted 5-aminoindole of general formula (IIIe) 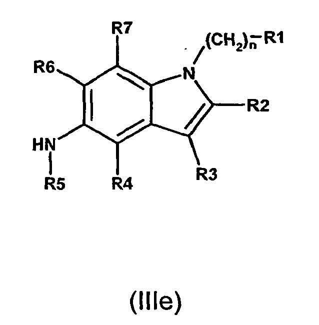 wherein R¹-R⁷ and n have the previously indicated meaning, or one of their suitable protected derivatives, and, if necessary, the protective groups are removed in order to obtain the corresponding sulfonamide derivative of formula (Ie), which can be purified and/or isolated by means of conventional methods known in the state of the art.

The reaction between the compounds of general Formula (IIe) and (IIIe) is usually carried out in the presence of an organic reaction medium such as dialkyl ether, particularly diethyl ether or a cyclic ether, particularly tetrahydrofuran or dioxane, an halogenated organic hydrocarbon, particularly methylene chloride or chloroform, an alcohol, particularly methanol or ethanol, a dipolar aprotic solvent, particularly acetonitrile, pyridine or dimethylformamide, or any other suitable reaction medium. Naturally, mixtures of at least two of the classes of the mentioned compounds or at least two compounds of one class can also be used.

The reaction is preferably carried out in the presence of a suitable base, for example, an inorganic base such as alkaline metal hydroxides and carbonates, or in the presence of an organic base, particularly triethylamine, N-ethyldiisopropylamine or pyridine.

The most suitable reaction temperatures range between 0°C and room temperature, that is, approximately 25°C, and the reaction time is preferably comprised between 5 minutes and 24 hours.

The resulting sulfonamide derivative of general Formula (le) can be purified and/or isolated according to conventional methods known in the state of the art.

Preferably, the sulfonamide derivatives of general Formula (le) can be isolated by evaporating the reaction medium, adding water and, if necessary, adjusting the pH so that a solid which can be isolated by filtration is obtained; or it can be extracted with a water immiscible solvent, such as chloroform, and be purified by chromatography or recrystallization of a suitable solvent.

The compounds of general formula (IIe) are commercially available, or they can be prepared according to standard methods known in the state of the art, for example by methods similar to those described in the literature [E.E.Gilbert, Synthesis, 1969, 1, 3]. The compounds of general formula (IIIe) can also be prepared according to standard methods known in the state of the art, for example by methods similar to those described in the literature: Pigerol, Charles; De Cointet de Fillain, Paul; Eymard, Pierre; Werbenec, Jean Pierre; Broil, Madeleine. (Labaz S. A., Fr.). Ger. Offen. (1977). DE 2727047 19771229. Schwink, Lothar; Stengelin, Siegfried; Gossel, Matthias. Preparation of indol-5-ylureas and relate compounds for the treatment of obesity and type II diabetes. WO 0315769 A1 20030227. One of them consists of nitro group reduction of derivatives of general formula (IVe) by methods known in the art, as for example: BRATTON, L. D.; ROTH, B. D.; TRIVEDI, B. K.; UNANGST, P. C.; J Heterocycl Chem , 2000, 37 (5), 1103-1108. FANGHAENEL, E.; CHTCHEGLOV, D.; J Prakt Chem/Chem-Ztg, 1996, 338 (8), 731-737. KUYPER, L. F.; BACCANARI, D. P.; JONES, M. L.; HUNTER, R. N.; TANSIK, R. L.; JOYNER, S. S.; BOYTOS, C. M.; RUDOLPH, S. K.; KNICK, V.; WILSON, H. R.; CADDELL, J. M.; FRIEDMAN, H. S.; ET AL.; J Med Chem, 1996, 39 (4), 892-903. 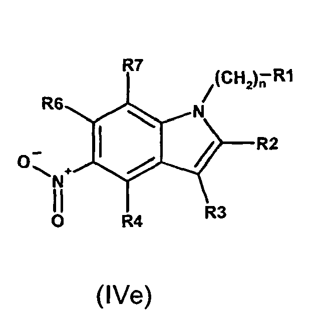 wherein R¹-R⁷ and n have the previously indicated meaning, or one of their suitably protected derivatives, and, if necessary, the protective groups are removed in order to obtain the corresponding amine of general formula (IIIe), which can be purified and/or isolated by means of conventional methods known in the state of the art.

The compounds of general formula (IVe) can also be prepared according to standard methods known in the state of the art, for example by methods similar to those described in the literature: Journal of Heterocyclic Chemistry, 37(5), 1103-1108; 2000; Schwink, Lothar; Stengelin, Siegfried; Gossel, Matthias. Preparation of indol-5-ylureas and relate compounds for the treatment of obesity and type II diabetes WO 0315769 A1 20030227; Baxter, Andrew; Brough, Stephen; Mcinally, Thomas; Mortimore, Michael; Cladingboel, David. Preparation of N-aryl-1-adamantaneacetamides and analogs as purinergic P2Z receptor antagonists WO 9929660 A1 19990617 ; Pigerol, Charles; De Cointet de Fillain, Paul; Eymard, Pierre; Werbenec, Jean Pierre; Broil, Madeleine. Indole derivatives. Ger. Offen. (1977), DE 2727047 19771229

One of them consists in the alkylation of nitro derivatives of general formula (Ve) by methods known in the art, as for example: BHAGWAT, S. S.; GUDE, C.; Tetrahedron Lett, 1994, 35 (12), 1847-1850. BRATTON, L. D.; ROTH, B. D.; TRIVEDI, B. K.; UNANGST, P. C.; J Heterocycl Chem, 2000, 37 (5), 1103-1108 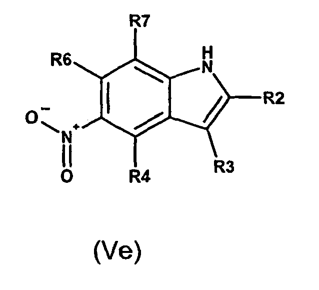 wherein R²-R⁷ and n have the previously mentioned meaning, or one of their suitably protected derivatives, and, if necessary, the protective groups are removed in order to obtain the corresponding amine of general formula (IIIe), which can be purified and/or isolated by means of conventional methods known in the state of the art.

The compounds of general formula (Ve) are commercially available or can also be prepared according to standard methods known in the state of the art, as for example YAMASHKIN, S. A.; YUROVSKAYA, M. A.; Chem Heterocycl Compd (N Y) 1999, 35 (12), 1426-1432. OTTONI, O.; CRUZ, R.; KRAMMER, N. H.; Tetrahedron Lett ,1999, 40 (6), 1117-1120. EZQUERRA, J.; PEDREGAL, C.; LAMAS, C.; BARLUENGA, J.; PEREZ, M.; GARCIA-MARTIN, M. A.; GONZALEZ, J. M.; J Org Chem ,1996, 61 (17), 5804-5812. FADDA, A. A.; Indian J Chem, Sect B: Org Chem Incl Med Chem , 1990, 29 (11), 1017-1019. KATRITZKY, A. R.; RACHWAL, S.; BAYYUK, S.; Org Prep Proced Int, 1991, 23 (3), 357-363. Inada, A.; Nakamura, Y.; Morita, Y.; Chem Lett , 1980, 1287.

The sulfonamide derivatives of general Formula (le), wherein R^{1e}-R^{4e}, R^{6e}-R^{7e}, A^{e}, ne and A^{e} have the previously indicated meaning and R^{5e} is an alkyl radical, preferably a linear or branched C₁-C₆ alkyl radical, optionally at least monosubstituted, they can also be prepared by alkylation of a sulfonamide derivative of general Formula (le), wherein R^{1e}-R^{4e}, R^{6e}-R^{7e} , ne and A^{e} have the previously indicated meaning, and R^{5e} is an hydrogen atom, with an alkyl halogenide or dialkyl sulfate.

The alkylation reaction is carried out preferably in the presence of a suitable base, such as alkaline metal hydroxides and carbonates, metal hydrides, alkoxides such as sodium metoxide or potassium tert-butoxide, organometallic compounds such as butyllithium or tert-butyllithium, in the presence of an organic reaction medium such as dialkyl ether, particularly diethyl ether, or a cyclic ether, particularly tetrahydrofuran or dioxane, an hydrocarbon, particularly toluene, an alcohol, particularly methanol or ethanol, a dipolar aprotic solvent, particularly acetonitrile, pyridine or dimethylformamide, or any other suitable reaction medium. Naturally, mixtures of at least two of the classes of the mentioned compounds or at least two compounds of one class can also be used.

The most suitable reaction temperatures range between 0°C and the boiling temperature of the reaction medium, and the reaction times are preferably comprised between 1 and 24 hours.

Preferably, the resulting sulfonamide derivative of general Formula (le) can be isolated by filtration, concentrating the filtrate under reduced pressure, adding water and, if necessary, adjusting the pH so that a solid which can be isolated by filtration is obtained; or it can be extracted with a water immiscible solvent, such as chloroform, and be purified by chromatography or recrystallization of a suitable solvent.

The pharmaceutically acceptable salts of the compounds of general formula (le), can be prepared by means of conventional methods known in the state of the art, preferably by reaction with a mineral acid, such as hydrochloric, hydrobromic, phosphoric, sulfuric, nitric acids, or with organic acids, such as citric, maleic, fumaric, tartaric acids, or their derivatives, p-toluenesulfonic, methanesulfonic, camphorsulfonic acids, etc., in a suitable solvent, such as methanol, ethanol, diethyl ether, ethyl acetate, acetonitrile or acetone, being obtained with the usual techniques for the precipitation or crystallization of the corresponding salts.

The preferred physiologically acceptable salts of the sulfonamide derivatives of general formula (le) are the addition salts of mineral acids, such as hydrochloric, hydrobromic, phosphoric, sulfuric, nitric acids, and of organic acids, such as citric, maleic, fumaric, tartaric acids, or their derivatives, p-toluenesulfonic, methanesulfonic, camphorsulfonic acids, etc.

The physiologically acceptable solvates, particularly hydrates, of the sulfonamide derivatives of general formula (Ie) or of the corresponding physiologically acceptable salts, can be prepared by methods known in the state of the art.

During some of the synthetic sequences described or in the preparation of the suitable reagents used, it may be necessary and/or desirable to protect sensitive or reactive groups in some of the molecules used. This can be carried out by means of the use of conventional protective groups such as those described in the literature [Protective groups in Organic Chemistry, ed. J.F.W. McOmie, Plenum Press, 1973; T.W. Greene & P.G.M. Wuts, Protective Groups in Organic Chemistry, John Wiley & Sons, 1991]. The protective groups can be removed in the suitable subsequent stage by methods known in the state of the art.

If the sulfonamide derivatives of general formula (le) are obtained in the form of a mixture of stereoisomers, particularly enantiomers or diastereomers, said mixtures can be separated by means of standard processes known in the state of the art, for example chromatographic methods or crystallization with chiral agents.

If one or more of the residues R^{2f}-R^{9f} are an alkyl radical, which is substituted by one or more substituents, unless otherwise defined, each one of the substituents can preferably be chosen from the group consisting of hydroxy, fluorine, chlorine, bromine and trifluoromethyl.

If R^{1f} is a saturated or unsaturated cycloaliphatic radical, optionally containing at least one heteroatom as a ring member, which is substituted by one or more substituents and/or if it comprises a saturated or unsaturated, mono- or bicyclic cycloaliphatic ring system, optionally containing at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl. The heteroatoms of the cycloaliphatic radical and/or of the mono- or bi- cyclic cycloaliphatic ring can, independently from one another, be chosen preferably from the group consisting of nitrogen, sulfur and oxygen, more preferably nitrogen as an heteroatom.

If R^{8f} and R^{9f} together with the nitrogen atom bridge form a saturated or unsaturated heterocyclic ring, which can contain at least one additional heteroatom as a ring member, which is substituted by one or more substituents and/or condensed with a saturated or unsaturated mono- or bi- cyclic cycloaliphatic ring system, which can contain at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl. If the heterocyclic ring contains one or more additional heteroatoms, and/or if one or both mono- or bicyclic rings contain one or more heteroatoms, these heteroatoms can, independently from one another, be preferably chosen from the group consisting of nitrogen, sulfur and oxygen, more preferably nitrogen as heteroatom.

If A^{f} is a mono or poly-cyclic aromatic ring system, substituted by one or more substituents, and which can be bonded by means of an optionally at least monosubstituted alkylene, alkenylene or alkynylene group, and/or can contain at least one heteroatom as a ring member, unless otherwise defined, each one of the substituents can be preferably chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, a phenyl radical, optionally at least monosubstituted, and heteroaryl of 5 or 6 members, preferably from the group consisting of halogen, linear or branched C₁-C₆ alkyl, phenyl optionally at least monosubstituted and heteroaryl of 5 or 6 members, more preferably from the group consisting of fluorine, chlorine, linear or branched C₁-C₆ alkyl, phenyl radical, optionally at least monosubstituted and heteroaryl of 5 or 6 members. If one or more of the rings of a mono or poly-cyclic aromatic ring system contains one or more heteroatoms, these heteroatoms - like the heteroatoms of a previously mentioned heteroaryl radical of 5 or 6 members - can be preferably chosen from the group consisting of nitrogen, sulfur and oxygen. If the previously mentioned phenyl radical is itself substituted by one or more substituents, each one of the substituents can be preferably chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and an NR^{12f}R^{13f} radical, wherein R^{12f} and R^{13f} identical or different, are hydrogen or linear or branched C₁-C₆ alkyl.

Also preferably the substituents for A^{f} may be selected from the group consisting of hydroxy, halogen, linear or branched C₁-C₆ alkyl, linear or branched C₁-C₆ alkoxy, -O-phenyl, linear or branched C₁-C₆ perfluoroalkyl, linear or branched C₁-C₆ perfluoroalkoxy, an optionally at least mono-substituted phenyl and 5- to 6-membered heteroaryl, more preferably from the group consisting of halogen, linear or branched C₁-C₆ alkyl, -O-phenyl, optionally at least mono-substituted phenyl and 5- to 6-membered heteroaryl, even more preferably from the group consisting of fluorine, chlorine, -O-phenyl, linear or branched C₁-C₆ alkyl, optionally at least mono-substituted phenyl and 5- to 6-membered heteroaryl.

If the previously mentioned alkylene, alkenylene or alkynylene group is substituted by one or more substituents, each of the substituents can be preferably chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy or a phenyl radical, optionally at least monosubstituted. If said phenyl radical is itself substituted by one or more substituents, each one of the substituents can be preferably chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and an NR^{12f}R^{13f} radical, wherein R^{12f} and R^{13f}, identical or different, are hydrogen or linear or branched C₁-C₆ alkyl.

Sulfonamide derivatives of general formula (If) are preferred, wherein R^{1f} is an-NR^{8f}R^{9f} radical or a saturated or unsaturated, optionally at least mono-substituted, optionally at least heteroatom as a ring member containing 5- or 6-membered cycloaliphatic radical, which may be condensed with a saturated or unsaturated, optionally at least mono-substituted, optionally at least one heteroatom as a ring member containing mono- or bicyclic cycloaliphatic ring system, whereby the rings of the ring system are 5- or 6-membered, preferably R¹ represents an -NR^{8f}R^{9f} radical or a radical chosen from the group consisting of 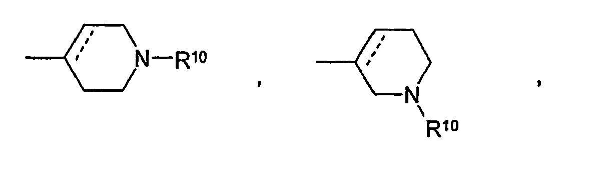 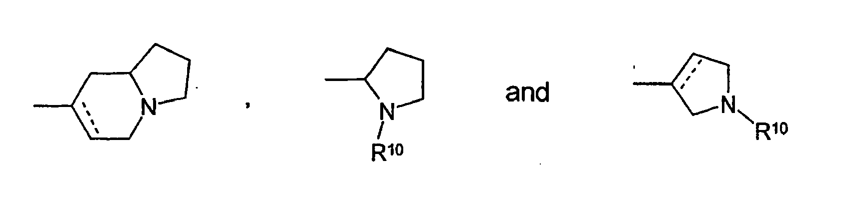 wherein, if present, the dotted line represents an optional chemical bond, and R¹⁰ represents hydrogen, a linear or branched C₁-C₆ alkyl radical or a benzyl radical, preferably hydrogen or a C₁-C₂ alkyl radical and R^{2f}-R^{9f}, A^{f} and nf are defined as above.

Sulfonamide derivatives of general formula (If) are also preferred, wherein R^{2f}, R^{3f}, R^{4f}, R^{5f} and R^{7f} are hydrogen, a linear or branched C₁-C₆ alkyl radical, a linear or branched C₂-C₆ alkenyl radical, or a linear or branched C₂-C₆ alkynyl radical, preferably hydrogen and R^{1f}, R^{6f}, R^{8f}-R^{9f}, A^{f} and nf are defined as above.

Sulfonamide derivatives of general formula (If) are also preferred, wherein R^{6f} is hydrogen, a linear or branched, optionally at least monosubstituted C₁-C₆ alkyl radical, preferably hydrogen or a linear or branched C₁-C₆ alkyl radical, more preferably hydrogen or an C₁-C₂ alkyl radical and R^{1f}-R^{5f}, R^{7f}-R^{9f}, A^{f} and nf are defined as above.

Furthermore, sulfonamide derivatives of general formula (If) are also preferred,
wherein R^{8f} and R^{9f}, identical or different, are hydrogen, a linear or branched, optionally at least mono-substituted C₁-C₆ alkyl radical, or R^{8f} and R^{9f}, together with the nitrogen atom bridge, form a saturated or unsaturated heterocyclic ring of 5 or 6 members, optionally at least monosubstituted, which can contain at least one additional heteroatom as a ring member, and/or can be condensed with a saturated or unsaturated, mono- or bi- cyclic cycloaliphatic ring system, optionally at least monosubstituted, which can optionally contain at least one heteroatom as a ring member, where the ring/rings is/are of 5, 6 or 7 members, and R^{1f}-R^{7f}, A^{f} and nf are defined as above.

Particularly preferred are sulfonamide derivatives of general formula (If),
wherein R^{8f} and R^{9f}, identical or different, are hydrogen or a linear or branched C₁-C₆ alkyl radical, preferably a linear or branched C₁-C₆ alkyl radical, or
R^{8f} and R^{9f} together with the nitrogen atom bridge form a radical chosen from the group consisting of 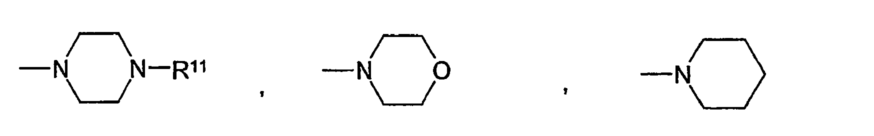 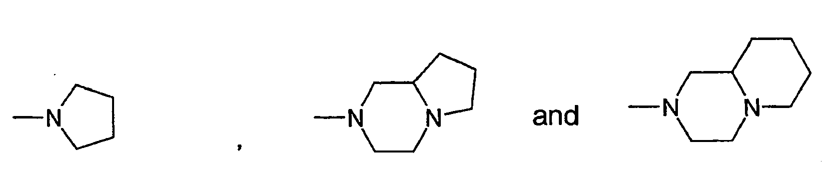 wherein R¹¹ is hydrogen, a linear or branched C₁-C₆ alkyl radical or a benzyl radical, preferably hydrogen, or a C₁-C₂ alkyl radical, and R^{1f}-R^{9f}, A^{f} and nf are defined as above.

Furthermore, sulfonamide derivatives of general formula (If) are preferred,
wherein A^{f} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member,
preferably A^{f} represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 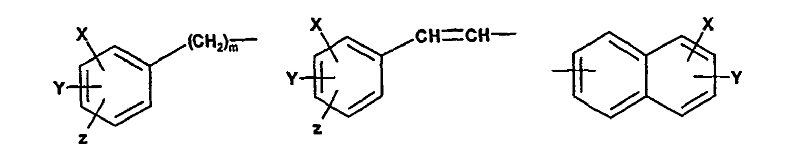 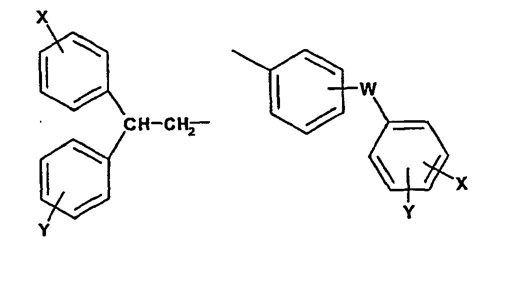 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl, W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
and R^{1f}-R^{11f} and nf are defined as above.

Furthermore, sulfonamide derivatives of general formula (If) are preferred, wherein A^{f} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member,
preferably A^{f} represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 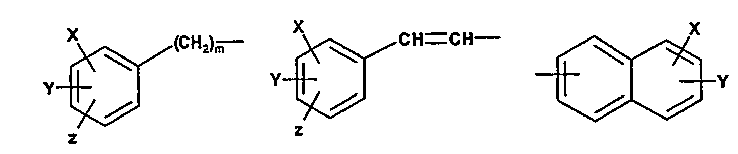 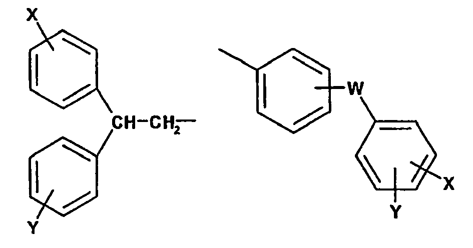 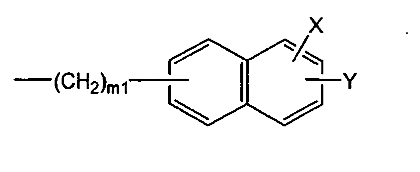 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R^{1f}-R^{11f} and nf are defined as above. Also preferred are compounds of general formula (If),
wherein
R^{1f} represents a -NR^{8f}R^{9f} radical,
R^{2f}, R^{3f}, R^{4f}, R^{5f} and R^{7f} each represent hydrogen,
R^{6f} represents hydrogen,
R^{8f} and R^{9f}, identical or different, each represent methyl, ethyl, n-propyl or n-propyl, more preferably methyl,
or
R^{8f} and R^{9f}, together with the bridging nitrogen atom form a 5- or 6-membered heterocyclic ring, more preferably form a pyrrolidine ring or a piperidine ring
and
A^{f} represents an aryl or heteroaryl radical selected from the group consisting of phenyl, naphthyl, benzo[b]thiophenyl and imidazo[2,1-b]thiazolyl which may be substituted by 1, 2 or 3 substituents selected from the group consisting of chlorine, methyl, phenyl and -O-phenyl and/or which may be bonded via a C₁₋₂ alkylene group,
and nf is 2,
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof.
The most preferred sulfonamide derivatives of general formula (If) may be selected from the group consisting of:
[1] N-[1-(2-Dimethylaminoethyl)-1H-indol-6-yl]-5-chloro-3-methylbenzo[b]thiophene-2-sulfonamide,
[2] N-[1-(2-Dimethylaminoethyl)-1H-indol-6-yl]-naphthalene-2-sulfonamide,
[3] N-[1-(2-Dimethylaminoethyl)-1H-indol-6-yl]-naphthalene-1-sulfonamide,
[4] N-[1-(2-Dimethylaminoethyl)-1H-indol-6-yl]-6-chloroimidazo[2,1-b]thiazole-5-sulfonamide,
[5] N-[1-(2-Dimethylaminoethyl)-1H-indol-6-yl]-4-phenylbenzenesulfonamide,
[6] N-[1-(2-Dimethylaminoethyl)-1H-indol-6-yl]-2-(naphthalene-1-yl)-ethanesulfonamide,
[7] N-[1-(2-Dimethylaminoethyl)-1H-indol-6-yl]-4-phenoxybenzenesulfonamide,
[8] N-[1-(2-Dimethylaminoethyl)-1H-indol-6-yl]-3,5-dichlorobenzenesulfonamide, and their corresponding salts and solvates.

The most preferred ones of the sulfonamide derivatives of general formula (If), may also be selected from the group consisting of:
[1] N-[1-(2-Dimethylaminoethyl)-1H-indol-6-yl]-5-chloro-3-methylbenzo[b]thiophene-2-sulfonamide,
[2] N-[1-(2-Dimethylaminoethyl)-1H-indol-6-yl]-naphthalene-2-sulfonamide,
[3] N-[1-(2-Dimethylaminoethyl)-1H-indol-6-yl]-naphthalene-1-sulfonamide,
[4] N-[1-(2-Dimethylaminoethyl)-1H-indol-6-yl]-6-chloroimidazo[2,1-b]thiazole-5-sulfonamide,
[5] N-[1-(2-Dimethylaminoethyl)-1H-indol-6-yl]-4-phenylbenzenesulfonamide,
[6] N-[1-(2-Dimethylaminoethyl)-1H-indol-6-yl]-2-(naphthalene-1-yl)-ethanesulfonamide,
[7] N-[1-(2-Dimethylaminoethyl)-1H-indol-6-yl]-4-phenoxybenzenesulfonamide,
[8] N-[1-(2-Dimethylaminoethyl)-1H-indol-6-yl]-3,5-dichlorobenzenesulfonamide,
[9] 5-Chloro-3-methyl-N-[1-[2-(pyrrolidin-1-yl)ethyl-1H-indol-6-yl]-benzo[b]thiophene-2-sulfonamide,
[10] N-(1-[2-(Pyrrolidin-1-yl)ethyl]-1H-indol-6-yl]-napthyl-2-sulfonamide,
[11] N-[1-[2-Pyrrolidin-1-yl]ethyl]-1H-indol-6-yl]-naphthalene-1-sulfonamide,
[12] 6-Chloro-N-[1-[2-(pyrrolidin-1-yl)ethyl]-1H-indol-6-yl]-imidazo[2,1-b]thiazole-5-sulfonamide,
[13] 4-Phenyl-N-(1-(2-(pyrrolidin-1-yl)ethyl-1H-indol-6-yl)-benzenesulfonamide
[14] 2-(Naphth-1-yl)-N-(1-(2-(pyrrolidin-1-yl)ethyl)-1H-indol-6-yl)-ethansulfonamide,
[15] 4-Phenoxy-N-(1-(2-(pyrrolidin-1-yl)ethyl)-1H-indol-6-yl)-benzenesulfonamide and
[16] 3,5-Dichloro-N-(1-(2-(pyrrolidin-1-yl)-1H-indol-6-yl)-benzenesulfonamide,
and their corresponding salts and solvates.

The present invention likewise refers to the physiologically acceptable salts of the compounds of general formula (If), particularly the addition salts of mineral acids, such as hydrochloric, hydrobromic, phosphoric, sulfuric, nitric acids, and with organic acids such as citric, maleic, fumaric, tartaric acids or their derivatives, p-toluenesulfonic, methanesulfonic, camphorsulfonic acids, etc.

Below, the residues R¹-R⁷, A and n in the general formulas (IIf) to (Vf) are R^{1f}-R^{7f} , A^{f} and nf.

The derivatives of general formula (If), wherein R^{1f}-R^{9t}, nf and A^{f} have the previously indicated meaning, may be preferably prepared in a way that:
At least one compound of general Formula (IIf), 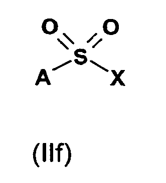
wherein A has the previously mentioned meaning in the general formula (If), and X is an acceptable leaving group, preferably an halogen atom, more preferably chlorine; reacts with at least one substituted 6-aminoindole of general formula (IIIf) 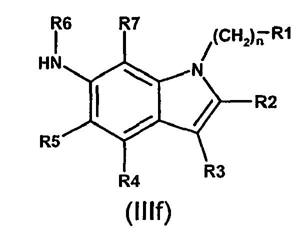 wherein R¹-R⁷ and n have the previously indicated meaning, or one of their suitable protected derivatives, and, if necessary, the protective groups are removed in order to obtain the corresponding sulfonamide derivative of formula (If), which can be purified and/or isolated by means of conventional methods known in the state of the art.

The reaction between the compounds of general Formula (IIf) and (IIIf) is usually carried out in the presence of an organic reaction medium such as dialkyl ether, particularly diethyl ether or a cyclic ether, particularly tetrahydrofuran or dioxane, an halogenated organic hydrocarbon, particularly methylene chloride or chloroform, an alcohol, particularly methanol or ethanol, a dipolar aprotic solvent, particularly acetonitrile, pyridine or dimethylformamide, or any other suitable reaction medium. Naturally, mixtures of at least two of the classes of the mentioned compounds or at least two compounds of one class can also be used.

The reaction is preferably carried out in the presence of a suitable base, for example, an inorganic base such as alkaline metal hydroxides and carbonates, or in the presence of an organic base, particularly triethylamine, N-ethyldiisopropylamine or pyridine.

The most suitable reaction temperatures range between 0°C and room temperature, that is, approximately 25°C, and the reaction time is preferably comprised between 5 minutes and 24 hours.

The resulting sulfonamide derivative of general Formula (If) can be purified and/or isolated according to conventional methods known in the state of the art.

Preferably, the sulfonamide derivatives of general Formula (If) can be isolated by evaporating the reaction medium, adding water and, if necessary, adjusting the pH so that a solid which can be isolated by filtration is obtained; or it can be extracted with a water immiscible solvent, such as chloroform, and be purified by chromatography or recrystallization of a suitable solvent.

The compounds of general formula (IIf) are commercially available, or they can be prepared according to standard methods known in the state of the art, for example by methods similar to those described in the literature [E.E.Gilbert, Synthesis, 1969, 1, 3]. The compounds of general formula (IIIf) can also be prepared according to standard methods known in the state of the art, for example by methods similar to those described in the literature [Ham, Peter, Gaster, Laramie Mary; King, Francis David; Duckworth, David Malcolm. Preparation of N-heteroaryl-4'-oxadiazolylbiphenylcarboxamides as 5HT1 D antagonists. WO 9532967 A1 19951207; Basanagoudar, L.D.; Siddappa, S. Cyanoethylation of nitroindoles. Journal of the Indian Chemical Society (1972), 49 (8), 811-13.; Chen, Guoqing; Adams, Jeffrey; Bemis, Jean; Booker, Shon; Cai, Guolin; Croghan, Michael; Dipietro, Lucian; Dominguez, Celia; Elbaum, Daniel; Germain, Julie; Geuns-Meyer, Stephanie; Handley, Michael; Huang, Qi; Kim, Joseph L.; Kim, Tae-seong; Kiselyov, Alexander; Ouyang, Xiaohu; Patel, Vinod F.; Smith, Leon M.; Stec, Markian; Tasker, Andrew; Xi, Ning; Xu, Shimin; Yuan, Chester Chenguang. Preparation of heterocyclylalkylamine derivatives as remedies for angiogenesis mediated diseases. WO 0266470 A1 20020829. European Journal of Medicinal Chemistry, 23 (4), 373-7; 1988]. One of them consists of nitro group reduction of derivatives of general formula (IVf) by methods known in the art, as for example YAMASHKIN, S.A.; YUROVSKAYA, M.A.; Chem Heterocycl Compd (N.Y.), 1999, 35 (12), 1426-1432. BOOTHROYD, S.R.; KERR, M.A.; Tetrahedron Lett, 1995, 36 (14), 2411-2414. MACOR, J.E.; POST, R.; RYAN, K.; Synth Common, 1993, 23 (1), 65-72, 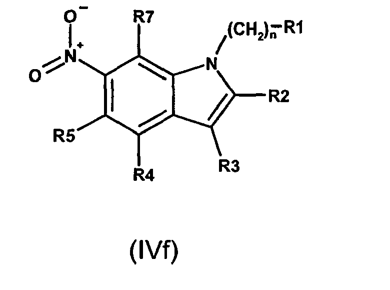 wherein R¹-R⁷ and n have the previously indicated meaning, or one of their suitably protected derivatives, and, if necessary, the protective groups are removed in order to obtain the corresponding amine of general formula (IIIf), which can be purified and/or isolated by means of conventional methods known in the state of the art.

The compounds of general formula (IVf) can also be prepared according to standard methods known in the state of the art, for example by methods similar to those described in the European Journal of Medicinal Chemistry, 23 (4), 373-7; 1988; Farmaco, 51 (1), 75-8; 1996; Heterocycles, 55 (6), 1151-1159; 2001; Ham, Peter; Gaster, Laramie Mary; King, Francis David; Duckworth, David Malcolm. Preparation of N-heteroaryl-4'-oxadiazolylbiphenylcarboxamides as 5HT1 D antagonists, WO 9532967 A1 19951207.

One of them consists in the alkylation of nitro derivatives of general formula (IVf) by methods known in the art: MACCHIA, M.; MANERA, C.; NENCETTI, S.; ROSSELLO, A.; BROCCALI, G.; LIMONTA, D.; Farmaco, Ed Sci [FRPSAX] 1996, 51 (1), 75-78. BHAGWAT, S. S.; GUDE, C.; Tetrahedron Lett, 1994, 35 (12), 1847-1850. BRATTON, L.D.; ROTH, B.D.; TRIVEDI, B.K.; UNANGST, P.C.; J Heterocycl Chem, 2000, 37 (5), 1103-1108, 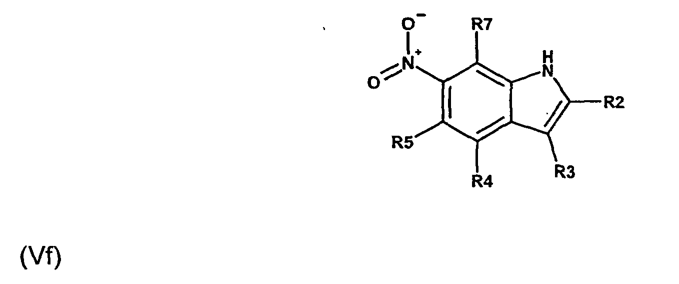 wherein R²-R⁷ and n have the previously mentioned meaning, or one of their suitably protected derivatives, and, if necessary, the protective groups are removed in order to obtain the corresponding amine of general formula (IIIf), which can be purified and/or isolated by means of conventional methods known in the state of the art.

The compounds of general formula (Vf) are commercially available or can also be prepared according to standard methods known in the state of the art, as for example OTTONI, O.; CRUZ, R.; KRAMMER, N.H.; Tetrahedron Lett [TELEAY] 1999, 40 (6), 1117-1120. VOROB'EVA, S.L.; BUYANOV, V.N.; SUVOROV, N.N.; Khim Geterosikl Soedin [KGSSAQ] 1991, (5), 636-637. KATRITZKY, A.R.; RACHWAL, S.; BAYYUK, S.; Org Prep Proceed Int [OPPIAK] 1991, 23 (3), 357-363. MOSKALEV, N.; MAKOSZA, M.; Heterocycles [HTCYAM] 2000, 52 (2), 533-536.

The sulfonamide derivatives of general formula (If), wherein R_{1f}, nf and A^{f} have the previously indicated meaning and R^{6f} is an alkyl radical, preferably a linear or branched C₁-C₆ alkyl radical, optionally at least monosubstituted, they can also be prepared by alkylation of a sulfonamide derivative of general Formula (If), wherein R^{1t}-R^{5f}, R^{7f}, nf and A^{f} have the previously indicated meaning, and R^{6f} is an hydrogen atom, with an alkyl halogenide or dialkyl sulfate.

The alkylation reaction is carried out preferably in the presence of a suitable base, such as alkaline metal hydroxides and carbonates, metal hydrides, alkoxides such as sodium metoxide or potassium tert-butoxide, organometallic compounds such as butyllithium or tert-butyllithium, in the presence of an organic reaction medium such as dialkyl ether, particularly diethyl ether, or a cyclic ether, particularly tetrahydrofuran or dioxane, an hydrocarbon, particularly toluene, an alcohol, particularly methanol or ethanol, a dipolar aprotic solvent, particularly acetonitrile, pyridine or dimethylformamide, or any other suitable reaction medium. Naturally, mixtures of at least two of the classes of the mentioned compounds or at least two compounds of one class can also be used.

The most suitable reaction temperatures range between 0°C and the boiling temperature of the reaction medium, and the reaction times are preferably comprised between 1 and 24 hours.

Preferably, the resulting sulfonamide derivative of general formula (If) can be isolated by filtration, concentrating the filtrate under reduced pressure, adding water and, if necessary, adjusting the pH so that a solid which can be isolated by filtration is obtained; or it can be extracted with a water immiscible solvent, such as chloroform, and be purified by chromatography or recrystallization of a suitable solvent.

The pharmaceutically acceptable salts of the compounds of general formula (If), can be prepared by means of conventional methods known in the state of the art, preferably by reaction with a mineral acid, such as hydrochloric, hydrobromic, phosphoric, sulfuric, nitric acids, or with organic acids, such as citric, maleic, fumaric, tartaric acids, or their derivatives, p-toluenesulfonic, methanesulfonic, camphorsulfonic acids, etc., in a suitable solvent, such as methanol, ethanol, diethyl ether, ethyl acetate, acetonitrile or acetone, being obtained with the usual techniques for the precipitation or crystallization of the corresponding salts.

The preferred physiologically acceptable salts of the sulfonamide derivatives of general formula (If) are the addition salts of mineral acids, such as hydrochloric, hydrobromic, phosphoric, sulfuric, nitric acids, and of organic acids, such as citric, maleic, fumaric, tartaric acids, or their derivatives, p-toluenesulfonic, methanesulfonic, camphorsulfonic acids, etc.

The physiologically acceptable solvates, particularly hydrates, of the sulfonamide derivatives of general formula (If) or of the corresponding physiologically acceptable salts, can be prepared by methods known in the state of the art.

During some of the synthetic sequences described or in the preparation of the suitable reagents used, it may be necessary and/or desirable to protect sensitive or reactive groups in some of the molecules used. This can be carried out by means of the use of conventional protective groups such as those described in the literature [Protective groups in Organic Chemistry, ed. J.F.W. McOmie, Plenum Press, 1973; T.W. Greene & P.G.M. Wuts, Protective Groups in Organic Chemistry, John Wiley & Sons, 1991]. The protective groups can be removed in the suitable subsequent stage by methods known in the state of the art.

If the sulfonamide derivatives of general formula (If) are obtained in the form of a mixture of stereoisomers, particularly enantiomers or diastereomers, said mixtures can be separated by means of standard processes known in the state of the art, for example chromatographic methods or crystallization with chiral agents.

If one or more of the substituents R^{2g}-R^{9g} represent an alkyl radical which is substituted by one or more substituents, unless otherwise defined, each one of the substituents can preferably be chosen from the group consisting of hydroxy, fluorine, chlorine, bromine and trifluoromethyl.

If R^{1g} is a saturated or unsaturated cycloaliphatic radical, optionally containing at least one heteroatom as a ring member, which is substituted by one or more substituents and/or if it comprises a saturated or unsaturated, mono- or bicyclic cycloaliphatic ring system, optionally containing at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl. The heteroatoms of the cycloaliphatic radical and/or of the mono- or bi- cyclic cycloaliphatic ring can, independently from one another, be chosen preferably from the group consisting of nitrogen, sulfur and oxygen, more preferably nitrogen as an heteroatom.

If R^{8g} and R^{9g} together with the nitrogen atom bridge form a saturated or unsaturated heterocyclic ring, which can contain at least one additional heteroatom as a ring member, which is substituted by one or more substituents and/or condensed with a saturated or unsaturated mono- or bi- cyclic cycloaliphatic ring system, which can contain at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, preferably from the group consisting of linear or branched C₁-C₆ alkyl and benzyl. If the heterocyclic ring contains one or more additional heteroatoms, and/or if one or both mono- or bicyclic rings contain one or more heteroatoms, these heteroatoms can, independently from one another, be preferably chosen from the group consisting of nitrogen, sulfur and oxygen, more preferably nitrogen as heteroatom.

If A^{g} is a mono or poly-cyclic aromatic ring system, substituted by one or more substituents, and which can be bonded by means of an optionally at least monosubstituted alkylene, alkenylene or alkynylene group, and/or can contain at least one heteroatom as a ring member, unless otherwise defined, each one of the substituents can be preferably chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, a phenyl radical, optionally at least monosubstituted, and heteroaryl of 5 or 6 members, more preferably from the group consisting of halogen, linear or branched C₁-C₆ alkyl, phenyl optionally at least monosubstituted and heteroaryl of 5 or 6 members, much more preferably from the group consisting of fluorine, chlorine, linear or branched C₁-C₆ alkyl, phenyl radical, optionally at least monosubstituted and heteroaryl of 5 or 6 members. If one or more of the rings of a mono or poly-cyclic aromatic ring system contains one or more heteroatoms, these heteroatoms - like the heteroatoms of a previously mentioned heteroaryl radical of 5 or 6 members - can be preferably chosen from the group consisting of nitrogen, sulfur and oxygen. If the previously mentioned phenyl radical is itself substituted by one or more substituents, each one of the substituents can be preferably chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and an NR^{12g}R^{13g} radical, wherein R^{12g} and R^{13g}, identical or different, are hydrogen or linear or branched C₁-C₆ alkyl.
The substituents for A^{g} may also preferably be selected from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, an optionally at least mono-substituted phenyl, -O-phenyl and 5- to 6-membered heteroaryl, more preferably from the group consisting of halogen, linear or branched C₁-C₆ alkyl, optionally at least mono-substituted phenyl, -O-phenyl and 5- to 6-membered heteroaryl, even more preferably from the group consisting of fluorine, chlorine, linear or branched C₁-C₆ alkyl, optionally at least mono-substituted phenyl, -O-phenyl, and 5- to 6-membered heteroaryl.

If the previously mentioned alkylene, alkenylene or alkynylene group is substituted by one or more substituents, each of the substituents can be preferably chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy or a phenyl radical, optionally at least monosubstituted. If said phenyl radical is itself substituted by one or more substituents, each one of the substituents can be preferably chosen from the group consisting of fluorine, chlorine, bromine, linear or branched C₁-C₆ alkyl, linear or branched C₁-C₆ alkoxy, linear or branched C₁-C₆ alkylthio, trifluoromethyl radical, cyano radical and an NR^{12g}R^{13g} radical, wherein R^{12g} and R^{13g}, identical or different, are hydrogen or linear or branched C₁-C₆ alkyl.

Sulfonamide derivatives of general formula (Ig) are preferred, wherein R^{1g} is an -NR^{8g}R^{9g} radical or a saturated or unsaturated, optionally at least mono-substituted 5- or 6-membered cycloaliphatic radical, which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member, whereby the rings of the ring system are 5- or 6-membered, preferably an -NR^{8g}F^{9g} radical or a radical chosen from the group consisting of 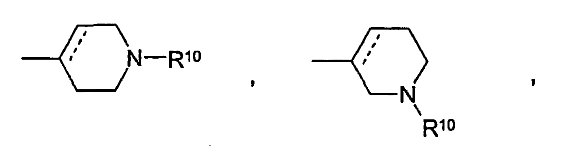 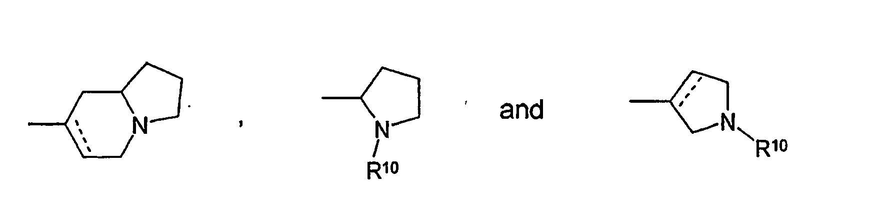 where, if present, the dotted line is an optional chemical bond, and R¹⁰ is hydrogen, a linear or branched C₁-C₆ alkyl radical or a benzyl radical, preferably hydrogen or a C₁-C₂ alkyl radical, and R^{2g}-R^{9g}, A^{g} and ng are defined as above.

Sulfonamide derivatives of general formula (Ig) are also preferred, wherein R^{2g}, R^{3g}, R^{4g}, R^{5g} and R^{6g}, are hydrogen or a linear or branched C₁-C₆ alkyl radical, a linear or branched C₂-C₆ alkenyl radical, a linear or branched C₂-C₆ alkynyl radical, preferably hydrogen and R^{1g}, R^{7g}-R^{9g}, A^{g} and ng are defined as above.

The use of sulfonamide derivatives of general formula (Ig) is also preferred, wherein R^{7g}, is hydrogen or a linear or branched, optionally at least monosubstituted C₁-C₆ alkyl radical, preferably hydrogen or a linear or branched C₁-C₆ alkyl radical, more preferably hydrogen or an C₁-C₂ alkyl radical and R^{1g}-R^{6g}, R^{8g}, R^{9g}, A^{g} and ng are defined as above.

Furthermore, sulfonamide derivatives of general formula (Ig) are also preferred,
wherein R^{8g} and R^{9g}, identical or different, are hydrogen or a linear or branched, optionally at least monosubstituted C₁-C₆ alkyl radical, or R^{8g} and R^{9g}, together with the nitrogen atom bridge, form a saturated or unsaturated heterocyclic ring of 5 or 6 members, optionally at least monosubstituted, which can contain at least one additional heteroatom as a ring member, and/or can be condensed with a saturated or unsaturated, mono- or bi- cyclic cycloaliphatic ring system, optionally at least monosubstituted, which can optionally contain at least one heteroatom as a ring member, where the ring/rings is/are of 5, 6 or 7 members, and R^{1g}-R^{7g}, A^{g} and ng are defined as above.

Particularly preferred is the use of sulfonamide derivatives of general formula (Ig), wherein R^{8g} and R^{9g}, identical or different, are hydrogen or a linear or branched C₁-C₆ alkyl radical, preferably a linear or branched C₁-C₆ alkyl radical, or R^{8g} and R^{9g} together with the nitrogen atom bridge form a radical chosen from the group consisting of 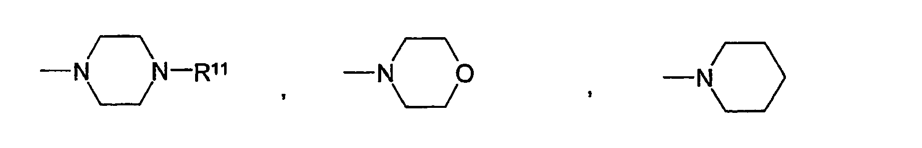 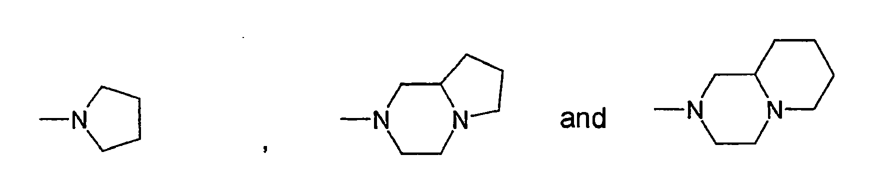 wherein R¹¹, if it is present, is hydrogen, a linear or branched C₁-C₆ alkyl radical or a benzyl radical, preferably hydrogen, or a C₁-C₂ alkyl radical, and R^{1g}-R^{9g}, A^{g} and ng are defined as above.

Furthermore, sulfonamide derivatives of general formula (Ig) are preferred, wherein A^{g} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member,
preferably A^{g} represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 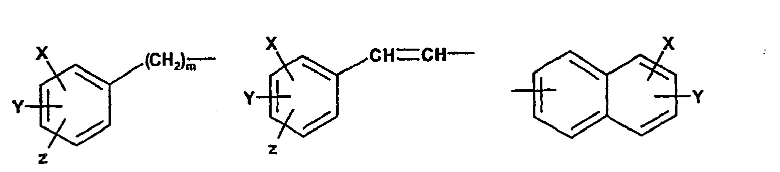 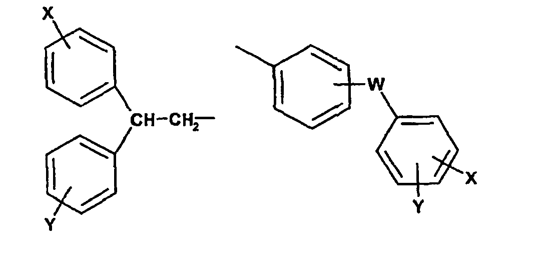 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl,
W represents a single chemical bond between the two rings, a CH₂, O, S group or a NMR¹⁴ radical,
wherein R¹⁴ is hydrogen or a linear or branched C₁-C₆ alkyl,
m is 0, 1, 2, 3 or 4 and
and R^{1g}-R^{11g} and ng are defined as above.

Furthermore, sulfonamide derivatives of general formula (Ig) are preferred, wherein A^{g} represents an optionally at least mono-substituted mono- or polycyclic aromatic ring system, wherein the ring(s) is/are 5- or 6-membered, which may be bonded via an optionally at least mono-substituted C₁-C₆ alkylene group, an optionally at least mono-substituted C₂-C₆ alkenylene group or an optionally at least mono-substituted C₂-C₆ alkynylene group and/or wherein the ring(s) may contain at least one heteroatom as a ring member,
preferably A^{g} represents an optionally at least mono-substituted mono- or polycyclic aromatic ring system, wherein the ring(s) is/are 5- or 6-membered and wherein one or more of the rings contain at least one heteroatom,
or a radical chosen from the group consisting of 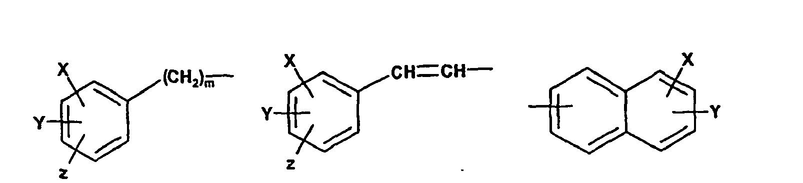 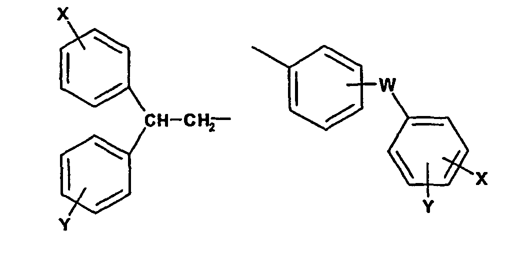 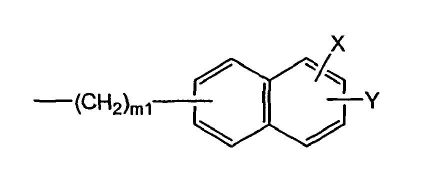 wherein X, Y, Z, independently from one another, each represent a radical selected from the group consisting of hydrogen, fluorine, chlorine, bromine, linear or branched C₁-C₆ alkyl, linear or branched C₁-C₆ alkoxy, linear or branched C₁-C₆ alkylthio, a trifluoromethyl radical, a cyano radical and a - NR¹²R¹³ radical,
wherein R¹² and R¹³, identical or different, each represent hydrogen or linear or branched C₁-C₆ alkyl,
W represents a single chemical bond between the two rings, a CH₂, O, S group or a NR¹⁴ radical,
wherein R¹⁴ is hydrogen or a linear or branched C₁-C₆ alkyl,
m is 0, 1, 2, 3 or 4 and
m1 is 1 or 2, preferably 2, and R^{1g} -R^{9g} andg n are defined as above.

Also preferred are compounds of general formula (Ig),
wherein
R^{1g} is a -NR^{8g}R^{9g} radical,
R^{2g}, R^{3g}, R^{4g}, R^{5g} and R^{6g} each represent hydrogen,
R^{7g} represents hydrogen,
R^{8g} and R^{9g}, identical or different, each represent methyl, ethyl, n-propyl or iso-propyl, more preferably methyl,
or
R^{8g} and R^{9g} together with the bridging nitrogen atom form a 5- or 6-membered heterocyclic ring, more preferably form a pyrrolidine or piperidine ring,
A^{g} represents an aryl or heteroaryl radical selected from the group consisting of phenyl, naphthyl, benzo[b]thiophenyl and imidazo[2,1-b]thiazolyl which may be substituted by 1, 2 or 3 substituents selected from the group consisting of chlorine, methyl and phenyl and/or which may be bonded via a C₁-₂ alkylene group,
and
ng is 2;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

Those most preferred ones are the sulfonamide derivatives of general formula Ig may be selected from the group consisting of:
[1] N-[1-(2-dimethylaminoethyl)-1H-indole-7-yl]-naphtalene-1-sulfonamide,
[2] N-[1-(2-dimethylaminoethyl)-1H-indole-7-yl]-5-chloro-3-methylbenzo[b]thiophene-2-sulfonamide,
[3] N-[1-(2-dimethylaminoethyl)-1H-indole-7-yl]-4-phenylbenzenesulfonamide and
[4] N-[1-(2-dimethylaminoethyl)-1H-indole-7-yl]-6-chloroimidazo[2,1-b]thiazole-5-sulfonamide
and their corresponding salts and solvates.

Also most preferably the sulfonamide derivatives of general formula (Ig) may be selected from the group consisting of:
[1] N-[1-(2-dimethylaminoethyl)-1H-indole-7-yl]-naphtalene-1-sulfonamide,
[2] N-[1-(2-dimethylaminoethyl)-1H-indole-7-yl]-5-chloro-3-methylbenzo[b]thiophene-2-sulfonamide,
[3] N-[1-(2-dimethylaminoethyl)-1H-indole-7-yl]-4-phenylbenzenesulfonamide and
[4] N-[1-(2-dimethylaminoethyl)-1H-indole-7-yl]-6-chloroimidazo[2,1-b]thiazole-5-sulfonamide
[5] 5-chloro-3-methyl-N-(1-(2-(pyrrolidin-1-yl)ethyl)-1H-indol-7-yl)-benzo[b]thiophen-2-sulfonamide,
[6] N-(1-(2-(pyrrolidin-1-yl)ethyl)-1H-indol-7-yl)naphthalene-1-sulfonamide,
[7] 6-chloro-N-(1-(2-(pyrroldin-1-yl)ethyl)-1H-indol-7-yl)imidazo[2,1-b]thiazole-5-sulfonamide and
[8] 2-(naphth-1-yl)-N-(1-(2-(pyrrolidin-1-yl)ethyl)-1H-indol-7-yl)ethansulfonamide and their corresponding salts and solvates.

The present invention likewise refers to the physiologically acceptable salts of the compounds of general formula (Ig), particularly the addition salts of mineral acids, such as hydrochloric, hydrobromic, phosphoric, sulfuric, nitric acids, and with organic acids such as citric, maleic, fumaric, tartaric acids or their derivatives, p-toluenesulfonic, methanesulfonic, camphorsulfonic acids, etc.

Below, the residues R¹-R⁷, A and n in the general formulas (IIg) and (IIIg) are R^{1g}-R^{7g}, A^{g} and ng.

The derivatives of general formula (Ig), wherein R^{1g}-R^{9g}, ng and A^{g} have the previously indicated meaning, may be preferably prepared in a way that:
At least one compound of general Formula (IIg), 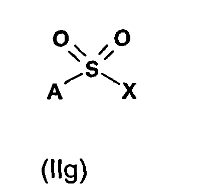
wherein A has the previously mentioned meaning in the general formula (Ig), and X is an acceptable leaving group, preferably an halogen atom, more preferably chlorine; reacts with at least one substituted 7-aminoindole of general formula (IIIg) 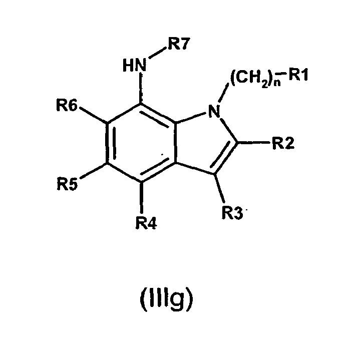 wherein R¹-R⁷ and n have the previously indicated meaning, or one of their suitable protected derivatives, and, if necessary, the protective groups are removed in order to obtain the corresponding sulfonamide derivative of general formula (Ig), which can be purified and/or isolated by means of conventional methods known in the state of the art.

The reaction between the compounds of general formula (IIg) and (IIIg) is usually carried out in the presence of an organic reaction medium such as dialkyl ether, particularly diethyl ether or a cyclic ether, particularly tetrahydrofuran or dioxane, an halogenated organic hydrocarbon, particularly methylene chloride or chloroform, an alcohol, particularly methanol or ethanol, a dipolar aprotic solvent, particularly acetonitrile, pyridine or dimethylformamide, or any other suitable reaction medium. Naturally, mixtures of at least two of the classes of the mentioned compounds or at least two compounds of one class can also be used.

The reaction is preferably carried out in the presence of a suitable base, for example, an inorganic base such as alkaline metal hydroxides and carbonates, or in the presence of an organic base, particularly triethylamine or pyridine.

The most suitable reaction temperatures range between 0°C and room temperature, that is, approximately 25°C, and the reaction time is preferably comprised between 5 minutes and 24 hours.

The resulting sulfonamide derivative of general Formula (Ig) can be purified and/or isolated according to conventional methods known in the state of the art.

Preferably, the sulfonamide derivatives of general Formula (Ig) can be isolated by evaporating the reaction medium, adding water and, if necessary, adjusting the pH so that a solid which can be isolated by filtration is obtained; or it can be extracted with a water immiscible solvent, such as chloroform, and be purified by chromatography or recrystallization of a suitable solvent.

The compounds of general formula (IIg) are commercially available, or they can be prepared according to standard methods known in the state of the art, for example by methods similar to those described in the literature [E.E.Gilbert, Synthesis, 1969, 1, 3]. The compounds of general formula (IIIg) can also be prepared according to standard methods known in the state of the art, for example by methods similar to those described in: [Abou-Gharbia, Magid; Patel, Usha; Tokolics, Joseph; Freed, Meier. European Journal of Medicinal Chemistry (1988), 23(4), 373-7].

The sulfonamide derivatives of general Formula (Ig), wherein R^{1g}, ng and A^{g} have the previously indicated meaning and R^{7g} is an alkyl radical, preferably a linear or branched C₁-C₆ alkyl radical, optionally at least monosubstituted, they can also be prepared by alkylation of a sulfonamide derivative of general formula (Ig), wherein R^{1g}-R^{6g}, ng and A^{g} have the previously indicated meaning, and R^{7g} is an hydrogen atom, with an alkyl halogenide or dialkyl sulfate.

The alkylation reaction is carried out preferably in the presence of a suitable base, such as alkaline metal hydroxides and carbonates, metal hydrides, alkoxides such as sodium metoxide or potassium tert-butoxide, organometallic compounds such as butyllithium or tert-butyllithium, in the presence of an organic reaction medium such as dialkyl ether, particularly diethyl ether, or a cyclic ether, particularly tetrahydrofuran or dioxane, an hydrocarbon, particularly toluene, an alcohol, particularly methanol or ethanol, a dipolar aprotic solvent, particularly acetonitrile, pyridine or dimethylformamide, or any other suitable reaction medium. Naturally, mixtures of at least two of the classes of the mentioned compounds or at least two compounds of one class can also be used.

The most suitable reaction temperatures range between 0°C and the boiling temperature of the reaction medium, and the reaction times are preferably comprised between 1 and 24 hours.

Preferably, the resulting sulfonamide derivative of general formula (Ig) can be isolated by filtration, concentrating the filtrate under reduced pressure, adding water and, if necessary, adjusting the pH so that a solid which can be isolated by filtration is obtained; or it can be extracted with a water immiscible solvent, such as chloroform, and be purified by chromatography or recrystallization of a suitable solvent.

The pharmaceutically acceptable salts of the compounds of general formula (Ig), can be prepared by means of conventional methods known in the state of the art, preferably by reaction with a mineral acid, such as hydrochloric, hydrobromic, phosphoric, sulfuric, nitric acids, or with organic acids, such as citric, maleic, fumaric, tartaric acids, or their derivatives, p-toluenesulfonic, methanesulfonic, camphorsulfonic acids, etc., in a suitable solvent, such as methanol, ethanol, diethyl ether, ethyl acetate, acetonitrile or acetone, being obtained with the usual techniques for the precipitation or crystallization of the corresponding salts.

The preferred physiologically acceptable salts of the sulfonamide derivatives of general formula (Ig) are the addition salts of mineral acids, such as hydrochloric, hydrobromic, phosphoric, sulfuric, nitric acids, and of organic acids, such as citric, maleic, fumaric, tartaric acids, or their derivatives, p-toluenesulfonic, methanesulfonic, camphorsulfonic acids, etc.

The physiologically acceptable solvates, particularly hydrates, of the sulfonamide derivatives of general formula (Ig) or of the salts, preferably the corresponding, physiologically acceptable salts, can be prepared by methods known in the state of the art.

During some of the synthetic sequences described or in the preparation of the suitable reagents used, it may be necessary and/or desirable to protect sensitive or reactive groups in some of the molecules used. This can be carried out by means of the use of conventional protective groups such as those described in the literature [Protective groups in Organic Chemistry, ed. J.F.W. McOmie, Plenum Press, 1973; T.W. Greene & P.G.M. Wuts, Protective Groups in Organic Chemistry, John Wiley & Sons, 1991]. The protective groups can be removed in the suitable subsequent stage by methods known in the state of the art.

If the sulfonamide derivatives of general formula (Ig) are obtained in the form of a mixture of stereoisomers, particularly enantiomers or diastereomers, said mixtures can be separated by means of standard processes known in the state of the art, for example chromatographic methods or crystallization with chiral agents.

If one or more of the R^{2h}-R^{8h} moieties, A and B, represent an alkyl radical, an alkenyl radical or an alkynyl radical, which is substituted by one or more substituents, each one of the substituents can preferably be chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, or a phenyl radical optionally at least monosubstituted. If said phenyl radical is the same one substituted by one or more substituents, each one of the substituents can preferably be chosen from the group consisting of fluorine, chlorine, bromine, a linear or branched C₁-C₆ alkyl, a linear or branched C₁-C₆ alkoxy, a linear or branched C₁-C₆ alkylthio, a trifluoromethyl radical, a cyano radical and an NR^{11h}R^{12h} radical, where R^{11h} and R^{12h} identical or different, are defined as R^{7h} and R^{8h}.

If R^{1h} is a saturated or unsaturated cycloaliphatic radical, optionally containing at least one heteroatom as a ring member, which is substituted by one or more substituents and/or if it comprises a saturated or unsaturated, mono- or bicyclic cycloaliphatic ring system, optionally containing at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branched C₁-C₆ perfluoroalkyl, linear or branched C₁-C₆ perfluoroalkoxy and benzyl, more preferably from the group consisting of linear or branched C₁-C₆ alkyl and benzyl. The heteroatoms of the cycloaliphatic radical and/or of the mono- or bicyclic cycloaliphatic ring can, independently from one another, be chosen preferably from the group consisting of nitrogen, sulfur and oxygen, more preferably nitrogen as a heteroatom.

If R^{7h} and R^{8h} together with the nitrogen atom to which they are bonded form a saturated or unsaturated heterocyclic ring, which can optionally contain at least one additional heteroatom as a ring member, which is substituted by one or more substituents and/or condensed with a saturated or unsaturated mono- or bi- cyclic cycloaliphatic ring system, which can contain at least one heteroatom as a ring member, which is substituted by one or more substituents, unless otherwise defined, each one of the substituents can preferably be chosen from the group consisting of hydroxy, fluorine, chlorine, bromine, linear or branched C₁-C₆ alkyl, linear or branched C₁-C₆ alkoxy, linear or branchedC₁-C₆ perfluoroalkyl, linear or branched C₁-C₆ perfluoroalkoxy and benzyl, preferably from the group consisting of linear or branched C₁-C₆ alkyl and benzyl. If the heterocyclic ring contains one or more additional heteroatoms, and/or if one or both mono- or bi- cyclic rings contains one or more heteroatoms, these heteroatoms can, independently from one another, be preferably chosen from the group consisting of nitrogen, sulfur and oxygen, more preferably nitrogen as a heteroatom.

If A^{h} is an alkyl radical, an alkenyl radical or an alkynyl radical, which is substituted by one or more substituents, each one of the substituents can preferably be chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy or a phenyl radical, optionally at least monosubstituted. If said phenyl radical is the same one substituted by one or more substituents, each one of the substituents can preferably be chosen from the group consisting of fluorine, chlorine, bromine, linear or branched C₁-C₆ alkyl, linear or branched C₁-C₆ alkoxy, linear or branched C₁-C₆ alkylthio, a trifluoromethyl radical, a cyano radical and an N^{13h} R^{14h} radical, where R^{13h} and R^{14h}, identical or different, are defined as R^{7h} and R^{8h}.

If B^{h} is an an alkyl radical, an alkenyl radical or an alkynyl radical, which substituted by one or more substituents, each one of the substituents can preferably be chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy, or a phenyl radical optionally at least monosubstituted. If said phenyl radical is the same one substituted by one or more substituents, each one of the substituents can preferably be chosen from the group consisting of fluorine, chlorine, bromine, linear or branched C₁-C₆ alkyl, linear or branched C₁-C₆ alkoxy, linear or branched C₁-C₆ alkylthio, a trifluoromethyl radical, a cyano radical and an NR^{15h}R1^{6h} radical, where R^{15h} and R^{16h}, identical or different, are defined as R^{7h} and R^{8h}.

If A^{h} and B^{h} together with the carbon atom to which they are bonded form a saturated or unsaturated, but not aromatic, cycloalkyl ring, substituted by one or more substituents, each one of the substituents can preferably be chosen from the group consisting of hydroxy, halogen, linear or branched C₁-C₆ alkyl, linear or branched C₁-C₆ alkoxy, linear or branched C₁-C₆ perfluoroalkyl, linear or branched C₁-C₆ perfluoroalkoxy or a phenyl radical, optionally at least monosubstituted. If said phenyl radical is the one substituted by one or more substituents, each one of the substituents can preferably be chosen from the group consisting of fluorine, chlorine, bromine, linear or branched C₁-C₆ alkyl, linear or branched C₁-C₆ alkoxy, linear or branched C₁-C₆ alkylthio, a trifluoromethyl radical, a cyano radical and an NR^{17h}R^{18h} radical, where R^{17h} and R^{18h}, identical or different, are defined as R^{7h} and R^{8h}.

Sulfonamide derivatives of general formula (Ih) are preferred, where R¹ represents an NR^{7h}R^{8h} radical or a saturated or unsaturated, optionally at least mono-substituted, optionally at least one heteroatom as a ring member containing 5- or 6-membered cycloaliphatic radical, which may be condensed with a saturated or unsaturated, optionally at least mono-substituted, optionally at least one heteroatom as a ring member containing mono- or bicyclic cycloaliphatic ring system, whereby the rings of the ring system are 5- or 6-membered, preferably an -NR^{7h}R^{8h} radical or a radical chosen from the group consisting of 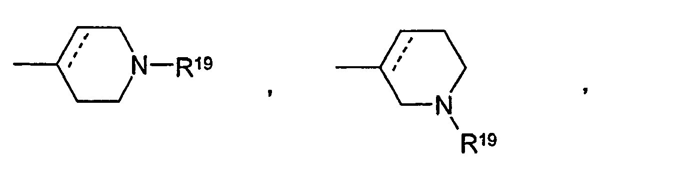 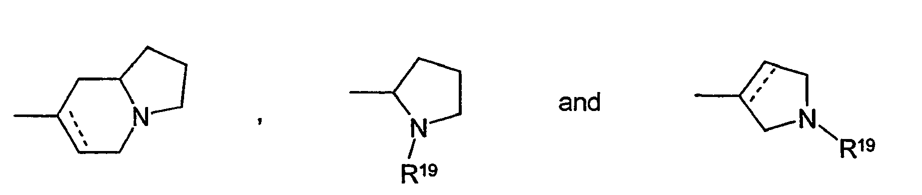 where, if present, the dotted line represents an optional chemical bond, and R¹⁹ is hydrogen, a linear or branched C₁-C₆ alkyl radical or a benzyl radical, preferably hydrogen or a C₁-C₂ alkyl radical, and R^{2h}-R^{6h} , A^{h}, B^{h} and nh are defined as above.

Furthermore, sulfonamide derivatives of general formula (Ih) are also preferred, where R^{7h} and R^{8h}, identical or different, are hydrogen, a optionally at least monosubstituted, linear or branched C₁₋₆ alkyl radical, a linear or branched optionally at least monosubstituted, C₂₋₆ alkenyl radical, or a linear or branched optionally at least monosubstituted, C₂₋₆ alkynyl, or

R^{7h} and R^{8h}, together with the nitrogen atom bridge, form a saturated or unsaturated heterocyclic ring of 5 or 6 members, optionally at least monosubstituted, which can contain at least one additional heteroatom as a ring member, and/or can be condensed with a saturated or unsaturated, mono- or bi- cyclic cycloaliphatic ring system, optionally at least monosubstituted, which can optionally contain at least one heteroatom as a ring member, where the ring/rings is/are of 5, 6 or 7 members, and R^{1h}-R^{6h}, A^{h}, B^{h} and nh are defined as above.

Particularly preferred are sulfonamide derivatives of general formula (Ih), where R^{7h} and R^{8h}, identical or different, are hydrogen or a linear or branched C₁-C₆ alkyl radical, preferably a linear or branched C₁-C₆ alkyl radical, or R^{7h} and R^{8h} together with the nitrogen atom bridge form a radical chosen from the group consisting of 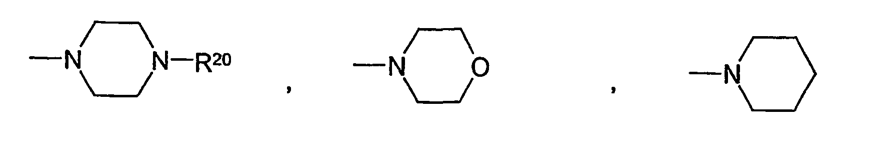 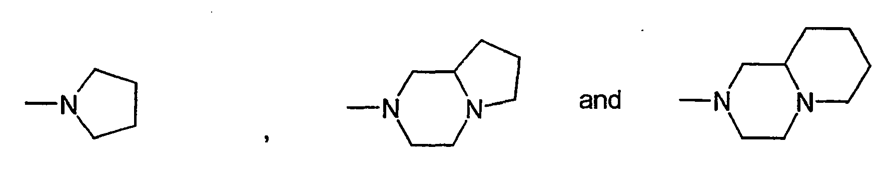 where R²⁰, if present, is hydrogen, a linear or branched C₁-C₆ alkyl radical or a benzyl radical, preferably hydrogen, or a C₁-C₂ alkyl radical, and R^{1h}-R^{6h}, A^{h}, B^{h} and nh are defined as above.

Furthermore, sulfonamide derivatives of general formula (Ih) are preferred, where A^{h} and B^{h}, identical or different, are a linear or branched C₁-C₆ alkyl radical, a linear or branched C₂-C₆ alkenyl radical or a linear or branched C₁-C₆ alkinyl radical, preferably a linear or branched C₁-C₆ alkyl radical radical, or
A^{h} and B^{h}, together with the carbon atom to which they are bonded, form a saturated or unsaturated, but not aromatic, cycloalkyl ring, optionally substituted by one or more substituents, preferably a C₃-C₈ cycloalkyl ring. Particularly preferably a cyclohexyl ring, and R^{1h}-R^{8h}, A^{h}, B^{h} and nh are defined as above.

Sulfonamide derivatives of general Formula (Ih) are also preferred, wherein R^{2h}, R^{3h} , R^{4h} , R^{5h} and R^{6h}, identical or different, independently from one another, are, hydrogen, halogen, cyano, nitro, C₁₋₆ alk(en/yn)yl, C₁₋₆-alkoxy, C₁₋₆-alkylthio, hydroxy, trifluoromethyl, C₃₋₈cycloalk(en)yl, C₁₋₆-alkylcarbonyl, phenylcarbonyl or a -NR^{9h}R^{10h} group, where where R^{9h} and R^{10h}, are defined as R^{7h} and R^{8h}.

Also preferred are compounds of general formula (Ih),
wherein
R^{1h} represents an unsaturated, optionally at least one nitrogen atom as a ring member containing 5- or 6-membered cycloaliphatic radical, which may be substituted by a methyl group and/or which may be condensed with a 5-membered cycloaliphatic ring, more preferably R¹ represents a moiety selected from the group consisting of 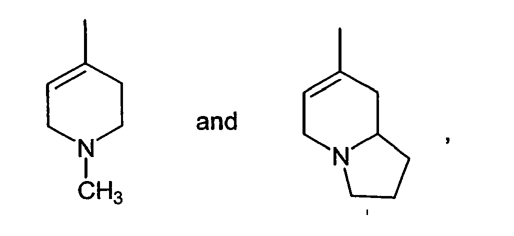 R^{2h}, R^{3h}, R^{4h} and R^{6h} each represent hydrogen,
R^{5h} represents H, fluorine, chlorine, nitro or a -NR^{9h}R^{10h} group,
R^{9h} and R^{10h} each represent hydrogen,
A^{h} and B^{h} together with the carbon atom to which they are bonded form a saturated or unsaturated, but not aromatic, C₃-C₈ cycloalkyl ring, more preferably form a cyclohexyl ring,
and
nh is 0;
optionally in form of one of their stereoisomers, preferably enantiomers or diastereomers, their racemate or in form of a mixture of at least two of their stereoisomers, preferably enantiomers or diastereomers, in any mixing ratio, or a salt thereof, preferably a corresponding physiologically acceptable salt thereof or a corresponding solvate thereof.

Those most preferred are the sulfonamide derivatives of general Formula (Ih), selected from the group consisting of:
[1] 1-Cyclohexanesulfonyl-3-(1-methyl-1,2,3,6-tetrahydropyridine-4-yl)-5-nitro-1H-indole,
[2] 5-Chloro-1-cyclohexanesulfonyl-3-(1-methyl-1,2,3,6-tetrahydropyridine-4-yl)-1H-indole,
[3] 5-Amino-1-cyclohexanesulfonyl-3-(1-methyl-1,2,3,6-tetrahydropyridine-4-yl)-1H-indole and
[4] 1-Cyclohexanesulfonyl-5-fluoro-3-(1,2,3,5,8,8a-hexahydro-indolizine-7-yl)-1 H-indole hydrochloride and their corresponding salts and solvates.

The present invention likewise refers to the physiologically acceptable salts of the compounds of general formula (Ih), particularly the addition salts of mineral acids, such as hydrochloric, hydrobromic, phosphoric, sulfuric, nitric acids, and of organic acids, such as citric, maleic, fumaric, tartaric acids, or their derivatives, *p*-toluenesulfonic, methanesulfonic, camphorsulfonic acids, etc.

Below, the residues R¹-R⁶, A, B and n in the general formulas (IIh) to (IVh) are R^{1h}-R^{6h} , A^{h}, B^{h} and nh.

The derivatives of general formula (Ih), wherein R^{1h}-R^{6h}, A^{h}, B^{h} and nh have the previously indicated meaning, may be preferably prepared in a way that:
At least one compound of general Formula (IIh), 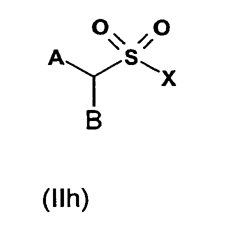
wherein A and B have the previously mentioned meaning in the general formula (Ih), and X is an acceptable leaving group, preferably an halogen atom, more preferably chlorine; reacts with at least one substituted indole of general formula (IIIh)

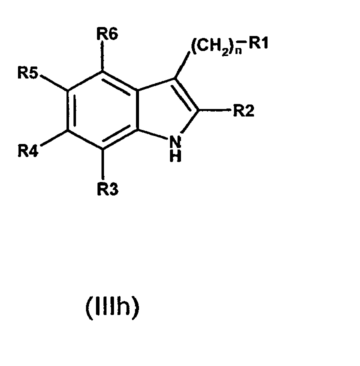

The reaction is preferably carried out in the presence of a suitable strong base, for example, lithium diisopropylamide, butyllithium, sodium hydride, or sodium bis(trimethylsilyl)amide in an inert solvent, such as tetrahydrofurane, hexane or dimethylformamide.

The most suitable reaction temperatures range between -100°C and room temperature, and the reaction time is preferably comprised between 5 minutes and 24 hours. The preferred conditions are sodium hydride in dimethylformamide at approximately 0°C.

The resulting sulfonamide derivative of general formula (Ih) can be purified and/or isolated according to conventional methods known in the state of the art.

Preferably, the sulfonamide derivatives of general formula (Ih) can be isolated by evaporating the reaction medium, adding water and, if necessary, adjusting the pH so that a solid which can be isolated by filtration is obtained; or it can be extracted with a water immiscible solvent, such as chloroform, and be purified by chromatography or recrystallization of a suitable solvent.

The compounds of general formula (IIh) are commercially available, or they can be prepared according to standard methods known in the state of the art, for example by methods similar to those described in the literature [KHANNA, V.; TAMILSELVAN, P.; KALRA, S.J.S.; IQBAL, J.; Tetrahedron 1994, 35 (32), 5935-5938; L.N. Aristarkhova et al., J. Org. Chem. USSR, 1970, 6, 2454-2458; E.E. Gilbert, Synthesis, 1969, 1,3]. The compounds of general Formula (IIIh) can also be prepared according to standard methods known in the state of the art, for example, methods similar to those described in the literature. Substituted aromatic 5-HT1f agonist, W09846570. Piperidine-indole compounds having 5-HT6 affinity, US 6,133,287.

The sulfonamide derivatives of general formula (Ih), wherein R^{2h}, R^{3h} , R^{4h} , R^{5h} or R^{6h} are an amino group by reduction of the nitro group of derivatives of general formula (IVh) by methods known in the art, for example BRATTON, L.D.; ROTH, B.D.; TRIVEDI, B.K.; UNANGST, P.C.; J. Heterocycl Chem, 2000, 37 (5), 1103-1108. FANGHAENEL, E.; CHTCHEGLOV, D.; J Prakt Chem/Chem-Ztg, 1996, 338 (8), 731-737. KUYPER, L.F.; BACCANARI, D.P.; JONES, M.L.; HUNTER, R.N.; TANSIK, R.L.; JOYNER, S.S.; BOYTOS, C.M.; RUDOLPH, S.K.; KNICK, V.; WILSON, H.R.; CADDELL, J.M.; FRIEDMAN, H.S.; ET AL.; J Med Chem, 1996, 39 (4), 892-903, 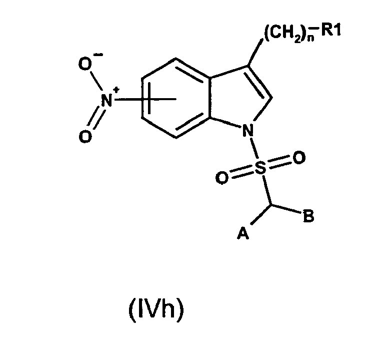 and the others R¹-R⁶, A, B and n have the previously mentioned meaning, or one of their derivatives suitably protected, and, if necessary, the protective groups are removed in order to obtain the corresponding amine of general Formula (Ih), which can be purified and/or isolated by means of conventional methods known in the state of the art.

The pharmaceutically acceptable salts of the compounds of general Formula (Ih), can be prepared by means of conventional methods known in the state of the art, preferably by reaction with a mineral acid, such as hydrochloric, hydrobromic, phosphoric, sulfuric, nitric acids, or with organic acids, such as citric, maleic, fumaric, tartaric acids, or their derivatives, p-toluenesulfonic, methanesulfonic acids, etc., in a suitable solvent, such as methanol, ethanol, diethyl ether, ethyl acetate, acetonitrile or acetone, being obtained with the usual techniques for the precipitation or crystallization of the corresponding salts.

The preferred physiologically acceptable salts of the sulfonamide derivatives of general formula (Ih) are the addition salts of mineral acids, such as hydrochloric, hydrobromic, phosphoric, sulfuric, nitric acids, and of organic acids, such as citric, maleic, fumaric, tartaric acids, or their derivatives, p-toluenesulfonic, methanesulfonic, camphorsulfonic acids, etc.

The physiologically acceptable solvates, particularly hydrates, of the sulfonamide derivatives of general formula (Ih) or of the corresponding physiologically acceptable salts, can be prepared by methods known in the state of the art.

During some of the synthetic sequences described or in the preparation of the suitable reagents used, it may be necessary and/or desirable to protect sensitive or reactive groups in some of the molecules used. This can be carried out by means of the use of conventional protective groups such as those described in the literature [Protective groups in Organic Chemistry, ed. J.F.W. McOmie, Plenum Press, 1973; T.W. Greene & P.G.M. Wuts, Protective Groups in Organic Chemistry, John Wiley & Sons, 1991]. The protective groups can be removed in the suitable subsequent stage by methods known in the state of the art.

If the sulfonamide derivatives of general Formula (Ih) are obtained in the form of a mixture of stereoisomers, particularly enantiomers or diastereomers, said mixtures can be separated by means of standard processes known in the state of the art, for example chromatographic methods or crystallization with chiral agents.

The active substance combination according to this invention comprises preferably 1-99% by weight of the component (A) and 99-1 % by weight of the component (B), more preferably 10-80% by weight of the component (A) and 90-20% by weight of the component (B), these percentages referring to the total weight of both components (A) and (B).

Another aspect of the present invention is a medicament, which comprises an inventive active substance combination and optionally one or more pharmacologically acceptable adjuvants.

Said medicament is particularly suitable for simultaneous regulation of neuropeptide Y-receptors, preferably neuropeptide Y5-receptors, and 5-HT₆ receptors, for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatoric diseases, immunologic diseases or for improvement of cognition.

Said medicament is more particularly suitable for simultaneous regulation of neuropeptide Y-receptors, preferably neuropeptide Y5-receptors, and 5-HT₆ receptors, for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome.

Another aspect of the present invention is the use of an inventive active substance combination for the manufacture of a medicament for simultaneous regulation of neuropeptide Y-receptors, preferably neuropeptide Y5-receptors, and 5-HT₆ receptors, for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Peripheral Nervous System Disorders, Central Nervous System Disorders, arthritis, epilepsy, anxiety, panic, depression, preferably bipolar disorders, cognitive disorders, memory disorders, cardiovascular diseases, senile dementia processes, neurodegenerative disorders, preferably Alzheimer's disease, Parkinson's diesease, Huntington's Disease and/or multiple sclerosis, schizophrenia, psychosis, infantile hyperkinesia (ADHD, attention deficit / hyperactivity disorder), pain, hypertensive syndrome, inflammatoric diseases, immunologic diseases or for improvement of cognition.

Particularly preferred is the use of an inventive active substance combination for the manufacture of a medicament for simultaneous regulation of neuropeptide Y-receptors, preferably neuropeptide Y5-receptors, and 5-HT₆ receptors, for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome.

Those skilled in the art understand that the components (A) and (B) of the active substance combination according to the present invention may be administered simultaneously or sequentially to one another, whereby in each case components (A) and (B) may be administerd via the same or different administration pathways, e.g. orally or parenterally. preferably both components (A) and (B) are administered simultaneously in one and the same administration form.

Yet another aspect of the present invention are pharmaceutical formulations in different pharmaceutical forms comprising an inventive active substance combination and optionally one or more pharmacologically acceptable adjuvants.

As well known to somebody skilled in the art the pharmaceutical formulations may - depending on their route of administration, also contain one or more auxiliary substances known to those skilled in the art.

The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986).

Preferred pharmaceutical formulations are solid pharmaceutical forms, preferably tablets, chewing tablets, chewing gums, dragées, capsules, suppositories, powder preparations, transdermal therapeutic systems, transmucosal therapeutic systems, preferably tablets or capsules.

Preferred pharmaceutical formulations are also liquid and semi-liquid pharmaceutical forms such as drops or such as juice, sirup, solution, emulsion, suspension, preferably drops or solutions.

In an additional preferred embodiment, the pharmaceutical formulations are in the form of multiparticulates, preferably microtablets, microcapsules, microspheroids, granules, crystals or pellets, optionally compacted in a tablet, filled in a capsule or suspended in a suitable liquid.

The pharmaceutical formulations according to the present invention are particularly suitable for oral, intravenous, intramuscular, subcutaneous, intrathecal, epidural, buccal, sublingual, pulmonal, rectal, transdermal, nasal or intracerebroventricular application, more particularly for oral, intravenous or intraperitoneal application.

In one embodiment of the present invention the pharmaceutical formulation comprises at least one of the components (A) and (B) of the active substance combination at least partially in a sustained-release form.

By incorporating one or both of these components (A) and (B) at least partially or completely in a sustained-release form it is possible to extend the duration of their effect, allowing for the beneficial effects of such a sustained-release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁₋₄)alkyl (meth)acrylates, po)y(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D^{®}), copolymers of ethyl acrylate, methyl methacrylate and trimethylammonium ethyl methacrylate-chloride with a monomer molar ratio of 1:2:0.1 (Eudragit RS^{®}), copolymers of ethyl acrylate, methyl methacrylate and trimethylammonium ethyl methacrylate-chloride with a monomer molar ratio of 1:2:0.2 (Eudragit RL^{®}), or a mixture of at least two of the above-mentioned copolymers. These coating materials are commercially available as 30 wt.% aqueous latex dispersions, i.e. as Eudragit RS30D^{®}, Eudragit NE30D^{®} or Eudragit RL30D^{®}, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat^{®} or Surelease^{®}.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet^{®} (acetylated mono- and diglycerides, C₂₃H₄₄O₅ to C₂₅H₄₇O₇), medium-chain triglycerides (Miglyol^{®}), oleic acid or mixtures of at least two of said plasticizers.

Aqueous dispersions of Eudragit RS^{®} and optionally Eudragit RL^{®} preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) coverd by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L^{®}), methacrylic acid/methyl methacrylate copolymers with a monomer molar ratio of 1:2 (Eudragit S^{®}), methacrylic acid/ethyl acrylate copolymers with a monomer molar ratio of 1:1 (Eudragit L30D-55^{®}), methacrylic acid/methyl acrylate/methyl methacrylate copolymers with a monomer molar ratio of 7:3:1 (Eudragit FS^{®}), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D^{®} and/or Eudragit RL^{®} and/or Eudragit RS^{®}.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311.

In another embodiment, the pharmaceutical formulation of the present invention contains one or both of components (A) and (B) not only in sustained-release form, but also in non-sustained-release form. By combination with the immediately released form, a high initial dose can be achieved for the rapid onset of the beneficial effect. The slow release from the sustained-release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

### Pharmacological Methods

### MEASUREMENTS OF FOOD INGESTION (BEHAVIOURAL MODEL)

Male W rats (200-270 g) from Harlan, S.A. are used. The animals are acclimatized to the housings during at least 5 days prior to being subjected to any treatment. During this period, the animals are housed (in groups of five) in translucent cages and have free access to water and food. The animals are housed in individual cages at least 24 hours prior to starting the treatment.

The effect of the active substance combination and of each one of the components (A) and (B) on food ingestion in rats in fasting conditions is then determined as follows:

The rats are kept in fasting conditions for 23 hours in their individual cages. After this period, the rats are distributed in four groups. To three of these groups doses of the component (A) (with vehicle), of the component (B) (with vehicle) and of the active substance combination (vehicle) have been administered respectively by the intraperitoneal route. To the fourth group just vehicle has been administered in the same way.

Immediately after this, the rat is left in the cage with pre-weighed food and the accumulated food intake is measured after 1, 2, 4 and 6 hours.

This food ingestion measuring method is also described in publications of Kask et al., European Journal of Pharmacology 414 (2001), 215-224, and Turnbull et al., Diabetes, Vol. 51, August, 2002.

### NEUROPEPTIDE Y5 RECEPTOR BINDING STUDIES:

The methods used for membrane preparation and binding are similar to those described by Y. Hu, B. T. Bloomquist et al. in Y. Hu, B. T. Bloomquist et al., The Journal of Biological Chemistry, 1996, 271, 26315-26319 with modifications. Cells C6 were transfected with the rat Y5 receptor. The cells were grown under standard culture conditions in 150 cm² dishes and they were harvested using a rubber scraper and 10 ml PBS. The cells from five dishes were collected and centrifuged 2.500 g for 5 min (4°C). The pellet was washed by resuspending in 3 ml buffer (Tris-HCl 10 mM, pH 7.4), homogenized using a Potter S homogenizer, 10 strokes at 600 rpm and centrifuged 48.000 g for 20 min (4°C). The pellet was resuspended in 8 ml membrane buffer (Tris-HCl 25 mM, NaCl 120 mM, KCl 5 mM, KH₂PO₄ 1,2 mM, CaCl₂ 2,5 mM, MgSO₄ 1,2 mM, BSA 0,15 mg/ml, Bacitracine 0,5 mg/ml, pH 7,4) and rehomogenized using the Potter S, 10 strokes at 600 rpm. The protein concentration in the incubation was 40 µg/ml. The radioligand was [¹²⁵I]-PYY (100 pM) in a total incubation volume of 200 µl. Following incubation at 25°C for 2 h, the reaction was stopped by addition of 5 ml ice-cold buffer (Tris-HCl 25 mM, NaCl 120 mM, KCI 5 mM, KH₂PO₄ 1,2 mM, CaCl₂ 2,5 mM, MgSO₄ 1,2 mM, pH 7,4) and rapid filtration in a Harvester Brandell Cell using filters (Schleicher & Schuell GF 3362) pretreated for two hours with 0,5% polyethyleneimine. Filters were washed one time with 5 ml ice-cold buffer. The filters were placed into plastic scintilation vials and 5 ml scintilation cocktail Ecoscint H were added. The quantity of radioactivity present was determined in a Wallac Winspectral 1414 counter. Non specific binding was determined in the presence of 1 µM de pNPY. All binding assays were done in triplicate.

### BINDING TO NEUROPEPTIDE Y₂ RECEPTOR

The experimental protocol follows the method by Y. Dumont et al. as described in Y. Dumont, A. Fournier, S. St-Pierre, R. Quirion: Characterization of Neuropeptide Y Binding Sites in Rat Brain Preparations Using [¹²⁵I][Leu³¹, Pro³⁴]Peptide YY and [¹²⁵I]Peptide YY₃₋₃₆ as Selective Y1 and Y2 Radioligands, The Journal of Pharmacology and Experimental Therapeutics, 1995, 272, 673-680, with slight modifications.

Male Wistar rats are sacrificed by decapitation, their brains are rapidly removed and the hypoccampus is dissected. Homogenization is performed in cold conditions in the buffer: 120 mM NaCl, 4.7 mM KCI, 2.2 mM CaCl₂, 1.2 mM KH₂PO₄, 1.2 mM MgSO₄, 25 mM NaHCO₃, 5.5 mM glucose, pH 7.4, by means of a Ultra-Turrax homogenizer for 15 seconds at 13,500 rpm. The ratio between fresh tissue weight and buffer volume is of ten times. The membrane is centrifuged for 10 min at 48,000 g. The supernatant is discarded and the pellet is washed, resuspended and recentrifuged two more times. The final membrane resuspension is performed in the buffer: 120 mM NaCl, 4.7 mM KCI, 2.2 mM CaCl₂, 1.2 mM KH₂PO₄, 1.2 mM MgSO₄, 25 mM NaHCO₃, 5.5 mM glucose, 0.1 % BSA, 0.05% bacitracin, pH 7.4, at a 90 ml/g ratio of fresh issue. The radioligand used is [¹²⁵I]-PYY₃₋₃₆ at the concentration of 28 pM. Incubation volume: 500 µl. Incubation is performed at 25 °C for 150 minutes and ended by rapid filtration in a Harvester Brandel Cell through fiber glass filters of the brand Schleicher & Schuell GF 3362 pretreated with a 0.5% polyethylenimine solution. The filters are cold-washed three times with three milliliters of the same buffer used in homogenization. The filters are transferred to vials and 5 ml of Ecoscint H liquid scintillation cocktail are added to each vial.The vials are allowed to reach steady state for a few hours before counting in a Wallac Winspectral 1414 scintillation counter. Non-specific binding is determined in the presence of 1 µM of pNPY (Neuropeptide Y of porcine origin). The assays are performed in triplicate.

### BINDING TO THE 5HT₆ SEROTONIN RECEPTOR

HEK-293 cell membranes expressing the recombinant human 5HT₆ receptor were supplied by Receptor Biology. The receptor concentration in said membranes is 2.18 pmol/mg of protein and the protein concentration is 9.17 mg/ml. The experimental protocol follows the method of B.L. Roth et al. [B.L. Roth, S.C. Craigo, M.S. Choudhary, A. Uluer, F.J. Monsma, Y. Shen, H.Y. Meltzer, D.R. Sibley: Binding of Typical and Atypical Antipshychotic Agents to 5-Hydroxytryptamine-6 and Hydroxytryptamine-7 Receptors. The Journal of Pharmacology and Experimental Therapeutics, 1994, 268, 1403], with following slight modifications. The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM, the final volume being 200 µl. Incubation begins by adding 100 µl of the membrane suspension (≈ 22.9 µg of membrane protein), and is prolonged for 60 minutes at a temperature of 37°C. Incubation ends by quick filtration in a Harvester Brandel Cell through fiberglass filters of the Schleicher & Schuell GF 3362 trademark, pretreated with a 0.5% polyethyleneimine solution. The filters are washed three times with three milliliters of 50 mM Tris HCl buffer, pH 7.4. The filters are transferred to vials and 5 ml of Ecoscint H. liquid scintillation cocktail are added to each vial. The vials are left to equilibrate for several hours prior to their counting in a 1414 Wallac Winspectral scintillation counter. The non-specific binding is determined in the presence of 100 µM of serotonin. The assays are carried out in triplicate. The inhibition constants (Kᵢ, nM) are calculated by non-linear regression analysis using the EBDA/LIGAND program [Munson and Rodbard, Analytical Biochemistry, 1980, 107, 220].

### Examples:

### Preparation of the compounds of general formula (Ia):

General method for obtaining haloamides derived of the general formula (IVa) 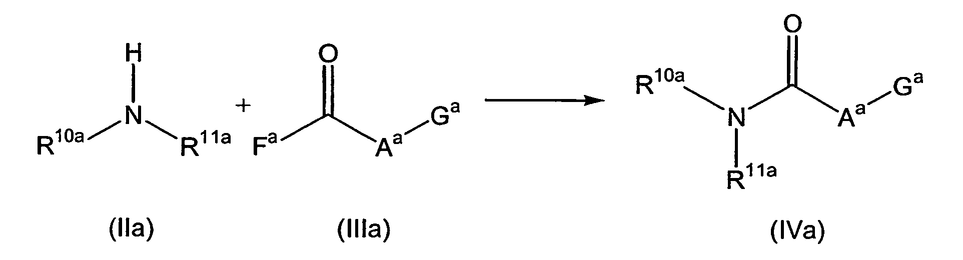 The haloamides used for obtaining the products object of our invention are either marketed ones or have been prepared according to the scheme 2, employing conventional methods. Esentially the corresponding amines are reacted with chloroacetyl chloride or with a derivative of the general formula (IIIa), the reaction is carried out using an organic solvent, usually dichloromethane, and a base, usually triethylamine.

### EXAMPLE A:

### 2-Chloro-N-(9-oxo-9H-fluoren-3-yl)-acetamide

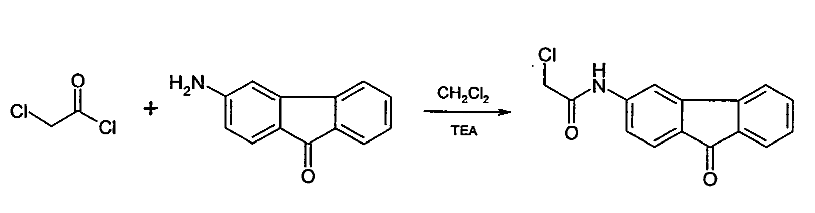

A solution of 3-amino-9-fluorenone ( 1.95 g, 10 mmols ), triethylamine ( 2.07 ml, 15 mmols), in 25 ml of dried dichloromethane, is cooled to 10° C and a solution of chloroacetyl chloride (1.18g, 10.5 mmoles) in 10 ml of dried dichloromethane is then added drop by drop. It is keeped under stirring during 1 hour and at room temperature overnight. It is washed 2x30 ml of water, dried over sodium sulfate and evaporated. 2,63 g 2-Chloro-N-(9-oxo-9H-fluoren-3-yl)-acetamide (97%) are obtained.
¹H NMR (d₆-DMSO): 10.7 (s, 1 H), 7.98 (s, 1 H), 7.66 (d, 1 H), 7.57 (m,3H), 7.50 (d,1 H), 7.37(t,1 H), 4,32(s, 2H)

### EXAMPLE 6a :

### 2-{1-[(9-Oxo-9H-fluoren-3-yl-carbamoyl)-methyl]-piperidin-4-yl-amino}benzoic acid methyl ester chlorohydrate

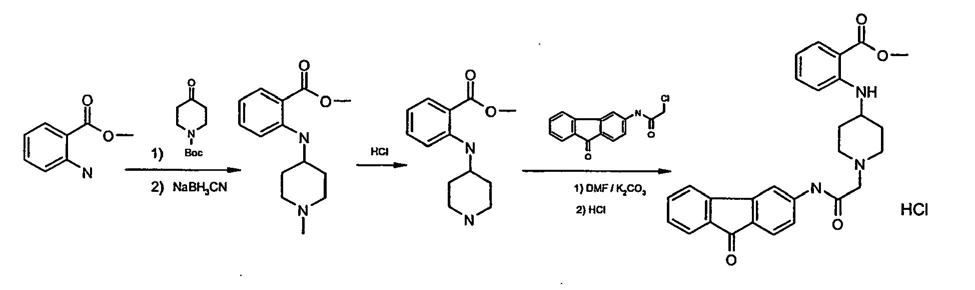

### a) 4-(2-metoxycarbonyl-phenylamino)-piperidine-1- tert-butylcarboxylate.

A solution of 1-(*tert*-butyloxycarbonyl)-4-piperidinone (2 g, 0.01 mol), methyl anthranylate (1.66 g, 0.011 mol) y acetic acid (1.4 ml, 0.022 mol) in dried toluene (50 mL) were heated at reflux temperature, removing the water by means of azeotropic distillation with a Dean-Stark, over 30 hours. Then, the mixture was cooled and concentrated under vacuum to the half of the volume. NaBH₃CN (2 g, 0.032 mol) and dried THF (30 mL) is added to a resulting solution.

Afterwards, acetic acid (1 mL, 0.017 mol) was aded drop by drop over one hour (1 mL, 0.017 mol). The reaction was stirred at room temperature over 24 hours. The mixture was concentrated under vacuum and the residue was solved in ethyl acetate (75 mL), washed with a saturated NaHCO3 (4 x 25 mL) and a saturated NaCl solution (25 mL), dried and evaporated to dryness. This raw material was used in the following step.

### b) 2-(Piperidine-4-yl-amino)-methyl benzoate

A solution of 3.2 g of the previous raw material in 40 mL of dried ethyl acetate, was cooled to 0°C. Then a 5 M hydrogen chloride solution in ethylic ether (40 mL) was added and the resulting mixture was keeped at 0°C over 4 hours. The solvent was evaporated and the residue was suspended in water and was alcalinized with sodium hydroxyde, and was extracted with chloroform ( 3 x 20 mL ), the combined organic extracts were washed wit water, dried over sodium sulfate and were evaporated. The raw material was passed through a cromatographic column by eluting with chloroform:methanol 9:1.

In this way 1,45 g of a yellow solid is obtained.

**IR** (cm⁻¹) KBr.: 3349, 3232, 2941, 2812, 1686, 1578, 1518, 1436, 1253, 1162, 1079, 742.
**M.P.:** 113-115° C

### c) 2-{1-[(9-Oxo-9H-fluoren-3-yl-carbamoyl)-methyl]-piperidine-4-yl-amino}benzoic acid methyl ester clorhidrate

A mixture of 2-(Piperidine-4-yl-amino)-methyl benzoate (1100 mg, 4.70 mmol), 2-Chloro-N-(9-oxo-9H-fluoren-3-yl)-acetamide (1358 mg, 5 mmol) and K₂CO₃ (1380 mg, 10 mmol) in DMF (40 mL) were keeped under stirring at 10°C over 2 hours and at room temperature overnight. The mixture of reaction was dropped over 50 mL water and 100 mL ethyl acetate, the organic phase was decanted and was washed with water ( 3 x 50 mL), was dried over sodium sulfate and a 2.8 M hydrogen chloride solution in absolute ethanol (1.80 mL) was added, the hydrochloride precipited, was filtrated and was washed with ethyl acetate. 1840 mg of a white solid were obtained. Yield: 77%.

### EXAMPLE 7a.

### Preparation of: 2-[4-2(2-Hidroxymethyl-4-methyl-phenylaminorpiperidine-1-yl]-N-phenyl-acetamide.

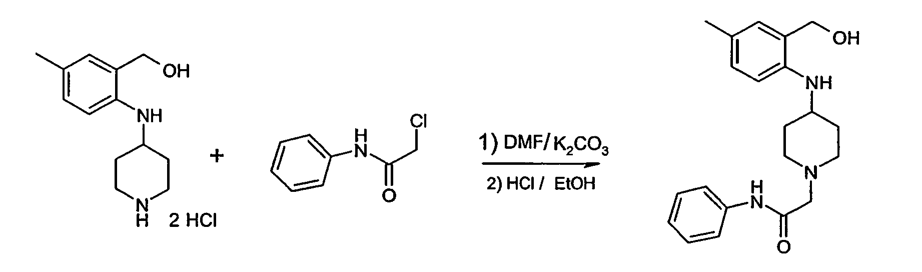

A mixture of 4-methyl-(2-hidroxymethylphenylamino)piperidine dihydrochloride (234mg, 0,80 mmol), 2-chloro-N-phenylacetamide (149 mg, 0,88 mmol) and K₂CO₃ (440 mg, 3.20 mmol) in DMF (10 mL) is keeped under stirring at room temperature overnight. The solvent is evaporated and then H₂O (15 mL) are added and the formed precipitate is extracted with ethyl acetate, washed with water, dried and evaporated to dryness. The raw material crystallizes from ethyl acetate, is filtrated and dried. 178 mg of a white solid are obtained. Yield: 63%.

### EXAMPLE 8a.

### Preparation of 2-[4-(2-Hydroxymethyl-phenylamino)-piperidine-1-yl]-N-(1-oxo-indan-5-yl)-acetamide.:

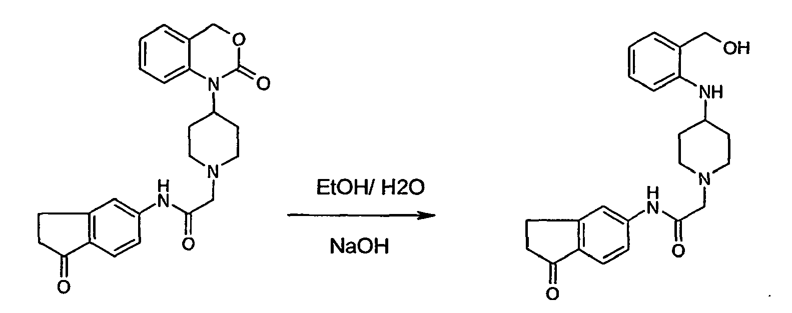

5 mL 10% sodium hydroxide were added to a suspension of 2-[4-(2-oxo-4H-benzo[d][1,3]oxazin-1-yl)-piperidine-1-yl]-N-(1-oxo-indan-5-yl)-acetamide (25 mg , 0.06 mmols) in 5 mL ethanol, it was heated at 50°C over 2 hours, was cooled, the ethanol was evaporated and the aqueous phase was neutralized and was extracted with methylene chloride (2x15mL). The organic extracts were washed with water, dried over sodium sulfate and evaporated to dryness. The raw material of reaction was passed through a silica gel column, eluting with ethyl acetate. 15 mg of a white solid were obtained, with a yield of 64%.

| | | |
|---|---|---|
| Ex. 1a | N-(9-Ethyl-9H-carbazol-3-yl)-2-[4-(2-hydroxymethyl-6-methyl-phenylamino)- piperidine-yl]acetamide dihydrochlorhide | |
| | 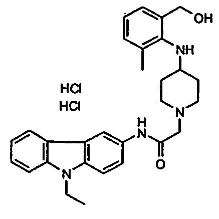 | 1 H-NMR 1 H NMR (300 MHz, DMSO-d6) δ ppm: 1.3 (t, *J*=7.0 Hz, 3 H) 2.1 (s, 4 H) 2.4 (s, 3 H) 3.2 (m, 2H) 3.5-4.1 (4 H) 4.2 (s, 2 H) 4.4 (m, 2 H) 4.7 (s, 2 H) 7.2 (m, 4 H) 7.4 (t, *J*=7.7 Hz, 1 H) 7.6 (d, *J*=8.2 Hz, 3 H) 8.0 (d, *J*=7.7 Hz, 1 H) 8.4 (s, 1 H) 10.3(s,1H) 11.0 (s, 1H) |
| | | IR (KBr) : 3398, 2974, 1685, 1597, 1560, 1491, 1471, 1230, 749. |
| | | Melting point: 218-222 °C |
| Ex. 2a | 2-[4-(2-Hydroxymethyl-phenylamino)-piperidine-1-yl]-N-(9-oxo-9H-fluoren-3-yl)-acetamide | |
| | 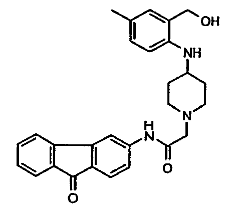 | 1 H NMR (300 MHz, CDCl₃-d) δ ppm: 1.6 (m, 2 H) 2.2 (d, *J*=13.9 Hz, 2 H) 2.2 (s, 3 H) 2.5 (t, *J*=10.2 Hz, 2 H) 2.9 (d, *J*=10.6 Hz, 2 H) 3.2 (s, 2 H) 3.4 (m, 1 H) 4.7 (s, 2 H) 6.6 (d, *J*=8.2 Hz, 1 H) 6.9 (d, *J*=1.6 Hz, 1 H) 7.0 (dd, *J*=8.1, 1.7 Hz, 1 H) 7.3 (m, 2 H) 7.5 (td, *J*=7.4, 1.1 Hz, 1 H) 7.6 (m, 3 H) 8.0 (d, *J*=1.6 Hz, 1 H) 9.5 (s, 1 H) |
| | | IR (KBr): 3330, 3148, 1710, 1590, 1516, 1291, 1109, 980, 722 |
| | | Melting point: 152 °C |
| Ex. 3a | 2-[4-(2-Hydroxymethyl-6-methyl-phenylamino)-piperidine-1-yl]-N-(9-oxo-9H-fluoren-3-yl)-acetamide | |
| | 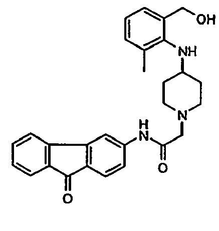 | 1 H NMR (300 MHz, CDCl₃-d) δ ppm: 1.6 (d, *J*=11.8, 2 H) 2.0 (d, *J*=12.3 Hz, 2 H) 2.3 (s, 3 H) 2.4 (m, 2 H) 2.9 (d, *J*=7.0 Hz, 2 H) 3.1 (m, 1 H) 3.2 (s, 2 H) 4.7 (s, 2 H) 6.9 (t, *J*=7.4 Hz, 1 H) 7.0 (m, 1 H) 7.1 (d, *J*=9.2 Hz, 1 H) 7.3 (m, 2 H) 7.5 (td, *J*=7.4, 1.1 Hz, 1 H) 7.6 (m, 3 H) 8.0 (d, *J*=1.8 Hz, 1 H) 9.4 (s, 1 H) |
| | | IR (KBr) :.3414, 3269, 2920, 1710, 1692,1609, 1508, 1230,1101, 1002, 737 |
| | | Melting point: 113 °C |
| Ex. 4a | N-(9-Hyd roxy-9H-fluoren-3-yl)-2-[4-(2-hydroxymethyl-4-methyl-phenylamino)- piperidine-1-yl]-acetamide | |
| | 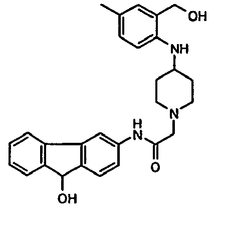 | 1 H NMR (300 MHz, CDCl₃-d) δ ppm: 1.6 (m, 2 H) 2.1 (d, *J*=14.3 Hz, 2 H) 2.2 (s, 3 H) 2.5 (m, 2 H) 2.9 (d, *J*=12.5 Hz, 2H)3.1 (s, 2 H) 3.4 (m, 1 H) 4.6 (s, 2 H) 5.6 (s, 1 H) 6.6 (d, *J*=8.2 Hz, 1 H) 6.9 (d, *J*=1.8 Hz, 1 H) 7.0 (dd, *J*=8.1, 1.9 Hz, 1 H) 7.4 (m, 3 H) 7.6 (m, 3 H) 8.0 (d, *J*=1.8 Hz, 1 H) 9.3 (s, 1 H) |
| | | IR (KBr) : 3300, 2920, 1670, 1613, 1521, 1025, 767 |
| | | Melting point: 124°C |
| Ex. 5a | N-(9-Hydroxy-9H-fluoren-3-yl)-2-[4-(2-hydroxymethyl-6-methyl-phenylamino)-piperidine-1-yl]-acetamide | |
| | 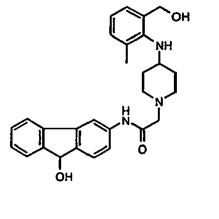 | 1 H NMR (300 MHz, CDCl₃-d) δ ppm: 1.6 (m, 2 H) 2.0 (d, *J*=10.4 Hz, 2 H) 2.3 (m, 5 H) 2.9 (d, *J*=11.9 Hz, 2 H) 3.0 (m, 1 H)3.1 (s, 2 H) 4.7 (s, 2 H) 5.6 (s, 1 H) 6.9 (t, *J*=7.4 Hz, 1 H) 7.0 (m, 1 H) 7.1 (d, *J*=9.0 Hz, 1 H) 7.4 (m, 3 H) 7.6 (m, 3 H) 8.0 (d, *J*=2.0 Hz, 1 H) 9.2 (s, 1 H) |
| | | IR (KBr): 3315, 2927, 1676, 1527, 1097, 1025, 771, 737 |
| | | Melting point: 133°C |
| Ex. 6a | 2-{1-[(9-Oxo-9H-fluoren-3-yl-carbamoyl)-methyl]-piperidine-4-yl-amino}benzoic acid methyl ester hydrochloride | |
| | 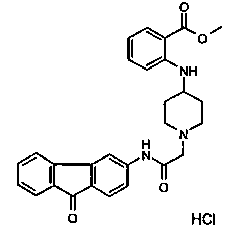 | 1 H NMR (300 MHz, DMSO-d6) δ ppm: 1.8 (m, 2 H) 2.2 (d, *J*=13.8 Hz, 2 H) 3.3 (m, 2 H) 3.6 (d, *J*=10.8 Hz, 2 H) 3.8 (s, 3 H) 3.8 (m, 1 H) 4.2 (s, 2 H) 6.6 (t, *J*= 7.8 Hz, 1 H) 6.9 (d, *J*=8.6 Hz, 1 H) 7.3 (m, 2 H) 7.5 (m, 4 H) 7.6 (m, 1 H) 7.8 (dd, *J*=8.0, 1.6 Hz, 1 H) 8.0 (d, *J*=1.3 Hz, 1 H) 10.1 (s, 1H) 11.1 (s, 1H) |
| | | IR (KBr): 2946, 2539, 1700, 1684, 1603, 1560, 1255, 748 |
| | | Melting point: 258°C |
| Ex. 7a | 2-[4-(2-Hydroxymethyl-4-methyl-phenylamino)-piperidine-1-yl]-N-phenyl-acetamide | |
| | 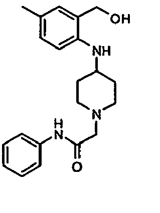 | 1H NMR (300 MHz, CDCl₃-d) δ ppm: 1.6 (m, 2 H) 2.1 (d, *J*=13_{.}0 Hz, 2 H) 2.2 (s, 3 H) 2.4 (t, *J*=10.3 Hz, 2 H) 2.9 (d, *J*=11.9 Hz, 2 H) 3.1 (s, 2 H) 3.4 (m, 1 H) 4.6 (s, 2 H) 6.6 (d, *J*=8.2 Hz, 1 H) 6.9 (s, 1 H) 7.0 (dd, *J*=8.2, 1.5 Hz, 1 H) 7.1 (t, *J*=7.4 Hz, 1 H) 7.3 (t, *J*=7.9 Hz, 2 H) 7.6 (d, *J*=7.7 Hz, 2 H) 9.2 (s, 1 H) |
| | | IR (KBr): 3346, 1691, 1598, 1543, 1438, 1317, 748 |
| | | Melting point: 128°C |
| Ex. 8a | 2-[4-(2-Hydroxymethyl-phenylamino)-piperidine-1-yl]-N-(1-oxo-indan-5-yl)-acetamide | |
| | 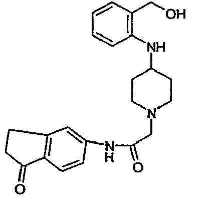 | 1H NMR |
| | | IR (KBr): 3398, 2923, 1710, 1655, 1590, 1541, 1425, 1287, 1126, 1013 |
| | | Melting point:138-140 ° C |

### Preparation of the compound of general formula (Ib):

### Example Ab:

### Synthesis of an intermediate compound of general formula (IIb)

### Preparation of 6-Chloro-1-(piperidine-4-yl)-1,4-dihydro-2H-3,1-benzoxazin-2-one hydrochloride

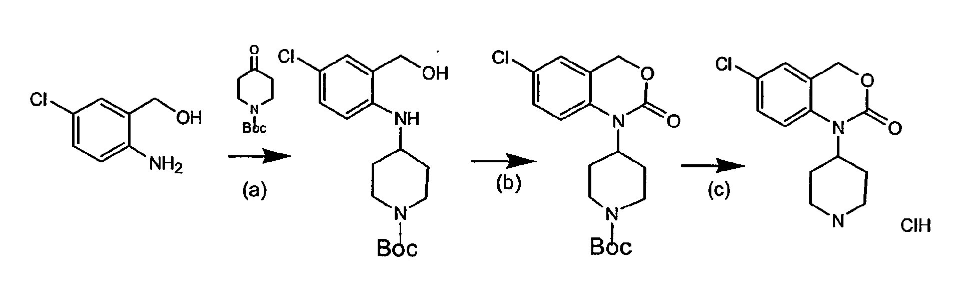

### a) 1-(tert-Butyloxycarbonyl)-4-[4-chloro-(2-hydroxymethylphenylamine)] piperidine

A solution of 1-(*tert*-butyloxycarbonyl)-4-piperidinone (20 g, 0.10 mol), 2-amino-5-chlorobenzylic alcohol (17.34 g, 0.11 mol) and acetic acid (14 mL, 0.22 mol) in dry toluene (500 mL) was heated at reflux temperature, with water elimination by means of azeotrope distillation with Dean-Stark, for 6 hours. The mixture was then cooled and vacuum concentrated up to half volume. NaBH₃CN (20 g, 0.32 mol) and dry THF (300 mL) were added to the resulting solution. Acetic acid (10 mL, 0.17 mol) was then dripped for one hour. The reaction was stirred at room temperature for 24 hours. The mixture was vacuum concentrated and the residue was dissolved in ethyl acetate (750 mL), washed with a NaHCO₃-saturated solution (4 x 250 mL) and a NaCl-saturated solution (250 mL), dried and evaporated to dryness. The residue was purified by means of flash chromatography eluting with a mixture of ethyl acetate: petroleum ether (1:3).

The desired product was thus obtained as an oil (32.7 g, 96%). ¹H NMR (CDCl₃): 1.32 (d, *J*=11.2 Hz, 2H), 1.41 (s, 9H), 1.92 (d, *J*=11.2 Hz, 2H), 2.92 (t, *J*=12.0 Hz, 1H), 3.10 (s, 1H), 3.37 (m, 1H), 3.88 (d, *J*= 13.7 Hz, 2H), 4.49 (s, 2H), 4.75 (s, 1H), 6.52 (d, *J*= 8.6 Hz, 1H), 6.96 (s, 1H), 7.07 (d, *J*= 8.6 Hz, 1H).

### b.) 1-(1-tert-Butyloxycarbonyl-4-piperidinyl)-6-chloro-1,4-dihydro-2H-3,1-benzoxazin-2-one

N, N-diisopropylethylamine (DIEA) (43 mL, 0.25 mol) and triphosgene (8.65 g, 29.2 mmol) were added to a solution of 1-(*tert*-Butyloxycarbonyl)-4-[(4-chloro-(2-hydroxymethyl) phenyl-amino)]piperidine (27.0 g, 79 mmol) in dry THF (250 mL) cooled at 0°C. The reaction was stirred at 0°C for 1 h and at room temperature for 72 h. Ethyl ether was added and the mixture was cooled at 0°C for 3 h and the DIEA hydrochloride was then filtered. The filtered solution was evaporated to dryness and the residue was dissolved in ethyl acetate (750 mL), washed with 5% solution of critic acid (2 x 500 mL), water (250 mL) and NaHCO₃-saturated solution (2 x 500 mL). The ethyl acetate solution was dried (MgSO₄), filtered and evaporated under reduced pressure. The residue was brought to the boil with ethyl ether until the whole solid was dissolved and then cooled overnight to yield the desired compound in crystalline form (28.9 g, 67%).
Melting point: 177-179 °C
¹H NMR (CDCl₃): 1.46 (s, 9H), 1.79 (d, *J*= 10.1 Hz, 1H), 2.54 (m, 2H), 2.78 (m, 2H), 3.96 (m, 1H), 4.28 (m, 2H), 5.02 (s, 2H), 6.98 (d, *J*= 8.7 Hz, 1H) 7.13 (d, *J*= 2.4 Hz, 1H), 7.28 (dd, *J*= 8.7 Hz, *J*= 2.4 Hz, 1H).

### c.) 6-chloro-1-(piperidin-4-yl)-1,4-dihydro-2H-3,1-benzoxazin-2-one hydrochloride

A solution of 1-[(1-*tert*-Butyloxycarbonyl)-4-piperidinyl]-6-chloro-1,4-dihydro-2H-3,1-benzoxazin-2-one (24 g, 65 mmol) in ethyl acetate (500 mL) was cooled at 0°C. A 5 M solution of hydrogen chloride in ethyl ether (500 mL) was then added and the resulting mixture was stirred at 0°C for 4 h. The precipitate formed was collected by filtration, washed with ether and vacuum dried to yield the desired product as a solid (16.95 g, 97%). Melting point: 254-257 °C
¹H NMR (CD₃OD): 2.13 (d, *J*= 12.2 Hz, 2H), 2.88 (m, 2H), 3.20 (m, 2H), 3.53 (d, *J*= 12.8 Hz, 2H), 4.24 (m, 1H), 5.16 (s, 2H), 7.31 (m, 2H), 7.41 (dd, *J*= 8.8 Hz, *J*= 2.6 Hz, 1H).

Several substituted 3,1-benzoxazin-2-one compounds were prepared via the respectively substituted benzyl alcohols by reducing the respectively substituted anthranilic acids with lithium aluminium hydride and other known reducing agents by methods well known to those skilled in the art (see scheme 1), e.g. por ejemplo 6-methyl-1-(piperidin-4-yl)-1,4-dihydro-2H-3,1-benzoxazin-2-one, 7-methyl-1-(piperidin-4-yl)-1,4-dihydro-2H-3,1-benzoxazin-2-one, 8-methyl-1-(piperidin-4-yl)-1,4-dihydro-2H-3,1-benzoxazin-2-one, 5-methoxy-1-(piperidin-4-yl)-1,4-dihydro-2H-3,1-benzoxazin-2-one, 6-fluoro-1-(piperidin-4-yl)-1,4-dihydro-2H-3,1-benzoxazin-2-one, 8-methoxy-1-(piperidinyl)-1,4-dihydro-2H-3,1-benzoxazin-2-one, 5-methyl-1-(piperidin-4-yl)-1,4-dihydro-2H-3,1-benzoxazin-2-one, 7-fluoro-1-(piperidin-4-yl)-1,4-dihydro-2H-3,1-benzoxazin-2-one, 5-fluoro-1-(piperidin-4-yl)-1,4-dihydro-2H-3,1-benzoxazin-2-one, 6-methoxy-1-(piperidinyl)-1,4-dihydro-2H-3,1-benzoxazin-2-one, 5-chloro-1-(piperidinyl)-1,4-dihydro-2H-3,1-benzoxazin-2-one, 7-chloro-1-(piperidinyl)-1,4-dihydro-2H-3,1-benzoxazin-2-one, 8-chloro-1-(piperidinyl)-1,4-dihydro-2H-3,1-benzoxazin-2-one and others. The reaction of the respective 5-methoxy-1-(piperidinyl)-1,4-dihydro-2H-3,1-benzoxazin-2-one, 8-methoxy-1-(piperidinyl)-1,4-dihydro-2H-3,1-benzoxazin-2-one and 6-methoxy-1-(piperidinyl)-1,4-dihydro-2H-3,1-benzoxazin-2-one compounds according to conventional methods, e.g. BBr₃ in an inert organic solvent yields the respective 5-hydroxy-1-(piperidinyl)-1,4-dihydro-2H-3,1-benzoxazin-2-one, 8-hydroxy-1-(piperidinyl)-1,4-dihydro-2H-3,1-benzoxazin-2-one and 6-hydroxy-1-(piperidinyl)-1,4-dihydro-2H-3,1-benzoxazin-2-one compounds. The unsubstituted benzoxazin-2-one 1-(piperidin-4-yl)-1,4-dihydro-2H-3,1-benzoxazin-2-one is prepared according the method described in J. Med. Chem. 1995, 38, 4634 and J.Med.Chem. 1998, 41, 2146.

The substituted anthranilic acids were reduced by conventional methods known to those skilled in the art, e.g. by the use of LiAlH₄ as reducing agent in anhydrous THF under an inert-gas atmosphere, e.g. argon or nitrogen. This process is very efficient and in most cases the respective 2-aminobenzylalcohols are obtained in very good yields.

### General instruction for the reduction of substituted anthranilic acids:

To a three neck flask, equipped with a mechanical stirrer and an inlet for gaseous nitrogen, 100 mL anhydrous THF and 116,6 mmoles of LiAlH₄ were given and the resulting suspension cooled to 0 °C. After the addition of 58,3 mmoles of the respective substituted anthranilic acid in 150 mL anhydrous THF, the resulting reaction mixture is warmed to room temperature and stirred or about an hour. Under cooling to 0° C 4,7 mL water, 4,7 mL NaOH 15 wt.-%, and finally 14 mL water are carefully added to the mixture. The resulting suspension is filtered and washed with ethylacetate.

The organic phase is washed with water, dried and the solvent evaporated. In most cases the resulting product may be used without further purification.

### Example 5b:

### Preparation of 1-[1-quinoline-8-sulfonyl)-piperidine-4-yl]-1,4-dihydro-benzo[d][1,3]oxazin-2-one

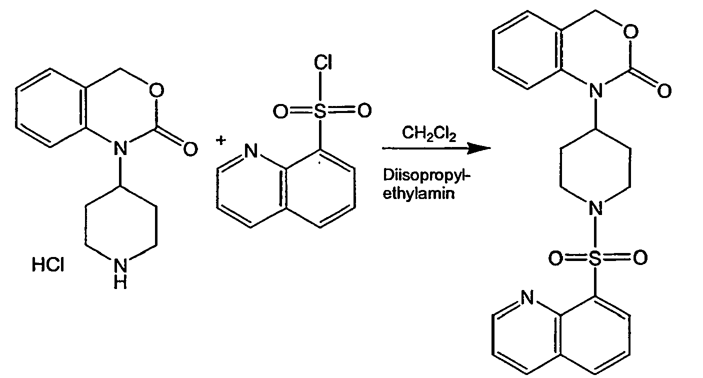

150 mg (0,66 mmol) quinoline-8-sulfonyl chloride are added to a mixture of 1-(4-piperidinyl)-1,4-dihydro-2H-3,1-benzoxazinone hydrochloride (161 mg, 0,60 mmol) and diisopropylethylamin (230 mg, 1,80 mmol) in dichloromethane (10 ml) and the resulting reaction mixture was stirred overnight at room temperature. The reaction mixture was then washed with water (3 x 15 mL) and the organic phase was separated, dryed and evaporated to dryness. A solid was obtained, which was recrystallized from ethanol. 182 mg of 1-[1-quinoline-8-sulfonyl)-piperidine-4-yl]-1,4-dihydro-benzo[d][1,3]oxazin-2-one were obtained as a white solid (yield 69 %).
IR (cm⁻¹) KBr: 1712, 1337, 1291, 1205, 1162, 1144, 1034, 717, 583
¹H-NMR(δ in ppm): 1.8 (d, *J*=9.5 Hz, 2 H) 2.6 (qd, *J*=12.6, 4.4 Hz, 2 H) 3.0 (td, *J*=12.8, 2.5 Hz, 2 H) 4.1 (tt, *J*=12.5, 3.8 Hz, 1 H) 4.3 (ddd, *J*=13.0, 2.3 Hz, 2 H) 5.0 (s, 2 H) 7.1 (m, 3 H) 7.3 (m, 1 H) 7.6 (dd, *J*=8.4, 4.2 Hz, 1 H) 7.6 (m, 1 H) 8.1 (dd, *J*=8.2, 1.3 Hz, 1 H) 8.3 (dd, *J*=8.3, 1.7 Hz, 1 H) 8.5 (dd, *J*=7.3, 1.5 Hz, 1 H) 9.1 (dd, *J*=4.2, 1.8 Hz, 1 H) (CDCl3-d).
Melting point: 170-172 °C.

The compounds according to examples 1b-4b and 6b-10b given in the following table Ib were prepared analogously to the methods described above:

**Table 1b:**

| | | |
|---|---|---|
| Ex. 1b | 1-[1-(Naphthyl-1-sulfonyl)-piperidine-4-yl]-1,4-dihydro-benzo[d][1,3]oxazine-2-one | |
| | 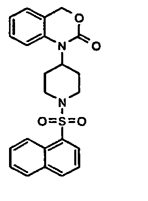 | 1H-NMR: 1.8 (d, *J*=10.5 Hz, 2 H) 2.4 (m, 2 H) 2.7 (t, *J*=11.6 Hz, 2 H) 3.9 (m, 3 H) 5.1 (s, 2 H) 7.1 (m, 2 H) 7.2 (m, 2 H) 7.7 (m, 3 H) 8.1 (d, *J*=8.1 Hz, 1 H) 8.2 (d, *J*=7.8 Hz, 1 H) 8.3 (d, *J*=8.1 Hz, 1 H) 8.7 (d, *J*=8.3 Hz, 1 H) (DMSO-d6) |
| | | IR (KBr) 1709, 1498, 1353, 1162, 1034, 770, 718, 579 |
| | | Melting point: 147-149°C |
| Ex. 2b | 1-(1-Phenylsulfonyl-piperidine-4-yl] -1,4-dihydro-benzo[d][1,3]oxazine-2-one | |
| | 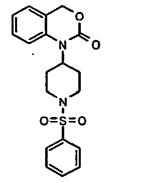 | 1 H-NMR: 1.9 (dd, *J*=12.1, 2.1 Hz, 2 H) 2.4 (td, *J*=12.2, 2.4 Hz, 2 H) 2.7 (qd, *J*=12.6, 4.3 Hz, 2 H) 3.9 (tt, *J*=12.3, 3.9 Hz, 1 H) 4.0 (dt, *J*=11.9, 2.1 Hz, 2 H) 5.0 (s, 2 H) 7.0 (d, *J*=8.3 Hz, 1 H) 7.0 (t, *J*=7.3 Hz, 1 H) 7.1 (m, 1 H) 7.3 (m, 1 H) 7.6 (m, 3 H) 7.8 (m, 2 H) (CDCl₃-d) |
| | | IR (KBr) 1705, 1497, 1340, 1293, 1205, 1160, 736, 691, 576 |
| | | Melting point: 172-174°C |
| Ex. 3b | 1-[1-(5-chloro-3-methyl-benzo[b]tiophene-2-sulfonyl)-piperidine-4-yl] -1,4-dihydrobenzo[d][1,3]oxazine-2-one | |
| | 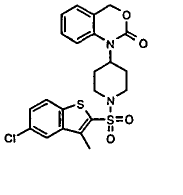 | 1 H-NMR: 1.8 (d, *J*=10.8 Hz, 2 H) 2.5 (m, 2 H) 2.7 (s, 3 H) 2.8 (t, *J*=11.4 Hz, 2 H) 3.8 (d, *J*=11.4 Hz, 2 H) 3.9 (m, 1 H) 5.1 (s, 2 H) 7.0 (t, *J*=7.2 Hz, 1 H) 7.2 (d, *J*=8.1 Hz, 1 H) 7.2 (m, 2 H) 7.6 (dd, *J*=8.6, 2.0 Hz, 1 H) 8.1 (d, *J*=2.0 Hz, 1 H) 8.2 (d, *J*=8.6 Hz, 1 H) (DMSO-d6) |
| | | IR (KBr) 1717, 1358, 1248, 1201, 1160, 1035, 712, 554 |
| | | Melting point: 204-206°C |
| Ex. 4b | 8-Methyl-l-[1-(naphthyl-1-sulfonylypiperidine-4-yl] -1,4-dihydro-benzo[d][1,3]oxazine-2- one | |
| | 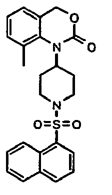 | 1 H-NMR: 1.9 (d, *J*=12.5 Hz, 2 H) 2.3 (s, 3 H) 2.7 (m, 4 H) 3.3 (m, 1 H) 4.0 (d, *J*=11.2 Hz, 2 H) 4.9 (s, 2 H) 7.0 (m, 2 H) 7.1 (d, *J*=7.0 Hz, 1 H) 7.6 (m, 3 H) 7.9 (m, 1 H) 8.1 (d, *J*=8.2 Hz, 1 H) 8.2 (dd, *J*=7.3, 1.1 Hz, 1 H) 8.7 (d, *J*=8.8 Hz, 1 H) (CDCl3-d) |
| | | IR (KBr) 1712, 1316, 1279, 1222, 1160, 1135, 1025, 768, 607 |
| | | Melting point: 203-204°C |
| Ex. 5b | 1-[1-(Quinolinyl-8-sulfonyl)-piperidine-4-yl] -1,4-dihydro-benzo[d][1,3]oxazine-2-one | |
| | 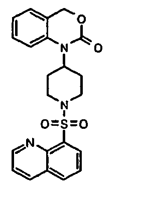 | 1H-NMR: 1.8 (d, *J*=9.5 Hz, 2 H) 2.6 (qd, *J*=12.6, 4.4 Hz, 2 H) 3.0 (td, *J*=12.8, 2.5 Hz, 2 H) 4.1 (tt, *J*=12.5, 3.8 Hz, 1 H) 4.3 (ddd, *J*=13.0, 2.3 Hz, 2 H) 5.0 (s, 2 H) 7.1 (m, 3 H) 7.3 (m, 1 H) 7.6 (dd, *J*=8.4, 4.2 Hz, 1 H) 7.6 (m, 1 H) 8.1 (dd, *J*=8.2, 1.3 Hz, 1 H) 8.3 (dd, *J*=8.3, 1.7 Hz, 1 H) 8.5 (dd, *J*=7.3, 1.5 Hz, 1 H) 9.1 (dd, *J*=4.2, 1.8 Hz, 1 H) (CDCl3-d) |
| | | IR (KBr) 1712, 1337, 1291, 1205, 1162, 1144, 1034, 717, 583 |
| | | Melting point: 170-172°C |
| Ex. 6b | 8-Methyl-1-[1-(quinolinyl-8-sulfonyl)-piperidine-4-yl] -1,4-dihydro-benzo[d][1,3]oxazine-2-one | |
| | 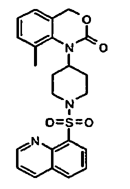 | 1 H-NMR: 1.9 (d, *J*=12.6 Hz, 2 H) 2.3 (s, 3 H) 2.7 (qd, *J*=12.2, 3.9 Hz, 2 H) 2.9 (m, 2 H) 3.3 (tt, *J*=11.7, 3.4 Hz, 1 H) 4.3 (d, *J*=12.8 Hz, 2 H) 4.9 (s, 2 H) 7.0 (m, 2 H) 7.1 (d, *J*=7.3 Hz, 1 H) 7.5 (dd, *J=*8.3, 4.1 Hz, 1 H) 7.6 (m, 1 H) 8.0 (dd, *J*=8.2, 1.3 Hz, 1 H) 8.2 (dd, *J*=8.3, 1.7 Hz, 1 H) 8.5 (dd, *J*=7.3, 1.5 Hz, 1 H) 9.1 (dd, *J*=4.2, 1.8 Hz, 1 H) (CDCl3-d) |
| | | IR (KBr) 1702, 1329, 1284, 1218, 1024, 785, 701, 582 |
| | | Melting point: 202-206°C |
| Ex. 7b | 1-[1-(5-Dimethylamino-naphthyl-1-sulfonyl)-piperidine-4-yl]-8-Methyl -1,4-dihydrobenzo[d][1,3]oxazine-2-one | |
| | 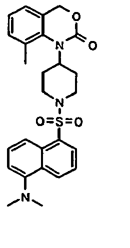 | 1 H-NMR: 1.9 (d, *J*=11.9 Hz, 2 H) 2.3 (s, 3 H) 2.7 (m, 4 H) 2.9 (s, 6 H) 3.3 (m, 1 H) 4.0 (d, *J*=9.9 Hz, 2 H) 4.9 (s, 2 H) 7.0 (m, 2 H) 7.2 (m, *J*=7.3 Hz, 2 H) 7.5 (m, 2 H) 8.2 (dd, *J*=7.3, 1.1 Hz, 1 H) 8.4 (d, *J*=8.6 Hz, 1 H) 8.6 (d, *J*=8.4 Hz, 1 H) (CDCI3-d) |
| | | IR (KBr) 2981, 1711, 1336, 1221, 1149, 1025, 794, 709, 571 |
| | | Melting point: 202-203°C |
| Ex. 8b | 1-[1-(5-Dimethylamino-naphthyl-1-sulfonyl)-piperidine-4-yl] -1,4-dihydro-benzo[d][1,3]oxazine-2-one | |
| | 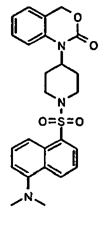 | 1H-NMR 1.8 (dd, *J*=12.3, 3.5 Hz, 2 H) 2.7 (m, 4 H) 2.9 (s, 6 H) 4.0 (m, 3 H) 5.0 (s, 2 H) 6.9 (d, *J*=8.2 Hz, 1 H) 7.1 (m, 2 H) 7.3 (m, 2 H) 7.6 (td, *J*=8.9, 7.4 Hz, 2 H) 8.3 (dd, *J*=7.3, 1.3 Hz, 1 H) 8.4 (d, *J*=8.8 Hz, 1 H) 8.6 (d, *J*=8.2 Hz, 1 H) (CDCI3-d) |
| | | IR (KBr) 2935, 1720, 1319, 1242, 1144, 920, 791, 755, 642 |
| | | Melting point: 182-186°C |
| Ex. 9b | 1-[1-(2,3-Dichloro-phenylsulfonyl)-piperidine-4-yl] -1,4-dihydro-benzo[d][1,3]oxazine-2-one | |
| | 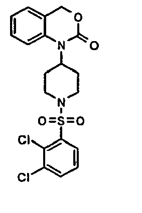 | 1H-NMR 1.9(d,*J*=10.1Hz, 2 H) 2.7 (qd, *J*=12.6, 4.2 Hz, 2 H) 3.0 (td, *J*=12.7, 2.3 Hz, 2 H) 4.1 (m, 3 H) 5.1 (s, 2 H) 7.1 (m, 3 H) 7.3 (m, 2 H) 7.7 (dd, *J*=8.0, 1.6 Hz, 1 H) 8.0 (dd, *J*=8.0, 1.6 Hz, 1H) (CDCl3-d) |
| | | IR (KBr) 1697, 1395, 1244, 1165, 1045, 942, 710, 582 |
| | | Melting point: 185-187 °C |
| Ex. 10b | 1-[1-(2,3-Dichloro-phenylsulfonyl)-piperidine-4-yl]-8-Methyl-1,4-dihydro- benzo[d][1,3]oxazine-2-one | |
| | 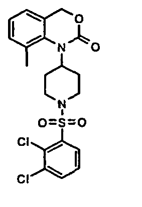 | 1 H-NMR: 2.0 (d, *J*=11.5 Hz, 2 H) 2.4 (s, 3 H) 2.8 (m, 4 H) 3.4 (m, 1 H) 4.0 (d, *J*=9.9 Hz, 2 H) 5.0 (s, 2 H) 7.0 (m, 2 H) 7.2 (d, *J*=7.7 Hz, 1 H) 7.3 (t, *J*=8.0 Hz, 1 H) 7.7 (dd, *J*=8.1, 1.5 Hz, 1 H) 8.0 (dd, *J*=8.0, 1.6 Hz, 1 H) (CDCl3-d) |
| | | IR (KBr) 1705, 1404, 1339, 1224, 1149, 939 |
| | | Melting point: 184-185°C |

### Preparation of the compounds of general formula (Ic):

### METHOD Ac

### Example 7c:

### Preparation of N-[3-(2-dimethylaminoethyl)-1H indol-5-yl]-5-chloro-3-methylbenzo[b]thiophene-2-sulphonamide.

To a solution of 3.05 g (15 mmol) of 5-amino-3-(2-dimethylaminoethyl)-1*H*-indol in 100 ml of pyridine is added dropwise at ambient temperature a solution of 4.21 g (15 mmol) of 5-chloro-3-methyl-benzo[b]thiophene-2-sulphonyl chloride in 20 ml of pyridine. The reaction mixture is stirred at ambient temperature for 20 hours. It is then evaporated to dryness, slightly alkalinised with diluted ammonia and dissolved in ethyl acetate. The organic phase is washed with water and a saturated solution of sodium bicarbonate, it is separated and dried with anhydrous sodium sulphate. The organic solution is evaporated to dryness and the resulting solid is repeatedly washed with ethyl ether, to yield 5.5 g (82%) of N-[3-(2-dimethylaminoethyl)-1*H* indol-5-yl]-5-chloro-3-methylbenzo[b]thiophene-2-sulphonamide as a solid with m.p. = 226-227°C.

### METHOD Bc

### Example 26c:

### Preparation of N-[3-(2-diethylaminoethyl)-1H-indol-5-yl]-N-ethyl-naphthalene-2-sulphonamide.

To a mixture of 285 mg (0.7 mMol) of N-[3-(2-diethylaminoethyl)-1H-indol-5yl]naphthalene-2-sulphonamide (example 17) and 80 mg (0.7 mmol) of potassium t-butoxide in 3 ml of DMSO are stirred for 30 minutes at ambient temperature.

Then are added 105 mg (0.7 mMol) of ethyl iodide and left with stirring for 3 hours. Water is added and is extracted with ethyl acetate. The organic solution is evaporated to dryness and the resulting crude is purified by chromatography on silica gel, using as an eluent mixtures of methylene chloride / methanol /ammonia, yielding N-[3-(2-diethylaminoethyl)-1*H-*indol-5-yl]-N-ethylnaphthalene-2-sulphonamide as a solid with m.p. = 49-50°C.

### METHOD Cc

### Example 18c:

### Preparation of N-[3-(1-methyl-1,2,3,6-tetrahydropyridin-4-yl)-1H-indol-5-yl]naphthalene-1-sulphonamide.

To a solution of 712 mg (13.2 mmol) of sodium methoxide in 100 ml of methanol are added 850 mg (2.64 mmol) of N-[1*H-*indol-5-yl]naphthalene-1-sulphonamide followed by 596 mg (5.28 mmol) of 1-methyl-4-piperidone and the resulting solution is heated to reflux for 48 hours. The reaction mixture is concentrated at reduced pressure and the residue obtained is purified by chromatography over silica gel, using as eluent mixtures of methylene chloride/ methanol /ammonia, to yield 573 mg (52%) of N-[3-(1-methyl-1,2,3,6-tetrahydropyridin-4-yl)-1*H-*indol-5-yl]naphthalene-1-sulphonamide as a solid with m.p. = 244-245°C.

### METHOD Dc

### Example 12c:

### Preparation of N-[3-(1-methyl-piperidin-4-yl)-1H-indol-5-yl]naphthalene-1-sulphonamide.

To a solution of 417 mg (1 mMol) of N-[3-(1-methyl-1,2,3,6-tetrahydropyridin-4-yl)-1*H*-indol-5-yl]naphthalene-1-sulphonamide in 50 ml of methanol are added 100 mg of 5% palladium on carbon. The mixture is hydrogenated at ambient temperature at an initial hydrogen pressure of 3 atmospheres for 20 hours. The reaction mixture is filtered and the filtrate is concentrated at reduced pressure to provide a crude that is suspended in ethyl ether, yielding 272 mg (65%) of N-[3-(1-methyl-piperidin-4-yl)-1H-indol-5-yl]naphthalene-1-sulphonamide as a solid with m.p.= 254-256°C

### METHOD Ec

### Example 3c:

### Preparation of N-[3-(2-diethylaminoethyl)-1H-indol-5-yl]naphthalene-1-sulphonamide hydrochloride.

1.05 g (2.5 mMol) of N-[3-(2-diethylaminoethyl)-1*H*-indol-5-yl]naphthalene-1-sulphonamide (example 2) are dissolved in 10 ml of ethanol and 0.6 ml are added of a 4.2 N solution of hydrochloric acid in ethanol. It is allowed to crystallise at ambient temperature. N-[3-(2-diethylaminoethyl)-1*H*-indol-5-yl]naphthalene-1-sulphonamide hydrochloride is obtained as a solid with m.p.= 255-257°C.

The melting point and spectroscopic data for identifying some of the compounds used according to the present invention are shown in the following table:

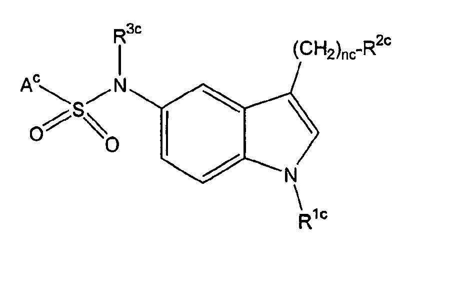

| Ex | R_{1c} | R_{2c} | nc | R_{3c} | A^{c} | Salt | m.p. °C | IR cm⁻¹ | ¹H-RMN (300 MHz), δ (solvent) |
|---|---|---|---|---|---|---|---|---|---|
| 1c | H | (CH₃CH₂)₂N- | 2 | H | 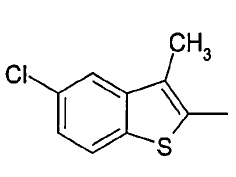 | - | 170-173 | 3387, 2970, 2931, 1466, 1236, 1158, 1107, 1080, 993, 862, 805, 657,565. | 0.88(t, 6H, J=7.1 Hz); 2.28(s, 3H); 2.30-2.46(m, 6H); 2.58(m, 2H); 6.85(dd, 1H, J=8.6, 2.0 Hz); 7.10(m, 2H); 7.20(d, 1H, J=8.6 Hz); 7.50(dd, 1H, J=8.7, 2.0 Hz); 7.90(d, 1H, J=2.0 Hz); 7.98(d, 1H, J=8.7 Hz); 10.10 (bb, 1 H); 10.80(s, 1 H). (DMSO-d6) |
| 2c | H | (CH₃CH₂)₂N- | 2 | H | 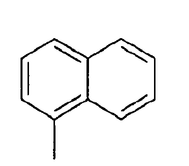 | - | 170 | 3451, 3337, 2972, 1466, 1319, 1237, 1157,1132, 1091, 991, 770, 675, 583, 481. | 3.90(t, 6H, J=7.1 Hz); 2.33-2,55(m, 8H); 6.69(dd, 1H, J=8.7, 1.8 Hz); 6, 95(s, 1 H); 7,02(d, 1 H, J=1,8 Hz); 7.05(d, 1H, J=8.7 Hz); 7.47(t, 1 H, J=7.7 Hz); 7.63(m, 1H); 7.70(m, 1H); 8.01 (m, 2H); 8.12(d, 1H, J=7.5 Hz); 8.77(d, 1H, J=8.1 Hz); 10.10(bb, 1H); 10,66(s, 1 H) (DMSO-d6) |
| 3c | H | (CH₃CH₂)₂N- | 2 | H | 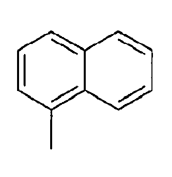 | HCl | 255-257 | 3378, 3065, 2558, 2489, 1460, 1317, 1162,1143, 1131, 811, 687, 602, 588. | 1.22(t, 6H, J=7.2 Hz); 2.91-3.18(m, 8H); 6.65(d, 1H, J=8.6 Hz); 7.08(d, 1H, J=8.6 Hz); 7.17(s, 1 H); 7.20(d, 1 H, J=1.8 Hz); 7.54(t, 1H, J=7.8 Hz); 7.63(m, 1H); 7.70(m, 1H); 8.03(d, 1H, J=7.8 Hz); 8.08(d, 1H, J=7.1 Hz); 8.14(d, 1H, J=8.2 Hz); 8.79(d, 1H, J=8.4 Hz); 10.26(s, 1H); 10.90(bb, 1 H); 11.01 (s, 1 H). (DMSO-d6) |
| 4c | H | (CH₃CH₂)₂N- | 2 | H | 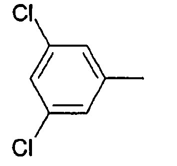 | - | 168-170 | 3309, 3047, 2974, 1566, 1467, 1235, 1167,1143, 1116, 1001, 910, 799, 672, 587. | 0.95(t, 6H, J=7.1 Hz); 2.44-2.58(m, 6H); 2.66(m 2H); 6.79(dd, 1H, J=8.6 1.7 Hz); 7.08(d, 1H, J=0.9 Hz); 7.13 (d, 1H , J=1.7 Hz); 7.2 3(d, 1H, J=8.6 Hz) 7.58 (m, 2H); 7.87(m, 1 H); 9,95(bb, 1H); 10.82(s, 1 H). (DMSO-d6) |
| 5c | H | (CH₃CH₂)₂N- | 2 | H | 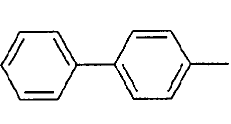 | - | 161-163 | 3387, 2971, 1323, 1157, 1095, 765, 670, 590 | 0.89(t, 6H, J=7.1 Hz); 2.32-2.55(m, 6H); 2.62(m, 2H); 6.85(d, 1H, J=8.6 Hz); 7.08(d, 1H, J=2.0 Hz); 7.13(s, 1H); 7.18(d, 1H, J=8.6 Hz); 7.33-750 (m, 3H); 7.64(d, 2H, J=7.5 Hz); 7.72(sys AB, 2H, J=8.6 Hz); 7,78(sys AB, 2H, J=8.6 Hz); 9.80(bb, 1H); 10.75(s, 1H). (DMSO-d6) |
| 6c | H | (CH₃CH₂)₂N- | 2 | H | 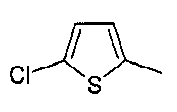 | - | 180-181 | 3375, 2978, 1467, 1417, 1236, 1212, 1115, 994, 624. | 0.96(t, 6H, J=7.1 Hz); 2.52(m, 4H); 2.57(m, 2H); 2.66(m, 2H); 6.83(dd, 1H, J=8.6, 1.9 Hz); 7.11(d, 1H, J=4.0 Hz); 7.14(d, 1 H, J=1.9 Hz); 7.17(d, 1 H, J=1.9 Hz); 7.20-7.24(m, 2H); 10.01(bb, 1H); 10.81(s, 1H). (DMSO-d6) |
| 7c | H | (CH₃)₂N- | 2 | H | 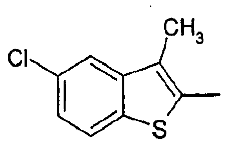 | - | 226-227 | 3422, 3238, 1332,1155, 1114, 1079, 986, 861, 803, 655, 564. | 2.04(s, 6H); 2.23(m, 2H); 2.28(s, 3H), 2.59(m, 2H); 6.83(dd, 1H, J=8.4, 1.5 Hz); 7,09 (s, 2H); 7.19(d, 1 H, J=8.4 Hz); 7.49(dd, 1 H, J=8.7, 1.6 Hz); 7.91 (d, 1H, J=1.6 Hz); 7.99(d, 1H, J=8.7 Hz); 10,13(bb, 1H), 10,79 (s, 1H) (DMSO-d6) |
| 8c | H | (CH₃)₂N- | 2 | H | 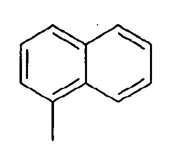 | - | 203-205 | 3357, 1475, 1282, 1157, 1127,990,957, 809, 773, 613, 587, 557, 498. | 2.09(s, 6H); 2.21 (m, 2H); 2.54(m, 2H); 6.69(dd, 1H, J=8.6, 1.7 Hz); 6,94 (s, 1H); 7.03 (s, 1H); 7.06(d, 1H, J=8.1 Hz); 7.49(t, 1H, J=7.8 Hz); 7.64(m, 1H); 7.71(m, 1 H); 8.02 (m, 2H); 8.13(d, 1H, J=8.1 Hz); 8.79(d, 1 H, J=8.4 Hz); 10.10(bb, 1H); 10.68(s, 1H) (DMSO-d6) |
| 9c | H | (CH₃)₂N- | 2 | H | 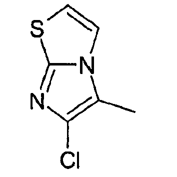 | - | 215 (desc) | 3247, 3094, 1467, 1272, 1261, 1230, 625 | 2,17(s, 6 H); 2.36(m, 2 H); 2.65(m, 2 H); 6.77(dd, J=8.6, 1.7 Hz, 1 H); 7.07(s, 1H); 7.09(s, 1 H); 7.18(d, J=8.6 Hz, 1 H); 7.51 (d, J=4.5 Hz, 1 H); 7.81(d, J=4.5 Hz, 1 H); 10.80 (s, 1H). (DMSO-d6). |
| 10c | H | 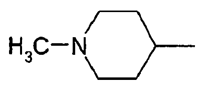 | 0 | H | 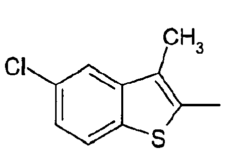 | - | 250 (desc) | 3407, 2390, 1466, 1334, 1156, 113, 1080, 651, 565. | 1.53-1.80(m, 4H); 2.26(s, 3H); 2.39-2.71 (m, 6H); 3.02(d, 2H, J=8.8 Hz); 6.76(d, 1H, J=8.8 Hz); 7.05(s, 1H); 7.11 (s, 1H); 7.19(d, 1H, J=8.8 Hz); 7.51 (d, 1H, J=8.7 Hz); 7.91 (s, 1H); 8.00(d, 1H, J=8.7 Hz); 10.10(bb, 1H);10.90(s, 1 H). (DMSO-d6) |
| 11c | H | 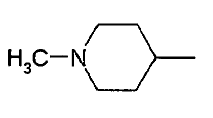 | 0 | H | 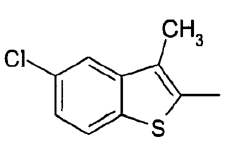 | HCl | 220 (desc) | 3423,3214, 3043, 2942, 2688, 1464, 1317,1149, 1114, 1080, 748, 670, 646 | 1.75-1.92(m, 4H); 2.31 (s, 3H); 2.66(s, 3H); 2.80(m, 1H); 2.95(m, 2H); 3.24(d, 2H, J=11.4 Hz); 6.76(d, 1H, J=8.7 Hz); 7.07(s, 1 H); 7.19(m, 2H); 7.50(d, 1H, J=8.6 Hz); 7.93(s, 1Hz); 8.01 (d, 1H, J=8.6 Hz); 8,34 (s, 1 H); 10.90(bb, 1 H); 11.01(s, 1H). (DMSO-d6) |
| 12c | H | 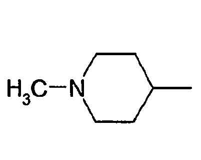 | 0 | H | 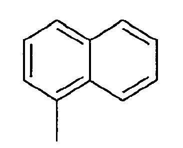 | - | 254-256 | 3343, 2938, 2929, 1470 1154, 1121, 1108, 988, 947, 805, 769, 589. | 1.49(m, 2H); 1.61(m, 2H); 2.14(m, 2H); 2.30(s, 3H); 2.40(m, 1 H); 2,90 (d, 2H, J=10.6 Hz); 6.65(d, 1H, J=8.6 Hz); 6.90(s, 1 H); 6.96(s, 1 H); 7.05(d, 1H, J=8.6Hz); 7.46(dt, 1H, J=7.51, 1.83 Hz); 7.64(m, 1H); 7.71(m, 1H); 7.99(d, 1H, J=8.6 Hz); 8.03(d, 1H, J=8.6Hz); 8.12(d, 1H, J=8.2 Hz); 8.77(d, 1H, J=8.6 Hz); 10.07(bb, 1H); 10.71 (s, 1H). (DMSO-d6) |
| 13c | H | 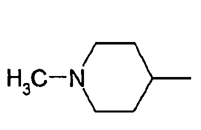 | 0 | H | 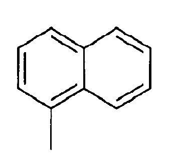 | HCl | 212 (desc) | 3423, 3269, 3114, 2955, 2733, 1469, 1321,1155, 1133,947,769. | 1.80(m, 4H); 2.74(m, 4H); 3.04(m, 2H); 3.39(m, 2H); 6.63(d, 1H, J=8.6 Hz); 7.00(s, 2H); 7.08(d, 1H, J=8.6 Hz);7.49(t, 1H, J=7.7 Hz); 7.60-7.77(m, 2H); 8.04(d, 2H, J=7.5 Hz) 8.13(d, 1H, J=8.2 Hz); 8.79(d, 1H, J=8.2 Hz); 10.16(s, 1H); 10.66(bb, 1H); 10.88(s, 1 H). (DMSO-d6) |
| 14c | H | 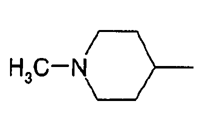 | 0 | H | 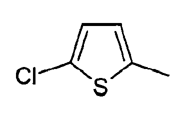 | - | 284 (desc) | 3371, 2943, 1468, 1410, 1324, 1148, 993, 604. | 1.62(m, 2H); 1.78(d, 2H, J=11.7 Hz); 1.99(m, 2H); 2.18(s, 3H); 2.55(m, 1 H); 2.84(d, 2H, J=10.6 Hz); 6.81 (d, 1H, J=8.6 Hz); 7.07(s, 1H); 7.13(m 1H); 7.16(s, 1H); 7.20-7.26 (m, 1H); 9.90 (bb, 1 H); 10.83 (s, 1 H). (DMSO-d6) |
| 15c | H | 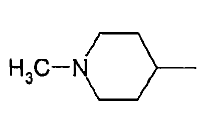 | 0 | H | 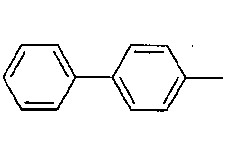 | - | 247-248 | 3361, 2936, 1318, 1155, 1095, 767, 670, 587. | 1.52(s, 2H); 1.67(m, 2H); 1.85(m, 2H) 2.08(s, 3H); 2.44(m, 1H); 2.67(d, 2H, 10.25Hz); 6.83(d, 1H, J=8.4 Hz); 7.01(s, 1H); 7,03(s, 1H); 7.19(d, 1H, J=8.4 Hz); 7.35-7.50(m, 3H); 7.63-7.73(m, 4H); 7.79(sys AB, 2H, J=7.6 Hz); 9.71(bb, 1 H); 10.76(s, 1H) (DMSO-d6). |
| 16c | H | 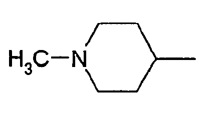 | 0 | H | 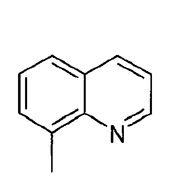 | - | 280 (desc) | 3398, 3257, 2933, 1161, 1143, 789, 589. | 1.25-1.52(m, 4 H); 1.85(m, 2 H); 2.18(s, 3 H); 2.27(m, 1 H); 2.74 (d, J=11.4 Hz, 2 H); 6.72(dd, J=8.6, 2.0 Hz, 1 H); 3.83(d, J=1.5 Hz, 1 H); 6.90(d, J=2.0 Hz, 1 H); 7.02(d, J=8.6 Hz, 1 H); 7.57(m, 1 H); 7.74(dd, J=8.4, 4.3 Hz, 1H); 8.12 (dd, J=7.3, 1.3 Hz, 1 H); 3.19(dd. J=8.2, 1.3 Hz, 1 H); 8.52(dd, J=8.4, 1.7 Hz, 1 H); 9.21 (dd, J=4.3, 1.7 Hz, 1H); 9.36(s, 1 H); 10.64(s, 1 H). (DMSO-d6). |
| 17c | H | (CH₃CH₂)₂N- | 2 | H | 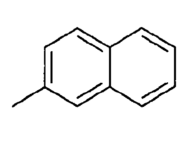 | - | 172-173 | 3199, 2970, 2930, 2870, 1327, 1153, 1130,1110, 1075, 956, 676, 658, 551, 476. | 0.87(t, J=7.1 Hz, 6 H); 2.39(m, 6 H); 2.55 (m, 2 H); ); 6.82(d, J=8.6 Hz, 1 H); 7.05 (s, 1 H); 7.09(s, 1 H); 7.13(d, J=8.6 Hz, 1 H); 7.60(m, 2 H); 7.73 (d, J=8.6 Hz, 1 H); 7.95(d, J=7.9 Hz, 1 H) 8.01 (m, 2 H); 8.26 (s, 1 H); 9.86(bb, 1 H); 10.71(s, 1 H). (DMSO-d6). |
| 18c | H | 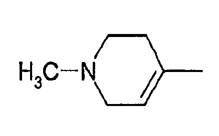 | 0 | H | 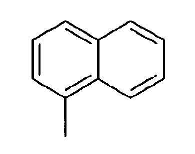 | - | 244-245 (desc) | 3346, 2943, 1474, 1283, 1261,1156, 1123, 801, 771, 589, 503. | 2.25(s, 3 H); 2.31(m, 2 H); 2.46(m, 2 H); 2.90(m, 2 H); 5.34(s, 1 H); 6.78(dd, J=8.6, 2.0 Hz, 1 H); 7.09(d, J=1.5 Hz, 1 H); 7.14 (d, J=8.6 Hz, 1 H); 7.25 (d, J=2.0 Hz, 1 H); 7.49(t, J=7.8 Hz, 1 H); 7.66(m, 1 H); 7.75(m, 1 H); 8.04(m, 2H); 8.14(d, J=8.2 Hz, 1 H); 8.83(d, J=8.6.Hz, 1 H); 10.14(bb, 1 H); 11.03(s, 1 H). (DMSO-d6). |
| 19c | H | 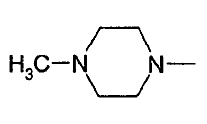 | 1 | H | 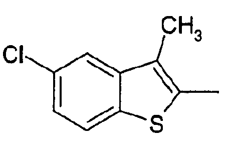 | - | 230 (desc) | 2796, 1452, 1316,1149, 1114, 1080, 1001,810,646, 559. | 1.80-2.26(m, 8 H); 2.04(s, 3 H); 2.30(s, 3 H); 3.41(s, 2 H); 6.89(dd, J=8.6, 1.56 Hz, 1 H); 7.16(s, 1 H); 7.22(d, J=8.6 Hz, 1 H); 7.29(s, 1 H); 7.49(dd, J=8.7, 1.7 Hz, 1 H); 7.90(d, J=1.7 Hz, 1 H); 7.98(d, J=8.7 Hz, 1 H); 10.13(bb, 1H): 10.93(s, 1H). (DMSO-d6). |
| 20c | H | (CH₃)₂N- | 2 | H | 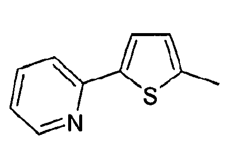 | - | 209-211 | 3377, 2951,2798, 1469,1429, 1321,1158, 777,594. | 2.05(s, 6 H); 2.32(m, 2 H); 2.65(m, 2 H); 6.86(dd, J=8.6, 1.8 Hz, 1 H); 7.10(d, J=1.8 Hz, 1 H); 7.18(d, J=1.8 Hz, 1H): 7.21 (d, J=8.6 Hz, 1 H); 7.32(dd, J=7.5, 4.6 Hz, 1 H); 7.36(d, J=3.9 Hz, 1 H); 7.71(d, J=3.9 Hz, 1 H); 7.83(m, 1 H); 7.93(m, 1 H); 8.49(d, J=4.6 Hz, 1 H); 9.97(bb, 1 H); 10.79(s, 1 H). (DMSO-d6). |
| 21c | H | (CH₃)₂N- | 2 | H | 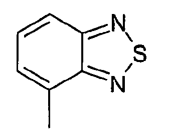 | - | 192 | 3321,2949, 1474, 1327, 1152,1138, 1104, 981, 614. | 2.10(s, 6 H); 2.21 (m, 2 H); 2.56(m, 2 H); 6.72(d, J=8.6 Hz, 1 H); 6.96(s, 1H); 7.03 (s, 1 H); 7.07(d, J=8.6 Hz, 1 H); 7.70(m, 1 H); 8.07(d, J=7.0 Hz, 1 H); 8.29(d, J=8.8 Hz, 1 H); 10.14(bb, 1 H); 10.69(s, 1 H). (DMSO-d6). |
| 22c | H | (CH₃)₂N- | 2 | H | 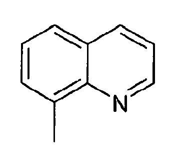 | - | 250 (desc) | 3252, 2857, 1459,1426, 1333,1161, 1144, 789, 680, 589. | 2.07(s, 6 H); 2.16(m, 2 H); 2.51(m, 2 H); 6.73(dd, J=8.6, 1.8 Hz, 1 H); 6.94(s, 1 H) 6.99(s, 1 H); 7.02(d, J=8.6 Hz, 1 H); 7.59(t, J=7.8 Hz, 1 H); 7.73(dd, J=8.4, 4.1 Hz, 1 H); 8.18(m, 2 H); 8.50(dd, J=8.4, 1.5 Hz, 1 H); 9.20(dd, J=4.1, 1.5 Hz, 1 H); 9.45(bb, 1 H); 10.64(s, 1H). (DMSO-d6). |
| 23c | H | (CH₃)₂N- | 2 | H | 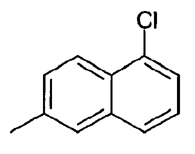 | - | 230-240 (desc) | 3404, 2944, 2918,2855, 1465, 1332, 1157, 1140, 1080,650,639, 526. | 2.01(s, 6 H); 2.18(m, 2 H); 2.57(m, 2 H); 6.81 (dd, J=8.6, 1.7 Hz, 1 H); 7.02 (s, 1H); 7.05(d, J=1.7 Hz, 1 H); 7.15(d, J=8.6 Hz, 1 H); 7.57(m, 1 H); 7.82(d, J=7.5 Hz, 1 H); 7.91(d, J=8.9 Hz, 1 H); 8.06(d, J=8.2 Hz, 1 H); 8.29(d, J=8.9 Hz, 1 H); 8.35(s, 1 H); 9.94(bb, 1 H); 10.74(s, 1 H). (DMSO-d6). |
| 24c | H | (CH₃)₂N- | 2 | H | 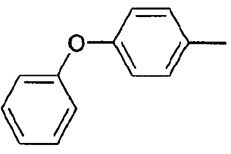 | - | 152-154 | 3232, 2862, 2827, 2785, 1583, 1488, 1333, 1248, 1155, 1091, 755, 693, 571, 541. | 2.16(s, 6 H); 2.37(m, 2 H); 2.66 (m, 2 H); 6.80 (d, J=8.6 Hz, 1 H); 6.96-7.12 (m, 6 H); 7.14-7.25 (m, 2 H); 7.41(m, 2 H); 7.64 (dd, J=8.5, 1.9 Hz, 2 H); 9.69(bb, 1 H); 10.75 (s, 1 H). (DMSO-d6). |
| 25c | H | (CH₃)₂N- | 2 | H | 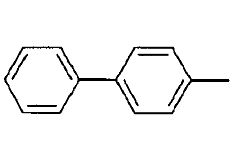 | - | 184-186 | 3451, 3388, 2950, 2775, 1466,1322, 1159, 1095, 763, 670, 591, | 2.08(s, 6 H); 2.32(m, 2 H); 2.64(m, 2 H); 6.83(dd, J=8.6, 1.9 Hz, 1 H); 7.08(d, J=2.0 Hz, 1 H); 7.11(d, J=1.9 Hz, 1 H); 7.17(d, J=8.6 Hz, 1 H); 7.34-7.50(m, 3H); 7.66(d, J=7.5 Hz, 2 H); 7.72(AB sys, J=8.6 Hz, 2 H); 7.79(AB sys, J=8.6 Hz, 2 H); 9.79(s, 1 H); 10.75(s, 1 H). (DMSO-d6). |
| 26c | H | (CH₃CH₂)₂N- | 2 | Et | 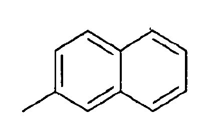 | - | 49-50 | 3386,2970, 2931, 1474, 1337, 1167, 1151,1130, 1073, 661,550 | 0.82(t, J=7.0 Hz, 6 H); 0.98(t, J=7.0 Hz, 3 H); 2.37(q, J=7.0 Hz, 4 H); 2.49(m, 2 H); 2.54(m, 2H); 3.66(q, J=7.1 Hz, 2H); 6.73 (dd, J=8.61, 1.6 Hz, 1 H); 6.98(s, 1H); 7.17 (d, J=1.6 Hz, 1 H); 7.26(d, J=8.61 Hz, 1 H); 7.56-7.72 (m, 3 H); 7.99-8.11(m, 3H); 8.26 (s, 1 H); 10.97(s, 1 H). (DMSO-d6). |

| Ex | R₁ | R₂ | n | R₃ | A | Salt | m.p. °C | IR cm⁻¹ | ¹H-RMN (300 MHz),δ (solvent) |
|---|---|---|---|---|---|---|---|---|---|
| 27c | H | 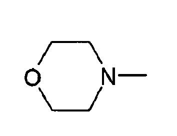 | 2 | H | 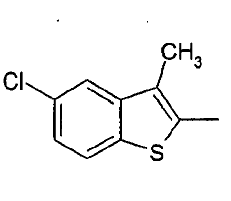 | - | 200-201 | 3366, 2951, 2816, 1460, 1421, 1319, 1283, 1157, 1114, 1078, 865, 651, 561 | 2.25(m, 6H); 2.27(s, 3H); 2.62(t, J=7.9 Hz, 2H); 3.52(m, 4H); 6.84(d, J=8.2 Hz, 1 H); 7.06(s, 1H); 7.10(s, 1 H); 7.20(d, J=8.6 Hz, 1 H); 7.50(d, J=8.6 Hz, 1H); 7.92(s, 1H); 8.00 (d, J=8.6 Hz, 1 H); 10.13(s, 1 H); 10.80(s, 1H). (DMSO-d6) |
| 28c | H | 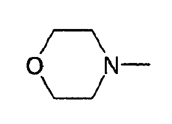 | 2 | H | 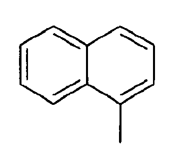 | - | 218-220 | 3389, 3152, 2916, 2819, 1466, 1313, 1157, 1129, 1108, 771, 587 | 2.30(m, 6H); 2.56(m, 2H); 3.56(m, 4H); 6.69(d, J=8.4 Hz, 1H); 6.93(s, 1H); 7.06(m, 2H); 7.48(t, J=7.3 Hz, 1 H); 7.67(m, 2H); 8.02(m, 2H); 8.13 (d, J=8.1 Hz, 1H); 8.78 (d, J=8.1 Hz, 1H); 10.10(s, 1H); 10.68(s, 1H). (DMSO-d6) |
| 29c | H | (CH₃CH₂)N- | 2 | CH₃ | 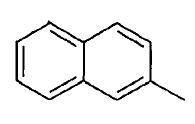 | - | 134-136 | 2968, 2930, 1488, 1329, 1159, 1131, 1074, 660, 550 | J.98(t, J=7.1 Hz, 6H); 2.55(m, 6H); 2.70(m, 2H); 3.67(s, 3H); 6.84 (s. 1H); 6.93(dd, J=8.6, 2 Hz, 1H); 7.10(d, J=8.7 Hz, 1H); 7.18(d, J=1.7 Hz, 1 H); 7.26(s, 1H); 7.57 (m, 2H); 7.67(dd, J=8.7, 1.8 Hz, 1H); 7.84(m, 3H); 8.27(d, J= 1.7 Hz, 1H). (DMSO-d6) |
| 30c | H | (CH₃)₂N- | 1 | H | 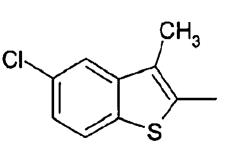 | - | 148-152 | 3398, 2930, 1467, 1158, 1113, 1079,861, 803, 651, 561 | 1.89(m, 6H); 2.29(s, 3H); 2.48(s, 2H); 6.83(m, 1 H); 7.18(m, 3H); 7.50(m, 1 H); 7.91(m, 1 H); 8.00 (m, 1 H); 10.13(b, 1 H); 10.92(s, 1 H). (DMSO-d6) |
| 31c | H | (CH₃CH₂CH₂)₂N- | 2 | H | 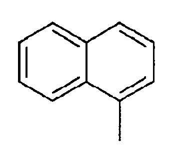 | - | 76-80 | 3399, 2959, 2931, 1466, 1159, 1132, 802, 770, 588 | 0.82(t, J=6.7 Hz, 6H); 1.34(q, J=6,71 Hz, 4H); 2.31(m, 4H); 2.40(m, 2H); 2.52(m, 2H); 6.69(d, J=8.6 Hz, 1H); 7.04(m. 3H); 7.47(m, 1 H); 7.66(m, 2H); 8.02(m, 2H); 8.11(d, J=8.1 Hz, 1H); 8.78(d, J=8.4 Hz, 1 H); 10.12(s, 1 H); 10.67(s, 1 H). (DMSO-d6) |
| 32c | H | (CH₃CH₂CH₂)₂N- | 2 | H | 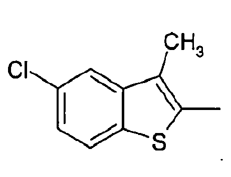 | - | 90-95 | 3406, 2959, 2932, 2872, 1466, 1157, 1079,861, 652,561 | 0.80(t, J=7.3 Hz, 6H); 1.31(q, J=7.3 Hz, 4H); 2.26(m, 7H); 2.38(m, 2H); 2.56(m, 2H); 6.83(dd, J=8.4, 1.8 Hz, 1 H); 7.08(s, 2H); 7.20(d, J=8.6 Hz, 1H); 7.50(dd, J=8.6, 2.0 Hz, 1 H); 7.90(d, J=2.0 Hz, 1 H); 7.99(d, J=8.6 Hz, 1 H); 10.12(b, 1 H); 10.79(s, 1 H). (DMSO-d6) |
| 33c | H | (CH₃CH₂CH₂CH₂)₂N- | 2 | H | 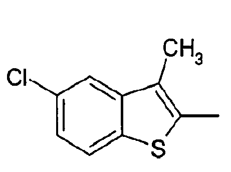 | - | 79-80 | 3398, 2956, 2930, 2870, 1466, 1158, 1080, 862, 801, 653, 562 | 0.84(t, J=6.8 Hz, 6H); 1.24(m, 8H); 2.26(s, 3H); 2.28(m, 4H); 2.39(m, 2H); 2.57(m, 2H); 6.82(dd, J=8.6, 1.9 Hz, 1 H); 7.09(d, J=1.8 Hz, 2H); 7.18(d, J=8.6 Hz, 1H); 7.50(dd, J=8.6, 1.9 Hz, 1 H); 7.89(d, J=1.8 Hz, 1 H); 7.98(d, J=8.6 Hz, 1H); 10.14(b, 1H); 10.78(s, 1H). (DMSO-d6) |
| 34c | H | (CH₃CH₂CH₂CH₂)₂N- | 2 | H | 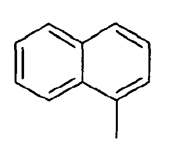 | - | 111-113 | 3291, 2955, 2926, 2870. 1327, 1158, 1136, 772, 676, 611, 585 | 0.86(t, J=7.0 Hz, 6H); 1.29(m, 8H); 2.35(m, 4H); 2.41(m, 2H); 2.53(m, 2H); 6.67(dd, J=8.5, 1.9 Hz, 1H); 7.09(m, 3H); 7.48(t, J=7.9 Hz, 1 H); 7.68(m, 2H); 8.01 (s, 1 H); 8.04(s, 1H); 8.12(d, J=8.2 Hz, 1H); 8.78(d, J=8.2 Hz, 1H); 10.13(s, 1H); 10.67(s, 1H). (DMSO-d6) |
| 35c | H | (CH₃CH₂)₂N- | 2 | H | 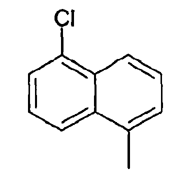 | - | 154-156 | 3402, 2978, 1471, 1285, 1162, 1135, 1018, 780, 629, 606 | 0.88(t, J=6.7 Hz, 6H); 2.41 (m, 6H); 2.49(m, 2H); 6.71 (d, J=8.1 Hz, 1 H); 6.88(s, 1H); 7.07(m, 2H); 7.66(m, 2H); 7.84(d, J=7.0 Hz, 1H); 8.09(d, J=7.0 Hz, 1H); 8.41 (d, J=8.2 Hz, 1H); 8.79(d, J=8.6 Hz, 1H); 10.17(b, 1H); 10.71(s, 1H). (DMSO-d6) |
| 36c | H | (CH₃CH2)₂N- | 2 | H | 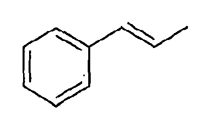 | - | 125-130 | 3404, 2972, 1473, 1319, 1142,967, 745,541 | 0.94(t, J=7.1 Hz, 6H); 2.50(q, J=7.1 Hz, 4H); 2.59(m, 2H); 2.68(m, 2H); 6.94(dd, J=8.6, 1.8 Hz, 1H); 7.26(m, 8H); 7.59(m, 2H); 9.54(b, 1 H); 10.77(s, 1 H). (DMSO-d6) |
| 37c | H | 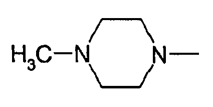 | 1 | H | 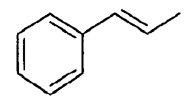 | - | 203 (desc) | 2809. 1340, 1150,746, 542 | 2.06(s, 3H); 2.22(m, 6H); 3.36(m 2H); 3.49 (s, 2H); 6.95(dd, J=8.6, 1.8 Hz, 1H); 7.18(s, 2H); 7.24(m, 2H); 7.37(m, 3H); 7.45(d, J=1.8 Hz, 1H); 7.61 (m, 2H); 9.53(s 1H); 10.90(s, 1H). (DMSO-d6) |
| 38c | H | 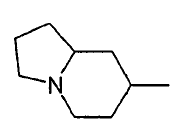 | 0 | H | 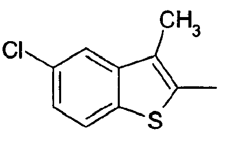 | - | 142-144 | 3413, 2929, 1157, 1113, 1080,862, 651, 564 | 1.12(m, 3H); 1.81(m, 9H); 2.22(s, 3H); 2.93(m, 2H); 6.84(dd, J=8.5, 1.7 Hz, 1 H); 6.99(s, 1H); 7.03(s, 1H); 7,20(d, J=8.6 Hz, 1H); 7.52(dd, J=8.6, 2.0 Hz, 1H); 7.90(d, J=1.7 Hz, 1 H); 8.00(d, J=8.6 Hz, 1H); 10.01(b, 1H); 10.61 (s, 1H). (DMSO-d6) |
| 39c | H | (CH₃CH₂)₂N- | 2 | H | 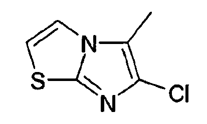 | - | 197-198 | 1 3338, 1466, 1270, 1237, 117, 986, 626 | 0.96(t, J=7.1 Hz, 6H); 2.53(m, 6H); 2.63(m, 2H); 6.78(dd, J=8.5, 1.6 Hz, 1H); 7.10(s, 2H); 7.18(d, J=8.6 Hz, 1H); 7.51 (d, J=4.6 Hz, 1 H); 7.80(d, J=4.6 Hz, 1 H); 10.78(s, 1H). (DMSO-d6) |
| 40c | H | 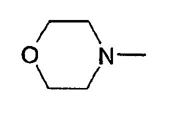 | 2 | H | 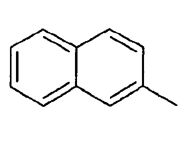 | - | 85-90 | 3399, 3257, 2920, 2855, 2814, 1460, 1330, 1157, 1131, 1113, 1074, 659, 551, 477 | 2.27(m, 6H); 2.61(t, J=7.9 Hz, 2H); 3.52(t, J=4.6 Hz, 4H); 6.82(dd, J=8.6, 2.0 Hz, 1H); 7.06(s, 1H); 7.07(s, 1H); 7.15(d, J=8.6 Hz, 1H); 7.61 (m, 2H); 7.74(dd, J=8.8, 1.8 Hz, 1H); 7.96(d, J=8.1 Hz, 1H): 8.03(m, 2H); 8.27 (s, 1H); 9.87(s, 1H); 10.74(s, 1 H). (DMSO-d6) |
| 41c | H | 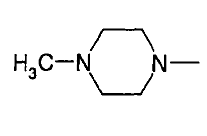 | 1 | H | 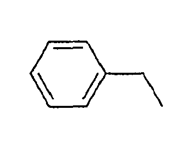 | - | 99-102 | 3398, 2934, 2806, 1458, 1331, 1284, 1153, 1127,700, 542 | 2.11(s, 3H); 2.32(m, 6H); 3.35(m, 2H); 3.56(s, 2H); 4.29(s, 2H); 6.98(d, J=8.2 Hz, 1H); 7.29(m, 7H); 7.53(s, 1H); 9.40(s, 1H); 10.94(s, 1H). (DMSO-d6) |
| 42c | H | (CH₃CH₂)₂N- | 3 | H | 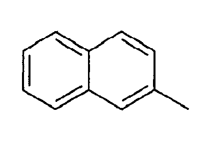 | - | 128-130 | 3259, 2973, 2939, 2827, 1468, 1332, 1159, 1131, 1075, 670, 555 | 0.86(t, J=7.0 Hz, 6H); 1.51 (t, J=6.9 Hz, 2H); 2.27(t, J=6.9 Hz, 2H); 2.35(q, J=7.0 Hz, 4H); 2.46(m, 2H); 6.77(d, J=8.6Hz, 1H); 7.00(s, 1H); 7.10(m, 2H); 7.60(m, 2H); 7.72(d, J=8.8 Hz, 1H); 7.95(d, J=7.9 Hz, 1 H); 8.02(m, 2H); 8.26(s, 1 H); 9.86 (b, 1 H); 10.67(s, 1H). (DMSO-d6) |
| 43c | H | (CH₃CH₂)₂N- | 3 | H | 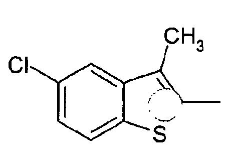 | - | 156-158 | 3247, 2969, 2938, 1467, 1340, 1159, 1113, 1080, 862, 666, 558 | 0.88(t, J=7.0 Hz, 6H); 1.52(m, 2H); 2.29(m, 5H); 2,37(q, J=7.0 Hz, 4H); 2.47(m, 2H); 3.81(dd, J=8.6, 1.5 Hz, 1H); 7.06(d, J=1.6 Hz, 1 H); 7.12(d, J=1.5 Hz, 1 H); 7.18(d, J=8.6 Hz, 1H); 7.51 (dd, J=8.6, 2.0 Hz, 1H); ); 7.91(d, J=2.0 Hz, 1 H); 7.99(d, J=8.6 Hz, 1H);10.06(b, 1 H); 10.76(s, 1 H). (DMSO-d6) |
| 44c | H | 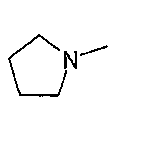 | 2 | H | 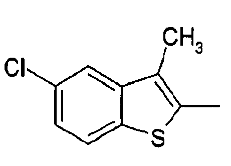 | - | 201-203 | 3386, 2929, 1466, 1157, 1106, 1080, 992, 361, 650, 564 | 1.62(m, 4H); 2.29(s, 3H); 2.30(m, 4H); 2.36(m, 2H); 2.63(m, 2H); 6.86(d, J=8.6 Hz, 1H ); 7.05(s, 1H); 7.09(s, 1H); 7.21 (dd, J=8.6, 2.2 Hz, 1H); 7.50(dd, J=8.7, 2.0 Hz, 1H): 7.92(s, 1H): 7.99(dd, J=8.7, 2.2 Hz, 1H); 10,10(b, 1H); 10.81 (s, 1 H). (DMSO-d6) |
| 45c | H | 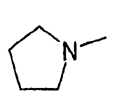 | 2 | H | 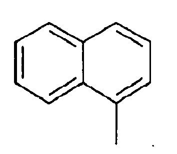 | - | 212-214 | 3354, 2964, 2812, 1466, 1201, 1157, 1124, 808, 773, 593 | 1.66(m, 4H); 2.36(m, 6H); 2.58(m, 2H); 6.71 (d, J=8.6 Hz, 1 H); 6.93(s, 1H); 7.02(s, 1H); 7.07(d, J=8.6 Hz, 1H); 7.48 (m, 1H); 7,68(m, 2H); 8.02(dd, J=7.2, 1.2 Hz, 2H); 8.12(d, J=8.2 Hz, 1 H); 8.79(d, J=8.6 Hz, 1H); 10.10(b, 1H); 10.68(s, 1H). (DMSO-d6) |
| 46c | H | 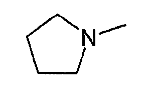 | 2 | H | 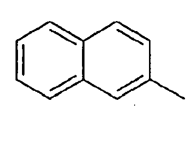 | - | 180-182 | 3375, 2968, 2821, 1467, 1323, 1313, 1146,1139, 1131, 1079, 972, 654,549 | 1.60(m, 4H); 2.26(m, 4H); 2.35(m, 2H); 2.61(m, 2H); 6.82(dd, J=8.6, 2.0 Hz, 1H); 7.05(m, 2H); 7.14(d, J=8.6 Hz, 1H); 7.61(m, 2H); 7.74(dd, J=8.6, 1.8 Hz, 1H); 7.95(d, J=7.9 Hz, 1H); 8.02(m, 2H); 8.27(s, 1H); 9.86(b, 1H); 10.72(s, 1H). (DMSO-d6) |
| 47c | H | (CH₃CH₂CH₂)₂N- | 2 | H | 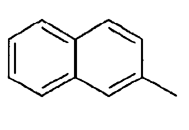 | - | 58-64 (desc) | 3398, 3255, 2958, 2931, 2872, 1466, 1330, 1156, 1130, 1074, 659, 551 | 0.79(t, J=7.3 Hz, 6H); 1.31(q, J=7.3 Hz, 4H); 2.28(t, J=7.3 Hz, 4H); 2.42(m, 2H); 2.57(m, 2H); 6.80(dd, J=8.6, 1.7 Hz, 1H); 7.04(d, J=1.7 Hz, 1 H); 7.12(m 2H); 7.60(m, 2H); 7.72(dd, J=8.6, 1.7 Hz, 1 H); 7.98(m, 3H); 8.25(s, 1H); 9.87(b, 1H); 10.70(s, 1H). (DMSO-d6) |
| 48c | H | (CH₃)₂N- | 2 | H | 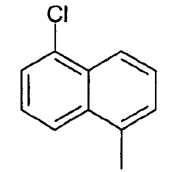 | - | 201-203 | 3369, 1473, 1161, 1125, 1017, 789, 619 | 2.06(s, 6H); 2.15(t, J=8.2 Hz, 2H); 2.52(t, J=8.2 Hz, 2H); 6.69(d, J=8.7 Hz, 1H); 6.85(s, 1 H); 7.02(s, 1 H); 7.08(d, J=8.7 Hz, 1H); 7.67(m, 2H); 7.84(d, J=7.3 Hz, 1H); 8.10(d, J=7.3 Hz, 1H); 8.41 (d, J=8.4 Hz, 1 H); 8.79(d, J=8.7 Hz, 1 H); 10.15(b, 1 H); 10.70(s, 1H). (DMSO-d6) |
| 49c | H | (CH₃)₂N- | 2 | H | 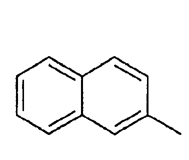 | - | 180-190 | 3399, 3255, 2943, 1466, 1330, 1156, 1131, 1075, 659, 550 | 2.03(s, 6H); 2.22(t, J=8.2 Hz, 2H); 2.58(t, J=8.2 Hz, 2H); 6.80(d, J=8.4 Hz, 1H); 7.04(s, 1 H); 7.07(s, 1 H); 7.13(d, J=8.6 Hz, 1H); 7.60(m, 2H); 7.74(d, J=8.6 Hz, 1H); 7.95(d, J=7.7 Hz, 1 H); 8.02(m, 2H); 8.26 (s,1H); 9.86(b, 1H); 10.71 (s, 1H). (DMSO-d6) |
| 50c | H | 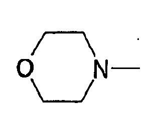 | 2 | H | 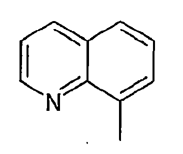 | - | 234-235 | 3400, 3279, 2913, 2852, 1464, 1420, 1315, 1163. 1118, 951, 592 | 2.29(m, 6H); 2.54(m, 2H); 3.57(m, 4H); 6.72(d, J=8.1 Hz, 1H); 7.01 (m, 3H); 7.60(t, J=7.7 Hz, 1 H); 7.74(d, J=8.4 Hz, 1 H); 8.19(m, 2H); 8.52(d, J=8.4 Hz, 1H); 9.21(s, 1H); 9.44(s, 1 H); 10.65(s, 1 H). (DMSO-d6) |
| 51c | H | 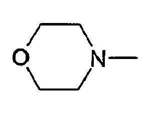 | 2 | H | 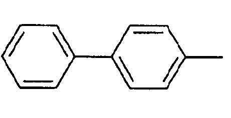 | - | 225-228 | 3340, 2857, 1479, 1324, 1153. 1116, 1094, 768, 670, 588 | 2.29(m, 6H); 2.66(m, 2H); 3.47(m, 4H); 6.84(d, J=8.6 Hz, 1H); 7.07(s, 1H); 7.09(s, 1H); 7.18(d, J=8.4 Hz, 1 H); 7.45(m, 3H); '.70(m, 4H); 7.79(m, 2H); 9.79(s, 1H); 10.77(s, 1H). (DMSO-d6) |
| 52c | H | 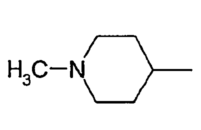 | 2 | H | 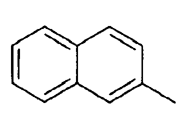 | - | 129-131 | 3367, 2924, 2852, 2799, 1465, 1311, 1154, 1130, 1077, 666, 557 | 40-1.60(m, 4H); 1.83(m, 2H); 2.14(s, 3H); 2.36(m, 1H); 2.67(d, J=11.2 Hz, 2H); 6.78(d, J=8.4 Hz, 1H); 6.97(s, 1H); 7.00(s, 1H); 7.12(d, J=8.6 Hz, 1H); 7.50-7.68(m, 2H); 7.73(d, J=9.0 Hz, 1H); 8.00(m, 3H); 8.23(s, 1H); 9.78(b, 1 H); 10.71 (s, 1H). DMSO-d6) |
| 53c | H | 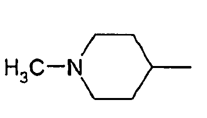 | 2 | H | 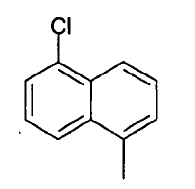 | - | 246-249 | 3329, 2940, 2916, 1470, 1158, 1125, 1110, 1015, 791, 598 | 1.35-1.47(m, 4H); 1.86(m, 2H); 2.17(s, 3H); 2.28(m, 1H); 2.76(d, J=10.6 Hz, 2H); 6 68(d, J=8.8 Hz, 1H); 6.75(s, 1H); 6.94(s, 1H); 7.08(d, J=9.0 Hz, 1H); 7.60-7.73(m, 2H); 7.85(d, J=7.1 Hz, 1 H); 8.06(d, J=7.1 Hz, 1 H); 8.40(d, J=7.9 Hz, 1 H); 8.79(d, J=9.0 Hz, 1H); 10.20(b, 1H); 10.68(s, 1H). (DMSO-d6) |

### Preparation of the compounds of general formula (Id):

### Example 1d.- Preparation of N-[1-(2-dimethylaminoethyl)-1 H-indole-4-yl]-5-chloro-3-methyl-benzo[b]thiophene-2-sulfonamide.

185.5 mg (0.66 mMol) of 5-chloro-3-methyl-benzo[b] thiophene-2-sulfonyl chloride were added to a solution of 122 mg (0.6 mMol) of 4-amino-3-(2-dimethylaminoethyl)-1H-indole in 2 ml of dimethylformamide and 116 mg of N-ethyldiisopropylamine. The reaction mixture was stirred at the room temperature for 20 hours. Then it was evaporated to dryness, slightly alkalinized with sodium bicarbonate solution and extracted with chloroform. The organic phase was repeatedly washed with water and saturated solution of sodium bicarbonate, it was separated and dried with anhydrous sodium sulfate. The organic solution was evaporated to dryness and the resulting solid was purified by chromatography, obtaining 111 mg (42%) of N-[1-(2-dimethylaminoethyl)-1 H-indole-4-yl]-5-choloro-3-methyl-benzo[b]thiophene-2-sulfonamide as a creamy solid.

### Example 2d.- Preparation of N-[1-(2-dimethylaminoethyl)-1 H-indole-4-yl]-naphtalene-2-sulfonamide

121 mg (51%) of the mentioned compound were obtained from 122 mg (0.6 mMol) of 4-amino-1-(2-dimethylaminoethyl)-1 H-indole and 149.5 mg (0.66 mMol) of naphtalene-2-sulfonyl chloride, by means of the process described in the Example 1d, as a creamy solid.

### Example 3d.- Preparation of N-[1-(2-dimethylaminoethyl)-1H-indole-4-yl]-naphtalene-1-sulfonamide

130 mg (55%) of the mentioned compound are obtained from 122 mg (0.6 mMol) of 4-amino-1-(2-dimethylaminoethyl)-1 H-indole and 149.5 mg (0.66 mMol) of naphtalene-1-sulfonyl chloride, by means of the process described in the Example 1d, as a creamy solid.

### Example 4d.- Preparation of N-[1-(2-dimethylaminoethyl)-1 H-indole-4-yl]-4 phenylbenzenesulfonamide

107 mg (42%) of the mentioned compound were obtained from 122 mg (0.6 mMol) of 4-amino-1-(2-dimethylaminoethyl)-1H-indole and 169 mg (0.66 mMol) of 4- phenylbenzenesulfonyl chloride, by means of the process described in the Example 1 d, as a creamy solid.

### Example 5d.- Preparation of N-[1-(2-dimethylaminoethyl)-1 H-indole-4-yl]-2-(naphthalene-1-yl)-ethanesulfonamide

52 mg (21 %) of the mentioned compound were obtained from 122 mg (0.6 mMol) of 4-amino-1-(2-dimethylaminoethyl)-1 H-indole and 168 mg (0.66 mMol) of 2-(naphthalene-1-yl)- ethanesulfonyl chloride, by means of the process described in the Example 1d, as a yellowish solid.

### Example 6d.- Preparation of N-[1-(2-dimethylaminoethyl)-1 H-indole-4-yl]-4-phenoxybenzenesulfonamide

220 mg (84%) of the mentioned compound were obtained from 122 mg (0.6 mMol) of 4-amino-1-(2-dimethylaminoethyl)-1H-indole and 177 mg (0.66 mMol) of 4-phenoxybenzenesulfonyl chloride, by means of the process described in the Example 1 d, as a oil.

### Example 7d.- Preparation of N-[1-(2-dimethylaminoethyl)-1H-indole-4-yl]-3,5-dichlorobenzenesulfonamide

93 mg (38%) of the mentioned compound are obtained from 122 mg (0.6 mMol) of 7-amino-1-(2-dimethylaminoethyl)-1 H-indole and 162 mg (0.66 mMol) of 3,5-dichlorobenzenesulfonyl chloride, by means of the process described in Example 1d, as a creamy solid.

### Example 8d.- Preparation of 6-chloro-N-[1-(2-dimethylaminoethylyl)-1H-indol-4-yl]-imidazo[2,1-b]thiazole-5-sulfonamide

100 mg of the mentioned compound are obtained from 122 mg (0.6 mmol) of 4-amino-1-(2-dimethylaminoethyl)-1 H-indole and 170 mg (0.66 mMol) of 6-chloroimidazo[2,1-b]-thiazole-5-sulfonyl chloride via the process described in Example 1 as a creamy solid.

The yields are indicative and no added effort was made to improve them.
The melting point and spectroscopic data for identifying some of the compounds object of the present invention are indicated in the following table.

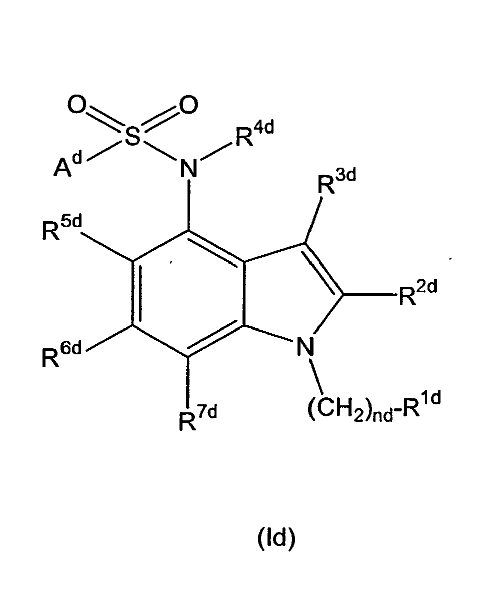

| Ex | R_{1d} | R2d | R_{3d} | R_{4d} | R_{5d} | R_{6d} | R_{7d} | nd | A^{d} | m.p. °C | IR cm⁻¹ | ¹H-NMR (300 MHz), δ (solvent) |
|---|---|---|---|---|---|---|---|---|---|---|---|---|
| 1d | (CH₃)₂N- | H | H | H | H | H | H | 2 | 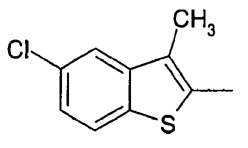 | 78-80 | 3430, 2951, 1492, 1328, 1156, 1115, 1079, 859, 750, 649, 569. | 2,10(s, 6H); 2,28(s, 3H); 2,50(m, 2H); 4,14(t, 2H, J=6,3 Hz); 6,43(d, 1H, J=2,0 Hz); 6,92(d, 1H, J=7,5 Hz); 7,00(t, 1H, J=7,7 Hz); 7,17(d, 1H, J=2,2 Hz); 7,25(d, 1H, J=7,5 Hz); 7,49(d, 1H, J=8,4 Hz); 7,85(s, 1H); 7,99(d, 1H, J=8,5 Hz). (DMSO-d6) |
| 2d | (CH₃)₂N- | H | H | H | H | H | H | 2 | 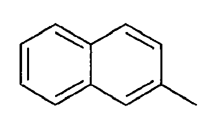 | 156-158 | 3448, 2821, 1492, 1314, 1238,1158, 1127, 1075, 1009, 752, 656, 645, 554, 543, 484. | 2,08(s, 6H); 2,48(m, 2H); 4,10(t, 2H, J=6,6 Hz); 6,58(d, 1H, J=3,1 Hz); 6,85-6,96(m, 2H); 7,15(d, 1H, J=7,8 Hz); 7,19(d, 1H, J=3,1 Hz); 7,54-7,68(m, 2Hz); 7,83(dd, 1H, J=8,6 Hz, J'=1,8 Hz); 7,94(d, 1H, J=8,1 Hz). (DMSO-d6) |
| 3d | (CH₃)₂N- | H | H | H | H | H | H | 2 | 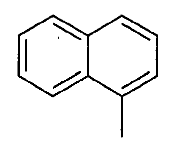 | 169-172 | 3279, 2943, 1403, 1318, 1162, 1132, 1003, 767, 745. | 2,08(s, 6H); 2,46(m, 2H); 4,07(t, 2H, J=6,7 Hz); 6,45(d, 1H, J=3,2 Hz); 6,81 (d, 1H, J=6,8 Hz); 6,88(t, 1H, J=7,7 Hz); 7,09(d, 1H, J=8,2 Hz); 7,12(d, 1H, J=3,2 Hz); 7,52(m, 1H); 7,62(m, 1 Hz); 7,70(m, 1H); 8,01(d, 1H, J=8,2 Hz); 8,11(m, 2H), 8,87(d, 1H, J=8,4 Hz). (DMSO-d6) |
| 4d | (CH₃)₂N- | H | H | H | H | H | H | 2 | 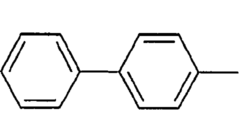 | 137-140 | 3262, 2943, 1492, 1330, 1160, 1096, 750,670,590, 531. | 2,10(s, 6H); 2,51(m, 2H); 4,14(t, 2H, J=6,6 Hz); 6,61 (d, 1H, J=3,0 Hz); 6,90(d, 1H, J=7,0 Hz); 6,97(t, 1H, J=7,8 Hz); 7,19(d, 1H, J=7,8 Hz); 7,23(d, 1H, J=3,2 Hz); 7,36-7,69(m, 3H); 7,65(d, 2H, J=6,8 Hz); 7,76(AB sys, 2H, J=8,6 Hz); 7,82(AB sys, 2H, J=8,5 Hz,). (DMSO-d6) |
| 5d | (CH₃)₂N- | H | H | H | H | H | H | 2 | 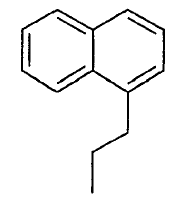 | 47-54 | 3430, 3255, 2941, 2760, 1492, 1322, 1150, 748. | 2,16(s, 6H); 2,59(m, 2H); 3,35(m, 4H); 4,24(t, 2H, J=6,3 Hz); 6,89(m, 1H, J=3,1 Hz); 7,05-7,11 (m, 2H); 7,22(m, 1 H); 7,28-7,38(m, 4H); 7,41(m, 2H); 7,74(d, 1H, J=7,18 Hz); 7,86(d, 1H, J=8,2 Hz). (DMSO-d6) |
| 6d | CH₃)₂N- | H | H | H | H | H | H | 2 | 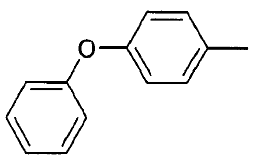 | oil | 2944, 2776, 1488, 1343, 1244,1156, 1094, 751, 695 | 2,12(s, 6H); 2,52(m, 2H); 4,15(t, 2H, J=6,5 Hz); 6,51 (d, 1H, J=3,0 Hz); 6,85(d, 1H, J=7,6 Hz); 6,97(m, 3H); 7,03(d, 2H, J=7,6 Hz); 7,20(d, 2H, J=8,1 Hz); 7,24(d, 1H, J=3,2 Hz); 7,42(t, 2H, J=7,9 Hz); 7,70(d, 2H, J=8,9 Hz). (DMSO-d6) |
| 7d | CH₃)₂N- | H | H | H | H | H | H | 2 | 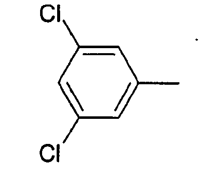 | 113-118 | 3255, 3072, 2935, 1570, 1492, 1340, 1169, 1138, 803, 747, 670, 594. | 2,12(s, 6H); 2,54(t, 2H, J=6,6); 4,17(t, 2H, J=6,5 Hz); 6,42(d, 1H, J=3,1 Hz); 6,82(d, 1H, J=7,6 Hz); 7,02(t, 1H, J=8,0 Hz); 7,26-7,30(m, 2H); 7,63(d, 2H, J=1,9 Hz); 7,86(t, 1H, J=1,8 Hz). (DMSO-d6) |
| 8d | (CH₃)₂N- | H | H | H | H | H | H | 2 | 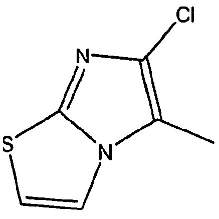 | 95-100 | | 2,15(s, 6H); 2,56(t, 2H, J=6,2 Hz); 4,17(t, 2H, J=6,6 Hz); 6,31 (d, 1 H, J=2,8 Hz); 6,89(d, 1H, J=7,3 Hz); 7,01(m, 1 H); 7,21(d, 1H, J=3,0 Hz); 7,27(d, 1H, 8,0 Hz); 7,49(d, 1 H, J=4,4 Hz); 7,72(d, 1 H, J=4,4 Hz). (DMSO-d6) |

### Preparation of the compounds of general formula (le):

### Example 2e.- Preparation of N-[1-(2-dimethylaminoethyl)-1 H-indole-5-yl]-naphthalene-2-sulfonamide.

150 mg (0.66 mMol) of Naphthalene-2-sulfonyl chloride were added to a solution of 122 mg (0.6 mMol) of 5-amino-1-(2-dimethylaminoethyl)-1 H-indole in 3 ml of dimethylformamide and 116 mg of N-ethyldiisopropylamine. The reaction mixture is stirred at the room temperature for 12 hours. Then it is evaporated to dryness, slightly alkalinized with sodium bicarbonate solution and extracted with chloroform. The organic phase is repeatedly washed with water and saturated solution of sodium bicarbonate, it is separated and dried with anhydrous sodium sulfate. The organic solution is evaporated to dryness and the resulting solid is purified by chromatography, obtaining 187 mg (80%) of N-[1-(2-dimethylaminoethyl)-1 H-indole-5-yl]-naphthalene-2-sulfonamide.

### Example 10e.- Preparation of N-[1-(2-dimethylaminoethyl)-1 H-indole-5-yl]-benzo-[1,2,5]thiadiazole-4-sulfonamide 4-sulfonamide

116 mg (0.66 mMol) of benzo-[1,2,5]thiadiazole-4-sulfonyl chloride were added to a solution of 168 mg (0.6 mMol) of 5-amino-1-(2-dimethylaminoethyl)-1H-indole in 5 ml of pyridine and 311 mg of N-ethyldiisopropylamine. The reaction mixture is stirred at the room temperature for 2 hours. Then it is evaporated to dryness, slightly alkalinized with sodium bicarbonate solution and extracted with chloroform. The organic phase is repeatedly washed with water and saturated solution of sodium bicarbonate, it is separated and dried with anhydrous sodium sulfate. The organic solution is evaporated to dryness and the resulting solid is treated with diethyl ether obtaining 183 mg (76%) of N-[1-(2-dimethylaminoethyl)-1 H-indole-5-yl]-benzo-[1,2,5]thiadiazole-4-sulfonamide 4-sulfonamide.

### Example 17e.- Preparation of N-[1-(2-pyrrolidine-1-yl-ethyl)-1 H-indole-5-yl]-naphthalene-1-sulfonamide,

199 mg (0.88 mMol) of naphthalene-1-sulfonyl chloride were added to a solution of 335 mg (0.8 mMol) of 5-amino-1-(2- pyrrolidine-1-yl-ethyl)-1 H-indole in 10 ml of methylene chloride and 0,44 mg of triethylamine. The reaction mixture is stirred at the room temperature for 12 hours. Then it is slightly alkalinized with sodium bicarbonate solution and extracted with methylene chloride. The organic phase is repeatedly washed with water and saturated solution of sodium bicarbonate, it is separated and dried with anhydrous sodium sulfate. The organic solution is evaporated to dryness and the resulting solid is treated with diethyl ether obtaining 264 mg (79%) of N-[1-(2-pyrrolidine-1-yl-ethyl)-1 H-indole-5-yl]-naphthalene-1-sulfonamide as a solid.

### Example 29e. Preparation of N-(1-(2-(diethylamino)ethyl)-1 H-indol-5-yl)-naphthalene-2-sulfonamide

The reaction was carried out according to the procedure given in Example 1. 139 mg (0.6 mMol) of 5-amino-1-(2-(diethylamino)ethyl)-1H-indole and 150 mg (0.66 mMol) of 2-naphthyl-sulfonyl chloride were reacted to give 115 mg (45 %) of the desired compound as a solid.

### Example 30e. Preparation of N-(1-(2-(diethylamino)ethyl)-1 H-indol-5-yl)-naphthalene-1-sulfonamide

The reaction was carried out according to the procedure given in Example 1. 139 mg (0.6 mMol) of 5-amino-1-(2-(diethylamino)ethyl)-1 H-indole and 150 mg (0.66 mMol) of 2-naphthyl-sulfonyl chloride were reacted to give 160 mg (63 %) of the desired compound as a solid.

### Example 31e. Preparation of N-(1-(2-(diethylamino)ethyl)-1H-indol-5-yl)-4-phenylbenzenesulfonamide

The reaction was carried out according to the procedure given in Example 1. 139 mg (0.6 mMol) of 5-amino-1-(2-(diethylamino)ethyl)-1H-indole and 167 mg (0.66 mMol) of 4-phenylbenzenesulfonyl chloride were reacted to give 181 mg (68 %) of the desired compound as an oil.

### Example 32e. Preparation of 5-chloro-N-(1-(2-(dimethylamino)ethyl)-2-methyl-1 H-indol-5-yl)-3-methylbenzo[b]thiophene-2-sulfonamide

The reaction was carried out according to the procedure given in Example 1. 130 mg (0.6 mMol) of 5-amino-1-(2-(dimethylamino)ethyl)-2-methyl-1H-indole and 186 mg (0.66 mMol) of 5-chloro-2-methylbenzo[b]thiophene-2-sulfonyl chloride were reacted to give 127 mg (46 %) of the desired compound as a solid.

### Example 33e. Preparation of N-(1-(2-(dimethylamino)ethyl)-2-methyl-1 H-indol-5-yl)-naphthalene-2-sulfonamide

The reaction was carried out according to the procedure given in Example 1. 130 mg (0.6 mMol) of 5-amino-1-(2-(dimethylamino)ethyl)-2-methyl-1 H-indole and 150 mg (0.66 mMol) of naphthyl-2-sulfonyl chloride were reacted to give 142 mg (58 %) of the desired compound as a solid.

### Example 34e. Preparation of N-(1-(2-(dimethylamino)ethyl)-2-methyl-1 H-indol-5-yl)-naphthalene-1-sulfonamide

The reaction was carried out according to the procedure given in Example 1. 130 mg (0.6 mMol) of 5-amino-1-(2-(dimethylamino)ethyl)-2-methyl-1 H-indole and 150 mg (0.66 mMol) of naphthyl-1-sulfonyl chloride were reacted to give 81 mg (33 %) of the desired compound as a solid.

Example 35e. Preparation of 6-chloro-N-(1-(2-(dimethylamino)ethyl)-2-methyl-1 H-indol-5-yl)imidazo[2,1-b]thiazole-5-sulfonamide

The reaction was carried out according to the procedure given in Example 1. 130 mg (0.6 mMol) of 5-amino-1-(2-(dimethylamino)ethyl)-2-methyl-1H-indole and 170 mg (0.66 mMol) of 6-chloro-imidazo[2,1-b]thiazole-5-sulfonyl chloride were reacted to give 96 mg (37 %) of the desired compound as a solid.

### Example 36e. Preparation of N-(1-(2-(dimethylamino)ethyl)-2-methyl-1 H-indol-5-yl)-4-phenylbenzenesulfonamide

The reaction was carried out according to the procedure given in Example 1. 130 mg (0.6 mMol) of 5-amino-1-(2-(dimethylamino)ethyl)-2-methyl-1 H-indole and 167 mg (0.66 mMol) of 4-phenylbenzenesulfonyl chloride were reacted to give 160 mg (62 %) of the desired compound as a solid.

### Example 37e. Preparation of N-(1-(2-dimethylamino)ethyl)-2-methyl-1 H-indol-5-yl)-2-(naphth-1-yl)-ethanesulfonamide

The reaction was carried out according to the procedure given in Example 1. 130 mg (0.6 mMol) of 5-amino-1-(2-(dimethylamino)ethyl)-2-methyl-1 H-indole and 168 mg (0.66 mMol) of 2-(naphth-1-yl)-ethanesulfonyl chloride were reacted to give 108 mg (41 %) of the desired compound as a solid.

### Example 38e. Preparation of N-(1-(2-(dimethylamino)ethyl)-2-methyl-1 H-indol-5-yl)-4-phenoxy-benzenesulfonamide

The reaction was carried out according to the procedure given in Example 1. 130 mg (0.6 mMol) of 5-amino-1-(2-(dimethylamino)ethyl)-2-methyl-1 H-indole and 177 mg (0.66 mMol) of 4-phenoxy-benzenesulfonyl chloride were reacted to give 89 mg (33 %) of the desired compound as a solid.

### Example 39e. Preparation of 3,5-dichloro-N-(1-(2-(dimethylamino)ethyl)-2-methyl-1 H-indol-5-yl)-benzenesulfonamide

The reaction was carried out according to the procedure given in Example 1. 130 mg (0.6 mMol) of 5-amino-1-(2-(dimethylamino)ethyl)-2-methyl-1 H-indole and 162mg (0.66 mMol) of 3,5-dichloro-benzenesulfonyl chloride were reacted to give 81 mg (32 %) of the desired compound as a solid.

### Example 40e. Preparation of N-(1-(2-(dimethylamino)ethyl)-2-methyl-1 H-indol-5-yl)benzo[b]thiophene-3-sulfonamide

The reaction was carried out according to the procedure given in Example 1. 108 mg (0.5 mMol) of 5-amino-1-(2-(dimethylamino)ethyl)-2-methyl-1 H-indole and 128 mg (0.55 mMol) of benzo[b]thiophene-3-sulfonyl chloride were reacted to give 82 mg (39 %) of the desired compound as a solid.

### Example 41 e. Preparation of N-(1-(2-(diethylamino)ethyl)-1H-indol-5-yl)benzo[b]thiophene-3-sulfonamide

The reaction was carried out according to the procedure given in Example 1. 115 mg (0.5 mMol) of 5-amino-1-(2-(diethylamino)ethyl)-1 H-indole and 128 mg (0.55 mMol) of benzo[b]thiophene-3-sulfonyl chloride were reacted to give 91 mg (43 %) of the desired compound as a solid.

### Example 42e. Preparation of N-(1-(2-(dimethylamino)ethyl)-1H-indol-5-yl)benzo[b]thiophene-3-sulfonamide

The reaction was carried out according to the procedure given in Example 1. 102 mg (0.5 mMol) of 5-amino-1-(2-(diethylamino)ethyl)-1 H-indole and 128 mg (0.55 mMol) of benzo[b]thiophene-3-sulfonyl chloride were reacted to give 91 mg (43 %) of the desired compound as a solid.

Example 43e. Preparation of 5-chloro-3-methyl-N-(1-(3-(piperidin-1-yl)propyl)-1 H-indol-5-yl)benzo[b]thiophene-2-sulfonamide

The reaction was carried out according to the procedure given in Example 1. 118 mg (0.46 mMol) of 5-amino-1-(3-(piperidin-1-yl)propyl))-1 H-indole and 143 mg (0.51 mMol) of 5-chloro-3-methyl-benzo[b]thiophene-2-sulfonyl chloride were reacted to give 89 mg (38 %) of the desired compound as a solid.

### Example 44e. Preparation of N-(1-(3-(piperidin-1-yl)propyl)-1 H-indol-5-yl)naphthalene-2-sulfonamide

The reaction was carried out according to the procedure given in Example 1. 118 mg (0.46 mMol) of 5-amino-1-(3-(piperidin-1-yl)propyl))-1 H-indole and 116 mg (0.51 mMol) of naphthyl-2-sulfonyl chloride were reacted to give 75 mg (37 %) of the desired compound as a solid.

### Example 45e. Preparation of N-(1-(3-(piperidin-1-yl)propyl)-1 H-indol-5-yl)naphthalene-1-sulfonamide

The reaction was carried out according to the procedure given in Example 1. 118 mg (0.46 mMol) of 5-amino-1-(3-(piperidin-1-yl)propyl))-1H-indole and 116 mg (0.51 mMol) of naphthyl-2-sulfonyl chloride were reacted to give 91 mg (44 %) of the desired compound as a solid.

### Example 46e. Preparation of 6-chloro-N-(1-(3-piperidin-1-yl)propyl)-1 H-indol-5-yl)imidazo[2,1-b]thiazole-5-sulfonamide

The reaction was carried out according to the procedure given in Example 1. 118 mg (0.46 mMol) of 5-amino-1-(3-(piperidin-1-yl)propyl))-1H-indole and 131 mg (0.51 mMol) of 6-chloro-imidazo[2,1-b]thiazole-5-sulfonyl chloride were reacted to give 91 mg (44 %) of the desired compound as a solid.

### Example 47e. Preparation of 4-phenyl-N-(1-(3-(piperidin-1-yl)propyl)-1H-indol-5-yl)benzenesulfonamide

The reaction was carried out according to the procedure given in Example 1. 118 mg (0.46 mMol) of 5-amino-1-(3-(piperidin-1-yl)propyl))-1 H-indole and 129 mg (0.51 mMol) of 4-phenylbenzenesulfonyl chloride were reacted to give 106 mg (49 %) of the desired compound as a solid.

### Example 48e. Preparation of 2-(naphth-1-yl)-N-(1-(3-(piperidin-1-yl)propyl)-1 H-indol-5-yl)ethanesulfonamide

The reaction was carried out according to the procedure given in Example 1. 118 mg (0.46 mMol) of 5-amino-1-(3-(piperidin-1-yl)propyl))-1H-indole and 130 mg (0.51 mMol) of 2-(naphth-1-yl)ethanesulfonyl chloride were reacted to give 68 mg (31 %) of the desired compound as a solid.

### Example 49e. Preparation of 4-phenoxy-N-(1-(3-(piperidin-1-yl)propyl)-1 H-indol-5-yl)benzenesulfonamide

The reaction was carried out according to the procedure given in Example 1. 118 mg (0.46 mMol) of 5-amino-1-(3-(piperidin-1-yl)propyl))-1 H-indole and 137 mg (0.51 mMol) of 4-phenoxybenzenesulfonyl chloride were reacted to give 86 mg (38 %) of the desired compound as a solid.

### Example 50e. Preparation of 3,5-dichloro-N-(1-(3-(piperidin-1-yl)propyl)-1 H-indol-5-yl)benzenesulfonylamide

The reaction was carried out according to the procedure given in Example 1. 118 mg (0.46 mMol) of 5-amino-1-(3-(piperidin-1-yl)propyl))-1H-indole and 125 mg (0.51 mMol) of 3,5-dichlorobenzenesulfonyl chloride were reacted to give 79 mg (37 %) of the desired compound as a solid.

### Example 51 e. Preparation of 4,5-dichloro-N-(1-(3-(piperidin-1-yl)propyl)-1H-indol-5-yl)thiophene-2-sulfonamide

The reaction was carried out according to the procedure given in Example 1. 118 mg (0.46 mMol) of 5-amino-1-(3-(piperidin-1-yl)propyl))-1 H-indole and 128 mg (0.51 mMol) of 4,5-dichlorothiophene-2-sulfonyl chloride were reacted to give 68 mg (31 %) of the desired compound as a solid.

### Example 52e. Preparation of 5-chloro-N-(1-(3-(piperidin-1-yl)propyl)-1 H-indol-5-yl)naphthalene-1-sulfonamide

The reaction was carried out according to the procedure given in Example 1. 118 mg (0.46 mMol) of 5-amino-1-(3-(piperidin-1-yl)propyl))-1 H-indole and 133 mg (0.51 mMol) of 5-chloro-napthyl-1-sulfonyl chloride were reacted to give 81 mg (37 %) of the desired compound as a solid.

The yields are indicative and no added effort was made to improve them The melting point and spectroscopic data for identifying some of the compounds object of the present invention are indicated in the following table

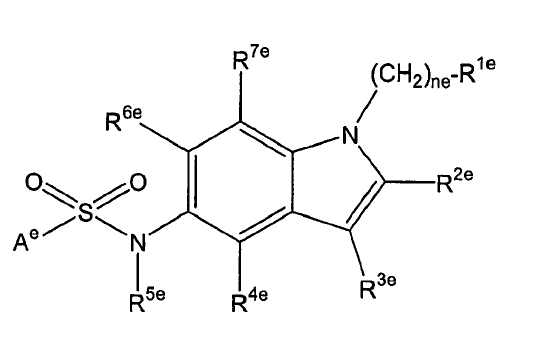

| Ex | R^{1e} | R^{2e} | R^{3e} | R^{4e} | R^{5e} | R^{6e} | R^{7e} | ne | A^{e} | m.p. °C | IR cm⁻¹ | ¹H-NMR (300 MHz),δ (solvent) |
|---|---|---|---|---|---|---|---|---|---|---|---|---|
| 1e | (CH₃)₂N- | H | H | H | H | H | H | 2 | 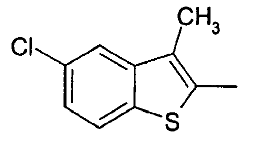 | 71-73 | 2950, 1334, 1160, 1080, 862, 652, 560. | 2,11(s, 6H); 2,36(s, 3H); 2,51(m, 2H); 4,14(t, 2H, J=6,6 Hz); 6,30(d, 1H, J=3 Hz); 7,32 (m, 2H); 7,50(dd, 1H, J=8,7 Hz, J'=2,0 Hz); 7,93(d, 1H, J=2,0 Hz); 7.99(d, 1H, J=8.7 Hz). (DMSO-d6) |
| 2e | (CH₃)₂N- | H | H | H | H | H | H | 2 | 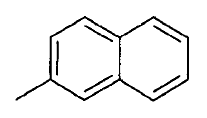 | 54-57 | 3254, 3049, 2945,1463, 1330, 1160, 1074,658, 550. | 2,26(s, 6H); 2,63(t, 2H, J=7,1 Hz); 4,14(t, 2H, J=7,1 Hz); 6,35(d, 1H, J=3,1 Hz); 6,88 (dd, 1H, J=8,6 Hz, J'=2,0 Hz); 7,10(d, 1H, 3,1 Hz); 7,15(d, 1H, J=8,6 Hz); 7,31(d, 1H, J=2,OHz); 7,50-7,63(m, 2H); 7,69(dd, 1H, J=8,7 Hz, J'=1,8 Hz); 7,84(m, 3H); 8,29(s, 1 H). (CDCl₃) |
| 3e | (CH₃)₂N- | H | H | H | H | H | H | 2 | 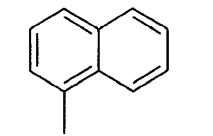 | 179-181 | 3106,2783, 1491,1318, 1159, 1130, 763, 586, 503. | 2,25(s, 6H); 2,63(t, 2H, J=7,0 Hz); 4,11(t, 2H, J=7,0 Hz); 6,28(d, 1H, J=3,1 Hz); 6,68 (dd, 1H, J=8,6 Hz, J'=2,0 Hz); 7,03-7,11(m, 3H); 7,37(m, 1H); 7,58-7,70(m, 2H); 7,94(d, 1H, J=8,7 Hz); 8,00(d, 1H, J=7,9 Hz); 8,06(d, 1H, J=7,3 Hz); 8,73(d, 1H, J=8,7 Hz). (CDCl₃) |
| 4e | (CH₃)₂N- | H | H | H | H | H | H | 2 | 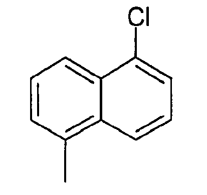 | 172-174 | 3257, 2935, 2768, 1488, 1334, 1167, 1138, 1013, 790, 606. | 2,26(s, 6H); 2,63(t, 2H, J=7,1 Hz); 4,13(t, 2H, J=7,1 Hz); 6,30(d, 1H, J=3,1 Hz); 6,66 (dd, 1H, J=8,6 Hz, J'=2,0 Hz); 7,05(d, 1H, J=8, 7Hz,); 7,08(d, 1H, J=3,1 Hz); 7,11 (d, 1H, J=2,0 Hz); 7,46-7,58(m, 2H); 7,69(d, 1H, J=7,5 Hz); 8,13(d, 1H, J=7,5 Hz); 8,50(d, 1H, J=8,6 Hz); 8,69(d, 1H, J=8,8 Hz). (CDCl₃) |
| 5e | (CH₃)₂N- | H | H | H | H | H | H | 2 | 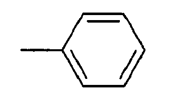 | 139-141 | 1463, 1334, 1306,1164, 1090, 725, 589. | 2,28(s, 6H); 2,66(t, 2H, J=7,1 Hz); 4,17(t, 2H, J=7,1 Hz); 6,38(d, 1H, J=3,1 Hz); 6,88 (dd, 1H, J=8,6 Hz, J'=2,0 Hz); 7,13(d, 1H, J=3,1 Hz); 7,18(d, 1H, J=8,6 Hz); 7,27(m, 1H); 7,39(m, 2H); 7,48(m, 1H); 7,69(m, 2H). (CDCl₃) |
| 6e | (CH₃)₂N- | H | H | H | H | H | H | 2 | 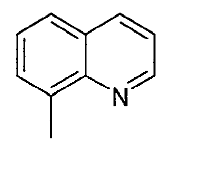 | 161-164 | 3095, 02821, 2776, 1492, 1459, 1322, 1158, 1141, 782, 736, 596, 507. | 2,23(s, 6H); 2,58(t, 2H, J=7,1 Hz); 4,08(t, 2H, J=7,1 Hz); 6,24(d, 1H, J=3,1 Hz); 6,88 (dd, 1H, J=8,8, J'=2,0 Hz); 7,03(d, 1H, J=3,1 Hz); 7,07(d, 1H, J=8,8 Hz); 7,10(d, 1H, J=2,0 Hz); 7,51 (t, 1H, J=7,8 Hz); 7,64(dd, 1H, J=8,5, J'=4,3 Hz); 8,00(d, 1H, J=8,2 Hz); 8,26(m, 1 H); 8,30(dd, 1H, J=8,2, J'=1,5 Hz); 8,40(s, 1H); 9,20(dd, 1H, J=4,1 J'=1,4 Hz). (CDCl₃) |
| 7e | (CH₃)₂N- | H | H | H | H | H | H | 2 | 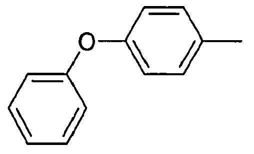 | 138-140 | 3255, 2951, 1583, 1488, 1332, 1245, 1156, 1092, , 866, 695, 569. | 2,28(s, 6H); 2,67(t, 2H, J=7,1 Hz); 4,18(t, 2H, J=7,1 Hz); 6,40(d, 1H, J=3,1 Hz); 6,92 (m, 3H); 7,02(d, 2H, J=7,7 Hz); 7,14(d, 1H, J=3,1 Hz); 7,20(d, 2H, J=8,5 Hz); 7,28(d, 1H, J=1,9 Hz); 7,37(m, 2H); 7,64(d, 2H, J=8,6Hz). (CDCl₃) |
| 8e | (CH₃)₂N- | H | H | H | H | H | H | 2 | 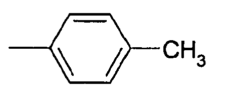 | 126-128 | 1474, 1287, ; 1156, 1088, 973, 730, 854, 554, 538. | 2,31 (s, 6H); 2,36(s, 3H); 2,72(t, 2H, J=7,1 Hz); 4,20(t, 2H, J=7,1 Hz); 6,39(d, 1H, J=3,1 Hz); 6,90 (dd, 1H, J=8,6 Hz, J'=1,6 Hz); 7,13(d, 1H, J=3,1 Hz); 7,16-7,20(m, 3H); 7,26(m, 1H); 7,57(d, 2H, J=8,3 Hz). (CDCl₃) |
| 9e | (CH₃)₂N- | H | H | H | H | H | H | 2 | 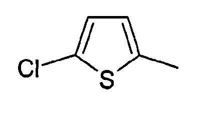 | 145-147 | 3095, 2951, 1416, 1319, 1148, 989, 730, 605, 537. | 2,33(s, 6H); 2,74(m, 2H); 4,24(t, 2H, J=7,1 Hz); 6,44(d, 1H, J=3,1 Hz); 6,79 (d, 1 H, J=4,0 Hz); 6,95(dd, 1H, J=8,7 Hz, J'=2,0 Hz); 7,15(d, 1H, J=4,0 Hz); 7,17(d, 1H, J=3,1 Hz); 7,24(d, 1H, J=8,7 Hz); 7,35(d, 1 H, J=2,0 Hz). (DMSO-d6) |
| 10e | (CH₃)₂N- | H | H | H | H | H | H | 2 | 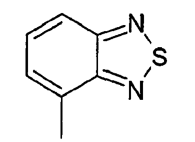 | 166-168 | 3103,2783, 1526, 1488, 1140, 973, 734, 607. | 2,26(s, 6H); 2,63(t, 2H, J=7,1 Hz); 4,12(t, 2H, J=7,1 Hz); 6,29(d, 1H, J=3,1 Hz); 6,80 (dd, 1H, J=8,7 Hz, J'=2,0 Hz); 7,07(m, 2H); 7,15(d, 1H, J=1,5 Hz); 7,57(dd, 1H, J=8,8 Hz, J'=7,1Hz); 8,10(d, 1H, J=7,1 Hz); 8,16(d, 1 H, J=8,8 Hz). (CDCl₃) |
| 11e | (CH₃)₂N- | H | H | H | H | H | H | 2 | 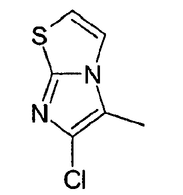 | 50-52 | 3103, 2943, 1457, 1336, 1326, 1244, 1177,1142, 727,628, 528. | 2,26(s, 6H); 2,64(t, 2H, J=6,4 Hz); 4,16(t, 2H, J=6,4 Hz); 6,39(m, 1 H); 6,78 (d, 1 H, J=4,0 Hz); 6,94(d, 1H, J=8,4 Hz); 7,15(m, 2H); 7,39(s, 1H); 7,55(d, 1H, J=4,0 Hz). (CDCl₃) |
| 12e | (CH₃)₂N- | H | H | H | H | H | H | 2 | 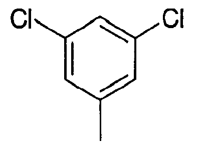 | 124-126 | 3064, 2935, 1333,1166, 1136, 596, 587. | 2,28(s, 6H); 2,67(t, 2H, J=7,0 Hz); 4,19(t, 2H, J=7,0 Hz); 6,43(d, 1H, J=3,1 Hz); 6,85 (dd, 1H, J=8,6 Hz, J'=2,0 Hz); 7,17(d, 1H, J=3,1 Hz); 7,22(d, 1H, J=8,6 Hz); 7,31(d, 1H, J=2,0 Hz); 7,48(t, 1H, J=1,8 Hz); 7,56(d, 2H, J=1,8 Hz). (CDCl₃) |
| 13e | (CH₃)₂N- | H | H | H | H | H | H | 2 | 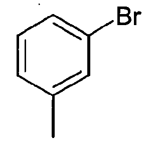 | 114-116 | 1464, 1335, 1286, 1161, 722, 584. | 2,28(s, 6H); 2,67(t, 2H, J=7,0 Hz); 4,19(t, 2H, J=7,0 Hz); 6,41 (d. 1H, J=2,9 Hz); 6,87 (d, 1H, J=8,8 Hz); 7,15(d, 1H, J=2,9 Hz); 7,19-7,29(m, 3H); 7,56(d, 1H, J=7,8 Hz); 7,63(d, 1H, J=7,9 Hz); 7,88(s,1 H). (CDCl₃) |
| 14e | (CH₃)₂N- | H | H | H | H | H | H | 2 | 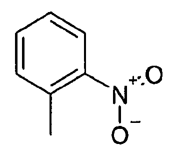 | 138-140 | 1541, 1481, 1365, 1330, 1235, 1150, 1124, 736, 580. | 2,28(s, 6H); 2,66(t, 2H, J=7,1 Hz); 4,17(t,2H, J=7,1 Hz); 6,40(d, 1H, J=2,9 Hz); 7,03 (dd, 1H, J=8,7 Hz, J'=1,8 Hz); 7,15(d, 1H, J=2,9 Hz); 7,21(d, 1H, J=8,7 Hz); 7,39(d, 1H. J=1,BHz); 7,48(m, 1H); 7,65(m, 1H); 7,71 (d, 1H, J=8,8 Hz); 7.86(d,1H, J=7,8Hz). (CDCl₃) |
| 15e | (CH₃)₂N- | H | H | H | H | H | H | 2 | 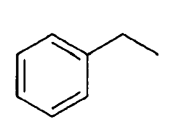 | 163-166 | 1329, 1288, 1153, 1128, 694, 545, 509. | 2,30(s, 6H); 2,70(t, 2H, J=7,1 Hz); 4,22(t, 2H, J=7,1 Hz); 4,29(s, 2H); 6,48(d, 1H, J=3,1 Hz); 7,04 (dd, 1H, J=8,8 Hz, J'=2,2 Hz); 7,19(d, 1 H, J=3,1 Hz); 7,31(d, 1 H, J=8,8 Hz); 7,33-7,40(m, 5H); 7,49(d, 1H, J=2,2 Hz). (CDCl₃) |
| 16e | 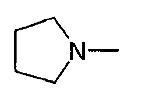 | H | H | H | H | H | H | 2 | 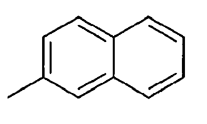 | 138-140 | 2960, 1481, 1323,1161, 1151, 1074, 659, 549, 480. | 1,57(m, 4H); 2,37(m, 4H); 2,66(t, 2H, J=6,8 Hz); 4,12(t, 2H, J=6,8 Hz); 6,25(d, 1H, J=3,1 Hz); 6,82 (dd, 1H, J=8,8 Hz, J'=2,0 Hz); 7,22(d, 1H, 2,0 Hz); 7,25(d, 1H, J=8,6 Hz); 7,29(d, 1H, J=3,1 Hz); 7,54-7,66(m, 2H); 7,74(dd, 1H, J=8,7 Hz, J'=1,8 Hz); 7,94(m, 1H); 8,03(m, 2H); 8,28(s, 1H). (CDCl₃) |
| 17e | 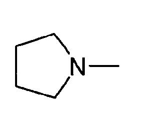 | H | H | H | H | H | H | 2 | 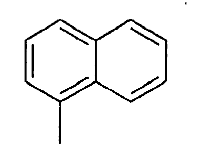 | 186-189 | 2814, 1491, 1291, 1158, 1128, 763, 585. | 1,59(m, 4H); 2,39(m, 4H); 2,67(t, 2H, J=6,8 Hz); 4,11 (t, 2H, J=6,8 Hz); 6,21 (d, 1H, J=3,1 Hz); 6,70 (dd, 1H, J=8,8 Hz, J'=1,8 Hz); 7,10(d, 1H, 1,8 Hz); 7,20(d, 1H, J=8,8 Hz); 7,27(d, 1H, J=3,1 Hz); 7,50(m, 1 H); 7,60-7,74(m, 2H); 8,03(m 2H); 8,11(d, 1H, J=8,1 Hz); 8,76(d, 1H, J=8,6 Hz). (CDCl₃) |
| 18e | 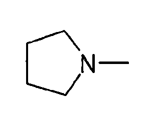 | H | H | H | H | H | H | 2 | 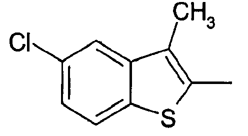 | 156-158 | 2950, 2803, 1491, 1325, 1156, 1078, 650, 564. | 1,59(m, 4H); 2,36(m, 4H); 2,69(t, 2H, J=6,6 Hz); 4,11 (t, 2H, J=6,6 Hz); 6, 30(d, 1H, J=3,1 Hz); 6,83 (dd, 1H, J=8,7 Hz, J'=1,9 Hz); 7,25(d, 1H, 1,9 Hz); 7,32(m, 2H); 7,50(dd, 1H, J=8,6Hz, J'=2,0 Hz); 7,92(d, 1H, J=2,0 Hz); 7,98(d, 1H, J=8,8 Hz). (CDCl₃) |
| 19e | (CH₃)₂N- | H | H | H | H | H | H | 2 | 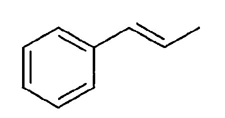 | 108-111 | 2943, 2821, 1516,1139, 752, 728, 542,531. | 2,28(s, 6H); 2,68(t, 2H, J=7,1 Hz); 4,19(t, 2H, J=7,1 Hz); 6.43(d, 1H, J=3,1 Hz); 6,82(d, 1H, J=16,6 Hz); 7,09(dd, 1H, J=8,6, J'=2,0Hz) 7,15(d, 1H, J=3,3 Hz); 7,25(m, 1 H); 7,33-7,44(m, 6H); 7,49(d, 1H, J=2,0 Hz). (CDCl₃) |
| 20e | (CH₃)₂N- | H | H | H | H | H | H | 2 | 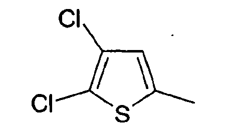 | 120-123 | 3095, 2943, 1421, 1325, 1148, 1020, 730, 609, 534. | 2,30(s, 6H); 2,69(t, 2H, J=7,1 Hz); 4,21 (t, 2H, J=7,1 Hz); 6.46(d, 1H, J=3,1 Hz); 6,93(dd, 1H, J=8,6, J'=2,0Hz); 7,17(s, 1H); 7,18(d, 1H, J=3,3 Hz); 7,25(m, 1H); 7,39(d, 1 H, J=2,0 Hz). (CDCl₃) |
| 21e | (CH₃)₂N- | H | H | H | H | H | H | 2 | 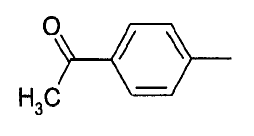 | 175 (desc) | 1686, 1164, 1094, 637, 534, 475. | 2,12(s, 6H); 2,53(t, 2H, J=6,6 Hz); 4,15(t, 2H, J=6,6 Hz); 6.30(d, 1H, J=3,1 Hz); 6,80 (dd, 1H, J=8,6, J'=2,2 Hz); 7,19(d, 1H, J=2,0 Hz); 7,30-7,34(m, 2H); 7,57(AB sist., 2H, J=8,4Hz); 7,70(AB sist., 2H, J=8,4Hz). (DMSO-d6). |
| 22e | (CH₃)₂N- | H | H | H | H | H | H | 2 | 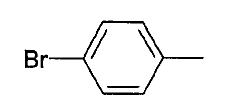 | 114-117 | 2943, 1573, 1469, 1321, 1161, 1088, 737, 630, 538. | 2,13(s, 6H); 2,50-2,55(m, 5H); 4,14(t, 2H, J=6,3 Hz); 6.29(d, 1H, J=3,0 Hz); 6,82 (d, 1 H, J=8,2 Hz); 7,20(s, 1 H); 7,29-7,32(m, 2H); 7,79(AB sist., 2H, J=8,4Hz); 8,02(AB sist., 2H, J=8,4Hz). (DMSO-d6). |
| 23e | (CH₃)₂N- | H | H | H | H | H | H | 2 | 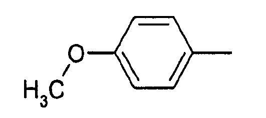 | 141-144 | 2935, 1598, 1497,1333, 1258, 1159, 1093,1018, 836, 568, 560. | 2,28(s, 6H); 2,67(t, 2H, J=7,1 Hz); 4,18(t, 2H, J=7,1 Hz); 6.38(d, 1H, J=3,1 Hz); 6,84 (AB Sist., 2H, J=9,0 Hz); 6,90(dd, 1H, J=8,8, J'=2,0 Hz); 7,13(d, 1H, J=3,3 Hz); 7,19(d, 1H, J=8,8 Hz); 7,27(d, 1H, J=2,0 Hz); 7,62(AB sist., 2H, J=9 Hz). (CDCl₃) |
| 24e | (CH₃CH₂)₂N- | H | H | H | H | H | H | 2 | 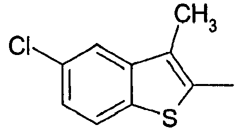 | 53-56 | 2969, 1486, 1334, 1161, 114,1080, 862, 729, 652,560. | 0,78(m, 6H); 2,32(s, 3H); 2,42(m, 4H); 2,65(m, 2H); 4,12(m, 2H); 6.30(d, 1H, J=3,0 Hz); 6,82 (d, 1H, J=8,6 Hz); 7,25(d, 1H, 1,7 Hz); 7,32(m, 2H); 7,50(dd, 1H, J=8,7Hz, J'=1,9 Hz); 7,91 (d, 1H, J=1,7 Hz); 7,99(d, 1 H, J=8,6 Hz). (CDCl₃) |
| 25e | (CH₃)₂N- | H | H | H | H | H | H | 2 | 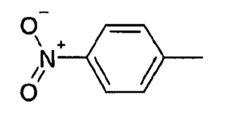 | 60-64 | 3095.2768, 1529, 1349, 1165,1090, 736. | 2,29(s, 6H); 2,67(t, 2H, J=7,0 Hz); 4,18(t, 2H, J=7,0 Hz); 6.40(d, 1H, J=3,1 Hz); 6,85 (dd, 1H, J=8,6 Hz, J'=2,0 Hz); 7,16(d, 1H, J=3,1 Hz); 7,18(d, 1H, J=8,6 Hz); 7,29(d, 1H, J=2,0 Hz) 7,85(AB sys, J=8.8 Hz, 2 H); 8,21(AB sys, J=8.8 Hz, 2 H). (CDCl₃) |
| 26e | (CH₃)₂N- | H | H | H | H | H | H | 2 | 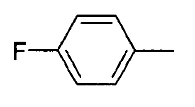 | 138-140 | 3103,2951. 1587, 1491, 1335, 1166, 1089,557. 542. | 2,35(s, 6H); 2,83(m, 2H); 4,28(t, 2H, J=6,7 Hz); 6.40(d, 1 H, J=3,0 Hz); 6,83 (dd, 1 H, J=8,6 Hz, J'=2,0 Hz); 7,20(d, 1H, J=1,9 Hz); 7,30-7,38(m, 4H); 7,70-7,75(m, 2H). (DMSO-d6). |
| 27e | (CH₃CH₂)₂N- | H | H | H | H | H | H | 2 | 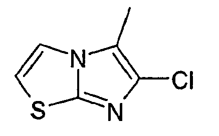 | 68-70 | 3110,2969, 1458, 1271, 1249,1179, 1140,727, 551. | 1,00(t, 6H, J=7,0 Hz); 2,60(q, 4H, J=7,0 Hz); 2,81(t, 2H, J=6,7 Hz); 4,21(t, 2H, J=6,7 Hz); 6.38(d, 1H J=3,0 Hz); 6,79 (d, 1H, J=4,5 Hz); 6,96(dd, 1H, J=8,6, J'=1,7 Hz); 7,14(d, 1H, 3,0 Hz); 7,19(d, 1H, J=8,8 Hz); 7,40(d, 1H, J=1,5 Hz); 7,59(d, 1H, J=4,4 Hz). (CDCl₃) |
| 28e | 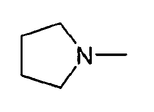 | H | H | H | H | H | H | 2 | 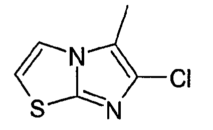 | 81-84 | 3119,2951, 2798, 1458, 1271, 1248, 1178,1140, 727,623. | 1,85(m, 6H); 2,68(m, 4H); 3,00(m, 2H); 4,38(m, 2H); 6.40(d, 1H J=3,1 Hz); 6,82 (d, 1H, J=4,5 Hz); 6,96(d, 1H, J=8,6 Hz); 7,19(d, 1H, 2,7 Hz); 7,22(m, 1 H); 7,41 (m, 1H); 7,64(d, 1H, J=4,5 Hz). (CDCl₃) |
| 29e | (CH₃CH₂)₂N- | H | H | H | H | H | H | 2 | 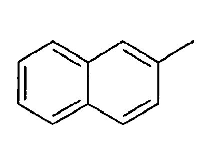 | 97-104 | | 0,95(t, 6H, J=7,1 Hz); 2,54(q, 4H, J=7,0 Hz); 2,76(t, 2H, J=6,7 Hz); 4,07(t, 2H, J=6,7 Hz); 6,66(dd, 1H, J=8,5 J'=1,7 Hz); 6,91 (s, 1 H); 6,97(s, 1H); 7,01 (d, 1H, J=8,8 Hz); 7,22(dd, 1H, *J*=8,6, *J*'=1,6 Hz); 7,26(s, 1H), 7,42-7,55(m, 3H); 7,63(d, 1H, J=8,1 Hz); 7,70(d, 1H, J=8,2 Hz); 8,03(s, 1 H); 9,95(s, 1 H). (CDCl3) |
| 30e | (CH₃CH₂)₂N- | H | H | H | H | H | H | 2 | 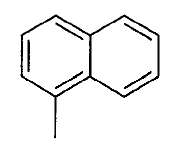 | 133-135 | | 0,87(m, 6H); 2,58(m, 4H); 2,76(m, 2H); 4,14(m, 2H); 6,24(s, 1 H); 6,73(d, 1H, J=8,8 Hz); 7,11 (s, 1 H); 7,21 (d, 1H, J=8,0 Hz); 7,29(s, 1 H); 7,50(t, 1H, J=7,8 Hz); 7,63-7,71 (m, 2H); 8,04(d, 2H, J=7,5 Hz); 8,13(d, 1H, J=8,2 Hz); 8,76(d, 1H, J=8,2 Hz); 10,21 (s, 1 H). (DMSO-d6) |
| 31e | (CH₃CH₂)₂N- | H | H | H | H | H | H | 2 | 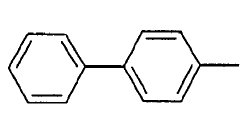 | Oil | | 0,83(m, 6H); 2,50(m, 4H); 2,70(m, 2H); 4,13(m, 2H); 6,30(d, 1H, J=2,6 Hz); 6,87(d, 1H, J=8,6 Hz); 7,24(s, 1 H); 7,30(m, 2H); 7,44(m, 3H); 7,66(d, 2H, J=7,2 Hz); 7,72(AB sys, 2H, J=8,5 Hz); 7,78(AB sys, 2H, J=8,5 Hz); 9,91 (s, 1H). (DMSO-d6) |
| 32e | (CH₃)₂N- | CH₃ | H | H | H | H | H | 2 | 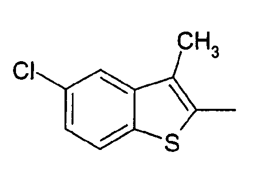 | 203-205 | | 2,13(s, 6H); 2,33(s, 6H); 2,39(t, 2H, J=7,0 Hz); 4,07(t, 2H, J=6,8 Hz); 6,08(s, 1 H); 6,76(dd, 1 H, J=8,6 , J'=2,0 Hz); 7,13(d, 1H, J=2,0 Hz); 7,20(d, 1H, J=8,6 Hz); 7,51 (dd, 1H, J=8,7, J'=2,0 Hz); 7,93(d, 1H, J=2,0 Hz); 8,00(d, 1 H, J=8,8 Hz); 10,20(s, 1H). (DMSO-d6) |
| 33e | (CH₃)₂N- | CH₃ | H | H | H | H | H | 2 | 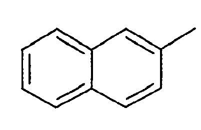 | 199-200 | | 2,11(s, 6H); 2,30(s, 3H); 2,35(t, 2H, J=7,0 Hz); 4,03(t, 2H, J=7,0 Hz); 6,03(s, 1 H); 6,75(dd, 1H, J=8,6 , J'=2,0 Hz); 7,10(d, 1H, J=2,0 Hz); 7,13(d, 1H, J=8,6 Hz); 7,54-7,67(m, 2H); 7,73(dd, 1H, J=8,6 , J'=1,8 Hz); 7,95(d, 1H, J=7,9 Hz); 8,02(d, 2H, J=8,6 Hz); 8,27(d, 1H, J=1,5 Hz); 9,89(s, 1H). (DMSO-d6) |
| 34e | (CH₃)₂N- | CH₃ | H | H | H | H | H | 2 | 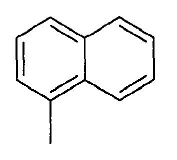 | 183-184 | | 2,12(s, 6H); 2,29(s, 3H); 2,35(t, 2H, J=7,0 Hz); 4,01(t, 2H, J=7,0 Hz); 5,98(s, 1 H); 6,62(dd, 1 H, J=8,7 , J'=1,9 Hz); 6,98(d, 1H, J=2,0 Hz); 7,07(d, 1H, J=8,6 Hz); 7,49(m, 1 H); 7,63(m, 1 H); 7,70(m, 1 H); 8,02(d, 2H, J=7,5 Hz); 8,12(d, 1H, J=8,0 Hz); 8,75(d, 1H, J=8,4 Hz); 10,15(s, 1H), (DMSO-d6) |
| 35e | (CH₃)₂N- | CH₃ | H | H | H | H | H | 2 | 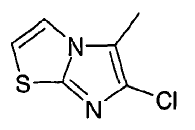 | 182-183 | | 2,14(s, 6H); 2,34(s, 3H); 2,39(m, 2H); 4,01 (m, 2H); 6,09(s, 1H); 6,70(dd, 1H, J=8,5 J' 1,8 Hz); 7,08(d, 1H, J=1,8 Hz); 7,21 (d, 1H, J=8,5 Hz); 7,51 (d, 1H, J=4,5 Hz); 7,80(d, 1 H, J=4,5 Hz). (DMSO-d6) |
| 36e | (CH₃)₂N- | CH₃ | H | H | H | H | H | 2 | 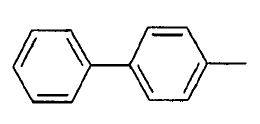 | 176-177 | | 2,14(s, 6H); 2,33(s, 3H); 2,39(t, 2H, J=7,1 Hz); 4,06(t, 2H, J=7,1 Hz); 6,07(s, 1H); 6,79(dd, 1H, J=8,8 , J'=2,0 Hz); 7,11 (d, 1H, J=1,8 Hz); 7,19(d, 1H, J=8,8 Hz); 7,36-7,48(m, 3H); 7,66(m, 2H); 7,72(AB sys, 2H, J=8,8 Hz); 7,79(AB sys, 2H, J=8,8 Hz); 9,85(s, 1 H). (DMSO-d6) |
| 37e | (CH₃)₂N- | CH₃ | H | H | H | H | H | 2 | 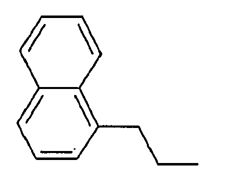 | 135-137 | | 2,17(s, 6H); 2,38(s, 3H); 2,45(m, 2H); 3,30-3,45(m, 4H); 4,14(t, 2H, J=6,7 Hz); 6,15(s, 1H); 7,04(d, 1 H, J=8,5 Hz); 7,26(m, 1 H); 7,30-7,38(m, 4H); 7,44(m, 1 H); 7,65(d, 1 H, J=8,2 Hz); 7,62(m, 1 H); 7,87(d, 1H, J=8,2 Hz); 9,56(s, 1 H). (DMSO-d6) |
| 38e | (CH₃)₂N- | CH₃ | H | H | H | H | H | 2 | 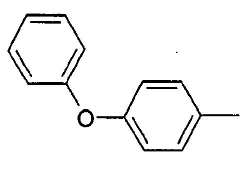 | 147-149 | | 2,20(s, 6H); 2,34(s, 3H); 2,45(m, 2H); 4,10(t, 2H, J=7,1 Hz); 6,08(s, 1 H); 6,76(dd, 1H, J=8,6 , J'=2,0 Hz); 6,99(d, 2H, J=8,8 Hz); 7,03-7,08(m, 3H); 7,17-7,24(m, 2H); 7,41(t, 2H, J=7,8 Hz); 7,63(d, 2H, J=8,8 Hz); 9,73(s, 1H). (DMSO-d6) |
| 39e | (CH₃)₂N- | CH₃ | H | H | H | H | H | 2 | 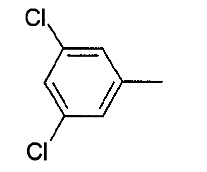 | 147-149 | | 2,15(s, 6H); 2,35(s, 3H); 2,41(t, 2H, J=6,7 Hz); 4,10(t, 2H, J=7,1 Hz); 6,12(s, 1 H); 6,71 (dd, 1 H, J=8,6, J'=2,0 Hz); 7,09(d, 1H, J=1,8 Hz); 7,24(d, 1H, J=9,0 Hz); 7,58(d, 2H, J=1,9 Hz); 7,90(t, 1 H, J=1,9 Hz). (DMSO-d6) |
| 40e | (CH₃)₂N- | CH₃ | H | H | H | H | H | 2 | 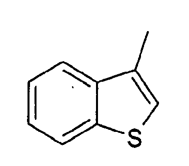 | 167-169 | | 2,14(s, 6H); 2,31(s, 3H); 2,38(m, 2H); 4,04(t, 2H, J=7,1 Hz); 6,02(s, 1 H); 6,66(dd, 1H, J=8,4, J'=1,8 Hz); 7,04(d, 1H, J=1,6 Hz); 7,12(d, 1H, J=8,2 Hz); 7,40-7,51(m, 2H); 8,03(d, 1H, J=7,6 Hz); 8,21 (d, 1H, J=7,9 Hz); 8,31(s, 1H); 10,08(s, 1 H). (DMSO-d6) |
| 41e | (CH₃CH₂)₂N- | H | H | H | H | H | H | 2 | 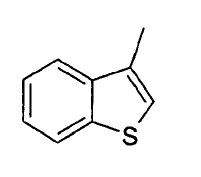 | 62-75 | | 0,98(m, 6H); 2,54(m, 4H); 2,70(m, 2H); 4,18(m, 2H); 6,29(s, 1 H); 6,77(d, 1H, J=8,5 Hz); 7,18(s, 1H); 7,34(m, 2H); 7,39-7,52(m, 2H); 8,03(d, 1 H, J=7,9 Hz); 8,22(d, 1 H, J=7,5 Hz); 8,34(s, 1 H); 10,18(s, 1 H). (CDCl3) |
| 42e | (CH₃)₂N- | H | H | H | H | H | H | 2 | 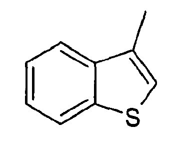 | 61-72 | | 2,12(s, 6H); 2,50(m, 2H); 4,12(t, 2H, J=6,7 Hz); 6,25(d, 1H, J=3,1 Hz); 6,75(dd, 1H, J=8,7, J'=2,1 Hz); 7,17(d, 1H, J=1,9 Hz); 7,25(d, 1H, J=8,9 Hz); 7,30(d, 1H, J=3,2 Hz); 7,48(m, 2H); 8,04(d, 1H, J=7,0 Hz); 8,24(d, 1H, J=7,4 Hz); 8,34(s, 1 H); 10,14(s, 1 H). (CDCl3) |
| 43e | 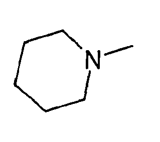 | H | H | H | H | H | H | 3 | 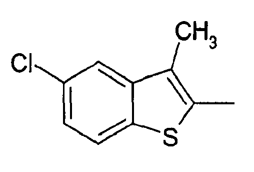 | 82-92 | | 1,20-1,55(m, 6H); 1,88(m, 2H); 2,33(s, 3H); 2,16-2,60(m, 6H); 4,10(t, 2H, J=6,6 Hz); 6,34(d, 1H, J=3,2 Hz); 6,82(d, 1H, J=9,9 Hz); 7,27-7,35(m, 3H); 7,50(dd, 1H, J=8,7, J'=2,0 Hz); 7,91 (d, 1 H, J=2,2 Hz); 7,99(d, 1H, J=8,6 Hz); 10,20(bs, 1H). (DMSO-d6) |
| 44e | 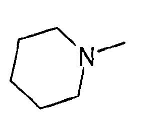 | H | H | H | H | H | H | 3 | 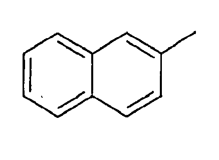 | 92-108 | | 1,20-1,55(m, 6H); 1,87(m, 2H); 2,22-2,62(m, 6H); 4,06(t, 2H, J=6,6 Hz); 6,28(d, 1H, J=2,9 Hz); 6,83(dd, 1H, J=8,7, J'=2,0 Hz); 7,24(d, 1H, J=2,0 Hz); 7,27 (m, 2H); 7,59(m, 1 H); 7,64(m, 1H); 7,75(dd, 1H, J=8,8, , J'=1,9 Hz); 7,95(d, 1H, J=7,5 Hz); 8,03( d, 2H, J=8,5 Hz); 8,28(s, 1H); 9,97(s, 1 H). (DMSO-d6) |
| 45e | 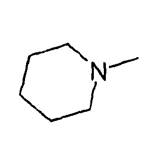 | H | H | H | H | H | H | 3 | 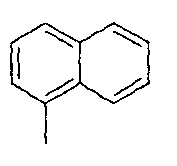 | 105-107 | | 1,25-1,55(m, 6H); 1,81 (m, 2H); 2,03-2,60(m, 6H); 4,03(t, 2H, J=6,4 Hz); 6,23(d, 1H, J=3,1 Hz); 6,70(d, 1H, J=8,9 Hz); 7,11 (d, 1H, J=1,8 Hz); 7,20(d, 1H, J=8,9 Hz); 7,24(d, 1H, *J*=3,1 Hz); 7,49(dd, 1H, J=8,1, J'=7, 4 Hz); 7,59-7,66(m, 1 H); 7,66-7,73(m, 1H); 8-8,05(m, 2H); 8,12(d, 1H, *J*=8,2 Hz); 8,75(d, H, *J*=7,8 Hz); 10,20(bs, 1H). (DMSO-d6) |
| 46e | 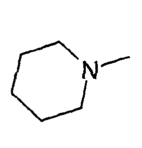 | H | H | H | H | H | H | 3 | 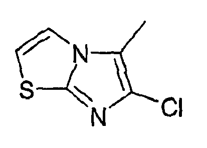 | 85-86 | | 1,36 (m, 2H); 1,49(m, 4H); 1,86(m, 2H); 2,15-2,44(m, 6H); 4,10(t, 2H, J=6,7 Hz); 6,33(d, 1H, J=3,1 Hz); 6,79(dd, 1H, J=8,7, J'=2,0 Hz); 7,21 (d, 1H, J=2,0 Hz); 7,30-7,36(m, 2H); 7,52(d, 1H, J=4,4 Hz); 7,83(d, 1 H, J=4,4 Hz); 10,25(bs, 1H). (DMSO-d6) |
| 47e | 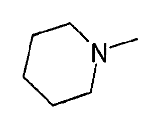 | H | H | H | H | H | H | 3 | 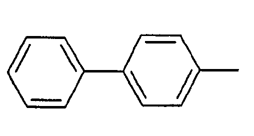 | 148-150 | | 1,34 (m, 2H); 1,47(m, 4H); 1,86(m, 2H); 2,03-2,55(m, 6H); 4,09(t, 2H, J=6,6 Hz); 6,32(d, 1 H, J=2,8 Hz); 6,87(dd, 1 H, J=8,9, J'=1,8 Hz); 7,26(d, 1H, J=1,9 Hz); 7,28-7,34(m, 2H); 7,36-7,49(m, 3H); 7,66(m, 2H); 7,73(AB sys, 2H, J=8,8 Hz); 7,79(AB sys, 2H, J=8,8 Hz); 9,91 (s, 1H). (DMSO-d6) |
| 48e | 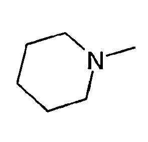 | H | H | H | H | H | H | 3 | 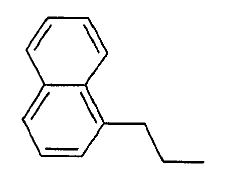 | 59-61 | | 1,20-1,56(m, 6H); 1,89(m, 2H); 2.12-2,50(m, 6H); 3,26-3,47(m, 4H); 4,16(t, 2H, J=6,2 Hz); 6,40(d, 1H, J=2,3 Hz); 7,13(d, 1H, J=8,6 Hz); 7,24(t, 1 H, J=7,5 Hz); 7,34-7,50(m, 6H); 7,64(d, 1H, J=8,4 Hz); 7,76(m, 1 H); 7,87(d, 1H, J=8,2 Hz); 9,65(s, 1H). (DMSO-d6) |
| 49e | 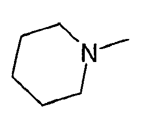 | H | H | H | H | H | H | 3 | 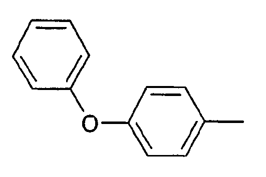 | 64-66 | | 1,20-1,58(m, 6H); 1,90(m, 2H); 2,20-2,55(m, 6H); 4,09(t, 2H, J=6,4 Hz); 6,33(s, 1H); 6,84(dd, 1H, J=8,4 Hz); 6,96-7,02(m, 2H); 7,04(d, 2H, J=7,9 Hz); 7,17-7,24(m, 2H); 7,32(m, 2H); 7,41 (m, 2H); 7,64(d, 2H, J=8,6 Hz); 9,79(s,1H). (DMSO-d6) |
| 50e | 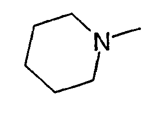 | H | H | H | H | H | H | 3 | 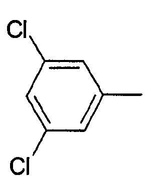 | 56-58 | | 1,35(m, 2H); 1,46(m, 4H); 1,83(m, 2H); 2,10(m, 2H); 2,24(m, 4H); 4,11 (t, 2H, J=6,4 Hz); 6,36(d, 1H, J=2,6 Hz); 6,78(dd, 1H, J=8,8, J'=1,8 Hz); 7,22(d, 1H, J=1,90 Hz); 7,33(d, 1H, J=2,9 Hz); 7,37(d, 1H, J=8,8 Hz); 7,58(d, 2H, J=8,6 Hz); 7,89(t, 1H, J=1,9 Hz); 9,99(s, 1H). (DMSO-d6) |
| 51e | 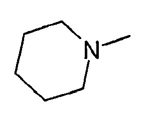 | H | H | H | H | H | H | 3 | 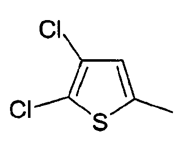 | 70-72 | | 1,38(m, 2H); 1,52(m, 4H); 1,91(m, 2H); 2,24(m, 2H); 2,37(m, 4H); 4,16(t, 2H, J=6,6 Hz); 6,42(d, 1H, J=2,5 Hz); 6,89(d, 1H, J=8,6 Hz); 7,32(s, 1 H); 7,37(d, 1H, J=2,8 Hz); 7,43(d, 1H, J=8,6 Hz); 7,48(s, 1H). (DMSO-d6) |
| 52e | 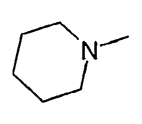 | H | H | H | H | H | H | 3 | 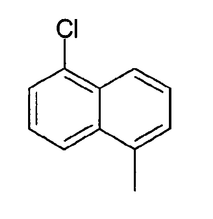 | 92-96 | | 1,13-1,70(m, 6H); 2,00(m, 2H); 2,68(m, 2H); 2,84(m, 2H); 3,26(m, 2H); 4,05(m, 2H); 6,22(d, 1H, J=3,1 Hz); 6,71(m, 1H); 7,10-7,18(m, 3H); 7,52-7,53(m, 2H); 7,72(m, 1 H); 8,15(d, 1H, J=7,3 Hz); 8,37(d, 1H, J=8,5 Hz); 8,77(d, 1 H, J=8,5 Hz); 8,97(bs, 1 H); 10,23(bs, 1 H). (DMSO-d6 + TFA) |

### Preparation of the compounds of general formula (If):

### Example 1f.- Preparation of N-[1-(2-dimethylaminoethyl)-1H-indole-6-yl]-5-chloro-3-methyl-benzo[b]thiophene-2-sulfonamide.

185.6 mg (0.66 mmol) of 5-chloro-3-methyl-benzo[b] thiophene-2-sulfonyl chloride were added to a solution of 122 mg (0.6 mmol) of 6-amino-1-(2-dimethylaminoethyl)-1 H-indole in 2 ml of dimethylformamide and 116 mg of N-ethyldiisopropylamine. The reaction mixture is stirred at the room temperature for 20 hours. Then it is evaporated to dryness, slightly alkalinized with sodium bicarbonate solution and extracted with chloroform. The organic phase is repeatedly washed with water and saturated solution of sodium bicarbonate, it is separated and dried with anhydrous sodium sulfate. The organic solution is evaporated to dryness and the resulting solid is purified by chromatography, obtaining 180 mg (67%) of N-[1-(2-dimethylaminoethyl)-1H-indole-6-yl]-5-choloro-3-methyl-benzo[b]thiophene-2-sulfonamide as an amorphous solid.

### Example 2f.- Preparation of N-[1-(2-dimethylaminoethyl)-1 H-indole-6-yl]-naphtalene-2-sulfonamide

187 mg (80%) of the mentioned compound are obtained from 122 mg (0.6 mmol) of 6-amino-1-(2-dimethylaminoethyl)-1H-indole and 150 mg (0.66 mmol) of 2-naphtalenesulfonyl chloride, by means of the process described in the Example 1f, as a solid.

### Example 3f.- Preparation of N-[1-(2-dimethylaminoethyl)-1H-indole-6-yl]-naphtalene-1-sulfonamide

157 mg (67%) of the mentioned compound are obtained from 122 mg (0.6 mmol) of 6-amino-1-(2-dimethylaminoethyl)-1H-indole and 150 mg (0.66 mmol) of 1-naphtalenesulfonyl chloride, by means of the process described in the Example 1f, as a solid.

### Example 4f.- Preparation of N-[1-(2-dimethylaminoethyl)-1H-indole-6-yl]-6-chloroimidazo[2,1-b]thiazole-5-sulfonamide

170 mg (67%) of the mentioned compound are obtained from 122 mg (0.6 mmol) of 6-amino-1-(2-dimethylaminoethyl)-1 H-indole and 170 mg (0.66 mmol) of 6-chloroimidazo[2,1-b]thiazole-5-sulfonyl chloride, by means of the process described in the Example 1f, as a solid.

### Example 5f.- Preparation of N-[1-(2-dimethylaminoethyl)-1 H-indole-6-yl]-4 phenylbenzenesulfonamide

184 mg (73%) of the mentioned compound are obtained from 122 mg (0.6 mmol) of 6-amino-1-(2-dimethylaminoethyl)-1 H-indole and 167 mg (0.66 mmol) of 4- phenylbenzenesulfonyl chloride, by means of the process described in the Example 1f, as a solid.

### Example 6f.- Preparation of N-[1-(2-dimethylaminoethyl)-1H-indole-6-yl]-2-(naphthalene-1-yl)-ethanesulfonamide

100 mg (40%) of the mentioned compound are obtained from 122 mg (0.6 mmol) of 6-amino-1-(2-dimethylaminoethyl)-1H-indole and 168 mg (0.66 mmol) of 2-(naphthalene-1-yl)- ethanesulfonyl chloride, by means of the process described in the Example 1 f, as a solid.

### Example 7f.- Preparation of N-[1-(2-dimethylaminoethyl)-1 H-indole-6-yl]-4-phenoxybenzenesulfonamide

190 mg (73%) of the mentioned compound are obtained from 122 mg (0.6 mmol) of 6-amino-1-(2-dimethylaminoethyl)-1 H-indole and 177 mg (0.66 mmol) of 4-phenoxybenzenesulfonyl chloride, by means of the process described in the Example 1f, as a solid.

### Example 8f.- Preparation of N-[1-(2-dimethylaminoethyl)-1H-indole-6-yl]-3,5-dichlorobenzenesulfonamide

100 mg (41%) of the mentioned compound are obtained from 122 mg (0.6 mmol) of 6-amino-1-(2-dimethylaminoethyl)-1H-indole and 162 mg (0.66 mmol) of 3,5-dichlorobenzenesulfonyl chloride, by means of the process described in Example 1f, as a solid.

### Example 9f.- Preparation of 5-Chloro-3-methyl-N-[1-[2-(pyrrolidin-1-yl)ethyl-1H-indol-6-yl]-benzo[b]thiophene-2-sulfonamide

165 mg (58 %) of the mentioned compound were obtained from 137 mg (0.6 mmol) of 6-amino-1-(2-(pyrrolidin-1-yl)ethyl)-1H-indol and 186 mg (0.66 mmol) of 5-chloro-3-methyl-benzo[b]-thiophene-2-sulfonyl chloride by means of the process described in Example 1 as a solid.

### Example 10f.- Preparation of N-(1-[2-(Pyrrolidin-1-yl)ethyl]-1H-indol-6-yl]-napthyl-2-sulfonamide

142 mg (57 %) of the mentioned compound were obtained from 137 mg (0.6 mmol) of 6-amino-1-(2-(pyrrolidin-1-yl)ethyl)-1 H-indol and 150 mg (0.66 mmol) naphthalenesulfonyl chloride by means of the process described in Example 1 as a solid.

### Example 11f.- Preparation of N-[1-[2-Pyrrolidin-1-yl]ethyl]-1H-indol-6-yl]-naphthalene-1-sulfonamide

166 (66 %) of the mentioned compound were obtained from 137 mg (0.6 mmol) of 6-amino-1-(2-(pyrrolidin-1-yl)ethyl)-1H-indol and 150 mg (0.66 mmol) naphthalenesulfonyl chloride by means of the process described in Example 1 as a solid.

### Example 12f.- Preparation of 6-Chloro-N-[1-[2-(pyrrolidin-1-yl)ethyl]-1H-indol-6-yl]-imidazo[2,1-b]thiazole-5-sulfonamide

170 mg (59%) of the mentioned compound were obtained from 137 mg (0.6 mmol) of 6-amino-1-(2-(pyrrolidin-1-yl)ethyl)-1 H-indol and 170 mg 6-chloroimidazo[2,1-b]thiazole-5-sulfonyl chloride by means of the process described in Example 1 as a solid.

### Example 13f.- Preparation of 4-Phenyl-N-(1-(2-(pyrrolidin-1-yl)ethyl)-1H-indol-6-yl)benzenesulfonamide

205 mg (77%) of the mentioned compound were obtained from 137 mg (0.6 mmol) of 6-amino-1-(2-(pyrrolidin-1-yl)ethyl)-1 H-indol and 169 mg (0.66 mmol) of 4-phenylbenzenesulfonyl chloride by means of the process described in Example 1 as an oil.

### Example 14f.- Preparation of 2-(Naphthyl-1-yl)-N-(1-(2-(pyrrolidin-1-yl)-ethansulfonamid

182 mg (68%) of the mentioned compound were obtained from 137 mg (0.6 mmol) of 6-amino-1-(2-(pyrrolidin-1-yl)ethyl)-1H-indol and 182 mg (0.66 mmol) of 2-naphthalene-ethansulfonyl chloride by means of the process described in Example 1 as a solid.

### Example 15f.- Preparation of 4-Phenoxy-N-(1-(2-(pyrrolidin-1-yl)ethyl)-1H-indol-6-yl)-benzenesulfonamide

185 mg (67%) of the mentioned compound were obtained from 137 mg (0.6 mmol) of 6-amino-1-(2-(pyrrolidin-1-yl)ethyl)-1H-indol and 177 mg (0.66 mmol) of 4-phenoxybenzenesulfonyl chloride by means of the process described in Example 1 as a solid.

### Example 16f.-Preparation of 3,5-Dichloro-N-(1-(2-(Pyrrolidin-1-yl)-1 H-indol-6-yl)-benzenesulfonamide

122 mg (46%) of the mentioned compound were obtained from 137 mg (0.6 mmol) of 6-amino-1-(2-(pyrrolidin-1-yl)ethyl)-1 H-indol and 162 mg (0.66 mmol) of 3,5-dichlorobenzenesulfonyl chloride by means of the process described in Example 1 as a solid.

The yields are indicative and no added effort was made to improve them.

The melting point and spectroscopic data for identifying some of the compounds of the present invention are indicated in the following table.

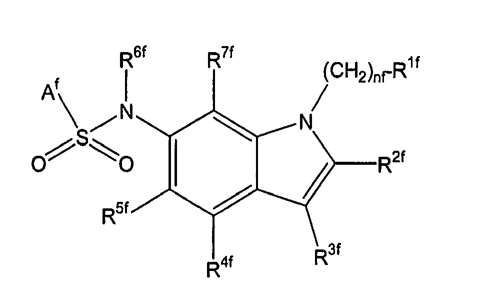

| Ex | R^{1f} | R^{2f} | R^{3f} | R^{4f} | R^{5f} | R^{6f} | R^{7f} | nf | A^{f} | m.p. °C | IR cm⁻¹ | ¹H-NMR (300MHz), δ (solvent) |
|---|---|---|---|---|---|---|---|---|---|---|---|---|
| 1f | (CH₃)₂N- | H | H | H | H | H | H | 2 | 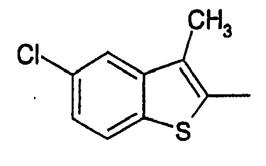 | amorphous | 3422, 3247, 2943,1467, 1340, 1158, 1114, 1080, 862, 651,557. | 2.19 (s, 9H); 2.55 (t, 2H, J=6.7 Hz); 4.13 (t, 2H, J=6.7 Hz); 6.42 (d, 1 H, 3.1 Hz); 6.69 (dd, 1H, J=8.3 Hz, J'=1.9 Hz); 7.13 (d, 1H, 3.1Hz); 7.23 (m, 1H); 7.45-7.37 (m, 2H); 7.63 (d, 1 H, J=2.0 Hz); 7. 69 (d, 1H, J=8.6 Hz). (DMSO-d6) |
| 2f | (CH₃)₂N- | H | H | H | H | H | H | 2 | 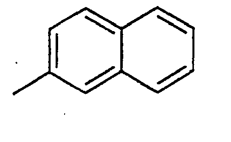 | 140-143 | 3422, 3246, 2935, 2760, 1468, 1336, 1159, 1132, 1074, 753, 711, 678, 553. | 2.19 (s, 6H); 2.55 (t, 2H, J=7.0 Hz); 4.11 (t, 2H, J=7.0 Hz); 6.39 (d, 1 H, J=3.1 Hz); 6.67 (dd, 1 H, J=8.3 Hz, J'=1.4 Hz); 7.10 (d, 1H, J=3.1 Hz); 7.19 (s, 1 H); 7.39 (d, 1H, J=8.4 Hz); 7.49-7.65 (m, 2H); 7.69 (dd, 1 H, J=8.9 Hz, J'=1.6 Hz); 7.81-7.88 (m, 3H); 8.29 (s, 1 H). (DMSO-d6) |
| 3f | (CH₃)₂N- | H | H | H | H | H | H | 2 | 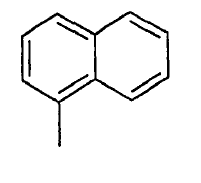 | 139-142 | 3437,2943, 1507,1461, 1330,1192, 1162, 1135, 961,813, 763, 580, 472. | 2.21 (s, 6H); 2.50 (t, 2H, J=7.0 Hz); 4.03 (t, 2H, J=7.0 Hz); 6.35 (d. 1H, J=3.1 Hz); 6.48 (dd, 1H, J=8.4 Hz, J'=1.7 Hz); 7.00 (s, 1 H); 7.05 (d, 1H, J=3.1Hz); 7.29 (m, 1H); 7.37 (t, 1H, J=7.8 Hz); 7. 60 (m, 1H); 7.67 (m, 1H); 7.92 (d, 1H, J=8.1 Hz); 7.98 (d, 1H, J=8.1Hz); 8.10 (d, 1H, J=7.3 Hz); 8.73 (d, 1H, J=8.8 Hz). (DMSO-d6) |
| 4f | (CH₃)₂N- | H | H | H | H | H | H | 2 | 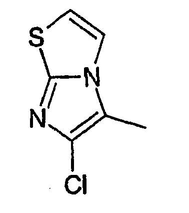 | amorphous | 3448, 3110, 2814, 1459, 1325, 1250, 1178, 1141, 621. | 2.28 (s, 6H); 2.61 (t, 2H, J=7.0 Hz); 4.14 (t, 2H, J=7.0 Hz); 6.41 (d, 1H, J=3.1 Hz); 6.81 (m, 2H); 7.12 (d, 1H, 3.1 Hz); 7.19 (m, 1 H); 7.42 (d, 1H, J=8.2 Hz); 7.56 (d, 1H, J=4.6 Hz) (DMSO-d6) |
| 5f | (CH₃)₂N- | H | H | H | H | H | H | 2 | 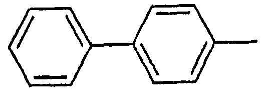 | amorphous | 3256, 2951, 2776, 1468, 1333,1159, 1095, 763, 670, 591. | 2.24 (s, 6H); 2.62 (t, 2H, J=7.0 Hz); 4.15 (t, 2H, J=7.0 Hz); 6.42 (d, 1H, J=3.1Hz); 6.70 (d, 1H, J=8.4 Hz); 7.12 (d, 1H, J=3.1 Hz); 7.25 (d, 1H, J=3.3 Hz); 7.34-7.48 (m, 4H); 7.53 (m, 2H); 7.59 (AB sys, 2H, J=8.3 Hz); 7.78 (AB sys, 2H, J=8.3 Hz). (DMSO-d6) |
| 6f | (CH₃)₂N- | H | H | H | H | H | H | 2 | 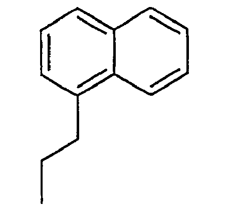 | amorphous | 3254, 2944, 1509,1468, 1326, 1147, 970, 782, 716,540. | 2.15 (s, 6H); 2.62 (t, 2H, J=7.1 Hz); 3.38 (m, 2H); 3.49 (m, 2H); 4.22 (t, 2H, J=7.1 Hz); 6.47 (d, 1 H, J=2.8Hz); 7.04 (m, 2H); 7.23 (d, 1H, J=3.1Hz); 7.26-7.45 (m, 5H); 7.56 (d, 1H, J=8.4 Hz); 7. 68 (dd, 1 H, J=7.5 Hz, J'=7.5 Hz); 7.77 (d, 1H, J=8.3Hz). (DMSO-d6) |
| 7f | (CH₃)₂N- | H | H | H | H | H | H | 2 | 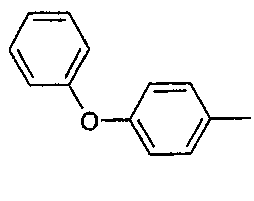 | amorphous | 3255, 2935, 2768, 1583, 1488, 1334, 1245,1154, 1093, 694, 570, 539. | 2.28 (s, 6H); 2.65 (t, 2H, J=7.0 Hz); 4.16 (t, 2H, J=7.0 Hz); 6.42 (d, 1 H, J=3.0Hz); 6.65 (dd, 1 H, J=8.4 Hz, J'=1.7 Hz); 6.90 (AB sys, 2H, J=8.8 Hz); 7.00 (AB sys, 2H, J=7.9 Hz); 7.13 (d, 1 H, J=3.1 Hz); 7.19 (m, 1H); 7.24 (m, 1H); 7.37 (m, 2H); 7.43 (d, 1H, J=8.3Hz); 7.65 (AB sys, 2H, J=8.9 Hz). (DMSO-d6) |
| 8f | (CH₃)₂N- | H | H | H | H | H | H | 2 | 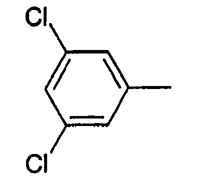 | 150-159 | 3437,3072, 2920, 1568, 1471,1346, 1303, 1171, 1140, 799, 670, 598. | 2.29 (s, 6H); 2.66 (t, 2H, J=6.8 Hz); 4.18 (t, 2H, J=6.8 Hz); 6.45 (d, 1 H); 6.67 (d, 1H, J=8.4Hz); 7.15 (m, 1 H); 7.19 (m, 1 H); 7.46 (m, 2H); 7.59 (m, 2H). (DMSO-d6) |

| Ex | R1 | R2 | R3 | R4 | R5 | R6 | R7 | n | A | p.f. °C | 1H-RMN (300 MHz), δ (disolvente) |
|---|---|---|---|---|---|---|---|---|---|---|---|
| 9f | 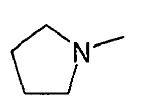 | H | H | H | H | H | H | 2 | 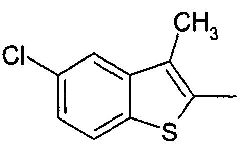 | 69-71 | 1,58(m, 4H); 2,31 (m, 4H); 2,36(s, 3H); 2,59(m, 2H); 4,11(m, 2H); 6,31(s, 1H); 6,79(d, 1H, J=8,4 Hz); 7,09(s, 1 H); 7,29(d, 1H, J=2,3 Hz); 7,38(d, 1H, J=8,5 Hz); 7,51 (d, 1 H, J=8,6 Hz); 7,94(d, 1H, J=1,0 Hz); 8,00(d, 1H, J=8,35 Hz); 10,39(b, 1H). (DMSO-d6) |
| 10f | 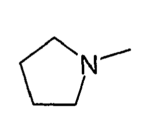 | H | H | H | H | H | H | 2 | 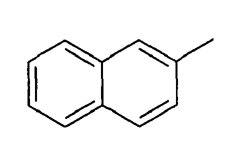 | 54-60 | 1,54(m, 4H); 2,24(m, 4H); 2,50(m, 2H); 4,06(m, 2H); 6,25(s, 1H); 6,77(d, 1H, J=8,4 Hz); 7,07(s, 1 H); 7,23(m, 1H); 7,32(d, 1H, J=8,1 Hz); 7,61 (m, 2H); 7,75(d, 1H, J=8,8 Hz); 7,95(d, 1 H, J=7,6 Hz); 8,03(m, 2H); 8,34(s, 1 H); 10,11 (b, 1H). (DMSO-d6) |
| 11f | 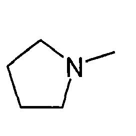 | H | H | H | H | H | H | 2 | 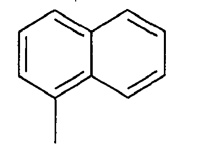 | 160-165 | 1,74(m, 4H); 2,71(m, 4H); 2,94(m, 2H); 4,24(m, 2H); 6,27(d, 1H, J=2,8 Hz); 6,61 (d, 1H, J=8,6 Hz); 7,09(s, 1H); 7,24(d, 1H, J=8,5 Hz); 7,28(d, 1H, J=2,8 Hz); 7,54(t, 1H, J=7,9 Hz); 7,63(m, 1 H); 7,71 (m 1H); 8,03(d, 1H, J=7,6 Hz); 8,11-8,23(m, 2H); 8,77(d, 1H, J=8,2 Hz); 10,46(b, 1 H). (DMSO-d6) |
| 12f | 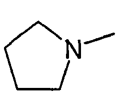 | H | H | H | H | H | H | 2 | 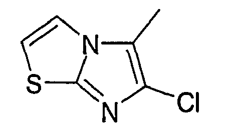 | 53-57 | 1,64(m, 4H); 2,50(m, 4H); 2,70(m, 2H); 4,14(m, 2H); 6,31 (d, 1H, J=2,8 Hz); 6,71(d, 1H, J=8,8 Hz); 7,11 (s, 1H); 7,31(d, 1H, J=2,9 Hz); 7,37(d, 1 H, J=8,6 Hz); 7,56(d, 1H, J=4,4 Hz); 7,91 (d, 1H, J=4,5 Hz); 10,63(b, 1 H). (DMSO-d6) |
| 13f | 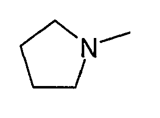 | H | H | H | H | H | H | 2 | 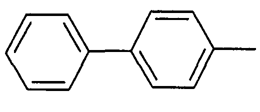 | 178-181 | 1,55(m, 4H); 2,33(m, 4H); 2,61 (m, 2H); 4,11 (m, 2H); 6,30(d, 1H, J=2,8 Hz); 6,79(dd, 1H, J=8,2 , J'=1,6 Hz); 7,09(s, 1H); 7,28(d, 1H, J=2,8 Hz); 7,34-7,49(m, 4H); 7,67(d, 2H, J=7,0 Hz); 7,76(AB sys, 2H, J=8,7 Hz); 7,80(AB sys, 2H, J=8,7 Hz); 10,05(bs, 1H). (DMSO-d6) |
| 14f | 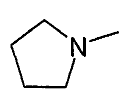 | H | H | H | H | H | H | 2 | 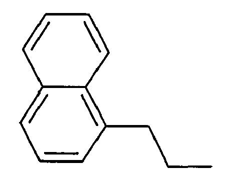 | oil | 1,49(m, 4H); 2,31 (m, 4H); 2,66(t, 2H, J=6,5 Hz); 3,3 (m, 4H); 4,16(t, 2H, J=6,5 Hz); 6,40(dd, 1H, J=3,1 J'=0,7 Hz); 7,04(dd, 1H, J=8,4 , J'=1,8 Hz); 7,13(m, 1H); 7,33-7,44(m, 5H); 7,48(d, 1H, J=8,6 Hz); 7,52(d, 1H, J=8,4 Hz); 7,75(t, 1H, J=4,8 Hz); 7,85(d, 1H, J=8,1 Hz); 9,84(s, 1H). (DMSO-d6) |
| 15f | 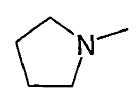 | H | H | H | H | H | H | 2 | 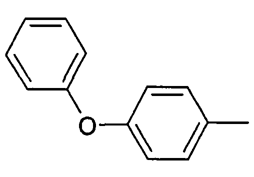 | 59-62 | 1,61(m, 4H); 2,41(m, 4H); 2,66(t, 2H, J=6,5 Hz); 4,12(t, 2H, J=6,5 Hz); 6,30(d, 1H, J=2,8 Hz); 6,75(dd, 1H, J=8,4, J'=1,4 Hz); 6,99(d, 2H, J=8,8 Hz); 7,04(d, 2H, J=7,9 Hz); 7,10(s, 1 H); 7,21 (t, 1 H, J=7,4 Hz); 7,29(d, 1H, J=3,1 Hz); 7,36(d, 1H, J=8,5 Hz); 7,41 (t, 2H, J=7,9 Hz); 7,69(d, 2H, J=8,8 Hz); 9,98(bs, 1H). (DMSO-d6) |
| 16f | 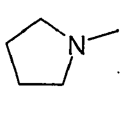 | H | H | H | H | H | H | 2 | 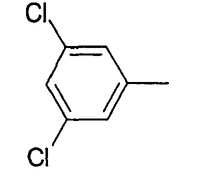 | 145-157 | 1,62(m, 4H); 2,39(m, 4H); 2,64(t, 2H, J=6,7 Hz); 4,15(t, 2H, J=6,7 Hz); 6,32(d, 1H, J=3,1 Hz); 6,73(dd, 1H, J=8,4, J'=1,8 Hz); 7,10(s, 1H); 7,33(d, 1H, J=3,2 Hz); 7,40(d, 1H, J=8,5 Hz); 7,63(d, 2H, J=1,9 Hz); 7,90(t, 1 H, J=1,9 Hz); 10,20(bs, 1H). (DMSO-d6) |

### Examples:

### Example 1g.- Preparation of N-[1-(2-dimethylaminoethyl)-1H-indole-7-yl]-naphtalene-1-sulfonamide.

149.5 mg (0.66 mmol) of naphtalene-1-sulfonyl chloride were added to a solution of 122 mg (0.6 mmol) of 7-amino-3-(2-dimethylaminoethyl)-1 H-indole in 2 ml of dimethylformamide and 116 mg of N-ethyldiisopropylamine. The reaction mixture was stirred at the room temperature for 20 hours. Then it was evaporated to dryness, slightly alkalinized with sodium bicarbonate solution and extracted with chloroform. The organic phase was repeatedly washed with water and saturated solution of sodium bicarbonate, it was separated and dried with anhydrous sodium sulfate. The organic solution was evaporated to dryness and the resulting solid was purified by chromatography, obtaining 120 mg (51%) of N-[1-(2-dimethylaminoethyl)-1H-indole-7-yl]-naphtalene-1-sulfonamide as a solid cream.

### Example 2g.- Preparation of N-[1-(2-dimethylaminoethyl)-1H-indole-7-yl]-5-chloro-3-methyl-benzo[b]thiophene-2-sulfonamide

80 mg (30%) of the mentioned compound are obtained from 122 mg (0.6 mmol) of 7-amino-1-(2-dimethylaminoethyl)-1 H-indole and 166 mg (0.66 mmol) of 5-chloro-3-methyl-benzo[b]thiophene-2-sulfonyl chloride, by means of the process described in the Example 1 g, as a yellowish solid.

### Example 3g.- Preparation of N-[1-(2-dimethylaminoethyl)-1 H-indole-7-yl]-4 phenylbenzenesulfonamide

27 mg (11 %) of the mentioned compound are obtained from 122 mg (0.6 mmol) of 7-amino-1-(2-dimethylaminoethyl)-1 H-indole and 167 mg (0.66 mmol) of 4-phenylbenzenesulfonyl chloride, by means of the process described in the Example 1g, as a solid cream.

### Example 4g.- Preparation of N-[1-(2-dimethylaminoethyl)-1H-indole-7-yl]-6-chloroimidazo[2,1-b]thiazole-5-sulfonamide

69 mg (27%) of the mentioned compound are obtained from 122 mg (0.6 mmol) of 7-amino-1-(2-dimethylaminoethyl)-1 H-indole and 170 mg (0.66 mmol) of 6-chloroimidazo[2,1-b]thiazole-5-sulfonyl chloride, by means of the process described in the Example 1g, as a solid cream.

### Example 5g.- Preparation of 5-chloro-3-methyl-N-(1-(2-(pyrrolidinyl)ethyl)-1 H-indol-7-yl)-benzo[b]thiophen-2-sulfonamide

146 mg (51%) of the mentioned compound were obtained from 137 mg (0.6 mmol) of 7-amino-1-(2-pyrrolidin-1-yl)ethyl)-1 H-indole and 186 mg (0.66 mmol) of 5-chloro-3-methyl-benzo[b]thiophen-2-sulfonyl chloride via the process described in Example 1, as a solid.

### Example 6g.-Preparation of N-(1-(2-(pyrrolidin-1-yl)ethyl)-1 H-indol-7-yl)naphthalene-1-sulfonamide

120 mg (48 %) of the mentioned compound were obtained from 137 mg (0.6 mmol) of 7-amino-1-(2-pyrrolidin-1-yl)ethyl)-1 H-indole and 150 mg (0.66 mmol) of naphthalene-1-sulfonyl chloride via the process described in Example 1, as a solid.

The yields are indicative and no added effort was made to improve them.
The melting point and spectroscopic data for identifying some of the compounds object of the present invention are indicated in the following table.

### Example 7g. Preparation of 6-chloro-N-(1-(2-(pyrroldin-1-yl)ethyl)-1H-indol-7-yl)imidazo[2,1-b]thiazole-5-sulfonamide

100 mg (37 %) of the mentioned compound were obtained from 137 mg (0.6 mmol) of 7-amino-1-(2-pyrrolidin-1-yl)ethyl)-1 H-indole and 170 mg (0.66 mmol) 6-chloro-imidazo[2,1-b]thiazole-5-sulfonyl chloride via the process described in Example 1, as a solid.

### Example 8g. Preparation of 2-(naphth-1-yl)-N-(1-(2-(pyrrolidin-1-yl)ethyl)-1 H-indol-7-yl)ethansulfonamide

130 mg (49 %) of the mentioned compound were obtained from 137 mg (0.6 mmol) of 7-amino-1-(2-pyrrolidin-1-yl)ethyl)-1 H-indole and 168 mg (0.66 mmol) of 2-(naphth-1-yl)ethansulfonyl chloride via the process described in Example 1, as a solid.

The yields are indicative and no added effort was made to improve them.
The melting point and spectroscopic data for identifying some of the compounds object of the present invention are indicated in the following table.

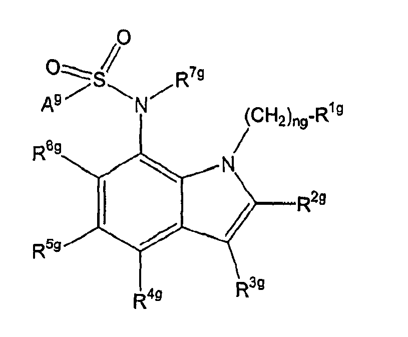

| Ex | R¹⁹ | R^{2g} | R^{3g} | R^{4g} | R^{5g} | R^{6g} | R^{7g} | ng | A^{g} | m.p. °C | IR cm⁻¹ | ¹H-NMR (300 MHz),δ (solvent) |
|---|---|---|---|---|---|---|---|---|---|---|---|---|
| 1g | (CH₃)₂N- | H | H | H | H | H | H | 2 | 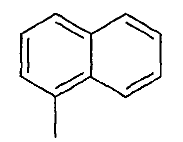 | 54-58 | 3422,3057, 2943, 1489, 1315, 1158, 1132, 772, 581. | 2,42(s, 6H); 2,89(t, 2H, J=6,4 Hz); 4,88(t, 2H, J=6,4 Hz); 6,17(d, 1H, J=7,6 Hz); 6,44(d, 1H, J=3,1 Hz); 6,60(t, 1H, J=7,8 Hz); 7,16(d, 1 H, J=3,3 Hz); 7,32(dd, 1H, J=7,9 Hz, J'=0,9 Hz); 7,53(m, 1 H); 7,63-7,67(m, 2H); 8,04-8,09(m, 2H); 8,17(d, 1H, J=8,4 Hz); 8,75(m, 1H).(CD₃OD) |
| 2g | (CH₃)₂N- | H | H | H | H | H | H | 2 | 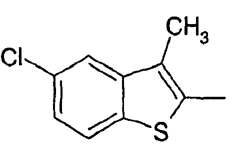 | 57-65 | 3448, 2951, 1488, 1315, 1278,1150, 1113, 1079, 861, 728, 648, 559. | 2,40(s, 6H); 2,52(s, 3H); 3,08(t, 2H, J=5,7 Hz); 4,66(t, 2H, J=5,7 Hz), 6,36(d, 1H, 3,1 Hz); 6, 70(m, 2H); 7,15(dd, 1H, J=7,0 Hz, J'=1,7Hz); 7,24(d, 1H, J=3,1 Hz); 7,49(dd, 1H, J=8,6 Hz, J'= 2,0 Hz); 7,91 (d, 1H, J=2,0 Hz); 8,00(d, 1H, J=8,8 Hz). (DMSO-d6) |
| 3g | (CH₃)₂N- | H | H | H | H | H | H | 2 | 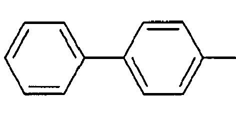 | 137-140 | 2943,1481, 1332,1316, 1158, 1096, 764, 729, 668, 581. | 2,33(s, 6 H); 2,78(m, 2 H); 4,24(m, 2 H); 6,46(d, 1H, J=3,1 Hz); 6,88(d, 1H, J=3,1 Hz); 7,00(t, 1H, J=7,8 Hz); 7,17(d, 1H, J=7,5 Hz); 7,40-7,49(m, 4H); 7,58 (m , 2H); 7,64(AB sys, 2H, J=8,4 Hz); 7,86(AB sys, 2H, J=8,4 Hz,). (CDCl₃). |
| 4g | (CH₃)₂N- | H | H | H | H | H | H | 2 | 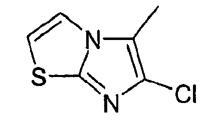 | 73-76 | 3448, 3110, 2928, 1485, 1459, 1318, 1270, 1238, 1182, 1124, 1091,723,622. | 2,66(s, 6 H); 3,28(t, 2 H, J=5,4 Hz); 4,74(t, 2 H); 6,30(d, 1H, J=3,1 Hz); 6,64-6,70(m, 2H); 7,01 (dd, 1H, J=6,5 Hz, J'=2,4 Hz); 7,19(d, 1H, J=3,1 Hz); 7,45(d, 1H, J=4,5 Hz); 7,89(d, 1H, J=4,5 Hz). (DMSO-d6) |
| 5g | 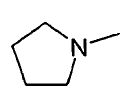 | H | H | H | H | H | H | 2 | 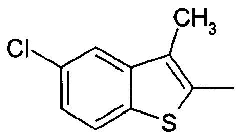 | 82-85 | | 1,87(m, 4H); 2,41(s, 3H); 3,02(m, 4H); 3,34(m, 2H); 4,70(m, 2H); 6,34(d, 1H, J=0,9 Hz); 6,62-6,80(m, 2H); 7,09(d, 1H, J=7,47 Hz); 7,21 (s, 1 H); 7,46(d, 1 H, J=8,2 Hz); 7,87(s, 1 H); 7,97(d, 1H, J=8,6 Hz). (DMSO-d6) |
| 6g | 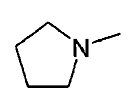 | H | H | H | H | H | H | 2 | 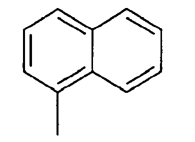 | 196-199 | | 1,79(m, 4H); 2,79(m, 4H); 3,18(m, 2H); 4,66(m, 2H); 6,30(d, 1H, J=8,3 Hz); 6,35(d, 1H, J=1,6 Hz); 6,60(m, 1H); 7,14(d, 1H, J=8,1 Hz); 7,25(m, 1 H); 7,56(m, 1 H); 7,60-7,74(m, 2H); 8,05(m, 2H); 8,16(d, 1H, J=8,2 Hz); 8,79(d, 1H, J=8,64 Hz). (DMSO-d6) |
| 7g | 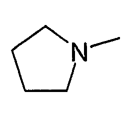 | H | H | H | H | H | H | 2 | 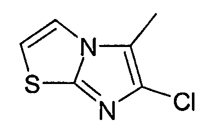 | 92-95 | | 1,84(m, 2H); 1,98(m, 2H); 3,04(m, 2H); 3,58(m, 4H); 4,87(t, 2H, J=6,7 Hz); 6,15(d, 1H, J=7,8 Hz); 6,49(d, 1H, J=2,6 Hz); 6,73(t, 1H, J=7,6 Hz);7,33-7,43(m, 3H, J=5,3 Hz); 7,46(d, 1H, J=7,9 Hz); 9,83(bs, 1 H); 10,32(s, 1 H). (DMSO-d6 + TFA ) |
| 8g | 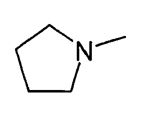 | H | H | H | H | H | H | 2 | 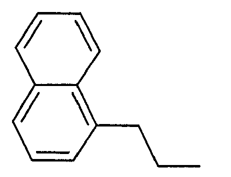 | 46-49 | | 1,69(m, 4H); 2,59(m, 4H); 2,90(m, 2H); 3,53(m, 4H); 4,65(t, 2H, J=6,2 Hz); 6,45(d, 1 H, J=3,1 Hz); 6,94(t, 1 H, J=7,6 Hz); 7,05(m, 1 H); 7,35(d, 1H, J=3,1 Hz); 7,39-7,56(m, 5H); 7,83(d, 1H, J=7,6 Hz); 7,94(m, 2H). (DMSO-d6) |

### Examples:

### Example 1h.- Preparation of 1-cyclohexanesulfonyl-3-(1-methyl-1,2,3,6-tetrahydropyridine-4-yl)-5-nitro-1 H-indole

468 mg (9.8 mmol) of 50% sodium hydride in oil were added at 0°C to a solution of 1.0 g (3.9 mmol) of 3-(1-methy)-1,2,3,6-tetrahydropyridine-4-yl)-5-nitro-1 H-indole in 50 ml of anhydrous dimethylformamide, and the mixture was left to stir for 30 minutes. Then 2.14 g of cyclohexanesulfonyl chloride were added, and the stirring continued for 3 hours at room temperature. Water was added and evaporated to dryness. The resulting crude was treated with sodium bicarbonate and was extracted with chloroform. The organic phase was dried with anhydrous sodium sulfate and evaporated to dryness; the resulting solid was purified by chromatography, obtaining 900 mg (57%) of 1-cyclohexanesulfonyl-3-(1-methyl-1,2,3,6-tetrahydropyridine-4-yl)-5-nitro-1 H-indole as a yellow solid.

### Example 2h.- 5-chloro-1-cyclohexanesulfonyl-3-(1-methyl-1,2,3,6-tetrahydropyridine-4-yl)-1 H-indole

900 mg (74%) of the mentioned compound were obtained from 770 mg (3.12 mMol) of 5-chloro-3-(1-methyl-1,2,3,6-tetrahydropyridine-4-yl)-1 H-indole, and 1.7 g (9.36 mmol) of cyclohexanesulfonyl chloride by means of the process described in Example 1h, as a yellow solid.

### Example 3h.- 5-amino-1-cyclohexanesulfonyl-3-(1-methyl-1,2,3,6-tetrahydropyridine-4-yl)-1 H-indole

200 mg of 50% Pd/C with a humidity of 5% were added to a solution of 403 mg (1 mmol) of 1-cyclohexanesulfonyl-3-(1-methyl-1,2,3,6-tetrahydropyridine-4-yl)-5-nitro-1 H-indole in 200 ml of ethanol. The resulting suspension was hydrogenized at 25 psi of overpressure for 20 hours. Then the catalyst was filtered and evaporated to drying. The resulting crude was purified by chromatography and 150 mg (40%) of the mentioned compound were obtained as a solid cream.

### Example 4h.- Preparation of 1-cyclohexanesulfonyl-5-fluoro-3-(1,2,3,5,8,8a-hexahydro-indolizine-7-yl)-1H-indole

1.95 g (78%) of 1-cyclohexanesulfonyl-5-fluoro-3-(1,2,3,5,8,8a-hexahydroindolizine-7-yl)-1H-indole were obtained as an oil from 1.6 g (6.25 mmol) of 5-fluoro-3-(1,2,3,5,8,8a-hexahydro-indolizine-7-yl)-1H-indole and 3.42 g (18.76 mmol) of cyclohexanesulfonyl chloride by means of the process described in Example 1. Then 2 ml of a 6N ethanol/HCl solution were added to a solution of 1.95 g (4.85 mmol) of 1-cyclohexanesulfonyl-5-fluoro-3-(1,2,3,5,8,8a-hexahydro-indolizine-7-yl)-1H-indole in 20 ml of ethanol, precipitating a solid which was recrystallized from ethanol, obtaining 1.5 g (71%) of the mentioned compound as a white solid.

The yields are indicative and no added effort was made to improve them.
The melting point and spectroscopic data for identifying some of the compounds object of the present invention are indicated in the following table.

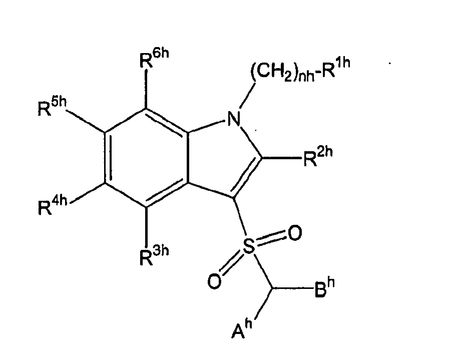

| Ex | R^{1h} | R² h | R³ h | R⁴ h | R^{5h} | R⁶ h | n h | 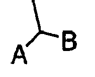 | Salt | m.p. °C | IR cm⁻¹ | ¹H-NMR (300MHz), δ (solvent) |
|---|---|---|---|---|---|---|---|---|---|---|---|---|
| 1h | 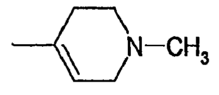 | H | H | H | NO₂ | H | 0 | 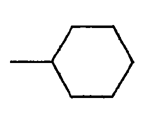 | - | 155-160 | 3433, 2938, 2859, 1522, 1371, 1340, 1158, 1126, 988,612. | 1.00-1.90 (m, 10H); 2.56 (s, 3H); 2.68 (m, 2H); 2.98 (m, 2H); 3.47 (m, 2H); 3.78 (m, 1H); 6.35 (s, 1 H); 7.87 (s, 1H); 8.08 (d, 1H, J=9.2 Hz); 8.26 (dd, 1H, J=9.2 Hz, J'=1.9 Hz); 8.69 (d, 1 H, J=1.8 Hz). (DMSO-d6) |
| 2h | 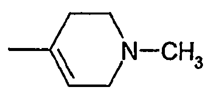 | H | H | H | Cl | H | 0 | 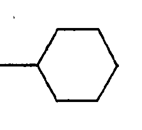 | - | 88-90 | 3433, 2941, 2858, 2787, 1447, 1364, 1158, 1128, 1116, 614, 557. | 1.00-1.90 (m, 10H); 2.41 (s, 3H); 2.55 (m, 2H); 2.67 (m, 2H); 3.15 (m, 3H); 6.18 (m, 1 H); 7.27 (dd, 1 H, J=8.9 Hz, J'=2.0 Hz); 7.32 (s, 1H); 7.79 (d, 1 H, J=2.0 Hz); 7.82 (d, 1 H, J=1.8 Hz). (CDCl₃) |
| 3h | 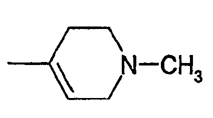 | H | H | H | NH₂ | H | 0 | 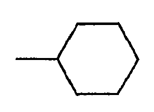 | - | 75 (dec) | 3376,2937, 2857,2784, 1455, 1363, 1342,1158, 1127,987, 617, 565. | 1.00-1.90 (m, 10H); 2.40 (s, 3H); 2.54 (m, 2H); 2.66 (m, 2H); 3.13 (m, 3H); 6.16 (m, 1H); 6.71 (dd, 1 H, J=8.8 Hz, J'=2.4 Hz); 7.09 (d, 1 H, J=2.2 Hz); 7.23 (s, 1 H); 7.67 (d, 1 H, J=8.8 Hz). (CDCl₃) |
| 4h | 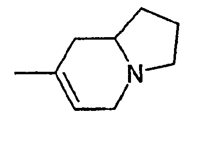 | H | H | H | F | H | 0 | 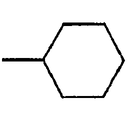 | HCl | 263 (dec) | 3424, 2941, 2499, 2451, 1466, 1445, 1371, 1348, 1188, 1157, 1127, 649, 619. | 1.18 (m, 3H); 1.38 (m, 2H); 1.54 (m, 1 H); 1.73 (m, 5H); 2.01 (m 2H); 2.31 (m, 1 H); 2.80 (m, 1 H); 3.09 (m, 2H); 3.44 (m,1H); 3.68 (m, 2H); 3.76 (m, 1H); 4.10 (m, 1 H); 6.39 (s, 1 H); 7.28 (m, 1 H); 7.78 (m, 2H); 7.90 (dd, 1 H, J=9.0 Hz, J'=4.6 Hz); (DMSO-d6) |

### Pharmacological Data:

### (Compounds of general formula Ia)

According to the methods given above Neuropeptide Y₅ and Y₂ Binding of the 1,4-disubstituted piperidine compounds of general formula (Ia) has been determined. Some of the Y₅ values are given in the following table 1 a.

**Table 1a:**

| **Compound according to Example** | **Neuropeptide Y₅ Binding** |
|---|---|
| 1a | 50 |
| 2a | 80,9 |
| 3a | 36,3 |
| 5a | 40,1 1 |

### (Compounds of general formula Ib)

The binding of the benzoxazinone derived sulphonamide compounds of general formula (Ib) was determined as described above.

The binding results of some these compounds are given in the following table 2b:

| **Table 2b:** | | |
|---|---|---|
| | | |
| | | |

| **Compound according to example:** | **% Inhibition 10-⁶ M** | **Kᵢ (nM)** |
|---|---|---|
| 1 b | 98.1 ± 4.0 | 51.7 |
| 3b | | 107.4 |
| 4b | | 246 |
| 5b | | 152 |
| 6b | | 165.9 |
| 7b | 88 | |
| 8b | 68 | |

### (Compounds of general formula Ic)

The binding of the inventively used sulphonamide derivatives of general formula (Ic) used inventively was determined as described above.

The binding results of some sulphonamide derivatives are given in the following table 1c:

**Table 1c:**

| **Compound according to example:** | **% Inhibition 10⁻⁶ M** | **Kᵢ (nM)** |
|---|---|---|
| 1c | 98.1 ± 4.0 | 0.28 |
| 3c | 96.6 ± 5.2 | 3.5 |
| 4c | 96.2 ± 0.6 | 9.3 |
| 5c | 101.2 ± 0.1 | 1.0 |
| 6c | 97.6 ± 1.8 | 8.7 |
| 7c | 103.0 ± 7.9 | 0.13 |
| 8c | 94.5 ± 7.0 | 0.76 |
| 9c | 96.8 ± 3.7 | 2.2 |
| 11c | 101.3 | 0.98 |
| 13c | 98.3 | 4.7 |
| 14c | 95.7 ± 3.4 | 24.3 |
| 15c | 97.4 ± 0.8 | 6.8 |
| 16c | 94.4 ± 8.6 | 21.2 |
| 17c | 102.0 | 5.3 |

### (Compounds of general formula Id)

Binding of the new compounds of general Formula (ld) to the 5-HT₆ receptor was determined as previously described.

The binding results for some of the compounds object of the present invention are indicated in the following table 1d:

**Table 1d**

| Example | % Inhibition |
|---|---|
| | 10⁻⁶ M |
| 1d | 83.9 |
| 2d | 104.3 |
| 3d | 94.8 |
| 4d | 46.6 |
| 5d | 98.1 |
| 6d | 55.8 |
| 7 | 72.3 |

### (Compounds of general formula Ie)

Binding of the new compounds of general formula (Ie) to the 5-HT₆ receptor was determined as previously described.

The binding results for some of the compounds object of the present invention are indicated in the following table 1e:

**Table 1e**

| Example | Kᵢ (nM) |
|---|---|
| 3e | 94,2 |
| 4e | 112,4 |
| 11e | 1,89 |
| 12e | 104,6 |
| 13e | 82,5 |
| 20e | 84,8 |

### (Compounds of general formula If)

### Pharmacological data:

Binding of the new compounds of general Formula (If) to the 5-HT₆ receptor was determined as previously described.
The binding results for some of the compounds object of the present invention are indicated in the following table 1f:

**Table 1f**

| Example | % Inhibition 10⁻⁶ M | Kᵢ (nM) |
|---|---|---|
| 1f | 98.6 | 90.2 |
| 2f | 97.7 | 41.2 |
| 3f | 95.3 | 19.8 |
| 4f | 90.8 | 55.2 |
| 5f | 93.4 | 129 4 |
| 6f | 94.5 | 74.5 |
| 7f | 95.1 | 118.6 |
| 8f | 86.9 | 159.1 |

### (Compounds of general formula Ih)

### Pharmacological data:

Binding of the new compounds of general Formula (lh) to the 5-HT₆ receptor was determined as previously described.

The binding results for some of the compounds object of the present invention are indicated in the following table 1h:

**Table 1h**

| Example | % Inhibition **10⁻⁶ M** | Kᵢ (nM) |
|---|---|---|
| 1 h | 59.8 ± 3.0 | |
| 2h | | 98.2 |
| 3h | | 55.1 |
| 4h | | 191 |

## Claims

1. An active substance combination, **characterized in that** it comprises:
(A) at least one compound with neuropeptide Y (NPY) -receptor affinity selected from the group consisting of the 1,4-disubstituted piperidine compounds of general formula (la) 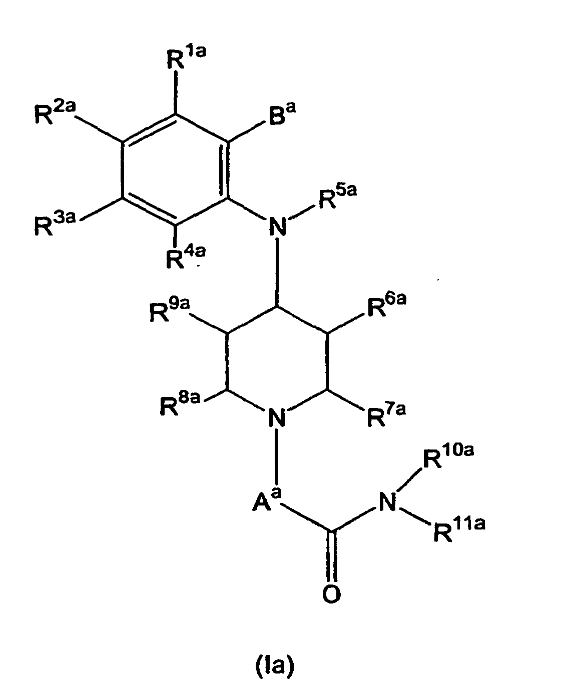 wherein
R^{1a}, R^{2a}, R^{3a}, R^{4a} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro, cyano, -OR^{12a}, -O-(C=O)R^{13a}, -(C=O)-OR^{13a}, -SR^{14a}, -SOR^{14a}, -SO₂R^{14a}, -NH-SO₂R^{14a}, -SO₂NH₂ and -NR^{15a}R^{16a} moiety,
R^{5a} represents hydrogen, an unbranched or branched, saturated or unsaturated, optionally at least mono-substituted aliphatic radical, or a saturated or unsaturated, optionally at least mono-substituted cycloaliphatic radical,
R^{6a}, R^{7a}, R^{8a}, R^{9a} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano and a COOR^{17a} moiety,
A^{a} represents a bridge member-CHR^{18a}- or -CHR^{18a}-CH₂-,
B^{a} represents an unbranched or branched, saturated or unsaturated, optionally at least mono-substituted aliphatic radical, a saturated or unsaturated, optionally at least mono-substituted cycloaliphatic radical, a COOR^{19a}-moiety, a -(C=O)R^{20a}-moiety, or a -CH₂OR^{23a}-moiety,
R^{10a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11a} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ringsystem, or an optionally at least mono substituted aryl- or heteroaryl radical, which may be bonded via an optionally at least mono-substituted alkylene group and/or may be condensed with an optionally at least mono-substituted mono- or polycyclic ringsystem, or
R^{10a} and R^{11a} together with the bridging nitrogen atom form an optionally at least mono-substituted, saturated, unsaturated or aromatic heterocyclic ring that may contain at least one further heteroatom as a ring member and/or may be condensed with an optionally at least mono-substituted mono- or polycyclic ringsystem,
R^{12a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{14a} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{15a} and R^{16a} each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{15a} and R^{16a} together with the bridging nitrogen atom form a saturated, unsaturated or aromatic heterocyclic ring, which may be at least mono-substituted and/or contain at least one further heteroatom as a ring member,
R^{17a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{18a} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{19a} represents hydrogen, an unbranched or branched, saturated or unsaturated, optionally at least mono-substituted aliphatic radical, a saturated or unsaturated, optionally at least mono-substituted cycloaliphatic radical, or an optionally at least mono-substituted aryl- or heteroaryl radical, which may be condensed with an optionally at least mono-substituted mono- or polycyclic ring-system,
R^{20a} represents hydrogen, an unbranched or branched, saturated or unsaturated, optionally at least mono-substituted aliphatic radical, a saturated or unsaturated, optionally at least mono-substituted cycloaliphatic radical, an optionally at least mono-substituted aryl- or heteroaryl radical, which may be condensed with an optionally at least mono-substituted mono- or polycyclic ring-system, or a NR^{21a}R^{22a}-moiety,
R^{21a} represents hydrogen, an unbranched or branched, saturated or unsaturated, optionally at least mono-substituted aliphatic radical, a saturated or unsaturated, optionally at least mono-substituted cycloaliphatic radical, or an optionally at least mono-substituted aryl- or heteroaryl radical, which may be condensed with an optionally at least mono-substituted mono- or polycyclic ring-system,
R^{22a} represents hydrogen, an unbranched or branched, saturated or unsaturated, optionally at least mono-substituted aliphatic radical, a saturated or unsaturated, optionally at least mono-substituted cycloaliphatic radical, or an optionally at least mono-substituted aryl- or heteroaryl radical, which may be condensed with an optionally at least mono-substituted mono- or polycyclic ring-system,
R^{23a} represents hydrogen, an unbranched or branched, saturated or unsaturated, optionally at least mono-substituted aliphatic radical, which may comprise at least one heteroatom as a chain member, or a -(C=O)R^{13a}-moiety,
optionally in form of one of its stereoisomers, preferably enantiomers or diastereomers, its racemate or in form of a mixture of at least two of its stereoisomers, preferably enantiomers or diastereomers, in any mixing ratio, or salts, preferably physiologically acceptable salts thereof, or corresponding solvates;
and
(B) at least one compound with 5-HT₆ receptor affinity selected from the group consisting of the benzoxazinone-derived sulfonamide compounds of general formula (lb) 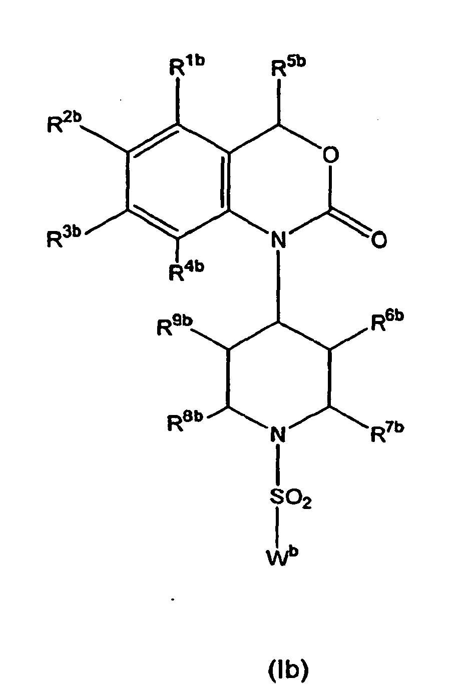
wherein
R^{1b}, R^{2b}, R^{3b}, R^{4b} are each independently selected from the group consisting of hydrogen, halogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, an optionally at least mono-substituted aryl- or heteroaryl radical, which may be bonded via an optionally at least mono-substituted alkylene group and/or may be condensed with an optionally at least mono-substituted mono- or polycyclic ringsystem, a nitro, cyano, -OR^{10b}, -O(C=O)R^{11b},-(C=O)OR^{11b}, -SR^{12b}, -SOR^{12b}, -SO₂R^{12b}, -NH-SO₂R^{12b}, -SO₂NH₂ and a-NR^{13b}R^{14b} moiety,
R^{5b} represents hydrogen, an unbranched or branched, saturated or unsaturated, optionally at least mono-substituted aliphatic radical or a saturated or unsaturated, optionally at least mono-substituted, optionally at least one heteroatom as ring member containing cycloaliphatic radical,
R^{6b}, R^{7b}, R^{8b}, R^{9b} are each independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, a cyano group and a COOR^{15b} moiety,
W^{b} represents an unbranched or branched, saturated or unsaturated, optionally at least mono-substituted aliphatic radical,
a saturated or unsaturated, optionally at least mono-substituted, optionally at least one heteroatom as ring member containing cycloaliphatic radical, which may be bonded via an optionally mono-substituted alkylene group and/or may be condensed with an optionally at least mono-substituted mono- or polycyclic ring-system,
an optionally at least mono-substituted aryl or heteroaryl radical, which may be bonded via an optionally at least mono-substituted alkylene or alkenylene group and/or may be condensed with an optionally at least mono-substituted mono- or polycyclic ring-system,
a NR^{16b}R^{17b}-moiety, or
a COR^{18b}-moiety,
R^{10b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{11b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{12b} represents an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{13b} and R^{14b} each are independently selected from the group consisting of hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical, which may be bonded via an optionally at least mono-substituted alkylene group and/or may be condensed with an optionally at least mono-substituted mono- or polycyclic ring-system, or an optionally at least mono-substituted aryl- or heteroaryl radical, which may be bonded via an optionally at least mono-substituted alkylene group and/or may be condensed with an optionally at least mono-substituted mono- or polycyclic ring-system,
or R^{13b} and R^{14b} together with the bridging nitrogen atom form a saturated, unsaturated or aromatic heterocyclic ring, which may be at least mono-substituted and/or contain at least one further heteroatom as a ring member,
R^{15b} represents hydrogen, an unbranched or branched, saturated or unsaturated, optionally at least mono-substituted aliphatic radical, a saturated or unsaturated, optionally at least mono-substituted, optionally at least one heteroatom as ring member containing cycloaliphatic radical or an optionally at least mono-substituted aryl- or heteroaryl radical, which may be bonded via an optionally at least mono-substituted alkylene group and/or may be condensed with an optionally at least mono-substituted mono- or polycyclic ring-system,
R^{16b} represents an unbranched or branched, saturated or unsaturated, optionally at least mono-substituted aliphatic radical,
R^{17b} represents an unbranched or branched, saturated or unsaturated, optionally at least mono-substituted aliphatic radical, and
R^{18b} represents an optionally at least mono-substituted aryl radical
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, and compounds derived from sulfonamide of general formula (Ic), 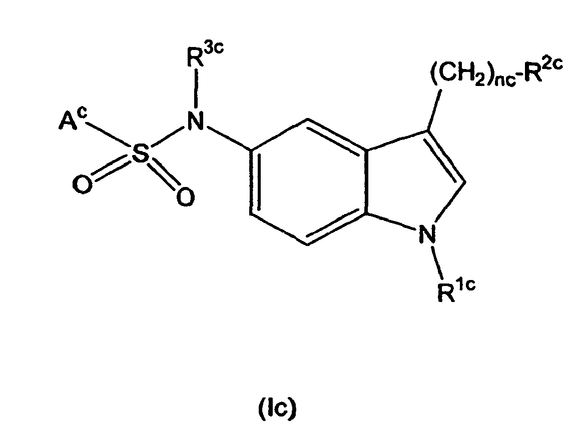 wherein
R^{1c} represents hydrogen, an optionally at least mono-substituted, linear or branched alkyl radical, an optionally at least mono-substituted phenyl radical or an optionally at least mono-substituted benzyl radical,
R^{2c} represents a -NR^{4c}R^{5c} moiety or a saturated or unsaturated, optionally at least mono-substituted, optionally at least one heteroatom as ring member containing cycloaliphatic radical, which may be condensed with a saturated or unsaturated, optionally at least mono-substituted, optionally at least one heteroatom as ring member containing mono- or bicyclic cycloaliphatic ringsystem,
R^{3c} represents hydrogen or an optionally at least mono- substituted, linear or branched alkyl radical,
R^{4c} and R^{5c}, identical or different, represent hydrogen or an optionally at least mono-substituted, linear or branched alkyl radical, or
R^{4c} and R^{5c} together with the bridging nitrogen atom form an optionally at least mono-substituted, saturated or unsaturated heterocyclic ring, which may contain at least one further heteroatom as a ring member and/or may be condensed with a saturated or unsaturated, optionally at least mono-substituted, optionally at least one heteroatom as ring member containing mono- or bicyclic cycloaliphatic ringsystem,
A^{c} represents an optionally at least mono-substituted mono- or polycyclic aromatic ringsystem, which may be bonded via an optionally at least mono-substituted alkylene-, an optionally at least mono-substituted alkenylene- or an optionally at least mono-substituted alkynylene group and/or may contain at least one heteroatom as a ring member in one or more of its rings,
nc represents 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a corresponding physiologically acceptable salt or a corresponding solvate,
and compounds of general formula (Id) 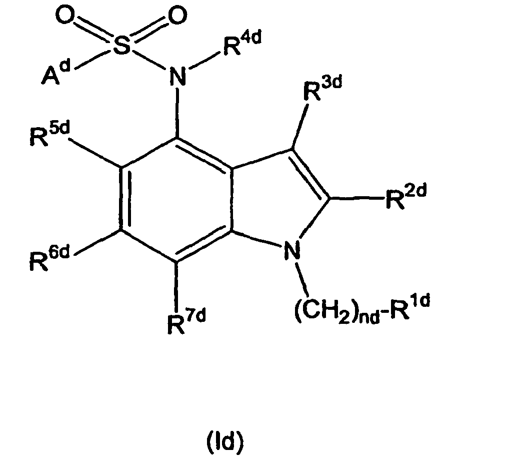 R^{1d} represents a -NR^{8d}R^{9d} radical or a saturated or unsaturated, optionally at least mono-substituted cycloaliphatic radical, which may contain at least one heteroatom as a ring member and/or which may be condensed with a saturated or unsaturated, optionally at least mono-substituted, optionally at least one heteroatom as a ring member containing mono- or bicyclic cycloaliphatic ring system,
R^{2d}, R^{3d}, R^{5d}, R^{6d} and R^{7d}, identical or different, each represent hydrogen, halogen, nitro, alkoxy, cyano, a saturated or unsaturated, linear or branched, optionally at least mono-substituted aliphatic radical, or an optionally at least mono-substituted phenyl or an optionally at least mono-substituted heteroaryl radical,
R^{4d} is hydrogen or a saturated or unsaturated, linear or branched, optionally at least mono-substituted aliphatic radical,
R^{8d} and R^{9d}, identical or different, each represent hydrogen or a saturated or unsaturated, linear or branched, optionally at least mono-substituted aliphatic radical,
or
R^{8d} and R^{9d} together with bridging nitrogen atom form a saturated or unsaturated, optionally at least mono-substituted heterocyclic ring, which may contain at least one additional heteroatom as a ring member and/or may be condensed with a saturated or unsaturated, optionally at least mono-substituted mono- or bicyclic cycloaliphatic ring system, which may optionally contain at least one heteroatom as a ring member,
A^{d} represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
and
nd is 0, 1, 2, 3 or 4;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.
and compounds derived from sulfonamide of general formula (le) 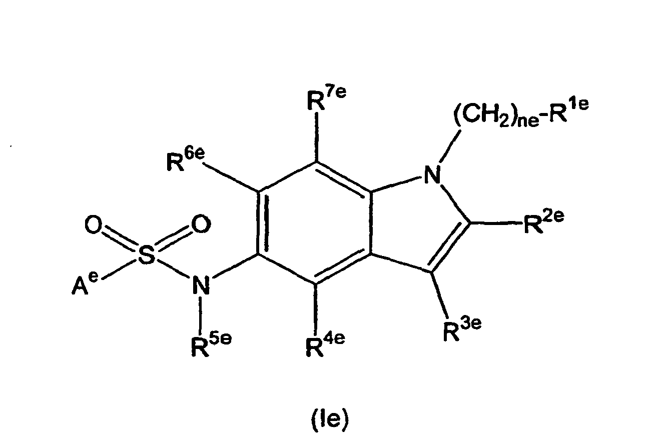 wherein
R^{1e} represents an -NR^{8e}R^{9e} radical or a saturated or unsaturated, optionally at least mono-substituted cycloaliphatic radical, which may optionally contain at least one heteroatom as a ring member and/or which may be condensed with a saturated or unsaturated, optionally at least mono-substituted mono- or bicyclic cycloaliphatic ring system, which may optionally contain at least one heteroatom as a ring member,
R^{2e}, R^{3e}, R^{4e}, R^{6e} and R^{7e}, identical or different, each represent hydrogen, halogen, nitro, alkoxy, cyano, a saturated or unsaturated, linear or branched, optionally at least mono-substituted aliphatic radical or an optionally at least mono-substituted phenyl radical or an optionally at least mono-substituted heteroaryl radical,
R^{5e} represents hydrogen or a saturated or unsaturated, linear or branched, optionally at least mono-substituted aliphatic radical,
R^{8e} and R^{9e}, identical or different, each represent hydrogen or a saturated or unsaturated, linear or branched, optionally at least mono-substituted aliphatic
radical,
or
R^{8e} and R^{9e} together with the bridging nitrogen atom form a saturated or unsaturated, optionally at least mono-substituted heterocyclic ring, which may contain at least one additional heteroatom as a ring member and/or which may be condensed with a saturated or unsaturated, optionally at least mono-substituted, mono- or bicyclic cycloaliphatic ring system which may optionally contain at least one heteroatom as a ring member,
A^{e} represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
and
ne is 0, 1, 2, 3 or 4;
optionally in the form of one of its stereoisomers, preferably enantiomers or diastereomers, its racemate or in the form of a mixture of at least two of its stereoisomers, preferably enantiomers or diastereomers, at any mixture ratio, or a corresponding, physiologically acceptable salt, or a corresponding solvate, and compounds derived from sulfonamide of general formula (If) 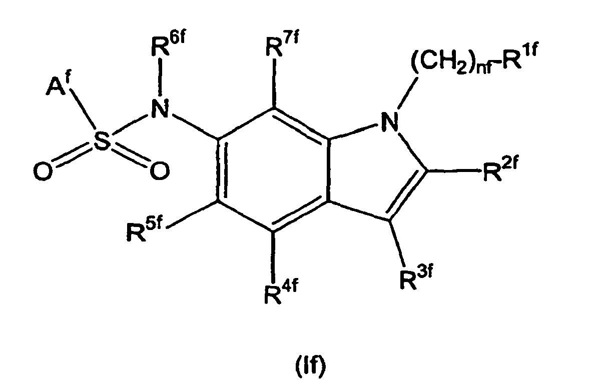 wherein
R^{1f} represents a -NR^{8f}R^{9f} radical or a saturated or unsaturated, optionally at least mono-substituted, optionally at least one heteroatom as a ring member containing cycloaliphatic radical, which may be condensed with a saturated or unsaturated, optionally at least mono-substituted, optionally at least one heteroatom as a ring member containing mono- or bicyclic cycloaliphatic ring system,
R^{2f}, R^{3f}, R^{4f}, R^{5f} and R^{7f}, identical or different, each represent hydrogen, halogen, nitro, alkoxy, cyano, a saturated or unsaturated, linear or branched, optionally at least mono-substituted aliphatic radical, or an optionally at least mono-substituted phenyl radical or an optionally at least mono-substituted heteroaryl radical,
R^{6f} represents hydrogen or a saturated or unsaturated, linear or branched, optionally at least mono-substituted aliphatic radical,
R^{8f} and R^{9f}, identical or different, each represent hydrogen or a saturated or unsaturated, linear or branched, optionally at least mono-substituted aliphatic radical,
or
R^{8f} and R^{9f}, together with the bridging nitrogen atom, form a saturated or unsaturated, optionally at least mono-substituted heterocyclic ring, which may contain at least one further heteroatom as a ring member and/or which may be condensed with a saturated or unsaturated, optionally at least mono-substituted, optionally at least one heteroatom as a ring member containing mono- or bicyclic cycloaliphatic ring system,
A^{f} represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings
and
nf is 0, 1, 2, 3 or 4;
optionally in the form of one of its stereoisomers, preferably enantiomers or diastereomers, its racemate or in the form of a mixture of at least two of its stereoisomers, preferably enantiomers or diastereomers, at any mixture ratio, or a corresponding, physiologically acceptable salt, or a corresponding solvate, and compounds derived from sulfonamide of general formula (Ig) 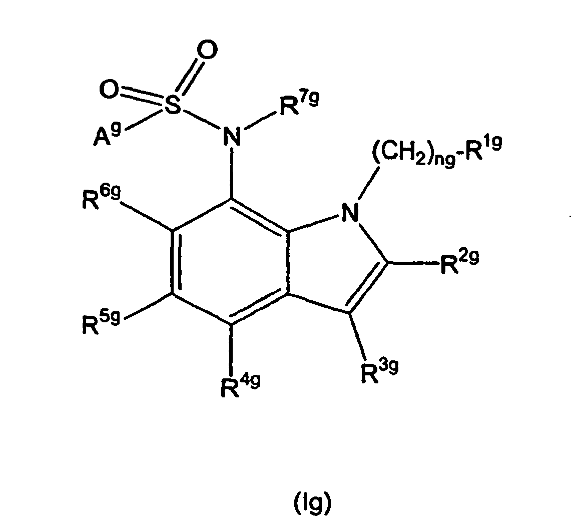 wherein
R^{1g} is a -NR^{8g}R^{9g} radical or a saturated or unsaturated, optionally at least mono-substituted cycloaliphatic radical, which may optionally contain at least one heteroatom as a ring member and which may be condensed with a saturated or unsaturated, optionally at least mono-substituted mono- or bicyclic cycloaliphatic ring systemwhich may optionally contain at least one heteroatom as a ring member,
R^{2g}, R^{3g}, R^{4g}, R^{5g} and R^{6g}, identical or different, each represent hydrogen, halogen, nitro, alkoxy, cyano, a saturated or unsaturated, linear or branched, optionally at least mono-substituted aliphatic radical, or an optionally at least mono-substituted phenyl radical or an optionally at least mono-substituted heteroaryl radical,
R^{7g} represents hydrogen or a saturated or unsaturated, linear or branched, optionally at least mono-substituted aliphatic radical,
R^{8g} and R^{9g}, identical or different, represent hydrogen or a saturated or unsaturated, linear or branched, optionally at least mono-substituted aliphatic radical,
or
R^{8g} and R^{9g} together with the bridging nitrogen atom form a saturated or unsaturated, optionally at least mono-substituted heterocyclic ring, which may contain at least one additional heteroatom as a ring member and/or which may be condensed with a saturated or unsaturated, optionally at least mono-substituted mono- or bicyclic cycloaliphatic ring system, which may optionally contain at least one heteroatom as a ring member,
A⁹ represents an optionally at least mono-substituted mono- or polycyclic aromatic ring system, which may be bonded via an optionally at least mono-substituted alkylene, alkenylene or alkynylene group and/or which may contain at least one heteroatom as a ring member in one or more of its rings,
ngis0, 1,2,3or4;
optionally in the form of one of its stereoisomers, preferably enantiomers or diastereomers, its racemate or in the form of a mixture of at least two of its stereoisomers, preferably enantiomers or diastereomers, at any mixture ratio, or a corresponding, physiologically acceptable salt, or a corresponding solvate and compounds derived from sulfonamide of general formula (Ih) 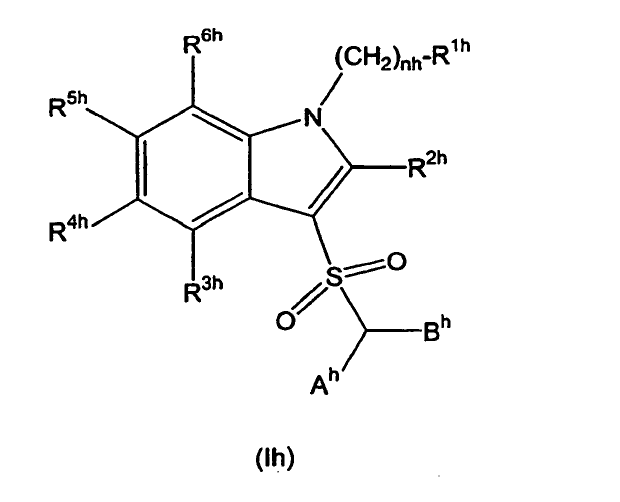 wherein
R^{1h} represents a -NR^{7h}R^{8h} radical or a saturated or unsaturated, optionally at least mono-substituted, optionally at least one heteroatom as a ring member containing cycloaliphatic radical, which may be condensed with a saturated or unsaturated, optionally at least mono-substituted, optionally at least one heteroatom as a ring member containing mono- or bicyclic cycloaliphatic ring system,
R^{2h}, R^{3h}, R^{4h}, R^{5h} and R^{6h}, identical or different, each represent hydrogen, halogen, cyano, nitro, a linear or branched alkyl radical, a linear or branched alkenyl radical, a linear or branched alkinyl radical, a linear or branched alkoxy radical, a linear or branched alkylthio radical, hydroxy, trifluoromethyl, a cycloalkyl radical, a cycloalkenyl radical, an alkylcarbonyl radical, a phenylcarbonyl or a -NR^{9h}R^{10h} group,
R^{7h} and R^{8h}, identical or different, each represent hydrogen or a saturated or unsaturated, optionally at least mono-substituted linear or branched aliphatic radical,
or
R^{7h} and R^{8h}, together with the bridging nitrogen atom form a saturated or unsaturated, optionally at least mono-substituted, optionally at least one further heteroatom as a ring member containing heterocyclic ring which may be condensed with a saturated or unsaturated, optionally at least mono-substituted, optionally at least one heteroatom as a ring member containing mono- or bicyclic cycloaliphatic ring system,
R^{9h} and R^{10h}, identical or different, each represent hydrogen or a saturated or unsaturated, linear or branched, optionally at least mono-substituted aliphatic radical,
or
R^{9h} and R^{10h}, together with the bridging nitrogen atom form a saturated or unsaturated, optionally at least mono-substituted, optionally at least one further heteroatom as a ring member containing heterocyclic ring which may be condensed with a saturated or unsaturated, optionally at least mono-substituted, optionally at least one heteroatom as a ring member containing mono- or bicyclic cycloaliphatic ring system,
A^{h} and B^{h}, identical or different, each represent a saturated or unsaturated, linear or branched, optionally at least mono-substituted aliphatic radical
or
A^{h} and B^{h}, together with the carbon atom to which they are bonded, form a saturated or unsaturated, but not aromatic, optionally at least mono-substituted cycloalkyl ring,
and
nh is 0, 1, 2, 3 or 4
optionally in the form of one of their stereoisomers, preferably enantiomers or diastereomers, their racemate or in the form of a mixture of at least two of their stereoisomers, preferably enantiomers or diastereomers, at any mixture ratio, or a corresponding physiologically acceptable salt or a corresponding solvate.

2. The combination according to claim 1, **characterized in that** it comprises 1-99% by weight of component (A) and 99-1 % by weight of component (B), more preferably 10-80% by weight of component (A) and 90-20% by weight of component (B), in each case referring to the total weight of both components (A) and (B).

3. A medicament comprising an active substance combination acording to claim 1 or 2 and optionally one or more pharmacologically acceptable adjuvants.

4. A medicament according to claim 3 for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatoric diseases, immunologic diseases or for improvement of cognition.

5. Use of the combination according to claim 1 or 2 for the manufacture of a medicament for regulation of appetite.

6. Use of the combination according to claim 1 or 2 for the manufacture of a medicament for maintenance, increase or reduction of body weight.

7. Use of the combination according to claim 1 or 2 for the manufacture of a medicament for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus).

8. Use of the combination according to claim 1 or 2 for the manufacture of a medicament for prophylaxis and/or treatment of gastrointestinal tract disorders, preferably of the irritable bowel syndrome.

9. Use of the combination according to claim 1 or 2 for the manufacture of a medicament for prophylaxis and/or treatment of Peripheral Nervous System Disorders.

10. Use of the combination according to claim 1 or 2, for the manufacture of a medicament for prophylaxis and/or treatment of Central Nervous System Disorders.

11. Use of the combination according to claim 1 or 2 for the manufacture of a medicament for prophylaxis and/or treatment arthritis.

12. Use of the combination according to claim 1 or 2 for the manufacture of a medicament for prophylaxis and/or treatment of epilepsy.

13. Use of the combination according to claim 1 or 2 for the manufacture of a medicament for prophylaxis and/or treatment of anxiety.

14. Use of the combination according to claim 1 or 2 for the manufacture of a medicament for prophylaxis and/or treatment of panic.

15. Use of the combination according to claim 1 or 2 for the manufacture of a medicament for prophylaxis and/or treatment of depression.

16. Use of the combination according to claim 1 or 2 for the manufacture of a medicament for prophylaxis and/or treatment of bipolar disorders.

17. Use of the combination according to claim 1 or 2 for the manufacture of a medicament for prophylaxis and/or treatment of cognitive disorders.

18. Use of the combination according to claim 1 or 2 for the manufacture of a medicament for prophylaxis and/or treatment of memory disorders.

19. Use of the combination according to claim 1 or 2 for the manufacture of a medicament for prophylaxis and/or treatment of cardiovascular diseases.

20. Use of the combination according to claim 1 or 2 for the manufacture of a medicament for prophylaxis and/or treatment of senile dementia processes.

21. Use of the combination according to claim 1 or 2 for the manufacture of a medicament for prophylaxis and/or treatment of neurodegenerative disorders, preferably Alzheimer's diseasse, Parkinson's disease, Huntington's disease and/or multiple sclerosis.

22. Use of the combination according to claim 1 or 2 for the manufacture of a medicament for prophylaxis and/or treatment of schizophrenia.

23. The use of the combination according to claim 1 or 2 for the manufacture of a medicament for prophylaxis and/or treatment of psychosis.

24. Use of the combination according to claim 1 or 2 for the manufacture of a medicament for prophylaxis and/or treatment of infantile hyperkinesia (ADHD, attention deficit / hyperactivity disorder).

25. The use of the combination according to claim 1 or 2 for the manufacture of a medicament for prophylaxis and/or treatment of pain.

26. Use of the combination according to claim 1 or 2 for the manufacture of a medicament for prophylaxis and/or treatment of hypertensive syndrome.

27. Use of the combination according to claim 1 or 2 for the manufacture of a medicament for prophylaxis and/or treatment of inflammatoric diseases.

28. Use of the combination according to claim 1 or 2 for the manufacture of a medicament for prophylaxis and/or treatment of immunologic diseases.

29. The use of the combination according to claim 1 or 2 for the manufacture of a medicament for improvement of cognition.

30. A pharmaceutical formulation, **characterized in that** it comprises an active substance combination according to claim 1 or 2 and optionally one or more pharmacologically acceptable adjuvants.

31. The pharmaceutical formulation according to claim 30, **characterized in that** it is present in solid pharmaceutical forms such as tablets, tablets, chewing tablets, chewing gums, dragées, capsules, suppositories, powder preparations, transdermal therapeutic systems, transmucosal therapeutic systems, or in liquid and semi-liquid pharmaceutical forms such as drops or such as juice, sirup, solution, emulsion, suspension, preferably in form of tablets, capsules, drops or solution.

32. The pharmaceutical formulation according to claim 30, **characterized in that** it is present in form of of multiple particles, preferably microtablets, microcapsules, microspheroids, granules, crystals or pellets, optionally compacted in a tablet, filled in a capsule or suspended in a suitable liquid.

33. The pharmaceutical formulation according to one or more of claims 30-32, **characterized in that** it is for oral, intravenous, intramuscular, subcutaneous, intrathecal, epidural, buccal, sublingual, pulmonal, rectal, transdermal, nasal or intracerebroventricular application, preferably oral or intravenous.

34. The pharmaceutical formulation according to one or more of claims 30-32, **characterized in that** at least one of the components of the active substance combination (A) or (B) is present at least partially in sustained-release form.

35. The pharmaceutical formulation according to claim 34, **characterized in that** the medicament has at least one coating or at least one matrix comprising at least one material, which sustains active substance release.

36. The pharmaceutical formulation according to claim 35, **characterized in that** the sustained-release material is based on optionally modified, water-insoluble, natural, semisynthetic or synthetic polymer, or a natural wax or fat or fatty alcohol or semisynthetic or synthetic fatty acid, or on a mixture of at least two of these afore mentioned components.

37. The pharmaceutical formulation according to claim 36, **characterized in that** the water-insoluble polymer is based on an acrylic resin, which is preferably selected from the group of poly(meth)acrylates, poly(C₁₋₄)dialkylamino(C₁₋₄)alkyl (meth)acrylates and/or copolymers thereof or a mixture of at least two of the afore-mentioned polymers.

38. The pharmaceutical formulation according to claim 36, **characterized in that** the water-insoluble polymers are cellulose derivatives, preferably alkyl cellulose and even more preferably ethyl cellulose, or cellulose esters.

39. The pharmaceutical formulation according to claim 36, **characterized in that** the wax is carnauba wax, beeswax, glycerol monostearate, glycerol monobehenate, glycerol ditripalmitostearate, microcrystalline wax or a mixture of at least two of these components.

40. The pharmaceutical formulation according to one or more of claims 36 to 39, **characterized in that** polymers have been used in combination with one or more plasticizers.

41. The pharmaceutical formulation according to one or more of claims 34 to 40, **characterized in that** besides the sustained-release form, at least one of the active substance components (A) or (B) is present in a non-sustained-release form.

## Patentansprüche

1. Wirkstoffkombination, **dadurch gekennzeichnet, dass** sie Folgendes umfasst:
(A) zumindest eine Verbindung mit Neuropeptid Y (NPY) -Rezeptoraffinität, ausgewählt aus der Gruppe, die aus 1,4-disubstituierten Piperidin-Verbindungen der allgemeinen Formel (Ia) besteht 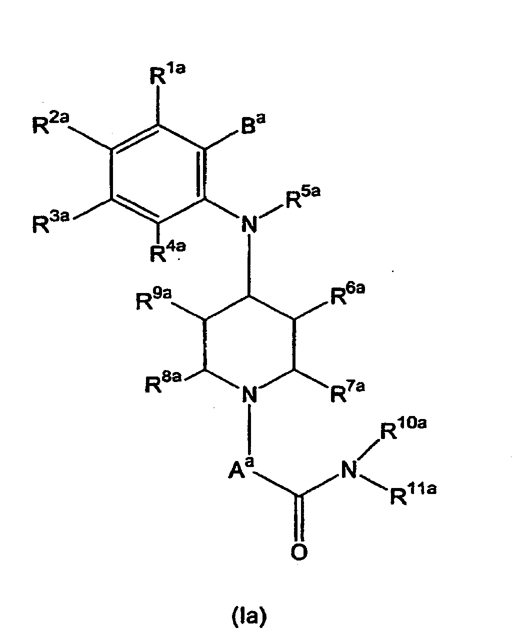 wobei R^{1a}, R^{2a}, R^{3a}, R^{4a} jeweils unabhängig aus der Gruppe ausgewählt sind, die aus Wasserstoff, Halogen, einem unverzweigten oder verzweigten, gesättigten oder ungesättigten, wahlweise zumindest mono-substituierten aliphatischen Radikal, einem gesättigten oder ungesättigten, wahlweise zumindest mono-substituierten, wahlweise zumindest ein Heteroatom als Ringelement enthaltenden cycloaliphatischen Radikal, das über eine wahlweise, zumindest monosubstituierte Alkylen-Gruppe gebunden sein kann und/oder mit einem wahlweise zumindest mono-substituierten mono- oder polycyclischen Ringsystem kondensiert ist, einem wahlweise zumindest mono-substituierten Aryl- oder Heteroarylradikal, das über eine wahlweise zumindest mono-substituierte Alkylen-Gruppe gebunden sein kann und/oder mit einem zumindest wahlweise mono-substituierten mono- oder polycyclischen Ringsystem kondensiert sein kann, einer Nitro, Cyano, -OR^{12a}, -O-(C=O)-OR^{13a}, -(C=O)-OR^{13a}, -SR^{14a}, -SOR^{14a}, -SO₂R^{14a}, -NH-SO₂R^{14a}, -SO₂NH₂ and -NR^{15a}R^{16a} -Komponente, besteht,
R^{5a} Wasserstoff, ein unverzweigtes oder verzweigtes, gesättigtes oder ungesättigtes, wahlweise zumindest mono-substituiertes aliphatisches Radikal oder ein gesättigtes oder ungesättigtes, wahlweise zumindest mono-substituiertes cycloaliphatisches Radikal repräsentiert,
R^{6a}, R^{7a}, R^{8a}, R^{9a} jeweils unabhängig aus der Gruppe ausgewählt sind, die aus Wasserstoff, einem unverzweigten oder verzweigten, gesättigten oder ungesättigten, wahlweise zumindest mono-substituierten aliphatischen Radikal, einem gesättigten oder ungesättigten, wahlweise zumindest mono-substituierten, wahlweise zumindest ein Heteroatom als Ringelement enthaltenden cycloaliphatischen Radikal, einer Cyano- und einer COOR^{17a}-Komponente, besteht,
A^{a} ein Brückenelement -CHR^{18a}- or -CHR^{18a}-CH₂- repräsentiert,
B^{a} ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes cycloaliphatisches Radikal, eine COOR^{19a}-Komponente, eine (C=O)R^{20a} -Komponente, or eine -CH₂OR^{23a}-Komponente repräsentiert,
R^{10a} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal oder ein wahlweise zumindest mono-substituiertes Aryl- oder Heteroarylradikal, das über eine wahlweise zumindest mono-substituierte Alkylen-Gruppe gebunden sein kann und/oder mit einem wahlweise zumindest mono-substituierten mono- oder polycyclischen Ringsystem kondensiert ist, repräsentiert,
R^{11a} ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal, das über eine wahlweise zumindest mono-substituierte Alkylen-Gruppe gebunden sein kann und/oder mit einer wahlweise zumindest mono-substituierten mono- oder polycyclischen Ringsystem kondensiert sein kann, oder ein wahlweise zumindest mono-substituiertes Aryl- oder Heteroarylradikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, repräsentiert, oder
R^{10a} and R^{11a} zusammen mit dem Brückenstickstoffatom einen wahlweise zumindest mono-substituierten, gesättigten, ungesättigten oder aromatischen heterocyclischen Ring bilden, der zumindest ein weiteres Heteroatom als Ringelement enthält und/oder mit einem wahlweise zumindest mono-substituierten mono- oder polycyclischen Ringsystem kondensiert sein kann,
R^{12a} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, oder ein wahlweise zumindest mono-substituiertes Aryl- oder Heteroarylradikal sein kann, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann,
R^{13a} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement als Ringelement enthaltendes cycloaliphatisches Radikal, das über eine wahlweise zumindest mono-substituierte Alkylen-Gruppe gebunden sein kann und/oder mit einem wahlweise mono-substituierten Mono- oder polycyclischen Ringsystem kondensiert sein kann, oder ein wahlweise zumindest mono-substituiertes Aryl- oder Heteroarylradikal, das über eine wahlweise zumindest mono-substituierte Alkylen-Gruppe gebunden sein kann und/oder mit einem wahlweise zumindest mono-substituierten oder polycyclischen Ringsystem kondensiert sein kann, repräsentiert,
R^{14a} ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal, das über eine wahlweise zumindest mono-substituierte Alkylen-Gruppe gebunden sein kann und/oder mit einem wahlweise zumindest mono-substituierten mono- oder polycyclischen Ringssystem kondensiert sein kann, oder ein wahlweise zumindest mono-substituiertes Aryl- oder Heteroarylradikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, repräsentiert,
R^{15a} and R^{16a} jeweils unabhängig aus der Gruppe ausgewählt sind, die aus Wasserstoff, einem unverzweigten oder verzweigten, gesättigten oder ungesättigten, wahlweise zumindest mono-substituierten aliphatischen Radikal, einem gesättigten oder ungesättigten, wahlweise zumindest mono-substituierten, wahlweise zumindest ein Heteroatom als Ringelement enthaltenden cycloaliphatischen Radikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, oder einem wahlweise zumindest mono-substituierten Aryl- oder Heteroarylradikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, ausgewählt ist,
oder R^{15a} and R^{16a} zusammen mit dem Brückenstickstoffatom einen gesättigten, ungesättigten oder aromatischen heterocyclischen Ring bilden, der zumindest mono-substituiert sein kann und/oder zumindest ein weiteres Heteroatom als Ringelement enthalten kann,
R^{17a} Wasserstoff, ein verzweigtes oder unverzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal oder ein wahlweise zumindest mono-substituiertes Aryl- oder Heteroarylradikal, das über eine wahlweise zumindest mono-substituierte Alkylen-Gruppe gebunden ist und/oder mit einem wahlweise zumindest mono-substituierten mono- oder polycyclischen Ringsystem kondensiert sein kann, repräsentiert,
R^{18a} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal oder ein wahlweise zumindest mono-substituiertes Aryl- oder Heteroarylradikal, das über eine wahlweise zumindest mono-substituierte Alkylen-Gruppe gebunden ist und/oder mit einem wahlweise zumindest mono-substituierten mono- oder polycyclischen Ringsystem kondensiert sein kann, repräsentiert,
R^{19a} Wasserstoff, ein unverzeigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes cycloaliphatisches Radikal, oder ein wahlweise zumindest mono-substituiertes Aryl- oder Heteroarylradikal, das mit einem wahlweise zumindest mono-substituierten mono- oder polycyclischen Ringsystem kondensiert sein kann, repräsentiert.
R^{20a} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes cycloaliphatisches Radikal, ein wahlweise zumindest mono-substituiertes Aryl- oder Heteroarylradikal, das mit einem wahlweise zumindest mono-substituierten mono- oder polycyclischen Ringsystem kondensiert sein kann, oder eine NR^{21a}R^{22a}-Komponente repräsentiert,
R^{21a} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes cycloaliphatisches Radikal oder ein wahlweise zumindest mono-substituiertes Aryl- oder Heteroarylradikal, das mit einem wahlweise zumindest mono-substituierten mono- oder polycyclischen Ringsystem kondensiert sein kann, repräsentiert.
R^{22a} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes cycloaliphatisches Radikal oder ein wahlweise zumindest mono-substituiertes Aryl- oder Heteroarylradikal, das mit einem wahlweise zumindest mono-substituierten mono- oder polycyclischen Ringsystem kondensiert sein kann, repräsentiert.
R^{23a} Wasserstoff, ein unverzweigtes oder verzweigtes, gesättigtes oder ungesättigtes, wahlweise zumindest mono-substituiertes aliphatisches Radikal, das zumindest ein Heteroatom als Kettenelement umfasst, oder eine -(C=O) R^{13a}-Komponente repräsentiert,
wahlweise in Form eines seiner Stereoisomere, vorzugsweise von Enantiomeren oder Diastereomeren, seines Racemats oder in Form eines Gemisches von zumindest zwei seiner Stereoisomere, vorzugsweise von Enantiomeren oder Diastereomeren, in jedem Mischungsverhältnis oder Salze, vorzugsweise physiologisch verträgliche Salze hiervon, oder entsprechende Solvate; und
(B) zumindest eine Verbindung mit einer 5-HT₆-Rezeptoraffinität, ausgewählt aus der Gruppe, die aus den Benzoxazinon-abgeleiteten Sulfonamid-Verbindungen der allgemeinen Formel (Ib) 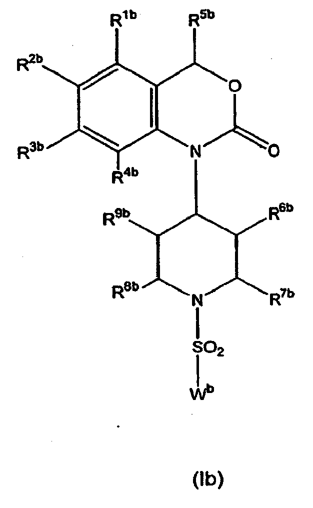
wobei
R^{1b}, R^{2b}, R^{3b}, R^{4b}, jeweils unabhängig aus der Gruppe ausgewählt sind, die aus Wasserstoff, Halogen, einem unverzweigten oder verzweigten, gesättigten oder ungesättigten, wahlweise zumindest mono-substituierten aliphatischen Radikal, einem gesättigten oder ungesättigten, wahlweise zumindest mono-substituierten, wahlweise zumindest ein Heteroatom als Ringelement enthaltenden cycloaliphatischen Radikal, das über eine wahlweise zumindest mono-substituierte Alkylengruppe gebunden sein kann und/oder mit einem wahlweise zumindest mono-substituierten mono- oder polycyclischen Ringsystem kondensiert sein kann, einem wahlweise zumindest mono-substituierten Aryl- oder Heteroarylradikal, das über eine wahlweise zumindest mono-substituierte Alkylengruppe gebunden sein kann und/oder mit einem wahlweise zumindest mono-substituierten mono- oder polycyclischen Ringssystem kondensiert sein kann, einer Nitro- Cyano, -OR^{10b}, -O(C=O)R^{11b},-(C=O)OR^{11b}, -SR^{12b}, -SOR^{12b}, -SO₂R^{12b}, -NH-SO₂R^{12b}, -SO₂NH₂ and a -NR^{13b}R^{14b}-Komponente, besteht,
R^{5b} Wasserstoff, ein unverzweigtes oder verzweigtes, gesättigtes oder ungesättigtes, wahlweise zumindest mono-substituiertes aliphatisches Radikal, oder ein gesättigtes oder ungesättigtes, wahlweise zumindest mono-substituiertes, wahlweise zumindest ein Heteroatom als Ringelement enthaltendes cycloaliphatisches Radikal repräsentiert,
R^{6b}, R^{7b}, R^{8b}, R^{9b} jeweils unabhängig aus der Gruppe ausgewählt sind, die aus Wasserstoff, einem unverzweigten oder verzweigten, gesättigten oder ungesättigten, wahlweise zumindest mono-substituierten aliphatischen Radikal, einem gesättigten oder ungesättigten, wahlweise zumindest mono-substituierten, wahlweise zumindest ein Heteroatom als Ringelement enthaltenden cycloaliphatischen Radikal, einer Cyano-Gruppe und einer COOR^{15b}-Komponente, besteht,
W^{b} ein unverzweigtes oder verzweigtes, gesättigtes oder ungesättigtes, wahlweise zumindest mono-substituiertes aliphatisches Radikal,
ein gesättigtes oder ungesättigtes, wahlweise zumindest mono-substituiertes, wahlweise zumindest ein Heteroatom als Ringelement enthaltendes cycloaliphatisches Radikal, das über eine wahlweise mono-substituierte Alkylen-Gruppe gebunden sein kann und/oder mit einem wahlweise zumindest mono-substituierten mono- oder polycyclischen Ringsystem kondensiert sein kann,
ein wahlweise zumindest mono-substituiertes Aryl- oder Heteroarylradikal, das über eine wahlweise zumindest mono-substituierte Alkylen- oder Alkenylen-Gruppe gebunden sein kann und/oder mit einem wahlweise zumindest mono-substituierten mono- oder polycyclischen Ringsystem kondensiert sein kann,
eine NR^{16b}R^{17b-}-Komponente oder
eine COR^{18b}-Komponente, repräsentiert,
R^{10b} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal, das über eine wahlweise zumindest mono-substituierte Alkylengruppe gebunden sein kann und/oder mit einem wahlweise zumindest mono-substituierten mono- oder polycyclischen Ringsystem kondensiert sein kann, oder ein wahlweise zumindest mono-substituiertes Aryl- oder Heteroarylradikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, repräsentiert,
R^{11b} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal, das über eine wahlweise zumindest mono-substituierte Alkylengruppe gebunden sein kann und/oder mit einem wahlweise zumindest mono-substituierten mono- oder polycyclischen Ringsystem kondensiert sein kann, oder ein wahlweise zumindest mono-substituiertes Aryl- oder Heteroarylradikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, repräsentiert,
R^{12b} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal, das über eine wahlweise zumindest mono-substituierte Alkylengruppe gebunden sein kann und/oder mit einem wahlweise zumindest mono-substituierten mono- oder polycyclischen Ringsystem kondensiert sein kann, oder ein wahlweise zumindest mono-substituiertes Aryl- und Heteroarylradikal, das über eine wahlweise zumindest mono-substituierte Alkylen-Gruppe gebunden ist und/oder mit zumindest mono-substituierten mono- oder polycyclischen Ringsystem kondensiert sein kann, repräsentiert,
R^{13b} and R^{14b} jeweils unabhängig aus der Gruppe ausgewählt sind, die aus Wasserstoff, einem unverzweigten oder verzweigten, gesättigten oder ungesättigten, wahlweise zumindest mono-substituierten aliphatischen Radikal, einem gesättigten oder ungesättigten, wahlweise zumindest mono-substituierten, wahlweise zumindest ein Heteroatom als Ringelement enthaltenden cycloaliphatischen Radikal, das über eine wahlweise zumindest mono-substituierte Alkylengruppe gebunden sein kann und/oder mit einem wahlweise zumindest mono-substituierten mono- oder polycyclischen Ringsystem kondensiert sein kann, oder einem wahlweise zumindest mono-substituiertenAryl- oder Heteroarylradikal, das über eine wahlweise zumindest mono-substituierte Alkylen-Gruppe gebunden sein kann und/oder mit einem wahlweise zumindest mono-substituierten mono- oder polycyclischen Ringsystem kondensiert sein kann, besteht,
oder R^{13b} und R^{14b} zusammen mit dem Brückenstickstoffatom einen gesättigten, ungesättigten oder aromatischen heterocyclischen Ring bilden, der zumindest mono-substituiert sein kann oder und/oder zumindest ein weiteres Heteroatom als Ringelement enthält,
R^{15b} Wasserstoff, ein unverzweigtes oder verzweigtes, gesättigtes oder ungesättigtes, wahlweise zumindest mono-substituiertes aliphatisches Radikal, ein gesättigtes oder ungesättigtes, wahlweise zumindest mono-substituiertes, wahlweise zumindest ein Heteroatom als Ringelement enthaltendes cycloaliphatisches Radikal, oder wahlweise zumindest mono-substituiertes Aryl- oder Heteroarylradikal, das über wahlweise zumindest mono-substituierte Alkylen-Gruppe gebunden sein kann und/oder mit einem wahlweise zumindest mono-substituierten mono- oder polycyclischen Ringsystem gebunden sein kann, repräsentiert,
R^{16b} ein unverzweigtes oder verzweigtes, gesättigtes oder ungesättigtes, wahlweise zumindest mono-substituiertes aliphatisches Radikal repräsentiert,
R^{17b} ein unverzweigtes oder verzweigtes, gesättigtes oder ungesättigtes, wahlweise zumindest mono-substituiertes aliphatisches Radikal repräsentiert, und
R^{18b} ein wahlweise zumindest mono-substituiertes Arylradikal repräsentiert,
wahlweise in Form eines seiner Stereoisomere, vorzugsweise Enantiomere oder Diastereomere, seines Racemats oder in Form eines Gemisches von zumindest zweien seiner Stereoisomere, vorzugsweise von Enantiomeren oder Diastereomeren, in jedem Mischungsverhältnis, oder ein physiologisch akzeptables Salz hiervon, oder ein Solvat; und
Verbindungen, abgeleitet von einem Sulfonamid der allgemeinen Formel (Ic) 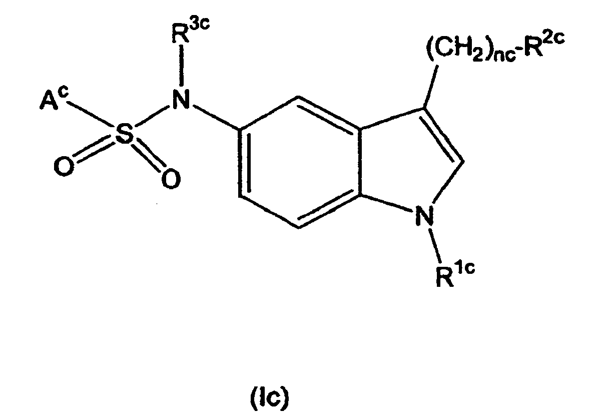 wobei
R^{1c} Wasserstoff, ein wahlweise zumindest mono-substituiertes, lineares oder verzweigtkettiges Alkylradikal, ein wahlweise zumindest mono-substituiertes Phenylradikal oder ein wahlweise zumindest mono-substituiertes Benzylradikal repräsentiert,
R^{2c} eine -NR^{4c}R^{5c}-Komponente oder ein gesättigtes oder ungesättigtes, wahlweise zumindest mono-substituiertes, wahlweise zumindest ein Heteroatom als Ringelement enthaltendes cycloaliphatisches Radikal, das mit einem gesättigten oder ungesättigten, wahlweise zumindest mono-substituierten, wahlweise zumindest ein Heteroatom als Ringelement enthaltenden mono- oder bicyclischen cycloaliphatischen Ringssystem kondensiert sein kann, repräsentiert,
R^{3c} Wasserstoff oder ein wahlweise zumindest mono-substituiertes, lineares oder verzweigtes Alkylradikal repräsentiert,
R^{4c} and R^{5c}, identisch oder verschieden, Wasserstoff oder ein wahlweise zumindest mono-substituiertes, lineares oder verzweigtes Alkylradikal repräsentiert oder
R^{4c} and R^{5c} zusammen mit dem Brückenstickstoffatom einen wahlweise zumindest mono-substituierten, gesättigten oder ungesättigten heterocyclischen Ring bilden, der zumindest ein weiteres Heteroatom als Ringelement enthalten kann und/oder mit einem gesättigten oder ungesättigten, wahlweise zumindest mono-substituierten, wahlweise zumindest ein Heteroatom als Ringelement enthaltenden mono- oder bicyclischen cycloaliphatischen Ringsystem kondensiert sein kann,
A^{c} ein wahlweise zumindest mono-substituiertes mono- oder polycyclisches aromatisches Ringsystem repräsentiert, das über eine wahlweise zumindest mono-substituierte Alkylen-, eine wahlweise zumindest mono-substituierte Alkenylen-, oder eine wahlweise zumindest mono-substituierte Alkinylen-Gruppe gebunden sein kann und/oder zumindest ein Heteroatom als Ringelement in einem oder mehreren seiner Ringe enthalten kann, repräsentiert,
nc 0, 1, 2, 3 oder 4 repräsentiert;
wahlweise in Form eines seiner Stereoisomere, vorzugsweise von Enantiomeren oder Diastereomeren, seines Racemats oder in Form eines Gemisches von zumindest zweien seiner Stereoisomere, vorzugsweise Enantiomeren oder Diastereomeren, in jedem Mischungsverhältnis, oder ein entsprechendes physiologisch akzeptables Salz oder ein entsprechendes Solvat;
und von Verbindungen der allgemeinen Formel (Id) 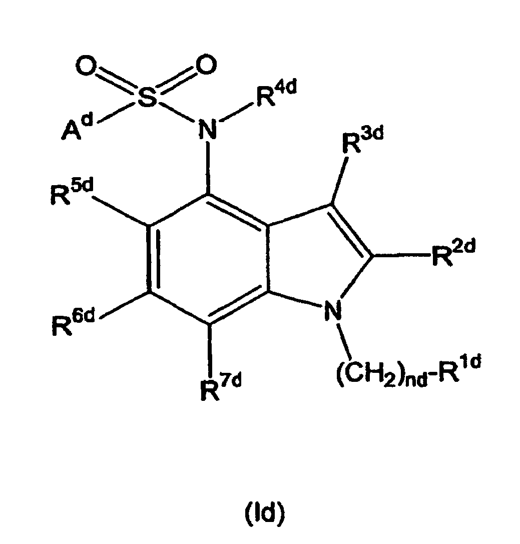 wobei
R^{1d} ein -NR^{3d}R^{9d} Radikal oder ein gesättigtes oder ungesättigtes, wahlweise zumindest mono-substituiertes cycloaliphatisches Radikal, das zumindest ein Heteroatom als Ringelement enthalten kann und/oder das mit einem gesättigten oder ungesättigten, wahlweise zumindest mono-substituierten, wahlweise zumindest ein Heteroatom als Ringelement enthaltenden mono- oder bicyclischen cycloaliphatischen Ringsystem kondensiert sein kann, repräsentiert,
R^{2d}, R^{3d}, R^{5d} and R^{6d} und R^{7d}, identisch oder verschieden, jeweils Wasserstoff, Halogen, Nitro, Alkoxy, Cyano, ein gesättigtes oder ungesättigtes, lineares oder verzweigtes, wahlweise zumindest mono-substituiertes aliphatisches Radikal oder ein wahlweise zumindest mono-substituiertes Phenyl oder ein wahlweise zumindest mono-substituiertes Heteroarylradikal repräsentiert,
R^{4d} ein gesättigtes oder ungesättigtes, lineares oder verzweigtes, wahlweise zumindest mono-substituiertes aliphatisches Radikal ist,
R^{8d} and R^{9d}, identisch oder verschieden, jeweils Wasserstoff oder ein gesättigtes oder ungesättigtes, lineares oder verzweigtes, wahlweise zumindest mono-substituiertes aliphatisches Radikal repräsentiert,
oder
R^{8d} and R^{9d} zusammen mit dem Brückenstickstoffatom einen gesättigten, ungesättigten, wahlweise mono-substituierten heterocyclischen Ring bilden, der ein zusätzliches Heteroatom als Ringelement enthalten kann und/oder mit einem gesättigten oder ungesättigten, wahlweise zumindest mono-substituierten mono- oder bicyclischen cycloaliphatischen Ringsystem kondensiert sein kann, das wahlweise zumindest ein Heteroatom als Ringelement enthalten kann,
A^{d} ein wahlweise zumindest mono-substituiertes mono- oder polycyclisches aromatisches Ringsystem repräsentiert, das über eine wahlweise zumindest mono-substituierte Alkylen-, Alkenylen- oder Alkinylen-Gruppe gebunden sein kann und/oder, die zumindest ein Heteroatom als Ringelement in einem oder mehreren seiner Ringe enthalten kann,
und
nd 0, 1, 2, 3 oder 4 ist;
wahlweise in Form eines seiner Stereoisomere, vorzugsweise Enantiomere oder Diastereomere, seines Racemats oder in Form eines Gemisches von zumindest zweien seiner Stereoisomere, vorzugsweise Enantiomere oder Diastereomere, in jedem Mischungsverhältnis, oder ein Salz hiervon, bevorzugt ein entsprechendes physiologisch akzeptables Salz hiervon, oder ein entsprechendes Solvat hiervon,
und Verbindungen, abgeleitet von einem Sulfonamid der allgemeinen Formel (Ie) 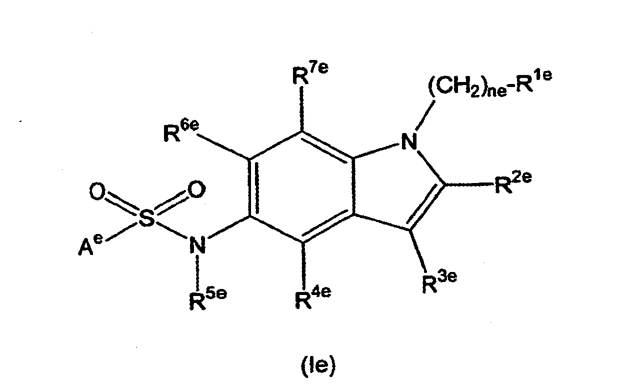 wobei R^{1e} ein -NR^{8e}R^{9e}-Radikal oder ein gesättigtes oder ungesättigtes, wahlweise zumindest mono-substituiertes cycloaliphatisches Radikal repräsentiert, das wahlweise zumindest ein Heteroatom als Ringelement enthalten kann und/oder das mit einem gesättigten oder ungesättigten, wahlweise zumindest mono-substituierten mono- oder bicyclischen cycloaliphatischen Ringsystem kondensiert sein kann, das wahlweise zumindest ein Heteroatom als Ringelement enthält,
R^{2e}, R^{3e}, R^{4e}, R^{6e} and R^{7e}, identisch oder verschieden, jeweils Wasserstoff, Halogen, Nitro, Alkoxy, Cyano, ein gesättigtes oder ungesättigtes, lineares oder verzweigtes, wahlweise zumindest mono-substituiertes aliphatisches Radikal oder ein wahlweise zumindest mono-substituiertes Phenyl oder ein wahlweise zumindest mono-substituiertes Heteroarylradikal repräsentiert,
R^{5e} Wasserstoff oder ein gesättigtes oder ungesättigtes, lineares oder verzweigtes, wahlweise zumindest mono-substituiertes aliphatisches Radikal repräsentiert,
R^{8e} and R^{9e} , identisch oder verschieden, jeweils Wasserstoff oder ein gesättigtes oder ungesättigtes, lineares oder verzweigtes, wahlweise zumindest mono-substituiertes aliphatisches Radikal repräsentieren,
oder
R^{8e} und R^{9e} zusammen mit dem Brückenstickstoffatom einen gesättigten oder ungesättigten, wahlweise zumindest mono-substituierten heterocyclischen Ring bilden, der zumindest ein zusätzliches Heteroatom als Ringelement enthalten kann und/oder, der mit einem gesättigten oder ungesättigten, wahlweise zumindest mono-substituierten, mono- oder bicyclischen cycloaliphatischen Ringsystem kondensiert sein kann, das wahlweise zumindest ein Heteroatom als Ringelement enthält,
A^{e} ein wahlweise zumindest mono-substituiertes mono- oder polycyclisches aromatisches Ringsystem repräsentiert, das über eine wahlweise zumindest mono-substituierte Alkylen-, Alkenylen- oder Alkinylen-Gruppe gebunden sein kann und/oder, das zumindest ein Heteroatom als Ringelement in einem oder mehreren seiner Ringe enthalten kann,
und
ne 0, 1, 2, 3 oder 4 ist;
wahlweise in Form eines seiner Stereoisomere, vorzugsweise von Enantiomeren oder Diastereomeren, seines Racemats oder in Form eines Gemisches von zumindest zweien seiner Stereoisomere, vorzugsweise von Enantiomeren oder Diastereomeren in irgendeinem Mischungsverhältnis, oder eines entsprechenden physiologisch akzeptablen Salzes oder ein entsprechendes Solvat,
und Verbindungen, die aus einem Sulfonamid der allgemeinen Formel (If) abgeleitet sind 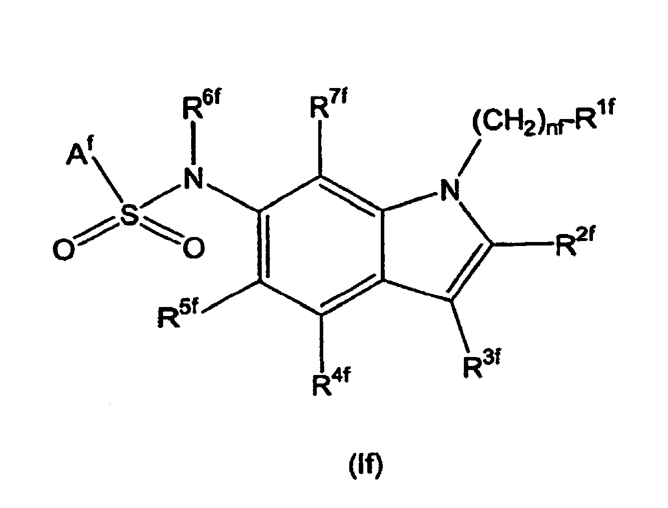 wobei R^{1f} ein -NR^{8f}R^{9f}-Radikal oder ein gesättigtes oder ungesättigtes, wahlweise zumindest mono-substituiertes, wahlweise zumindest ein Heteroatom als Ringelement enthaltendes cycloaliphatisches Radikal, das mit einem gesättigten oder ungesättigten, wahlweise zumindest mono-substituierten, wahlweise zumindest ein Heteroatom als Ringelement enthaltenden mono- oder bicyclischen cycloaliphatischen Ringsystem kondensiert ist, repräsentiert,
R^{2f}, R^{3f}, R^{4f}, R^{5f} and R^{7f}, identisch oder verschieden, jeweils Wasserstoff, Halogen, Nitro, Alkoxy, Cyano, ein gesättigtes oder ungesättigtes, lineares oder verzweigtes, wahlweise zumindest mono-substituiertes aliphatisches Radikal, oder wahlweise zumindest mono-substituiertes Phenylradikal oder ein wahlweise zumindest mono-substituiertes Heteroarylradikal repräsentieren,
R^{6f} Wasserstoff oder ein gesättigtes oder ungesättigtes, lineares oder verzweigtes, wahlweise zumindest mono-substituiertes aliphatisches Radikal repräsentiert,
R^{8f} and R^{9f}, identisch oder verschieden, jeweils Wasserstoff oder ein gesättigtes oder ungesättigtes, lineares oder verzweigtes, wahlweise zumindest mono-substituiertes aliphatisches Radikal repräsentieren,
oder
R^{8f} und R^{9f}, zusammen mit dem Brückenstickstoffatom, einen gesättigten oder ungesättigten, wahlweise zumindest mono-substituierten heterocyclischen Ring bilden, der zumindest ein weiteres Heteroatom als Ringelement enthalten kann und/oder, der mit einem gesättigten oder ungesättigten, wahlweise zumindest mono-substituierten, wahlweise zumindest ein Heteroatom als Ringelement enthaltenden mono- oder bicyclischen cycloaliphatischen Ringsystem kondensiert sein kann,
A^{f} ein wahlweise zumindest mono-substituiertes mono- oder polycyclisches aromatisches Ringsystem, das über eine wahlweise zumindest mono-substituierte Alkylen-, Alkenylen- oder Alkinylen-Gruppe gebunden sein kann und/oder die zumindest ein Heteroatom als Ringelement in einem oder mehreren seiner Ringe enthalten kann, repräsentiert,
und
n^{f} 0, 1, 2, 3 oder 4 ist;
wahlweise in Form eines seiner Stereoisomere, vorzugsweise Enantiomeren oder Diastereomeren, seines Racemats oder in Form eines Gemisches von zumindest zweien seiner Stereoisomere, vorzugsweise von Enantiomeren oder Diastereomeren, in irgendeinem Mischungsverhältnis, oder ein entsprechendes, physiologisch akzeptables Salz oder ein entsprechendes Solvat,
und Verbindungen, die aus einem Sulfonamid der allgemeinen Formel (Ig) abgeleitet sind 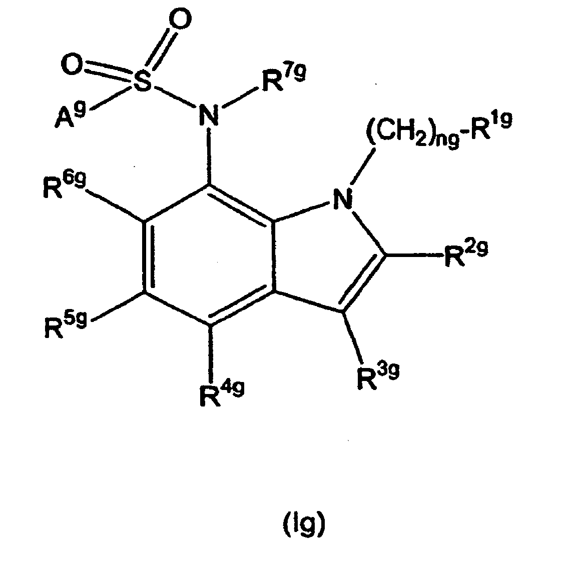 wobei
R^{1g} ein -NR^{8g}R^{9g}-Radikal oder ein gestättigtes oder ungesättigtes, wahlweise zumindest mono-substituiertes cycloaliphatisches Radikal ist, das wahlweise zumindest ein Heteroatom als Ringelement enthalten kann und das mit einem gesättigten, oder ungesättigten, wahlweise zumindest mono-substituierten mono- oder bicyclischen cycloaliphatischen Ringsystem kondensiert sein kann, das wahlweise zumindest ein Heteroatom als Ringelement enthält,
R^{2g}, R^{3g}, R^{4g}, R^{5g} und R^{6g} , identisch oder verschieden, jeweils Wasserstoff, Halogen, Nitro, Alkoxy, Cyano, oder ein gesättigtes oder ungesättigtes, lineares oder verzweigtes, wahlweise zumindest mono-substituiertes aliphatisches Radikal oder ein wahlweise zumindest mono-substituiertes Phenylradikal oder ein wahlweise zumindest mono-substituiertes Heteroarylradikal repräsentieren,
R^{7g} Wasserstoff oder ein gesättigtes oder ungesättigtes, lineares oder verzweigtes, wahlweise zumindest mono-substituiertes aliphatisches Radikal repräsentiert,
R^{8g} und R^{9g}, identisch oder verschieden, Wasserstoff oder ein gesättigtes, lineares oder verzweigtes, wahlweise zumindest mono-substituiertes aliphatisches Radikal repräsentieren,
oder
R^{8g} and R^{9g} zusammen mit dem Brückenstickstoffatom einen gesättigten oder ungesättigten, wahlweise zumindest mono-substituierten heterocyclischen Ring bilden, der zumindest ein zusätzliches Heteroatom als Ringelement enthalten kann und/oder, der mit einem gesättigten oder ungesättigten, wahlweise mono-substituierten mono- oder bicyclischen cycloaliphatischen Ringsystem kondensiert sein kann, das wahlweise zumindest ein Heteroatom als Ringelement enthält,
A⁹ ein wahlweise zumindest mono-substituiertes mono- oder polycyclisches aromatisches Ringsystem enthält, das über eine wahlweise zumindest mono-substituierte Alkylen, Alkenylen oder Alkinylen-Gruppe gebunden ist und/oder, die zumindest ein Heteroatom als Ringelement in einem oder mehreren seiner Ringe enthalten kann,
ng 0,1, 2, 3 or 4 ist;
wahlweise in Form eines seiner Stereoisomere, vorzugsweise von Enantiomeren oder Diastereomeren, seines Racemats oder in Form eines Gemisches aus zumindest zweien seiner Stereoisomere, vorzugsweise Enantiomere oder Diastereomere, in jedem Mischungsverhältnis, oder ein entsprechendes, physiologisch akzeptables Salz oder ein entsprechendes Solvat,
und Verbindungen, die aus einem Sulfonamid der allgemeinen Formel (Ih) abgeleitet sind, 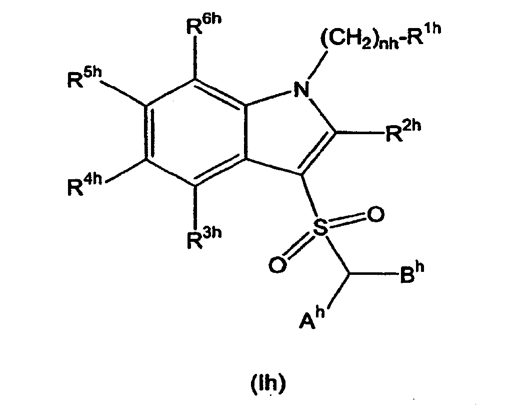 wobei
R^{1h} ein -NR^{7h}R^{8h}-Radikal oder ein gesättigtes oder ungesättigtes, wahlweise zumindest mono-substituiertes, wahlweise zumindest ein Heteroatom als Ringelement enthaltendes cycloaliphatisches Radikal, das mit einem gesättigten oder ungesättigten, wahlweise zumindest mono-substituierten, wahlweise zumindest ein Heteroatom als Ringelement enthaltenden mono- oder bicyclischen cycloaliphatischen Ringsystem kondensiert sein kann, repräsentiert,
R^{2h}, R^{3h}, R^{4h}, R^{5h} und R^{6h}, identisch oder verschieden, jeweils Wasserstoff, Halogen, Cyano, Nitro, ein lineares oder verzweigtes Alkylradikal, ein lineares oder verzweigtes Alkenyl-Radikal, ein lineares oder verzweigtes Alkinyl-Radikal, ein lineares oder verzweigtes Alkoxy-Radikal, ein lineares oder verzweigtes Alkylthio-Radikal, Hydroxy, Trifluormethyl, ein Cycloalkyl-Radikal, ein Cycloalkenyl-Radikal, ein Alkylcarbonyl-Radikal, eine Phenylcarbonyl- oder eine -NR^{9h}-R^{10h}-Gruppe repräsentiert,
R^{7h} und R^{8h} identisch oder verschieden, jeweils Wasserstoff oder ein gesättigtes oder ungesättigtes, wahlweise zumindest mono-substituiertes, lineares oder verzweigtes aliphatisches Radikal repräsentieren,
oder
R^{7h} and R^{8h}, zusammen mit dem Brückenstickstoffatom, einen gesättigten oder ungesättigten, wahlweise zumindest mono-substituierten, wahlweise zumindest ein weiteres Heteroatom als Ringelement enthaltenden heterocyclischen Ring bilden, der mit einem gesättigten oder ungesättigten, wahlweise zumindest mono-substituierten, wahlweise zumindest ein Heteroatom als Ringelement enthaltenden mono- oder bicyclischen cycloaliphatischen Ringsystem kondensiert sein kann,
R^{9h}R^{10h}, identisch oder verschieden, jeweils Wasserstoff oder ein gesättigtes oder ungesättigtes, lineares oder verzweigtes, wahlweise zumindest mono-substituiertes aliphatisches Radikal repräsentieren,
oder
R^{9h} und R^{10h} zusammen mit dem Brückenstickstoffatom, einen gesättigten oder ungesättigten, wahlweise zumindest mono-substituierten, wahlweise zumindest ein weiteres Heteroatom als Ringelement enthaltenden heterocyclischen Ring bilden, der mit einem gesättigten oder ungesättigten, wahlweise zumindest mono-substituierten, wahlweise zumindest ein Heteroatom als Ringelement enthaltenden mono- oder bicyclischen cycloaliphatischen Ringsystem kondensiert sein kann,
A^{h} und B^{h}, identisch oder verschieden, jeweils ein gesättigtes oder ungesättigtes, lineares oder verzweigtes, wahlweise zumindest mono-substituiertes aliphatisches Radikal repräsentieren,
oder
A^{h} und B^{h}, zusammen mit dem Kohlenstoffatom, an das sie gebunden sind, einen gesättigten oder ungesättigten, jedoch nicht aromatischen, wahlweise zumindest mono-substituierten Cycloalkyl-Ring bilden,
und
nh 0, 1,2, 3 or 4 ist, wahlweise in Form eines seiner Stereoisomere, vorzugsweise Enantiomere oder Diastereomere, seiner Racemate oder in Form eines Gemisches von zumindest zweien seiner Stereoisomere, vorzugsweise Enantiomere oder Diastereomere, in jedem Mischungsverhältnis, oder ein entsprechendes, physiologisch akzeptables Salz oder ein entsprechendes Solvat,
besteht.

2. Kombination nach Anspruch 1, **dadurch gekennzeichnet, dass** sie 1-99% Gew.-% von Bestandteil (A) and 99-1% Gew.-% von Bestandteil (B), besonders bevorzugt 10-80% Gew.-% von Bestandteil (A) and 90-20% Gew.-% von Bestandteil (B) umfasst, in jedem Fall bezogen auf das Gesamtgewicht der beiden Bestandteile (A) and (B).

3. Medikament, das eine Wirkstoffkombination gemäß Anspruch 1 oder 2 und wahlweise ein oder mehrere pharmakologisch akzeptable Adjuvantien umfasst.

4. Medikament nach Anspruch 3 zur Regulierung des Appetits, zur Aufrechterhaltung, Zunahme oder Reduktion des Körpergewichts, zur Prophylaxe und/oder Behandlung von Störungen, die mit der Nahrungsaufnahme verbunden sind, vorzugsweise zur Prophylaxe und/oder Behandlung der Adipositas, Anorexie, Cachexie, Bulämie, Diabetes, vorzugsweise Typ 11-Diabetes (nicht insulin-abhängige Diabetes mellitus), oder zur Prophylaxe und/oder Behandlung von Störungen des gastrointestinalen Trakts, vorzugsweise des Reizdarmsyndroms, zur Prophylaxe und/oder zur Behandlung von Störungen des peripheren Nervensystems, des zentralen Nervensystems, der Arthritis, der Epilepsie, von Angstzuständen, der Panik, von Depressionen, von kognitiven Störungen, von Gedächtnisstörungen, von kardiovaskulären Erkrankungen, von senilen Demenz-Prozessen, wie beispielsweise Alzheimer Krankheit, Parkinson Krankheit und/oder Huntington-Krankheit, Schizophrenie, Psychosen, infantiler Hyperkinesie (ADHD, Aufmerksamkeitsdefizit/Hyperaktivitätsstörungen), Schmerz, hypertensivem Syndrom, entzündlichen Erkrankungen, immunologischen Erkrankungen oder Verbesserung der Wahrnehmung.

5. Verwendung der Kombination nach Anspruch 1 und 2 zur Herstellung eines Medikamentes zur Regulierung des Appetits.

6. Verwendung der Kombination nach Anspruch 1 oder 2 zur Herstellung eines Medikamentes zur Aufrechterhaltung, Erhöhung oder Reduktion des Körpergewichts.

7. Verwendung der Kombination nach Anspruch 1 oder 2 zur Herstellung eines Medikamentes zur Prophylaxe und/oder Behandlung von Störungen, die mit der Nahrungsaufnahme in Verbindung stehen, vorzugsweise zur Prophylaxe und/oder Behandlung der Adipositas, der Anorexie, der Cachexie, Bulämie, Diabetes, vorzugsweise Typ II-Diabetes (nicht insulin-abhängiger Diabetes mellitus).

8. Verwendung der Kombination nach Anspruch 1 oder 2, zur Herstellung eines Medikamentes zur Prophylaxe und/oder Behandlung von Störungen des Gastrointestinal-Trakts, vorzugsweise des Reizdarmssyndroms.

9. Verwendung der Kombination nach Anspruch 1 oder 2 zur Herstellung eines Medikamentes zur Prophylaxe und/oder Behandlung von Störungen des peripheren Nervensystems.

10. Verwendung der Kombination nach Anspruch 1 oder 2 zur Herstellung eines Medikamentes zur Prophylaxe und/oder Behandlung von Störungen des zentralen Nervensystems.

11. Verwendung der Kombination nach Anspruch 1 oder 2 zur Herstellung eines Medikamentes zur Prophylaxe und/oder Behandlung der Arthritis.

12. Verwendung der Kombination nach Anspruch 1 oder 2 zur Herstellung eines Medikamentes zur Prophylaxe und/oder Behandlung der Epilepsie.

13. Verwendung der Kombination nach Anspruch 1 oder 2 zur Herstellung eines Medikamentes zur Prophylaxe und/oder Behandlung von Angstzuständen.

14. Verwendung der Kombination nach Anspruch 1 oder 2 zur Herstellung eines Medikamentes zur Prophylaxe und/oder Behandlung der Panik.

15. Verwendung der Kombination nach Anspruch 1 oder 2 zur Herstellung eines Medikamentes zur Prophylaxe und/oder Behandlung der Depression.

16. Verwendung der Kombination nach Anspruch 1 oder 2 zur Herstellung eines Medikamentes zur Prophylaxe und/oder Behandlung von bipolaren Störungen.

17. Verwendung der Kombination nach Anspruch 1 oder 2 zur Herstellung eines Medikamentes zur Prophylaxe und/oder Behandlung von kognitiven Störungen.

18. Verwendung der Kombination nach Anspruch 1 oder 2 zur Herstellung eines Medikamentes zur Prophylaxe und/oder Behandlung von Gedächtnisstörungen.

19. Verwendung der Kombination nach Anspruch 1 oder 2 zur Herstellung eines Medikamentes zur Prophylaxe und/oder Behandlung von kardiovaskulären Erkrankungen.

20. Verwendung der Kombination nach Anspruch 1 oder 2 zur Herstellung eines Medikamentes zur Prophylaxe und/oder Behandlung von senilen Demenz-Prozessen.

21. Verwendung der Kombination nach Anspruch 1 oder 2 zur Herstellung eines Medikamentes zur Prophylaxe und/oder Behandlung von neurodegenerativen Störungen, vorzugsweise der Alzheimer Krankheit, Parkinson-Krankheit, Huntington-Krankheit und/oder der multiplen Sklerose.

22. Verwendung der Kombination nach Anspruch 1 oder 2 zur Herstellung eines Medikamentes zur Prophylaxe und/oder Behandlung der Schizophrenie.

23. Verwendung der Kombination nach Anspruch 1 oder 2 zur Herstellung eines Medikamentes zur Prophylaxe und/oder Behandlung der Psychose.

24. Verwendung der Kombination nach Anspruch 1 oder 2 zur Herstellung eines Medikamentes zur Prophylaxe und/oder Behandlung der infantilen Hyperkinesie (ADHD, Aufmerksamkeitsdefizit/Hyperaktivitätsstörung).

25. Verwendung der Kombination nach Anspruch 1 oder 2 zur Herstellung eines Medikamentes zur Prophylaxe und/oder Behandlung von Schmerz.

26. Verwendung der Kombination nach Anspruch 1 oder 2 zur Herstellung eines Medikamentes zur Prophylaxe und/oder Behandlung des hypertensiven Syndroms.

27. Verwendung der Kombination nach Anspruch 1 oder 2 zur Herstellung eines Medikamentes zur Prophylaxe und/oder Behandlung von entzündlichen Erkrankungen.

28. Verwendung der Kombination nach Anspruch 1 oder 2 zur Herstellung eines Medikamentes zur Prophylaxe und/oder Behandlung von immunologischen Erkrankungen.

29. Verwendung der Kombination nach Anspruch 1 oder 2 zur Herstellung eines Medikamentes zur Verbesserung der Wahrnehmung.

30. Pharmazeutische Formulierung, **dadurch gekennzeichnet, dass** sie eine Wirkstoffkombination gemäß Anspruch 1 oder 2 und wahlweise ein oder mehrere pharmakologisch akzeptable Hilfsstoffe umfasst.

31. Pharmazeutische Formulierung nach Anspruch 30, **dadurch gekennzeichnet, dass** sie in festen pharmazeutischen Formen, wie beispielsweise Tabletten, Kautabletten, Kaugummis, Dragees, Kapseln, Suppositorien, Pulverzubereitungen, transdermalen therapeutischen Systemen, transmukosalen therapeutischen Systemen oder in flüssigen oder halbflüssigen pharmazeutischen Formen, wie beispielsweise Tropfen oder wie beispielsweise Saft, Sirup, Lösung, Emulsion, Suspension, vorzugsweise in Form von Tabletten, Kapseln, Tropfen oder Lösung vorliegt.

32. Pharmazeutische Formulierung nach Anspruch 30, **dadurch gekennzeichnet, dass** sie in Form von multiplen Teilchen, vorzugsweise Mikrotabletten, Mikrokapseln, Mikrospheroiden, Granulaten, Kristallen oder Pellets, wahlweise zu einer Tablette verpresst, in einer Kapsel gefüllt oder in einer geeigneten Flüssigkeit suspendiert, vorliegt.

33. Pharmazeutische Zubereitung nach einem der Ansprüche 30 bis 32, **dadurch gekennzeichnet, dass** sie für eine orale, intravenöse, intramuskuläre subkutane, intrathekale, epidurale, bukkale, sublinguale, pulmonale, rektale, transdermale, nasale oder intrazerebroventrikuläre Verabreichung vorgesehen ist, vorzugsweise oral oder intravenös.

34. Pharmazeutische Zubereitung nach einem oder mehreren der Ansprüchen 30 bis 32, **dadurch gekennzeichnet, dass** zumindest eine der Bestandteile der Wirkstoffkombination (A) oder (B) zumindest teilweise zur verzögerten Freisetzungsform vorliegt.

35. Pharmazeutische Zubereitung nach Anspruch 34, **dadurch gekennzeichnet, dass** das Medikament zumindest eine Beschichtung oder zumindest eine Matrix aufweist, die zumindest ein Material umfasst, das die Freisetzung des Wirkstoffes verzögert.

36. Pharmazeutische Formulierung nach Anspruch 35, **dadurch gekennzeichnet, dass** das Material für die verzögerte Freisetzung auf wahlweise modifiziertem, wasserunlöslichem, natürlichem, halbsynthetischem oder synthetischem Polymer, auf einen natürlichem Wachs oder Fett oder Fettalkohol oder halbsynthetischen oder synthetischen Fettsäuren oder auf einem Gemisch von zumindest zwei dieser vorher genannten Bestandteile basiert.

37. Pharmazeutische Formulierung nach Anspruch 36, **dadurch gekennzeichnet, dass** das wasserunlösliche Polymer auf einem Acrylharz basiert, das vorzugsweise aus der Gruppe von Poly(meth)acrylaten, Poly(C₁₋₄)dialkylamino(C₁₋₄)alkyl(meth)acrylaten und/oder Copolymeren hiervon oder einem Gemisch aus zumindest zwei der vorher erwähnten Polymere ausgewählt ist.

38. Pharmazeutische Formulierung nach Anspruch 36, **dadurch gekennzeichnet, dass** die wasserunlöslichen Polymere Cellulosederivate, vorzugsweise Alkylcellulose sind und noch mehr bevorzugt Ethylcellulose oder Celluloseester sind.

39. Pharmazeutische Formulierung nach Anspruch 36, **dadurch gekennzeichnet, dass** das Wachs Carnaubawachs, Bienenwachs, Glycerolmonostearat, Glycerolmonobehenat, Glycerolditripalmitostearat, microcristallines Wachs oder ein Gemisch aus zumindest zwei dieser Bestandteile, ist.

40. Pharmazeutische Formulierung nach einem oder mehreren der Ansprüche 36 bis 39, **dadurch gekennzeichnet, dass** Polymere in Kombination mit ein oder mehreren Weichmachern verwendet wurden.

41. Pharmazeutische Formulierung nach einem oder mehreren der Ansprüche 34 bis 40, **dadurch gekennzeichnet, dass** neben der Form mit verzögerter Freisetzung zumindest eine der Wirkstoffbestandteile (A) oder (B) in einer nicht-verzögerten-Freisetzungsform vorliegt.

## Revendications

1. Combinaison de substances actives, **caractérisée en ce qu'**elle comprend :
(A) au moins un composé avec une affinité pour le récepteur du neuropeptide Y (NPY), choisi parmi le groupe consistant en les composés dérivés de pipéridine 1,4-disubstituée de formule générale (Ia) : 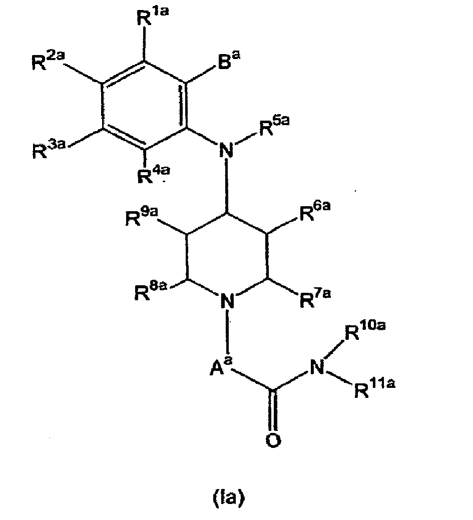 dans laquelle :
R^{1a} R^{2a}, R^{3a} et R^{4a} sont indépendamment choisis chacun dans le groupe consistant en un atome d'hydrogène, un atome d'halogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, et un groupe nitro, cyano, -OR^{12a}, -O-(C=O)R^{13a}, -(C=O)-OR^{13a}, -SR^{14a}, -SOR^{14a}, -SO₂R^{14a}, -NH-SO₂R^{14a}, -SO₂NH2 et -NR^{15a}R^{16a},
R^{5a} représente un atome d'hydrogène, un radical aliphatique non ramifié ou ramifié, saturé ou insaturé, facultativement au moins monosubstitué, ou un radical cycloaliphatique saturé ou insaturé, facultativement au moins monosubstitué,
R^{6a}, R^{7a} , R^{8a} et R^{9a} sont indépendamment choisis chacun dans le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que que chaînon de cycle, et un groupe cyano et un groupe COOR^{17a},
A^{a} représente un chaînon en pont -CHR^{18a}- ou -CHR^{18a}-CH₂-,
B^{a} représente un radical aliphatique non ramifié ou ramifié, saturé ou insaturé, facultativement au moins monosubstitué, un radical cycloaliphatique saturé ou insaturé, facultativement au moins monosubstitué, un groupe-COOR^{19a}, un groupe -(C=O)R^{20a} ou un groupe -CH₂OR^{23a},
R^{10a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{11a} représente un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué et/ou peut être condensé avec un système de noyau mono ou polycyclique facultativement au moins monosubstitué, ou
R^{10a} et R^{11a} forment ensemble avec l'atome d'azote pontant, un noyau hétérocyclique saturé, insaturé ou aromatique, facultativement au moins monosubstitué, qui peut contenir au moins un autre hétéroatome en tant que chaînon de cycle, et/ou peut être condensé avec un système de noyau mono ou polycyclique facultativement au moins monosubstitué,
R^{12a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{13a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{14a} représente un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{15a} et R^{16a} sont indépendamment choisis chacun dans le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, ou
R^{15a} et R^{16a} forment ensemble avec l'atome d'azote pontant, un noyau hétérocyclique saturé, insaturé ou aromatique, qui peut être au moins monosubstitué, et/ou contenir au moins un autre hétéroatome en tant que chaînon de cycle,
R^{17a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{18a} représente un atome d'hydrogène, un radical aliphatique non ramifié ou ramifié, saturé ou insaturé, facultativement au moins monosubstitué, ou un radical cycloaliphatique saturé ou insaturé, facultativement au moins monosubstitué, contenant facultativement au moins un hétéroatome en tant que chaînon de cycle, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{19a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, ou un radical aryle ou hétéroaryle facultativement au moins monosubstitué, qui peut être condensé avec un système de noyau mono ou polycyclique facultativement au moins monosubstitué,
R^{20a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, un radical aryle ou hétéroaryle facultativement au moins monosubstitué, qui peut être condensé avec un système de noyau mono ou polycyclique facultativement monosubstitué, ou un groupe NR^{21a}R^{22a},
R^{21a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, ou un radical aryle ou hétéroaryle facultativement au moins monosubstitué, qui peut être condensé avec un système de noyau mono ou polycyclique facultativement au moins monosubstitué,
R^{22a} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, ou un radical aryle ou hétéroaryle facultativement au moins monosubstitué, qui peut être condensé avec un système de noyau mono ou polycyclique facultativement au moins monosubstitué,
R^{23a} représente un atome d'hydrogène, un radical aliphatique non ramifié ou ramifié, saturé ou insaturé, facultativement au moins monosubstitué, qui peut comprendre au moins un hétéroatome en tant que chaînon, ou un groupe -(C=O)R^{13a},
facultativement sous la forme de l'un de leurs stéréoisomères, de préférence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport de mélange, ou des sels, de préférence, les sels physiologiquement acceptables de ceux-ci, ou les solvats correspondants ; et
(B) au moins un composé ayant une affinité pour le récepteur de 5-HT₆, choisi dans le groupe consistant en les composés de type sulfonamide dérivés de benzoxazinone de formule générale (Ib) : 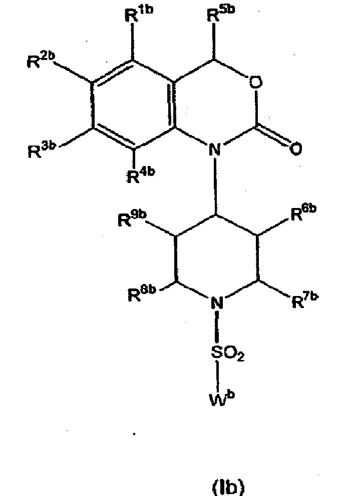 dans laquelle :
R^{1b}, , R^{2b} R^{3b} et R^{4b} sont indépendamment choisis chacun dans le groupe consistant en un atome d'hydrogène, un atome d'halogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, et un groupe nitro, cyano, -OR^{10b}, -O-(C=O)R^{11b}, -(C=O)-OR^{11b,} , -SR^{12b} , -SOR^{12b} -SO₂R^{12b} NH-SO₂R^{12b} -SO2NH₂ et -NR^{13b}R^{14b},
R^{5b} représente un atome d'hydrogène, un radical aliphatique non ramifié ou ramifié, saturé ou insaturé, facultativement au moins monosubstitué, ou un radical cycloaliphatique saturé ou insaturé, facultativement au moins monosubstitué, contenant facultativement au moins un hétéroatome en tant que chaînon de cycle,
R^{6b}, R^{7b} R^{8b} et R^{9b} sont indépendamment choisis chacun dans le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que que chaînon de cycle, et les groupes cyano et COOR^{15b},
W^{b} représente un radical aliphatique non ramifié ou ramifié, saturé ou insaturé, facultativement au moins monosubstitué,
un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement monosubstitué, et/ou peut être condensé avec un système de noyau mono ou polycyclique facultativement au moins monosubstitué,
un radical aryle ou hétéroaryle facultativement au moins monosubstitué, qui peut être lié par l'intermédiaire d'un groupe alkylène ou alcénylène facultativement au moins monosubstitué, et/ou peut être condensé avec un système de noyau mono ou polycyclique facultativement au moins monosubstitué,
un groupe NR^{16b}R^{17b}, ou
un groupe COR^{18b},
R^{10b} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène au moins monosubstitué et/ou peut être condensé avec un système de noyau mono ou ploycyclique au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{11b} représente l'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène au moins monosubstitué et/ou peut être condensé avec un système de noyau mono ou polycyclique facultativement au moins monosubstitué,
R^{12b} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{13b} et R^{14b} sont indépendamment choisis chacun dans le groupe consistant en un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, qui peut être lié par l'intermédiaire d'un groupe alkylène facultativement au moins monosubstitué, et/ou peut être condensé avec un système de noyau mono ou polycyclique facultativement au moins monosubstitué, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué, ou
R^{13b} et R^{14b} forment ensemble avec l'atome d'azote pontant, un noyau hétérocyclique saturé, insaturé ou aromatique, qui peut être au moins monosubstitué, et/ou contenir au moins un autre hétéroatome en tant que chaînon de cycle,
R^{15b} représente un atome d'hydrogène, un radical aliphatique non ramifié ou ramifié, saturé ou insaturé, facultativement au moins monosubstitué, un radical cycloaliphatique saturé ou insaturé, facultativement au moins monosubstitué, contenant facultativement au moins un hétéroatome en tant que chaînon de cycle, ou un radical aryle ou hétéroaryle facultativement au moins monosubstitué, qui peut être lié par l'intermédiaire d'un groupe alkylène facultativement au moins monosubstitué, et/ou peut être condensé avec un système de noyau mono ou polycyclique facultativement au moins monosubstitué,
R^{16b} représente un radical aliphatique non ramifié ou ramifié, saturé ou insaturé, facultativement au moins monosubstitué,
R^{17b} représente un radical aliphatique non ramifié ou ramifié, saturé ou insaturé, facultativement au moins monosubstitué, et
R^{18b} représente un radical aryle facultativement au moins monosubstitué,
facultativement, respectivement, sous la forme de l'un de leurs stéréoisomères, de préférence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport de mélange, ou un sel physiologiquement acceptable de ceux-ci, ou un solvat, et
les composés dérivés de sulfonamide de formule générale (Ic), 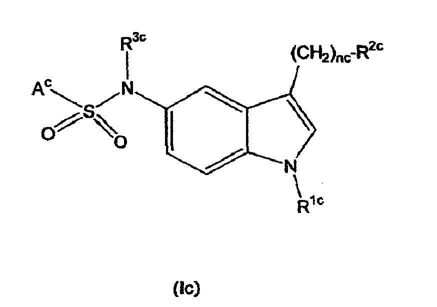
dans laquelle :
R^{1c} représente un atome d'hydrogène, un radical alkyle linéaire ou ramifié, facultativement au moins monosubstitué, un radical phényle facultativement au moins monosubstitué ou un radical benzyle facultativement au moins monosubstitué,
R^{2c} représente un groupe -NR^{4c}R^{5c} ou un radical cycloaliphatique saturé ou insaturé, facultativement au moins monosubstitué, contenant facultativement au moins un hétéroatome en tant que chaînon de cycle, qui peut être condensé avec un système de noyau cycloaliphatique mono ou bicyclique saturé ou insaturé, facultativement au moins monosubstitué, contenant facultativement au moins un hétéroatome en tant que chaînon de cycle,
R^{3c} représente un atome d'hydrogène ou un radical alkyle linéaire ou ramifié, facultativement au moins monosubstitué,
R^{4c} et R^{5c}, qui sont identiques ou différents, représentent un atome d'hydrogène ou un radical alkyle linéaire ou ramifié, facultativement au moins monosubstitué, ou
R^{4c} et R^{5c} forment ensemble, avec l'atome d'azote pontant, un noyau hétérocyclique saturé ou insaturé, facultativement au moins monosubstitué, qui peut contenir au moins un autre hétéroatome en tant que chaînon de cycle, et/ou peut être condensé avec un système de noyau cycloaliphatique mono ou bicyclique saturé ou insaturé, facultativement au moins monosubstitué, contenant facultativement au moins un hétéroatome en tant que chaînon de cycle,
A^{c} représente un système de noyau aromatique mono ou polycyclique facultativement au moins monosubstitué, qui peut être lié par l'intermédiaire d'un groupe alkylène facultativement au moins monosubstitué, alcénylène facultativement au moins monosubstitué ou alcynylène facultativement au moins monosubstitué, et/ou peut contenir au moins un hétéroatome en tant que chaînon de cycle dans un ou plusieurs de ses cycles,
ne représente 0, 1, 2, 3 ou 4 ;
facultativement sous la forme de l'un de leurs stéréoisomères, de préférence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport de mélange, ou un sel physiologiquement acceptable correspondant ou un solvat correspondant, et
les composés de formule générale (Id) : 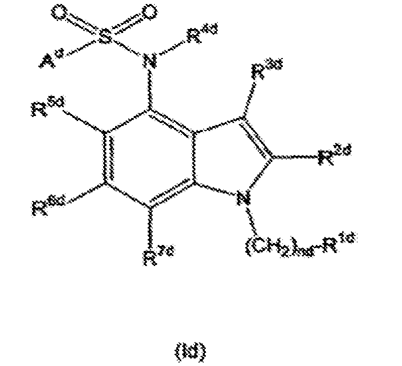
R^{1d} représente un radical -NR^{8d}R^{9d} ou un radical cycloaliphatique saturé ou insaturé, facultativement au moins monosubstitué, qui peut contenir au moins un hétéroatome en tant que chaînon de cycle, et/ou qui peut être condensé avec un système de noyau cycloaliphatique mono ou bicyclique saturé ou insaturé, facultativement au moins monosubstitué, contenant facultativement au moins un hétéroatome en tant que chaînon de cycle,
R^{2d}, R^{3d}, R^{5d}, R^{6d} et R^{7d} qui sont identiques ou différents, représentent chacun un atome d'hydrogène, un atome d'halogène, un groupe nitro, alkoxy ou cyano, un radical aliphatique saturé ou insaturé, linéaire ou ramifié, facultativement au moins monosubstitué, un radical phényle facultativement au moins monosubstitué ou un radical hétéroaryle facultativement au moins monosubstitué,
R^{4d} représente un atome d'hydrogène ou un radical aliphatique saturé ou insaturé, linéaire ou ramifié, facultativement au moins monosubstitué,
R^{8d} et R^{9d}, qui sont identiques ou différents, représentent chacun un atome d'hydrogène ou un radical aliphatique saturé ou insaturé, linéaire ou ramifié, facultativement au moins monosubstitué, ou
R^{8d} et R^{9d} forment ensemble, avec l'atome d'azote pontant, un noyau hétérocyclique saturé ou insaturé, facultativement au moins monosubstitué, qui peut contenir au moins un hétéroatome supplémentaire en tant que chaînon de cycle, et/ou peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
A^{d} représente un système de noyau aromatique mono ou polycyclique, facultativement au moins monosubstitué, qui peut être lié par l'intermédiaire d'un groupe alkylène, alcénylène ou alcynylène facultativement au moins monosubstitué, et/ou qui peut contenir au moins un hétéroatome en tant que chaînon de cycle dans un ou plusieurs de ses cycles, et
nd est égal à 0, 1, 2, 3 ou 4 ;
facultativement sous la forme de l'un de leurs stéréoisomères, de préférence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport de mélange, ou un sel de ceux-ci, de préférence, un sel physiologiquement acceptable correspondant de ceux-ci, ou un solvat correspondant de ceux-ci, et
les composés dérivés de sulfonamide de formule générale (Ie) : 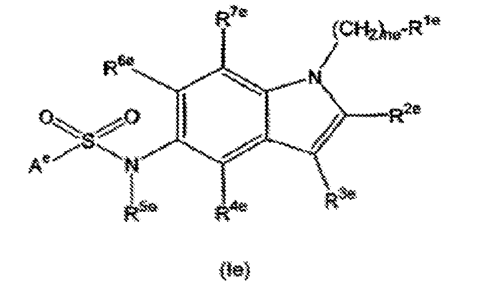
dans laquelle :
R^{1e} représente un radical -NR^{8e}R^{9e} ou un radical cycloaliphatique saturé ou insaturé, facultativement au moins monosubstitué, qui peut contenir facultativement au moins un hétéroatome en tant que chaînon de cycle, et/ou qui peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
R^{2e}, R^{3e}, R^{4e}, R^{6e} et R^{7e}, qui sont identiques ou différents, représentent chacun un atome d'hydrogène, un atome d'halogène, un groupe nitro, alkoxy ou cyano, un radical aliphatique saturé ou insaturé, linéaire ou ramifié, facultativement au moins monosubstitué, ou un radical phényle facultativement au moins monosubstitué ou un radical hétéroaryle facultativement au moins monosubstitué,
R^{5e} représente un atome d'hydrogène ou un radical aliphatique saturé ou insaturé, linéaire ou ramifié, facultativement au moins monosubstitué,
R^{8e} et R^{9e}, qui sont identiques ou différents, représentent chacun un atome d'hydrogène ou un radical aliphatique saturé ou insaturé, linéaire ou ramifié, facultativement au moins monosubstitué, ou
R^{8e} et R^{9e} forment ensemble, avec l'atome d'azote pontant, un noyau hétérocyclique saturé ou insaturé, facultativement au moins monosubstitué, qui peut contenir facultativement au moins un hétéroatome supplémentaire en tant que chaînon de cycle, et/ou peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
A^{e} représente un système de noyau aromatique mono ou polycyclique, facultativement au moins monosubstitué, qui peut être lié par l'intermédiaire d'un groupe alkylène, alcénylène ou alcynylène facultativement au moins monosubstitué, et/ou qui peut contenir au moins un hétéroatome en tant que chaînon de cycle dans un ou plusieurs de ses cycles, et
ne est égal à 0, 1, 2, 3 ou 4 ;
facultativement sous la forme de l'un de leurs stéréoisomères, de préférence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport de mélange, ou un sel physiologiquement acceptable correspondant, ou un solvat correspondant, et
les composés dérivés de sulfonamide de formule générale (If) : 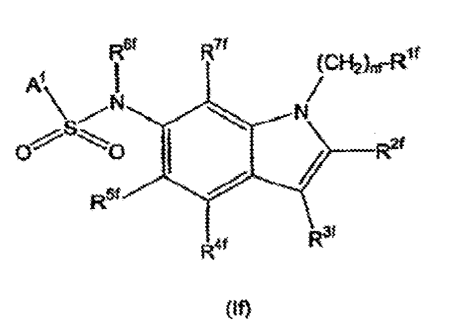
dans laquelle :
R^{1f} représente un radical -NR^{8f}R^{9f} ou un radical cycloaliphatique saturé ou insaturé, facultativement au moins monosubstitué, qui peut contenir facultativement au moins un hétéroatome en tant que chaînon de cycle, qui peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
R^{2f}, R^{3f}, R^{4f}, R^{5f} et R^{7f} qui sont identiques ou différents, représentent chacun un atome d'hydrogène, un atome d'halogène, un groupe nitro, alkoxy ou cyano, un radical aliphatique saturé ou insaturé, linéaire ou ramifié, facultativement au moins monosubstitué, un radical phényle facultativement au moins monosubstitué ou un radical hétéroaryle facultativement au moins monosubstitué,
R^{6f} représente un atome d'hydrogène ou un radical aliphatique saturé ou insaturé, linéaire ou ramifié, facultativement au moins monosubstitué,
R^{8f} et R^{9f}, qui sont identiques ou différents, représentent chacun un atome d'hydrogène ou un radical aliphatique saturé ou insaturé, linéaire ou ramifié, facultativement au moins monosubstitué, ou
R^{8f} et R^{9f} forment ensemble, avec l'atome d'azote pontant, un noyau hétérocyclique saturé ou insaturé, facultativement au moins monosubstitué, qui peut contenir au moins un hétéroatome supplémentaire en tant que chaînon de cycle, et/ou peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
A^{f} représente un système de noyau aromatique mono ou polycyclique, facultativement au moins monosubstitué, qui peut être lié par l'intermédiaire d'un groupe alkylène, alcénylène ou alcynylène facultativement au moins monosubstitué, et/ou qui peut contenir au moins un hétéroatome en tant que chaînon de cycle dans un ou plusieurs de ses cycles, et
nf est égal à 0, 1, 2, 3 ou 4 ;
facultativement sous la forme de l'un de leurs stéréoisomères, de préférence, les énantiomères ou les stéréoisomères, leur racémate, ou sous la forme d'un mélange d'au moins deux de leurs stéréoisomères, de préférence, les énantiomères ou les diastéréoisomères, en un quelconque rapport de mélange, ou un sel physiologiquement acceptable correspondant, ou un solvat correspondant, et
les composés dérivés de sulfonamide de formule générale (Ig) : 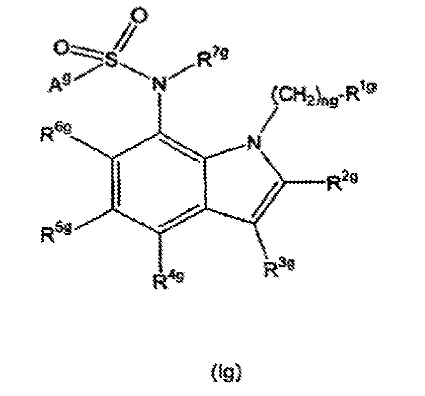
dans laquelle :
R^{1g} représente un radical -NR^{8g}R^{9g} ou un radical cycloaliphatique saturé ou insaturé, facultativement au moins monosubstitué, qui peut contenir facultativement au moins un hétéroatome en tant que chaînon de cycle, et qui peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
R^{2g}, R^{3g}, R^{4g}, R^{5g} et R^{6g}, qui sont identiques ou différents, représentent chacun un atome d'hydrogène, un atome d'halogène, un groupe nitro, alkoxy ou cyano, un radical aliphatique saturé ou insaturé, linéaire ou ramifié, facultativement au moins monosubstitué, un radical phényle facultativement au moins monosubstitué ou un radical hétéroaryle facultativement au moins
monosubstitué,
R^{7g} représente un atome d'hydrogène ou un radical aliphatique saturé ou insaturé, linéaire ou ramifié, facultativement au moins monosubstitué,
R^{8g} et R^{9g}, qui sont identiques ou différents, représentent chacun un atome d'hydrogène ou un radical aliphatique saturé ou insaturé, linéaire ou ramifié, facultativement au moins monosubstitué, ou
R^{8g} et R^{9g} forment ensemble, avec l'atome d'azote pontant, un noyau hétérocyclique saturé ou insaturé, facultativement au moins monosubstitué, qui peut contenir au moins un hétéroatome supplémentaire en tant que chaînon de cycle, et/ou peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
A^{g} représente un système de noyau aromatique mono ou polycyclique, facultativement au moins monosubstitué, qui peut être lié par l'intermédiaire d'un groupe alkylène, alcénylène ou alcynylène facultativement au moins monosubstitué, et/ou qui peut contenir au moins un hétéroatome en tant que chaînon de cycle dans un ou plusieurs de ses cycles, et
ng est égal à 0, 1, 2, 3 ou 4 ;
facultativement sous la forme de l'un de leurs stéréoisomères, de préférence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport de mélange, ou un sel physiologiquement acceptable correspondant, ou un solvat correspondant, et
les composés dérivés de sulfonamide de formule générale (Ih) : 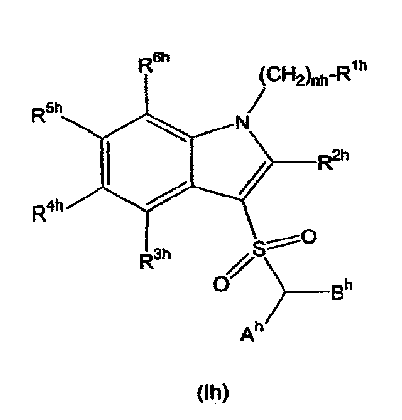 dans laquelle :
R^{1h} représente un radical -NR^{7h}R^{8h} ou un radical cycloaliphatique saturé ou insaturé, facultativement au moins monosubstitué, qui peut contenir au moins un hétéroatome en tant que chaînon de cycle, qui peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
R^{2h} , R^{3h} , R⁴h R^{5h} et R^{6h}, qui sont identiques ou différents, représentent chacun un atome d'hydrogène, un atome d'halogène, un groupe cyano ou nitro, un radical alkyle linéaire ou ramifié, un radical alcényle linéaire ou ramifié, un radical alcynyle linéaire ou ramifié, un radical alkoxy linéaire ou ramifié, un radical alkylthio linéaire ou ramifié, un groupe hydroxy ou trifluorométhyle, un radical cycloalkyle, un radical cycloalcényle, un radical alkylcarbonyle, un radical phénylcarbonyle ou un groupe -NR^{9h}R^{10h},
R^{7h} et R^{8h} , qui sont identiques ou différents, représentent chacun un atome d'hydrogène ou un radical aliphatique saturé ou insaturé, linéaire ou ramifié, facultativement au moins monosubstitué, ou
R^{7h} et R^{8h} forment ensemble, avec l'atome d'azote pontant, un noyau hétérocyclique saturé ou insaturé, facultativement au moins monosubstitué, qui peut contenir au moins un hétéroatome supplémentaire en tant que chaînon de cycle, qui peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
R^{9h} et R^{10h}, qui sont identiques ou différents, représentent chacun un atome d'hydrogène ou un radical aliphatique saturé ou insaturé, linéaire ou ramifié, facultativement au moins monosubstitué, ou
R^{9h} et R^{10h} forment ensemble, avec l'atome d'azote pontant, un noyau hétérocyclique saturé ou insaturé, facultativement au moins monosubstitué, qui peut contenir au moins un hétéroatome supplémentaire en tant que chaînon de cycle, et/ou peut être condensé avec un système de noyau cycloaliphatique mono ou bicyclique saturé ou insaturé, facultativement au moins monosubstitué, qui peut facultativement contenir au moins un hétéroatome en tant que chaînon de cycle,
A^{h} et B^{h}, qui sont identiques ou différents, représentent chacun un radical aliphatique saturé ou insaturé, linéaire ou ramifié, facultativement au moins monosubstitué, ou
A^{h} et B^{h} forment ensemble, avec l'atome de carbone auquel ils sont liés, un cycle cycloalkyle saturé ou insaturé, mais non aromatique, facultativement au moins monosubstitué, et
nh est égal à 0, 1, 2, 3 ou 4 ;
facultativement sous la forme de l'un de leurs stéréoisomères, de préférence, les énantiomères ou les diastéréoisomères, leur racémate, ou sous la forme d'un mélange d'au moins deux de leurs stéréoisomères, de préférence, les énantiomères ou les diastéréoisomères, en un quelconque rapport de mélange, ou un sel physiologiquement acceptable correspondant, ou un solvat correspondant.

2. La combinaison selon la revendication 1, **caractérisée en ce qu'**elle comprend de 1 à 99 % en poids du composant (A) et de 99 à 1 % en poids du composant (B), plus préférablement, de 10 à 80 % en poids du composant (A) et de 90 à 20 % en poids du composant (B), dans chaque cas, par rapport au poids total des deux composants (A) et (B).

3. Médicament comprenant une combinaison de substances actives selon la revendication 1 ou 2, et, facultativement, un ou plusieurs adjuvants pharmacologiquement acceptables.

4. Médicament selon la revendication 3 pour la régulation de l'appétit, pour le maintien, l'accroissement ou la diminution du poids corporel, pour la prophylaxie et/ou le traitement de troubles liés à l'ingestion d'aliments, de préférence, pour la prophylaxie et/ou le traitement de l'obésité, l'anorexie, la cachéxie, la boulimie, le diabète, de préférence, le diabète de type II (diabète sucré non-insulinodépendant), ou pour la prophylaxie et/ou le traitement de troubles de l'appareil digestif, de préférence, du syndrome du côlon irritable, pour la prophylaxie et/ou le traitement de troubles du système nerveux périphérique, de troubles du système nerveux central, de l'arthrite, de l'épilepsie, de l'anxiété, de la panique, de la dépression, de troubles cognitifs, de troubles de la mémoire, de maladies cardio-vasculaires, de processus de démence sénile, tels que les maladies d'Alzheimer, de Parkinson et/ou de Huntington, la schizophrénie, une psychose, l'hyperkinésie infantile (ADHD, insuffisance d'attention/trouble d'hyperactivité), la douleur, le syndrome hypertensif, de maladies inflammatoires, de maladies immunologiques ou pour l'amélioration de la cognition.

5. Utilisation de la combinaison selon la revendication 1 ou 2, pour la fabrication d'un médicament destiné à la régulation de l'appétit.

6. Utilisation de la combinaison selon la revendication 1 ou 2, pour la fabrication d'un médicament destiné au maintien, l'accroissement ou la diminution du poids corporel.

7. Utilisation de la combinaison selon la revendication 1 ou 2, pour la fabrication d'un médicament destiné à la prophylaxie et/ou le traitement de troubles liés à l'ingestion d'aliments, de préférence, pour la prophylaxie et/ou le traitement de l'obésité, l'anorexie, la cachéxie, la boulimie, le diabète, de préférence, le diabète de type II (diabète sucré non-insulinodépendant).

8. Utilisation de la combinaison selon la revendication 1 ou 2, pour la fabrication d'un médicament destiné à la prophylaxie et/ou le traitement de troubles de l'appareil digestif, de préférence, du syndrome du côlon irritable.

9. Utilisation de la combinaison selon la revendication 1 ou 2, pour la fabrication d'un médicament destiné à la prophylaxie et/ou le traitement de troubles du système nerveux périphérique.

10. Utilisation de la combinaison selon la revendication 1 ou 2, pour la fabrication d'un médicament destiné à la prophylaxie et/ou le traitement de troubles du système nerveux central.

11. Utilisation de la combinaison selon la revendication 1 ou 2, pour la fabrication d'un médicament destiné à la prophylaxie et/ou le traitement de l'arthrite.

12. Utilisation de la combinaison selon la revendication 1 ou 2, pour la fabrication d'un médicament destiné à la prophylaxie et/ou le traitement de l'épilepsie.

13. Utilisation de la combinaison selon la revendication 1 ou 2, pour la fabrication d'un médicament destiné à la prophylaxie et/ou le traitement de l'anxiété.

14. Utilisation de la combinaison selon la revendication 1 ou 2, pour la fabrication d'un médicament destiné à la prophylaxie et/ou le traitement de la panique.

15. Utilisation de la combinaison selon la revendication 1 ou 2, pour la fabrication d'un médicament destiné à la prophylaxie et/ou le traitement de la dépression.

16. Utilisation de la combinaison selon la revendication 1 ou 2, pour la fabrication d'un médicament destiné à la prophylaxie et/ou le traitement de troubles bipolaires.

17. Utilisation de la combinaison selon la revendication 1 ou 2, pour la fabrication d'un médicament destiné à la prophylaxie et/ou le traitement de troubles cognitifs.

18. Utilisation de la combinaison selon la revendication 1 ou 2, pour la fabrication d'un médicament destiné à la prophylaxie et/ou le traitement de troubles de la mémoire.

19. Utilisation de la combinaison selon la revendication 1 ou 2, pour la fabrication d'un médicament destiné à la prophylaxie et/ou le traitement de maladies cardio-vasculaires.

20. Utilisation de la combinaison selon la revendication 1 ou 2, pour la fabrication d'un médicament destiné à la prophylaxie et/ou le traitement du processus de démence sénile.

21. Utilisation de la combinaison selon la revendication 1 ou 2, pour la fabrication d'un médicament destiné à la prophylaxie et/ou le traitement de troubles neurodégénératifs, de préférence, la maladie d'Alzheimer, la maladie de Parkinson, la maladie de Huntington et/ou la sclérose en plaques.

22. Utilisation de la combinaison selon la revendication 1 ou 2, pour la fabrication d'un médicament destiné à la prophylaxie et/ou le traitement de la schizophrénie.

23. Utilisation de la combinaison selon la revendication 1 ou 2, pour la fabrication d'un médicament destiné à la prophylaxie et/ou le traitement d'une psychose.

24. Utilisation de la combinaison selon la revendication 1 ou 2, pour la fabrication d'un médicament destiné à la prophylaxie et/ou le traitement de l'hyperkinésie infantile (ADHD, insuffisance d'attention/trouble d'hyperactivité).

25. Utilisation de la combinaison selon la revendication 1 ou 2, pour la fabrication d'un médicament destiné à la prophylaxie et/ou le traitement de la douleur.

26. Utilisation de la combinaison selon la revendication 1 ou 2, pour la fabrication d'un médicament destiné à la prophylaxie et/ou le traitement du syndrome hypertensif.

27. Utilisation de la combinaison selon la revendication 1 ou 2, pour la fabrication d'un médicament destiné à la prophylaxie et/ou le traitement de maladies inflammatoires.

28. Utilisation de la combinaison selon la revendication 1 ou 2, pour la fabrication d'un médicament destiné à la prophylaxie et/ou le traitement de maladies immunologiques.

29. Utilisation de la combinaison selon la revendication 1 ou 2, pour la fabrication d'un médicament destiné à l'amélioration de la cognition.

30. Préparation pharmaceutique **caractérisée en ce qu'**elle comprend une combinaison de substances actives selon la revendication 1 ou 2, et, facultativement, un ou plusieurs adjuvants pharmacologiquement acceptables.

31. La préparation pharmaceutique selon la revendication 30, **caractérisée en ce qu'**elle est présente dans des formes pharmaceutiques solides, telles que des tablettes, des comprimés, des tablettes à mâcher, des gommes à mâcher, des dragées, des capsules, des suppositoires, des produits en poudre, des systèmes thérapeutiques transdermiques, des systèmes thérapeutiques transmuqueux, ou dans des formes pharmaceutiques liquides et semi-liquides, telles que des gouttes, ou telles qu'un jus, un sirop, une solution, une émulsion, une suspension, de préférence, sous la forme de comprimés, de capsules, de gouttes ou d'une solution.

32. La préparation pharmaceutique selon la revendication 30, **caractérisée en ce qu'**elle est sous la forme de multiples particules, de préférence, des microcomprimés, des microcapsules, des microsphéroïdes, des granules, des cristaux ou des pastilles, facultativement compactées en un comprimé, introduites dans une capsule ou en suspension dans un liquide approprié.

33. La préparation pharmaceutique selon une ou plusieurs des revendications 30 à 32, **caractérisée en ce qu'**elle est destinée à une application orale, intraveineuse, intramusculaire, sous cutanée, intrathécale, épidurale, buccale, sublinguale, pulmonaire, rectale, transdermique, nasale ou intracérébroventriculaire, de préférence, orale ou intraveineuse.

34. La préparation pharmaceutique selon une ou plusieurs des revendications 30 à 32, **caractérisée en ce qu'**au moins un des composants (A) (B) de la combinaison de substances actives est au moins partiellement présent sous une forme à libération prolongée.

35. La préparation pharmaceutique selon la revendication 34, **caractérisée en ce que** le médicament comporte au moins un enrobage ou au moins une matrice comprenant au moins une matière qui prolonge la libération de la substance active.

36. La préparation pharmaceutique selon la revendication 35, **caractérisée en ce que** la matière à libération prolongée est basée sur un polymère naturel, semisynthétique ou synthétique, insoluble dans l'eau, facultativement modifié, ou une cire, une matière grasse ou un alcool gras naturel, ou un acide gras semisynthétique ou synthétique, ou sur un mélange d'au moins deux de ces composants mentionnés ci-dessus.

37. La préparation pharmaceutique selon la revendication 36, **caractérisée en ce que** le polymère insoluble dans l'eau est basé sur une résine acrylique qui est, de préférence, choisie dans le groupe des poly(méth)acrylates, des poly(méth)acrylates de di(alkyle en C₁₋₄)amino(alkyle en C₁₋₄) et/ou les copolymères de ceux-ci, ou un mélange d'au moins deux des polymères mentionnés ci-dessus.

38. La préparation pharmaceutique selon la revendication 36, **caractérisée en ce que** les polymères insolubles dans l'eau sont des dérivés de cellulose, de préférence, une alkyl cellulose et, encore plus préférablement, l'éthyl cellulose, ou des esters cellulosiques.

39. La préparation pharmaceutique selon la revendication 36, **caractérisée en ce que** la cire est la cire de carnauba, la cire d'abeille, le monostéarate de glycérol, le monobéhénate de glycérol, le ditripalmitostéarate de glycérol, une cire microcristalline ou un mélange d'au moins deux de ces composants.

40. La préparation pharmaceutique selon une ou plusieurs des revendications 36 à 39, **caractérisée en ce que** les polymères ont été employés en association avec un ou plusieurs plastifiants.

41. La préparation pharmaceutique selon une ou plusieurs des revendications 34 à 40, **caractérisée en ce que**, hormis la forme à libération prolongée, au moins l'un des composants formant substance active (A) ou (B) est présent dans une forme à libération non prolongée.
